(19) Europäisches Patentamt
European Patent Office
Office européen des brevets

(11) EP 4 089 080 A1

(12) EUROPEAN PATENT APPLICATION
published in accordance with Art. 153(4) EPC

(43) Date of publication:
**16.11.2022 Bulletin 2022/46**

(21) Application number: **20912782.8**

(22) Date of filing: **31.12.2020**

(51) International Patent Classification (IPC):
***C07D 295/155*** (2006.01) ***C07D 417/04*** (2006.01)
***C07D 413/04*** (2006.01) ***A61K 31/5377*** (2006.01)
***A61K 31/5375*** (2006.01) ***A61P 19/02*** (2006.01)
***A61P 35/00*** (2006.01)

(52) Cooperative Patent Classification (CPC):
**A61K 31/40; A61K 31/4178; A61K 31/4439; A61K 31/501; A61K 31/506; A61K 31/5375; A61K 31/5377; A61P 11/06; A61P 19/02; A61P 29/00; A61P 35/00; A61P 37/02; C07D 207/12; C07D 213/75; C07D 213/82;** (Cont.)

(86) International application number:
**PCT/CN2020/141903**

(87) International publication number:
**WO 2021/139595 (15.07.2021 Gazette 2021/28)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.01.2020 CN 202010008803**

(71) Applicant: **Sunshine Lake Pharma Co., Ltd.**
**Dongguan, Guangdong 523000 (CN)**

(72) Inventors:
- **LIU, Bing**
  **Dongguan, Guangdong 523871 (CN)**
- **ZHANG, Yingjun**
  **Dongguan, Guangdong 523871 (CN)**
- **PAN, Wei**
  **Dongguan, Guangdong 523871 (CN)**
- **WANG, Feng**
  **Dongguan, Guangdong 523871 (CN)**
- **LI, Xuke**
  **Dongguan, Guangdong 523871 (CN)**
- **HE, Wei**
  **Dongguan, Guangdong 523871 (CN)**
- **HUANG, Jiuzhong**
  **Dongguan, Guangdong 523871 (CN)**

(74) Representative: **Hoffmann Eitle**
**Patent- und Rechtsanwälte PartmbB**
**Arabellastraße 30**
**81925 München (DE)**

# (54) RORγT INHIBITOR, PREPARATION METHOD THEREFOR AND USE THEREOF

(57) The present invention relates to the technical field of medicines, in particular to a RORγt inhibitor as well as a preparation method thereof and uses thereof, and also relates to a pharmaceutical composition comprising the compound, a method for preparing the pharmaceutical composition, and use of the compound or the pharmaceutical composition in the treatment or prevention of RORγt-mediated cancer, inflammation, or autoimmune diseases in mammals, especially humans.

EP 4 089 080 A1

(52) Cooperative Patent Classification (CPC): (Cont.)
**C07D 217/04; C07D 231/56; C07D 241/04;
C07D 295/155; C07D 401/04; C07D 401/14;
C07D 403/04; C07D 403/12; C07D 413/04;
C07D 417/04**

## Description

**CROSS-REFERENCE** TO RELATED APPLICATION

This application claims the priority and benefit of Chinese Patent Application No. 202010008803.7, filed with the State Intellectual Property Office of China on January 06, 2020, which is incorporated herein by reference in its entirety.

FIELD

The invention belongs to the technical field of medicines, and in particular relates to a class of small molecule compounds, compositions, preparation methods and uses thereof, wherein the compounds or compositions can be used as Retinoid-related orphan receptor gamma t (RORyt) inhibitors and used for the prevention or treatment of cancer, inflammation or immune-related diseases.

BACKGROUND

Retinoid-related orphan receptor is a subfamily of transcription factors in the superfamily of steroid hormone nuclear receptors. The retinoid-related orphan receptor family includes ROR$\alpha$, ROR$\beta$, and ROR$\gamma$, which are encoded by different genes (RORA, RORB, and RORC), respectively. Retinoid-related orphan receptor contains four major domains: an N-terminal A/B domain, a DNA-binding domain, a hinge domain, and a ligand-binding domain.

Retinoid-related orphan receptor gamma t (RORyt) is one of the two isoforms of retinoid-related orphan receptor gamma (RORy), and it is also known as RORy2. Studies have shown that RORyt is only expressed in lymphoid lineage and embryonic lymphoid tissue inducer cells (Sun et al., Science 288: 2369-2372, 2000; Eberl et al., Nat Immunol. 5: 64-73, 2004). As a characteristic transcription factor of T helper cells (Th17), RORyt plays an important role in the differentiation of Th17 cells and is a key regulator in the differentiation of Th17 cells (Ivanov, II, McKenzie BS, Zhou L, Tadokoro CE, Lepelley A, Lafaille JJ, et al. Cell 2006; 126(6): 1121-33).

Th17 can secrete interleukin 17 (IL-17) and other pro-inflammatory cytokines, which play an important role in autoimmune diseases and the body's defense response. IL-17 is a pro-inflammatory cytokine in the development of inflammation and various autoimmune diseases, and is closely related to a variety of autoimmune and inflammatory diseases, such as rheumatoid arthritis, psoriasis, psoriatic arthritis, spondyloarthritis, asthma, inflammatory bowel disease, systemic lupus erythematosus and multiple sclerosis, *etc.* (Jetten et al., Nucl. Recept. Signal, 2009, 7:e003; Manel et al., Nat. Immunol., 2008, 9, 641-649). It is also implicated in the occurrence of inflammation-related tumors, where Th17 cells are activated during disease and are responsible for recruiting other inflammatory cell types, such as neutrophils, to mediate pathology in target tissues (Korn et al., Annu. Rev. Immunol., 2009, 27:485-517).

The role of RORyt in the pathogenesis of autoimmune disease or inflammation has been extensively studied and well elucidated (Jetten et al., Adv. Dev.Biol, 2006, 16: 313-355; Meier et al. Immunity, 2007, 26:643-654; Aloisi et al., Nat. Rev. Immunol., 2006, 6:205-217; Jager et al., J. Immunol., 2009, 183:7169-7177; Barnes et al., Nat. Rev. Immunol., 2008, 8: 183-192). Therefore, the inhibition of RORyt will effectively inhibit the cell differentiation of Th17, regulate the production and secretion levels of IL-17 and other pro-inflammatory cytokines, thereby regulating the body's immune system, and treating cancer, immune and inflammatory diseases related to the regulation of RORyt.

**SUMMARY OF THE INVENTION**

The following only outlines some aspects of the present invention, and is not limited thereto. These and other sections are described more fully later. All references of this specification are incorporated herein by reference in their entirety. When there is a discrepancy between the disclosure content of this specification and the cited documents, the disclosure content of this specification shall prevail.

The invention provides a class of compounds with retinoid-related orphan receptor gamma t (RORyt) inhibitory activity, which are used in the manufacture of a medicament for preventing or treating cancer, inflammation or autoimmune diseases mediated by RORyt, such as cancer, psoriasis, rheumatoid arthritis, systemic lupus erythematosus, multiple sclerosis, inflammatory bowel disease, colitis, ulcerative colitis, rheumatoid arthritis, autoimmune eye disease, ankylosing spondylitis, asthma, chronic obstructive pulmonary disease, osteoarthritis, allergic rhinitis, atopic dermatitis, Crohn's disease, or Kawasaki disease, *etc.;* The compounds of the present invention can inhibit RORyt well and have excellent physicochemical properties and pharmacokinetic properties.

The present invention also provides methods of preparing these compounds, pharmaceutical compositions comprising these compounds, and methods of using these compounds and compositions to treat the above-mentioned diseases in mammals, especially humans.

Specifically:

In one aspect, the invention relates to a compound having Formula (I), or a stereoisomer, a geometric isomer, a tautomer, an *N*-oxide, a hydrate, a solvate, a metabolite, an ester, a pharmaceutically acceptable salt or a prodrug thereof,

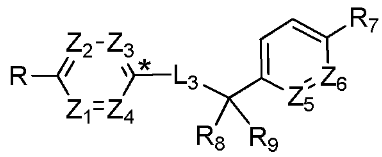

(I),

wherein,

R is $R_0$, $-(CH_2)_m$-B-$L_1$-**A or -$L_2$-G;

$Z_1$ is $CR_1$ or N; $Z_2$ is $CR_2$ or N; $Z_3$ is $CR_3$ or N; $Z_4$ is $CR_4$ or N; $Z_5$ is $CR_5$ or N; $Z_6$ is $CR_6$ or N;

each of Ro, $R_1$, $R_2$, $R_3$, $R_4$, $R_5$ and $R_6$ is independently H, deuterium, F, Cl, Br, I, $C_{1-6}$ alkyl, $C_{1-6}$ haloalkyl, $C_{1-6}$ alkoxy, hydroxy-substituted $C_{1-6}$ alkyl, hydroxy-substituted $C_{1-6}$ haloalkyl, -Si($C_{1-6}$ alkyl)$_3$, $C_{1-6}$ haloalkoxy or -N($R_dR_e$);

$R_7$ is -S(=O)$_2$-$C_{1-6}$ alkyl, -S(=O)$_2$-$C_{1-6}$ alkoxy, -S(=O)$_2$-$C_{1-6}$ alkylamino, -S(=O)$_2$-$C_{1-6}$ haloalkyl, -S(=O)$_2$-$C_{3-8}$ cycloalkyl, -S(=O)$_2$-$C_{1-6}$ alkylene-$C_{3-8}$ cycloalkyl, -S(=O)-$C_{1-6}$ alkyl, -S(=O)$_2$H, -COOH, -C(=O)-N($R_gR_h$), -N($R_g$)-C(=O)-$C_{1-6}$ alkyl, -C(=O)-O-$C_{1-6}$ alkyl, $C_{1-6}$ alkyl, $C_{1-6}$ alkoxy, $C_{1-6}$ haloalkyl or $C_{3-8}$ cycloalkyl;

each $R_g$ and $R_h$ is independently H, deuterium or $C_{1-6}$ alkyl;

each of A and G is independently $C_{3-8}$ cycloalkyl, $C_{6-10}$ aryl, 5- to 10-membered heteroaryl, or 5- to 10-membered heterocyclyl; wherein, each of A and G is independently and optionally substituted with 1, 2, 3, 4 or 5 $R_a$;

B is 4- to 10-membered heterocyclyl or thiazolyl; wherein, the 4- to 10-membered heterocyclyl is optionally substituted with 1, 2, 3, 4 or 5 $R_b$;

each $R_a$ and $R_b$ is independently deuterium, F, Cl, Br, I, -OH, -CN, -$NH_2$, -$NO_2$, -COOH, oxo, $C_{1-6}$ alkyl, $C_{1-6}$ haloalkyl, $C_{1-6}$ alkoxy, $C_{1-6}$ haloalkoxy, -$C_{1-6}$ alkylene-O-$C_{1-6}$ alkyl, $C_{3-8}$ cycloalkyl, $C_{6-10}$ aryl, 5- to 10-membered heterocyclyl, 5- to 10-membered heteroaryl or -C(=O)-N($R_dR_e$); wherein, each of the $C_{1-6}$ alkyl, $C_{1-6}$ haloalkyl, $C_{1-6}$ alkoxy, $C_{1-6}$ haloalkoxy, -$C_{1-6}$ alkylene-O-$C_{1-6}$ alkyl, $C_{3-8}$ cycloalkyl, $C_{6-10}$ aryl, 5- to 10-membered heterocyclyl and 5- to 10-membered heteroaryl is independently and optionally substituted with 1, 2 or 3 $R_c$;

each $R_c$ is independently deuterium, F, Cl, Br, I, -OH, -CN, -$NH_2$, $C_{1-6}$ alkyl, $C_{1-6}$ alkoxy, $C_{1-6}$ haloalkyl, $C_{1-6}$ haloalkoxy, $C_{3-8}$ cycloalkyl, 5- to 10-membered heterocyclyl, $C_{6-10}$ aryl or 5-to 10-membered heteroaryl;

$R_8$ is H, deuterium, -OH, -CN, -$NH_2$, -$NO_2$, -COOH, $C_{1-6}$ alkyl, $C_{1-6}$ alkoxy, $C_{1-6}$ haloalkyl, cyano-substituted $C_{1-6}$ alkyl, carboxy-substituted $C_{1-6}$ alkyl, -$C_{1-6}$ alkylene-O-$C_{1-6}$ alkyl, -$C_{1-6}$ alkylene-C(=O)-O-$C_{1-6}$ alkyl, -$C_{1-6}$ alkylene-C(=O)-N($R_dR_e$), -$C_{1-6}$ alkylene-OC(=O)-N($R_dR_e$), -$C_{1-6}$ alkylene-N($R_f$)-C(=O)-N($R_dR_e$), -$C_{1-6}$ alkylene-N($R_dR_e$) or -N($R_f$)-C(=O)-$C_{1-6}$ alkyl;

$R_9$ is deuterium, -OH, -CN, -$NH_2$, -$NO_2$, -COOH, hydroxyethyl, $C_{1-6}$ alkoxy, $C_{1-6}$ haloalkyl, cyano-substituted $C_{1-6}$ alkyl, carboxy-substituted $C_{1-6}$ alkyl, -$C_{1-6}$ alkylene-O-$C_{1-6}$ alkyl, -$C_{1-6}$ alkylene-C(=O)-O-$C_{1-6}$ alkyl, -$C_{1-6}$ alkylene-C(=O)-N($R_dR_e$), -$C_{1-6}$ alkylene-OC(=O)-N($R_dR_e$), -$C_{1-6}$ alkylene-N($R_f$)-C(=O)-N($R_dR_e$), -$C_{1-6}$ alkylene-N($R_dR_e$) or -N($R_f$)-C(=O)-$C_{1-6}$ alkyl;

wherein, each of the $C_{1-6}$ alkyl, $C_{1-6}$ alkoxy, $C_{1-6}$ haloalkyl, cyano-substituted $C_{1-6}$ alkyl, carboxy-substituted $C_{1-6}$ alkyl, -$C_{1-6}$ alkylene-O-$C_{1-6}$ alkyl and -$C_{1-6}$ alkylene-C(=O)-O-$C_{1-6}$ alkyl described in $R_8$ and $R_9$ is independently and optionally substituted with 1, 2, 3 or 4 substituents selected from deuterium, F, Cl, Br, I, -OH, -CN, -$NH_2$, -COOH, -N($R_dR_e$), $C_{1-6}$ alkyl, $C_{1-6}$ haloalkyl, $C_{1-6}$ alkoxy or $C_{1-6}$ haloalkoxy;

or, Rs and $R_9$ together with the carbon atom to which they are attached, form $C_{3-8}$ cycloalkyl or 3- to 8-membered heterocyclyl; wherein, each of the $C_{3-8}$ cycloalkyl and 3- to 8-membered heterocyclyl is independently and optionally substituted with 1, 2, 3 or 4 substituents selected from deuterium, F, Cl, Br, I, -OH, -CN, -$NH_2$, -COOH, -N($R_dR_e$), $C_{1-6}$ alkyl, $C_{1-6}$ haloalkyl, $C_{1-6}$ alkoxy or $C_{1-6}$ haloalkoxy;

each $R_d$ and $R_e$ is independently H, deuterium, -OH, $C_{1-6}$ alkyl, -C(=O)H, -C(=O)-O-$C_{1-6}$ alkyl, -C(=O)-$C_{1-6}$ alkyl, -$C_{1-6}$ alkylene-C(=O)-O-$C_{1-6}$ alkyl or -$C_{1-6}$ alkylene-O-$C_{1-6}$ alkyl; wherein, each of the $C_{1-6}$ alkyl, -C(=O)-O-$C_{1-6}$ alkyl, -C(=O)-$C_{1-6}$ alkyl, -$C_{1-6}$ alkylene-C(=O)-O-$C_{1-6}$ alkyl and -$C_{1-6}$ alkylene-O-$C_{1-6}$ alkyl is independently and optionally substituted with 1, 2, 3 or 4 substituents selected from deuterium, F, Cl, Br, I, -OH, -CN, -$NH_2$ or -COOH;

$L_1$ is a bond, **-O-, **-C(=O)-, **-NH-, **-$CH_2$-, **-$C_{1-6}$ alkylene-O-, **-O-$C_{1-6}$ alkylene-, **-C(=O)-N($R_f$)-, **-N($R_f$)-C(=O)-, **-N($R_f$)-$C_{1-6}$ alkylene- or **-$C_{1-6}$ alkylene-N($R_f$)-; wherein, each of the **-$CH_2$-, **-$C_{1-6}$ alkylene-O-, **-O-$C_{1-6}$ alkylene-, **-N($R_f$)-$C_{1-6}$ alkylene- and **-$C_{1-6}$ alkylene-N($R_f$)- is independently and optionally substituted

with 1, 2, 3 or 4 substituents selected from deuterium, oxo, halogen, $C_{1-6}$ alkyl or $C_{1-6}$ haloalkyl;

$L_2$ is a bond, -O-, -C(=O)-, -NH-, $-CH_2-$, $-C_{1-6}$ alkylene-O-, $-O-C_{1-6}$ alkylene-, $-C(=O)-N(R_f)-$, $-N(R_f)-C(=O)-$, $-N(R_f)-C_{1-6}$ alkylene- or $-C_{1-6}$ alkylene-$N(R_f)$-; wherein, each of the $-CH_2-$, $-C_{1-6}$ alkylene-O-, $-O-C_{1-6}$ alkylene-, $-N(R_f)-C_{1-6}$ alkylene- and $-C_{1-6}$ alkylene-$N(R_f)$-is independently and optionally substituted with 1, 2, 3 or 4 substituents selected from deuterium, oxo, halogen, $C_{1-6}$ alkyl or $C_{1-6}$ haloalkyl;

$L_3$ is $*-S(=O)_2-NH-$, $*-NH-S(=O)_2-$, *-S(=O)-NH-, *-NH-S(=O)-, *-C(=O)NH- or *-NHC(=O)-;

each $R_f$ is independently H, deuterium, $C_{1-6}$ alkyl, $-C_{1-6}$ alkylene-$O-C_{1-6}$ alkyl, $-C_{1-6}$ alkylene-(5- to 10-membered heterocyclyl), $-C_{1-6}$ alkylene-$C_{3-8}$ cycloalkyl, $C_{3-8}$ cycloalkyl, 3- to 8-membered heterocyclyl, -C(=O)-(3- to 8-membered heterocyclyl) or $-C(=O)-C_{3-8}$ cycloalkyl; wherein, each of the $C_{1-6}$ alkyl, $-C_{1-6}$ alkylene-$O-C_{1-6}$ alkyl, $-C_{1-6}$ alkylene-(5- to 10-membered heterocyclyl), $-C_{1-6}$ alkylene-$C_{3-8}$ cycloalkyl, $C_{3-8}$ cycloalkyl, 3- to 8-membered heterocyclyl, -C(=O)-(3- to 8-membered heterocyclyl) and $-C(=O)-C_{3-8}$ cycloalkyl is independently and optionally substituted with 1, 2 or 3 substituents selected from deuterium, F, Cl, Br, I, -OH, -CN, $-NH_2$ or COOH;

m is 0, 1 or 2.

In some embodiments, each of Ro, $R_1$, $R_2$, $R_3$, $R_4$, $R_5$ and $R_6$ is independently H, deuterium, F, Cl, Br, I, $C_{1-4}$ alkyl, $C_{1-4}$ haloalkyl, $C_{1-4}$ alkoxy, hydroxy-substituted $C_{1-4}$ alkyl, hydroxy-substituted $C_{1-4}$ haloalkyl, $-Si(C_{1-4}\ alkyl)_3$, $C_{1-4}$ haloalkoxy or $-N(R_dR_e)$.

In some embodiments, $R_7$ is $-S(=O)_2-C_{1-4}$ alkyl, $-S(=O)_2-C_{1-4}$ alkoxy, $-S(=O)_2-C_{1-4}$ alkylamino, $-S(=O)_2-C_{1-4}$ haloalkyl, $-S(=O)_2-C_{3-6}$ cycloalkyl, $-S(=O)_2-C_{1-4}$ alkylene-$C_{3-6}$ cycloalkyl, $-S(=O)-C_{1-4}$ alkyl, $-S(=O)_2H$, -COOH, $-C(=O)-N(R_gR_h)$, $-N(R_g)-C(=O)-C_{1-4}$ alkyl, $-C(=O)-O-C_{1-4}$ alkyl, $C_{1-4}$ alkyl, $C_{1-4}$ alkoxy, $C_{1-4}$ haloalkyl or $C_{3-6}$ cycloalkyl; each $R_g$ and $R_h$ is independently H, deuterium or $C_{1-4}$ alkyl.

In some embodiments, each of A and G is independently cyclopropyl, cyclobutyl, cyclopentyl, cyclohexyl, $C_{6-10}$ aryl, 5- to 10-membered heteroaryl or 5- to 7-membered heterocyclyl; wherein, each of A and G is independently and optionally substituted with 1, 2, 3, 4 or 5 $R_a$.

In some embodiments, B is 4- to 7-membered heterocyclyl; wherein, the 4- to 7-membered heterocyclyl is optionally substituted with 1, 2, 3, 4 or 5 $R_b$.

In some embodiments, each $R_a$ and $R_b$ is independently deuterium, F, Cl, Br, I, -OH, -CN, $-NH_2$, $-NO_2$, -COOH, oxo, $C_{1-4}$ alkyl, $C_{1-4}$ haloalkyl, $C_{1-4}$ alkoxy, $C_{1-4}$ haloalkoxy, $-C_{1-4}$ alkylene-$O-C_{1-4}$ alkyl, $C_{3-6}$ cycloalkyl, $C_{6-10}$ aryl, 5- to 7-membered heterocyclyl, 5- to 7-membered heteroaryl or $-C(=O)-N(R_dR_e)$; wherein, each of the $C_{1-4}$ alkyl, $C_{1-4}$ haloalkyl, $C_{1-4}$ alkoxy, $C_{1-4}$ haloalkyl, $-C_{1-4}$ alkylene-$O-C_{1-4}$ alkyl, $C_{3-6}$ cycloalkyl, $C_{6-7}$ aryl, 5- to 7-membered heterocyclyl and 5- to 7-membered heteroaryl is independently and optionally substituted with 1, 2 or 3 $R_c$.

In some embodiments, each $R_c$ is independently deuterium, F, Cl, Br, I, -OH, -CN, $-NH_2$, $C_{1-4}$ alkyl, $C_{1-4}$ alkoxy, $C_{1-4}$ haloalkyl, $C_{1-4}$ haloalkoxy, $C_{3-6}$ cycloalkyl, 5- to 7-membered heterocyclyl, $C_{6-10}$ aryl or 5- to 7-membered heteroaryl.

In some embodiments, $R_8$ is H, deuterium, -OH, -CN, $-NH_2$, $-NO_2$, -COOH, $C_{1-4}$ alkyl, $C_{1-4}$ alkoxy, $C_{1-4}$ haloalkyl, cyano-substituted $C_{1-4}$ alkyl, carboxy-substituted $C_{1-4}$ alkyl, $-C_{1-4}$ alkylene-$O-C_{1-4}$ alkyl, $-C_{1-4}$ alkylene-$C(=O)-O-C_{1-4}$ alkyl, $-C_{1-4}$ alkylene-$C(=O)-N(R_dR_e)$, $-C_{1-4}$ alkylene-$OC(=O)-N(R_dR_e)$, $-C_{1-4}$ alkylene-$N(R_f)-C(=O)-N(R_dR_e)$, $-C_{1-4}$ alkylene-$N(R_dR_e)$ or $-N(R_f)-C(=O)-C_{1-4}$ alkyl;

$R_9$ is deuterium, -OH, -CN, $-NH_2$, $-NO_2$, -COOH, $C_{1-4}$ alkoxy, $C_{1-4}$ haloalkyl, cyano-substituted $C_{1-4}$ alkyl, carboxy-substituted $C_{1-4}$ alkyl, $-C_{1-4}$ alkylene-$O-C_{1-4}$ alkyl, $-C_{1-4}$ alkylene-$C(=O)-O-C_{1-4}$ alkyl, $-C_{1-4}$ alkylene-$C(=O)-N(R_dR_e)$, $-C_{1-4}$ alkylene-$OC(=O)-N(R_dR_e)$, $-C_{1-4}$ alkylene-$N(R_f)-C(=O)-N(R_dR_e)$, $-C_{1-4}$ alkylene-$N(R_dR_e)$ or $-N(R_f)-C(=O)-C_{1-4}$ alkyl;

wherein, each of the $C_{1-4}$ alkyl, $C_{1-4}$ alkoxy, $C_{1-4}$ haloalkyl, cyano-substituted $C_{1-4}$ alkyl, carboxy-substituted $C_{1-4}$ alkyl, $-C_{1-4}$ alkylene-$O-C_{1-4}$ alkyl and $-C_{1-4}$ alkylene-$C(=O)-O-C_{1-4}$ alkyl described in $R_8$ and $R_9$ is independently and optionally substituted with 1, 2, 3 or 4 substituents selected from deuterium, F, Cl, Br, I, -OH, -CN, $-NH_2$, -COOH, $-N(R_dR_e)$, $C_{1-4}$ alkyl, $C_{1-4}$ haloalkyl, $C_{1-4}$ alkoxy or $C_{1-4}$ haloalkoxy;

or, Rs and $R_9$ together with the carbon atom to which they are attached, form $C_{3-6}$ cycloalkyl or 3- to 6-membered heterocyclyl; wherein, each of the $C_{3-6}$ cycloalkyl and 3- to 6-membered heterocyclyl is independently and optionally substituted with 1, 2, 3 or 4 substituents selected from deuterium, F, Cl, Br, I, -OH, -CN, $-NH_2$, -COOH, $-N(R_dR_e)$, $C_{1-4}$ alkyl, $C_{1-4}$ haloalkyl, $C_{1-4}$ alkoxy or $C_{1-4}$ haloalkoxy;

In some embodiments, each $R_d$ and $R_e$ is independently H, deuterium, -OH, $C_{1-4}$ alkyl, -C(=O)H, $-C(=O)-O-C_{1-4}$ alkyl, $-C(=O)-C_{1-4}$ alkyl, $-C_{1-4}$ alkylene-$C(=O)-O-C_{1-4}$ alkyl or $-C_{1-4}$ alkylene-$O-C_{1-4}$ alkyl; wherein, each of the $C_{1-4}$ alkyl, $-C(=O)-O-C_{1-4}$ alkyl, $-C(=O)-C_{1-4}$ alkyl, $-C_{1-4}$ alkylene-$C(=O)-O-C_{1-4}$ alkyl and $-C_{1-4}$ alkylene-$O-C_{1-4}$ alkyl is independently and optionally substituted with 1, 2, 3 or 4 substituents selected from deuterium, F, Cl, Br, I, -OH, -CN , $-NH_2$ or -COOH.

In some embodiments, $L_1$ is a bond, **-O-, **-C(=O)-, **-NH-, $**-CH_2-$, $**-O-C_{1-3}$ alkylene-, $**-C_{1-3}$ alkylene-O-, $**-N(R_f)-C(=O)-$, $**-C(=O)-N(R_f)-$, $**-N(R_f)-C_{1-3}$ alkylene- or $**-C_{1-3}$ alkylene-$N(R_f)$-; wherein, each of the $**-CH_2-$, **-O-

$C_{1-3}$ alkylene-, **-$C_{1-3}$ alkylene-O-, **-N($R_f$)-$C_{1-3}$ alkylene- and **-$C_{1-3}$ alkylene-N($R_f$)- is independently and optionally substituted with 1, 2, 3 or 4 substituents selected from deuterium, oxo, halogen, $C_{1-4}$ alkyl or $C_{1-4}$ haloalkyl.

In some embodiments, $L_2$ is a bond, -O-, -C(=O)-, -NH-, -$CH_2$-, -O-$C_{1-3}$ alkylene-, -$C_{1-3}$ alkylene-O-, -N($R_f$)-C(=O)-, -C(=O)-N($R_f$)-, -N($R_f$)-$C_{1-3}$ alkylene- or -$C_{1-3}$ alkylene-N($R_f$)-; wherein, each of the **-O-$C_{1-3}$ alkylene-, **-$C_{1-3}$ alkylene-O-, **-N($R_f$)-$C_{1-3}$ alkylene- and **-$C_{1-3}$ alkylene-N($R_f$)- is independently and optionally substituted with 1, 2, 3 or 4 substituents selected from deuterium, oxo, halogen, $C_{1-4}$ alkyl or $C_{1-4}$ haloalkyl.

In some embodiments, each $R_f$ is independently H, deuterium, $C_{1-4}$ alkyl, -$C_{1-4}$ alkylene-O-$C_{1-4}$ alkyl, -$C_{1-4}$ alkylene-(5- to 7-membered heterocyclyl), -$C_{1-4}$ alkylene-$C_{3-6}$ cycloalkyl, $C_{3-6}$ cycloalkyl, 3- to 6-membered heterocyclyl, -C(=O)-(3- to 6-membered heterocyclyl) or -C(=O)-$C_{3-6}$ cycloalkyl; wherein, each of the $C_{1-4}$ alkyl, -$C_{1-4}$ alkylene-O-$C_{1-4}$ alkyl, -$C_{1-4}$ alkylene-(5- to 7-membered heterocyclyl), -$C_{1-4}$ alkylene-$C_{3-6}$ cycloalkyl, $C_{3-6}$ cycloalkyl, 3- to 6-membered heterocyclyl, -C(=O)-(3- to 6-membered heterocyclyl) and -C(=O)-$C_{3-6}$ cycloalkyl is independently and optionally substituted with 1, 2 or 3 substituents selected from deuterium, F, Cl, Br, I, -OH, -CN, -$NH_2$ or COOH.

In some embodiments, each of Ro, $R_1$, $R_2$, $R_3$, $R_4$, $R_5$, and $R_6$ is independently H, deuterium, F, Cl, Br, I, methyl, ethyl, *n*-propyl, isopropyl, *n*-butyl, tert-butyl, -$CH_2F$, -$CHF_2$, -$CF_3$, -$CH_2CH_2F$, -$CH_2CHF_2$, -$CHFCH_2F$, -$CH_2CF_3$, -$CH(CF_3)_2$, -$CF_2CH_2CH_3$, -$CH_2CH_2CH_2F$, -$CH_2CH_2CHF_2$, -$CH_2CH_2CF_3$, methoxy, ethoxy, *n*-propoxy, isopropoxy, *n*-butoxy, *tert*-butoxy, hydroxymethyl, 2-hydroxyethyl, 2-hydroxypropyl, 3-hydroxypropyl, -$C(OH)(CF_3)_2$, -$Si(CH_3)_3$, -$Si(CH_2CH_3)_3$, -$OCH_2F$, -$OCHF_2$, -$OCF_3$, -$OCH_2CH_2F$, -$OCH_2CHF_2$, -$OCHFCH_2F$, -$OCH_2CF_3$, -$OCH(CF_3)_2$, -$OCF_2CH_2CH_3$, -$OCH_2CH_2CH_2F$, -$OCH_2CH_2CHF_2$, -$OCH_2CH_2CF_3$ or -N($R_dR_e$).

In some embodiments, $R_7$ is -S(=O)$_2$-$CH_3$, -S(=O)$_2$-$CH_2CH_3$, -S(=O)$_2$-$CH_2CH_2CH_3$, -S(=O)$_2$-$CH(CH_3)CH_3$, -S(=O)$_2$-$OCH_3$, -S(=O)$_2$-$OCH_2CH_3$, -S(=O)$_2$-$OCH_2CH_2CH_3$, -S(=O)$_2$-$OCH(CH_3)CH_3$, -S(=O)$_2$-cyclopropyl, -S(=O)$_2$-cyclobutyl, -S(=O)$_2$-cyclopentyl, -S(=O)$_2$-cyclohexyl, -S(=O)-$CH_2$-cyclopropyl, -S(=O)-$CH_2$-cyclobutyl, -S(=O)-$CH_2$-cyclopentyl, -S(=O)-$CH_2$-cyclohexyl, -S(=O)-$CH_3$, -S(=O)-$CH_2CH_3$, -S(=O)-$CH_2CH_2CH_3$, -S(=O)-$CH(CH_3)CH_3$, -S(=O)$_2$H, -COOH, -C(=O)-N($R_gR_h$), -N($R_g$)-C(=O)-$CH_3$, -N($R_g$)-C(=O)-$CH_2CH_3$, -N($R_g$)-C(=O)-$CH_2CH_2CH_3$, -N($R_g$)-C(=O)-$CH(CH_3)CH_3$, -C(=O)-O-$CH_3$, -C(=O)-O-$CH_2CH_3$, -C(=O)-O-$CH_2CH_2CH_3$, -C(=O)-O-$CH(CH_3)CH_3$, methyl, ethyl, *n*-propyl, isopropyl, methoxy, ethoxy, *n*-propoxy, *iso*-propoxy, -$CH_2F$, -$CHF_2$, -$CF_3$, -$CH_2CH_2F$, -$CH_2CHF_2$, -$CHFCH_2F$, -$CH_2CF_3$, -$CH(CF_3)_2$, -$CF_2CH_2CH_3$, -$CH_2CH_2CH_2F$, -$CH_2CH_2CHF_2$ or -$CH_2CH_2CF_3$;

each $R_g$ and $R_h$ is independently H, deuterium, methyl, ethyl, *n*-propyl, isopropyl, *n*-butyl, isobutyl, sec-butyl or tert-butyl.

In some embodiments, each of A and G is independently cyclopropyl, cyclobutyl, cyclopentyl, cyclohexyl, phenyl, naphthyl, thiazolyl, pyrazolyl, imidazolyl, furanyl, oxazolyl, isoxazolyl, triazolyl, thienyl, pyrrolyl, pyridyl, pyrimidinyl, morpholinyl, thiomorpholinyl, tetrahydrofuranyl, tetrahydropyranyl, pyrrolidinyl, piperidinyl, piperazinyl,

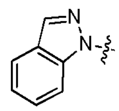, 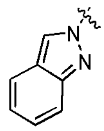,

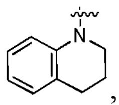, 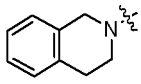, 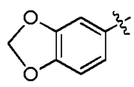 or 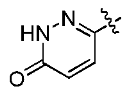;

wherein, each of A and G is independently and optionally substituted with 1, 2, 3, 4 or 5 $R_a$.

In some embodiments, B is

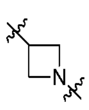, 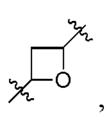, 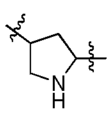, 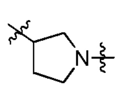, 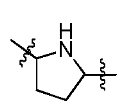, 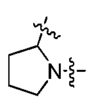,

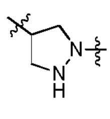, 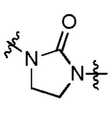, 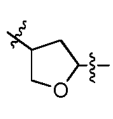, 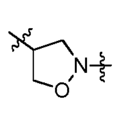, 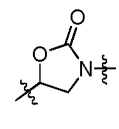, 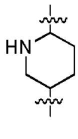, 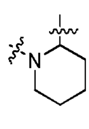, 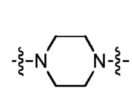, 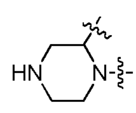,

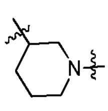, 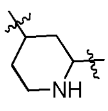, 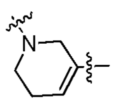, 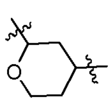, 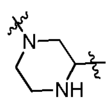, 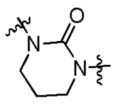, 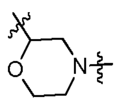, 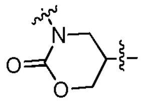, 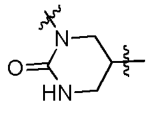,

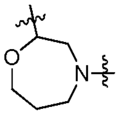, 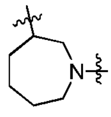, 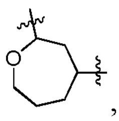, 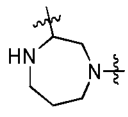 or 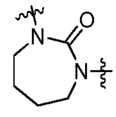;

wherein, B is optionally substituted with 1, 2, 3, 4 or 5 $R_b$.

In some embodiments, each $R_a$ and $R_b$ is independently deuterium, F, Cl, Br, I, -OH, -CN, $-NH_2$, $-NO_2$, -COOH, oxo, methyl, ethyl, *n*-propyl, isopropyl, *n*-butyl, tert-butyl, $-CH_2F$, $-CHF_2$, $-CF_3$, $-CH_2CH_2F$, $-CH_2CHF_2$, $-CHFCH_2F$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, methoxy, ethoxy, *n*-propoxy, isopropoxy, *n*-butoxy, tert-butoxy, $-OCH_2F$, $-OCHF_2$, $-OCF_3$, $-OCH_2CH_2F$, $-OCH_2CHF_2$, $-OCHFCH_2F$, $-OCH_2CF_3$, $-OCH(CF_3)_2$, $-OCF_2CH_2CH_3$, $-OCH_2CH_2CH_2F$, $-OCH_2CH_2CHF_2$, $-OCH_2CH_2CF_3$, $-CH_2OCH_3$, $-CH_2OCH_2CH_3$, $-CH_2OCH_2CH_2CH_3$, $-CH_2OCH(CH_3)_2$, $-CH_2CH_2OCH_3$, $-CH_2CH_2OCH_2CH_3$, $-CH_2CH_2OCH_2CH_2CH_3$, $-CH_2CH_2OCH(CH_3)_2$, $-CH_2CH_2CH_2OCH_3$, $-CH_2CH_2CH_2OCH_2CH_3$, $-CH_2CH_2CH_2OCH_2CH_2CH_3$, $-CH_2CH_2CH_2OCH(CH_3)_2$, cyclopropyl, cyclobutyl, cyclopentyl, cyclohexyl, phenyl, naphthyl, pyridyl, pyrimidinyl, pyrrolyl, pyrazolyl, thiazolyl, imidazolyl, oxazolyl, triazolyl, tetrazolyl, piperazinyl, piperidinyl, morpholinyl, thiomorpholinyl, tetrahydropyranyl, pyrrolidinyl or $-C(=O)-N(R_dR_e)$;

wherein, each of the methyl, ethyl, *n*-propyl, isopropyl, *n*-butyl, tert-butyl, $-CH_2F$, $-CHF_2$, $-CH_2CH_2F$, $-CH_2CHF_2$, $-CHFCH_2F$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, methoxy, ethoxy, *n*-propoxy, isopropoxy, *n*-butoxy, tert-butoxy, $-OCH_2F$, $-OCHF_2$, $-OCH_2CH_2F$, $-OCH_2CHF_2$, $-OCHFCH_2F$, $-OCH_2CF_3$, $-OCH(CF_3)_2$, $-OCF_2CH_2CH_3$, $-OCH_2CH_2CH_2F$, $-OCH_2CH_2CHF_2$, $-OCH_2CH_2CF_3$, $-CH_2OCH_3$, $-CH_2OCH_2CH_3$, $-CH_2OCH_2CH_2CH_3$, $-CH_2OCH(CH_3)_2$, $-CH_2CH_2OCH_3$, $-CH_2CH_2OCH_2CH_3$, $-CH_2CH_2OCH_2CH_2CH_3$, $-CH_2CH_2OCH(CH_3)_2$, $-CH_2CH_2CH_2OCH_3$, $-CH_2CH_2CH_2OCH_2CH_3$, $-CH_2CH_2CH_2OCH_2CH_2CH_3$, $-CH_2CH_2CH_2OCH(CH_3)_2$, cyclopropyl, cyclobutyl, cyclopentyl, cyclohexyl, phenyl, naphthyl, pyridyl, pyrimidinyl, pyrrolyl, pyrazolyl, thiazolyl, imidazolyl, oxazolyl, triazolyl, tetrazolyl, piperazinyl, piperidinyl, morpholinyl, thiomorpholinyl, tetrahydropyranyl and pyrrolidinyl is independently and optionally substituted with 1, 2 or 3 $R_c$.

In some embodiments, each $R_c$ is independently deuterium, F, Cl, Br, I, -OH, -CN, $-NH_2$, methyl, ethyl, *n*-propyl, isopropyl, *n*-butyl, tert-butyl, $-CH_2F$, $-CHF_2$, $-CF_3$, $-CH_2CH_2F$, $-CH_2CHF_2$, $-CHFCH_2F$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, methoxy, ethoxy, *n*-propoxy, isopropoxy, *n*-butoxy, tert-butoxy, $-OCH_2F$, $-OCHF_2$, $-OCF_3$, $-OCH_2CH_2F$, $-OCH_2CHF_2$, $-OCHFCH_2F$, $-OCH_2CF_3$, $-OCH(CF_3)_2$, $-OCF_2CH_2CH_3$, $-OCH_2CH_2CH_2F$, $-OCH_2CH_2CHF_2$, $-OCH_2CH_2CF_3$, cyclopropyl, cyclobutyl, cyclopentyl, cyclohexyl, phenyl, naphthyl, pyridyl, pyrimidinyl, pyrrolyl, pyrazolyl, thiazolyl, imidazolyl, oxazolyl, triazolyl, tetrazolyl, piperazinyl, piperidinyl, morpholinyl, thiomorpholinyl, tetrahydropyranyl or pyrrolidinyl.

In some embodiments, $R_8$ is H, deuterium, -OH, -CN, $-NH_2$, $-NO_2$, -COOH, methyl, ethyl, *n*-propyl, isopropyl, *n*-butyl, tert-butyl, $-CH_2F$, $-CHF_2$, $-CF_3$, $-CH_2CH_2F$, $-CH_2CHF_2$, $-CHFCH_2F$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, methoxy, ethoxy, *n*-propoxy, isopropoxy, *n*-butoxy, *tert*-butoxy, $-CH_2CN$, $-CH_2CH_2CN$, $-CH_2CH_2CH_2CN$, $-CH(CH_3)CH_2CN$, $-CH_2(CH_2)_3CN$, $-CH_2COOH$, $-CH_2CH_2COOH$, $-CH_2CH_2CH_2COOH$, $-CH(CH_3)CH_2COOH$, $-CH_2(CH_2)_3COOH$, $-CH_2OCH_3$, $-CH_2OCH_2CH_3$, $-CH_2OCH_2CH_2CH_3$, $-CH_2OCH(CH_3)_2$, $-CH_2CH_2OCH_3$, $-CH_2CH_2OCH_2CH_3$, $-CH_2CH_2OCH_2CH_2CH_3$, $-CH_2CH_2OCH(CH_3)_2$, $-CH_2CH_2CH_2OCH_3$, $-CH_2CH_2CH_2OCH_2CH_3$, $-CH_2CH_2CH_2OCH_2CH_2CH_3$, $-CH_2CH_2CH_2OCH(CH_3)_2$, $-CH_2-C(=O)-OCH_3$, $-CH_2-C(=O)-OCH_2CH_3$, $-CH_2-C(=O)-OCH_2CH_2CH_3$, $-CH_2-C(=O)-OCH(CH_3)_2$, $-CH_2CH_2-C(=O)-OCH_3$, $-CH_2CH_2-C(=O)-OCH_2CH_3$, $-CH_2CH_2-C(=O)-OCH_2CH_2CH_3$, $-CH_2CH_2-C(=O)-OCH_2(CH_3)_2$, $-CH_2CH_2CH_2-C(=O)-OCH_3$, $-CH_2CH_2CH_2-C(=O)-OCH_2CH_3$, $-CH_2CH_2CH_2-C(=O)-OCH_2CH_2CH_3$, $-CH_2CH_2CH_2-C(=O)-OCH(CH_3)_2$, $-CH_2-C(=O)-N(R_dR_e)$, $-CH_2CH_2-C(=O)-N(R_dR_e)$, $-CH_2CH_2CH_2-C(=O)-N(R_dR_e)$, $-CH_2-O-C(=O)-N(R_dR_e)$, $-CH_2CH_2-O-C(=O)-N(R_dR_e)$, $-CH_2CH_2CH_2-O-C(=O)-N(R_dR_e)$, $-CH_2-N(R_f)-C(=O)-N(R_dR_e)$, $-CH_2CH_2-N(R_f)-C(=O)-N(R_dR_e)$, $-CH_2CH_2CH_2-N(R_f)-C(=O)-N(R_dR_e)$, $-CH_2N(R_dR_e)$, $-CH_2CH_2N(R_dR_e)$, $-CH_2CH_2CH_2N(R_dR_e)$, $-N(R_f)-C(=O)-CH_3$, $-N(R_f)-C(=O)-CH_2CH_3$ or $-N(R_f)-C(=O)-CH(CH_3)_2$;

$R_9$ is deuterium, -OH, -CN, $-NH_2$, $-NO_2$, -COOH, $-CH_2F$, $-CHF_2$, $-CF_3$, $-CH_2CH_2F$, $-CH_2CHF_2$, $-CHFCH_2F$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, methoxy, ethoxy, *n*-propoxy, isopropoxy, *n*-butoxy, tert-butoxy, $-CH_2CN$, $-CH_2CH_2CN$, $-CH_2CH_2CH_2CN$, $-CH(CH_3)CH_2CN$, $-CH_2(CH_2)_3CN$, $-CH_2COOH$, $-CH_2CH_2COOH$, $-CH_2CH_2CH_2COOH$, $-CH(CH_3)CH_2COOH$, $-CH_2(CH_2)_3COOH$, $-CH_2OCH_3$, $-CH_2OCH_2CH_3$,$-CH_2OCH_2CH_2CH_3$, $-CH_2OCH(CH_3)_2$, $-CH_2CH_2OCH_3$, $-CH_2CH_2OCH_2CH_3$,

$-CH_2CH_2OCH_2CH_2CH_3$, $-CH_2CH_2OCH(CH_3)_2$, $-CH_2CH_2CH_2OCH_3$, $-CH_2CH_2CH_2OCH_2CH_3$, $-CH_2CH_2CH_2OCH_2CH_2CH_3$, $-CH_2CH_2CH_2OCH(CH_3)_2$, $-CH_2-C(=O)-OCH_3$, $-CH_2-C(=O)-OCH_2CH_3$, $-CH_2-C(=O)-OCH_2CH_2CH_3$, $-CH_2-C(=O)-OCH(CH_3)_2$, $-CH_2CH_2-C(=O)-OCH_3$, $-CH_2CH_2-C(=O)-OCH_2CH_3$, $-CH_2CH_2-C(=O)-OCH_2CH_2CH_3$, $-CH_2CH_2-C(=O)-OCH_2(CH_3)_2$, $-CH_2CH_2CH_2-C(=O)-OCH_3$, $-CH_2CH_2CH_2-C(=O)-OCH_2CH_3$, $-CH_2CH_2CH_2-C(=O)-OCH_2CH_2CH_3$, $-CH_2CH_2CH_2-C(=O)-OCH(CH_3)_2$, $-CH_2-C(=O)-N(R_dR_e)$, $-CH_2CH_2-C(=O)-N(R_dR_e)$, $-CH_2CH_2CH_2-C(=O)-N(R_dR_e)$, $-CH_2-O-C(=O)-N(R_dR_e)$, $-CH_2CH_2-O-C(=O)-N(R_dR_e)$, $-CH_2CH_2CH_2-O-C(=O)-N(R_dR_e)$, $-CH_2-N(R_f)-C(=O)-N(R_dR_e)$, $-CH_2CH_2-N(R_f)-C(=O)-N(R_dR_e)$, $-CH_2CH_2CH_2-N(R_f)-C(=O)-N(R_dR_e)$, $-CH_2N(R_dR_e)$, $-CH_2CH_2N(R_dR_e)$, $-CH_2CH_2CH_2N(R_dR_e)$, $-N(R_f)-C(=O)-CH_3$, $-N(R_f)-C(=O)-CH_2CH_3$ or $-N(R_f)-C(=O)-CH(CH_3)_2$;

wherein, each of methyl, ethyl, *n*-propyl, isopropyl, *n*-butyl, *tert*-butyl, $-CH_2F$, $-CHF_2$, $-CH_2CH_2F$, $-CH_2CHF_2$, $-CHFCH_2F$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, methoxy, ethoxy, *n*-propoxy, isopropoxy, *n*-butoxy, *tert*-butoxy, $-CH_2CN$, $-CH_2CH_2CN$, $-CH_2CH_2CH_2CN$, $-CH(CH_3)CH_2CN$, $-CH_2(CH_2)_3CN$, $-CH_2COOH$, $-CH_2CH_2COOH$, $-CH_2CH_2CH_2COOH$, $-CH(CH_3)CH_2COOH$, $-CH_2(CH_2)_3COOH$, $-CH_2OCH_3$, $-CH_2OCH_2CH_3$,$-CH_2OCH_2CH_2CH_3$, $-CH_2OCH(CH_3)_2$, $-CH_2CH_2OCH_3$, $-CH_2CH_2OCH_2CH_3$, $-CH_2CH_2OCH_2CH_2CH_3$, $-CH_2CH_2OCH(CH_3)_2$, $-CH_2CH_2CH_2OCH_3$, $-CH_2CH_2CH_2OCH_2CH_3$, $-CH_2CH_2CH_2OCH_2CH_2CH_3$, $-CH_2CH_2CH_2OCH(CH_3)_2$, $-CH_2-C(=O)-OCH_3$, $-CH_2-C(=O)-OCH_2CH_3$, $-CH_2-C(=O)-OCH_2CH_2CH_3$, $-CH_2-C(=O)-OCH(CH_3)_2$, $-CH_2CH_2-C(=O)-OCH_3$, $-CH_2CH_2-C(=O)-OCH_2CH_3$, $-CH_2CH_2-C(=O)-OCH_2CH_2CH_3$, $-CH_2CH_2-C(=O)-OCH(CH_3)_2$, $-CH_2CH_2CH_2-C(=O)-OCH_3$, $-CH_2CH_2CH_2-C(=O)-OCH_2CH_3$, $-CH_2CH_2CH_2-C(=O)-OCH_2CH_2CH_3$ and $-CH_2CH_2CH_2-C(=O)-OCH(CH_3)_2$ described in $R_8$ and $R_9$ is independently and optionally substituted with 1, 2, 3 or 4 substituents selected from deuterium, F, Cl, Br, I, -OH, -CN, $-NH_2$, -COOH, methyl, ethyl, *n*-propyl, isopropyl , *n*-butyl, tert-butyl, trifluoromethyl, methoxy, ethoxy, *n*-propoxy, isopropoxy, *n*-butoxy, tert-butoxy, trifluoromethoxy or $-N(R_dR_e)$;

or, Rs and $R_9$ together with the carbon atom to which they are attached, form cyclopentyl, cyclohexyl, oxetanyl, tetrahydrofuranyl, tetrahydropyranyl, azetidinyl, pyrrolidinyl, piperidinyl or piperazinyl; wherein, each of the cyclopentyl, cyclohexyl, oxetanyl, tetrahydrofuranyl, tetrahydropyranyl, azetidinyl, pyrrolidinyl, piperidinyl and piperazinyl is independently and optionally substituted with 1, 2, 3 or 4 substituents selected from deuterium, F, Cl, Br, I, -OH, -CN, -NH2, -COOH, methyl, ethyl, *n*-propyl, isopropyl, *n*-butyl, tert-butyl, trifluoromethyl, methoxy, ethoxy, *n*-propoxy, isopropoxy, *n*-butoxy, tert-butoxy, trifluoromethoxy or $-N(R_dR_e)$.

In some embodiments, each $R_d$ and $R_e$ is independently H, deuterium, -OH, methyl, ethyl, *n*-propyl, isopropyl, *n*-butyl, tert-butyl, -C(=O)H, $-C(=O)-O-CH_3$, $-C(=O)-O-CH_2CH_3$, $-C(=O)-O-CH_2CH_2CH_3$, $-C(=O)-O-CH(CH_3)_2$, $-C(=O)-CH_3$, $-C(=O)-CH_2CH_3$, $-C(=O)-CH_2CH_2CH_3$, $-C(=O)-CH(CH_3)_2$, $-CH_2OCH_3$, $-CH_2OCH_2CH_3$,$-CH_2OCH_2CH_2CH_3$, $-CH_2OCH(CH_3)_2$, $-CH_2CH_2OCH_3$, $-CH_2CH_2OCH_2CH_3$,$-CH_2CH_2OCH_2CH_2CH_3$, $-CH_2CH_2OCH(CH_3)_2$, $-CH_2CH_2CH_2OCH_3$, $-CH_2CH_2CH_2OCH_2CH_3$, $-CH_2CH_2CH_2OCH_2CH_2CH_3$, $-CH_2CH_2CH_2OCH(CH_3)_2$, $-CH_2-C(=O)-OCH_3$, $-CH_2-C(=O)-OCH_2CH_3$, $-CH_2-C(=O)-OCH_2CH_2CH_3$, $-CH_2-C(=O)-OCH(CH_3)_2$, $-CH_2CH_2-C(=O)-OCH_3$, $-CH_2CH_2-C(=O)-OCH_2CH_3$, $-CH_2CH_2-C(=O)-OCH_2CH_2CH_3$, $-CH_2CH_2-C(=O)-OCH(CH_3)_2$, $-CH_2CH_2CH_2-C(=O)-OCH_3$, $-CH_2CH_2CH_2-C(=O)-OCH_2CH_3$, $-CH_2CH_2CH_2-C(=O)-OCH_2CH_2CH_3$ or $-CH_2CH_2CH_2-C(=O)-OCH(CH_3)_2$;

wherein, each of the methyl, ethyl, *n*-propyl, isopropyl, *n*-butyl, *tert*-butyl, $-C(=O)-O-CH_3$, $-C(=O)-O-CH_2CH_3$, $-C(=O)-O-CH_2CH_2CH_3$, $-C(=O)-O-CH(CH_3)_2$, $-C(=O)-CH_3$, $-C(=O)-CH_2CH_3$, $-C(=O)-CH_2CH_2CH_3$, $-C(=O)-CH(CH_3)_2$, $-CH_2OCH_3$, $-CH_2OCH_2CH_3$, $-CH_2OCH_2CH_2CH_3$, $-CH_2OCH(CH_3)_2$, $-CH_2CH_2OCH_3$, $-CH_2CH_2OCH_2CH_3$, $-CH_2CH_2OCH_2CH_2CH_3$, $-CH_2CH_2OCH(CH_3)_2$, $-CH_2CH_2CH_2OCH_3$, $-CH_2CH_2CH_2OCH_2CH_3$, $-CH_2CH_2CH_2OCH_2CH_2CH_3$, $-CH_2CH_2CH_2OCH(CH_3)_2$, $-CH_2-C(=O)-OCH_3$, $-CH_2-C(=O)-OCH_2CH_3$, $-CH_2-C(=O)-OCH_2CH_2CH_3$, $-CH_2-C(=O)-OCH(CH_3)_2$, $-CH_2CH_2-C(=O)-OCH_3$, $-CH_2CH_2-C(=O)-OCH_2CH_3$, $-CH_2CH_2-C(=O)-OCH_2CH_2CH_3$, $-CH_2CH_2-C(=O)-OCH(CH_3)_2$, $-CH_2CH_2CH_2-C(=O)-OCH_3$, $-CH_2CH_2CH_2-C(=O)-OCH_2CH_3$, $-CH_2CH_2CH_2-C(=O)-OCH_2CH_2CH_3$ and $-CH_2CH_2CH_2-C(=O)-OCH(CH_3)_2$ is independently and optionally substituted with 1, 2, 3 or 4 substituents selected from deuterium, F, Cl, Br, I, -OH, -CN, $-NH_2$ or -COOH.

In some embodiments, L1 is a bond, **-O-, **-C(=O)-, **-NH-, $**-CH_2-$, $**-CH_2O-$, $**-CH_2CH_2O-$, $**-O-CH_2-$, $**-O-CH_2CH_2-$, $**-C(=O)-N(R_f)-$, $**-N(R_f)-C(=O)-$, $**-N(R_f)-CH_2-$, $**-N(R_f)-CH_2CH_2-$, $**-CH_2-N(R_f)-$ or $**-CH_2CH_2-N(R_f)-$; wherein, each of the $**-CH_2-$, $**-CH_2O-$, $**-CH_2CH_2O-$, $**-O-CH_2-$, $**-O-CH_2CH_2-$, $**-N(R_f)-CH_2-$, $**-N(R_f)-CH_2CH_2-$, $**-CH_2-N(R_f)-$ and $**-CH_2CH_2-N(R_f)-$ is independently and optionally substituted with 1, 2, 3 or 4 substituents selected from deuterium, oxo, F, Cl, Br, methyl, ethyl, *n*-propyl, isopropyl, *n*-butyl, isobutyl, *tert*-butyl, $-CH_2F$, $-CHF_2$, $-CF_3$, $-CH_2CH_2F$, $-CH_2CHF_2$, $-CHFCH_2F$, $-CH_2CF_3$, $-CH(CF_3)_2$, $-CF_2CH_2CH_3$, $-CH_2CH_2CH_2F$, $-CH_2CH_2CHF_2$ or $-CH_2CH_2CF_3$.

In some embodiments, $L_2$ is a bond, -O-, -C(=O)-, -NH-, $-CH_2-$, $-CH_2O-$, $-CH_2CH_2O-$, $-O-CH_2-$, $-O-CH_2CH_2-$, $-C(=O)-N(R_f)-$, $-N(R_f)-C(=O)-$, $-N(R_f)-CH_2-$, $-N(R_f)-CH_2CH_2-$, $-CH_2-N(R_f)-$ or $-CH_2CH_2-N(R_f)-$; wherein, each of the $-CH_2-$, $-CH_2O-$, $-CH_2CH_2O-$, $-O-CH_2-$, $-O-CH_2CH_2-$, $-N(R_f)-CH_2-$, $-N(R_f)-CH_2CH_2-$, $-CH_2-N(R_f)-$ and $-CH_2CH_2-N(R_f)-$ is independently and optionally substituted with 1, 2, 3 or 4 substituents selected from deuterium, oxo, F, Cl, Br, methyl, ethyl,

*n*-propyl, isopropyl, *n*-butyl, isobutyl, tert-butyl, $-CH_2F$, $-CHF_2$, $-CF_3$, $-CH_2CH_2F$, $-CH_2CHF_2$, $-CHFCH_2F$, $-CH_2CF_3$, $-CH(CF_3)_2$, $-CF_2CH_2CH_3$, $-CH_2CH_2CH_2F$, $-CH_2CH_2CHF_2$ or $-CH_2CH_2CF_3$.

In some embodiments, each $R_f$ is independently H, deuterium, methyl, ethyl, *n*-propyl, isopropyl, *n*-butyl, isobutyl, *tert*-butyl, $-CH_2OH_3$, $-CH_2OH_2CH_3$, $-CH_2OH_2CH_2CH_3$, $-CH_2OH(CH_3)_2$, $-CH_2CH_2OH_3$, $-CH_2CH_2OH_2CH_3$, $-CH_2CH_2OH_2CH_2CH_3$, $-CH_2CH_2OH(CH_3)_2$, $-CH_2CH_2CH_2OH_3$, $-CH_2CH_2CH_2OH_2CH_3$, $-CH_2CH_2CH_2OH_2CH_2CH_3$, $-CH_2CH_2CH_2OH(CH_3)_2$, tetrahydrofurylmethylene, tetrahydropyranylmethylene, pyrrolidinylmethylene, piperazinylmethylene, cyclopropylmethylene, cyclopropylethylene, cyclopropyl-*n*-propylene, cyclobutylmethylene, cyclobutylethylene, cyclobutyl-*n*-propylene, cyclopentylmethylene, cyclopentylethylene, cyclopentyl-*n*-propylene, cyclohexylmethylene, cyclohexylethylene, cyclohexyl-*n*-propylene, cyclopropyl, cyclobutyl, cyclopentyl, cyclohexyl, oxiranyl, aziridinyl, oxetanyl, azetidinyl, pyrrolidinyl, tetrahydrofuranyl, tetrahydrothienyl, piperazinyl, piperidinyl, morpholinyl, thiomorpholinyl, tetrahydropyranyl, -C(=O)-cyclopropyl, -C(=O)-cyclobutyl, -C(=O)-cyclopentyl or -C(=O)-cyclohexyl;
wherein, each of the methyl, ethyl, *n*-propyl, isopropyl, *n*-butyl, isobutyl, tert-butyl, $-CH_2OH_3$, $-CH_2OH_2CH_3$, $-CH_2OH_2CH_2CH_3$, $-CH_2OH(CH_3)_2$, $-CH_2CH_2OH_3$, $-CH_2CH_2OH_2CH_3$, $-CH_2CH_2OH_2CH_2CH_3$, $-CH_2CH_2OH(CH_3)_2$, $-CH_2CH_2CH_2OH_3$, $-CH_2CH_2CH_2OH_2CH_3$, $-CH_2CH_2CH_2OH_2CH_2CH_3$, $-CH_2CH_2CH_2OH(CH_3)_2$, tetrahydrofurylmethylene, tetrahydropyranylmethylene, pyrrolidinylmethylene, piperazinylmethylene, cyclopropylmethylene, cyclopropylethylene, cyclopropyl-*n*-propylene, cyclobutylmethylene, cyclobutylethylene, cyclobutyl-*n*-propylene, cyclopentylmethylene, cyclopentylethylene, cyclopentyl-*n*-propylene, cyclohexylmethylene, cyclohexylethylene, cyclohexyl-*n*-propylene, cyclopropyl, cyclobutyl, cyclopentyl, cyclohexyl, oxiranyl, aziridinyl, oxetanyl, azetidinyl, pyrrolidinyl, tetrahydrofuranyl, tetrahydrothienyl, piperazinyl, piperidinyl, morpholinyl, thiomorpholinyl, tetrahydropyranyl, -C(=O)-cyclopropyl, -C(=O)-cyclobutyl, -C(=O)-cyclopentyl and -C(=O)-cyclohexyl is independently and optionally substituted with 1, 2 or 3 substituents selected from deuterium, F, Cl, Br, I, -OH, -CN, $-NH_2$ or -COOH.

In some embodiments, the present invention provides a compound having Formula (II), or a stereoisomer, a geometric isomer, a tautomer, an *N*-oxide, a hydrate, a solvate, a metabolite, an ester, a pharmaceutically acceptable salt or a prodrug thereof,

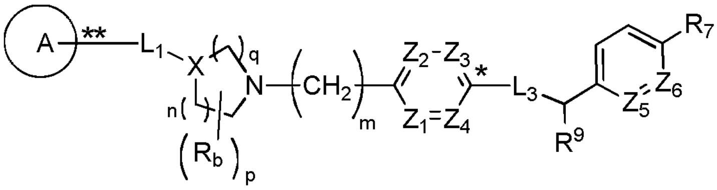

(II),

wherein, n is 0, 1 or 2; p is 0, 1, 2, 3 or 4; q is 1 or 2; X is N or CH.

In some embodiments, the present invention provides a compound having Formula (III) or a stereoisomer, a geometric isomer, a tautomer, an *N*-oxide, a hydrate, a solvate, a metabolite, an ester, a pharmaceutically acceptable salt or a prodrug thereof,

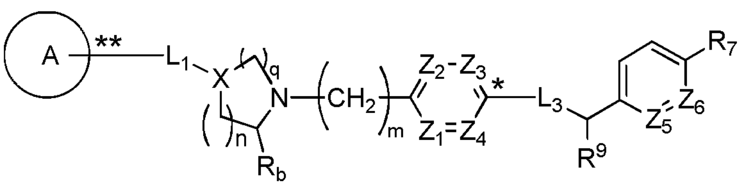

(III),

wherein, n is 0, 1 or 2; q is 1 or 2; X is N or CH.

In some embodiments, the present invention provides a compound having Formula (IV) or Formula (V), or a stereoisomer, a geometric isomer, a tautomer, an *N*-oxide, a hydrate, a solvate, a metabolite, an ester, a pharmaceutically acceptable salt or a prodrug thereof,

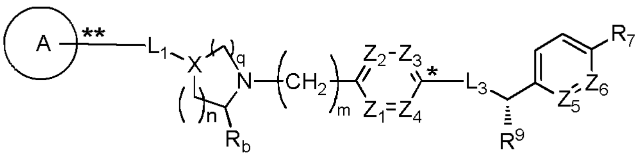

(IV)

or

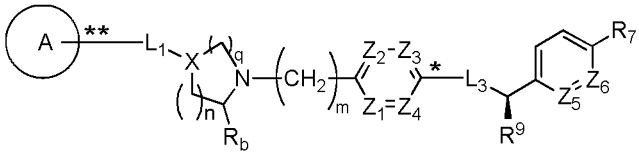

(V),

wherein, n is 0, 1 or 2; q is 1 or 2; X is N or CH.

In another aspect, the present invention relates to a pharmaceutical composition comprising a compound having formula (I), formula (II), formula (III), formula (IV) or formula (V), or a stereoisomer, a geometric isomer, a tautomer, an *N*-oxide, a hydrate, a solvate, a metabolite, a pharmaceutically acceptable salt or a prodrug thereof, and a pharmaceutically acceptable excipient, a carrier, an adjuvant or a combination thereof.

In some embodiments, the pharmaceutical composition further comprises other drugs for preventing or treating inflammatory syndromes, disorders or diseases or any combination thereof.

In another aspect, the present invention relates to use of the compound having formula (I), formula (II), formula (III), formula (IV) or formula (V), or a pharmaceutical composition thereof in the manufacture of a medicament for preventing or treating cancer, inflammation or autoimmune diseases mediated by RORγt in mammals, including humans.

In some embodiments, the present invention relates to use of the compound having formula (I), formula (II), formula (III), formula (IV) or formula (V), or a pharmaceutical composition thereof in the manufacture of a medicament for preventing or treating cancer, psoriasis, rheumatoid arthritis, systemic lupus erythematosus, multiple sclerosis, inflammatory bowel disease, colitis, ulcerative colitis, rheumatoid arthritis, autoimmune eye disease, ankylosing spondylitis, asthma, chronic obstructive pulmonary disease, osteoarthritis, allergic rhinitis, atopic dermatitis, Crohn's disease, or Kawasaki disease.

In another aspect, the present invention relates to a method of preparing, separating or purifying the compound having formula (I), formula (II), formula (III), formula (IV) or formula (V).

The biological test results show that the compounds provided by the present invention have good inhibitory activity on RORγt, and also have good pharmacokinetic characteristics.

Any embodiment disclosed herein can be combined with other embodiments as long as they are not contradictory to one another, even though the embodiments are described under different aspects of the invention. In addition, any technical feature in one embodiment can be applied to the corresponding technical feature in other embodiments as long as they are not contradictory to one another, even though the embodiments are described under different aspects of the invention.

The foregoing merely summarizes certain aspects disclosed herein and is not intended to be limiting in nature. These aspects and other aspects and embodiments are described more fully below.

## DETAILED DESCRIPTION OF THE INVENTION

## DEFINITIONS AND GENERAL TERMINOLOGY

Reference will now be made in detail to certain embodiments of the invention, examples of which are illustrated in the accompanying structures and formulas. The invention is intended to cover all alternatives, modifications, and equivalents which may be included within the scope of the present invention as defined by the claims. One skilled in the art will recognize many methods and materials similar or equivalent to those described herein, which could be used in the practice of the present invention. The present invention is in no way limited to the methods and materials described herein. In the event that one or more of the incorporated literature, patents, and similar materials differs from or contradicts this application, including but not limited to defined terms, term usage, described techniques, or the like, this application controls.

It is further appreciated that certain features of the invention, which are, for clarity, described in the context of separate embodiments, can also be provided in combination in a single embodiment. Conversely, various features of the invention which are, for brevity, described in the context of a single embodiment, can also be provided separately or in any suitable subcombination.

Unless defined otherwise, all technical and scientific terms used herein have the same meaning as is commonly understood by one skilled in the art to which this invention belongs. All patents and publications referred to herein are incorporated by reference in their entirety.

As used herein, the following definitions shall apply unless otherwise indicated. For purposes of this invention, the chemical elements are identified in accordance with the Periodic Table of the Elements, CAS version, and the

Handbook of Chemistry and Physics, 75th Ed. 1994. Additionally, general principles of organic chemistry are described in "Organic Chemistry", Thomas Sorrell, University Science Books, Sausalito: 1999, and "March's Advanced Organic Chemistry" by Michael B. Smith and Jerry March, John Wiley & Sons, New York: 2007, the entire contents of which are hereby incorporated by reference.

The grammatical articles "a", "an" and "the", as used herein, are intended to include "at least one" or "one or more" unless otherwise indicated herein or clearly contradicted by the context. Thus, the articles used herein refer to one or more than one *(i.e.* at least one) articles of the grammatical objects. By way of example, "a component" means one or more components, and thus, possibly, more than one component is contemplated and may be employed or used in an implementation of the described embodiments.

As used herein, the term "subject" refers to an animal. Typically the animal is a mammal. A subject also refers to for example, primates (*e.g*, humans, male or female), cows, sheep, goats, horses, dogs, cats, rabbits, rats, mice, fish, birds and the like. In certain embodiments, the subject is a primate. In yet other embodiments, the subject is a human.

As used herein, "patient" refers to a human (including adults and children) or other animal. In some embodiments, "patient" refers to a human.

The term "comprise" is an open expression, it means comprising the contents disclosed herein, but don't exclude other contents.

"Stereoisomers" refers to compounds which have identical chemical constitution, but differ with regard to the arrangement of the atoms or groups in space. Stereoisomers include enantiomer, diastereomers, conformer (rotamer), geometric (cis/trans) isomer, atropisomer, *etc.*

"Chiral" refers to molecules which have the property of non-superimposability of the mirror image partner, while the term "achiral" refers to molecules which are superimposable on their mirror image partner.

"Enantiomers" refers to two stereoisomers of a compound which are non-superimposable mirror images of one another.

"Diastereomer" refers to a stereoisomer with two or more centers of chirality and whose molecules are not mirror images of one another. Diastereomers have different physical properties, *e.g.*, melting points, boling points, spectral properties or biological activities. Mixture of diastereomers may separate under high resolution analytical procedures such as electrophoresis and chromatography such as HPLC.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994.

Many organic compounds exist in optically active forms, *i.e.,* they have the ability to rotate the plane of plane-polarized light. In describing an optically active compound, the prefixes *D* and *L,* or R and S, are used to denote the absolute configuration of the molecule about its chiral center(s). The prefixes *d* and *l* or (+) and (-) are employed to designate the sign of rotation of plane-polarized light by the compound, with (-) or *l* meaning that the compound is levorotatory. A compound prefixed with (+) or *d* is dextrorotatory. A specific stereoisomer may be referred to as an enantiomer, and a mixture of such stereoisomers is called an enantiomeric mixture. A 50:50 mixture of enantiomers is referred to as a racemic mixture or a racemate, which may occur where there has been no stereoselection or stereospecificity in a chemical reaction or process.

Any asymmetric atom (*e.g.*, carbon or the like) of the compound(s) disclosed herein can be present in racemic or enantiomerically enriched, for example the *(R)-,* (S)- or (*R,S*)-configuration. In certain embodiments, each asymmetric atom has at least 50 % enantiomeric excess, at least 60 % enantiomeric excess, at least 70 % enantiomeric excess, at least 80 % enantiomeric excess, at least 90 % enantiomeric excess, at least 95 % enantiomeric excess, or at least 99 % enantiomeric excess in the (R)- or (*S*)-configuration.

Depending on the choice of the starting materials and procedures, the compounds can be present in the form of one of the possible stereoisomers or as mixtures thereof, such as racemates and diastereoisomer mixtures, depending on the number of asymmetric carbon atoms. Optically active (*R*)- and (*S*)-isomers may be prepared using chiral synthons or chiral reagents, or resolved using conventional techniques. If the compound contains a double bond, the substituent may be E or Z configuration. If the compound contains a disubstituted cycloalkyl, the cycloalkyl substituent may have a cis- or trans-configuration.

Any resulting mixtures of stereoisomers can be separated on the basis of the physicochemical differences of the constituents, into the pure or substantially pure geometric isomers, enantiomers, diastereomers, for example, by chromatography and/or fractional crystallization. Cis and trans isomers are diastereomer.

Any resulting racemates of final products or intermediates can be resolved into the optical antipodes by methods known to those skilled in the art, *e.g.,* by separation of the diastereomeric salts thereof. Racemic products can also be resolved by chiral chromatography, *e.g.,* high performance liquid chromatography (HPLC) using a chiral adsorbent. Preferred enantiomers can also be prepared by asymmetric syntheses. See, for example, Jacques, et al., Enantiomers, Racemates and Resolutions (Wiley Interscience, New York, 1981); Principles of Asymmetric Synthesis (2nd Ed. Robert E. Gawley, Jeffrey Aube, Elsevier, Oxford, UK, 2012); Eliel, E.L. Stereochemistry of Carbon Compounds (McGraw-Hill,

NY, 1962); Wilen, S.H. Tables of Resolving Agents and Optical Resolutions p. 268 (E.L. Eliel, Ed., Univ. of Notre Dame Press, Notre Dame, IN 1972); Chiral Separation Techniques: A Practical Approach (Subramanian, G. Ed., Wiley-VCH Verlag GmbH & Co. KGaA, Weinheim, Germany, 2007).

As described herein, compounds disclosed herein may optionally be substituted with one or more substituents, such as are illustrated generally below, or as exemplified by particular classes, subclasses, and species of the invention.

In general, the term "substituted" refers to the replacement of one or more hydrogen radicals in a given structure with the radical of a specified substituent. Unless otherwise indicated, an optionally substituted group may have a substituent at each substitutable position of the group. When more than one position in a given structure can be substituted with more than one substituent selected from a specified group, the substituent may be either the same or different at each position.

The term "unsubstituted" means that the specified group has no substituents.

The term "optionally substituted" may be used interchangeably with the term "unsubstituted or substituted", *i.e.,* the structure is unsubstituted or substituted with one or more substituents described in the present invention.

Furthermore, what need to be explained is that the phrases "each...is independently" and "each of...and...is independently", unless otherwise stated, should be broadly understood, which can mean that the specific options expressed by the same symbol are independent of each other in different groups; or the specific options expressed by the same symbol are independent of each other in same groups.

At each part of the present specification, substitutes of compounds disclosed herein are disclosed in groups or in ranges. It is specifically intended that the invention includes each and every individual subcombination of the members of such groups and ranges. For example, the term "$C_{1-6}$ alkyl" is specifically intended to individually disclose methyl, ethyl, $C_3$ alkyl, $C_4$ alkyl, $C_5$ alkyl, and $C_6$ alkyl.

At various places in the present specification, linking substituents are described. Where the structure clearly requires a linking group, the Markush variables listed for that group are understood to be linking groups. For example, if the structure requires a linking group and the Markush group definition for that variable lists "alkyl" or "aryl" then it is understood that the "alkyl" or "aryl" represents a linking alkylene group or arylene group, respectively.

The term "alkyl" or "alkyl group" refers to a saturated linear or branched-chain monovalent hydrocarbon group, wherein the alkyl group is optionally substituted with one or more substituents described herein. Unless otherwise stated, the alkyl group contains 1-20 carbon atoms. In one embodiment, the alkyl group contains 1-12 carbon atoms. In another embodiment, the alkyl group contains 3-12 carbon atoms. In another embodiment, the alkyl group contains 1-6 carbon atoms. In still other embodiment, the alkyl group contains 1-4 carbon atoms. In yet other embodiment, the alkyl group contains 1-3 carbon atoms.

Some non-limiting examples of the alkyl group include, methyl (Me, $-CH_3$), ethyl (Et, $-CH_2CH_3$), *n*-propyl (*n*-Pr, $-CH_2CH_2CH_3$), isopropyl (*i*-Pr, $-CH(CH_3)_2$), *n*-butyl (*n*-Bu, $-CH_2CH_2CH_2CH_3$), isobutyl (*i*-Bu, $-CH_2CH(CH_3)_2$), *sec*-butyl (*s*-Bu, $-CH(CH_3)CH_2CH_3$), *tert*-butyl (*t*-Bu, $-C(CH_3)_3$), *n*-pentyl ($-CH_2CH_2CH_2CH_2CH_3$), 2-pentyl ($-CH(CH_3)CH_2CH_2CH_3$), 3-pentyl ($-CH(CH_2CH_3)_2$), 2-methyl-2-butyl ($-C(CH_3)_2CH_2CH_3$), 3-methyl-2-butyl ($-CH(CH_3)CH(CH_3)_2$), 3-methyl-1-butyl ($-CH_2CH_2CH(CH_3)_2$), 2-methyl-1-butyl ($-CH_2CH(CH_3)CH_2CH_3$), *n*-hexyl ($-CH_2CH_2CH_2CH_2CH_2CH_3$), 2-hexyl ($-CH(CH_3)CH_2CH_2CH_2CH_3$), 3-hexyl ($-CH(CH_2CH_3)(CH_2CH_2CH_3)$), 2-methyl-2-pentyl ($-C(CH_3)_2CH_2CH_2CH_3$), 3-methyl-2-pentyl ($-CH(CH_3)CH(CH_3)CH_2CH_3$), 4-methyl-2-pentyl ($-CH(CH_3)CH_2CH(CH_3)_2$), 3-methyl-3-pentyl ($-C(CH_3)(CH_2CH_3)_2$), 2-methyl-3-pentyl ($-CH(CH_2CH_3)CH(CH_3)_2$), 2,3-dimethyl-2-butyl ($-C(CH_3)_2CH(CH_3)_2$), 3,3-dimethyl-2-butyl ($-CH(CH_3)C(CH_3)_3$, *n*-heptyl and *n*-octyl, *etc.*

The term "alkylene" refers to a saturated divalent hydrocarbon group derived from a straight or branched chain saturated hydrocarbon by the removal of two hydrogen atoms. Unless otherwise specified, the alkylene group contains 1-12 carbon atoms. In some embodiments, the alkylene group contains 1-6 carbon atoms. In other embodiments, the alkylene group contains 1-4 carbon atoms. In still other embodiments, the alkylene group contains 1-3 carbon atoms. In yet other embodiments, the alkylene group contains 1-2 carbon atoms. Such examples include methylene ($-CH_2-$), ethylene ($-CH_2CH_2-$), *n*-propylene ($-CH_2CH_2CH_2-$), isopropylene ($-CH(CH_3)CH_2-$), and the like.

The term "carboxy" , whether used alone or in conjunction with other terms, such as "carboxyalkyl", means $-CO_2H$ or -COOH.

The term "deuterium" refers to a single deuterium atom. For example, one deuterium atom replaces one hydrogen atom in a methyl group to form mono-deuteromethyl ($-CDH_2$), and two deuterium atoms replace two hydrogen atoms in a methyl group to form bis-deuterated methyl ($-CD_2H$)), and three deuterium atoms replace the three hydrogen atoms in the methyl group to form tri-deuteromethyl ($-CD_3$).

The term "cyano-substituted alkyl" means that an alkyl group is substituted with one or more cyano groups, wherein the alkyl group is as defined herein. Such examples include, but are not limited to, cyanomethyl, cyanoethyl, and the like.

The term "hydroxy-substituted alkyl" or "hydroxy-substituted haloalkyl" means that an alkyl or haloalkyl group is substituted with one or more hydroxyl groups, wherein the alkyl and haloalkyl groups have the meaning as described herein. Some non-limiting examples of such groups include hydroxymethyl, 1-hydroxyethyl, 2-hydroxyethyl,

$-C(OH)(CF_3)_2$, and the like.

The term "carboxy-substituted alkyl" means that an alkyl group is substituted with one or more carboxy groups, wherein the alkyl group is as defined herein. Such examples include, but are not limited to, $-CH_2COOH$, $-CH_2CH_2COOH$, $-CH_2CH_2CH_2COOH$, $-CH(CH_3)CH_2COOH$, $-CH_2(CH_2)_3COOH$, and the like.

The term "alkoxy" refers to an alkyl group, as previously defined, attached to parent molecular moiety via an oxygen atom. Unless otherwise specified, the alkoxy group contains 1-12 carbon atoms. In one embodiment, the alkoxy group contains 1-6 carbon atoms. In other embodiment, the alkoxy group contains 1-4 carbon atoms. In still other embodiment, the alkoxy group contains 1-3 carbon atoms. The alkoxy group may be optionally substituted with one or more substituents disclosed herein.

Some non-limiting examples of the alkoxy group include, but are not limited to, methoxy (MeO, $-OCH_3$), ethoxy (EtO, $-OCH_2CH_3$), 1-propoxy (*n*-PrO, *n*-propoxy, $-OCH_2CH_2CH_3$), 2-propoxy (*i*-PrO, *i*-propoxy, $-OCH(CH_3)_2$), 1-butoxy (*n*-BuO, *n*-butoxy, $-OCH_2CH_2CH_2CH_3$), 2-methyl-1-propoxy (*i*-BuO, *i*-butoxy, $-OCH_2CH(CH_3)_2$), 2-butoxy (s-BuO, s-butoxy, $-OCH(CH_3)CH_2CH_3$), 2-methyl-2-propoxy (*t*-BuO, *t*-butoxy, $-OC(CH_3)_3$), 1-pentoxy (*n*-pentoxy, $-OCH_2CH_2CH_2CH_2CH_3$), 2-pentoxy ($-OCH(CH_3)CH_2CH_2CH_3$), 3-pentoxy ($-OCH(CH_2CH_3)_2$), 2-methyl-2-butoxy ($-OC(CH_3)_2CH_2CH_3$), 3-methyl-2-butoxy ($-OCH(CH_3)CH(CH_3)_2$), 3-methyl-1-butoxy ($-OCH_2CH_2CH(CH_3)_2$), 2-methyl-1-butoxy ($-OCH_2CH(CH_3)CH_2CH_3$), and the like.

The term "alkylamino" embraces "*N*-alkylamino" and "*N,N*-dialkylamino", that is an amino group is independently substituted with one or two alkyl groups and wherein the alkyl group is as defined herein. In some embodiments, the alkylamino group is lower alkylamino group having one or two $C_{1-6}$ alkyl groups attached to a nitrogen atom. In another embodiments, the alkylamino group is lower alkylamino group having one or two $C_{1-4}$ alkyl groups attached to a nitrogen atom. Some non-limiting examples of suitable alkylamino radical include mono or dialkylamino. Some examples include, but are not limited to, *N*-methylamino, *N*-ethylamino, *N,N*-dimethylamino and *N,N*-diethylamino, and the like.

The term "haloalkyl" or "haloalkoxy" means an alkyl or alkoxy group substituted with one or more halogen atoms, wherein the alkyl or alkoxy group has the meaning as described herein. Such examples include, but are not limited to, $-CH_2F$, $-CHF_2$, $-CF_3$, $-CH_2CH_2F$, $-CH_2CHF_2$, $-CHFCH_2F$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, $-OCH_2F$, $-OCHF_2$, $-OCF_3$, $-OCH_2CH_2F$, $-OCH_2CHF_2$, $-OCHFCH_2F$, $-OCH_2CF_3$, $-OCH(CF_3)_2$, $-OCF_2CH_2CH_3$, $-OCH_2CH_2CH_2F$, $-OCH_2CH_2CHF_2$, $-OCH_2CH_2CF_3$, and the like.

The term "cycloalkyl" refers to a monovalent or multivalent saturated ring having 3 to 12 carbon atoms as a monocyclic, bicyclic, or tricyclic ring system. In some embodiments, the cycloalkyl group contains 7 to 12 carbon atoms. In other embodiments, the cycloalkyl group contains 3 to 8 carbon atoms. In still other embodiments, the cycloalkyl group contains 3 to 6 carbon atoms. The cycloalkyl group may be optionally substituted with one or more substituents disclosed herein. Examples of cycloalkyl groups include, but are not limited to, cyclopropyl, cyclobutyl, cyclopentyl, cyclohexyl and the like.

The term "cycloalkylamino" embraces "*N*-cycloalkylamino" and "*N,N*-dicycloalkylamino", wherein an amino group is independently substituted with one or two cycloalkyl groups and wherein the cycloalkyl group is as defined herein. In some embodiments, cycloalkylamino is a cycloalkylamino group having one or two $C_{3-8}$ cycloalkyl groups attached to a nitrogen atom. In another embodiments, cycloalkylamino is a cycloalkylamino group having one or two $C_{3-6}$ cycloalkyl groups attached to a nitrogen atom. Some non-limiting examples of suitable cycloalkylamino radical include mono or dicycloalkylamino. Some examples include, but are not limited to, *N*-cyclopropylamino, *N*-cyclobutylamino, *N*-cyclohexylamino, *N,N*-dicyclopropylamino, and the like.

The terms "heterocyclyl" and "heterocycle" are used interchangeably herein, refer to a saturated or partially unsaturated non-aromatic monovalent or polyvalent monocyclic, bicyclic or tricyclic ring system containing 3 to 12 ring atoms, wherein at least one ring member is selected from nitrogen, sulfur and oxygen; polycyclic heterocyclic groups include spiro heterocyclic groups and fused heterocyclic groups. Wherein, in some embodiments, heterocyclyl is a 3- to 10-membered heterocyclyl; in other embodiments, heterocyclyl is a 3- to 8-membered heterocyclyl; in still other embodiments, heterocyclyl is a 3- to 6-membered heterocyclyl; and in some embodiments, heterocyclyl is a 5- to 6-membered heterocyclyl. Unless otherwise specified, the heterocyclyl group may be carbon linked or nitrogen linked, and a $-CH_2-$ group can be optionally replaced by a $-C(=O)-$ group. In which, the sulfur can be optionally oxygenized to *S*-oxide and the nitrogen can be optionally oxygenized to *N*-oxide, Some non-limiting examples of the heterocyclyl group include oxiranyl, azetidinyl, oxetanyl, thietanyl, pyrrolidinyl, 2-pyrrolinyl, 3-pyrrolinyl, pyrazolinyl, pyrazolidinyl, imidazolinyl, imidazolidinyl, tetrahydrofuranyl, dihydrofuranyl, tetrahydrothienyl, dihydrothienyl, 1,3-dioxolanyl, dithiolanyl, tetrahydropyranyl, dihydropyranyl, 2*H*-pyranyl, 4*H*-pyranyl, tetrahydrothiopyranyl, piperidinyl,

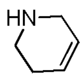,

morpholinyl, thiomorpholinyl, piperazinyl, dioxanyl, dithianyl, thioxanyl, homopiperazinyl, homopiperidinyl, diazepanyl,

oxepanyl, thiepanyl, oxazepinyl, diazepinyl, thiazepinyl, 2-oxa-5-azabicyclo[2.2.1]hept-5-yl, and the like. Some non-limiting examples of the heterocyclyl group wherein $-CH_2-$ group is replaced by -C(=O)- moiety include 2-oxopyrrolidinyl, oxo-1,3-thiazolidinyl, 2-piperidinonyl, pyridazinonyl, 3,5-dioxopiperidinyl, pyrimidinedione-yl, and the like. Some non-limited examples of heterocyclyl wherein the ring sulfur atom is oxidized is sulfolanyl, 1,1-dioxo-thiomorpholinyl. The heterocyclyl group may be optionally substituted with one or more substituents disclosed herein.

The term "aryl" refers to monocyclic, bicyclic and tricyclic aromatic carbocyclic ring systems having a total of six to fourteen ring members, or six to twelve ring members, or six to ten ring members, wherein at least one ring is aromatic and has a single point or multipoint of attachment to the rest of the molecule. The term "aryl" and "aromatic ring" can be used interchangeably herein. In one embodiment, aryl is a carbocyclic ring system consisting of 6-10 ring atoms containing at least one aromatic ring. Examples of the aryl group may include phenyl, naphthyl and anthracenyl. The aryl group may be independently and optionally substituted with one or more substituents disclosed herein.

The term "heteroaryl" refers to monocyclic, bicyclic and tricyclic carbocyclic ring systems having a total of five to twelve ring members, wherein at least one ring is aromatic and contains one or more heteroatoms; the heteroaryl group has a single point or multipoint of attachment to the rest of the molecule. The term "heteroaryl" and "heteroaromatic ring" or "heteroaromatic compound" can be used interchangeably herein. Wherein, in some embodiments, the heteroaryl group contains 1-9 carbon atoms in the 5-12 ring atoms; in other embodiments, the heteroaryl group contains 1-7 carbon atoms in the 5-12 ring atoms; in still other embodiments, the heteroaryl group contains 1-5 carbon atoms in the 5-12 ring atoms; the heteroaryl group is optionally substituted with one or more substituents described herein. In some embodiments, heteroaryl is a heteroaryl group of 5-12 ring atoms comprising 1, 2, 3 or 4 heteroatoms independently selected from O, S and N; in other embodiments, heteroaryl is a heteroaryl group of 5-10 ring atoms comprising 1, 2, 3 or 4 heteroatoms independently selected from O, S and N; in still other embodiments, heteroaryl is a heteroaryl group of 5-7 ring atoms comprising 1, 2, 3 or 4 heteroatoms independently selected from O, S and N.

Examples of heteroaryl include, but are not limited to, furyl (such as 2-furyl, 3-furyl), imidazolyl (such as *N*-imidazolyl, 2-imidazolyl, 4-imidazolyl, 5-imidazolyl), isoxazolyl (such as 3-isoxazolyl, 4-isoxazolyl, 5-isoxazolyl), oxazolyl (such as 2-oxazolyl, 4-oxazolyl, 5-oxazolyl), oxadiazolyl (such as 1,2,3-oxadiazolyl, 1,2,5-oxadiazolyl, 1,2,4-oxadiazolyl), oxatriazolyl (such as 1,2,3,4-oxatriazolyl), thiazolyl (such as 2-thiazolyl, 4-thiazolyl, 5-thiazolyl), isothiazolyl, 2-thiadiazolyl (such as 1,3,4-thiadiazolyl, 1,2,3-thiadiazolyl, 1,2,5-thiadiazolyl), thiatriazolyl (such as 1,2,3,4-thiatriazolyl), tetrazolyl (such as 2*H*-1,2,3,4-tetrazolyl, 1*H*-1,2,3,4-tetrazolyl), triazolyl (such as 2*H*-1,2,3-triazolyl, 1*H*-1,2,4-triazolyl, 4*H*-1,2,4-triazolyl), thienyl (such as 2-thienyl, 3-thienyl), 1*H*-pyrazolyl (such as 1*H*-pyrazol-3-yl, 1*H*-pyrazol-4-yl, 1*H*-pyrazol-5-yl), 1,2,3-thiodiazolyl, 1,3,4-thiodiazolyl, 1,2,5-thiodiazolyl, pyrrolyl (such as *N*-pyrrolyl, 2-pyrrolyl, 3-pyrrolyl), pyridyl (such as 2-pyridyl, 3-pyridyl, 4-pyridyl), pyrimidinyl (such as 2-pyrimidinyl, 4-pyrimidinyl, 5-pyrimidinyl), pyridazinyl (such as 3-pyridazinyl, 4-pyridazinyl), 2-pyrazinyl, triazinyl (such as 1,3,5-triazinyl), tetrazinyl (such as 1,2,4,5-tetrazinyl, 1,2,3,5-tetrazinyl); examples of heteroaryl also include, but are in no way limited to, the following bicycles: benzimidazolyl, benzopyrazolyl (such as

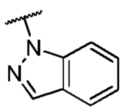, 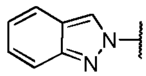),

benzofuranyl, benzothienyl, benzobisoxazolyl, indolyl (such as 2-indolyl), purinyl, quinolinyl (such as 2-quinolinyl, 3-quinolinyl, 4-quinolinyl), isoquinolinyl (such as 1-isoquinolinyl, 3-isoquinolinyl or 4-isoquinolinyl), imidazo[1,2-a]pyridyl, pyrazolo[1,5-a]pyridyl, pyrazolo[1,5-a]pyrimidinyl, imidazo[1,2-*b*]pyridazinyl, [1,2,4]triazolo[4,3-*b*]pyridazinyl, [1,2,4]triazolo[1,5-*a*]pyrimidinyl, [1,2,4]triazolo[1,5-a]pyridyl,

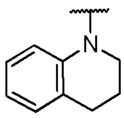, 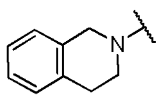,

*etc.*

The term "j- to k-membered" means that the cyclic group consists of j to k ring atoms, and the ring atoms include carbon atoms and/or heteroatoms such as O, N, S, P, *etc.;* each of j and k is independently any non-zero natural numbers, and k>j; the "j-k" or "j to k" or "j- to k-" includes j, k and any natural numbers between them. For example, "5- to 12-membered", "5- to 10-membered" or "3- to 7-membered" means that the cyclic group consists of 5-12 *(i.e.,* 5, 6, 7, 8, 9, 10, 11 or 12), 5-10 *(i.e.,* 5, 6, 7, 8, 9 or 10), 5-6 *(i.e.,* 5 or 6) or 3-7 *(i.e.,* 3, 4, 5, 6 or 7) ring atoms, and the ring atoms include carbon atoms and/or heteroatoms such as O, N, S, P, *etc.* The term "unsaturated" refers to a moiety having one or more units of unsaturation.

The term "heteroatom" refers to one or more of oxygen, sulfur, nitrogen, phosphorus and silicon, including any oxidized form of nitrogen, sulfur, or phosphorus; the quaternized form of any basic nitrogen; or a substitutable nitrogen of a heterocyclic ring, for example, N (as in 3,4-dihydro-2*H*-pyrrolyl), NH (as in pyrrolidinyl) or NR (as in *N*-substituted pyrrolidinyl).

The term "halogen" or "halogen atom" refers to fluorine atom (F), chlorine atom (Cl), bromine atom (Br) or iodine atom (I).

The term "cyano" or "CN" refers to a cyano structure, and such groups can be attached to other groups.

The term "nitro" or "$NO_2$" refers to a nitro structure, and such groups can be attached to other groups.

As described herein, a bond drawn from a substituent to the center of one ring within a ring system (as shown in formula b) represents substitution of the substituent at any substitutable or reasonable position on the ring. For example, formula c represents mono- or poly-substitution of the substituent R at any substitutable position on the ring C, as shown in formulas c1 to c19.

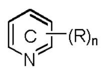
formula c

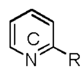
formula c1

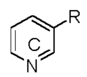
formula c2

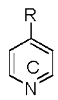
formula c3

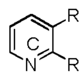
formula c4

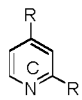
formula c5

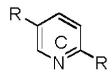
formula c6

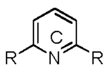
formula c7

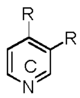
formula c8

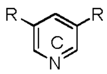
formula c9

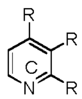
formula c10

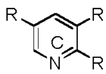
formula c11

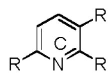
formula c12

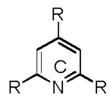
formula c13

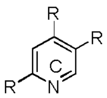
formula c14

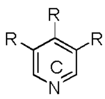
formula c15

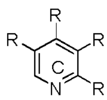
formula c16

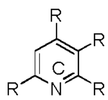
formula c17

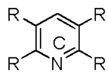
formula c18

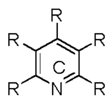
formula c19

As described herein, a linker attached to a ring system (as shown in formula d) means that the linker can be attached to the rest of the molecule at any linkable position on the ring system. Formula d represents that any possible linking position on the ring can be attached to the rest of the molecule, as shown in formulas d1 to d5.

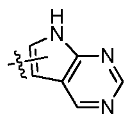
formula d

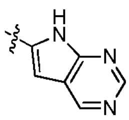
formula d1

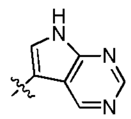
formula d2

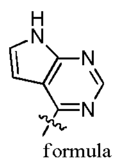
formula d3

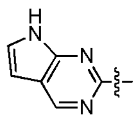
formula d4

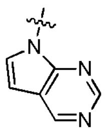
formula d5

As described herein, when a group is attached to other parts of the molecule through two sites, unless otherwise stated, each of the two sites is independently and optionally attached to other groups of the molecule, and the groups connected to the two sites can be interchanged; for example, the piperidinyl in formula e can be linked to other parts of the molecule through the $E_1$ end and the $E_2$ end, and when the other parts of the molecule remain unchanged, the $E_1$ end and the $E_2$ end can be interchanged.

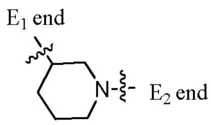
formula e

The term "prodrug" refers to a compound that is transformed in vivo into a compound of Formula (I). Such a transformation can be affected, for example, by hydrolysis of the prodrug form in blood or enzymatic transformation to the parent form in blood or tissue. Prodrugs of the compounds disclosed herein may be, for example, esters. Some

common esters which have been utilized as prodrugs are phenyl esters, aliphatic ($C_1$-$C_{24}$) esters, acyloxymethyl esters, carbonates, carbamates and amino acid esters. For example, a compound disclosed herein that contains a hydroxy group may be acylated at this position in its prodrug form. Other prodrug forms include phosphates, such as, those phosphate compounds derived from the phosphonation of a hydroxy group on the parent compound. A thorough discussion of prodrugs is provided in T. Higuchi and V. Stella, Pro-drugs as Novel Delivery Systems, Vol. 14 of the A.C.S. Symposium Series, Edward B. Roche, ed., Bioreversible Carriers in Drug Design, American Pharmaceutical Association and Pergamon Press, 1987, J. Rautio et al., Prodrugs: Design and Clinical Applications, Nature Review Drug Discovery, 2008, 7, 255-270, and S. J. Hecker et al., Prodrugs of Phosphates and Phosphonates, Journal of Medicinal Chemistry, 2008, 51, 2328-2345, all of which are incorporated herein by reference in their entireties.

A "metabolite" is a product produced through metabolism in the body of a specified compound or salt thereof. The metabolites of a compound may be identified using routine techniques known in the art and their activities determined using tests such as those described herein. Such products may result for example from oxidation, reduction, hydrolysis, amidation, deamidation, esterification, deesterification, enzyme cleavage, and the like, of the administered compound. Accordingly, the invention includes metabolites of compounds disclosed herein, including metabolites produced by contacting a compound disclosed herein with a mammal for a sufficient time period.

A "pharmaceutically acceptable salt" refers to an organic or inorganic salt of a compound disclosed herein. Pharmaceutically acceptable salts are well known in the art. For example, S. M. Berge et al., describe pharmaceutically acceptable salts in detail in J. Pharmaceutical Sciences, 1977, 66: 1-19, which is incorporated herein by reference. Some non-limiting examples of pharmaceutically acceptable and nontoxic salts include salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid and malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, laurylsulfate, malate, methanesulfonate, 2-naphthalenesulfonate, nicotinate, nitrate, oleate, palmitate, pamoate, pectinate, persulfate, 3-phenylpropionate, picrate, pivalate, propionate, stearate, thiocyanate, *p*-toluenesulfonate, undecanoate, valerate, and the like. Salts derived from appropriate bases include alkali metal, alkaline earth metal, ammonium and $N^+(C_{1-4}\ alkyl)_4$ salts. This invention also envisions the quaternization of any basic nitrogen-containing groups of the compounds disclosed herein. Water or oil soluble or dispersable products may be obtained by such quaternization. Representative alkali or alkaline earth metal salts include sodium, lithium, potassium, calcium, magnesium, and the like. Further pharmaceutically acceptable salts include appropriate and nontoxic ammonium, quaternary ammonium, and amine cations formed using counterions, such as halide, hydroxide, carboxylate, sulfate, phosphate, nitrate, $C_{1-8}$ sulfonate or aryl sulfonate.

The term "solvate" refers to an association or complex of one or more solvent molecules and a compound disclosed herein. Examples of solvents that form solvates include, but are not limited to, water, isopropanol, ethanol, methanol, DMSO, ethyl acetate, acetic acid and ethanolamine. The term "hydrate" refers to the complex where the solvent molecule is water.

The term "hydrate" can be used when said solvent is water. In one embodiment, one solvent molecule is associated with one molecule of the compounds disclosed herein, such as a hydrate. In another embodiment, more than one solvent molecule may be associated with one molecule of the compounds disclosed herein, such as a dihydrate. In still another embodiment, less than one solvent molecule may be associated with one molecule of the compounds disclosed herein, such as a hemihydrate. Furthermore, all the solvates of the invention retain the biological effectiveness of the non-hydrate form of the compounds disclosed herein.

The term "*N*-oxide" refers to one or more than one nitrogen atoms oxidised to form an *N*-oxide, where a compound contains several amine functions. Particular examples of *N*-oxides are the *N*-oxides of a *tert*iary amine or a nitrogen atom of a nitrogen-containing heterocycle. *N*-oxides can be formed by treatment of the corresponding amine with an oxidizing agent such as hydrogen peroxide or a per-acid *(e.g.,* a peroxycarboxylic acid) (See, Advanced Organic Chemistiy, by Jerry March, 4th Edition, Wiley Interscience, pages). More particularly, *N*-oxides can be made by the procedure of L. W. Deady (Syn. Comm. 1977, 7, 509-514) in which the amine compound is reacted with m-chloroperoxybenzoic acid (MCPBA), for example, in an inert solvent such as dichloromethane.

The term "carrier" includes any solvents, dispersion media, coating agents, surfactants, antioxidants, preservatives (e.g., antibacterial agents, antifungal agents), isotonic agents, salt, drug stabilizers, binders, excipients, dispersants, lubricants, sweetening agents, flavoring agents, coloring agents, or a combination thereof, all of which are well kown to the skilled in the art. (e.g., Remington's Pharmaceutical Sciences, 18th Ed. Mack Printing Company, 1990, pp. 1289-1329, all of which are incorporated herein by reference). Except any conventional carrier is incompatible with the active ingredient, the pharmaceutically acceptable carriers are effectively used in the treatment or pharmaceutical compositions.

As used herein, the"treat", "treating" or "treatment" of any disease or disorder refers to all that can slow, interrupt, arrest, control or stop the progression of the disease or disorder, but does not necessarily mean that all symptoms of the disease or disorder disappear, which also includes prophylactic treatment of said symptoms, especially in patients prone to such disease or disorder. In some embodiments, the"treat", "treating" or "treatment" of any disease or disorder refers to ameliorating the disease or disorder *(i.e.,* slowing or arresting or reducing the development of the disease or at least one of the clinical symptoms thereof). In another embodiment "treat", "treating" or "treatment" refers to alleviating or ameliorating at least one physical parameter including those which may not be discernible by the patient. In yet another embodiment, "treat", "treating" or "treatment" refers to modulating the disease or disorder, either physically, (*e.g.,* stabilization of a discernible symptom), physiologically, *(e.g.,* stabilization of a physical parameter), or both. In yet another embodiment, "treat", "treating" or "treatment" refers to preventing or delaying the onset or development or progression of the disease or disorder.

The term "therapeutically effective amount" or "therapeutically effective dose" as used herein refers to the amount of a compound of the present invention that is capable of eliciting a biological or medical response (For example, reducing or inhibiting the activity of enzyme or protein, or improving symptoms, alleviating symptoms, slowing or delaying progression of disease, or preventing disease, *etc.)* in a subject. In a non-limiting embodiment, the term "therapeutically effective amount" refers to an amount effective to: (1) at least partially alleviate, inhibit, prevent and/or ameliorate a disorder or disease (i) mediated by RORyt, or (ii) associated with the activity of RORyt, or (iii) characterized by aberrant activity of RORyt; or (2) reduce or inhibit the activity of RORyt; or (3) reduce or inhibit the expression of RORyt when a compound of the present invention is administered to an individual. In another embodiment, the term "therapeutically effective amount" refers to an effective amount of a compound of the invention capable of at least partially reducing or inhibiting the activity of RORyt; or at least partially reducing or inhibiting the expression of RORyt when administered to a cell, or organ, or non-cellular biological material, or vehicle.

The term "administering" a compound as used herein are to be understood as providing a compound of the present invention or a prodrug of a compound of the present invention to an individual in need thereof. It will be appreciated that one of skill in the art treats a patient currently suffering from this disorder or prophylactically treats a patient suffering from this disorder by administering an effective amount of a compound of the present invention.

The term "composition" as used herein refers to a product comprising the specified ingredients in the specified amounts, as well as any product that results, directly or indirectly, from combination of the specified ingredients in the specified amounts. The meaning of this term in relation to a pharmaceutical composition includes a product comprising the active ingredient (single or multiple) and inert ingredient (single or multiple) that make up the carrier, as well as a mixture, complex or aggregate of any two or more ingredients, or any product that results directly or indirectly from the decomposition of one or more components, or from other types of reactions or interactions of one or more components. Accordingly, the pharmaceutical compositions of the present invention include any composition prepared by admixing a compound of the present invention and a pharmaceutically acceptable carrier.

## DESCRIPTION OF COMPOUNDS OF THE INVENTION

The invention discloses a class of (hetero) aromatic ring derivatives, pharmaceutically acceptable salts thereof, pharmaceutical preparations and compositions thereof, which can be used as RORyt inhibitors, and have potential use in the treatment of cancer, inflammation or autoimmune diseases mediated by RORyt, such as cancer, psoriasis, rheumatoid arthritis, systemic lupus erythematosus, multiple sclerosis, inflammatory bowel disease, colitis, ulcerative colitis, rheumatoid arthritis, autoimmune eye disease, ankylosing spondylitis, asthma, chronic obstructive pulmonary disease, osteoarthritis, allergic rhinitis, atopic dermatitis, Crohn’s disease, or Kawasaki disease.

In one aspect, the present invention provides a compound having Formula (I) or a stereoisomer, an *N*-oxide, a solvate, a metabolite, a pharmaceutically acceptable salt or a prodrug thereof,

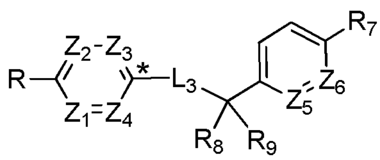

(I);

wherein, R, $R_7$, $R_8$, $R_9$, $Z_1$, $Z_2$, $Z_3$, $Z_4$, $Z_5$ and $Z_6$ have the meanings described in the present invention; * indicates the direction in which $L_3$ is connected to the left (hetero)aryl group.

In some embodiments, R is $R_0$, $-(CH_2)_m$-B-$L_1$-**A or -$L_2$-G; wherein Ro, B, A, G, $L_1$, $L_2$ and m have the meanings described herein; ** indicates the connection direction of $L_1$ and A.

In some embodiments, $Z_1$ is $CR_1$ orN; $Z_2$ is $CR_2$ orN; $Z_3$ is $CR_3$ orN; $Z_4$ is $CR_4$ orN; $Z_5$ is $CR_5$ or N; $Z_6$ is $CR_6$

or N; wherein $R_1$, $R_2$, $R_3$, $R_4$, $R_5$ and $R_6$ have the meanings described in the present invention.

In some embodiments, each of $R_0$, $R_1$, $R_2$, $R_3$, $R_4$, $R_5$ and $R_6$ is independently H, deuterium, F, Cl, Br, I, $C_{1-6}$ alkyl, $C_{1-6}$ haloalkyl, $C_{1-6}$ alkoxy, hydroxy-substituted $C_{1-6}$ alkyl, hydroxy-substituted $C_{1-6}$ haloalkyl, -Si($C_{1-6}$ alkyl)$_3$, $C_{1-6}$ haloalkoxy or -N($R_dR_e$); wherein, $R_d$ and $R_e$ have the meanings described in the present invention.

In some embodiments, $R_7$ is -S(=O)$_2$-$C_{1-6}$ alkyl, -S(=O)$_2$-$C_{1-6}$ alkoxy, -S(=O)$_2$-$C_{1-6}$ alkylamino, -S(=O)$_2$-$C_{1-6}$ haloalkyl, -S(=O)$_2$-$C_{3-8}$ cycloalkyl, -S(=O)$_2$-$C_{1-6}$ alkylene-$C_{3-8}$ cycloalkyl, -S(=O)-$C_{1-6}$ alkyl, -S(=O)$_2$H, -COOH, -C(=O)-N($R_gR_h$), -N($R_g$)-C(=O)-$C_{1-6}$ alkyl, -C(=O)-O-$C_{1-6}$ alkyl, $C_{1-6}$ alkyl, $C_{1-6}$ alkoxy, $C_{1-6}$ haloalkyl or $C_{3-8}$ cycloalkyl; wherein, $R_g$ and $R_h$ have the meanings described in the present invention.

In some embodiments, each $R_g$ and $R_h$ is independently H, deuterium, or $C_{1-6}$ alkyl.

In some embodiments, each of A and G is independently $C_{3-8}$ cycloalkyl, $C_{6-10}$ aryl, 5-to 10-membered heteroaryl, or 5- to 10-membered heterocyclyl; wherein, each of A and G is independently and optionally substituted with 1, 2, 3, 4 or 5 $R_a$; wherein, $R_a$ has the meaning described in the present invention.

In some embodiments, B is 4- to 10-membered heterocyclyl or thiazolyl; wherein, the 4- to 10-membered heterocyclyl is optionally substituted with 1, 2, 3, 4 or 5 $R_b$; wherein, $R_b$ has the meaning described in the present invention.

In some embodiments, each $R_a$ and $R_b$ is independently deuterium, F, Cl, Br, I, -OH, -CN, -NH$_2$, -NO$_2$, -COOH, oxo, $C_{1-6}$ alkyl, $C_{1-6}$ haloalkyl, $C_{1-6}$ alkoxy, $C_{1-6}$ haloalkoxy, -$C_{1-6}$ alkylene-O-$C_{1-6}$ alkyl, $C_{3-8}$ cycloalkyl, $C_{6-10}$ aryl, 5- to 10-membered heterocyclyl, 5- to 10-membered heteroaryl or -C(=O)-N($R_dR_e$); wherein, each of the $C_{1-6}$ alkyl, $C_{1-6}$ haloalkyl, $C_{1-6}$ alkoxy, $C_{1-6}$ haloalkyl, -$C_{1-6}$ alkylene-O-$C_{1-6}$ alkyl, $C_{3-8}$ cycloalkyl, $C_{6-10}$ aryl, 5- to 10-membered heterocyclyl and 5- to 10-membered heteroaryl is independently and optionally substituted with 1, 2 or 3 $R_e$; wherein, $R_e$, $R_d$ and $R_e$ have the meanings described in the present invention.

In some embodiments, each $R_e$ is independently deuterium, F, Cl, Br, I, -OH, -CN, -NH$_2$, $C_{1-6}$ alkyl, $C_{1-6}$ alkoxy, $C_{1-6}$ haloalkyl, $C_{1-6}$ haloalkoxy, $C_{3-8}$ cycloalkyl, 5- to 10-membered heterocyclyl, $C_{6-10}$ aryl or 5- to 10-membered heteroaryl.

In some embodiments, $R_8$ is H, deuterium, -OH, -CN, -NH$_2$, -NO$_2$, -COOH, $C_{1-6}$ alkyl, $C_{1-6}$ alkoxy, $C_{1-6}$ haloalkyl, cyano-substituted $C_{1-6}$ alkyl, carboxy-substituted $C_{1-6}$ alkyl, -$C_{1-6}$ alkylene-O-$C_{1-6}$ alkyl, -$C_{1-6}$ alkylene-C(=O)-O-$C_{1-6}$ alkyl, -$C_{1-6}$ alkylene-C(=O)-N($R_dR_e$), -$C_{1-6}$ alkylene-OC(=O)-N($R_dR_e$), -$C_{1-6}$ alkylene-N(Rf)-C(=O)-N($R_dR_e$), -$C_{1-6}$ alkylene-N($R_dR_e$) or -N($R_f$)-C(=O)-$C_{1-6}$ alkyl;

$R_9$ is deuterium, -OH, -CN, -NH$_2$, -NO$_2$, -COOH, hydroxyethyl, $C_{1-6}$ alkoxy, $C_{1-6}$ haloalkyl, cyano-substituted $C_{1-6}$ alkyl, carboxy-substituted $C_{1-6}$ alkyl, -$C_{1-6}$ alkylene-O-$C_{1-6}$ alkyl, -$C_{1-6}$ alkylene-C(=O)-O-$C_{1-6}$ alkyl, -$C_{1-6}$ alkylene-C(= O)-N($R_dR_e$), -$C_{1-6}$ alkylene-OC(=O)-N($R_dR_e$), -$C_{1-6}$ alkylene-N(Rf)-C(=O)-N($R_dR_e$), -$C_{1-6}$ alkylene-N($R_dR_e$) or -N($R_f$)-C(=O)-$C_{1-6}$ alkyl;

wherein, each of the $C_{1-6}$ alkyl, $C_{1-6}$ alkoxy, $C_{1-6}$ haloalkyl, cyano-substituted $C_{1-6}$ alkyl, carboxy-substituted $C_{1-6}$ alkyl, -$C_{1-6}$ alkylene-O-$C_{1-6}$ alkyl and -$C_{1-6}$ alkylene-C(=O)-O-$C_{1-6}$ alkyl described in $R_8$ and $R_9$ is independently and optionally substituted with 1, 2, 3 or 4 substituents selected from deuterium, F, Cl, Br, I, -OH, -CN, -NH$_2$, -COOH, -N($R_dR_e$), $C_{1-6}$ alkyl, $C_{1-6}$ haloalkyl, $C_{1-6}$ alkoxy or $C_{1-6}$ haloalkoxy;

or, $R_8$ and $R_9$ together with the carbon atom to which they are attached, form $C_{3-8}$ cycloalkyl or 3- to 8-membered heterocyclyl; wherein, each of the $C_{3-8}$ cycloalkyl and 3- to 8-membered heterocyclyl is independently and optionally substituted with 1, 2, 3 or 4 substituents selected from deuterium, F, Cl, Br, I, -OH, -CN, -NH$_2$, -COOH, -N($R_dR_e$), $C_{1-6}$ alkyl, $C_{1-6}$ haloalkyl, $C_{1-6}$ alkoxy or $C_{1-6}$ haloalkoxy;

wherein, $R_d$, $R_e$ and $R_f$ have the meanings described in the present invention.

In some embodiments, each $R_d$ and $R_e$ is independently H, deuterium, -OH, $C_{1-6}$ alkyl, -C(=O)H, -C(=O)-O-$C_{1-6}$ alkyl, -C(=O)-$C_{1-6}$ alkyl, -$C_{1-6}$ alkylene-C(=O)-O-$C_{1-6}$ alkyl or -$C_{1-6}$ alkylene-O-$C_{1-6}$ alkyl; wherein, each of the $C_{1-6}$ alkyl, -C(=O)-O-$C_{1-6}$ alkyl, -C(=O)-$C_{1-6}$ alkyl, -$C_{1-6}$ alkylene-C(=O)-O-$C_{1-6}$ alkyl and -$C_{1-6}$ alkylene-O-$C_{1-6}$ alkyl is independently and optionally substituted with 1, 2, 3 or 4 substituents selected from deuterium, F, Cl, Br, I, -OH, -CN , -NH$_2$ or -COOH.

In some embodiments, $L_1$ is a bond, **-O-, **-C(=O)-, **-NH-, **-CH$_2$-, **-$C_{1-6}$ alkylene-O-, **-O-$C_{1-6}$ alkylene-, **-C(=O)-N($R_f$)-, **-N($R_f$)-C(=O)-, **-N($R_f$)-$C_{1-6}$ alkylene- or **-$C_{1-6}$ alkylene-N($R_f$)-; wherein, each of the **-CH$_2$-, **-$C_{1-6}$ alkylene-O-, **-O-$C_{1-6}$ alkylene-, **-N($R_f$)-$C_{1-6}$ alkylene- and **-$C_{1-6}$ alkylene-N($R_f$)- is independently and optionally substituted with 1, 2, 3 or 4 substituents selected from deuterium, oxo, halogen, $C_{1-6}$ alkyl or $C_{1-6}$ haloalkyl; wherein, Rf has the meaning described in the present invention.

In some embodiments, $L_2$ is a bond, -O-, -C(=O)-, -NH-, -CH$_2$-, -$C_{1-6}$ alkylene-O-, -O-$C_{1-6}$ alkylene-, -C(=O)-N($R_f$)-, -N($R_f$)-C(=O)-, -N($R_f$)-$C_{1-6}$ alkylene- or -$C_{1-6}$ alkylene-N($R_f$)-; wherein, each of the -$C_{1-6}$ alkylene-O-, -O-$C_{1-6}$ alkylene-, -N($R_f$)-$C_{1-6}$ alkylene- and -$C_{1-6}$ alkylene-N($R_f$)- is independently and optionally substituted with 1, 2, 3 or 4 substituents selected from deuterium, oxo, halogen, $C_{1-6}$ alkyl or $C_{1-6}$ haloalkyl; wherein, $R_f$ has the meaning described in the present invention.

In some embodiments, $L_3$ is *-S(=O)$_2$-NH-, *-NH-S(=O)$_2$-, *-S(=O)-NH-, *-NH-S(=O)-, *-C(=O)NH- or

*-NHC(=O)-.

In some embodiments, each $R_f$ is independently H, deuterium, $C_{1-6}$ alkyl, $-C_{1-6}$ alkylene-O-$C_{1-6}$ alkyl, $-C_{1-6}$ alkylene-(5- to 10-membered heterocyclyl), $-C_{1-6}$ alkylene-$C_{3-8}$ cycloalkyl, 3- to 8-membered heterocyclyl, -C(=O)-(3- to 8-membered heterocyclyl), $C_{3-8}$ cycloalkyl or -C(=O)-$C_{3-8}$ cycloalkyl; wherein, each of the $C_{1-6}$ alkyl, $-C_{1-6}$ alkylene-O-$C_{1-6}$ alkyl, $-C_{1-6}$ alkylene-(5- to 10-membered heterocyclyl), $-C_{1-6}$ alkylene-$C_{3-8}$ cycloalkyl, $C_{3-8}$ cycloalkyl, 3- to 8-membered heterocyclyl, -C(=O)-(3- to 8-membered heterocyclyl) and -C(=O)-$C_{3-8}$ cycloalkyl is independently and optionally substituted with 1, 2 or 3 substituents selected from deuterium, F, Cl, Br, I, -OH, -CN, $-NH_2$ or COOH.

In some embodiments, m is 0, 1 or 2.

In other embodiments, each of $R_0$, $R_1$, $R_2$, $R_3$, $R_4$, $R_5$ and $R_6$ is independently H, deuterium, F, Cl, Br, I, $C_{1-4}$ alkyl, $C_{1-4}$ haloalkyl, $C_{1-4}$ alkoxy, hydroxy-substituted $C_{1-4}$ alkyl, hydroxy-substituted $C_{1-4}$ haloalkyl, $-Si(C_{1-4}\ alkyl)_3$, $C_{1-4}$ haloalkoxy or $-N(R_dR_e)$; wherein, $R_d$ and $R_e$ have the meanings described in the present invention.

In other embodiments, $R_7$ is $-S(=O)_2$-$C_{1-4}$ alkyl, $-S(=O)_2$-$C_{1-4}$ alkoxy, $-S(=O)_2$-$C_{1-4}$ alkylamino, $-S(=O)_2$-$C_{1-4}$ haloalkyl, $-S(=O)_2$-$C_{3-6}$ cycloalkyl, $-S(=O)_2$-$C_{1-4}$ alkylene-$C_{3-6}$ cycloalkyl, -S(=O)-$C_{1-4}$ alkyl, $-S(=O)_2H$, -COOH, $-C(=O)-N(R_gR_h)$, $-N(R_g)$-C(=O)-$C_{1-4}$ alkyl, -C(=O)-O-$C_{1-4}$ alkyl, $C_{1-4}$ alkyl, $C_{1-4}$ alkoxy, $C_{1-4}$ haloalkyl or $C_{3-6}$ cycloalkyl; wherein, $R_g$ and $R_h$ have the meanings described in the present invention.

In other embodiments, each $R_g$ and $R_h$ is independently H, deuterium, or $C_{1-4}$ alkyl.

In other embodiments, each of A and G is independently cyclopropyl, cyclobutyl, cyclopentyl, cyclohexyl, $C_{6-10}$ aryl, 5- to 10-membered heteroaryl or 5- to 7-membered heterocyclyl; wherein, each of A and G is independently and optionally substituted with 1, 2, 3, 4 or 5 $R_a$, and $R_a$ has the meaning described in the present invention.

In other embodiments, each of A and G is independently cyclopropyl, cyclobutyl, cyclopentyl, cyclohexyl, $C_{6-10}$ aryl, 5- to 6-membered heteroaryl or 5- to 6-membered heterocyclyl; wherein, each of A and G is independently and optionally substituted with 1, 2, 3, 4 or 5 $R_a$, and $R_a$ has the meaning described in the present invention.

In other embodiments, B is 4- to 7-membered heterocyclyl; wherein, the 4- to 7-membered heterocyclyl is optionally substituted with 1, 2, 3, 4 or 5 $R_b$; wherein, $R_b$ has the meaning described in the present invention.

In other embodiments, B is 5- to 6-membered heterocyclyl; wherein, the 5- to 6-membered heterocyclyl is optionally substituted with 1, 2, 3, 4 or 5 $R_b$; wherein, $R_b$ has the meaning described in the present invention.

In other embodiments, each $R_a$ and $R_b$ is independently deuterium, F, Cl, Br, I, -OH, -CN, $-NH_2$, $-NO_2$, -COOH, oxo, $C_{1-4}$ alkyl, $C_{1-4}$ haloalkyl, $C_{1-4}$ alkoxy, $C_{1-4}$ haloalkoxy, $-C_{1-4}$ alkylene-O-$C_{1-4}$ alkyl, $C_{3-6}$ cycloalkyl, $C_{6-10}$ aryl, 5- to 7-membered heterocyclyl, 5- to 7-membered heteroaryl or $-C(=O)-N(R_dR_e)$; wherein, each of the $C_{1-4}$ alkyl, $C_{1-4}$ haloalkyl, $C_{1-4}$ alkoxy, $C_{1-4}$ haloalkoxy, $-C_{1-4}$ alkylene-O-$C_{1-4}$ alkyl, $C_{3-6}$ cycloalkyl, $C_{6-10}$ aryl, 5- to 7-membered heterocyclyl and 5- to 7-membered heteroaryl is independently and optionally substituted with 1, 2 or 3 $R_c$; wherein, $R_c$, $R_d$ and $R_e$ have the meanings described in the present invention.

In other embodiments, each $R_c$ is independently deuterium, F, Cl, Br, I, -OH, -CN, $-NH_2$, $C_{1-4}$ alkyl, $C_{1-4}$ alkoxy, $C_{1-4}$ haloalkyl, $C_{1-4}$ haloalkoxy, $C_{3-6}$ cycloalkyl, 5- to 7-membered heterocyclyl, $C_{6-10}$ aryl or 5- to 7-membered heteroaryl.

In other embodiments, $R_8$ is H, deuterium, -OH, -CN, $-NH_2$, $-NO_2$, -COOH, $C_{1-4}$ alkyl, $C_{1-4}$ alkoxy, $C_{1-4}$ haloalkyl, cyano-substituted $C_{1-4}$ alkyl, carboxy-substituted $C_{1-4}$ alkyl, $-C_{1-4}$ alkylene-O-$C_{1-4}$ alkyl, $-C_{1-4}$ alkylene-C(=O)-O-$C_{1-4}$ alkyl, $-C_{1-4}$ alkylene-$C(=O)-N(R_dR_e)$, $-C_{1-4}$ alkylene-$OC(=O)-N(R_dR_e)$, $-C_{1-4}$ alkylene-$N(R_f)-C(=O)-N(R_dR_e)$, $-C_{1-4}$ alkylene-$N(R_dR_e)$ or $-N(R_f)$-C(=O)-$C_{1-4}$ alkyl;

$R_9$ is deuterium, -OH, -CN, $-NH_2$, $-NO_2$, -COOH, $C_{1-4}$ alkoxy, $C_{1-4}$ haloalkyl, cyano-substituted $C_{1-4}$ alkyl, carboxy-substituted $C_{1-4}$ alkyl, $-C_{1-4}$ alkylene-O-$C_{1-4}$ alkyl, $-C_{1-4}$ alkylene-C(=O)-O-$C_{1-4}$ alkyl, $-C_{1-4}$ alkylene-$C(=O)-N(R_dR_e)$, $-C_{1-4}$ alkylene-$OC(=O)-N(R_dR_e)$, $-C_{1-4}$ alkylene-$N(R_f)-C(=O)-N(R_dR_e)$, $-C_{1-4}$ alkylene-$N(R_dR_e)$ or $-N(R_f)$-C(=O)-$C_{1-4}$ alkyl;

wherein, each of the $C_{1-4}$ alkyl, $C_{1-4}$ alkoxy, $C_{1-4}$ haloalkyl, cyano-substituted $C_{1-4}$ alkyl, carboxy-substituted $C_{1-4}$ alkyl, $-C_{1-4}$ alkylene-O-$C_{1-4}$ alkyl and $-C_{1-4}$ alkylene-C(=O)-O-$C_{1-4}$ alkyl described in $R_8$ and $R_9$ is independently and optionally substituted with 1, 2, 3 or 4 substituents selected from deuterium, F, Cl, Br, I, -OH, -CN, $-NH_2$, -COOH, $-N(R_dR_e)$, $C_{1-4}$ alkyl, $C_{1-4}$ haloalkyl, $C_{1-4}$ alkoxy or $C_{1-4}$ haloalkoxy;

or, $R_8$ and $R_9$ together with the carbon atom to which they are attached, form $C_{3-6}$ cycloalkyl or 3- to 6-membered heterocyclyl; wherein, each of the $C_{3-6}$ cycloalkyl and 3- to 6-membered heterocyclyl is independently and optionally substituted with 1, 2, 3 or 4 substituents selected from deuterium, F, Cl, Br, I, -OH, -CN, $-NH_2$, -COOH, $-N(R_dR_e)$, $C_{1-4}$ alkyl, $C_{1-4}$ haloalkyl, $C_{1-4}$ alkoxy or $C_{1-4}$ haloalkoxy;

wherein, $R_d$, $R_e$ and Rf have the meanings described in the present invention.

In other embodiments, each $R_d$ and $R_e$ is independently H, deuterium, -OH, $C_{1-4}$ alkyl, -C(=O)H, -C(=O)-O-$C_{1-4}$ alkyl, -C(=O)-$C_{1-4}$ alkyl, $-C_{1-4}$ alkylene-C(=O)-O-$C_{1-4}$ alkyl or $-C_{1-4}$ alkylene-O-$C_{1-4}$ alkyl; wherein, each of the $C_{1-4}$ alkyl, -C(=O)-O-$C_{1-4}$ alkyl, -C(=O)-$C_{1-4}$ alkyl, $-C_{1-4}$ alkylene-C(=O)-O-$C_{1-4}$ alkyl and $-C_{1-4}$ alkylene-O-$C_{1-4}$ alkyl is independently and optionally substituted with 1, 2, 3 or 4 substituents selected from deuterium, F, Cl, Br, I, -OH, -CN, $-NH_2$ or -COOH.

In other embodiments, $L_1$ is a bond, **-O-, **-C(=O)-, **-NH-, **-$CH_2$-, **-O-$C_{1-3}$ alkylene-, **-$C_{1-3}$ alkylene-O-, **-N($R_f$)-C(=O)-, **-C(=O)-N($R_f$)-, **-N($R_f$)-$C_{1-3}$ alkylene- or **-$C_{1-3}$ alkylene-N($R_f$)-; wherein, each of the **-$CH_2$-, **-O-$C_{1-3}$ alkylene-, **-$C_{1-3}$ alkylene-O-, **-N($R_f$)-$C_{1-3}$ alkylene- and **-$C_{1-3}$ alkylene-N($R_f$)- is independently and optionally substituted with 1, 2, 3 or 4 substituents selected from deuterium, oxo, halogen, $C_{1-4}$ alkyl or $C_{1-4}$ haloalkyl; wherein, Rf has the meaning described in the present invention.

In some embodiments, $L_2$ is a bond, -O-, -C(=O)-, -NH-, -$CH_2$-, -O-$C_{1-3}$ alkylene-, -$C_{1-3}$ alkylene-O-, -N($R_f$)-C(=O)-, -C(=O)-N($R_f$)-, -N($R_f$)-$C_{1-3}$ alkylene- or -$C_{1-3}$ alkylene-N($R_f$)-; wherein, each of the -O-$C_{1-3}$ alkylene-, -$C_{1-3}$ alkylene-O-, -N($R_f$)-$C_{1-3}$ alkylene- and -$C_{1-3}$ alkylene-N($R_f$)- is independently and optionally substituted with 1, 2, 3 or 4 substituents selected from deuterium, oxo, halogen, $C_{1-4}$ alkyl or $C_{1-4}$ haloalkyl; wherein, $R_f$ has the meaning described in the present invention.

In other embodiments, each $R_f$ is independently H, deuterium, $C_{1-4}$ alkyl, -$C_{1-4}$ alkylene-O-$C_{1-4}$ alkyl, -$C_{1-4}$ alkylene-(5- to 7-membered heterocyclyl), -$C_{1-4}$ alkylene-$C_{3-6}$ cycloalkyl, 3- to 6-membered heterocyclyl, -C(=O)-(3- to 6-membered heterocyclyl), $C_{3-6}$ cycloalkyl or -C(=O)-$C_{3-6}$ cycloalkyl; wherein, each of the $C_{1-4}$ alkyl, -$C_{1-4}$ alkylene-O-$C_{1-4}$ alkyl, -$C_{1-4}$ alkylene-(5- to 7-membered heterocyclyl), -$C_{1-4}$ alkylene-$C_{3-6}$ cycloalkyl, 3- to 6-membered heterocyclyl, $C_{3-6}$ cycloalkyl, -C(=O)-(3- to 6-membered heterocyclyl) and -C(=O)-$C_{3-6}$ cycloalkyl is independently and optionally substituted with 1, 2 or 3 substituents selected from deuterium, F, Cl, Br, I, -OH, -CN, -$NH_2$ or COOH.

In still other embodiments, each of $R_0$, $R_1$, $R_2$, $R_3$, $R_4$, $R_5$, and $R_6$ is independently H, deuterium, F, Cl, Br, I, methyl, ethyl, *n*-propyl, isopropyl, *n*-butyl, tert-butyl, -$CH_2F$, -$CHF_2$, -$CF_3$, -$CH_2CH_2F$, -$CH_2CHF_2$, -$CHFCH_2F$, -$CH_2CF_3$, -$CH(CF_3)_2$, -$CF_2CH_2CH_3$, -$CH_2CH_2CH_2F$, -$CH_2CH_2CHF_2$, -$CH_2CH_2CF_3$, methoxy, ethoxy, *n*-propoxy, isopropoxy, *n*-butoxy, *tert*-butoxy, hydroxymethyl, 2-hydroxyethyl, 2-hydroxypropyl, 3-hydroxypropyl, -$C(OH)(CF_3)_2$, -$Si(CH_3)_3$, -$Si(CH_2CH_3)_3$, -$OCH_2F$, -$OCHF_2$, -$OCF_3$, -$OCH_2CH_2F$, -$OCH_2CHF_2$, -$OCHFCH_2F$, -$OCH_2CF_3$, -$OCH(CF_3)_2$, -$OCF_2CH_2CH_3$, -$OCH_2CH_2CH_2F$, -$OCH_2CH_2CHF_2$, -$OCH_2CH_2CF_3$ or -N($R_dR_e$); wherein, $R_d$ and $R_e$ have the meanings described in the present invention.

In still other embodiments, $R_7$ is -$S(=O)_2$-$CH_3$, -$S(=O)_2$-$CH_2CH_3$, -$S(=O)_2$-$CH_2CH_2CH_3$, -$S(=O)_2$-$CH(CH_3)CH_3$, -$S(=O)_2$-$OCH_3$, -$S(=O)_2$-$OCH_2CH_3$, -$S(=O)_2$-$OCH_2CH_2CH_3$, -$S(=O)_2$-$OCH(CH_3)CH_3$, -$S(=O)_2$-cyclopropyl, -$S(=O)_2$-cyclobutyl, -$S(=O)_2$-cyclopentyl, -$S(=O)_2$-cyclohexyl, -S(=O)-$CH_2$-cyclopropyl, -S(=O)-$CH_2$-cyclobutyl, -S(=O)-$CH_2$-cyclopentyl, -S(=O)-$CH_2$-cyclohexyl, -S(=O)-$CH_3$, -S(=O)-$CH_2CH_3$, -S(=O)-$CH_2CH_2CH_3$, -S(=O)-$CH(CH_3)CH_3$, -$S(=O)_2H$, -COOH, -C(=O)-N($R_gR_h$), -N($R_g$)-C(=O)-$CH_3$, -N($R_g$)-C(=O)-$CH_2CH_3$, -N($R_g$)-C(=O)-$CH_2CH_2CH_3$, -N($R_g$)-C(=O)-$CH(CH_3)CH_3$, -C(=O)-O-$CH_3$, -C(=O)-O-$CH_2CH_3$, -C(=O)-O-$CH_2CH_2CH_3$, -C(=O)-O-$CH(CH_3)CH_3$, methyl, ethyl, *n*-propyl, isopropyl, methoxy, ethoxy, *n*-propoxy, *iso*-propoxy, -$CH_2F$, -$CHF_2$, -$CF_3$, -$CH_2CH_2F$, -$CH_2CHF_2$, -$CHFCH_2F$, -$CH_2CF_3$, -$CH(CF_3)_2$, -$CF_2CH_2CH_3$, -$CH_2CH_2CH_2F$, -$CH_2CH_2CHF_2$ or -$CH_2CH_2CF_3$; wherein, $R_g$ and $R_h$ have the meanings described in the present invention.

In still other embodiments, each $R_g$ and $R_h$ is independently H, deuterium, methyl, ethyl, *n*-propyl, isopropyl, *n*-butyl, isobutyl, sec-butyl or tert-butyl.

In still other embodiments, each of A and G is independently cyclopropyl, cyclobutyl, cyclopentyl, cyclohexyl, phenyl, naphthyl, thiazolyl, pyrazolyl, imidazolyl, furanyl, oxazolyl, isoxazolyl, triazolyl, thienyl, pyrrolyl, pyridyl, pyrimidinyl, morpholinyl, thiomorpholinyl, tetrahydrofuranyl, tetrahydropyranyl, pyrrolidinyl, piperidinyl, piperazinyl,

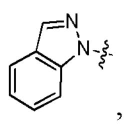, 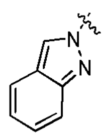, 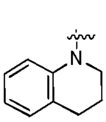, 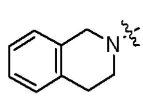, 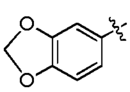 or 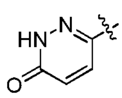;

wherein, each of A and G is independently and optionally substituted with 1, 2, 3, 4 or 5 $R_a$; and $R_a$ has the meaning described in the present invention.

In still other embodiments, B is

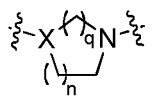, 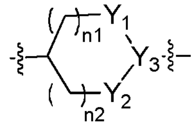, 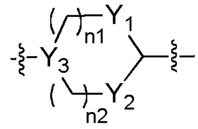,

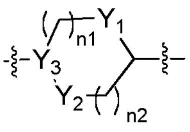 or 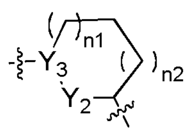 ;

wherein X is CH or N; each $Y_1$ and $Y_2$ is independently $CH_2$, NH, O, or S; $Y_3$ is CH or N; n is 0, 1 or 2; q is 1 or 2; n1 is 0, 1, 2 or 3; n2 is 0, 1 or 2; B is optionally substituted with 1, 2, 3, 4 or 5 $R_b$; wherein, $R_b$ has the meaning described in the present invention.

In still other embodiments, B is

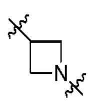, 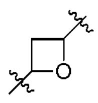, 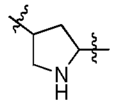, 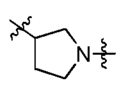, 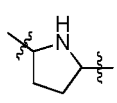,

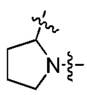, 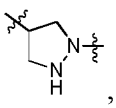, 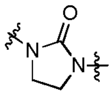, 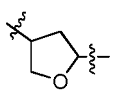, 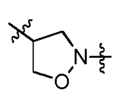, 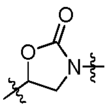, 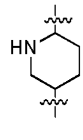, 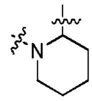, 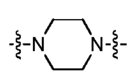, 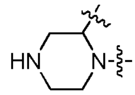,

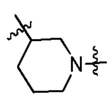, 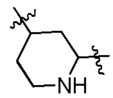, 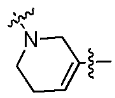, 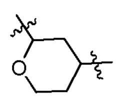, 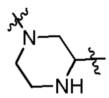, 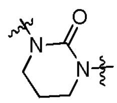, 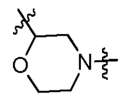, 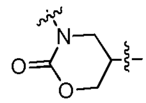, 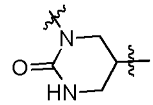,

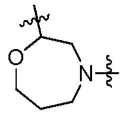, 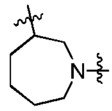, 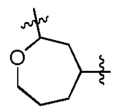, 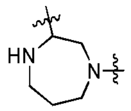 or 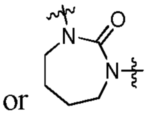

; wherein, B is optionally substituted with 1, 2, 3, 4 or 5 $R_b$; $R_b$ has the meaning described in the present invention.

In still other embodiments, each $R_a$ and $R_b$ is independently deuterium, F, Cl, Br, I, -OH, -CN, $-NH_2$, $-NO_2$, -COOH, oxo, methyl, ethyl, *n*-propyl, isopropyl, *n*-butyl, tert-butyl, $-CH_2F$, $-CHF_2$, $-CF_3$, $-CH_2CH_2F$, $-CH_2CHF_2$, $-CHFCH_2F$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, methoxy, ethoxy, *n*-propoxy, isopropoxy, *n*-butoxy, tert-butoxy, $-OCH_2F$, $-OCHF_2$, $-OCF_3$, $-OCH_2CH_2F$, $-OCH_2CHF_2$, $-OCHFCH_2F$, $-OCH_2CF_3$, $-OCH(CF_3)_2$, $-OCF_2CH_2CH_3$, $-OCH_2CH_2CH_2F$, $-OCH_2CH_2CHF_2$, $-OCH_2CH_2CF_3$, $-CH_2OH_3$, $-CH_2OH_2CH_3$, $-CH_2OH_2CH_2CH_3$, $-CH_2OH(CH_3)_2$, $-CH_2CH_2OH_3$, $-CH_2CH_2OH_2CH_3$, $-CH_2CH_2OH_2CH_2CH_3$, $-CH_2CH_2OH(CH_3)_2$, $-CH_2CH_2CH_2OH_3$, $-CH_2CH_2CH_2OH_2CH_3$, $-CH_2CH_2CH_2OH_2CH_2CH_3$, $-CH_2CH_2CH_2OH(CH_3)_2$, cyclopropyl, cyclobutyl, cyclopentyl, cyclohexyl, phenyl, naphthyl, pyridyl, pyrimidinyl, pyrrolyl, pyrazolyl, thiazolyl, imidazolyl, oxazolyl, triazolyl, tetrazolyl, piperazinyl, piperidinyl, morpholinyl, thiomorpholinyl, tetrahydropyranyl, pyrrolidinyl or $-C(=O)-N(R_dR_e)$;

wherein, each of the methyl, ethyl, *n*-propyl, isopropyl, *n*-butyl, tert-butyl, $-CH_2F$, $-CHF_2$, $-CH_2CH_2F$, $-CH_2CHF_2$, $-CHFCH_2F$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, methoxy, ethoxy, *n*-propoxy, isopropoxy, *n*-butoxy, tert-butoxy, $-OCH_2F$, $-OCHF_2$, $-OCH_2CH_2F$, $-OCH_2CHF_2$, $-OCHFCH_2F$, $-OCH_2CF_3$, $-OCH(CF_3)_2$, $-OCF_2CH_2CH_3$, $-OCH_2CH_2CH_2F$, $-OCH_2CH_2CHF_2$, $-OCH_2CH_2CF_3$, $-CH_2OH_3$, $-CH_2OH_2CH_3$, $-CH_2OH_2CH_2CH_3$, $-CH_2OH(CH_3)_2$, $-CH_2CH_2OH_3$, $-CH_2CH_2OH_2CH_3$, $-CH_2CH_2OH_2CH_2CH_3$, $-CH_2CH_2OH(CH_3)_2$, $-CH_2CH_2CH_2OH_3$, $-CH_2CH_2CH_2OH_2CH_3$, $-CH_2CH_2CH_2OH_2CH_2CH_3$, $-CH_2CH_2CH_2OH(CH_3)_2$, cyclopropyl, cyclobutyl, cyclopentyl, cyclohexyl, phenyl, naphthyl, pyridyl, pyrimidinyl, pyrrolyl, pyrazolyl, thiazolyl, imidazolyl, oxazolyl, triazolyl, tetrazolyl, piperazinyl, piperidinyl, morpholinyl, thiomorpholinyl, tetrahydropyranyl and pyrrolidinyl is independently and optionally substituted with 1, 2 or 3 $R_c$;
wherein, $R_c$, $R_d$ and $R_e$ have the meanings described in the present invention.

In still other embodiments, each $R_c$ is independently deuterium, F, Cl, Br, I, -OH, -CN, $-NH_2$, methyl, ethyl, *n*-propyl, isopropyl, *n*-butyl, tert-butyl, $-CH_2F$, $-CHF_2$, $-CF_3$, $-CH_2CH_2F$, $-CH_2CHF_2$, $-CHFCH_2F$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, methoxy, ethoxy, *n*-propoxy, isopropoxy, *n*-butoxy, *tert*-bu-

toxy, $-OCH_2F$, $-OCHF_2$, $-OCF_3$, $-OCH_2CH_2F$, $-OCH_2CHF_2$, $-OCHFCH_2F$, $-OCH_2CF_3$, $-OCH(CF_3)_2$, $-OCF_2CH_2CH_3$, $-OCH_2CH_2CH_2F$, $-OCH_2CH_2CHF_2$, $-OCH_2CH_2CF_3$, cyclopropyl, cyclobutyl, cyclopentyl, cyclohexyl, phenyl, naphthyl, pyridyl, pyrimidinyl, pyrrolyl, pyrazolyl, thiazolyl, imidazolyl, oxazolyl, triazolyl, tetrazolyl, piperazinyl, piperidinyl, morpholinyl, thiomorpholinyl, tetrahydropyranyl or pyrrolidinyl.

In still other embodiments, $R_8$ is H, deuterium, -OH, -CN, $-NH_2$, $-NO_2$, -COOH, methyl, ethyl, *n*-propyl, isopropyl, *n*-butyl, tert-butyl, $-CH_2F$, $-CHF_2$, $-CF_3$, $-CH_2CH_2F$, $-CH_2CHF_2$, $-CHFCH_2F$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, methoxy, ethoxy, *n*-propoxy, isopropoxy, *n*-butoxy, tert-butoxy, $-CH_2CN$, $-CH_2CH_2CN$, $-CH_2CH_2CH_2CN$, $-CH(CH_3)CH_2CN$, $-CH_2(CH_2)_3CN$, $-CH_2COOH$, $-CH_2CH_2COOH$, $-CH_2CH_2CH_2COOH$, $-CH(CH_3)CH_2COOH$, $-CH_2(CH_2)_3COOH$, $-CH_2OH_3$, $-CH_2OH_2CH_3$, $-CH_2OH_2CH_2CH_3$, $-CH_2OH(CH_3)_2$, $-CH_2CH_2OH_3$, $-CH_2CH_2OH_2CH_3$, $-CH_2CH_2OH_2CH_2CH_3$, $-CH_2CH_2OH(CH_3)_2$, $-CH_2CH_2CH_2OH_3$, $-CH_2CH_2CH_2OH_2CH_3$, $-CH_2CH_2CH_2OH_2CH_2CH_3$, $-CH_2CH_2CH_2OH(CH_3)_2$, $-CH_2-C(=O)-OCH_3$, $-CH_2-C(=O)-OCH_2CH_3$, $-CH_2-C(=O)-OCH_2CH_2CH_3$, $-CH_2-C(=O)-OCH(CH_3)_2$, $-CH_2CH_2-C(=O)-OCH_3$, $-CH_2CH_2-C(=O)-OCH_2CH_3$, $-CH_2CH_2-C(=O)-OCH_2CH_2CH_3$, $-CH_2CH_2-C(=O)-OCH_2(CH_3)_2$, $-CH_2CH_2CH_2-C(=O)-OCH_3$, $-CH_2CH_2CH_2-C(=O)-OCH_2CH_3$, $-CH_2CH_2CH_2-C(=O)-OCH_2CH_2CH_3$, $-CH_2CH_2CH_2-C(=O)-OCH(CH_3)_2$, $-CH_2-C(=O)-N(R_dR_e)$, $-CH_2CH_2-C(=O)-N(R_dR_e)$, $-CH_2CH_2CH_2-C(=O)-N(R_dR_e)$, $-CH_2-O-C(=O)-N(R_dR_e)$, $-CH_2CH_2-O-C(=O)-N(R_dR_e)$, $-CH_2CH_2CH_2-O-C(=O)-N(R_dR_e)$, $-CH_2-N(R_f)-C(=O)-N(R_dR_e)$, $-CH_2CH_2-N(R_f)-C(=O)-N(R_dR_e)$, $-CH_2CH_2CH_2-N(R_f)-C(=O)-N(R_dR_e)$, $-CH_2N(R_dR_e)$, $-CH_2CH_2N(R_dR_e)$, $-CH_2CH_2CH_2N(R_dR_e)$, $-N(R_f)-C(=O)-CH_3$, $-N(R_f)-C(=O)-CH_2CH_3$ or $-N(R_f)-C(=O)-CH(CH_3)_2$;

$R_9$ is deuterium, -OH, -CN, $-NH_2$, $-NO_2$, -COOH, $-CH_2F$, $-CHF_2$, $-CF_3$, $-CH_2CH_2F$, $-CH_2CHF_2$, $-CHFCH_2F$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, methoxy, ethoxy, *n*-propoxy, isopropoxy, *n*-butoxy, *tert*-butoxy, $-CH_2CN$, $-CH_2CH_2CN$, $-CH_2CH_2CH_2CN$, $-CH(CH_3)CH_2CN$, $-CH_2(CH_2)_3CN$, $-CH_2COOH$, $-CH_2CH_2COOH$, $-CH_2CH_2CH_2COOH$, $-CH(CH_3)CH_2COOH$, $-CH_2(CH_2)_3COOH$, $-CH_2OCH_3$, $-CH_2OCH_2CH_3$, $-CH_2OCH_2CH_2CH_3$, $-CH_2OCH(CH_3)_2$, $-CH_2CH_2OCH_3$, $-CH_2CH_2OCH_2CH_3$, $-CH_2CH_2OCH_2CH_2CH_3$, $-CH_2CH_2OCH(CH_3)_2$, $-CH_2CH_2CH_2OCH_3$, $-CH_2CH_2CH_2OCH_2CH_3$, $-CH_2CH_2CH_2OCH_2CH_2CH_3$, $-CH_2CH_2CH_2OCH(CH_3)_2$, $-CH_2-C(=O)-OCH_3$, $-CH_2-C(=O)-OCH_2CH_3$, $-CH_2-C(=O)-OCH_2CH_2CH_3$, $-CH_2-C(=O)-OCH(CH_3)_2$, $-CH_2CH_2-C(=O)-OCH_3$, $-CH_2CH_2-C(=O)-OCH_2CH_3$, $-CH_2CH_2-C(=O)-OCH_2CH_2CH_3$, $-CH_2CH_2-C(=O)-OCH_2(CH_3)_2$, $-CH_2CH_2CH_2-C(=O)-OCH_3$, $-CH_2CH_2CH_2-C(=O)-OCH_2CH_3$, $-CH_2CH_2CH_2-C(=O)-OCH_2CH_2CH_3$, $-CH_2CH_2CH_2-C(=O)-OCH(CH_3)_2$, $-CH_2-C(=O)-N(R_dR_e)$, $-CH_2CH_2-C(=O)-N(R_dR_e)$, $-CH_2CH_2CH_2-C(=O)-N(R_dR_e)$, $-CH_2-O-C(=O)-N(R_dR_e)$, $-CH_2CH_2-O-C(=O)-N(R_dR_e)$, $-CH_2CH_2CH_2-O-C(=O)-N(R_dR_e)$, $-CH_2-N(R_f)-C(=O)-N(R_dR_e)$, $-CH_2CH_2-N(R_f)-C(=O)-N(R_dR_e)$, $-CH_2CH_2CH_2-N(R_f)-C(=O)-N(R_dR_e)$, $-CH_2N(R_dR_e)$, $-CH_2CH_2N(R_dR_e)$, $-CH_2CH_2CH_2N(R_dR_e)$, $-N(R_f)-C(=O)-CH_3$, $-N(R_f)-C(=O)-CH_2CH_3$ or $-N(R_f)-C(=O)-CH(CH_3)_2$;
wherein, each of methyl, ethyl, *n*-propyl, isopropyl, *n*-butyl, *tert*-butyl, $-CH_2F$, $-CHF_2$, $-CH_2CH_2F$, $-CH_2CHF_2$, $-CHFCH_2F$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, methoxy, ethoxy, *n*-propoxy, isopropoxy, *n*-butoxy, *tert*-butoxy, $-CH_2CN$, $-CH_2CH_2CN$, $-CH_2CH_2CH_2CN$, $-CH(CH_3)CH_2CN$, $-CH_2(CH_2)_3CN$, $-CH_2COOH$, $-CH_2CH_2COOH$, $-CH_2CH_2CH_2COOH$, $-CH(CH_3)CH_2COOH$, $-CH_2(CH_2)_3COOH$, $-CH_2OCH_3$, $-CH_2OCH_2CH_3$, $-CH_2OCH_2CH_2CH_3$, $-CH_2OCH(CH_3)_2$, $-CH_2CH_2OCH_3$, $-CH_2CH_2OCH_2CH_3$, $-CH_2CH_2OCH_2CH_2CH_3$, $-CH_2CH_2OCH(CH_3)_2$, $-CH_2CH_2CH_2OCH_3$, $-CH_2CH_2CH_2OCH_2CH_3$, $-CH_2CH_2CH_2OCH_2CH_2CH_3$, $-CH_2CH_2CH_2OCH(CH_3)_2$, $-CH_2-C(=O)-OCH_3$, $-CH_2-C(=O)-OCH_2CH_3$, $-CH_2-C(=O)-OCH_2CH_2CH_3$, $-CH_2-C(=O)-OCH(CH_3)_2$, $-CH_2CH_2-C(=O)-OCH_3$, $-CH_2CH_2-C(=O)-OCH_2CH_3$, $-CH_2CH_2-C(=O)-OCH_2CH_2CH_3$, $-CH_2CH_2-C(=O)-OCH(CH_3)_2$, $-CH_2CH_2CH_2-C(=O)-OCH_3$, $-CH_2CH_2CH_2-C(=O)-OCH_2CH_3$, $-CH_2CH_2CH_2-C(=O)-OCH_2CH_2CH_3$ and $-CH_2CH_2CH_2-C(=O)-OCH(CH_3)_2$ described in $R_8$ and $R_9$ is independently and optionally substituted with 1, 2, 3 or 4 substituents selected from deuterium, F, Cl, Br, I, -OH, -CN, $-NH_2$, -COOH, methyl, ethyl, *n*-propyl, isopropyl , *n*-butyl, *tert*-butyl, trifluoromethyl, methoxy, ethoxy, *n*-propoxy, isopropoxy, *n*-butoxy, *tert*-butoxy, trifluoromethoxy or $-N(R_dR_e)$;
or, $R_8$ and $R_9$ together with the carbon atom to which they are attached, form cyclopentyl, cyclohexyl, oxetanyl, tetrahydrofuranyl, tetrahydropyranyl, azetidinyl, pyrrolidinyl, piperidinyl or piperazinyl; wherein, each of the cyclopentyl, cyclohexyl, oxetanyl, tetrahydrofuranyl, tetrahydropyranyl, azetidinyl, pyrrolidinyl, piperidinyl and piperazinyl is independently and optionally substituted with 1, 2, 3 or 4 substituents selected from deuterium, F, Cl, Br, I, -OH, -CN, $-NH_2$, -COOH, methyl, ethyl, *n*-propyl, isopropyl, *n*-butyl, *tert*-butyl, trifluoromethyl, methoxy, ethoxy, *n*-propoxy, isopropoxy, *n*-butoxy, *tert*-butoxy, trifluoromethoxy or $-N(R_dR_e)$;
wherein, $R_d$, $R_e$ and $R_f$ have the meanings described in the present invention.

In still other embodiments, each $R_d$ and $R_e$ is independently H, deuterium, -OH, methyl, ethyl, *n*-propyl, isopropyl, *n*-butyl, *tert*-butyl, -C(=O)H, $-C(=O)-O-CH_3$, $-C(=O)-O-CH_2CH_3$, $-C(=O)-O-CH_2CH_2CH_3$, $-C(=O)-O-CH(CH_3)_2$, $-C(=O)-CH_3$, $-C(=O)-CH_2CH_3$, $-C(=O)-CH_2CH_2CH_3$, $-C(=O)-CH(CH_3)_2$, $-CH_2OCH_3$, $-CH_2OCH_2CH_3$, $-CH_2OCH_2CH_2CH_3$, $-CH_2OCH(CH_3)_2$, $-CH_2CH_2OCH_3$, $-CH_2CH_2OCH_2CH_3$, $-CH_2CH_2OCH_2CH_2CH_3$,

$-CH_2CH_2OCH(CH_3)_2$, $-CH_2CH_2CH_2OCH_3$, $-CH_2CH_2CH_2OCH_2CH_3$, $-CH_2CH_2CH_2OCH_2CH_2CH_3$, $-CH_2CH_2CH_2OCH(CH_3)_2$, $-CH_2-C(=O)-OCH_3$, $-CH_2-C(=O)-OCH_2CH_3$, $-CH_2-C(=O)-OCH_2CH_2CH_3$, $-CH_2-C(=O)-OCH(CH_3)_2$, $-CH_2CH_2-C(=O)-OCH_3$, $-CH_2CH_2-C(=O)-OCH_2CH_3$, $-CH_2CH_2-C(=O)-OCH_2CH_2CH_3$, $-CH_2CH_2-C(=O)-OCH(CH_3)_2$, $-CH_2CH_2CH_2-C(=O)-OCH_3$, $-CH_2CH_2CH_2-C(=O)-OCH_2CH_3$, $-CH_2CH_2CH_2-C(=O)-OCH_2CH_2CH_3$ or $-CH_2CH_2CH_2-C(=O)-OCH(CH_3)_2$;
wherein, each of the methyl, ethyl, *n*-propyl, isopropyl, *n*-butyl, *tert*-butyl, $-C(=O)-O-CH_3$, $-C(=O)-O-CH_2CH_3$, $-C(=O)-O-CH_2CH_2CH_3$, $-C(=O)-O-CH(CH_3)_2$, $-C(=O)-CH_3$, $-C(=O)-CH_2CH_3$, $-C(=O)-CH_2CH_2CH_3$, $-C(=O)-CH(CH_3)_2$, $-CH_2OCH_3$, $-CH_2OCH_2CH_3$, $-CH_2OCH_2CH_2CH_3$, $-CH_2OCH(CH_3)_2$, $-CH_2CH_2OCH_3$, $-CH_2CH_2OCH_2CH_3$, $-CH_2CH_2OCH_2CH_2CH_3$, $-CH_2CH_2OCH(CH_3)_2$, $-CH_2CH_2CH_2OCH_3$, $-CH_2CH_2CH_2OCH_2CH_3$, $-CH_2CH_2CH_2OCH_2CH_2CH_3$, $-CH_2CH_2CH_2OCH(CH_3)_2$, $-CH_2-C(=O)-OCH_3$, $-CH_2-C(=O)-OCH_2CH_3$, $-CH_2-C(=O)-OCH_2CH_2CH_3$, $-CH_2-C(=O)-OCH(CH_3)_2$, $-CH_2CH_2-C(=O)-OCH_3$, $-CH_2CH_2-C(=O)-OCH_2CH_3$, $-CH_2CH_2-C(=O)-OCH_2CH_2CH_3$, $-CH_2CH_2-C(=O)-OCH(CH_3)_2$, $-CH_2CH_2CH_2-C(=O)-OCH_3$, $-CH_2CH_2CH_2-C(=O)-OCH_2CH_3$, $-CH_2CH_2CH_2-C(=O)-OCH_2CH_2CH_3$ and $-CH_2CH_2CH_2-C(=O)-OCH(CH_3)_2$ is independently and optionally substituted with 1, 2, 3 or 4 substituents selected from deuterium, F, Cl, Br, I, -OH, -CN, $-NH_2$ or -COOH.

In still other embodiments, $L_1$ is a bond, **-O-, **-C(=O)-, **-NH-, **-$CH_2$-, **-$CH_2O$-, **-$CH_2CH_2O$-, **-O-$CH_2$-, **-O-$CH_2CH_2$-, **-C(=O)-N($R_f$)-, **-N($R_f$)-C(=O)-, **-N($R_f$)-$CH_2$-, **-N($R_f$)-$CH_2CH_2$-, **-$CH_2$-N($R_f$)- or **-$CH_2CH_2$-N($R_f$)-;

wherein, each of the **-$CH_2$-, **-$CH_2O$-, **-$CH_2CH_2O$-, **-O-$CH_2$-, **-O-$CH_2CH_2$-, **-N($R_f$)-$CH_2$-, **-N($R_f$)-$CH_2CH_2$-, **-$CH_2$-N($R_f$)- and **-$CH_2CH_2$-N($R_f$)- is independently and optionally substituted with 1, 2, 3 or 4 substituents selected from deuterium, oxo, F, Cl, Br, methyl, ethyl, *n*-propyl, isopropyl, *n*-butyl, isobutyl, *tert*-butyl, $-CH_2F$, $-CHF_2$, $-CF_3$, $-CH_2CH_2F$, $-CH_2CHF_2$, $-CHFCH_2F$, $-CH_2CF_3$, $-CH(CF_3)_2$, $-CF_2CH_2CH_3$, $-CH_2CH_2CH_2F$, $-CH_2CH_2CHF_2$ or $-CH_2CH_2CF_3$;
wherein, $R_f$ is as defined herein.

In still other embodiments, $L_2$ is a bond, -O-, -C(=O)-, -NH-, $-CH_2-$, $-CH_2O-$, $-CH_2CH_2O-$, $-O-CH_2-$, $-O-CH_2CH_2-$, -C(=O)-N($R_f$)-, -N($R_f$)-C(=O)-, -N($R_f$)-$CH_2$-, -N($R_f$)-$CH_2CH_2$-, -$CH_2$-N($R_f$)- or -$CH_2CH_2$-N($R_f$)-;

wherein, each of the $-CH_2-$, $-CH_2O-$, $-CH_2CH_2O-$, $-O-CH_2-$, $-O-CH_2CH_2-$, -N($R_f$)-$CH_2$-, -N($R_f$)-$CH_2CH_2$-, -$CH_2$-N($R_f$)- and -$CH_2CH_2$-N($R_f$)- is independently and optionally substituted with 1, 2, 3 or 4 substituents selected from deuterium, oxo, F, Cl, Br, methyl, ethyl, *n*-propyl, isopropyl, *n*-butyl, isobutyl, *tert*-butyl, $-CH_2F$, $-CHF_2$, $-CF_3$, $-CH_2CH_2F$, $-CH_2CHF_2$, $-CHFCH_2F$, $-CH_2CF_3$, $-CH(CF_3)_2$, $-CF_2CH_2CH_3$, $-CH_2CH_2CH_2F$, $-CH_2CH_2CHF_2$ or $-CH_2CH_2CF_3$;
wherein, $R_f$ is as defined herein.

In still other embodiments, each $R_f$ is independently H, deuterium, methyl, ethyl, *n*-propyl, isopropyl, *n*-butyl, isobutyl, *tert*-butyl, $-CH_2OCH_3$, $-CH_2OCH_2CH_3$, $-CH_2OCH_2CH_2CH_3$, $-CH_2OCH(CH_3)_2$, $-CH_2CH_2OCH_3$, $-CH_2CH_2OCH_2CH_3$, $-CH_2CH_2OCH_2CH_2CH_3$, $-CH_2CH_2OCH(CH_3)_2$, $-CH_2CH_2CH_2OCH_3$, $-CH_2CH_2CH_2OCH_2CH_3$, $-CH_2CH_2CH_2OCH_2CH_2CH_3$, $-CH_2CH_2CH_2OCH(CH_3)_2$, tetrahydrofurylmethylene, tetrahydropyranylmethylene, pyrrolidinylmethylene, piperazinylmethylene, cyclopropylmethylene, cyclopropylethylene, cyclopropyl-*n*-propylene, cyclobutylmethylene, cyclobutylethylene, cyclobutyl-*n*-propylene, cyclopentylmethylene, cyclopentylethylene, cyclopentyl-*n*-propylene, cyclohexylmethylene , cyclohexylethylene, cyclohexyl-*n*-propylene, cyclopropyl, cyclobutyl, cyclopentyl, cyclohexyl, oxiranyl, aziridinyl, oxetanyl, azetidinyl, pyrrolidinyl, tetrahydrofuranyl, tetrahydrothienyl, piperazinyl, piperidinyl, morpholinyl, thiomorpholinyl, tetrahydropyranyl,-C(=O)-oxiranyl, -C(=O)-aziridinyl, -C(=O)-oxetanyl, -C(=O)-azetidinyl, -C(=O)-pyrrolidinyl, -C(=O)-tetrahydrofuranyl, -C(=O)-tetrahydrothienyl, -C(=O)-piperazinyl, -C(=O)-piperidinyl, -C(=O)-morpholinyl, -C(=O)-thiomorpholinyl, -C(=O)-tetrahydropyranyl, -C(=O)-cyclopropyl, -C(=O)-cyclobutyl, -C(=O)-cyclopentyl or -C(=O)-cyclohexyl;
each of the methyl, ethyl, *n*-propyl, isopropyl, *n*-butyl, isobutyl, tert-butyl, $-CH_2OCH_3$, $-CH_2OCH_2CH_3$, $-CH_2OCH_2CH_2CH_3$, $-CH_2OCH(CH_3)_2$, $-CH_2CH_2OCH_3$, $-CH_2CH_2OCH_2CH_3$, $-CH_2CH_2OCH_2CH_2CH_3$, $-CH_2CH_2OCH(CH_3)_2$, $-CH_2CH_2CH_2OCH_3$, $-CH_2CH_2CH_2OCH_2CH_3$, $-CH_2CH_2CH_2OCH_2CH_2CH_3$, $-CH_2CH_2CH_2OCH(CH_3)_2$, tetrahydrofurylmethylene, tetrahydropyranylmethylene, pyrrolidinylmethylene, piperazinylmethylene, cyclopropylmethylene, cyclopropylethylene, cyclopropyl-*n*-propylene, cyclobutylmethylene, cyclobutylethylene, cyclobutyl-*n*-propylene, cyclopentylmethylene, cyclopentylethylene, cyclopentyl-*n*-propylene, cyclohexylmethylene, cyclohexylethylene, cyclohexyl-*n*-propylene, cyclopropyl, cyclobutyl, cyclopentyl, cyclohexyl, oxiranyl, aziridinyl, oxetanyl, azetidinyl, pyrrolidinyl, tetrahydrofuranyl, tetrahydrothienyl, piperazinyl, piperidinyl, morpholinyl, thiomorpholinyl, tetrahydropyranyl,-C(=O)-oxiranyl, -C(=O)-aziridinyl, -C(=O)-oxetanyl, -C(=O)-azetidinyl, -C(=O)-pyrrolidinyl, -C(=O)-tetrahydrofuranyl, -C(=O)-tetrahydrothienyl, -C(=O)-piperazinyl, -C(=O)-piperidinyl, -C(=O)-morpholinyl, -C(=O)-thiomorpholinyl, -C(=O)-tetrahydropyranyl, -C(=O)-cyclopropyl, -C(=O)-cyclobutyl, -C(=O)-cyclopentyl and -C(=O)-cyclohexyl is independently and optionally substituted with 1, 2 or 3 substituents selected from deuterium, F, Cl, Br, I, -OH, -CN, $-NH_2$ or -COOH.

In some embodiments, the present invention provides a compound having Formula (II) or a stereoisomer, a

geometric isomer, a tautomer, an *N*-oxide, a hydrate, a solvate, a metabolite, an ester, a pharmaceutically acceptable salt or a prodrug thereof,

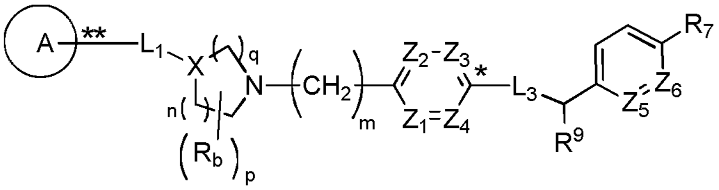

(II),

wherein, n is 0, 1 or 2; p is 0, 1, 2, 3 or 4; q is 1 or 2; X is N or CH.

In other embodiments, the present invention provides a compound having Formula (III) or a stereoisomer, a geometric isomer, a tautomer, an *N*-oxide, a hydrate, a solvate, a metabolite, an ester, a pharmaceutically acceptable salt or a prodrug thereof,

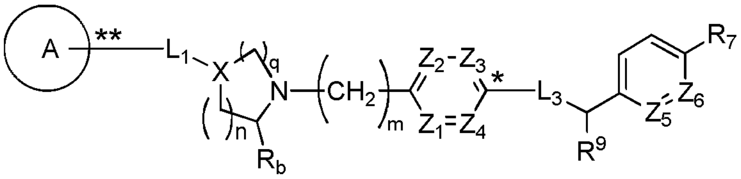

(III),

wherein, n is 0, 1 or 2; q is 1 or 2; X is N or CH.

In still other embodiments, the present invention provides a compound having Formula (IV) or a compound having Formula (V), or a stereoisomer, a geometric isomer, a tautomer, an *N*-oxide, a hydrate, a solvate, a metabolite, an ester, a pharmaceutically acceptable salt or a prodrug thereof,

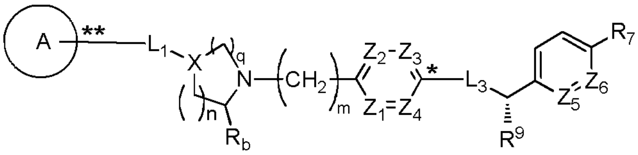

(IV) or

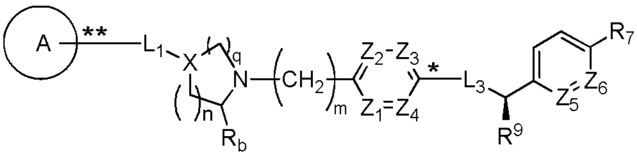

(V),

wherein, n is 0, 1 or 2; q is 1 or 2; X is N or CH.

In some embodiments, the present invention relates to, but is by no means limited to, one of the following compounds, or a stereoisomer, an *N*-oxide, a solvate, a metabolite, a pharmaceutically acceptable salt or a prodrug thereof:

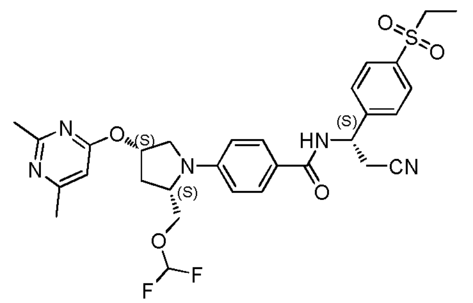

(1),

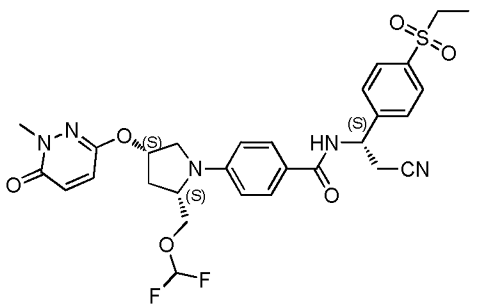

(2),

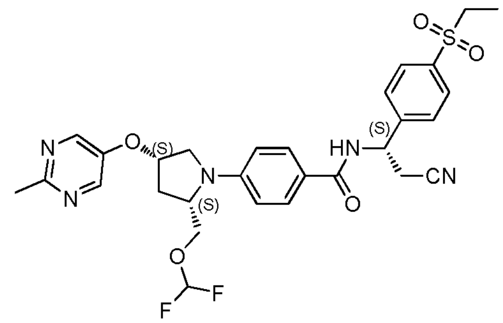

(3),

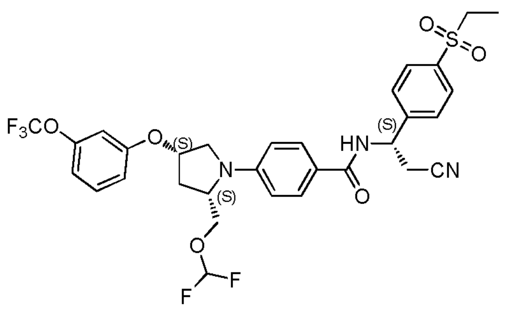

(4),

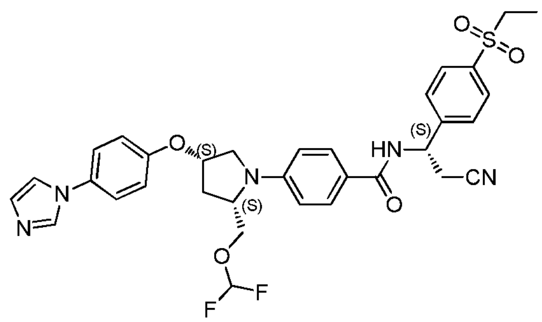

(5),

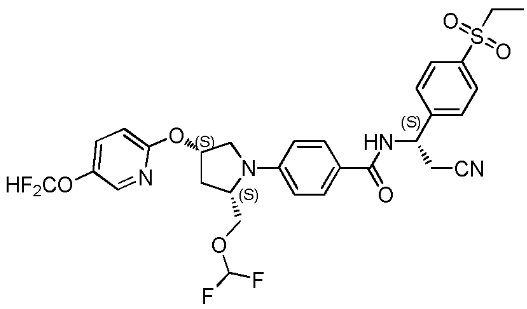

(6),

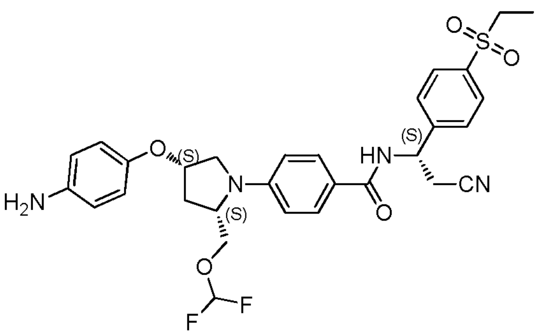

(7),

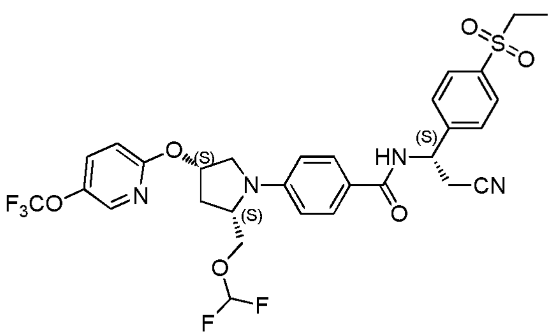

(8),

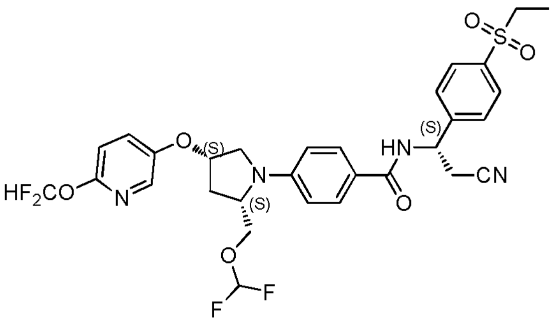

(9),

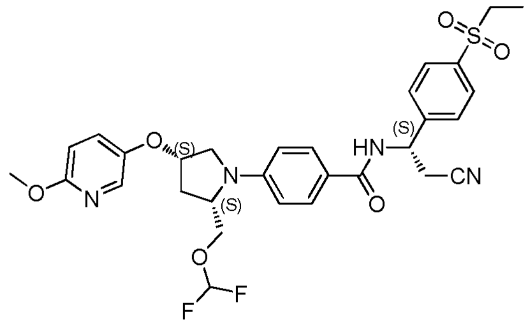

(10),

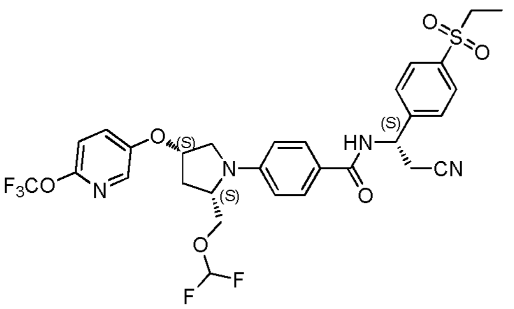

(11),

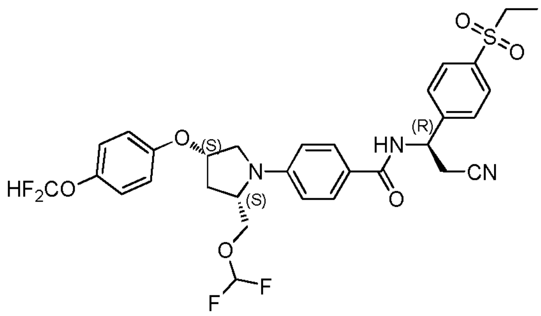

(12),

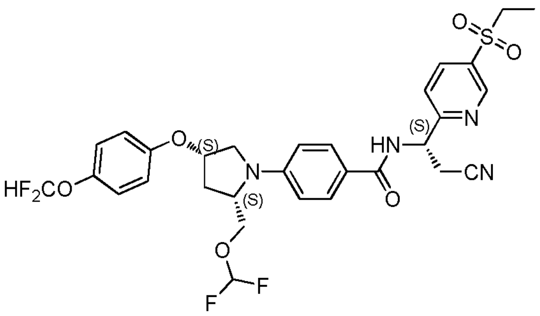

(13),

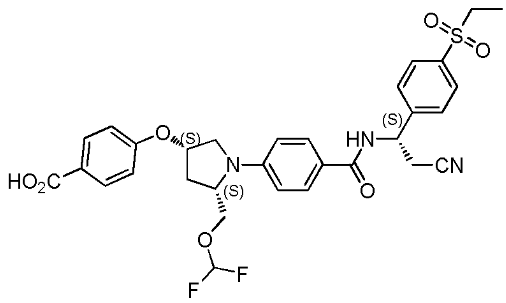

(14),

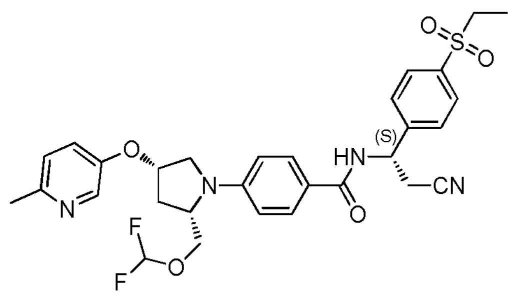

(15),

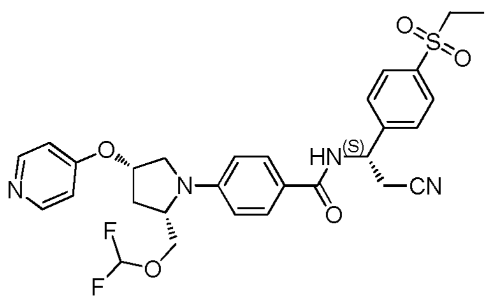

(16),

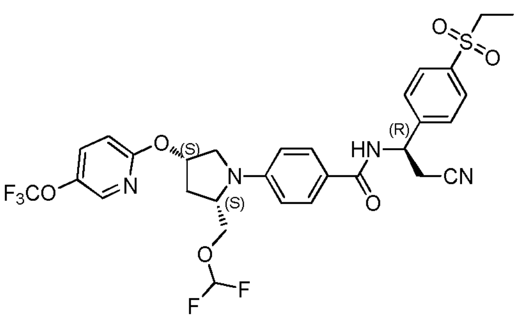

(17),

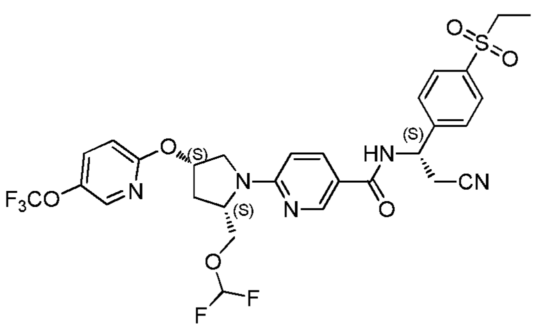

(18),

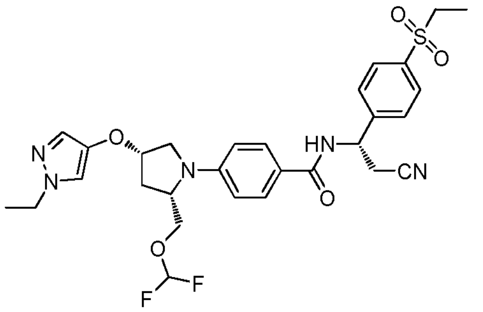
(19),

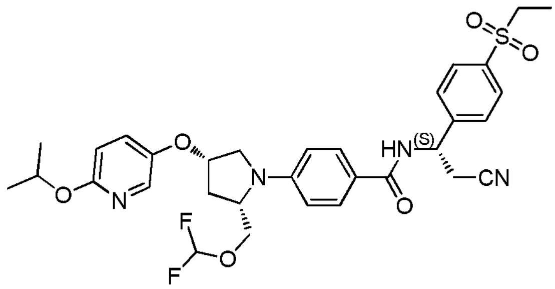
(20),

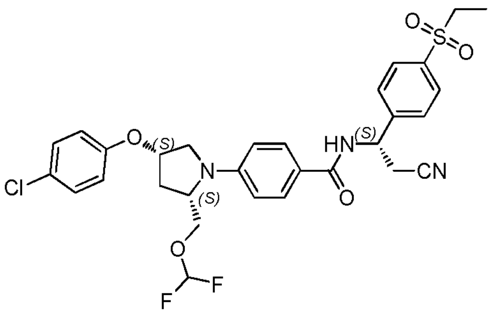
(21),

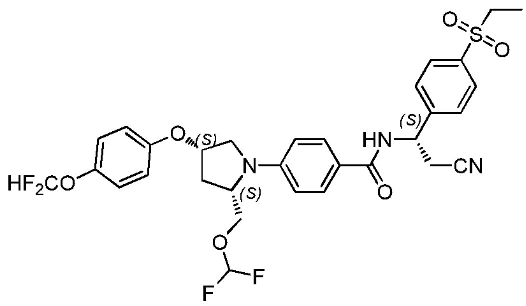
(22),

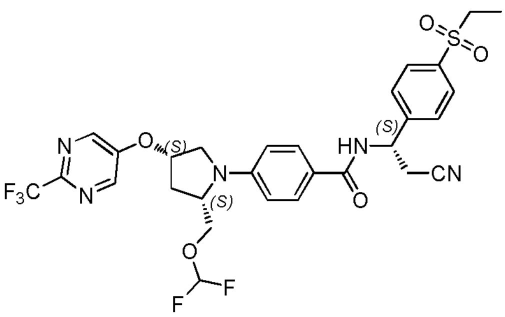
(23),

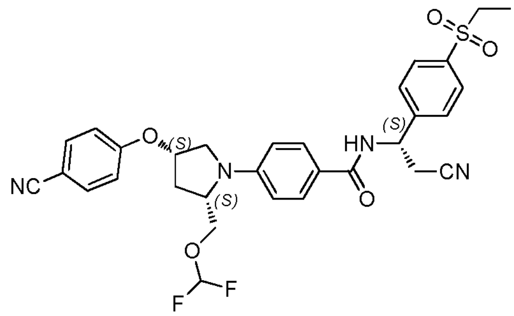
(24),

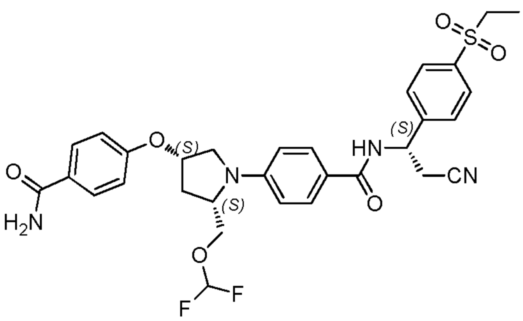
(25),

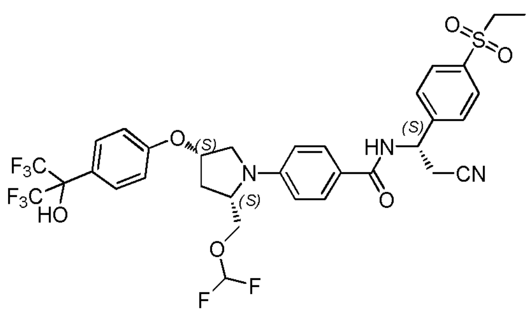
(26),

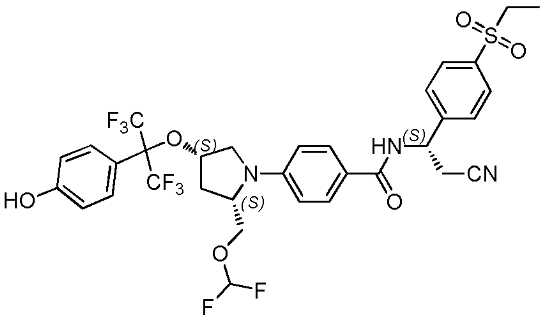

(27),

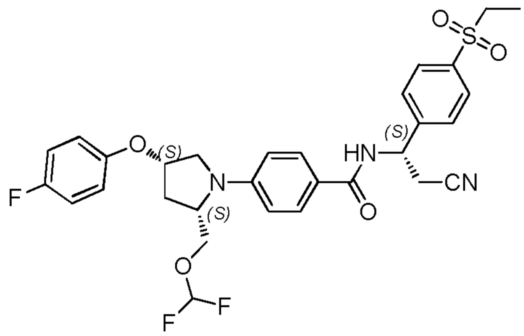

(28),

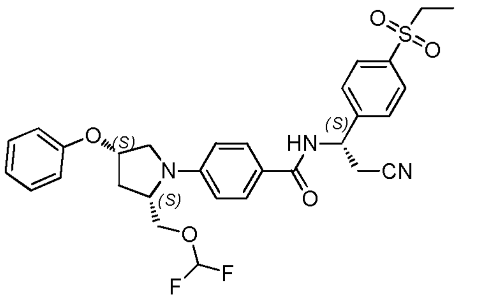

(29),

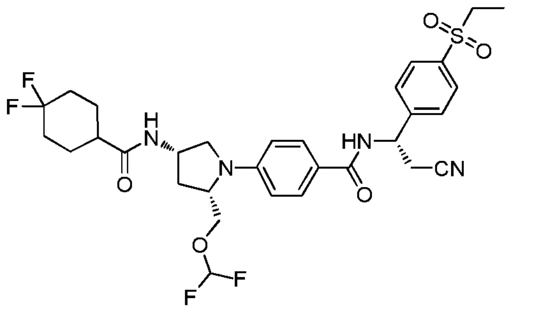

(30),

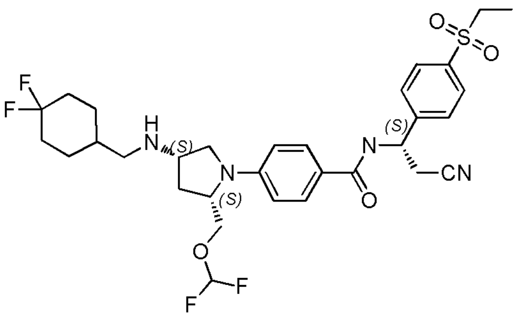

(31),

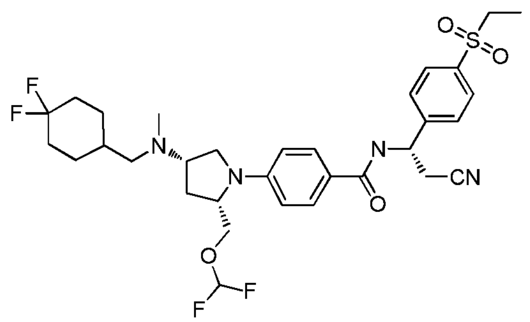

(32),

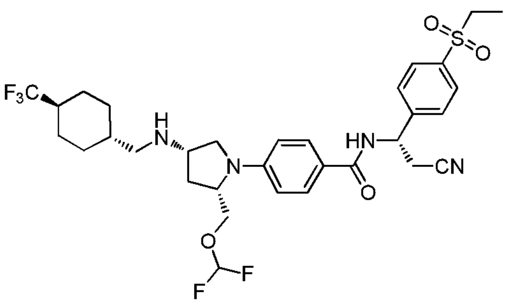

(33),

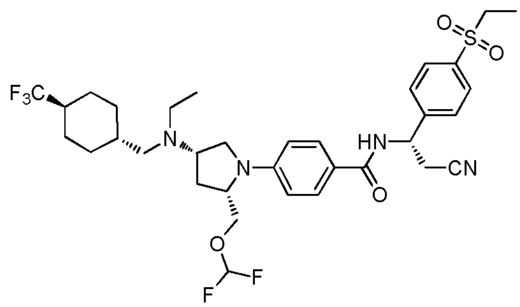

(34),

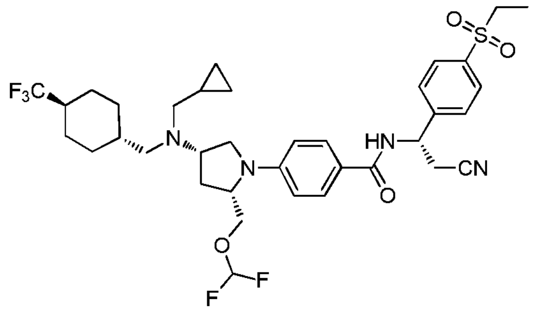

(35),

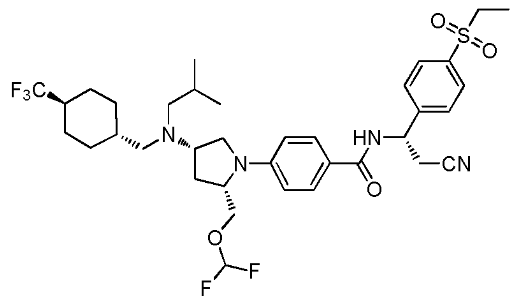

(36),

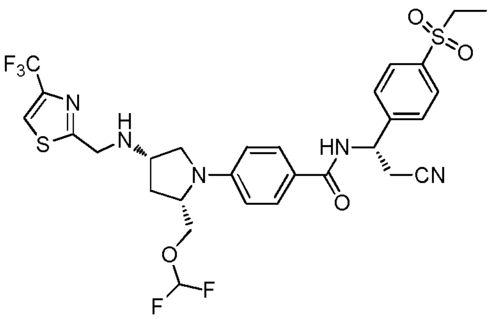

(37),

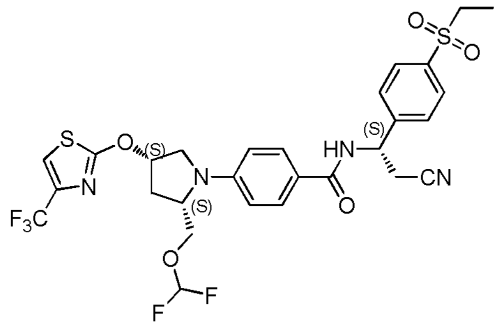

(38),

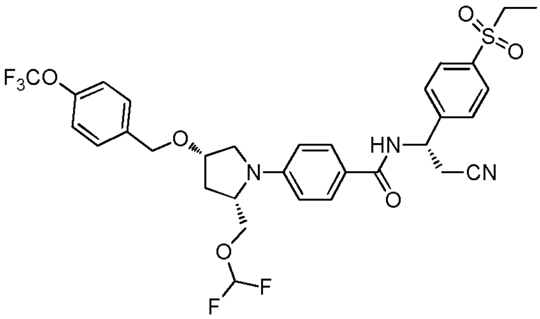

(39),

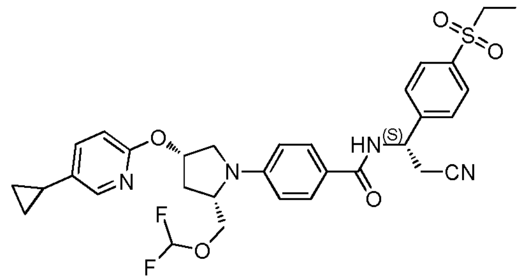

(40),

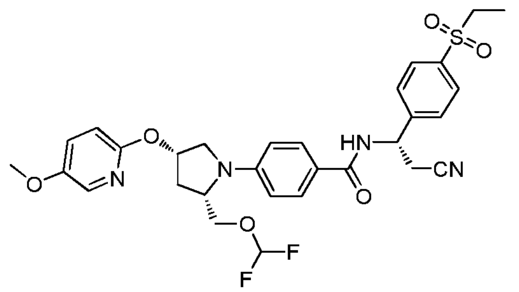

(41),

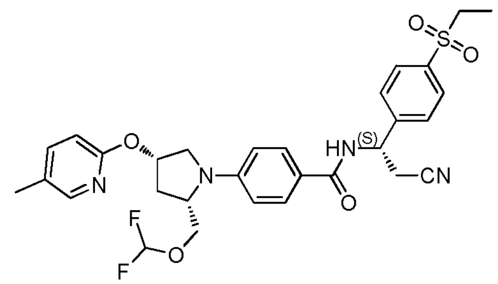

(42),

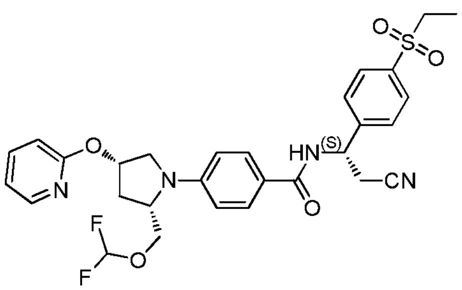

(43),

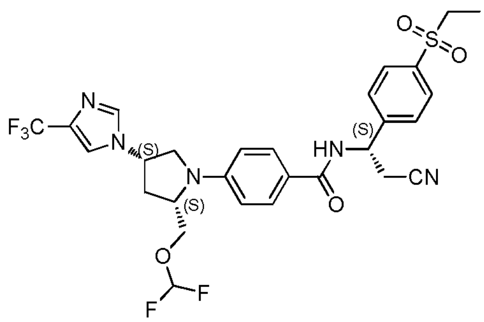

(44),

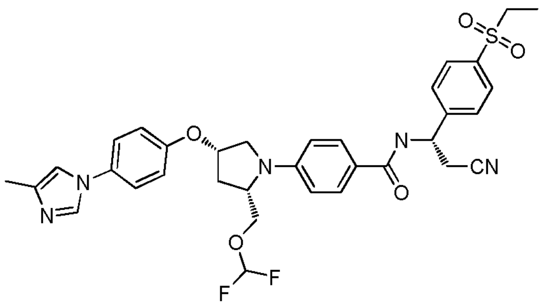
(45),

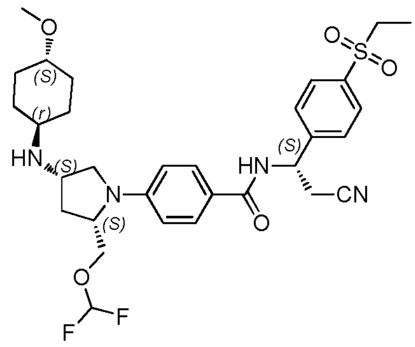
(46),

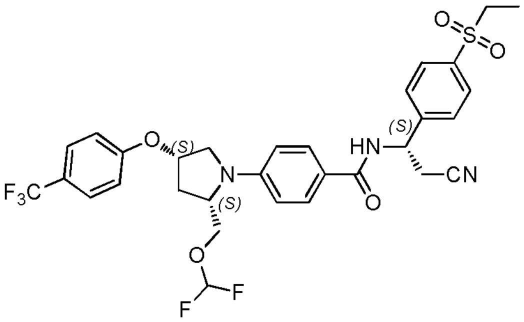
(47),

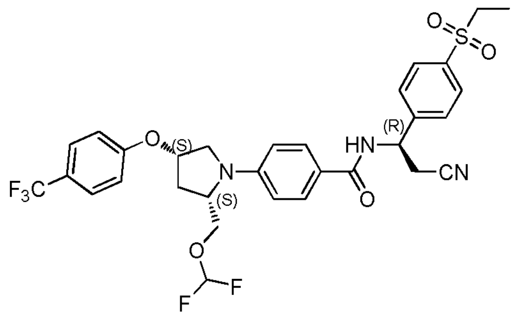
(48),

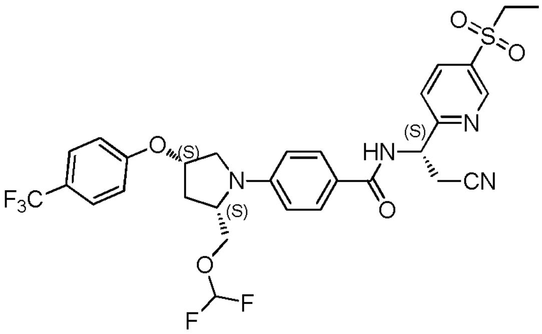
(49),

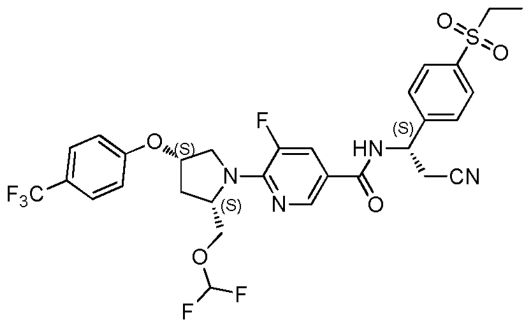
(50),

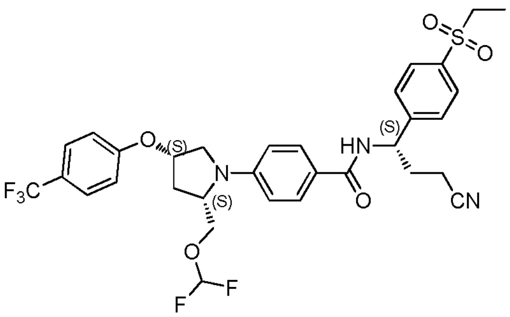
(51),

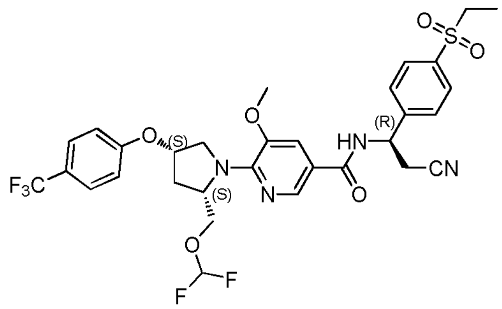
(52),

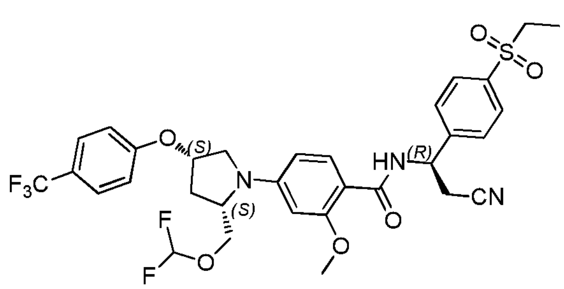
(53),

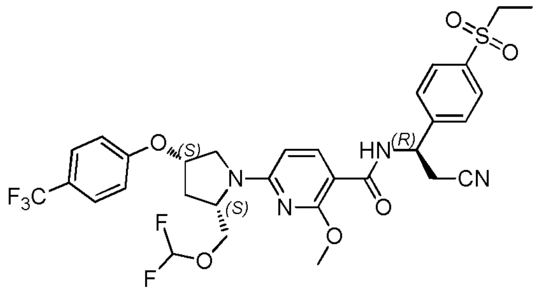
(54),

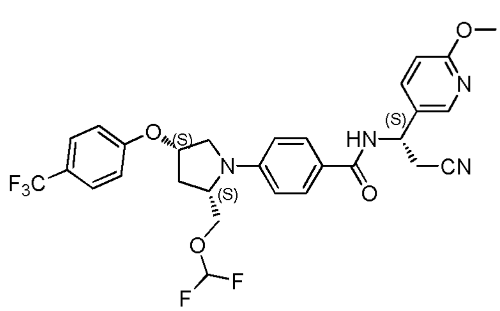
(55),

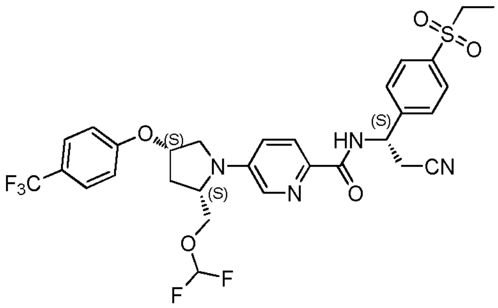
(56),

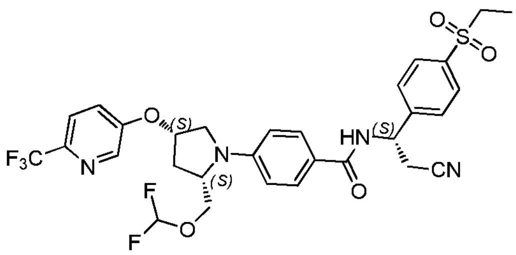
(57),

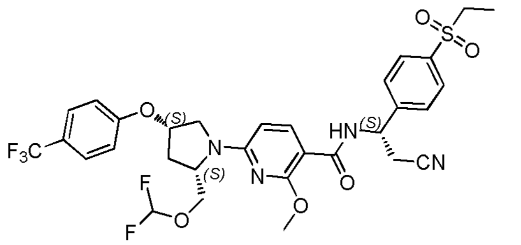
(58),

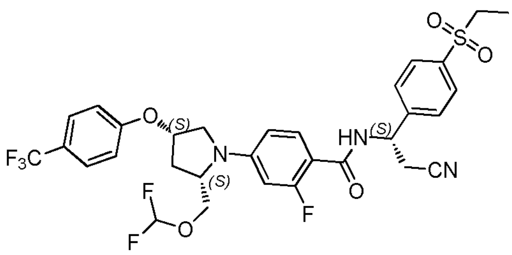
(59),

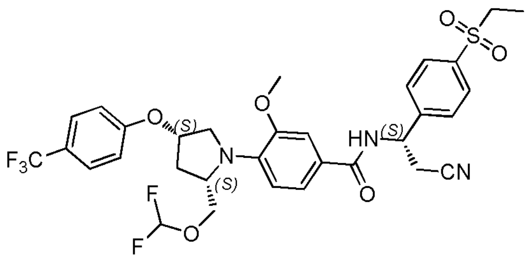
(60),

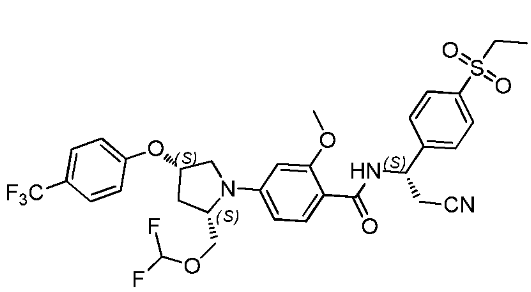
(61),

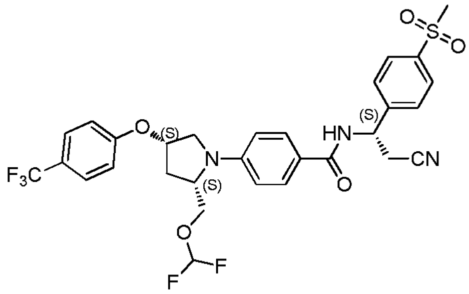
(62),

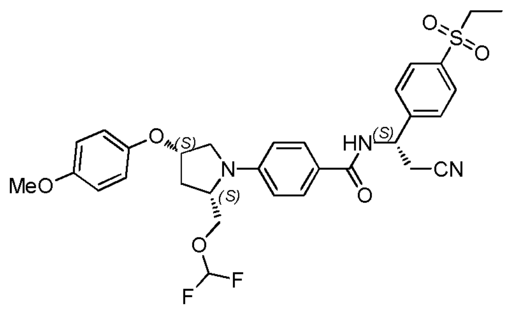

(63),

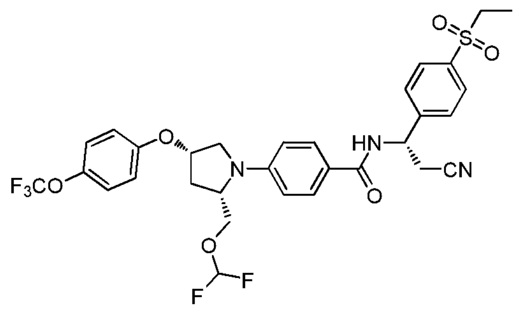

(64),

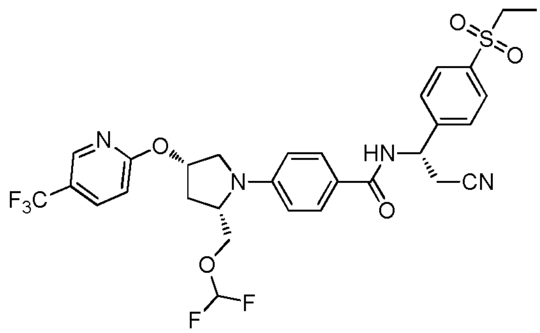

(65),

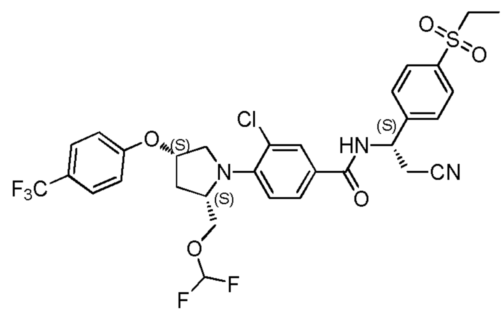

(66),

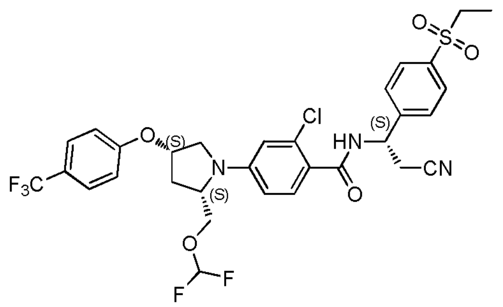

(67),

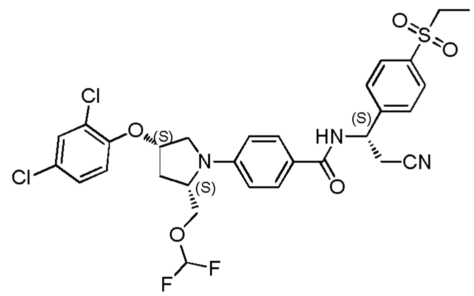

(68),

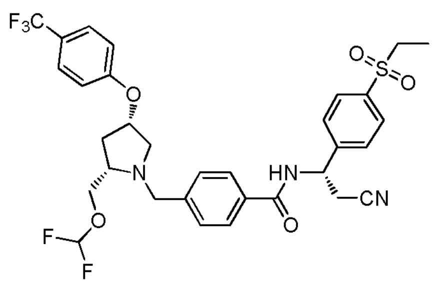

(69),

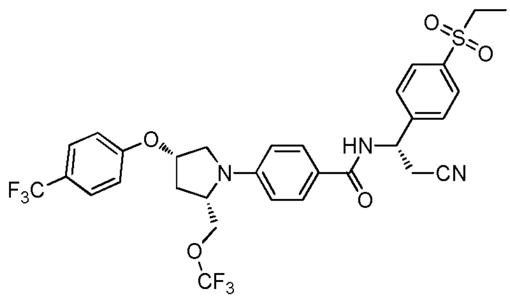

(70),

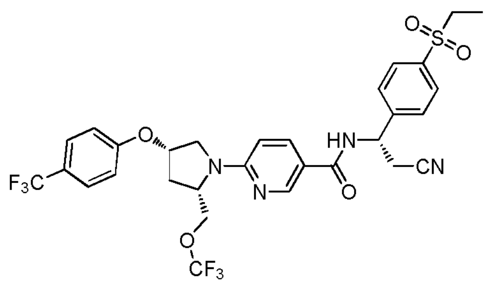

(71),

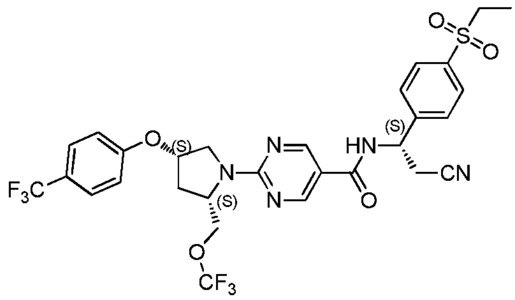

(72),

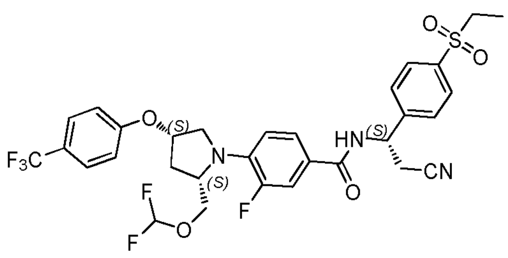
(73),

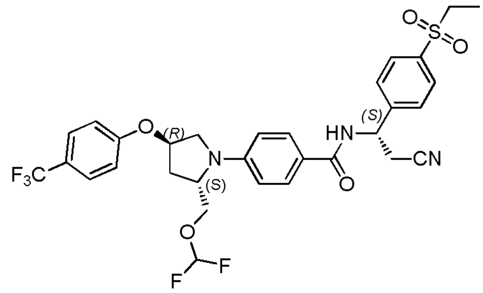
(74),

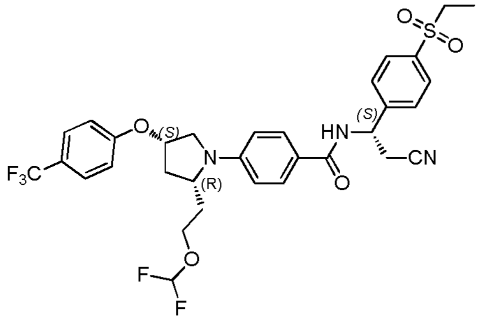
(75),

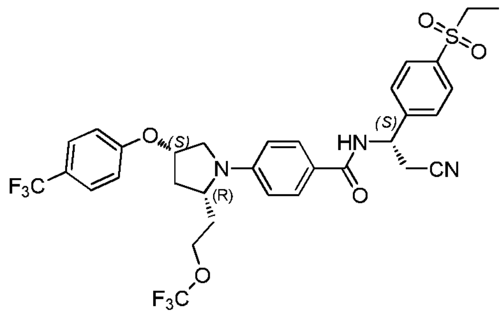
(76),

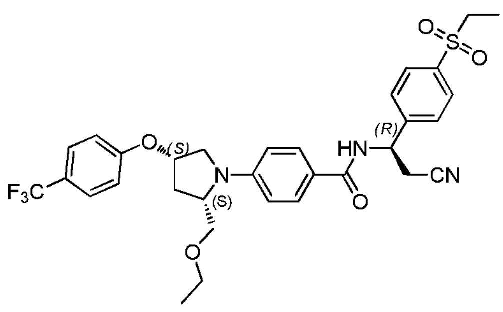
(77),

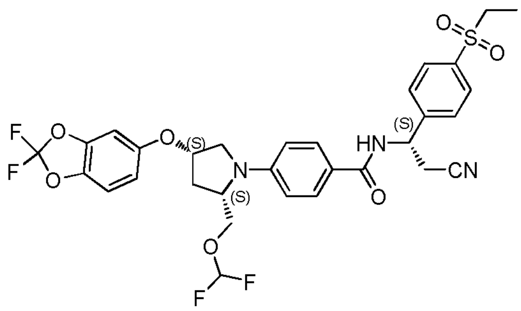
(78),

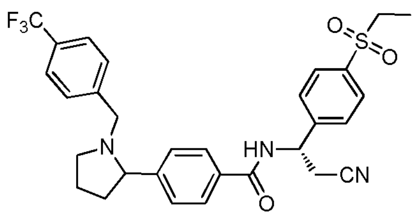
(79),

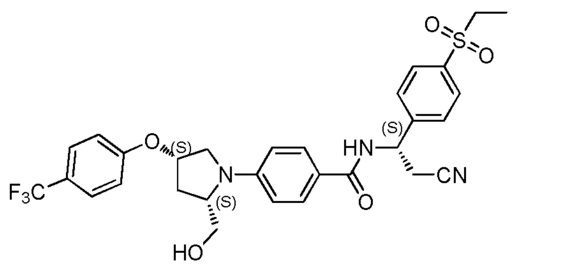
(80),

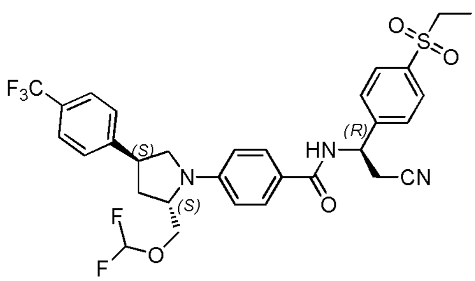
(81),

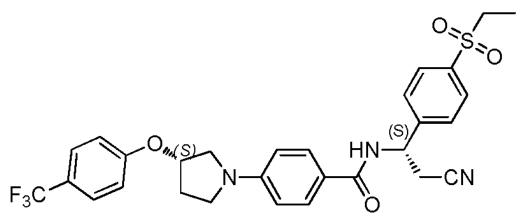
(82),

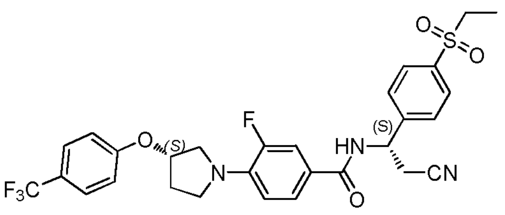 (83), 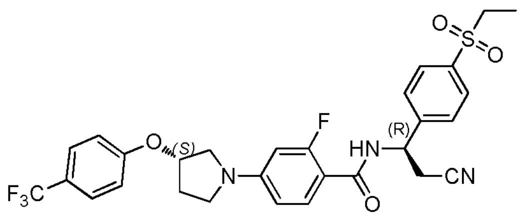 (84),

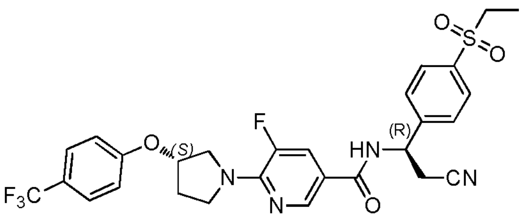 (85), 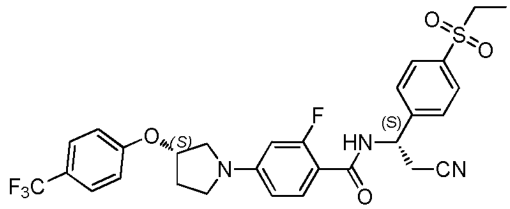 (86),

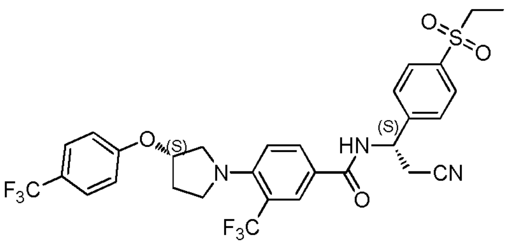 (87), 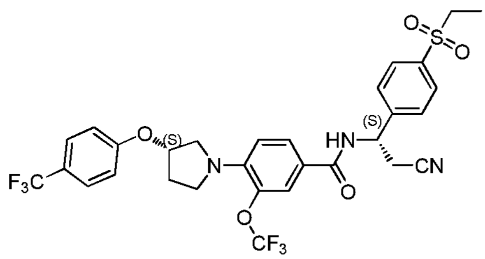 (88),

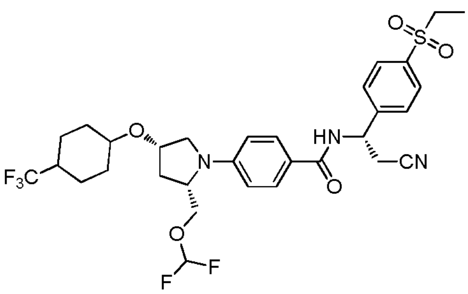 (89), 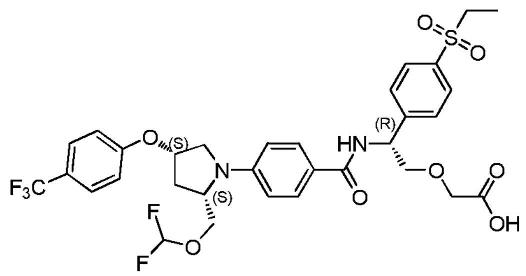 (90),

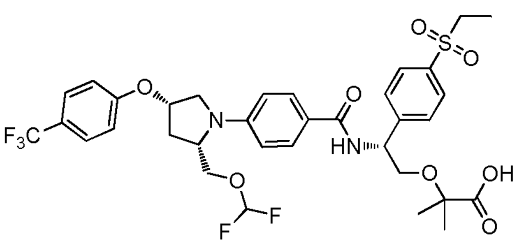 (91), 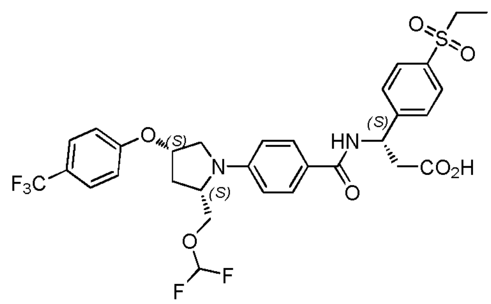 (92),

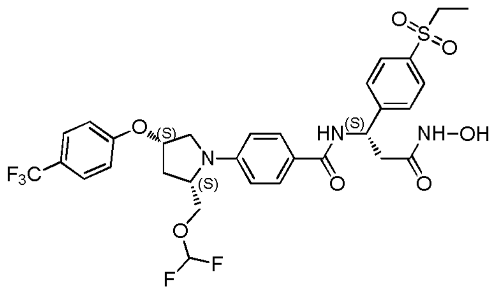

(93),

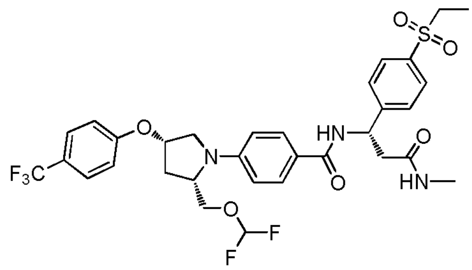

(94),

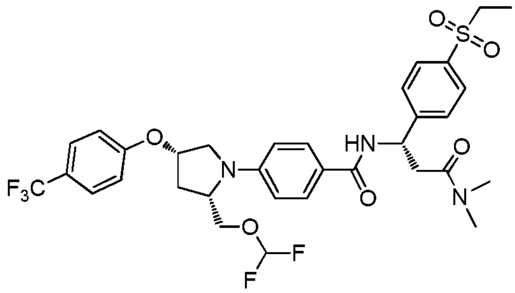

(95),

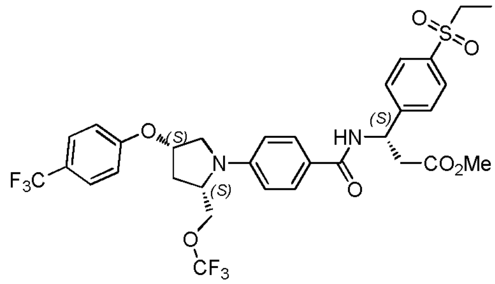

(96),

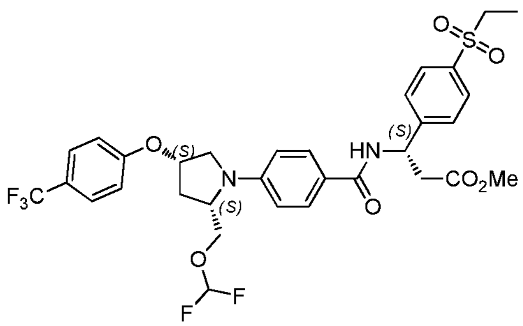

(97),

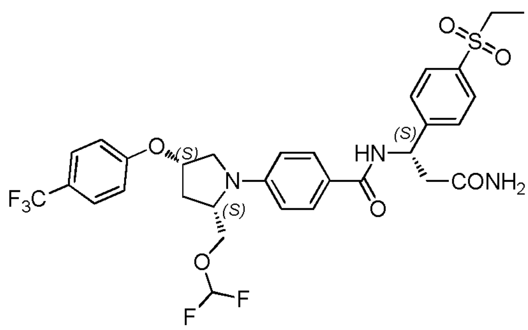

(98),

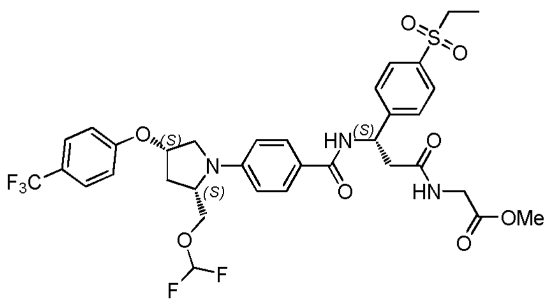

(99),

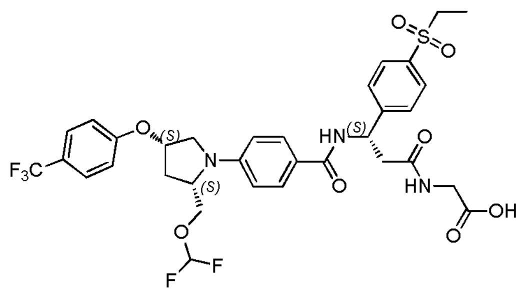

(100),

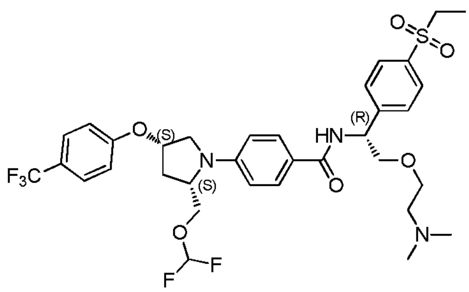

(101),

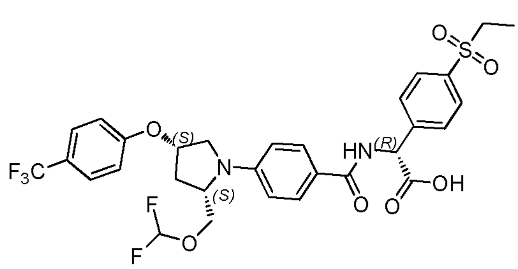

(102),

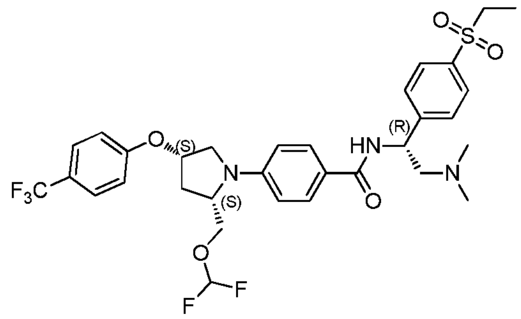

(103),

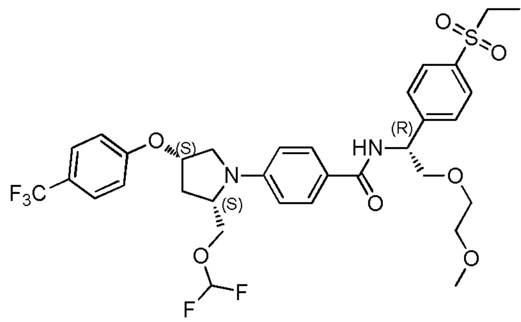

(104),

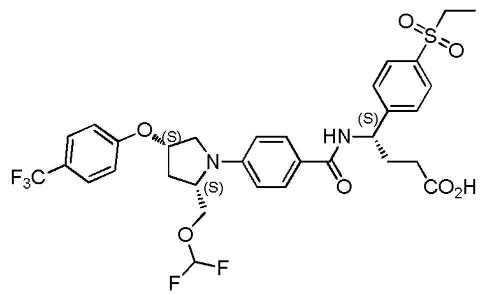

(105),

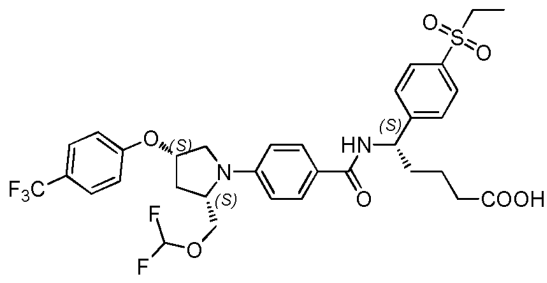

(106),

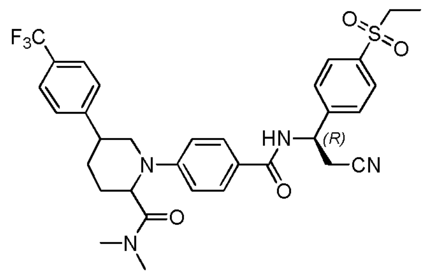

(107),

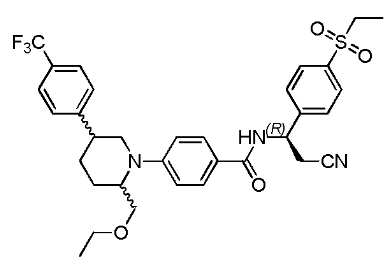

(108),

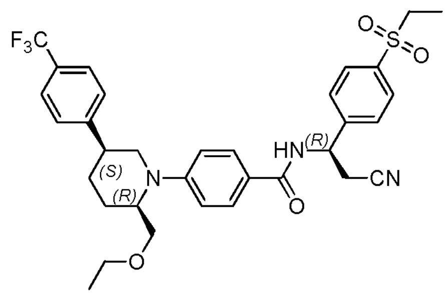

(109),

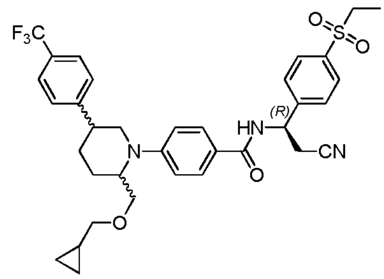

(110),

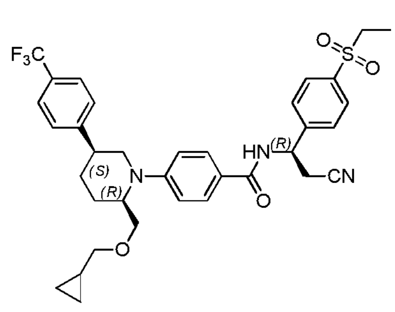

(111),

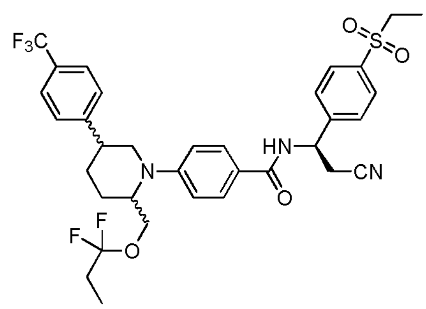

(112),

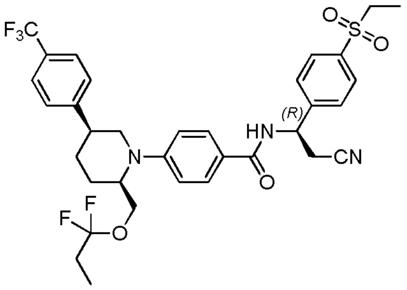

(113),

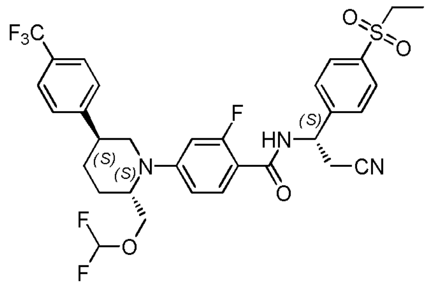

(114),

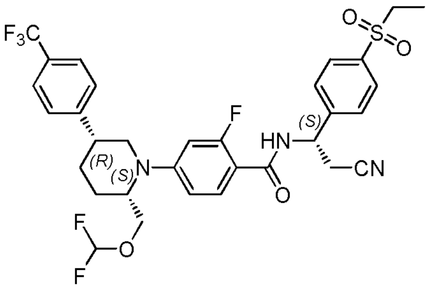

(115),

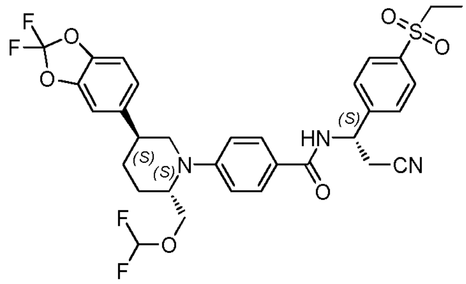

(116),

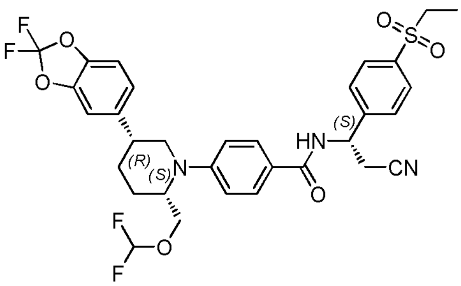

(117),

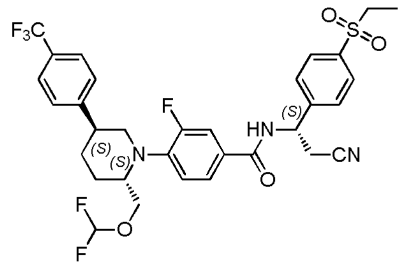

(118),

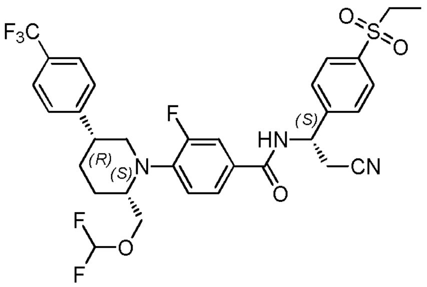

(119),

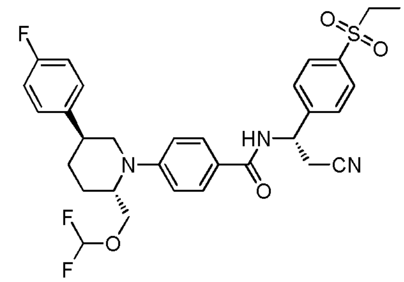

(120),

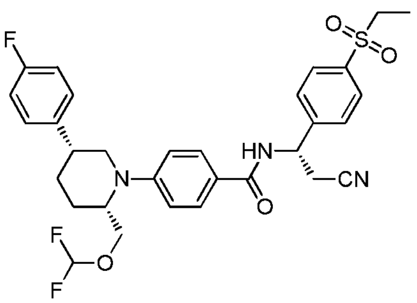

(121),

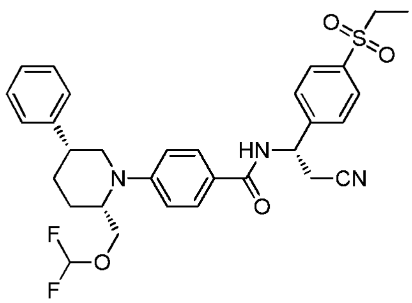

(122),

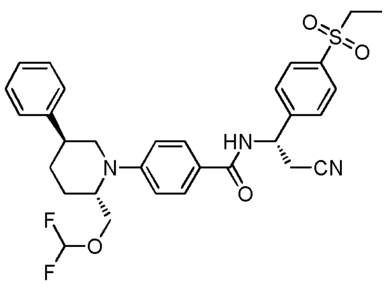

(123),

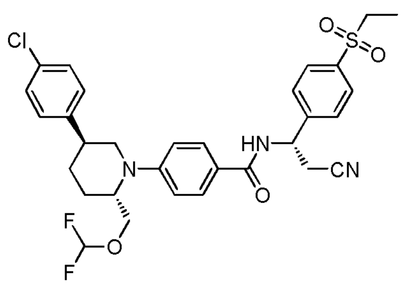

(124),

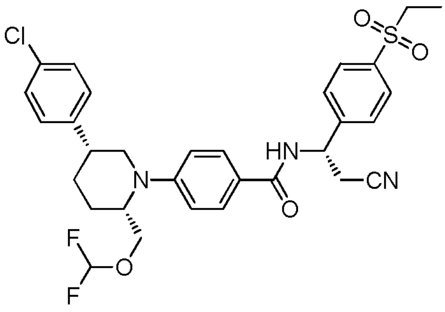

(125),

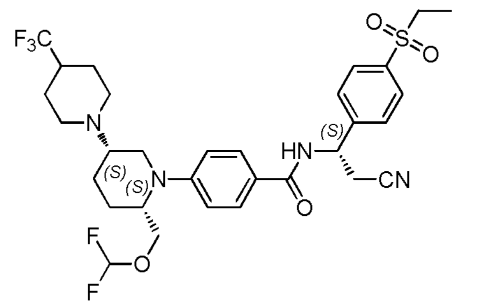

(126),

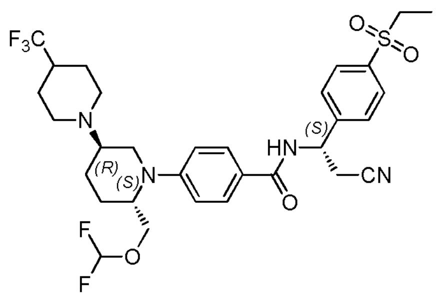

(127),

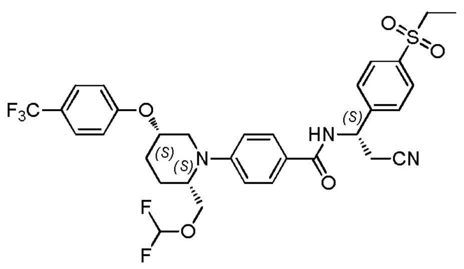

(128),

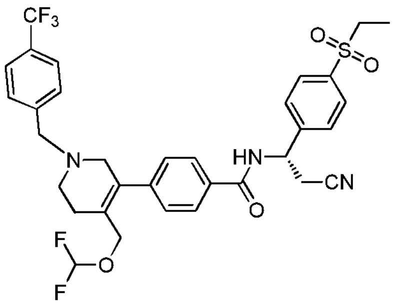

(129),

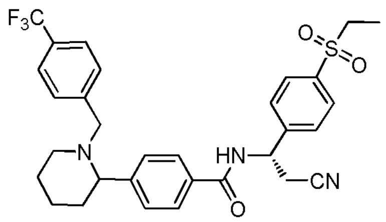

(130),

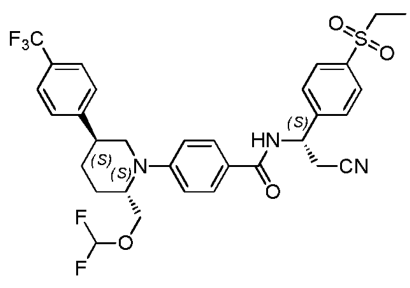

(131),

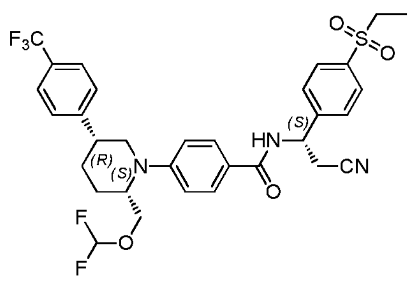

(132),

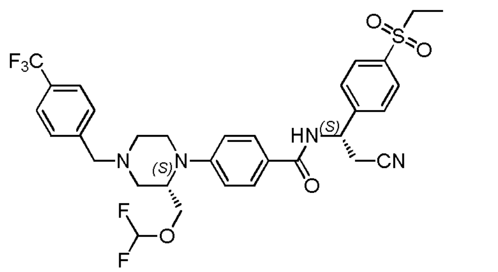
(133),

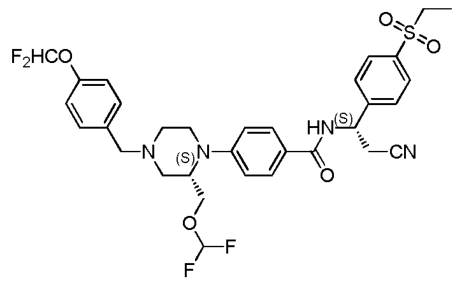
(134),

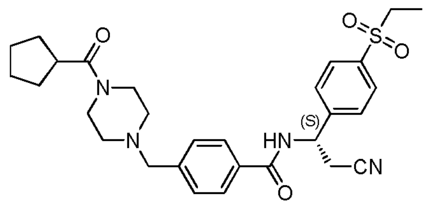
(135),

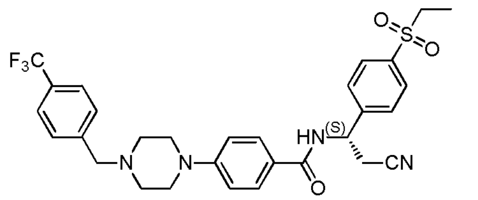
(136),

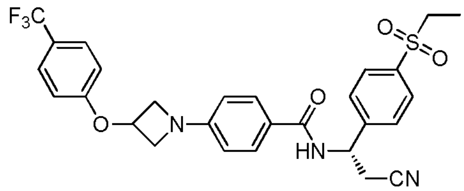
(137),

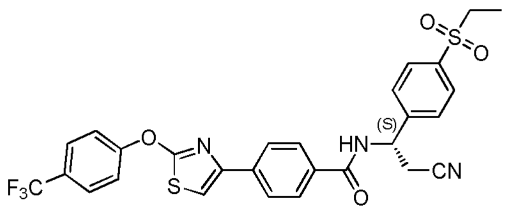
(138),

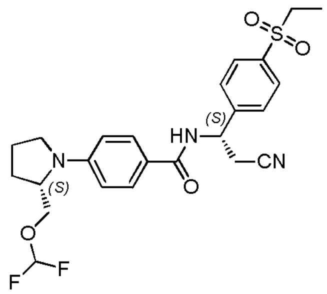
(139),

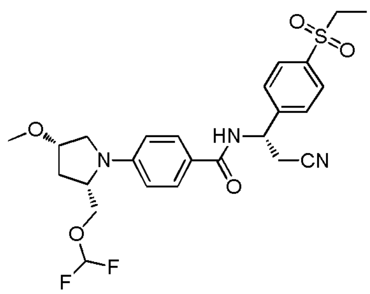
(140),

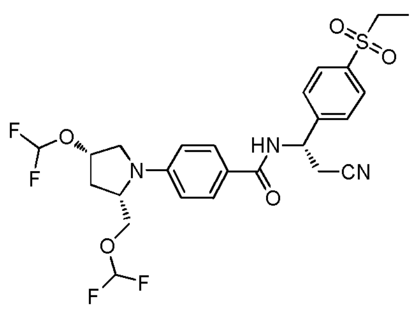
(141),

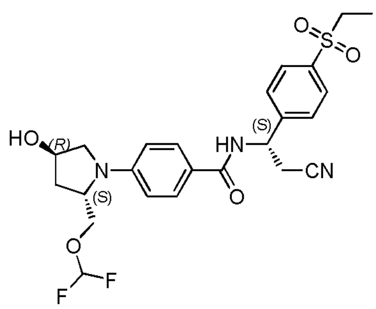
(142),

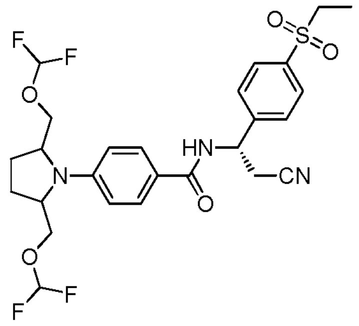
(143),

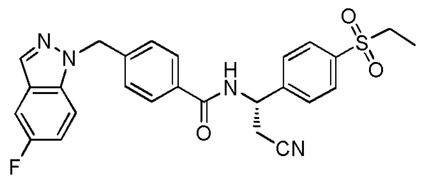
(144),

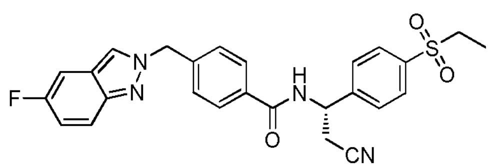
(145),

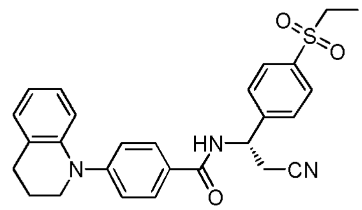
(146),

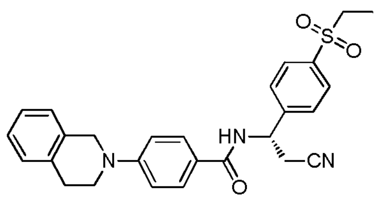
(147),

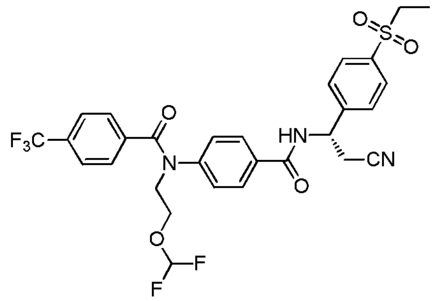
(148),

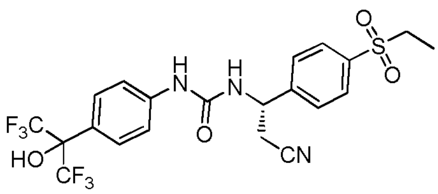
(149),

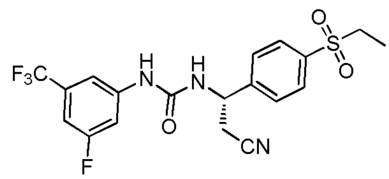
(150),

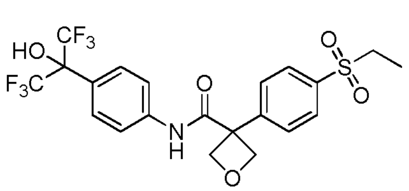
(151),

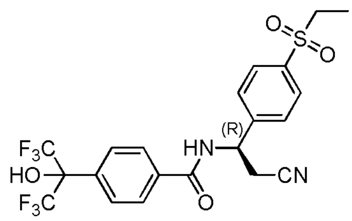
(152),

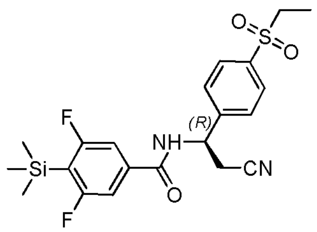
(153),

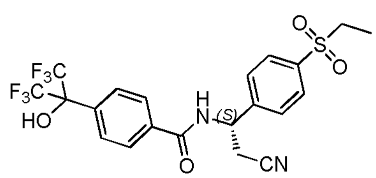
(154),

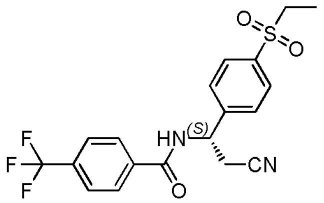 (155),

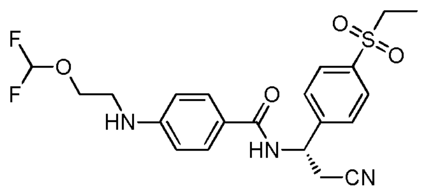 (156),

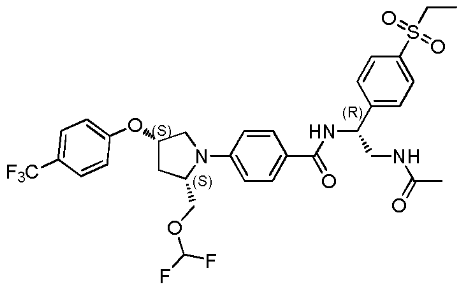 (157),

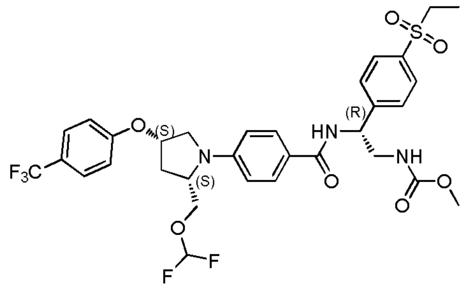 (158),

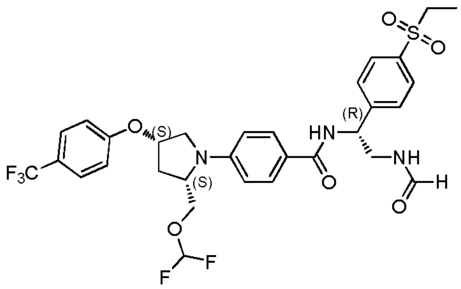 (159),

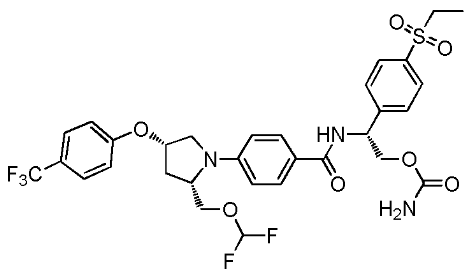 (160),

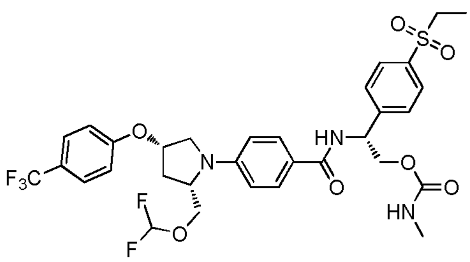 (161),

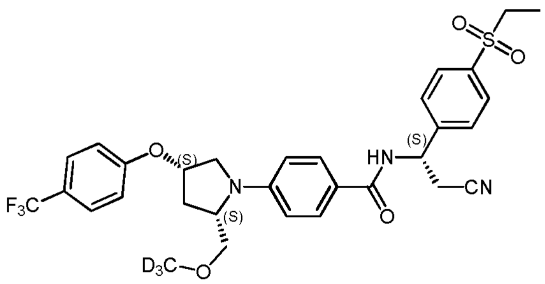 (162),

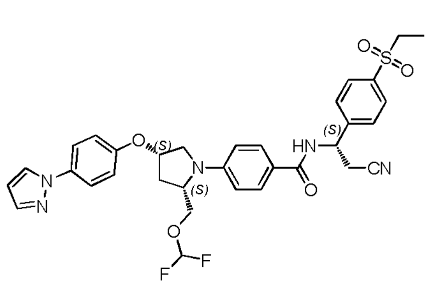 (163),

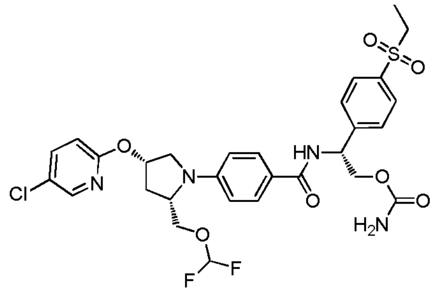 (164),

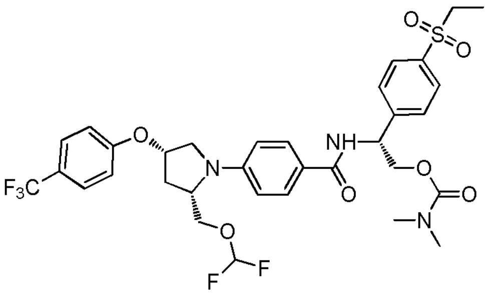

(165),

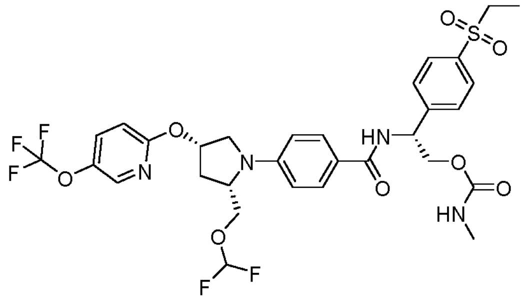

(166),

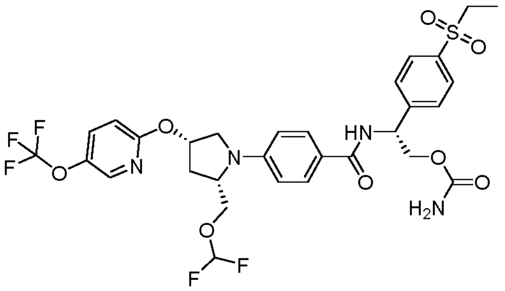

(167),

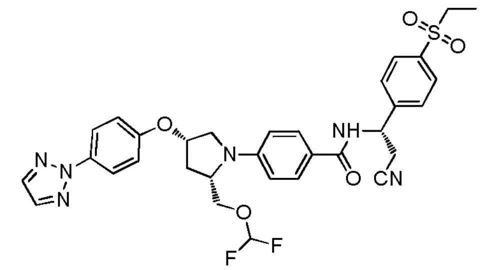

(168),

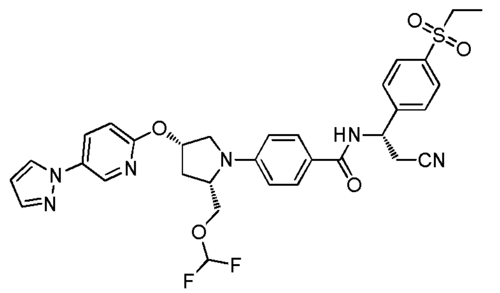

(169),

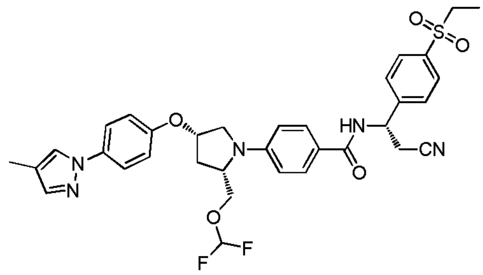

(170),

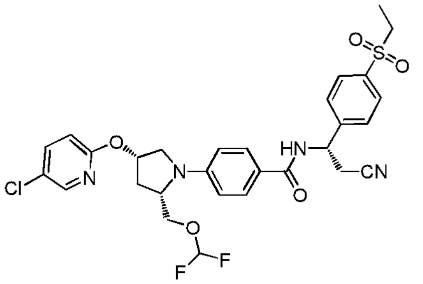

(171),

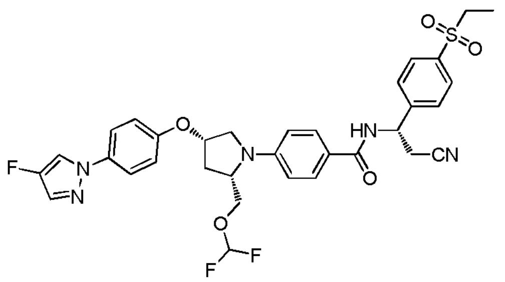

(172),

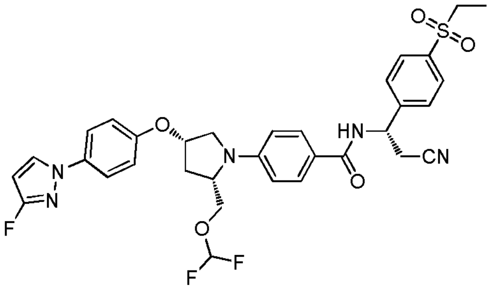

(173),

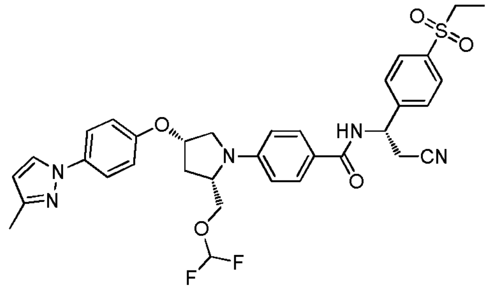

(174),

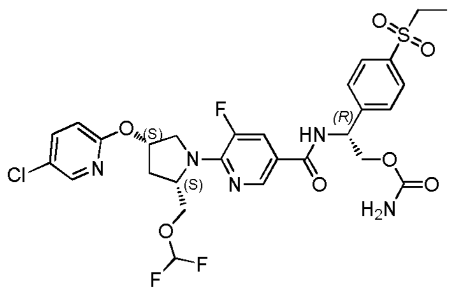

(175),

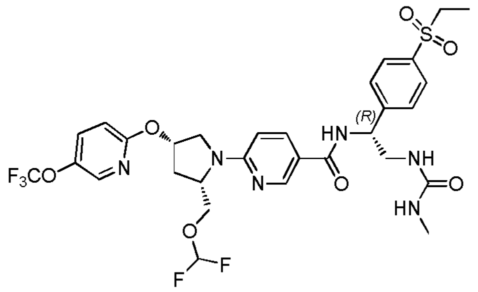

(176),

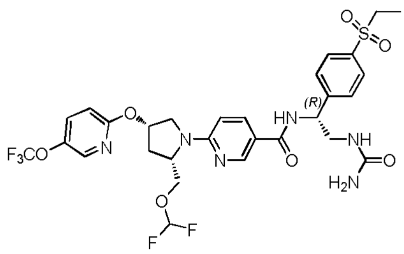

(177),

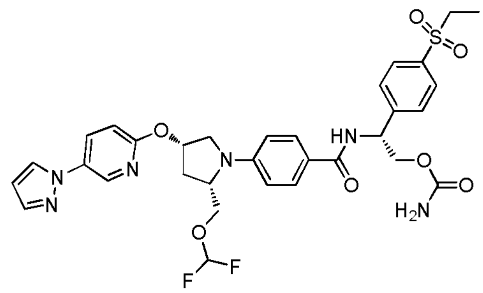

(178),

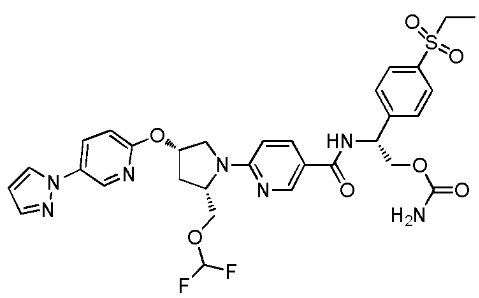

(179),

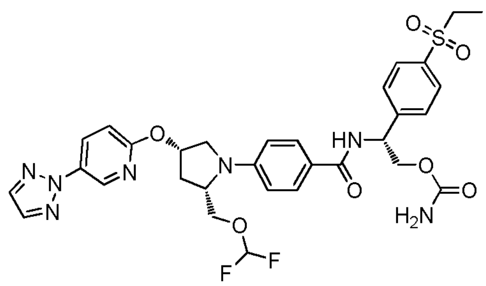

(180),

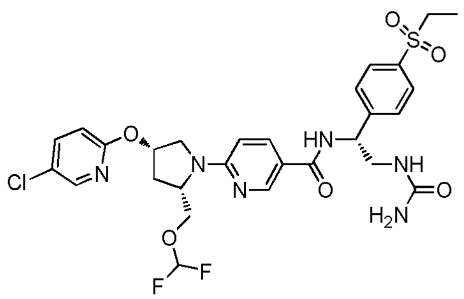

(181),

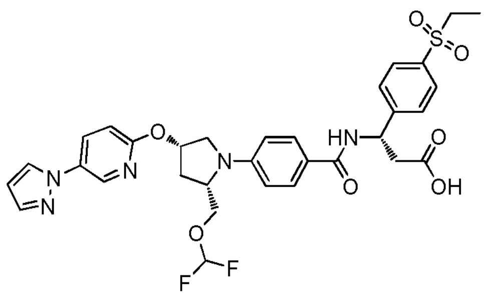

(182),

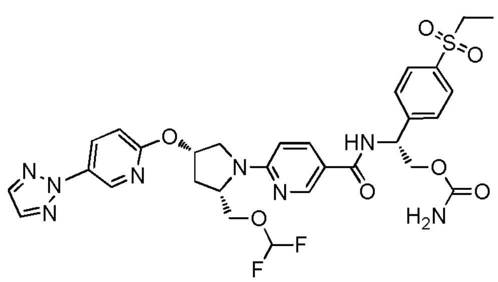

(183),

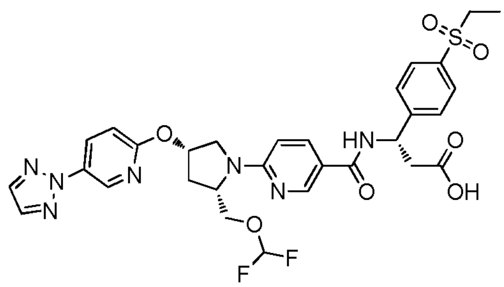

(184),

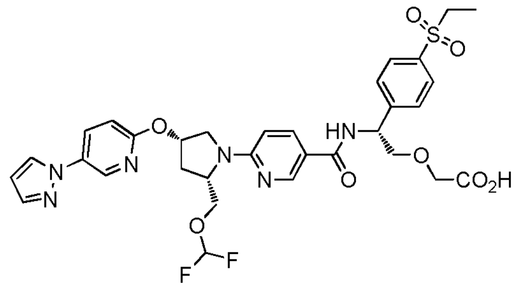 (185),

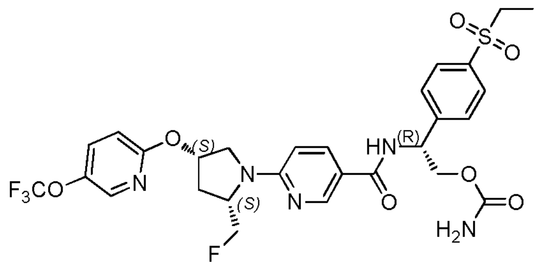 (186),

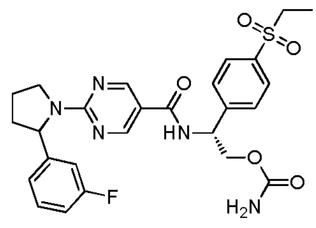 (187),

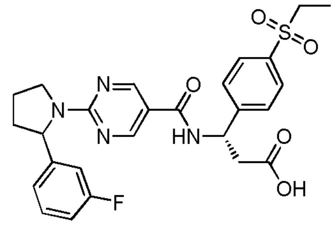 (188),

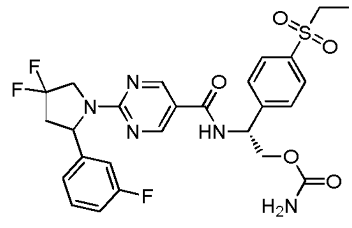 (189),

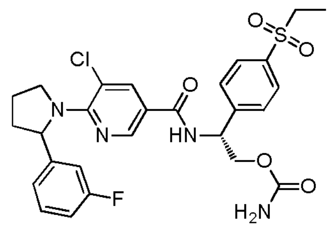 (190),

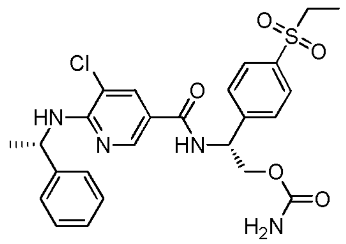 (191),

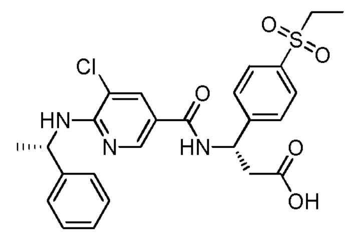 (192),

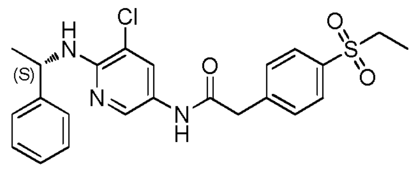 (193),

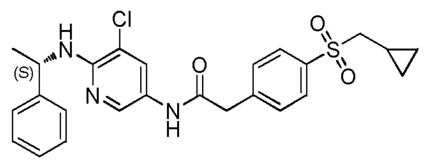 (194),

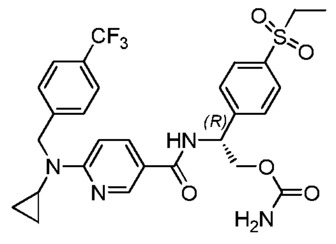 (195),

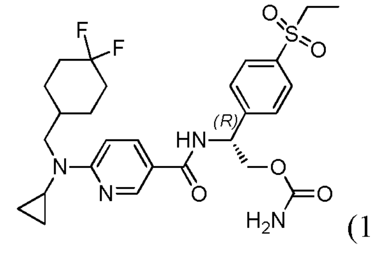 (196),

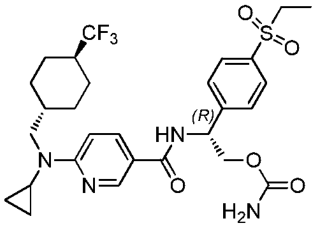 (197),

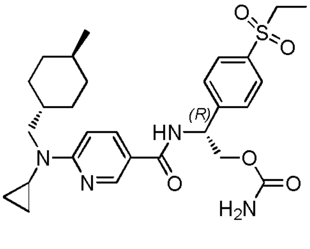 (198),

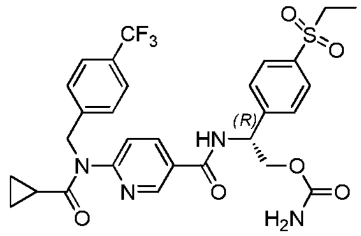

(199),

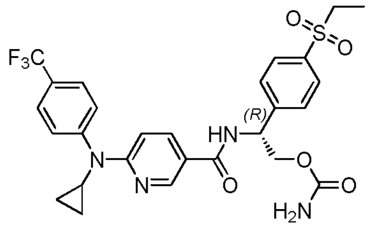

(200),

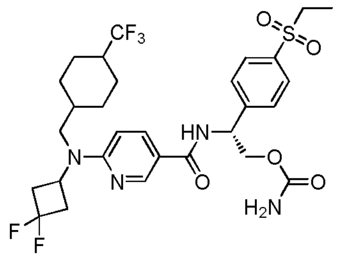

(201),

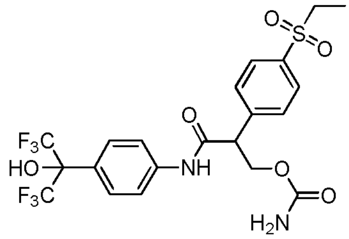

(202),

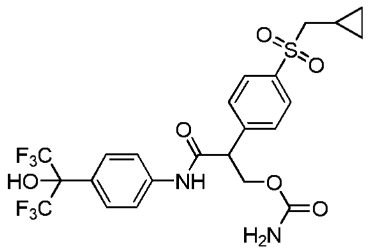

(203),

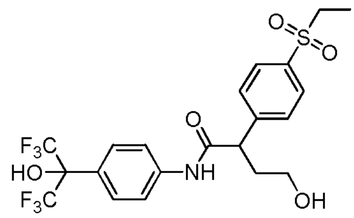

(204),

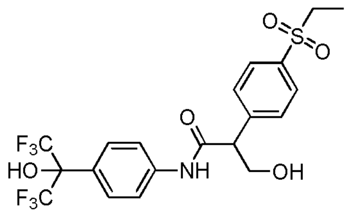

(205),

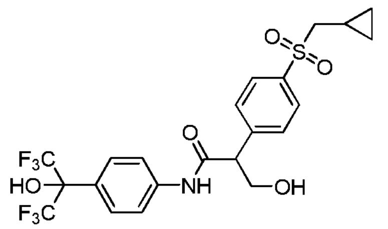

(206)

or

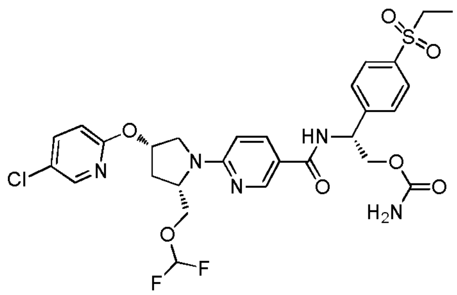

(207).

Unless otherwise specified, a compound having Formula (I), formula (II), formula (III), formula (IV) or formula (V), or a stereoisomer, a solvate, a metabolite, a pharmaceutically acceptable salt or a prodrug thereof are all embraced within the scope of the invention.

In another aspect, the present invention relates to a pharmaceutical composition comprising a compound having formula (I), formula (II), formula (III), formula (IV) or formula (V), or a stereoisomer, a geometric isomer, a tautomer, an *N*-oxide, a hydrate, a solvate, a metabolite, a pharmaceutically acceptable salt or a prodrug thereof, and a pharmaceutically acceptable excipient, a carrier, an adjuvant or a combination thereof.

In some embodiments, the pharmaceutical composition comprises other drugs for preventing or treating inflammatory syndromes, disorders or diseases or any combination thereof.

In some embodiments, the pharmaceutical composition can be in the form of a liquid, solid, semi-solid, gel or spray.

In another aspect, the present invention relates to use of the compound having formula (I), formula (II), formula (III), formula (IV) or formula (V), or a pharmaceutical composition thereof in the manufacture of a medicament for preventing or treating cancer, inflammation or autoimmune diseases mediated by RORγt in mammals, including humans.

In some embodiments, the present invention relates to use of the compound having formula (I), formula (II), formula (III), formula (IV) or formula (V), or a pharmaceutical composition thereof in the manufacture of a medicament for preventing or treating cancer, psoriasis, rheumatoid arthritis, systemic lupus erythematosus, multiple sclerosis, inflammatory bowel disease, colitis, ulcerative colitis, rheumatoid arthritis, autoimmune eye disease, ankylosing spondylitis, asthma, chronic obstructive pulmonary disease, osteoarthritis, allergic rhinitis, atopic dermatitis, Crohn's disease, or Kawasaki disease.

In another aspect, the present invention relates to a method of preparing, separating or purifying the compound having formula (I), formula (II), formula (III), formula (IV) or formula (V).

In another aspect, the present invention relates to intermediates for the preparation of compound having formula (I), formula (II), formula (III), formula (IV) or formula (V).

The compounds disclosed herein may contain asymmetric or chiral centers, and therefore exist in different stereoisomeric forms. It is intended that all stereoisomeric forms of the compound having formula (I), formula (II), formula (III), formula (IV) or formula (V) disclosed herein, including, but not limited to, diastereomers, enantiomers, atropisomers and geometric (or conformational) isomers, as well as mixtures thereof such as racemic mixtures, form part of the present invention.

In a structure disclosed herein, when the stereochemistry of any particular chiral atom is not specified, then all stereoisomers of that structure are contemplated within the present invention and are included in the present invention as compounds disclosed herein. When stereochemistry is indicated by a solid wedge or dashed line representing a particular configuration, then the stereoisomers of that structure are identified and defined.

Compound having formula (I), formula (II), formula (III), formula (IV) or formula (V) may exist in different tautomeric forms, and all such tautomers are included within the scope of the present invention.

Compound having formula (I), formula (II), formula (III), formula (IV) or formula (V) may exist in the form of salts. In one embodiment, the salt refers to a pharmaceutically acceptable salt. The phrase "pharmaceutically acceptable" refers to that the substance or composition must be chemically and/or toxicologically compatible with the other ingredients comprising a formulation, and/or the mammal being treated therewith. In another embodiment, the salt is not necessarily a pharmaceutically acceptable salt, but can be used for the preparation and/or purification of compound having formula (I), formula (II), formula (III), formula (IV) or formula (V) and/or can be used for the separation of intermediates of enantiomers of compound having formula (I), formula (II), formula (III), formula (IV) or formula (V).

Pharmaceutically acceptable acid addition salts can be formed by the action of the disclosed compounds with inorganic acids or organic acids, e.g., acetate, aspartate, benzoate, besylate, bromide/hydrobromide, bicarbonate/carbonate, bisulfate/sulfate, camphorsulfonate, chloride/hydrochloride, chlortheophyllonate, citrate, ethandisulfonate, fumarate, gluceptate, gluconate, glucuronate, hippurate, hydroiodide/iodide, isethionate, lactate, lactobionate, laurylsulfate, malate, maleate, malonate, mandelate, mesylate, methylsulphate, naphthoate, napsylate, nicotinate, nitrate, octadecanoate, oleate, oxalate, palmitate, pamoate, phosphate/hydrogen phosphate/dihydrogen phosphate, polygalacturonate, propionate, stearate, succinate, subsalicylate, tartrate, tosylate and trifluoroacetate salts.

Pharmaceutically acceptable base addition salts can be formed by the action of the disclosed compounds with inorganic or organic bases.

Inorganic bases from which salts can be derived include, for example, ammonium salts and metals from columns I to XII of the periodic table. In certain embodiments, the salts are derived from sodium, potassium, ammonium, calcium, magnesium, iron, silver, zinc, and copper; particularly suitable salts include ammonium, potassium, sodium, calcium and magnesium salts.

Organic bases from which salts can be derived include, for example, primary, secondary, and tertiary amines, substituted amines including naturally occurring substituted amines, cyclic amines, basic ion exchange resins, and the like. Certain organic amines include isopropylamine, benzathine, cholinate, diethanolamine, diethylamine, lysine, meglumine, piperazine and tromethamine.

The pharmaceutically acceptable salts of the present invention can be synthesized from a basic or acidic moiety, by conventional chemical methods. Generally, such salts can be prepared by reacting free acid forms of these compounds with a stoichiometric amount of the appropriate base (such as Na, Ca, Mg, or K hydroxide, carbonate, bicarbonate or the like), or by reacting free base forms of these compounds with a stoichiometric amount of the appropriate acid. Such reactions are typically carried out in water or in an organic solvent, or in a mixture of the two. Generally, use of non-aqueous media like ether, ethyl acetate, ethanol, isopropanol, or acetonitrile is desirable, where practicable. Lists of

additional suitable salts can be found, *e.g.,* in "Remington's Pharmaceutical Sciences", 20th ed., Mack Publishing Company, Easton, Pa., (1985); and in "Handbook of Pharmaceutical Salts: Properties, Selection, and Use" by Stahl and Wermuth (Wiley-VCH, Weinheim, Germany, 2002).

Furthermore, the compounds disclosed herein, including their salts, can also be obtained in the form of their hydrates, or include other solvents such as ethanol, DMSO, and the like, used for their crystallization. The compounds of the present invention may inherently or by design form solvates with pharmaceutically acceptable solvents (including water); therefore, it is intended that the invention embrace both solvated and unsolvated forms of the compounds disclosed herein.

Any formula given herein is also intended to represent isotopically unenriched forms as well as isotopically enriched forms of the compounds. Isotopically enriched compounds have the structure depicted by the general formula given herein, except that one or more atoms are replaced by the atom having a selected atomic mass or mass number. Examples of isotopes that can be incorporated into compounds of the invention include isotopes of hydrogen, carbon, nitrogen, oxygen, phosphorous, sulfur, fluorine, and chlorine, such as $^{2}H$ (deuterium, D), $^{3}H$, $^{11}C$, $^{13}C$, $^{14}C$, $^{15}N$, $^{17}O$, $^{18}O$, $^{18}F$, $^{31}P$, $^{32}P$, $^{35}S$, $^{36}Cl$, $^{125}I$, respectively.

In another aspect, the compounds of the invention include isotopically enriched compounds as defined herein, for example those into which radioactive isotopes, such as $^{3}H$, $^{14}C$ and $^{18}F$, or those into which non-radioactive isotopes, such as $^{2}H$ and $^{13}C$ are present. Such isotopically enriched compounds are useful in metabolic studies (with $^{14}C$), reaction kinetic studies (with, for example $^{2}H$ or $^{3}H$), detection or imaging techniques, such as positron emission tomography (PET) or single-photon emission computed tomography (SPECT) including drug or substrate tissue distribution assays, or in radioactive treatment of patients. In particular, an $^{18}F$-enriched compound may be particularly desirable for PET or SPECT studies. Isotopically-enriched compound having formula (I) can generally be prepared by conventional techniques known to those skilled in the art or by processes analogous to those described in the accompanying Examples and Preparations using an appropriate isotopically-labeled reagent in place of the non-labeled reagent previously employed.

Further, substitution with heavier isotopes, particularly deuterium (*i.e.*,$^{2}H$ or D) may afford certain therapeutic advantages resulting from greater metabolic stability. For example increased *in vivo* half-life or reduced dosage requirements or an improvement in therapeutic index. It is understood that deuterium in this context is regarded as a substituent of a compound having formula (I). The concentration of such a heavier isotope, specifically deuterium, may be defined by the isotopic enrichment factor. The term "isotopic enrichment factor" as used herein means the ratio between the isotopic abundance and the natural abundance of a specified isotope. If a substituent in a compound of this invention is denoted deuterium, such compound has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation). Pharmaceutically acceptable solvates in accordance with the invention include those wherein the solvent of crystallization may be isotopically substituted, *e.g.*, $D_2O$, $d_6$-acetone, DMSO-$d_6$.

## PHARMACEUTICAL COMPOSITION OF THE COMPOUND OF THE INVENTION AND PREPARATIONS AND ADMINISTRATION

The present invention provides a pharmaceutical composition comprising the compounds disclosed herein, such as those listed in the Examples; and pharmaceutically acceptable excipient, carrier, adjuvant and a combination thereof.

The present invention provides methods used in the treatment, prevention or improvement of diseases or symptoms thereof, comprising administrating to a patient a safe and effective combination drug containing a compound of the invention and one or more therapeutic active agents. Wherein, the combination drug includes one or more other drugs for preventing or treating inflammatory syndrome, disorder or disease, and the other drugs include but are not limited to:

1) TNF-$\alpha$ inhibitors; 2) non-selective COX-1/COX-2 inhibitors; 3) COX-2 inhibitors; 4) other therapeutic agents for the treatment of inflammatory syndromes and autoimmune diseases, including glucocorticoids, methotrexate, leflunomide, sulfasalazine, azathioprine, cyclosporine, tacrolimus, penicillamine, bucillamine, actarib, mizoribine, clobenzaprine, ciclesonide, hydroxychloroquine, aurothiomalate, auranofin, cyclophosphamide, BAFF/APRIL inhibitors, CTLA-4-immunoglobulin or analogs; 5) leukotriene biosynthesis inhibitors, 5-lipoxygenase inhibitors or 5-lipoxygenase-activated protein (FLAP) antagonists; 6) LTD4 receptor antagonists; 7) PDE4 inhibitors; 8) antihistamine HI receptor antagonists; $\alpha$1 and $\alpha$2-adrenoceptor agonists; 9) anticholinergics; 10) P-adrenergic receptor agonists; 11) insulin-like growth factor type I analogs 12) kinase inhibitors selected from Janus kinase inhibitors (JAK1 and/or JAK2 and/or JAK3 and/or TYK2), p38 MAPK and IKK2; 13) B cell targeting biological drugs such as rituximab; 14 ) selective costimulatory modulators such

as abatacept; 15) interleukin inhibitors selected from IL-1 inhibitors such as anakinra, IL-6 inhibitors such as tocilizumab and IL-12/IL-23 inhibitors such as ustekinumab.

The amount of the compound of the pharmaceutical composition disclosed herein refers to an amount which can be effectively detected to inhibit the retinoid-related orphan receptor γt of biology sample and patient. The dosage of active ingredient in the compositions of the present invention may vary, however, the amount of active ingredient must be such that an appropriate dosage form can be obtained. The active ingredient can be administered to patients (animals and humans) in need of such treatment in doses that provide optimal drug efficacy. The dose chosen will depend on the desired therapeutic effect, on the route of administration and on the duration of treatment. Dosages will vary from patient to patient, depending on the nature and severity of the disease, the weight of the patient, the patient's specific diet, concomitant medications, and other factors that will be recognized by those skilled in the art. In one embodiment, the dose ranges from about 0.5 mg to 500 mg per patient per day; in another embodiment, the dose ranges from about 0.5 mg to 200 mg per patient per day.

It will also be appreciated that certain of the compounds of present invention can exist in free form for treatment, or where appropriate, as a pharmaceutically acceptable derivative or a prodrug thereof. A pharmaceutically acceptable derivative includes pharmaceutically acceptable prodrugs, salts, esters, salts of such esters, or any other adduct or derivative which upon administration to a patient in need thereof is capable of providing, directly or indirectly, a compound as otherwise described herein, or a metabolite or residue thereof.

The medicament or pharmaceutical compositions of the invention may be prepared and packaged in bulk form wherein a safe and effective amount of a compound having formula (I), formula (II), formula (III), formula (IV) or formula (V) disclosed herein can be extracted and then given to the patient, such as with powders or syrups. Typically, patients are administered at dose levels between 0.0001 and 10 mg/kg body weight daily to obtain potent inhibition of the retinoid-related orphan receptor γt. Alternatively, the pharmaceutical compositions of the invention may be prepared and packaged in unit dosage form wherein each physically discrete unit contains a safe and effective amount of a compound having formula (I), formula (II), formula (III), formula (IV) or formula (V) disclosed herein. When prepared in unit dosage form, the pharmaceutical compositions disclosed herein may generally contain an effective dose of a compound disclosed herein.

When the pharmaceutical composition of the present invention contains one or more other active ingredients in addition to the compound of the present invention, the compound weight ratio of the compound of the present invention to the second active ingredient may vary and will depend on the effective dose of each ingredient. Typically, an effective dose of each is used. Thus, for example, when a compound of the present invention is mixed with another agent, the weight ratio of the compound of the present invention to the other agent will generally range from about 1000:1 to about 1:1000, such as about 200:1 to about 1:200. Mixtures of compounds of the present invention with other active ingredients are generally also within the above ranges, but in each case an effective dose of each active ingredient should be used.

"Pharmaceutically acceptable excipient" as used herein means a pharmaceutically acceptable material, composition or vehicle involved in giving form or consistency to the pharmaceutical composition. Each excipient must be compatible with the other ingredients of the pharmaceutical composition when commingled, such that interactions which would substantially reduce the efficacy of the compound of the invention when administered to a patient and would result in pharmaceutically unacceptable compositions are avoided. In addition, each excipient must of course be of sufficiently high purity to render it is pharmaceutically acceptable.

Suitable pharmaceutically acceptable excipients will vary depending upon the particular dosage form chosen. In addition, suitable pharmaceutically acceptable excipients may be chosen for a particular function that they may serve in the composition. For example, certain pharmaceutically acceptable excipients may be chosen for their ability to facilitate the production of uniform dosage forms. Certain pharmaceutically acceptable excipients may be chosen for their ability to facilitate the production of stable dosage forms. Certain pharmaceutically acceptable excipients may be chosen for their ability to facilitate the carrying or transporting the compound of the present invention once administered to the patient from one organ, or portion of the body, to another organ, or portion of the body. Certain pharmaceutically acceptable excipients may be chosen for their ability to enhance patient compliance.

Suitable pharmaceutically acceptable excipients include the following types of excipients: diluents, fillers, binders, disintegrants, lubricants, glidants, granulating agents, coating agents, wetting agents, solvents, co-solvents, suspending agents, emulsifiers, sweetners, flavoring agents, flavor masking agents, coloring agents, anticaking agents, humectants, chelating agents, plasticizers, viscosity increasing agents, antioxidants, preservatives, stabilizers, surfactants, and buffering agents. The skilled artisan will appreciate that certain pharmaceutically acceptable excipients may serve more than one function and may serve alternative functions depending on how much of the excipient is present in the formulation and what other ingredients are present in the formulation.

Skilled artisans possess the knowledge and skill in the art to enable them to select suitable pharmaceutically acceptable excipients in appropriate amounts for use in the invention. In addition, there are a number of resources that are available to the skilled artisan which describe pharmaceutically acceptable excipients and may be useful in selecting suitable pharmaceutically acceptable excipients. Examples include Remington's Pharmaceutical Sciences (Mack Publishing Company), The Handbook of Pharmaceutical Additives (Gower Publishing Limited), and The Handbook of Pharmaceutical Excipients (the American Pharmaceutical Association and the Pharmaceutical Press)

Various carriers used in formulating pharmaceutically acceptable compositions and known techniques for the preparation thereof are disclosed in Remington: The Science and Practice of Pharmacy, 21st edition, 2005, ed. D.B. Troy, Lippincott Williams & Wilkins, Philadelphia, and Encyclopedia of Pharmaceutical Technology, eds. J. Swarbrick and J. C. Boylan, 1988-1999, Marcel Dekker, New York, the contents of each of which are incorporated by reference herein. Except insofar as any conventional carrier medium is incompatible with the compounds of the invention, such as by producing any undesirable biological effect or otherwise interacting in a deleterious manner with any other component(s) of the pharmaceutically acceptable composition, its use is contemplated to be within the scope of this invention.

The pharmaceutical compositions of the invention are prepared using techniques and methods known to those skilled in the art. Some of the methods commonly used in the art are described in Remington's Pharmaceutical Sciences (Mack Publishing Company).

Therefore, another aspect of the present invention is related to a method for preparing a pharmaceutical composition, the pharmaceutical composition contains the compound disclosed herein and pharmaceutically acceptable excipient, carrier, adjuvant or a combination thereof, the method comprises mixing various ingredients. The pharmaceutical composition containing the compound disclosed herein can be prepared at for example environment temperature and under barometric pressure.

The compound of the invention will typically be formulated into a dosage form adapted for administration to the patient by the desired route of administration. For example, dosage forms include those adapted for (1) oral administration such as tablets, capsules, caplets, pills, troches, powders, syrups, elixers, suspensions, solutions, emulsions, sachets, and cachets; (2) parenteral administration such as sterile solutions, suspensions, and powders for reconstitution; (3) transdermal administration such as transdermal patches; (4) rectal administration such as suppositories; (5) inhalation such as aerosols, solutions, and dry powders; and (6) topical administration such as creams, ointments, lotions, solutions, pastes, sprays, foams, and gels.

In one embodiment, the compounds disclosed herein can be prepared to oral. In the other embodiment, the compounds disclosed herein can be prepared to inhalation. In the still other embodiment, the compounds disclosed herein can be prepared to nasal administration. In the yet other embodiment, the compounds disclosed herein can be prepared to transdermal administration. In the still yet other embodiments, the compounds disclosed herein can be prepared to topical administration.

The pharmaceutical compositions provided herein may be provided as compressed tablets, tablet triturates, chewable lozenges, rapidly dissolving tablets, multiple compressed tablets, or enteric-coating tablets, sugar-coated, or film-coated tablets. Enteric-coated tablets are compressed tablets coated with substances that resist the action of stomach acid but dissolve or disintegrate in the intestine, thus protecting the active ingredients from the acidic environment of the stomach. Enteric-coatings include, but are not limited to, fatty acids, fats, phenylsalicylate, waxes, shellac, ammoniated shellac, and cellulose acetate phthalates. Sugar-coated tablets are compressed tablets surrounded by a sugar coating, which may be beneficial in covering up objectionable tastes or odors and in protecting the tablets from oxidation. Film-coated tablets are compressed tablets that are covered with a thin layer or film of a water-soluble material. Film coatings include, but are not limited to, hydroxyethylcellulose, sodium carboxymethylcellulose, polyethylene glycol 4000, and cellulose acetate phthalate. Film coating imparts the same general characteristics as sugar coating. Multiple compressed tablets are compressed tablets made by more than one compression cycle, including layered tablets, and press-coated or dry-coated tablets.

The tablet dosage forms may be prepared from the active ingredient in powdered, crystalline, or granular forms, alone or in combination with one or more carriers or excipients described herein, including binders, disintegrants, controlled-release polymers, lubricants, diluents, and/or colorants. Flavoring and sweetening agents are especially useful in the formation of chewable tablets and lozenges.

The pharmaceutical compositions provided herein may be provided as soft or hard capsules, which can be made from gelatin, methylcellulose, starch, or calcium alginate. The hard gelatin capsule, also known as the dry-filled capsule (DFC), consists of two sections, one slipping over the other, thus completely enclosing the active ingredient. The soft elastic capsule (SEC) is a soft, globular shell, such as a gelatin shell, which is plasticized by the addition of glycerin, sorbitol, or a similar polyol. The soft gelatin shells may contain a preservative to prevent the growth of microorganisms. Suitable preservatives are those as described herein, including methyl- and propyl-parabens, and sorbic acid. The liquid, semisolid, and solid dosage forms provided herein may be encapsulated in a capsule. Suitable liquid and semisolid dosage forms include solutions and suspensions in propylene carbonate, vegetable oils, or triglycerides. Capsules containing such solutions can be prepared as described in U.S. Pat. Nos. 4,328,245; 4,409,239; and 4,410,545. The capsules may also be coated as known by those of skill in the art in order to modify or sustain dissolution of the active ingredient.

The pharmaceutical compositions provided herein may be provided in liquid and semisolid dosage forms, including emulsions, solutions, suspensions, elixirs, and syrups. An emulsion is a two-phase system, in which one liquid

is dispersed in the form of small globules throughout another liquid, which can be oil-in-water or water-in-oil. Emulsions may include a pharmaceutically acceptable non-aqueous liquids or solvent, emulsifying agent, and preservative. Suspensions may include a pharmaceutically acceptable suspending agent and preservative. Aqueous alcoholic solutions may include a pharmaceutically acceptable acetal, such as a di(lower alkyl) acetal of a lower alkyl aldehyde, e.g., acetaldehyde diethyl acetal; and a water-miscible solvent having one or more hydroxy groups, such as propylene glycol and ethanol. Elixirs are clear, sweetened, and hydroalcoholic solutions. Syrups are concentrated aqueous solutions of a sugar, for example, sucrose, and may also contain a preservative. For a liquid dosage form, for example, a solution in a polyethylene glycol may be diluted with a sufficient quantity of a pharmaceutically acceptable liquid carrier, e.g., water, to be measured conveniently for administration.

Other useful liquid and semisolid dosage forms include, but are not limited to, those containing the active ingredient(s) provided herein, and a dialkylated mono- or poly-alkylene glycol, including, 1,2-dimethoxymethane, diglyme, triglyme, tetraglyme, polyethylene glycol-350-dimethyl ether, polyethylene glycol-550-dimethyl ether, polyethylene glycol-750-dimethyl ether, wherein 350, 550, and 750 refer to the approximate average molecular weight of the polyethylene glycol. These formulations may further comprise one or more antioxidants, such as butylated hydroxytoluene (BHT), butylated hydroxyanisole (BHA), propyl gallate, vitamin E, hydroquinone, hydroxycoumarins, ethanolamine, lecithin, cephalin, ascorbic acid, malic acid, sorbitol, phosphoric acid, bisulfite, sodium metabisulfite, thiodipropionic acid and its esters, and dithiocarbamates.

Where appropriate, dosage unit formulations for oral administration can be microencapsulated. The formulation can also be prepared to prolong or sustain the release as for example by coating or embedding particulate material in polymers, wax, or the like.

The pharmaceutical compositions provided herein for oral administration may be also provided in the forms of liposomes, micelles, microspheres, or nanosystems. Miccellar dosage forms can be prepared as described in U.S. Pat. No. 6,350,458.

The pharmaceutical compositions provided herein may be provided as non-effervescent or effervescent, granules and powders, to be reconstituted into a liquid dosage form. Pharmaceutically acceptable carriers and excipients used in the non-effervescent granules or powders may include diluents, sweeteners, and wetting agents. Pharmaceutically acceptable carriers and excipients used in the effervescent granules or powders may include organic acids and a source of carbon dioxide.

Coloring and flavoring agents can be used in all of the above dosage forms.

The compounds disclosed herein can also be coupled to soluble polymers as targeted medicament carriers. Such polymers may encompass polyvinylpyrrolidone, pyran copolymer, polyhydroxypropylmethacrylamidophenol, polyhydroxyethylaspartamidophenol or polyethylene oxide polylysine, substituted by palmitoyl radicals. The compounds may furthermore be coupled to a class of biodegradable polymers which are suitable for achieving controlled release of a medicament, for example polylactic acid, poly-epsilon-caprolactone, polyhydroxybutyric acid, polyorthoesters, polyacetals, polydihydroxypyrans, polycyanoacrylates and crosslinked or amphipathic block copolymers of hydrogels.

The pharmaceutical compositions provided herein may be formulated as immediate or modified release dosage forms, including delayed, sustained, pulsed, controlled, targeted, and programmed-release forms.

The pharmaceutical compositions provided herein may be co-formulated with other active ingredients which do not impair the desired therapeutic action, or with substances that supplement the desired action.

The pharmaceutical compositions provided herein may be administered parenterally by injection, infusion, or implantation, for local or systemic administration. Parenteral administration, as used herein, include intravenous, intraarterial, intraperitoneal, intrathecal, intraventricular, intraurethral, intrasternal, intracranial, intramuscular, intrasynovial, and subcutaneous administration.

The pharmaceutical compositions provided herein may be formulated in any dosage forms that are suitable for parenteral administration, including solutions, suspensions, emulsions, micelles, liposomes, microspheres, nanosystems, and solid forms suitable for solutions or suspensions in liquid prior to injection. Such dosage forms can be prepared according to conventional methods known to those skilled in the art of pharmaceutical science (see, Remington: The Science and Practice of Pharmacy, supra).

The pharmaceutical compositions intended for parenteral administration may include one or more pharmaceutically acceptable carriers and excipients, including, but not limited to, aqueous vehicles, water-miscible vehicles, nonaqueous vehicles, antimicrobial agents or preservatives against the growth of microorganisms, stabilizers, solubility enhancers, isotonic agents, buffering agents, antioxidants, local anesthetics, suspending and dispersing agents, wetting or emulsifying agents, complexing agents, sequestering or chelating agents, cryoprotectants, lyoprotectants, thickening agents, pH adjusting agents, and inert gases.

Suitable aqueous vehicles include, but are not limited to, water, saline, physiological saline or phosphate buffered saline (PBS), sodium chloride injection, Ringers injection, isotonic dextrose injection, sterile water injection, dextrose and lactated Ringers injection. Non-aqueous vehicles include, but are not limited to, fixed oils of vegetable origin, castor oil, corn oil, cottonseed oil, olive oil, peanut oil, peppermint oil, safflower oil, sesame oil, soybean oil,

hydrogenated vegetable oils, hydrogenated soybean oil, and medium-chain triglycerides of coconut oil, and palm seed oil. Water-miscible vehicles include, but are not limited to, ethanol, 1,3-butanediol, liquid polyethylene glycol (*e.g.*, polyethylene glycol 300 and polyethylene glycol 400), propylene glycol, glycerin, *N*-methyl-2-pyrrolidone, *N*,*N*-dimethylacetamide, and dimethyl sulfoxide.

Suitable antimicrobial agents or preservatives include, but are not limited to, phenols, cresols, mercurials, benzyl alcohol, chlorobutanol, methyl and propyl p-hydroxybenzoates, thimerosal, benzalkonium chloride (*e.g.*, benzethonium chloride), methyl- and propyl-parabens, and sorbic acid. Suitable isotonic agents include, but are not limited to, sodium chloride, glycerin, and dextrose. Suitable buffering agents include, but are not limited to, phosphate and citrate. Suitable antioxidants are those as described herein, including bisulfite and sodium metabisulfite. Suitable local anesthetics include, but are not limited to, procaine hydrochloride. Suitable suspending and dispersing agents are those as described herein, including sodium carboxymethylcelluose, hydroxypropyl methylcellulose, and polyvinylpyrrolidone. Suitable emulsifying agents include those described herein, including polyoxyethylene sorbitan monolaurate, polyoxyethylene sorbitan monooleate 80, and triethanolamine oleate. Suitable sequestering or chelating agents include, but are not limited to EDTA. Suitable pH adjusting agents include, but are not limited to, sodium hydroxide, hydrochloric acid, citric acid, and lactic acid. Suitable complexing agents include, but are not limited to, cyclodextrins, including $\alpha$-cyclodextrin, $\beta$-cyclodextrin, hydroxypropyl-$\beta$-cyclodextrin, sulfobutylether-$\beta$-cyclodextrin, and sulfobutylether 7-$\beta$-cyclodextrin.

The pharmaceutical compositions provided herein may be formulated for single or multiple dosage administration. The single dosage formulations are packaged in an ampoule, a vial, or a syringe. The multiple dosage parenteral formulations must contain an antimicrobial agent at bacteriostatic or fungistatic concentrations. All parenteral formulations must be sterile, as known and practiced in the art.

In one embodiment, the pharmaceutical compositions are provided as ready-to-use sterile solutions. In another embodiment, the pharmaceutical compositions are provided as sterile dry soluble products, including lyophilized powders and hypodermic tablets, to be reconstituted with a vehicle prior to use. In yet another embodiment, the pharmaceutical compositions are provided as ready-to-use sterile suspensions. In yet another embodiment, the pharmaceutical compositions are provided as sterile dry insoluble products to be reconstituted with a vehicle prior to use. In still another embodiment, the pharmaceutical compositions are provided as ready-to-use sterile emulsions.

The pharmaceutical compositions may be formulated as a suspension, solid, semi-solid, or thixotropic liquid, for administration as an implanted depot. In one embodiment, the pharmaceutical compositions provided herein are dispersed in a solid inner matrix, which is surrounded by an outer polymeric membrane that is insoluble in body fluids but allows the active ingredient in the pharmaceutical compositions diffuse through.

Suitable inner matrixes include polymethylmethacrylate, polybutyl-methacrylate, plasticized or unplasticized polyvinylchloride, plasticized nylon, plasticized polyethylene terephthalate, natural rubber, polyisoprene, polyisobutylene, polybutadiene, polyethylene, ethylene-vinyl acetate copolymers, silicone rubbers, polydimethylsiloxanes, silicone carbonate copolymers, hydrophilic polymers, such as hydrogels of esters of acrylic and methacrylic acid, collagen, cross-linked polyvinyl alcohol, and cross-linked partially hydrolyzed polyvinyl acetate.

Suitable outer polymeric membranes include polyethylene, polypropylene, ethylene/propylene copolymers, ethylene/ethyl acrylate copolymers, ethylene/vinyl acetate copolymers, silicone rubbers, polydimethyl siloxanes, neoprene rubber, chlorinated polyethylene, polyvinylchloride, vinyl chloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer.

In another aspect, the pharmaceutical composition of the invention is prepared to a dosage form adapted for administration to a patient by inhalation, for example as a dry powder, an aerosol, a suspension, or a solution composition. In one embodiment, the invention is directed to a dosage form adapted for administration to a patient by inhalation as a dry powder. In one embodiment, the invention is directed to a dosage form adapted for administration to a patient by inhalation as a dry powder. Dry powder compositions for delivery to the lung by inhalation typically comprise a compound disclosed herein or a pharmaceutically acceptable salt thereof as a finely divided powder together with one or more pharmaceutically-acceptable excipients as finely divided powders. Pharmaceutically-acceptable excipients particularly suited for use in dry powders are known to those skilled in the art and include lactose, starch, mannitol, and mono-, di-, and polysaccharides. The finely divided powder may be prepared by, for example, micronisation and milling. Generally, the size-reduced (e.g., micronised) compound can be defined by a $D_{50}$ value of about 1 to about 10 microns (for example as measured using laser diffraction).

Aerosols may be formed by suspending or dissolving a compound disclosed herein or a pharmaceutically acceptable salt thereof in a liquified propellant. Suitable propellants include halocarbons, hydrocarbons, and other liquified gases. Representative propellants include: trichlorofluoromethane (propellant 11), dichlorofluoromethane (propellant 12), dichlorotetrafluoroethane (propellant 114), tetrafluoroethane (HFA-134a), 1,1-difluoroethane (HFA-152a), difluoromethane (HFA-32), pentafluoroethane (HFA-12), heptafluoropropane (HFA-227a), perfluoropropane, perfluorobutane, perfluoropentane, butane, isobutane, and pentane. Aerosols comprising a compound of formula (I) or a pharmaceutically

acceptable salt thereof will typically be administered to a patient via a metered dose inhaler (MDI). Such devices are known to those skilled in the art.

The aerosol may contain additional pharmaceutically-acceptable excipients typically used with MDIs such as surfactants, lubricants, cosolvents and other excipients to improve the physical stability of the formulation, to improve valve performance, to improve solubility, or to improve taste.

Pharmaceutical compositions adapted for transdermal administration may be presented as discrete patches intended to remain in intimate contact with the epidermis of the patient for a prolonged period of time. For example, the active ingredient may be delivered from the patch by iontophoresis as generally described in Pharmaceutical Research, 318(1986), 318 (3).

Pharmaceutical compositions adapted for topical administration may be formulated as ointments, creams, suspensions, lotions, powders, solutions, pastes, gels, sprays, aerosols or oils. Ointments, creams and gels, may, for example, be formulated with an aqueous or oily base with the addition of suitable thickening and/or gelling agent and/or solvents. Such bases may thus, for example, include water and/or an oil such as liquid paraffin or a vegetable oil such as arachis oil or castor oil, or a solvent such as polyethylene glycol. Thickening agents and gelling agents which may be used according to the nature of the base include soft paraffin, aluminium stearate, cetostearyl alcohol, polyethylene glycols, woolfat, beeswax, carboxypolymethylene and cellulose derivatives, and/or glyceryl monostearate and/or non-ionic emulsifying agents.

Lotions may be formulated with an aqueous or oily base and will in general also contain one or more emulsifying agents, stabilising agents, dispersing agents, suspending agents or thickening agents.

Powders for external application may be formed with the aid of any suitable powder base, for example, talc, lactose or starch. Drops may be formulated with an aqueous or non-aqueous base also comprising one or more dispersing agents, solubilising agents, suspending agents or preservatives.

Topical preparations may be administered by one or more applications per day to the affected area; over skin areas occlusive dressings may advantageously be used. Continuous or prolonged delivery may be achieved by an adhesive reservoir system.

## USE OF THE COMPOUNDS AND PHARMACEUTICAL COMPOSITIONS

The compounds or pharmaceutical compositions disclosed in the present invention can be used in the manufacture of a medicament for treating, preventing, improving, controlling or alleviating cancer, inflammation or autoimmune diseases mediated by RORγt in mammals, including humans, and can also be used in the manufacture of other medicaments for inhibiting RORγt.

In particular, the amount of the compound in the composition of the present invention is effective to detectably inhibit RORγt, and the compound of the present invention can be used as a medicament for preventing or treating cancer, psoriasis, rheumatoid arthritis, systemic lupus erythematosus, multiple sclerosis, inflammatory bowel disease, colitis, ulcerative colitis, rheumatoid arthritis, autoimmune eye disease, ankylosing spondylitis, asthma, chronic obstructive pulmonary disease, osteoarthritis, allergic rhinitis, atopic dermatitis, Crohn's disease, or Kawasaki disease in humans.

The compounds or compositions of the present invention may be used, but in no way limited to, to prevent, treat or ameliorate cancer, psoriasis, rheumatoid arthritis, systemic lupus erythematosus, multiple sclerosis, inflammatory bowel disease, colitis, ulcerative colitis, rheumatoid arthritis, autoimmune eye disease, ankylosing spondylitis, asthma, chronic obstructive pulmonary disease, osteoarthritis, allergic rhinitis, atopic dermatitis, Crohn's disease, or Kawasaki disease in mammals, including humans, using an effective amount of the compounds or compositions of the present invention administered to a patient.

Besides being useful for human treatment, the compounds of the present invention or pharmaceutically compositions are also useful for veterinary treatment of animals such as companion animals, exotic animals and mammals in farm animals. In other embodiments, the animals disclosed herein include horses, dogs, and cats. As used herein, the compounds disclosed herein include the pharmaceutically acceptable derivatives thereof.

### General synthetic steps:

To describe the invention, the following examples are listed. However, it should be understood that the present invention is not limited to these embodiments, but merely provides a method for practicing the present invention.

Generally, the compounds disclosed herein may be prepared by methods described herein, wherein the substituents are as defined for formula (I), formula (II), formula (III), formula (IV) or formula (V) above, except where further noted. The following non-limiting schemes and examples are presented to further exemplify the invention.

Persons skilled in the art will recognize that the chemical reactions described may be readily adapted to prepare a number of other compounds disclosed herein, and alternative methods for preparing the compounds disclosed herein are deemed to be within the scope disclosed herein. For example, the synthesis of non-exemplified compounds according

to the invention may be successfully performed by modifications apparent to those skilled in the art, *e.g.,* by appropriately protecting interfering groups, by utilizing other suitable reagents known in the art other than those described, and/or by making routine modifications of reaction conditions. Alternatively, other reactions disclosed herein or known in the art will be recognized as having applicability for preparing other compounds disclosed herein.

In the examples described below, unless otherwise indicated all temperatures are set forth in degrees Celsius. Reagents were purchased from commercial suppliers such as Aldrich Chemical Company, Arco Chemical Company and Alfa Chemical Company, and were used without further purification unless otherwise indicated. Common solvents were purchased from commercial suppliers such as Shantou XiLong Chemical Factory, Guangdong Guanghua Reagent Chemical Factory Co. Ltd., Guangzhou Reagent Chemical Factory, Tianjin YuYu Fine Chemical Ltd., Tianjin Fuchen Chemical Reagent Factory, Wuhan Xinhuayuan Technology Development Co., Ltd., Qingdao Tenglong Reagent Chemical Ltd., and Qingdao Ocean Chemical Factory.

Anhydrous THF, dioxane, toluene, and ether were obtained by refluxing the solvent with sodium. Anhydrous $CH_2Cl_2$ and $CHCl_3$ were obtained by refluxing the solvent with $CaH_2$. EtOAc, PE, n-hexane, *N*,*N*-dimethylacetamide and DMF were treated with anhydrous $Na_2SO_4$ prior to use.

The reactions set forth below were done generally under a positive pressure of nitrogen or argon or with a drying tube (unless otherwise stated) in anhydrous solvents, and the reaction flasks were typically fitted with rubber septa for the introduction of substrates and reagents via syringe. Glassware was oven dried and/or heat dried.

Column chromatography was conducted using a silica gel column. Silica gel (300-400 mesh) was purchased from Qingdao Ocean Chemical Factory.

$^1$H NMR spectra were recorded by a Bruker 400 MHz spectrometer or Bruker 600 spectrometer, using $CDCl_3$, DMSO-$d_6$, $CD_3OD$ or acetone-$d_6$ (reported in ppm) as solvent, and using TMS (0 ppm) or chloroform (7.26 ppm) as the reference standard. When peak multiplicities were reported, the following abbreviations were used: s (singlet), d (doublet), t (triplet), m (multiplet), br (broadened), dd (doublet of doublets), and dt (doublet of triplets). Coupling constants, when given, were reported in Hertz (Hz).

The measurement conditions for low-resolution mass spectrometry (MS) data are: Agilent 6120 quadrupole HPLC-MS (column model: Zorbax SB-C18, 2.1 × 30 mm, 3.5 μm, 6 min, flow rate 0.6 mL / min. Mobile phase: 5%-95% ($CH_3CN$ with 0.1% formic acid) in ($H_2O$ with 0.1% formic acid) using electrospray ionization (ESI) at 210 nm / 254 nm with UV detection.

The purity of the compound was determined by high performance liquid chromatography (HPLC) using an Agilent 1260 HPLC (column model: Agilent zorbax Eclipse Plus C18) and detected by a DAD detector, and finally the area normalization method was used to calculate the compound purity.

The following abbreviations are used throughout the specification:

| | |
|---|---|
| AcOH | acetic acid; |
| $Boc_2O$ | Di-*tert*-butyl dicarbonate; |
| $Bu_4NBr$ | tetrabutylammonium bromide |
| CbzCl | benzyl chloroformate; |
| DCM | dichloromethane; |
| DMF | *N*,*N*-dimethylformamide; |
| DMP | dimethyl phthalate; |
| DMAP | 4-dimethylaminopyridine; |
| DIAD | diisopropyl azodicarboxylate; |
| DIPEA | *N*,*N*-diisopropylethylamine; |
| DMSO | dimethylsulfoxide; |
| DMSO-$d_6$ | dimethyl sulfoxide-$d_6$, deuterated dimethyl sulfoxide; |
| EA, EtOAc | ethyl acetate; |
| EtOH | ethyl alcohol; |
| EDCI | 1-(3-dimethylaminopropyl)-3-ethylcarbodiimide hydrochloride; |
| HATU | 2-(7-aza-1*H*-benzotriazole-1-yl)-1,1,3,3-tetramethyluronium hexafluorophosphate; |
| HOBt, HOBT | 1-hydroxybenzotriazole; |
| KOAc | potassium acetate; |
| LDA | lithium diisopropylamide; |
| MeCN, ACN | acetonitrile; |
| MeOH | methanol; |
| MeONa | sodium methoxide; |
| MsCl | methanesulfonyl chloride; |
| $Pd_2(dba)_3$ | tris(dibenzylideneacetone)dipalladium; |
| $Pd(OAc)_2$ | palladium diacetate; |

| | |
|---|---|
| PE | petroleum ether; |
| $Pd(PPh_3)_2Cl_2$ | bis(triphenylphosphine)palladium(II) chloride; |
| Pd/C | palladium on activated carbon; |
| $P(OPh_3)$ | triphenylphosphine oxide; |
| $PPh_3$ | triphenylphosphine; |
| $Pd(dppf)Cl_2$ | [1,1'-bis(diphenylphosphino)ferrocene]dichloropalladium(II); |
| *i*-PrOH, IPA | isopropyl alcohol; |
| Ruphos | 2-bicyclohexylphosphine-2',6'-diisopropoxybiphenyl; |
| STAB | Sodium triacetoxyborohydride; |
| *t*-BuOK | potassium *tert*-butoxide; |
| TEA | triethylamine; |
| $TMSCF_2Br$ | (bromo(difluoro)methyl)trimethylsilane; |
| $Tf_2O$ | trifluoromethanesulfonic anhydride; |
| TBSOTf | *tert*-butyldimethylsilyl trifluoromethanesulfonate; |
| TFAA | trifluoroacetic anhydride; |
| TLC | thin layer chromatography; |
| THF | tetrahydrofuran; |
| TFA | trifluoroacetic acid; |
| Xantphos | dimethylbisdiphenylphosphinoxanthene; |
| g | gram; |
| PTLC | preparative thin layer chromatography; |
| min | minute, minutes; |
| mmol | millimole; |
| M | mole per liter; |
| °C | Celsius; |
| mL, ml | milliliter; |
| rpm | revolution per minute; |
| Rt | retention time; |
| h | hour, hours; |

Typical synthetic steps for preparing the disclosed compounds of the present invention are shown in the following synthetic schemes. Rings A, $R_b$, $Z_1$, $Z_2$, $Z_3$, $Z_4$, $Z_5$, $Z_6$, $R_7$, $R_8$ and $R_9$ have the meanings as described herein, unless otherwise specified.

## Synthetic scheme 1

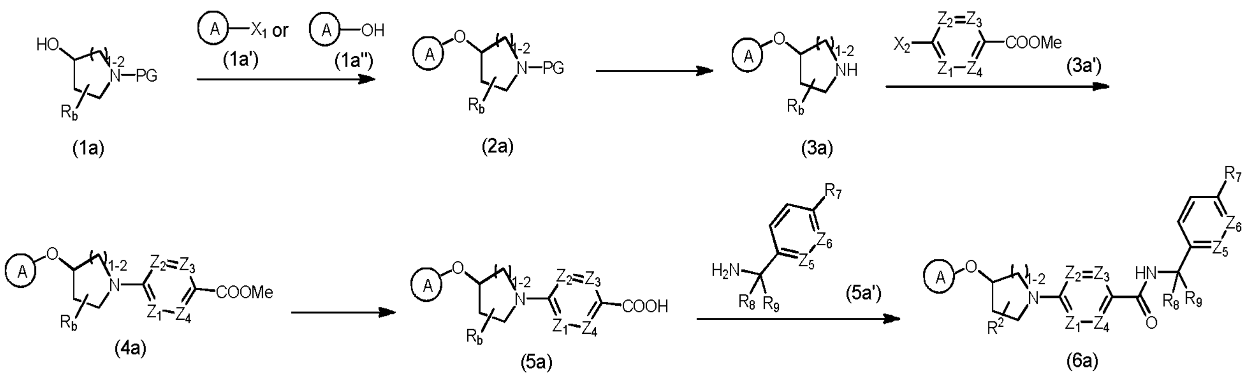


In the formula, each $X_1$ and $X_2$ represents a halogen atom, and PG represents an amino protecting group.

Compound (6a) can be prepared by the following procedure:

Compound (1a) can react with compound (1a') or compound (1a") to obtain compound (2a), compound (2a) can be deprotected to obtain compound (3a), which can be then subjected to coupling reaction with compound (3a') to obtain compound (4a), compound (4a) can be subjected to hydrolysis reaction to obtain compound (5a), and compound (5a) can be condensed with compound (5a') to obtain compound (6a).

## Synthetic scheme 2

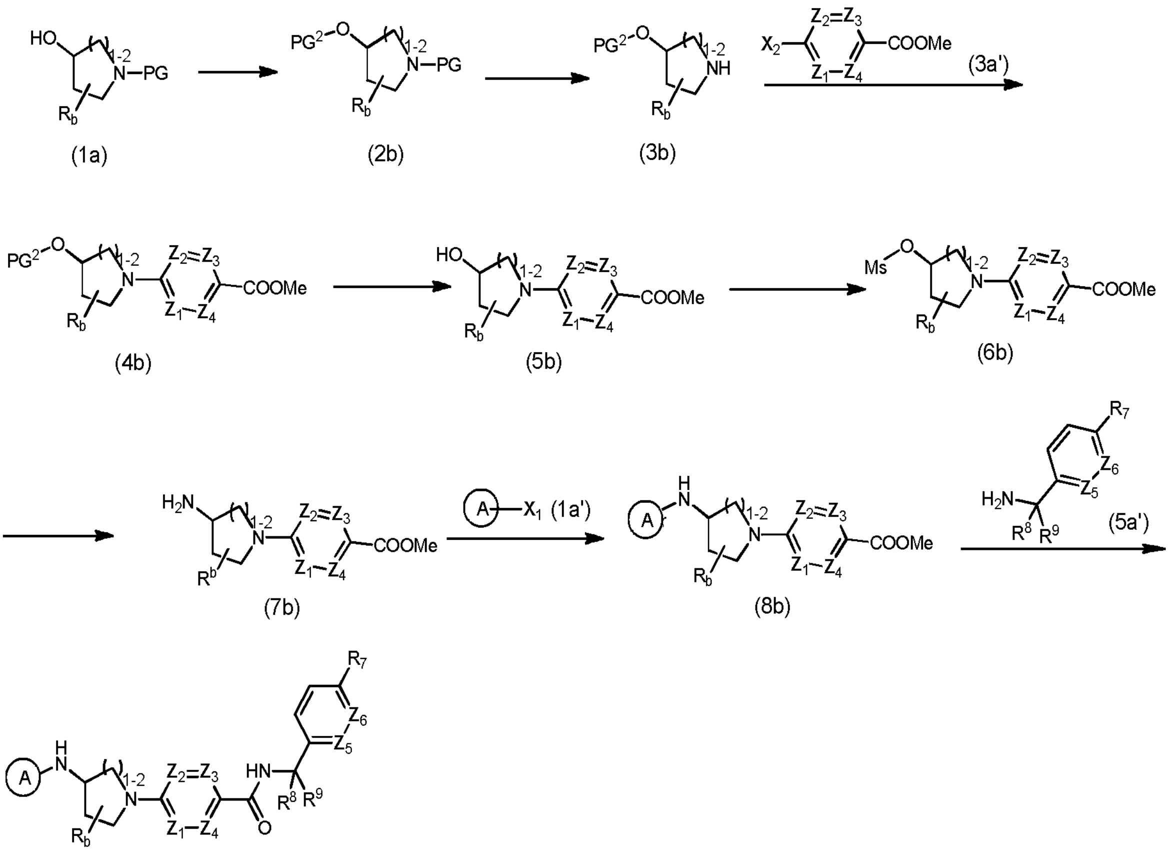


(1a) (2b) (3b) (4b) (5b) (6b) (7b) (8b) (9b)

In the formula, each $X_1$ and $X_2$ represents a halogen atom, $PG^2$ represents a hydroxy protecting group, PG represents an amino protecting group, and Ms represents a methanesulfonyl group.

Compound (9b) can be prepared by the following procedure:

Compound (1a) can undergo a hydroxy protection reaction to obtain compound (2b), compound (2b) can be deaminated under acidic conditions (such as trifluoroacetic acid) to obtain compound (3b), which can be then subjected to coupling reaction with compound (3a') to obtain compound (4b), the hydroxy protecting group of compound (4b) can be removed to obtaine compound (5b), compound (5b) can react with a sulfonyl compound to obtain compound (6b), compound (6b) can remove the leaving group and undergo amino substitution reaction to obtain compound (7b), compound (7b) and compound (5a') can undergo a substitution or coupling reaction to obtain compound (8b), compound (8b) can be condensed with compound (8b') to obtain compound (9b).

## Synthetic scheme 3

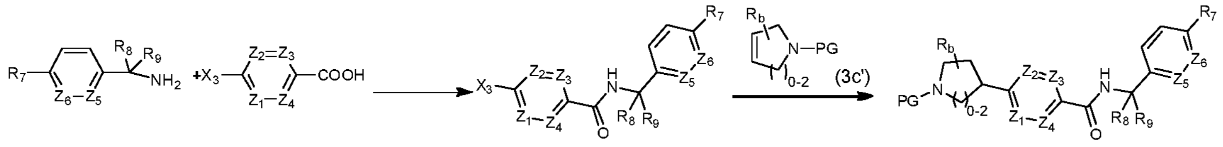


(1c) (2c) (3c) (4c)

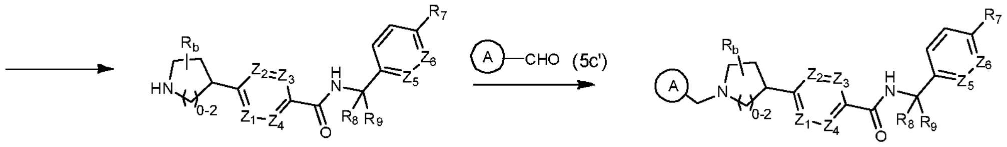


(5c) (6c)

In the formula, $X_3$ represents a halogen atom, and PG represents an amino protecting group.

Compound (6c) can be prepared by the following procedure:

Compound (1c) and compound (2c) can undergo acylation to obtain compound (3c), compound (3c) and compound (3c') can undergo addition reaction to obtain compound (4c), compound (4c) can be deaminated to obtain compound (5c), compound (5c) can react with compound (5c') to obtain compound (6c).

The compounds, pharmaceutical compositions and uses thereof provided by the present invention will be further described below with reference to the examples.

### Synthesis of Intermediates

### Intermediate: (*S*)-3-Amino-3-(4-(ethylsulfonyl)phenyl)propionitrile hydrochloride

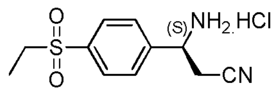

### Step 1: Synthesis of (3*S*)-3-(((*tert*-butoxy)carbonyl)amino)-3-(4-(ethylsulfonyl) phenyl)propionic acid

A solution of LiOH (10 g, 459.10 mmol) in $H_2O$ (50 mL) was added to a solution of methyl (3*S*)-3-(((*tert*-butoxy)carbonyl)amino)-3-(4-(ethylsulfonyl)phenyl)propionate (18.00 g, 41.91 mmol) in MeOH (50 mL) at room temperature. The mixture was reacted at room temperature for 16 h. The reaction solution was concentrated under reduced pressure, and the remaining aqueous phase was added with concentrated HCl solution (12 mol/L) to adjust the pH to 5. The resulting mixture was extracted with EtOAc (100 mL × 2), washed with saturated NaCl solution (80 mL), dried over anhydrous $Na_2SO_4$, and concentrated under reduced pressure to give a white solid (14 g, 93%).
MS (ESI, pos.ion) m/z: 302.1 $[M-56+H]^+$.

### Step 2: Synthesis of *tert*-butyl (*S*)-(3-amino-1-(4-(ethylsulfonyl)phenyl) -3-oxopropyl)carbamate

To a solution of (3*S*)-3-(((*tert*-butoxy)carbonyl)amino)-3-(4-(ethylsulfonyl)phenyl)propionic acid (14 g, 39.17 mmol) in DCM (160 mL) were added $NH_4Cl$ (6.28 g, 117.41 mmol), HATU (20.00 g, 52.60 mmol), TEA (16.3 mL, 117.27 mmol) sequentially at room temperature. The mixture was reacted at room temperature for 16 h. The reaction solution was filtered to obtain a white solid. The white solid was dissolved in MeOH, filtered, and the filtrate was concentrated under reduced pressure to obtain a white solid. The filtrate was washed with 0.5 mol/L HCl (100 mL × 2), filtered, and the filtrate was then dried over anhydrous $Na_2SO_4$, concentrated under reduced pressure to precipitate a large amount of white insoluble solid product, which was filtered to give a white solid (12.5 g, 89%).
MS (ESI, pos.ion) m/z: 301.1 $[M-56+H]^+$.

**Step 3: Synthesis of *tert*-butyl (*S*)-(2-cyano-1-(4-(ethylsulfonyl)phenyl)ethyl)carbamate**

To a solution of *tert*-butyl (*S*)-(3-amino-1-(4-(ethylsulfonyl)phenyl)-3-oxopropyl) carbamate (48 g, 134.70 mmol) and pyridine (40.00 mL, 497.00 mmol) in THF (600 mL) was added TFAA (33 mL, 237.4 mmol) under nitrogen protection at -10 °C (about 30 min). The mixture was transferred to room temperature after 1 h and reacted for 12 h. Saturated $NaHCO_3$ solution (200 mL) was added to the reaction solution to quench the reaction, and the resulting mixture was concentrated under reduced pressure. During the concentration, a white solid product was precipitated. The mixture was filtered and the filter cake was washed with water to obtain a white solid. The white solid was dissolved in DCM (100 mL), washed with saturated NaCl solution (50 mL), dried over anhydrous $Na_2SO_4$, and concentrated under reduced pressure to give a white solid (43.0 g, 94%).
MS (ESI, pos.ion) m/z: 361.1 $[M+Na]^+$.

**Step 4: Synthesis of (*S*)-3-amino-3-(4-(ethylsulfonyl)phenyl)propionitrile hydrochloride**

To a solution of *tert*-butyl (*S*)-(2-cyano-1-(4-(ethylsulfonyl)phenyl)ethyl)carbamate (45.00 g, 133.00 mmol) in DCM (300 mL) was added dropwise a solution of HCl in 1,4-dioxane (100 mL, 4 mol/L) at 0 °C. After the addition was completed, the mixture was transferred to room temperature and reacted overnight, and the reaction solution was filtered to obtain a white solid (34.2 g, 94%).

MS (ESI, pos.ion) m/z: 239.1 $[M+H]^+$.
$^1H$ NMR (400 MHz, $CD_3OD$) δ (ppm): 8.09 (d, *J* = 8.4 Hz, 2H), 7.87 (d, *J* = 8.3 Hz, 2H), 4.96 (t, *J* = 7.0 Hz, 1H), 3.37 (d, *J*= 6.9 Hz, 2H), 3.29 (q, *J*= 7.4 Hz, 2H), 1.25 (t, *J*= 7.4 Hz, 3H).

According to the preparation method of the above-mentioned intermediate, the following intermediate compounds can be prepared with suitable raw materials:

(*S*)-3-Amino-3-(5-(ethylsulfonyl)pyridin-2-yl)propionitrile hydrochloride;

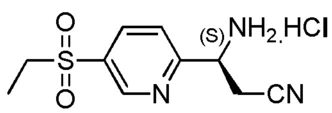

(*S*)-4-Amino-4-(4-(ethylsulfonyl)phenyl)butyronitrile;

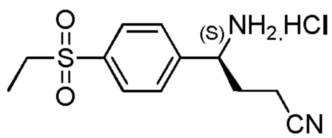

(*R*)-3-Amino-3-(4-(ethylsulfonyl)phenyl)propionitrile hydrochloride;

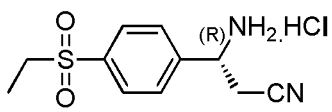

(*S*)-3-Amino-3-(6-methoxypyridin-3-yl)propionitrile hydrochloride;

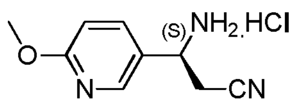

(*S*)-3-Amino-3-(4-(methylsulfonyl)phenyl)propionitrile hydrochloride.

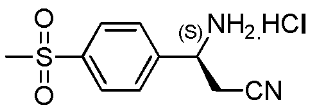

Examples

**Example 1 *N*-((*S*)-2-cyano-1-(4-(ethylsulfonyl)phenyl)ethyl)-4-((2*S*,4*S*)-2-((difluoromethoxy) methyl)-4-((2,6-dimethylpyrimidin-4-yl)oxy)pyrrolidin-1-yl)benzamide**

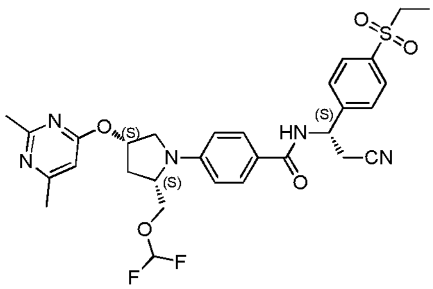

**Step 1: Synthesis of (2*S*,4*R*)-1-*tert*-butyl 2-methyl 4-(((benzyloxy)carbonyl)oxy)pyrrolidine-1,2-dicarboxylate**

To a solution of *tert*-butyl (2*S*,4*R*)-2-methyl-4-hydroxypyrrolidine-1,2-dicarboxylate (20.00 g, 81.54 mmol) in DMAP (31.46 g, 244.60 mmol) and DCM (200 mL) was slowly added dropwise CbzCl (18.0 mL, 126.09 mmol) under an ice bath. The mixture was stirred at room temperature for 24 h. The reaction solution was filtered, and the filtrate was washed successively with saturated $NaHCO_3$ solution (100 mL) and saturated NaCl solution (100 mL), dried over anhydrous $Na_2SO_4$, concentrated under reduced pressure, and the concentrated solution was separated by silica gel column chromatography (eluent: PE/EtOAc (v/v) = 4/1) to give a white solid (30 g, 96%).
MS (ESI, pos.ion) m/z: 324.2 $[M-56+H]^+$.

**Step 2: Synthesis of *tert*-butyl (2*S*,4*R*)-4-(((benzyloxy)carbonyl)oxy) -2-(hydroxymethyl)pyrrolidine-1-carboxylate**

To THF (80 mL) solution was added (2*S*,4*R*)-1-*tert*-butyl 2-methyl 4-(((benzyloxy)carbonyl)oxy)pyrrolidine-1,2-dicarboxylate (28.30 g, 74.59 mmol), then the mixture was transferred to 0 °C, and a solution of $BH_3$ in THF solution (370 mL, 1 mol/L) was added. After the addition was completed, the mixture was reacted at 45 °C for 11 h. Under vigorous stirring, saturated $NH_4Cl$ (200 mL) solution was added to the reaction solution to quench the reaction. The mixture was left standing for layers, the organic phase was concentrated under reduced pressure, the aqueous phase was extracted with EtOAc (100 mL × 2). The combined organic phases were dissolved, washed with saturated NaCl solution (100 mL), dried over anhydrous $Na_2SO_4$, concentrated under reduced pressure, and the concentrated solution was separated by silica gel column chromatography (eluent: PE/EtOAc (v/v) = 3/2) to give colorless liquid (19.17 g, 73%).
MS (ESI, pos.ion) m/z: 296.2 $[M-56+H]^+$.

**Step 3: Synthesis of *tert*-butyl (2*S*,4*R*)-4-(((benzyloxy)carbonyl)oxy) -2-((difluoromethoxy)methyl)pyrrolidine-1-carboxylate**

To a solution of *tert*-butyl (2*S*,4*R*)-4-(((benzyloxy)carbonyl)oxy)-2-(hydroxymethyl) pyrrolidine-1-carboxylate (25.00 g, 71.15 mmol) in DCM (40 mL) were sequentially added $H_2O$ (40.0 mL), KOAc (28.00 g, 285.31 mmol), $TMSCF_2Br$ (23.0 mL, 147.90 mmol). The mixture was stirred at room temperature for 16 h. The reaction solution was diluted with DCM (100 mL) and $H_2O$ (100 mL), extracted and separated. The organic phase was washed with saturated NaCl solution (50 mL), dried over anhydrous $Na_2SO_4$, concentrated under reduced pressure, and the concentrated solution was separated by silica gel column chromatography (eluent: PE/EtOAc (v/v) = 4/1) to give colorless liquid (20 g, 70%).
MS (ESI, pos.ion) m/z: 346.1 $[M-56+H]^+$.

**Step 4: Synthesis of benzyl ((3*R*,5*S*)-5-((difluoromethoxy)methyl)pyrrolidin-3-yl)carboxylate**

To a solution of *tert*-butyl (2*S*,4*R*)-4-(((benzyloxy)carbonyl)oxy)-2-((difluoromethoxy) methyl)pyrrolidine-1-carboxylate (20.00 g, 49.83 mmol) in DCM (20 mL) was added a solution of HCl in 1,4-dioxane (30 mL, 4 mol/L). The mixture was stirred at room temperature for 24 h. The reaction solution was concentrated under reduced pressure, and the concentrated solution was diluted with EtOAc (80 mL), washed successively with saturated $Na_2CO_3$ solution (30 mL) and saturated NaCl solution (30 mL), dried over anhydrous $Na_2SO_4$, and concentrated under reduced pressure to give brown liquid (15.00 g, 100%).
MS (ESI, pos.ion) m/z: 302.3 $[M+H]^+$.

**Step 5: Synthesis of methyl 4-((2*S*,4*R*)-4-(((benzyloxy)carbonyl)oxy) -2-((difluoromethoxy)methyl)pyrrolidin-1-yl)benzoate**

Under nitrogen protection, benzyl ((3*R*,5*S*)-5-((difluoromethoxy)methyl) pyrrolidin-3-yl)carboxylate (12.00 g, 39.83 mmol), $Pd_2(dba)_3$ (1.83 g, 2.00 mmol), 2-bicyclohexylphosphine-2',6'-diisopropoxybiphenyl (1.40 g, 3.00 mmol), $Cs_2CO_3$ (15.00 g, 46.04 mmol), methyl 4-iodobenzoate (10.44 g, 39.84 mmol) were successively added to 1,4-dioxane (140 mL) and the mixture was reacted at 100 °C for 20 h. The reaction solution was cooled to room temperature, filtered through a celite pad, the filtrate was concentrated under reduced pressure, and the concentrated solution was separated by silica gel column chromatography (eluent: PE/EtOAc (v/v) = 4/1) to give pale yellow liquid (14.84 g, 86%).
MS (ESI, pos.ion) m/z: 436.2 $[M+H]^+$.

Step 6: Synthesis of methyl **4-((2*S*,4*R*)-2-((difluoromethoxy)methyl)-4-hydroxypyrrolidin-1-yl)benzoate**

To a solution of methyl 4-((2*S*,4*R*)-4-(((benzyloxy)carbonyl)oxy)-2-((difluoromethoxy) methyl)pyrrolidin-1-yl)benzoate (15.00 g, 34.45 mmol) in MeOH (60 mL) was added Pd/C (1.00 g, 10%). The mixture was degassed and refilled with hydrogen, and was reacted at room temperature for 24 h. The reaction solution was filtered through a celite pad, and concentrated under reduced pressure, and the concentrated solution was separated by silica gel column chromatography (eluent: PE/EtOAc (v/v) = 3/2) to give pale yellow liquid (10.0 g, 96%).
MS (ESI, pos.ion) m/z: 302.2 $[M+H]^+$.

**Step 7: Synthesis of methyl 4-((2*S*,4*S*)-2-((difluoromethoxy)methyl)-4-((2,6-dimethylpyrimidin-4-yl)oxy)pyrrolidin-1-yl)benzoate**

Methyl 4-((2*S*,4*R*)-2-((difluoromethoxy)methyl)-4-hydroxypyrrolidin-1-yl)benzoate (560 mg, 1.86 mmol), 2,6-dimethylpyrimidine-alcohol (255 mg, 2.05 mmol), $PPh_3$ (540 mg, 2.06 mmol) were added to THF (8 mL). The mixture was transferred to 0 °C, and DIAD (0.5 mL, 2.54 mmol) was slowly added. After the addition was complete, the mixture was transferred to room temperature and stirred for 18 h. The reaction solution was concentrated under reduced pressure. The concentrated solution was diluted with methyl tert-butyl ether (10 mL) and stirred at -20 °C. A white insoluble solid was precipitated, filtered while cold, and the filter cake was washed with cold methyl tert-butyl ether. The filtrate was concentrated under reduced pressure, and the concentrated solution was separated by silica gel column chromatography (eluent: PE/EtOAc (v/v) = 3/1) to obtain pale yellow liquid (652 mg, 86%).
MS (ESI, pos.ion) m/z: 408.2 $[M+H]^+$.

**Step 8: Synthesis of 4-((2*S*,4*S*)-2-((difluoromethoxy)methyl)-4-((2,6-dimethylpyrimidin -4-yl)oxy)pyrrolidin-1-yl)benzoic acid**

To a solution of methyl 4-((2*S*,4*S*)-2-((difluoromethoxy)methyl)-4-((2,6-dimethylpyrimidin-4-yl)oxy)pyrrolidin-1-yl)benzoate (100 mg, 0.24 mmol) in MeOH (2 mL) and THF (2 mL) was added a solution of $LiOH\cdot H_2O$ (100 mg, 2.38 mmol) in $H_2O$ (2 mL). The mixture was stirred at room temperature for 24 h. The reaction solution was concentrated under reduced pressure, HCl solution (1 mol/L) was added to the concentrated solution to adjust the pH to about 4. The resulting mixture was extracted with EtOAc (30 mL), washed with saturated NaCl (15 mL) solution, dried over anhydrous $Na_2SO_4$, concentrated under reduced pressure, and the concentrated solution was separated by silica gel column chromatography (eluent: DCM/EtOAc (v/v) = 1/1) to give a pale yellow solid (50 mg, 54%).
MS (ESI, pos.ion) m/z: 394.2 $[M+H]^+$.

**Step 9: Synthesis of *N*-((*S*)-2-cyano-1-(4-(ethylsulfonyl)phenyl)ethyl)-4-((2*S*,4*S*)-2-((difluoromethoxy)methyl)-4-((2,6-dimethylpyrimidin-4-yl)oxy)pyrrolidin-1-yl)benzamide**

HATU (60 mg, 0.16 mmol), (*S*)-3-amino-3-(4-(ethylsulfonyl)phenyl)propionitrile hydrochloride (40 mg, 0.15 mmol), 4-((2*S*,4*S*)-2-((difluoromethoxy)methyl)-4-((2,6-dimethylpyrimidin-4-yl)oxy) pyrrolidin-1-yl)benzoic acid (50 mg, 0.13 mmol) and TEA (40 mg, 0.40 mmol) were successively added to DCM (3 mL) and the mixture was stirred at room temperature for 16 h. The reaction solution was concentrated under reduced pressure, the residue was added with DCM (30 mL), washed successively with HCl solution (15 mL, 1 mol/L) and saturated NaCl solution (15 mL), dried over anhydrous $Na_2SO_4$, concentrated under reduced pressure, and the concentrated solution was separated by silica gel column chromatography (eluent: DCM/EtOAc (v/v) = 2/1) to give a white solid (40 mg, 51%).

MS (ESI, pos.ion) m/z: 614.2 $[M+H]^+$.
$^1$H NMR (400 MHz, $CDCl_3$) δ (ppm): 7.94 (d, *J* = 8.2 Hz, 2H), 7.73 (d, *J* = 8.7 Hz, 2H), 7.66 (d, *J* = 8.3 Hz, 2H), 6.65 (d, *J* = 8.7 Hz, 2H), 6.59 (d, *J* = 7.6 Hz, 1H), 6.39 (s, 1H), 6.24 (t, *J* = 74.3 Hz, 1H), 5.87 (s, 1H), 5.60 (dd, *J* = 12.2, 6.3 Hz, 1H), 4.23 - 4.13 (m, 2H), 3.95 (t, *J* = 9.2 Hz, 1H), 3.74 - 3.63 (m, 2H), 3.17 (dd, *J* = 15.3, 4.7 Hz, 1H), 3.12 (q, *J* = 7.50 Hz, 2H), 3.06 (dd, *J* = 16.9, 4.9 Hz, 1H), 2.60 (s, 3H), 2.42 (d, *J* = 9.7 Hz, 5H), 1.29 (t, *J* = 7.5 Hz, 3H).

The material (2,6-dimethylpyrimidine-alcohol) in Step 7 of Example 1 was replaced with other reaction substrates, and the target compounds in Table 1 were prepared according to the methods of Step 7 to Step 9 of Example 1.

Table 1 The target compound prepared according to the synthetic method of Example 1

| Example No. | Target compound structure and name | Target compound characterization data |
|---|---|---|
| Example 2 | 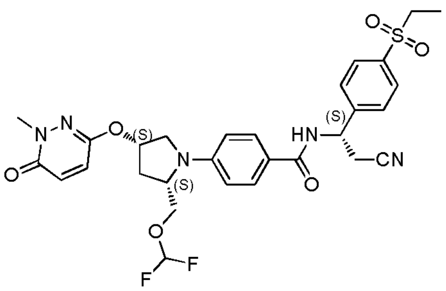 *N*-((*S*)-2-cyano-1-(4-(ethylsulfonyl) phenyl)ethyl)-4-((2*S*,4*S*)-2-((difluor omethoxy)methyl)-4-((1-methyl-6-oxo-1,6-dihydropyridazin-3-yl)oxy) pyrrolidin- 1 -yl)benzamide | white solid; MS (ESI, pos.ion) m/z: 616.2 $[M+H]^+$; $^1$H NMR (400 MHz, $CDCl_3$) δ (ppm): 7.93 (d, *J*= 8.3 Hz, 2H), 7.78 (d, *J* = 8.8 Hz, 2H), 7.67 (d, *J* = 8.3 Hz, 2H), 6.98 - 6.87 (m, 3H), 6.67 (d, *J* = 8.8 Hz, 2H), 6.26 (t, *J* = 74.2 Hz, 1H), 5.62 (dd, *J* = 12.9, 6.1 Hz, 1H), 5.51 (s, 1H), 4.25 - 4.12 (m, 2H), 3.92 (t, *J* = 9.7 Hz, 1H), 3.76 - 3.67 (m, 5H), 3.20 - 3.04 (m, 4H), 2.50 (d, *J* = 14.6 Hz, 1H), 2.45 - 2.35 (m, 1H), 1.30 (t, *J* = 7.4 Hz, 3H). |
| Example 3 | 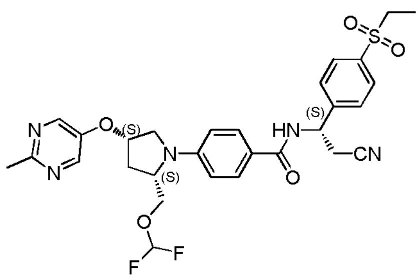 *N*-((*S*)-2-cyano-1-(4-(ethylsulfonyl) phenyl)ethyl)-4-((2*S*,4*S*)-2-((difluor omethoxy)methyl)-4-((2-methylpyr imidin-5-yl)oxy)pyrrolidin-1-yl)ben zamide | white solid; MS (ESI, pos.ion) m/z: 600.0 $[M+H]^+$; $^1$H NMR (400 MHz, $CDCl_3$) δ (ppm): 8.33 (s, 2H), 7.92 (d, *J* = 8.3 Hz, 2H), 7.75 (d, *J* = 8.8 Hz, 2H), 7.65 (d, *J* = 8.3 Hz, 2H), 6.73 (d, *J* = 7.6 Hz, 1H), 6.64 (d, *J* = 8.8 Hz, 2H), 6.22 (t, *J* = 74.1 Hz, 1H), 5.59 (dd, *J* = 12.6, 6.3 Hz, 1H), 5.15 (t, *J* = 4.5 Hz, 1H), 4.26 - 4.14 (m, 2H), 3.92 (t, *J* = 9.7 Hz, 1H), 3.77 (d, *J* = 11.5 Hz, 1H), 3.71 (dd, *J* = 11.6, 4.6 Hz, 1H), 3.19 - 3.08 (m, 3H), 3.05 (dd, *J* = 17.0, 5.2 Hz, 1H), 2.69 (s, 3H), 2.52 (d, *J* = 14.4 Hz, 1H), 2.45 - 2.36 (m, 1H), 1.28 (t, *J* = 7.4 Hz, 3H). |

(continued)

| Example No. | Target compound structure and name | Target compound characterization data |
|---|---|---|
| Example 4 | 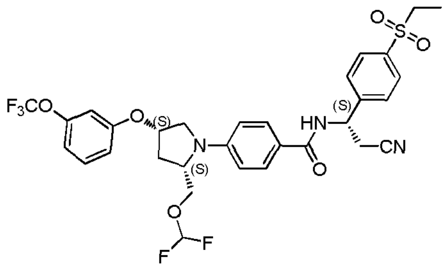 *N*-((*S*)-2-cyano-1-(4-(ethylsulfonyl) phenyl)ethyl)-4-((2*S*,4*S*)-2-((difluor omethoxy)methyl)-4-(3-(trifluorom ethoxy)phenoxy)pyrrolidin-1-yl)be nzamide | white solid; MS (ESI, pos.ion) m/z: 668.2 $[M+H]^+$; $^1H$ NMR (400 MHz, $CDCl_3$) δ (ppm): 7.93 (d, *J* = 7.2 Hz, 2H), 7.74 (d, *J* = 8.8 Hz, 2H), 7.65 (d, *J* = 8.2 Hz, 2H), 7.33 (t, *J* = 8.3 Hz, 1H), 6.88 (d, *J* = 8.0 Hz, 1H), 6.83 (dd, *J* = 8.3, 2.1 Hz, 1H), 6.75 (s, 1H), 6.65 (d, *J* = 8.7 Hz, 3H), 6.22 (t, *J* = 74.3 Hz, 1H), 5.59 (dd, *J* = 12.4, 6.2 Hz, 1H), 5.11 (t, *J* = 4.6 Hz, 1H), 4.23 - 4.13 (m, 2H), 3.96 (t, *J* = 9.4 Hz, 1H), 3.76 (d, *J* = 11.3 Hz, 1H), 3.68 (dd, *J* = 11.4, 4.7 Hz, 1H), 3.20 - 3.02 (m, 4H), 2.51 (d, *J* = 14.4 Hz, 1H), 2.41 - 2.32 (m, 1H), 1.29 (t, *J* = 7.4 Hz, 3H). |
| Example 5 | 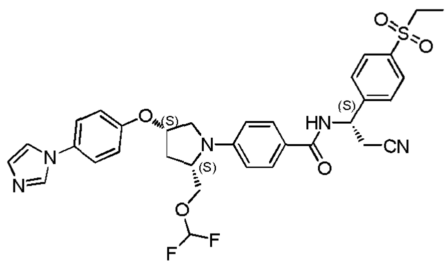 4-((2*S*,4*S*)-4-(4-(1*H*-imidazol-1-yl) phenoxy)-2-((difluoromethoxy)met hyl)pyrrolidin-1-yl)-*N*-((*S*)-2-cyano -1-(4-(ethylsulfonyl)phenyl)ethyl)b enzamide | white solid; MS (ESI, pos.ion) m/z: 650.2 $[M+H]^+$; $^1H$ NMR (400 MHz, DMSO-$d_6$) δ (ppm): 8.95 (d, *J* = 8.2 Hz, 1H), 8.16 (s, 1H), 7.87 (d, *J* = 8.3 Hz, 2H), 7.79 (d, *J* = 8.8 Hz, 2H), 7.70 (d, *J* = 8.3 Hz, 2H), 7.65 (s, 1H), 7.57 (d, *J* = 8.9 Hz, 2H), 7.12 (d, *J* = 9.0 Hz, 2H), 7.09 (s, 1H), 6.73 (d, *J* = 8.8 Hz, 2H), 6.68 (t, *J* = 75.7 Hz, 1H), 5.48 (q, *J* = 8.1 Hz, 1H), 5.27 (s, 1H), 4.21 (d, *J* = 5.1 Hz, 1H), 4.07 (dd, *J* = 9.9, 4.7 Hz, 1H), 3.94 - 3.87 (m, 2H), 3.25 (q, *J* = 7.3 Hz, 3H), 3.15 - 3.11 (m, 2H), 2.45 - 2.40 (s, 1H), 2.25 (d, *J* = 14.2 Hz, 1H), 1.08 (t, *J*= 7.3 Hz, 3H). |
| Example 6 | 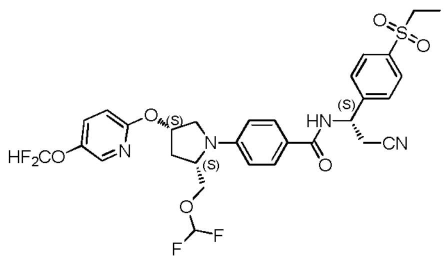 *N*-((*S*)-2-cyano-1-(4-(ethylsulfonyl) phenyl)ethyl)-4-((2*S*,4*S*)-2-((difluor omethoxy)methyl)-4-((5-(difluorom ethoxy)pyridin-2-yl)oxy)pyrrolidin-1-yl)benzamide | white solid; MS (ESI, pos.ion) m/z: 651.1 $[M+H]^+$; $^1H$ NMR (400 MHz, $CDCl_3$) δ (ppm): 8.04 (d, *J* = 2.6 Hz, 1H), 7.91 (d, *J* = 8.3 Hz, 2H), 7.73 (d, *J* = 8.8 Hz, 2H), 7.64 (d, *J* = 8.3 Hz, 2H), 7.44 (dd, *J* = 8.9, 2.8 Hz, 1H), 6.78 - 6.70 (m, 2H), 6.66 - 6.62 (m, 2H), 6.48 - 6.02 (m, 2H), 5.73 (d, *J* = 3.1 Hz, 1H), 5.59 (dd, *J* = 12.8, 6.2 Hz, 1H), 4.23 - 4.13 (m, 2H), 3.98 (t, *J* = 9.2 Hz, 1H), 3.71 (d, *J* = 2.7 Hz, 2H), 3.18 - 3.01 (m, 4H), 2.46 (d, *J* = 14.3 Hz, 1H), 2.43 - 235 (m, 1H), 1.28 (t, *J* = 7.4 Hz, 3H). |

(continued)

| Example No. | Target compound structure and name | Target compound characterization data |
|---|---|---|
| Example 7 | 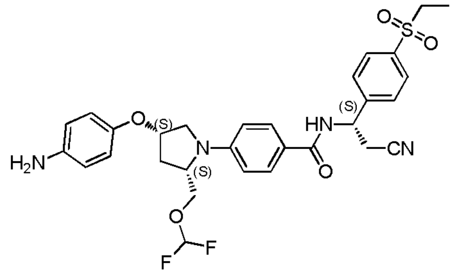 4-((2*S*,4*S*)-4-(4-aminophenoxy)-2-( (difluoromethoxy)methyl)pyrrolidi n-1-yl)-*N*-((*S*)-2-cyano-1-(4-(ethyls ulfonyl)phenyl)ethyl)benzamide | white solid; MS (ESI, pos.ion) m/z: 599.2 $[M+H]^+$; $^1H$ NMR (400 MHz, $CDCl_3$) δ (ppm): 7.93 (d, *J* = 8.2 Hz, 2H), 7.72 (d, *J* = 8.8 Hz, 2H), 7.65 (d, *J* = 8.3 Hz, 2H), 6.74 (d, *J* = 8.8 Hz, 2H), 6.69 - 6.58 (m, 5H), 6.22 (t, *J* = 74.6 Hz, 1H), 5.59 (dd, *J* = 12.6, 6.4 Hz, 1H), 4.98 (t, *J* = 4.7 Hz, 1H), 4.20 - 4.12 (m, 2H), 4.02 (t, *J* = 10.8 Hz, 1H), 3.73 (d, *J* = 11.2 Hz, 1H), 3.57 (dd, *J* = 11.3, 4.8 Hz, 1H), 3.19 - 3.02 (m, 4H), 2.48 (d, *J* = 14.3 Hz, 1H), 2.31 - 2.22 (m, 1H), 1.42 (s, 2H), 1.29 (t, *J* = 7.4 Hz, 3H). |
| Example 8 | 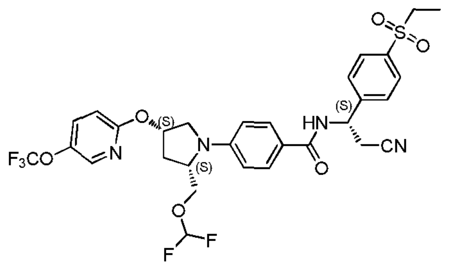 *N*-((*S*)-2-cyano-1-(4-(ethylsulfonyl) phenyl)ethyl)-4-((2*S*,4*S*)-2-((difluor omethoxy)methyl)-4-((5-(trifluoro methoxy)pyridin-2-yl)oxy)pyrrolidi n-1-yl)benzamide | white solid; MS (ESI, pos.ion) m/z: 669.1 $[M+H]^+$; $^1H$ NMR (400 MHz, $CDCl_3$) δ (ppm): 8.11 (d, *J* = 2.6 Hz, 1H), 7.93 (d, *J* = 8.2 Hz, 2H), 7.73 (d, *J* = 8.8 Hz, 2H), 7.65 (d, *J* = 8.3 Hz, 2H), 7.50 (dd, *J* = 8.8, 2.3 Hz, 1H), 6.77 (d, *J* = 9.0 Hz, 1H), 6.64 (t, *J* = 8.4 Hz, 3H), 6.24 (t, *J* = 74.0 Hz, 1H), 5.75 (s, 1H), 5.59 (dd, *J* = 12.6, 6.3 Hz, 1H), 4.24 - 4.14 (m, 2H), 3.98 (t, *J* = 9.4 Hz, 1H), 3.71 (d, *J* = 2.8 Hz, 2H), 3.19 - 3.02 (m, 4H), 2.48 (d, *J* = 14.4 Hz, 1H), 2.44 - 2.36 (m, 1H), 1.29 (t, *J*= 7.4 Hz, 3H). |
| Example 9 | 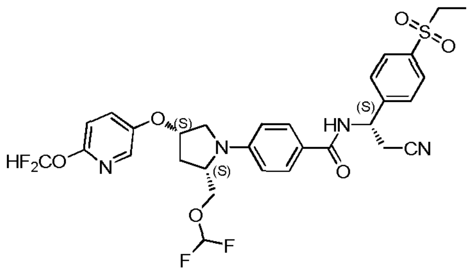 *N*-((*S*)-2-cyano-1-(4-(ethylsulfonyl) phenyl)ethyl)-4-((2*S*,4*S*)-2-((difluor omethoxy)methyl)-4-((6-(difluorom ethoxy)pyridin-3-yl)oxy)pyrrolidin-1-yl)benzamide | white solid; MS (ESI, pos.ion) m/z: 651.2 $[M+H]^+$; $^1H$ NMR (400 MHz, $CDCl_3$) δ (ppm): 7.91 (d, *J* = 8.3 Hz, 2H), 7.84 (d, *J* = 3.0 Hz, 1H), 7.74 (d, *J* = 8.8 Hz, 2H), 7.64 (d, *J* = 8.3 Hz, 2H), 7.34 (t, *J* = 73.3 Hz, 1H), 7.32 (dd, *J* = 8.9, 3.0 Hz, 1H), 6.89 (d, *J* = 8.9 Hz, 1H), 6.73 (d, *J* = 7.7 Hz, 1H), 6.64 (d, *J* = 8.8 Hz, 2H), 6.23 (t, *J* = 74.2 Hz, 1H), 5.59 (dd, *J* = 12.7, 6.2 Hz, 1H), 5.08 (t, *J* = 4.6 Hz, 1H), 4.25 - 4.13 (m, 2H), 3.94 (t, *J* = 9.5 Hz, 1H), 3.76 (d, *J* = 11.4 Hz, 1H), 3.68 (dd, *J* = 11.5, 4.8 Hz, 1H), 3.19 - 3.02 (m, 4H), 2.51 (d, *J* = 14.4 Hz, 1H), 2.42 - 2.32 (m, 1H), 1.28 (t, *J* = 7.4 Hz, 3H). |

(continued)

| Example No. | Target compound structure and name | Target compound characterization data |
|---|---|---|
| Example 10 | 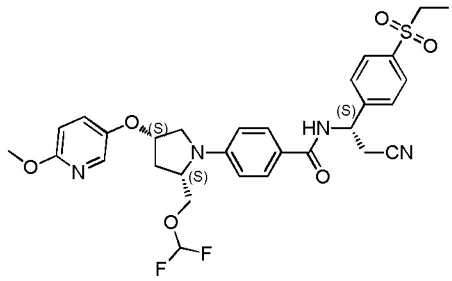 *N*-((*S*)-2-cyano-1-(4-(ethylsulfonyl) phenyl)ethyl)-4-((2*S*,4*S*)-2-((difluor omethoxy)methyl)-4-((6-methoxyp yridine-3-yl)oxy)pyrrolidin-1-yl)be nzamide | white solid; MS (ESI, pos.ion) m/z: 615.2 $[M+H]^+$; $^1H$ NMR (400 MHz, $CDCl_3$) δ (ppm): 7.92 (d, *J* = 8.3 Hz, 2H), 7.81 (d, *J* = 2.9 Hz, 1H), 7.74 (d, *J* = 8.8 Hz, 2H), 7.65 (d, *J* = 8.3 Hz, 2H), 7.22 (dd, *J* = 8.9, 3.0 Hz, 1H), 6.72 (dd, *J* = 8.2, 3.5 Hz, 2H), 6.64 (d, *J* = 8.8 Hz, 2H), 6.23 (t, *J* = 74.4 Hz, 1H), 5.59 (dd, *J* = 12.6, 6.2 Hz, 1H), 5.03 (t, *J* = 4.7 Hz, 1H), 4.22 - 4.14 (m, 2H), 3.97 (t, *J* = 9.5 Hz, 1H), 3.90 (s, 3H), 3.75 (d, *J* = 11.3 Hz, 1H), 3.63 (dd, *J* = 11.5, 4.8 Hz, 1H), 3.19 - 3.02 (m, 4H), 2.51 (d, *J* = 14.4 Hz, 1H), 2.36 - 2.29 (m, 1H), 1.28 (t, *J* = 7.4 Hz, 3H). |
| Example 11 | 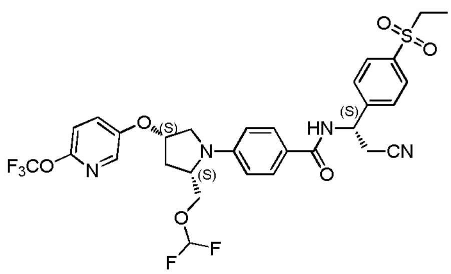 *N*-((*S*)-2-cyano-1-(4-(ethylsulfonyl) phenyl)ethyl)-4-((2*S*,4*S*)-2-((difluor omethoxy)methyl)-4-((6-(trifluoro methoxy)pyridin-3-yl)oxy)pyrrolidi n-1-yl)benzamide | white solid; MS (ESI, pos.ion) m/z: 669.2 $[M+H]^+$; $^1H$ NMR (400 MHz, $CDCl_3$) δ (ppm): 7.98 (d, *J*= 3.0 Hz, 1H), 7.93 (d, *J* = 8.3 Hz, 2H), 7.75 (d, *J* = 8.8 Hz, 2H), 7.65 (d, *J* = 8.3 Hz, 2H), 7.35 (dd, *J* = 8.8, 3.0 Hz, 1H), 7.03 (d, *J* = 8.8 Hz, 1H), 6.66 (t, *J* = 9.5 Hz, 3H), 6.22 (t, *J* = 74.1 Hz, 1H), 5.59 (dd, *J* = 12.5, 6.2 Hz, 1H), 5.13 (t, *J* = 4.7 Hz, 1H), 4.26 - 4.19 (m, 1H), 4.16 (dd, *J* = 10.0, 4.0 Hz, 1H), 3.93 (t, *J* = 9.6 Hz, 1H), 3.77 (d, *J* = 11.5 Hz, 1H), 3.71 (dd, *J* = 11.6, 4.7 Hz, 1H), 3.20 - 3.02 (m, 4H), 2.53 (d, *J* = 14.4 Hz, 1H), 2.43 - 2.36 (m, 1H), 1.29 (t, *J* = 7.4 Hz, 3H). |
| Example 12 | 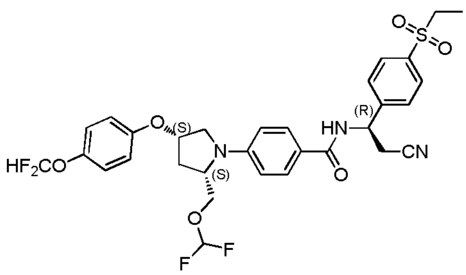 *N*-((*R*)-2-cyano-1 -(4-(ethylsulfonyl) phenyl)ethyl)-4-((2*S*,4*S*)-2-((difluor omethoxy)methyl)-4-(4-(difluorom ethoxy)phenoxy)pyrrolidin-1-yl)be nzamide | white solid; MS (ESI, pos.ion) m/z: 650.3 $[M+H]^+$; $^1H$ NMR (400 MHz, $CDCl_3$) δ (ppm): 7.92 (d, *J* = 8.3 Hz, 2H), 7.73 (d, *J* = 8.8 Hz, 2H), 7.65 (d, *J* = 8.3 Hz, 2H), 7.10 (d, *J* = 9.0 Hz, 2H), 6.88 (d, *J* = 9.0 Hz, 2H), 6.68 (d, *J* = 7.8 Hz, 1H), 6.65 (d, *J* = 8.8 Hz, 2H), 6.45 - 6.03 (m, 2H), 5.59 (dd, *J* = 12.7, 6.3 Hz, 1H), 5.07 (t, *J* = 4.7 Hz, 1H), 4.22 - 4.14 (m, 2H), 3.98 (t, *J* = 9.3 Hz, 1H), 3.74 (d, *J* = 11.3 Hz, 1H), 3.65 (dd, *J* = 11.4, 4.8 Hz, 1H), 3.19 - 3.01 (m, 4H), 2.50 (d, *J* = 14.3 Hz, 1H), 2.38 - 2.31 (m, 1H), 1.29 (t, *J* = 7.4 Hz, 3H). |

(continued)

| Example No. | Target compound structure and name | Target compound characterization data |
|---|---|---|
| Example 13 | 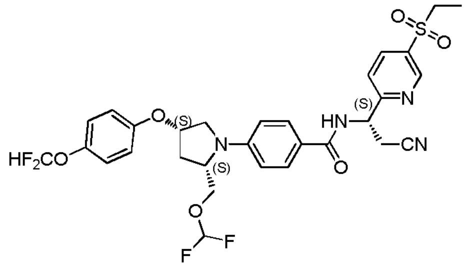 *N*-((*S*)-2-cyano-1-(5-(ethylsulfonyl) pyridin-2-yl)ethyl)-4-((2*S*,4*S*)-2-((d ifluoromethoxy)methyl)-4-(4-(diflu oromethoxy)phenoxy)pyrrolidin- 1 -yl)benzamide | white solid; MS (ESI, pos.ion) m/z: 651.3 $[M+H]^+$; $^1H$ NMR (400 MHz, $CDCl_3$) δ (ppm): 8.79 (s, 1H), 8.04 (s, 2H), 7.74 (d, *J* = 8.8 Hz, 2H), 7.10 (d, *J* = 8.9 Hz, 2H), 7.00 (d, *J* = 7.6 Hz, 1H), 6.87 (d, *J* = 9.0 Hz, 2H), 6.63 (d, *J* = 8.6 Hz, 2H), 6.45 - 6.01 (m, 2H), 5.63 (dd, *J* = 13.1, 6.3 Hz, 1H), 5.07 (t, *J* = 4.6 Hz, 1H), 4.23 - 4.12 (m, 2H), 3.98 (t, *J* = 9.1 Hz, 1H), 3.74 (d, *J* = 11.4 Hz, 1H), 3.65 (dd, *J* = 11.4, 4.7 Hz, 1H), 3.39 (q, *J* = 7.5 Hz, 2H), 3.19 - 3.06 (m, 2H), 2.49 (d, *J* = 14.3 Hz, 1H), 2.39 - 2.29 (m, 1H), 1.30 (t, *J* = 7.5 Hz, 3H). |
| Example 14 | 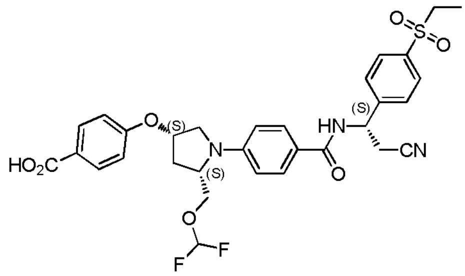 4-(((3*S*,5*S*)-1-(4-(((*S*)-2-cyano-1-(4 -(ethylsulfonyl)phenyl)ethyl)carba moyl)phenyl)-5-((difluoromethoxy) methyl)pyrrolidin-3-yl)oxy)benzoic acid | white solid; MS (ESI, pos.ion) m/z: 628.1 $[M+H]^+$; $^1H$ NMR (400 MHz, DMSO-$d_6$) δ (ppm): 8.90 (d, J = 8.3 Hz, 1H), 7.92 (d, J = 8.8 Hz, 2H), 7.88 (d, J = 8.3 Hz, 2H), 7.80 (d, *J*= 8.7 Hz, 2H), 7.71 (d, *J* = 8.3 Hz, 2H), 7.08 (d, *J* = 8.7 Hz, 2H), 6.74 (d, *J* = 8.7 Hz, 2H), 6.71 (t, *J*= 75.6 Hz, 1H), 5.50 (dd, *J* = 15.2, 8.2 Hz, 1H), 5.33 (s, 1H), 4.26 - 4.19 (m, 1H), 4.07 (dd, *J* = 9.6, 4.2 Hz, 1H), 3.88 (t, *J* = 9.3 Hz, 1H), 3.73 - 3.65 (m, 2H), 3.29 - 3.25 (m, 2H), 3.17 - 3.11 (m, 2H), 2.26 (d, *J* = 14.3 Hz, 1H), 1.28 (d, *J* = 16.0 Hz, 1H), 1.09 (t, *J* = 7.3 Hz, 3H). |
| Example 15 | 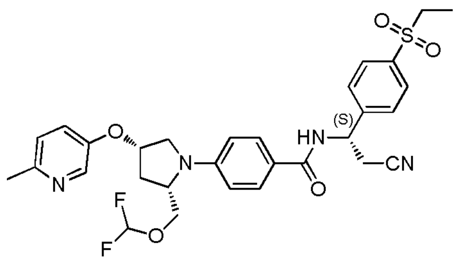 *N*-((*S*)-2-cyano-1-(4-(ethylsulfonyl) phenyl)ethyl)-4-((2*S*,4*S*)-2-((difluor omethoxy)methyl)-4-((6-methylpyr idin-3-yl)oxy)pyrrolidin-1-yl)benza mide | white solid; MS (ESI, pos.ion) m/z: 599.1 $[M+H]^+$; $^1H$ NMR (400 MHz, $CDCl_3$) δ (ppm): 8.18 (d, *J* = 1.9 Hz, 1H), 7.91 (d, *J* = 8.3 Hz, 2H), 7.74 (d, *J* = 8.8 Hz, 2H), 7.64 (t, *J* = 8.0 Hz, 2H), 7.18 - 7.06 (m, 2H), 6.73 (d, *J* = 7.6 Hz, 1H), 6.64 (d, *J* = 8.8 Hz, 2H), 6.22 (t, *J* = 74.3 Hz, 1H), 5.59 (dd, *J* = 12.6, 6.2 Hz, 1H), 5.11 (t, *J* = 4.5 Hz, 1H), 4.18 (dt, *J* = 12.6, 6.2 Hz, 2H), 3.96 (t, *J* = 9.1 Hz, 1H), 3.75 (d, *J* = 11.4 Hz, 1H), 3.67 (dd, *J* = 11.5, 4.8 Hz, 1H), 3.20 - 3.00 (m, 4H), 2.50 (d, *J* = 7.9 Hz, 3H), 2.42 - 2.29 (m, 1H), 1.28 (t, *J* = 7.4 Hz, 3H). |

(continued)

| Example No. | Target compound structure and name | Target compound characterization data |
|---|---|---|
| Example 16 | 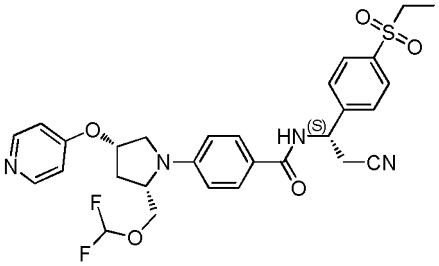 *N*-((*S*)-2-cyano-1-(4-(ethylsulfonyl) phenyl)ethyl)-4-((2S,4S)-2-((difluor omethoxy)methyl)-4-(pyridin-4-ylo xy)pyrrolidin-l-yl)benzamide | white solid; MS (ESI, pos.ion) m/z: 585.2 $[M+H]^+$; $^1H$ NMR (400 MHz, DMSO-$d_6$) δ (ppm): 8.91 (d, *J* = 7.6 Hz, 1H), 8.42 (d, *J* = 4.8 Hz, 2H), 7.88 (d, *J* = 7.9 Hz, 2H), 7.80 (d, *J* = 8.3 Hz, 2H), 7.71 (d, *J* = 8.0 Hz, 2H), 7.03 (d, *J* = 5.0 Hz, 2H), 6.89 - 6.70(m, 3H), 5.50 (d, *J* = 7.7 Hz, 1H), 5.35 (s, 1H), 4.22 (s, 1H), 4.05 (s, 1H), 3.85 (t, *J* = 8.9 Hz, 1H), 3.66 (d, *J* = 11.8 Hz, 3H), 3.27 (d, *J* = 7.3 Hz, 2H), 3.14 (d, *J* = 7.3 Hz, 2H), 2.24 (d, *J* = 14.4 Hz, 1H), 1.09 (t, *J* = 7.4 Hz, 3H). |
| Example 17 | 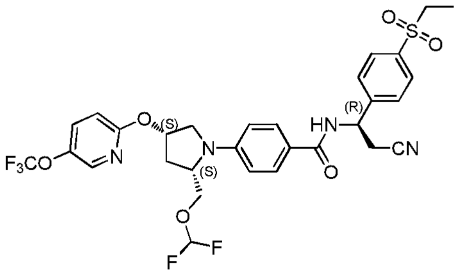 *N*-((*R*)-2-cyano-1-(4-(ethylsulfonyl) phenyl)ethyl)-4-((2S,4S)-2-((difluor omethoxy)methyl)-4-((5-(trifluoro methoxy)pyridin-2-yl)oxy)pyrrolidi n-1-yl)benzamide | white solid; MS (ESI, pos.ion) m/z: 669.1 $[M+H]^+$; $^1H$ NMR (400 MHz, $CDCl_3$) δ (ppm): 8.11 (s, 1H), 7.90 (d, *J* = 8.1 Hz, 2H), 7.74 (d, *J* = 8.5 Hz, 2H), 7.64 (d, *J* = 8.1 Hz, 2H), 7.50 (d, *J* = 7.9 Hz, 1H), 6.77 (d, J = 8.7 Hz, 2H), 6.64 (d, *J* = 8.5 Hz, 2H), 6.24 (t, *J* = 74.3 Hz, 1H), 5.75 (s, 1H), 5.59 (d, *J* = 6.2 Hz, 1H), 4.24 - 4.13 (m, 2H), 3.98 (t, *J* = 9.0 Hz, 1H), 3.71 (s, 2H), 3.18 - 3.01 (m, 4H), 2.50 - 2.35 (m, 2H), 1.28 (t, *J* = 7.4 Hz, 3H). |
| Example 18 | 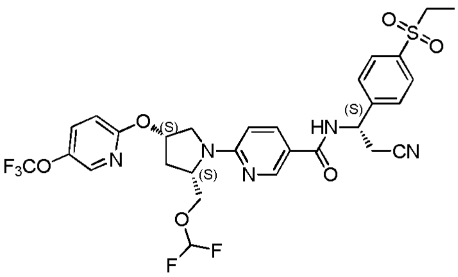 *N*-((*S*)-2-cyano-1-(4-(ethylsulfonyl) phenyl)ethyl)-6-((2*S*,4*S*)-2-((difluor omethoxy)methyl)-4-((5-(trifluoro methoxy)pyrrolidin-2-yl)oxy)pyrrol idin-1-yl)nicotinamide | white solid; MS (ESI, pos.ion) m/z: 670.2 $[M+H]^+$; $^1H$ NMR (400 MHz, $CDCl_3$) δ (ppm): 8.67 (s, 1H), 8.10 (s, 1H), 7.93 (dd, *J* = 8.9, 2.1 Hz, 1H), 7.88 (d, *J* = 8.2 Hz, 2H), 7.64 (d, *J* = 8.2 Hz, 2H), 7.49 (d, *J* = 8.5 Hz, 1H), 6.89 (d, *J* = 7.5 Hz, 1H), 6.77 (d, *J* = 9.0 Hz, 1H), 6.44 (d, *J* = 9.3 Hz, 1H), 6.24 (t, *J* = 74.8 Hz, 1H), 5.73 (s, 1H), 5.63 - 5.55 (m, 1H), 4.54 (s, 1H), 4.32 (dd, *J* = 9.1, 4.1 Hz, 1H), 3.98 (t, *J* = 9.3 Hz, 1H), 3.86 (dd, *J* = 12.0, 4.8 Hz, 1H), 3.75 (d, *J* = 12.2 Hz, 1H), 3.18 - 3.03 (m, 4H), 2.49 (d, *J* = 14.3 Hz, 1H), 2.42 - 2.37 (m, 1H), 1.27 (t, *J* = 7.4 Hz, 3H). |

(continued)

| Example No. | Target compound structure and name | Target compound characterization data |
|---|---|---|
| **Example 19** | 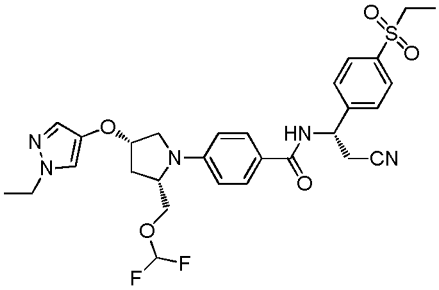 *N*-((*S*)-2-cyano-1-(4-(ethylsulfonyl) phenyl)ethyl)-4-((2S,4S)-2-((difluor omethoxy)methyl)-4-((1-ethyl-1H-pyrazol-4-yl)oxy)pyrrolidin-1-yl)be nzamide | white solid; MS (ESI, pos.ion) m/z: 602.2 $[M+H]^+$; $^1H$ NMR (400 MHz, $CDCl_3$) δ (ppm): 7.94 (d, *J* = 8.3 Hz, 2H), 7.76 (d, *J* = 8.8 Hz, 2H), 7.67 (d, *J* = 8.3 Hz, 2H), 7.26 (s, 1H), 7.15 (s, 1H), 6.74 (d, *J* = 7.5 Hz, 1H), 6.66 (d, *J* = 8.8 Hz, 2H), 6.26 (t, *J* = 74.5 Hz, 1H), 5.61 (dd, *J* = 12.6, 6.0 Hz, 1H), 4.80 (t, *J* = 4.6 Hz, 1H), 4.18 - 4.19 (m, 4H), 3.98 (t, *J* = 10.7 Hz, 1H), 3.79 (d, *J* = 11.4 Hz, 1H), 3.58 (dd, *J* = 11.4, 4.8 Hz, 1H), 3.20 - 3.05 (m, 4H), 2.54 (d, *J* = 14.3 Hz, 1H), 2.31 - 2.21 (m, 1H), 1.49 (t, *J* = 7.3 Hz, 3H), 1.31 (t, *J* = 7.4 Hz, 3H). |
| **Example 20** | 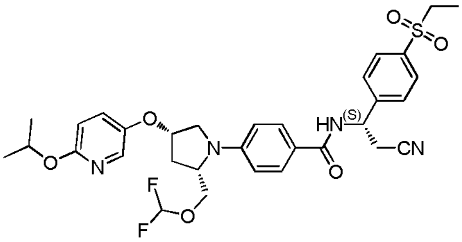 *N*-((*S*)-2-cyano-1-(4-(ethylsulfonyl) phenyl)ethyl)-4-((2*S*,4*S*)-2-((difluor omethoxy)methyl)-4-((6-isopropox ypyridin-3-yl)oxy)pyrrolidin-1-yl)b enzamide | white solid; MS (ESI, pos.ion) m/z: 643.2 $[M+H]^+$; $^1H$ NMR (400 MHz, $CDCl_3$) δ (ppm): 7.93 (d, *J* = 7.9 Hz, 2H), 7.80 (s, 1H), 7.74 (d, *J* = 8.2 Hz, 2H), 7.65 (d, *J* = 7.7 Hz, 2H), 7.20 (d, *J* = 8.8 Hz, 1H), 6.65 (d, *J* = 7.7 Hz, 4H), 6.23 (t, *J* = 74.3 Hz, 1H), 5.59 (d, *J* = 5.3 Hz, 1H), 5.25 - 5.14 (m, 1H), 5.01 (s, 1H), 4.17 (d, *J* = 9.4 Hz, 2H), 4.00 (d, *J* = 9.8 Hz, 1H), 3.75 (d, *J* = 10.9 Hz, 1H), 3.63 (d, *J* = 10.6 Hz, 1H), 3.20 - 3.02 (m, 4H), 2.51 (d, *J* = 14.3 Hz, 1H), 2.32 (s, 1H), 1.33 (d, *J* = 5.8 Hz, 6H), 1.28 (t, *J* = 7.4 Hz, 3H). |
| **Example 21** | 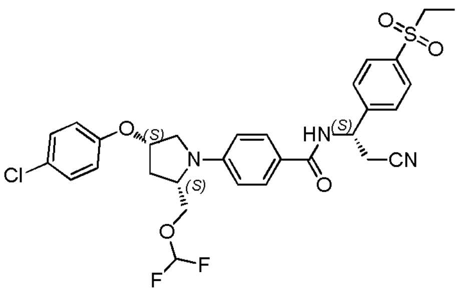 4-((2S)-4-(4-chlorophenoxy)-2-((dif luoromethoxy)methyl)pyrrolidin-1-yl)-*N*-((*S*)-2-cyano-1-(4-(ethylsulfo nyl)phenyl)ethyl)benzamide | white solid; MS (ESI, pos.ion) m/z: 618.2 $[M+H]^+$; $^1H$ NMR (400 MHz, $CDCl_3$) δ (ppm): 7.85 (d, *J* = 8.2 Hz, 2H), 7.76 (d, *J* = 8.8 Hz, 2H), 7.63 (d, *J* = 8.2 Hz, 2H), 7.27 (d, *J* = 8.8 Hz, 3H), 6.84 (d, *J* = 8.9 Hz, 2H), 6.62 (d, *J* = 8.7 Hz, 2H), 6.23 (t, *J* = 74.5 Hz, 1H), 5.59 (dd, *J* = 13.7, 6.5 Hz, 1H), 5.07 (t, *J* = 4.5 Hz, 1H), 4.17 (qd, *J* = 8.4, 4.4 Hz, 2H), 3.96 (t, *J* = 9.1 Hz, 1H), 3.67 (dt, *J* = 11.3, 8.0 Hz, 2H), 3.17 - 3.02 (m, 4H), 2.48 (d, *J* = 14.3 Hz, 1H), 2.40 - 2.28 (m, 1H), 1.26 (t, *J* = 7.4 Hz, 3H). |

(continued)

| Example No. | Target compound structure and name | Target compound characterization data |
|---|---|---|
| **Example 22** | 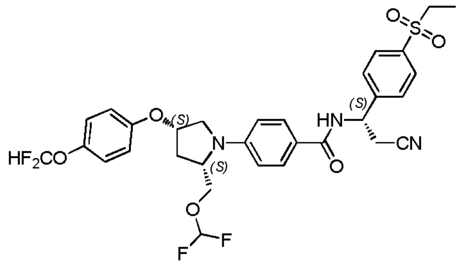 *N*-((*S*)-2-cyano-1-(4-(ethylsulfonyl) phenyl)ethyl)-4-((2*S*,4*S*)-2-((difluor omethoxy)methyl)-4-(4-(difluorom ethoxy)phenoxy)pyrrolidin-1-yl)be nzamide | white solid; MS (ESI, pos.ion) m/z: 650.2 $[M+H]^+$; $^1H$ NMR (400 MHz, DMSO-$d_6$) δ (ppm): 8.91 (d, *J* = 8.2 Hz, 1H), 7.89 (d, *J* = 8.3 Hz, 2H), 7.81 (d, *J* = 8.6 Hz, 2H), 7.72 (d, *J* = 8.3 Hz, 2H), 7.16 (d, *J* = 8.9 Hz, 2H), 7.11 (t, *J* = 74.4 Hz, 1H), 7.04 (d, *J* = 9.0 Hz, 2H), 6.74 (d, *J* = 8.8 Hz, 2H), 6.72 (t, *J* = 76 Hz, 1H), 5.51 (dd, *J* = 15.4, 8.0 Hz, 1H), 5.22 (s, 1H), 4.22 (dd, *J* = 13.0, 8.1 Hz, 1H), 4.08 (dd, *J* = 9.8, 4.7 Hz, 1H), 3.90 (t, *J* = 9.2 Hz, 1H), 3.64 (d, *J* = 11.3 Hz, 2H), 3.28 (q, *J* = 7.3 Hz, 2H), 3.18 - 3.12 (m, 2H), 2.48 - 2.39 (m, 1H), 2.24 (d, *J* = 14.2 Hz, 1H), 1.10 (t, *J* = 7.3 Hz, 3H). |
| **Example 23** | 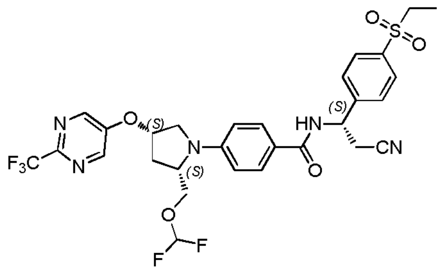 *N*-((*S*)-2-cyano-1-(4-(ethylsulfonyl) phenyl)ethyl)-4-((2*S*,4*S*)-2-((difluor omethoxy)methyl)-4-((2-(trifluoro methyl)pyrimidin-5-yl)oxy)pyrrolid in-1-yl)benzamide | yellow solid; MS (ESI, pos.ion) m/z: 654.2 $[M+H]^+$; $^1H$ NMR (400 MHz, DMSO-$d_6$) δ (ppm): 8.92 (d, *J* = 8.2 Hz, 1H), 8.85 (s, 2H), 7.89 (d, *J* = 8.3 Hz, 2H), 7.81 (d, *J* = 8.7 Hz, 2H), 7.72 (d, *J* = 8.3 Hz, 2H),, 6.76 (d, *J* = 8.8 Hz, 2H), 6.72 (t, *J* = 76 Hz, 1H), 5.55 (s, 1H), 5.52 - 5.46 (m, 1H), 4.27 (dd, *J* = 13.1, 8.3 Hz, 1H), 4.07 (dd, *J* = 10.0, 4.6 Hz, 1H), 3.90 (t, *J* = 9.2 Hz, 1H), 3.80 (d, *J* = 12.1 Hz, 1H), 3.74 (dd, *J* = 12.0, 4.4 Hz, 1H), 3.46 (d, *J* = 18.5 Hz, 1H), 3.26 (d, *J* = 7.3 Hz, 2H), 3.18 - 3.12 (m, 2H), 2.32 (d, *J* = 14.6 Hz, 1H), 1.09 (t, *J* = 7.3 Hz, 3H). |
| **Example 24** | 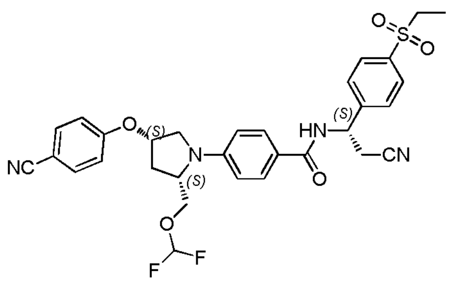 *N*-((*S*)-2-cyano-1-(4-(ethylsulfonyl) phenyl)ethyl)-4-((2*S*,4*S*)-4-(4-cyan ophenoxy)-2-((difluoromethoxy)me thyl)pyrrolidin-1-yl)benzamide | white solid; MS (ESI, pos.ion) m/z: 609.1 $[M+H]^+$; $^1H$ NMR (400 MHz, DMSO-$d_6$) δ (ppm): 8.91 (d, *J* = 8.2 Hz, 1H), 7.89 (d, *J* = 8.3 Hz, 2H), 7.82 (d, *J* = 8.7 Hz, 4H), 7.72 (d, *J* = 8.3 Hz, 2H), 7.19 (d, *J* = 8.8 Hz, 2H), 6.75 (d, *J* = 8.8 Hz, 2H), 6.71 (t, *J* = 76 Hz, 1H), 5.51 (dd, *J* = 15.2, 7.9 Hz, 1H), 5.37 (s, 1H), 4.24 (dd, *J* = 12.8, 8.0 Hz, 1H), 4.07 (dd, *J* = 9.8, 4.6 Hz, 1H), 3.87 (t, *J* = 9.3 Hz, 1H), 3.76 - 3.64 (m, 2H), 3.28 (dd, *J* = 14.7, 7.4 Hz, 2H), 3.20 - 3.11 (m, 2H), 2.47 (d, *J* = 7.8 Hz, 1H), 2.25 (d, *J* = 14.3 Hz, 1H), 1.10 (t, *J* = 7.3 Hz, 3H). |

(continued)

| Example No. | Target compound structure and name | Target compound characterization data |
|---|---|---|
| Example 25 | 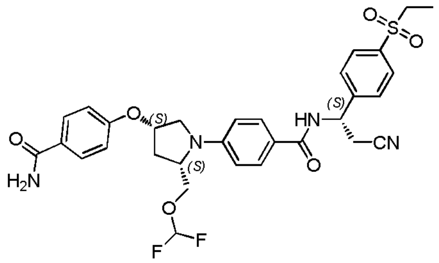 4-((2*S*,4*S*)-4-(4-carbamoylphenoxy) -2-((difluoromethoxy)methyl)pyrrol idin-1-yl)-*N*-((*S*)-2-cyano-1-(4-(eth ylsulfonyl)phenyl)ethyl)benzamide | white solid; MS (ESI, pos.ion) m/z: 627.2 $[M+H]^+$; $^1H$ NMR (400 MHz, DMSO-$d_6$) δ (ppm): 8.91 (d, *J* = 8.3 Hz, 1H), 7.88 (dd, *J* = 8.1,5.1 Hz, 5H), 7.81 (d, *J* = 8.5 Hz, 2H), 7.72 (d, *J* = 8.2 Hz, 2H), 7.21 (s, 1H), 7.04 (d, *J* = 8.7 Hz, 2H), 6.74 (d, *J* = 8.7 Hz, 2H), 6.72 (t, *J* = 76 Hz, 1H), 5.50 (dd, *J* = 15.5, 7.9 Hz, 1H), 5.32 (s, 1H), 4.23 (d, *J* = 4.7 Hz, 1H), 4.08 (dd, *J* = 9.7, 4.5 Hz, 1H), 3.89 (t, *J* = 9.2 Hz, 1H), 3.68 (q, *J* = 12.2 Hz, 2H), 3.28 (dd, *J* = 14.6, 7.3 Hz, 2H), 3.16 (s, 1H), 3.14 (s, 1H), 2.46 (d, *J* = 5.8 Hz, 1H), 2.26 (d, *J* = 14.3 Hz, 1H), 1.09 (t, *J* = 7.3 Hz, 3H). |
| Example 26 | 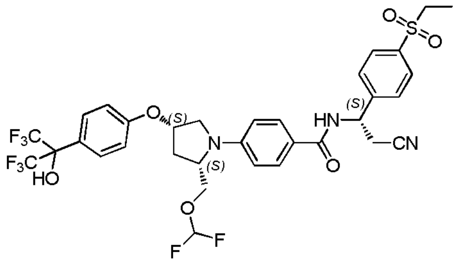 *N*-((*S*)-2-cyano-1-(4-(ethylsulfonyl) phenyl)ethyl)-4-((2*S*,4*S*)-2-((difluor omethoxy)methyl)-4-(4-(1,1,1,3,3,3 -hexafluoro-2-hydroxypropan-2-yl) phenoxy)pyrrolidin-1-yl)benzamide | white solid; MS (ESI, neg.ion) m/z:748.1 $[M-H]^-$; $^1H$ NMR (600 MHz, DMSO-$d_6$) δ (ppm): 8.92 (d, *J* = 8.2 Hz, 1H), 8.63 (s, 1H), 7.89 (d, *J* = 8.1 Hz, 2H), 7.81 (d, *J* = 8.4 Hz, 2H), 7.72 (d, *J* = 8.1 Hz, 2H), 7.63 (d, *J* = 8.5 Hz, 2H), 7.14 (d, *J* = 8.8 Hz, 2H), 6.76 (d, *J* = 8.6 Hz, 2H), 6.72 (t, *J* = 76 Hz, 1H)., 5.51 (dd, *J* = 15.2, 8.1 Hz, 1H), 5.29 (s, 1H), 4.24 (d, *J* = 4.8 Hz, 1H), 4.08 (dd, *J* = 9.8, 4.7 Hz, 1H), 3.91 (t, *J* = 9.2 Hz, 1H), 3.70 (dt, *J* = 23.4, 8.0 Hz, 2H), 3.29 (dd, *J* = 14.6, 7.3 Hz, 2H), 3.19 - 3.11 (m, 2H), 2.49 - 2.44 (m, 1H), 2.26 (d, *J* = 14.2 Hz, 1H), 1.10 (t, *J* = 7.3 Hz, 3H). |
| Example 27 | 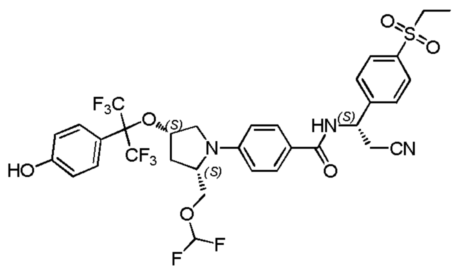 *N*-((*S*)-2-cyano-1-(4-(ethylsulfonyl) phenyl)ethyl)-4-((2S,4S)-2-((difluor omethoxy)methyl)-4-((1,1,1,3,3,3-h exafluoro-2-(4-hydroxyphenyl)prop an-2-yl)oxy)pyrrolidin-1-yl)benzam ide | white solid; MS (ESI,pos.ion) m/z:750.1 $[M+H]^+$; $^1H$ NMR (600 MHz, DMSO-$d_6$) δ (ppm): 10.18 (s, 1H), 8.92 (d, *J* = 8.2 Hz, 1H), 7.90 (d, *J* = 8.3 Hz, 2H), 7.81 (d, *J* = 8.7 Hz, 2H), 7.72 (d, *J* = 8.3 Hz, 2H), 7.48 (d, *J* = 8.3 Hz, 2H), 6.98 (d, *J* = 8.8 Hz, 2H), 6.75 (s, 2H), 6.74 (t, *J* = 76 Hz, 1H), 5.51 (dd, *J* = 15.1, 8.3 Hz, 1H), 4.49 (s, 1H), 4.16 (s, 1H), 4.09 (dd, *J* = 9.6, 4.2 Hz, 1H), 3.95 (t, *J* = 9.1 Hz, 1H), 3.69 (d, *J* = 11.1 Hz, 1H), 3.55 (dd, *J* = 11.5, 5.2 Hz, 1H), 3.29 (dd, *J* = 14.7, 7.3 Hz, 2H), 3.16 (dd, *J* = 12.9, 8.9 Hz, 2H), 2.28 (dd, *J* = 21.8, 10.0 Hz, 2H), 1.10 (t, *J* = 7.3 Hz, 3H). |

(continued)

| Example No. | Target compound structure and name | Target compound characterization data |
|---|---|---|
| **Example 28** | 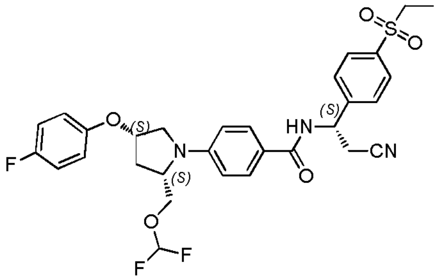 *N*-((*S*)-2-cyano-1-(4-(ethylsulfonyl) phenyl)ethyl)-4-((2*S*,4*S*)-2-((difluor omethoxy)methyl)-4-(4-fluorophen oxy)pyrrolidin-1-yl)benzamide | white solid; MS (ESI, pos.ion) m/z: 602.5 $[M+H]^+$; $^1H$ NMR (600 MHz, DMSO-$d_6$) $\delta$ (ppm): 8.91 (d, *J* = 7.8 Hz, 1H), 7.89 (d, *J* = 7.8 Hz, 2H), 7.81 (d, *J* = 7.6 Hz, 2H), 7.72 (d, *J* = 7.6 Hz, 2H), 7.16 (t, *J* = 8.5 Hz, 2H), 7.07 - 6.98 (m, 2H), 6.74 (d, *J* = 8.4 Hz, 2H), 6.72 (t, *J* = 76 Hz, 1H), 5.51 (d, *J* = 6.9 Hz, 1H), 5.20 (s, 1H), 4.21 (d, *J* = 4.5 Hz, 1H), 4.13 - 4.06 (m, 1H), 3.91 (t, *J* = 9.0 Hz, 1H), 3.65 (s, 2H), 3.28 (dd, *J* = 14.4, 7.1 Hz, 2H), 3.16 (s, 2H), 2.46 - 2.38 (m, 1H), 2.24 (d, *J* = 14.1 Hz, 1H), 1.10 (t, *J* = 7.2 Hz, 3H). |
| **Example 29** | 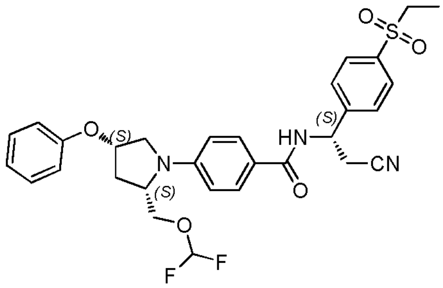 *N*-((*S*)-2-cyano-1-(4-(ethylsulfonyl) phenyl)ethyl)-4-((2*S*,4*S*)-2-((difluor omethoxy)methyl)-4-phenoxypyrro 1-1-yl)benzamide | white solid; MS (ESI, pos.ion) m/z:584.2 $[M+H]^+$; $^1H$ NMR (600 MHz, DMSO-$d_6$) $\delta$ (ppm): 8.92 (d, *J* = 7.6 Hz, 1H), 7.90 (d, *J* = 7.4 Hz, 2H), 7.82 (d, *J* = 7.6 Hz, 2H), 7.73 (d, *J* = 7.3 Hz, 2H), 7.34 (t, *J* = 6.9 Hz, 2H), 7.00 (d, *J* = 6.9 Hz, 3H), 6.75 (d, *J* = 7.9 Hz, 2H), 6.72 (t, *J* = 76 Hz, 1H), 5.51 (d, *J* = 6.5 Hz, 1H), 5.24 (s, 1H), 4.22 (d, *J* = 3.3 Hz, 1H), 4.14 - 4.06 (m, 1H), 3.92 (t, *J* = 8.8 Hz, 1H), 3.66 (s, 2H), 3.29 (d, *J* = 7.0 Hz, 2H), 3.16 (s, 2H), 2.43 (d, *J* = 5.3 Hz, 1H), 2.26 (d, *J* = 14.1 Hz, 1H), 1.10 (t, *J* = 6.7 Hz, 3H). |

**Example 30 *N*-((*S*)-2-cyano-1-(4-(ethylsulfonyl)phenyl)ethyl)-4-((2*S*,4*S*)-4-((4,4-difluorocyclohexane-1-yl)carboxamido)-2-((difluoromethoxy)methyl)pyrrolidin-1-yl) benzamide**

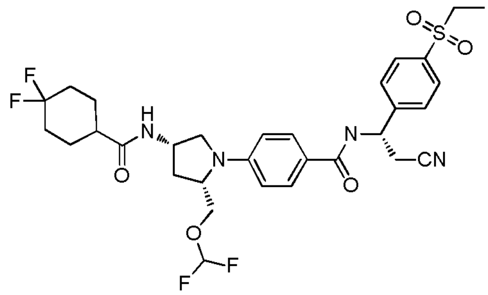

**Step 1: Synthesis of (2*S*,4*S*)-1-*tert*-butyl 2-methyl 4-(((benzyloxy)carbonyl)amino)pyrrolidine-1,2-dicarboxylate**

Saturated $NaHCO_3$ solution (25 mL) was added to a solution of (2*S*,4*S*)-1-*tert*-butyl 2-methyl 4-aminopyrrolidine-1,2-dicarboxylate hydrochloride (3.00 g, 10.69 mmol) in THF (25 mL), then the mixture was transferred to 0 °C, and CbzCl (2.0 mL, 14.21 mmol) was slowly added. After the addition was completed, the mixture was transferred to room temperature and stirred for 12 h. The reaction solution was left standing for layers, the upper organic phase was separated and concentrated under reduced pressure, the aqueous phase was extracted with EtOAc (30 mL × 2) and the combined organic phase was dissolved, washed with saturated NaCl solution (20 mL), dried over anhydrous $Na_2SO_4$, concentrated

under reduced pressure to give pale yellow liquid (3.92 g, 100%).
MS (ESI, pos.ion) m/z: 323.1 $[M-56+H]^+$.

**Step 2: Synthesis of *tert*-butyl (2*S*,4*S*)-4-(((benzyloxy)carbonyl)amino) -2-(hydroxymethyl)pyrrolidine-1-carboxylate**

To THF (30 mL) solution was added (2*S*,4*S*)-1-*tert*-butyl 2-methyl 4-(((benzyloxy)carbonyl)amino)pyrrolidine-1,2-dicarboxylate (3.92 g, 10.36 mmol), then the mixture was transferred to 0 °C, and $LiBH_4$ (400 mg, 11.02 mmol) was added slowly. After the addition was completed, the mixture was stirred at room temperature for 16 h. Saturated $NH_4Cl$ (40 mL) solution was added to the reaction solution to quench the reaction. The mixture was left standing for layers, the upper organic phase was separated, the aqueous phase was extracted with EtOAc (30 mL × 2). The combined organic phases were washed with saturated NaCl solution (20 mL), dried over anhydrous $Na_2SO_4$, concentrated under reduced pressure, and the concentrated solution was separated by silica gel column chromatography (eluent: DCM/EtOAc (v/v) = 4/1) to give pale yellow liquid (3.63 g, 100%).
MS (ESI, pos.ion) m/z: 295.4 $[M-56+H]^+$.

**Step 3: Synthesis of *tert*-butyl (2*S*,4*S*)-4-(((benzyloxy)carbonyl)amino)-2-((difluoromethoxy)methyl)pyrrolidine-1-carboxylate**

To a solution of *tert*-butyl (2*S*,4*S*)-4-(((benzyloxy)carbonyl)amino)-2-(hydroxymethyl)pyrrolidine-1-carboxylate (3.60 g, 10.27 mmol) in DCM (8 mL) were sequentially added $H_2O$ (8 mL), KOAc (5.04 g, 51.40 mmol) and $TMSCF_2Br$ (4.0 mL, 25.72 mmol). The mixture was stirred at room temperature for 24 h. The reaction solution was diluted with DCM (50 mL), washed with saturated NaCl solution (20 mL × 2), dried over anhydrous $Na_2SO_4$, concentrated under reduced pressure, and the concentrated solution was separated by silica gel column chromatography (eluent: PE/EtOAc (v/v) = 4/1) to give pale yellow liquid (2.79 mg, 68%).
MS (ESI, pos.ion) m/z: 345.1 $[M-56+H]^+$.

**Step 4: Synthesis of benzyl ((3*S*,5*S*)-5-((difluoromethoxy)methyl)pyrrolidin-3-yl)carbamate**

To a solution of *tert*-butyl (2*S*,4*S*)-4-(((benzyloxy)carbonyl)oxy)-2-((difluoromethoxy) methyl)pyrrolidine-1-carboxylate (1.50 mg, 3.75 mmol) in DCM (4 mL) was added a solution of HCl in 1,4-dioxane (8 mL, 4 mol/L). The mixture was stirred at room temperature for 4 h. The reaction solution was concentrated under reduced pressure. The concentrated solution was diluted with DCM (20 mL), washed successively with $NaHCO_3$ solution (10 mL, 1 mol/L) and saturated NaCl solution (10 mL), dried over anhydrous $Na_2SO_4$, concentrated under reduced pressure, and the concentrated solution was separated by silica gel column chromatography (eluent: DCM/MeOH (v/v) = 10/1) to give brown liquid (950 mg, 84%).
MS (ESI, pos.ion) m/z: 301.2 $[M+H]^+$.

**Step 5: Synthesis of ethyl 4-((2*S*,4*S*)-4-(((benzyloxy)carbonyl)amino)-2-((difluoromethoxy) methyl)pyrrolidin-1-yl)benzoate**

Under nitrogen protection, benzyl ((3*S*,5*S*)-5-((difluoromethoxy)methyl)pyrrolidin-3-yl)carbamate (900 mg, 3.00 mmol), $Pd_2(dba)_3$ (274 mg, 0.30 mmol), 2-bicyclohexylphosphine-2',6'-diisopropoxybiphenyl (210 mg, 0.45 mmol), $CS_2CO_3$ (1.20 g, 3.68 mmol), ethyl 4-iodobenzoate (930 mg, 3.30 mmol) were successively added to 1,4-dioxane (16 mL) and the mixture was reacted at 100°C for 14 h. The reaction solution was cooled to room temperature, filtered through a celite pad. The filtrate was concentrated under reduced pressure, and the concentrated solution was separated by silica gel column chromatography (eluent: PE/EtOAc (v/v) = 4/1) to give yellow liquid (1.30 g, 97%).
MS (ESI, pos.ion) m/z: 449.2 $[M+H]^+$.

**Step 6: Synthesis of 4-((2*S*,4*S*)-4-(((benzyloxy)carbonyl)amino)-2-((difluoromethoxy) methyl)pyrrolidin-1-yl)benzoic acid**

To a solution of ethyl 4-((2*S*,4*S*)-4-(((benzyloxy)carbonyl)amino)-2-((difluoromethoxy)methyl)pyrrolidin-1-yl)benzoate (1.30 g, 2.90 mmol) in MeOH (6 mL) and THF (6 mL) was added a solution of $LiOH{\cdot}H_2O$ (1.22 g, 29.08 mmol) in $H_2O$ (8 mL). The mixture was stirred at room temperature for 16 h. The reaction solution was concentrated under reduced pressure, HCl solution (1 mol/L) was added to the concentrated solution to adjust the pH to about 4. The resulting mixture was extracted with EtOAc (30 mL), washed with saturated NaCl (10 mL) solution, dried over anhydrous $Na_2SO_4$, concentrated under reduced pressure, and the concentrated solution was separated by silica gel column

chromatography (eluent: DCM/EtOAc (v/v) = 4/1) to give a pale yellow solid (620 mg, 51%).
MS (ESI, pos.ion) m/z: 421.1 $[M+H]^+$.

**Step 7: Synthesis of benzyl ((3*S*,5*S*)-1-(4-(((*S*)-2-cyano-1-(4-(ethylsulfonyl)phenyl)ethyl) carbamoyl)phenyl)-5-((difluoromethoxy)methyl)pyrrolidin-3-yl)carbamate**

HATU (705 mg, 1.85 mmol), (*S*)-3-amino-3-(4-(ethylsulfonyl)phenyl)propionitrile hydrochloride (470 mg, 1.71 mmol), 4-((2*S*,4*S*)-4-(((benzyloxy)carbonyl)amino)-2-((difluoromethoxy)methyl) pyrrolidin-1-yl)benzoic acid (600 mg, 1.43 mmol) and TEA (0.6 mL, 4.32 mmol) were successively added to DCM (8 mL) and the mixture was stirred at room temperature for 16 h. The reaction solution was concentrated under reduced pressure, the concentrated solution was diluted with DCM (40 mL), washed successively with HCl solution (20 mL, 1 mol/L), saturated $NaHCO_3$ solution (15 mL) and saturated NaCl solution (15 mL), dried over anhydrous $Na_2SO_4$, concentrated under reduced pressure, and the concentrated solution was separated by silica gel column chromatography (eluent: DCM/EtOAc (v/v) = 4/1) to give pale yellow liquid (900 mg, 98%).
MS (ESI, pos.ion) m/z: 641.3 $[M+H]^+$.

**Step 8: Synthesis of 4-((2*S*,4*S*)-4-amino-2-((difluoromethoxy)methyl)pyrrolidin-1-yl) -*N*-((*S*)-2-cyano-1-(ethylsulfonyl)phenyl)ethyl)benzamide**

To a solution of benzyl ((3*S*,5*S*)-1-(4-(((*S*)-2-cyano-1-(4-(ethylsulfonyl)phenyl)ethyl) carbamoyl)phenyl)-5-((difluoromethoxy)methyl)pyrrolidin-3-yl)carbamate (900 mg, 1.41 mmol) in MeOH (8 mL) was added Pd/C (80 mg, 10%). The mixture was degassed and refilled with hydrogen, and was stirred at room temperature for 12 h. The reaction solution was filtered through a celite pad, the filtrate was concentrated under reduced pressure, and the concentrated solution was separated by silica gel column chromatography (eluent: DCM/MeOH (v/v) = 10/1) to give a white solid (489 mg, 69%).
MS (ESI, pos.ion) m/z: 507.2 $[M+H]^+$.

**Step 9: Synthesis of *N*-((*S*)-2-cyano-1-(4-(ethylsulfonyl)phenyl)ethyl)-4-((2*S*,4*S*)-4-((4,4-difluorocyclohexane-1-yl)carboxamido)-2-((difluoromethoxy)methyl)pyrrolidin-1-yl)ben zamide**

HATU (211 mg, 0.55 mmol), 4,4-difluorocyclohexylcarboxylic acid (76 mg, 0.46 mmol), 4-((2S,4,S)-4-amino-2-((difluoromethoxy)methyl)pyrrolidin-1-yl)-*N*-((*S*)-2-cyano -1-(ethylsulfonyl)phenyl)ethyl)benzamide (233 mg, 0.46 mmol) and TEA(140 mg, 1.38 mmol) were successively added to DCM (6 mL) and the mixture was stirred at room temperature for 16 h. The reaction solution was concentrated under reduced pressure. The residue was diluted with DCM (40 mL), washed successively with HCl solution (15 mL, 1 mol/L), saturated $NaHCO_3$ solution (15 mL) and saturated NaCl solution (15 mL), dried over anhydrous $Na_2SO_4$, concentrated under reduced pressure, and the concentrated solution was separated by silica gel column chromatography (eluent: DCM/EtOAc (v/v) = 1/3) to give a white solid (250 mg, 83%).

MS (ESI, pos.ion) m/z: 653.2 $[M+H]^+$.
$^1H$ NMR (600 MHz, DMSO-$d_6$) $\delta$ (ppm): 8.91 (d, *J* = 8.2 Hz, 1H), 8.21 (d, *J* = 5.9 Hz, 1H), 7.88 (d, *J* = 8.2 Hz, 2H), 7.79 (d, *J* = 8.7 Hz, 2H), 7.71 (d, *J* = 8.3 Hz, 2H), 6.70 (t, *J* = 75.2, 1H), 6.71 (d, *J* = 8.7 Hz, 2H), 5.49 (dd, *J* = 15.3, 7.9 Hz, 1H), 4.28 (d, *J* = 5.3 Hz, 1H), 4.12 (s, 1H), 3.99 (dd, *J* = 9.9, 3.3 Hz, 1H), 3.92 - 3.85 (m, 1H), 3.59 (dd, *J* = 10.2, 7.1 Hz, 1H), 3.28 (dd, *J* = 14.9, 7.4 Hz, 3H), 3.14 (d, *J* = 8.5 Hz, 2H), 2.39 - 2.24 (m, 2H), 2.09 - 1.97 (m, 3H), 1.88 - 1.73 (m, 4H), 1.67 - 1.59 (m, 2H), 1.09 (t, *J* = 7.3 Hz, 3H).

**Example 31 *N*-((*S*)-2-cyano-1-(4-(ethylsulfonyl)phenyl)ethyl)-4-((2*S*,4*S*)-4-((4,4-difluorocyclohexyl) methyl)amino)-2-((difluoromethoxy)methyl)pyrrolidin-1-yl)benzamide**

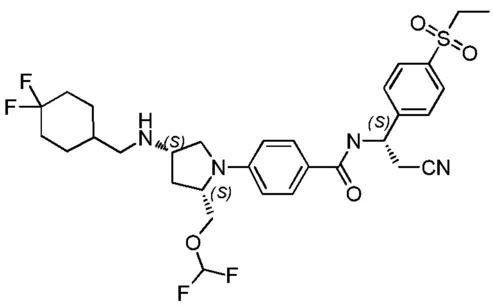

AcOH (225 mg, 3.75 mmol), 4,4-difluorocyclohexylcarbaldehyde (139 mg, 0.94 mmol) and 4-((2*S*,4*S*)-4-amino-2-((difluoromethoxy((*S*)-2-cyano-1 -(ethylsulfonyl)phenyl)ethyl)benzamide (426 mg, 0.94 mmol) were successively added to MeOH (6 mL). After the mixture was stirred at room temperature for 1 h, $NaBH_3CN$ (177 mg, 2.82 mmol) was added, and the mixture was stirred at room temperature for 16 h. The reaction solution was concentrated under reduced pressure. The concentrated solution was diluted with DCM (30 mL), washed successively with $NaHCO_3$ solution (15 mL) and saturated NaCl solution (15 mL), dried over anhydrous $Na_2SO_4$, concentrated under reduced pressure, and the concentrated solution was separated by silica gel column chromatography (eluent: DCM/EtOAc (v/v) = 3/2) to give a white solid (576 mg, 96%).

MS (ESI, pos.ion) m/z: 639.2 $[M+H]^+$.

$^1H$ NMR (400 MHz, $CDCl_3$) δ (ppm): 7.92 (d, *J* = 8.3 Hz, 2H), 7.72 (d, *J* = 8.8 Hz, 2H), 7.65 (d, *J* = 8.3 Hz, 2H), 6.67 (d, *J* = 7.7 Hz, 1H), 6.61 (d, *J* = 8.8 Hz, 2H), 6.22 (t, *J* = 74.6 Hz, 1H), 5.59 (dd, *J* = 12.7, 6.2 Hz, 1H), 4.15 - 4.04 (m, 3H), 3.55 - 3.46 (m, 2H), 3.28 (d, *J* = 7.9 Hz, 1H), 3.18 - 3.01 (m, 4H), 2.60 - 2.49 (m, 2H), 2.29 - 2.21 (m, 1H), 2.15 - 2.12 (t, *J* = 13.0 Hz, 3H), 1.84 (d, *J* = 11.0 Hz, 2H), 1.79 - 1.69 (m, 1H), 1.67 - 1.61 (m, 1H), 1.28 (t, *J* = 7.5 Hz, 6H).

**Example 32 *N*-((*S*)-2-cyano-1-(4-(ethylsulfonyl)phenyl)ethyl)-4-((2*S*,4*S*)-4-((4,4-difluorocyclohexyl)methyl)(methyl)amino)-2-((difluoromethoxy)methyl)pyrrolidin-1-yl) benzamide**

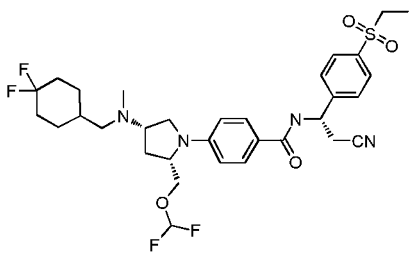

AcOH (40 mg, 0.67 mmol), paraformaldehyde (20 mg, 0.67 mmol) and *N*-((*S*)-2-cyano-1-(4-(ethylsulfonyl)phenyl)ethyl)-4-((2*S*,4*S*)-4-(((4,4-difluorocyclohexyl)methyl)am ino)-2-((difluoromethoxy)methyl)pyrrolidin-1-yl)benzamide (100mg, 0,16mmol) were successively added to MeOH (4 mL). After the mixture was stirred at room temperature for 1 h, $NaBH_3CN$ (50 mg, 0.80 mmol) was added, and the mixture was stirred at room temperature for 16 h. The reaction solution was concentrated under reduced pressure. The residue was diluted with DCM (30 mL), washed successively with $NaHCO_3$ solution (15 mL) and saturated NaCl solution (15 mL), dried over anhydrous $Na_2SO_4$, concentrated under reduced pressure, and the crude product was separated by silica gel column chromatography (eluent: DCM/EtOAc (v/v) = 1/1) to give a pale yellow solid (75 mg,73%).

MS (ESI, pos.ion) m/z: 653.3 $[M+H]^+$.

$^1H$ NMR (400 MHz, $CDCl_3$) δ (ppm): 7.91 (d, *J* = 8.3 Hz, 2H), 7.71 (d, *J* = 8.8 Hz, 2H), 7.64 (d, *J* = 8.3 Hz, 2H), 6.70 (d, *J* = 7.4 Hz, 1H), 6.61 (d, *J* = 8.8 Hz, 2H), 6.19 (t, *J* = 74.4 Hz, 1H), 5.59 (dd, *J* = 12.8, 6.2 Hz, 1H), 4.16 - 4.04 (m, 2H), 3.86 (dd, *J* = 9.8, 7.1 Hz, 1H), 3.55 (dd, *J* = 9.3, 6.9 Hz, 1H), 3.36 - 3.27 (m, 1H), 3.18 - 3.01 (m, 4H), 2.96 - 2.86 (m, 1H), 2.41 - 2.34 (m, 1H), 2.29 - 2.22 (m, 4H), 2.18 (dd, *J* = 12.4, 7.3 Hz, 1H), 2.14 - 2.05 (m, 2H), 2.04 - 1.96 (m, 1H), 1.86 (s, 2H), 1.79 - 1.63 (m, 5H), 1.27 (d, *J* = 7.5 Hz, 3H).

**Example 33 *N*-((*S*)-2-cyano-1-(4-(ethylsulfonyl)phenyl)ethyl)-4-((2*S*,4*S*)-2-((difluoromethoxy)methyl)-4-((((1*r*,4*S*)-4-(trifluoromethyl)cyclohexyl)methyl)amino) pyrrolidin-1-yl)benzamide**

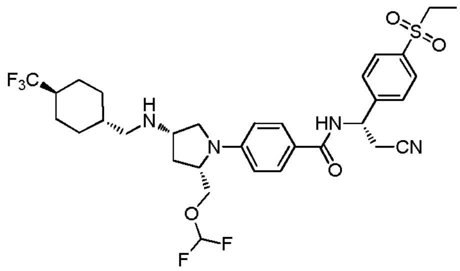

AcOH (170 mg, 2.83 mmol), (1*r*,4*r*)-4-(trifluoromethyl)cyclohexylcarbaldehyde (187 mg, 1.04 mmol) and 4-((2*S*,4*S*)-4-amino-2-((difluoromethoxy) methyl)pyrrolidin-1-yl)-*N*((*S*)-2-cyano-1-(4-(ethylsulfonyl)phenyl)ethyl)benzamide (480 mg, 0.95 mmol) were successively added to MeOH (8 mL). After the mixture was stirred at room temperature for 1 h, $NaBH_3CN$ (177 mg, 2.82 mmol) was added, and the mixture was stirred at room temperature for 16 h. The reaction solution was concentrated under reduced pressure. The concentrated solution was diluted with DCM (40 mL), washed successively with $NaHCO_3$ solution (20 mL) and saturated NaCl solution (20 mL), dried over anhydrous $Na_2SO_4$, concentrated under reduced pressure, and the concentrated solution was separated by silica gel column chromatography (eluent: DCM/MeOH (v/v) = 20/1) to give a pale yellow solid (500 mg, 79%).

MS (ESI, pos.ion) m/z: 671.2 $[M+H]^+$.
$^1$H NMR (400 MHz, $CDCl_3$) δ (ppm): 7.92 (d, *J* = 7.6 Hz, 2H), 7.71 (d, *J* = 8.9 Hz, 2H), 7.65 (d, *J* = 6.9 Hz, 2H), 6.68 (s, 1H), 6.60 (d, *J* = 7.8 Hz, 2H), 6.22 (t, *J* = 74.6 Hz, 1H), 5.59 (dd, *J* = 12.6, 6.4 Hz, 1H), 4.14 - 4.10 (m, 1H), 4.08 (dd, *J* = 10.1, 3.5 Hz, 2H), 3.53 - 3.46 (m, 2H), 3.28 (d, *J* = 9.5 Hz, 1H), 3.17 - 3.09 (m, 3H), 3.05 (dd, *J* = 17.0, 5.0 Hz, 1H), 2.53 (dd, *J* = 11.4, 6.8 Hz, 1H), 2.48 (dd, *J* = 11.4, 6.4 Hz, 1H), 2.27 - 2.21 (m, 1H), 2.03 (d, *J* = 13.4 Hz, 1H), 1.98 - 1.88 (m, 4H), 1.34 - 1.27 (m, 6H), 0.97 (q, *J* = 12.7 Hz, 2H).

**Example 34 *N*-((*S*)-2-cyano-1-(4-(ethylsulfonyl)phenyl)ethyl)-4-((2*S*,4*S*)-2-((difluoromethoxy)methyl)-4-(ethyl(((1*r*,4*S*)-4-(trifluoromethyl)cyclohexyl)methyl)amino) pyrrolidin-1-yl)benzamide**

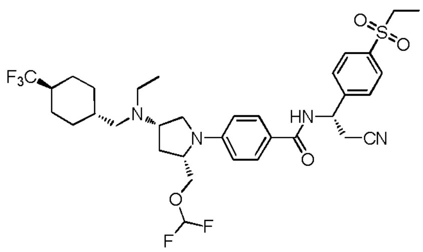

AcOH (45 mg, 0.75 mmol), aqueous acetaldehyde solution (82 mg, 0.74 mmol, 40%) and *N*-((*S*)-2-cyano-1-(4-(ethylsulfonyl)phenyl)ethyl)-4-((2*S*,4*S*)-2-((difluoromethoxy)methyl) -4-((((1*r*,4*S*)-4-(trifluoromethyl)cyclohexyl)methyl)amino)pyrrolidin-1-yl)benzamide (100 mg, 0.15 mmol) were successively added to MeOH (4 mL). After the mixture was stirred at room temperature for 1 h, $NaBH_3CN$ (50 mg, 0.80 mmol) was added, and the mixture was stirred at room temperature for 16 h. The reaction solution was concentrated under reduced pressure. The residue was diluted with DCM (30 mL), washed successively with $NaHCO_3$ solution (15 mL) and saturated NaCl solution (15 mL), dried over anhydrous $Na_2SO_4$, concentrated under reduced pressure, and the concentrated solution was separated by silica gel column chromatography (eluent: DCM/EtOAc (v/v) = 1/1) to give a white solid (60 mg, 58%).

MS (ESI, pos.ion) m/z: 699.2 $[M+H]^+$.
$^1$H NMR (400 MHz, $CDCl_3$) δ (ppm): 7.93 (d, *J* = 6.9 Hz, 2H), 7.71 (d, *J* = 8.8 Hz, 2H), 7.65 (d, *J* = 8.2 Hz, 2H), 6.61 (d, *J* = 8.6 Hz, 3H), 6.19 (t, *J* = 74.4 Hz, 1H), 5.59 (dd, *J* = 12.4, 6.3 Hz, 1H), 4.15 - 4.05 (m, 2H), 3.87 (dd, *J* = 9.7,6.9 Hz, 1H), 3.53 - 3.47 (m, 1H), 3.33 - 3.23 (m, 2H), 3.19 - 3.02 (m, 4H), 2.73 - 2.62 (m, 2H), 2.40 - 2.30 (m, 2H), 2.25

(dd, $J$ = 13.1, 6.9 Hz, 1H), 1.96 (d, $J$ = 11.6 Hz, 5H), 1.31 - 1.25 (m, 6H), 0.99 (t, $J$ = 7.0 Hz, 3H), 0.92- 0.82 (m, 3H).

**Example 35 *N*-((*S*)-2-cyano-1-(4-(ethylsulfonyl)phenyl)ethyl)-4-((2*S*,4*S*)-4-((cyclopropylmethyl) (((1*r*,4*S*)-4-(trifluoromethyl)cyclohexyl)methyl)amino)-2-((difluoromethoxy)methyl)pyrrolidin e-1-yl)benzamide**

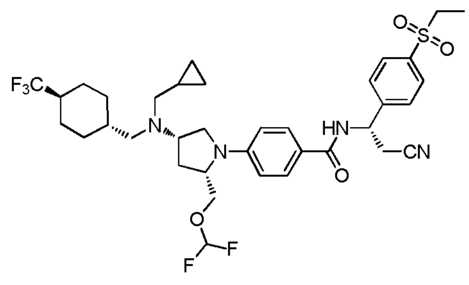

AcOH (53 mg, 0.88 mmol), cyclopropanecarbaldehyde (70 mg, 0.98 mmol) and ***N*-((*S*)-2-cyano-1-(4-(ethylsulfonyl)phenyl)ethyl)-4-((2*S*,4*S*)-2-((difluoromethoxy)methyl)-4-((((1*r*,** 4*S*)-4-(trifluoromethyl)cyclohexyl)methyl)amino)pyrrolidin-1-yl)benzamide (100 mg , 0.15 mmol) were successively added to MeOH (3 mL). After the mixture was stirred at room temperature for 1 h, $NaBH_3CN$ (50 mg, 0.80 mmol) was added, and the mixture was stirred at room temperature for 16 h. The reaction solution was concentrated under reduced pressure. The concentrated solution was diluted with DCM (30 mL), washed successively with $NaHCO_3$ solution (15 mL) and saturated NaCl solution (15 mL), dried over anhydrous $Na_2SO_4$, concentrated under reduced pressure, and the concentrated solution was separated by silica gel column chromatography (eluent: DCM/EtOAc (v/v) = 1/1) to give a white solid (60 mg, 56%).

MS (ESI, pos.ion) m/z: 725.3 $[M+H]^+$.
$^1$H NMR (400 MHz, $CDCl_3$) $\delta$ (ppm): 7.93 (t, $J$ = 7.0 Hz, 2H), 7.71 (d, $J$ = 8.7 Hz, 2H), 7.65 (d, $J$ = 6.8 Hz, 2H), 6.62 (d, $J$ = 8.3 Hz, 3H), 6.18 (t, $J$ = 76.5 Hz, 1H), 5.60 (dd, $J$ = 12.4, 6.4 Hz, 1H), 4.14 -4.09 (m, 1H), 4.07 (dd, $J$ = 10.1, 2.9 Hz, 1H), 3.87 (dd, $J$ = 10.0, 6.9 Hz, 1H), 3.56 - 3.51 (m, 1H), 3.50 - 3.44 (m, 1H), 3.30 (t, $J$ = 8.7 Hz, 1H), 3.19 - 3.10 (m, 3H), 3.06 (dd, $J$ = 16.9, 4.9 Hz, 1H), 2.51 - 2.46 (m, 2H), 2.43 (dd, $J$ = 13.3, 7.1 Hz, 1H), 2.41 - 2.34 (m, 2H), 2.00 - 1.94 (m, 5H), 1.31 - 1.25 (m, 6H), 0.92 - 0.80 (m, 4H), 0.54 - 0.48 (d, $J$ = 7.6 Hz, 2H), 0.12 - 0.06 (m, 2H).

**Example 36 *N*-((*S*)-2-cyano-1-(4-(ethylsulfonyl)phenyl)ethyl)-4-((2*S*,4*S*)-2-((difluoromethoxy) methyl)-4-(isobutyl(((1*r*,4*S*)-4-(trifluoromethyl)cyclohexyl)methyl)amino)pyrrolidin-1-yl)benz amide**

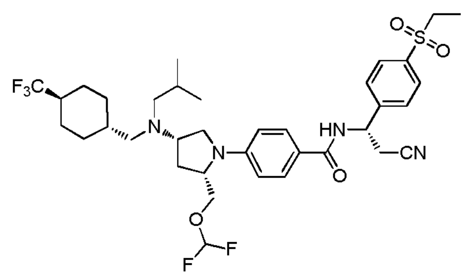

AcOH (40 mg, 0.67 mmol), isobutyraldehyde (86 mg, 0.48 mmol) and *N*-((*S*)-2-cyano-1-(4-(ethylsulfonyl)phenyl)ethyl)-4-((2*S*,4*S*)-2-((difluoromethoxy)methyl)-4-((((1*r*, 4*S*)-4-(trifluoromethyl)cyclohexyl)methyl)amino)pyrrolidin-1-yl)benzamide (80 mg, 0.12 mmol) were successively added to MeOH (4 mL). After the mixture was stirred at room temperature for 1 h, $NaBH_3CN$ (40 mg, 0.64 mmol) was added, and the mixture was stirred at room temperature for 16 h. The reaction solution was concentrated under reduced pressure. The concentrated solution was diluted with DCM (30 mL), washed successively with $NaHCO_3$ solution (15 mL) and saturated NaCl solution (15 mL), dried over anhydrous $Na_2SO_4$, concentrated under reduced pressure, and the concentrated solution was separated by silica gel column chromatography (eluent: DCM/EtOAc (v/v) = 4/1) to give a white solid (50 mg, 58%).

MS (ESI, pos.ion) m/z: 727.3 $[M+H]^+$.

$^1H$ NMR (400 MHz, $CDCl_3$) δ (ppm): 7.97 (d, *J* = 8.2 Hz, 2H), 7.73 (d, *J* = 8.7 Hz, 2H), 7.68 (d, *J* = 8.2 Hz, 2H), 6.64 (d, *J* = 8.7 Hz, 2H), 6.59 (d, *J* = 7.0 Hz, 1H), 6.20 (t, *J* = 74.4 Hz, 1H), 5.62 (dd, *J* = 12.2, 6.3 Hz, 1H), 4.10 (t, *J* = 8.7 Hz, 2H), 3.93 - 3.85 (m, 1H), 3.51 - 3.89 (m, 2H), 3.28 (t, *J* = 8.7 Hz, 1H), 3.23 - 3.04 (m, 4H), 2.46 - 2.29 (m, 3H), 2.26 - 2.23 (m, 2H), 2.04 - 1.92 (m, 5H), 1.74 - 1.64 (m, 2H), 1.32 (t, *J* = 7.4 Hz, 6H), 0.91 (d, *J* = 5.6 Hz, 6H), 0.86 (s, 2H).

**Example 37 *N*-((*S*)-2-cyano-1-(4-(ethylsulfonyl)phenyl)ethyl)-4-((2*S*,4*S*)-2-((difluoromethoxy) methyl)-4-(((4-(trifluoromethyl)thiazol-2-yl)methyl)amino)pyrrolidin-1-yl)benzamide**

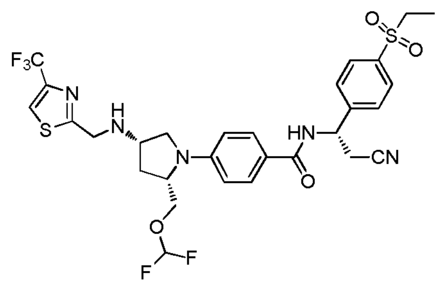

AcOH (81 mg, 1.35 mmol), 4-(trifluoromethyl)thiazole-2-carbaldehyde (82 mg, 0.45 mmol) and 4-((2*S*,4*S*)-4-amino-2-((difluoromethoxy)methyl)pyrrolidin-1-yl)-*N*-((*S*)-2-cyano-1-(4-(ethylsulfonyl)phenyl)ethyl)benzamide (230 mg, 0.45 mmol) were successively added to MeOH (4 mL). After the mixture was stirred at room temperature for 1 h, $NaBH_3CN$ (85 mg, 1.35 mmol) was added, and the mixture was stirred at room temperature for 16 h. The reaction solution was concentrated under reduced pressure. The residue was diluted with DCM (40 mL), washed successively with $NaHCO_3$ solution (20 mL) and saturated NaCl solution (20 mL), dried over anhydrous $Na_2SO_4$, concentrated under reduced pressure, and the crude product was separated by silica gel column chromatography (eluent: DCM/EtOAc (v/v) = 2/1) to give a white solid (200 mg, 66%).

MS (ESI, pos.ion) m/z: 672.1 $[M+H]^+$.
$^1H$ NMR (400 MHz, $CDCl_3$) δ (ppm): 7.92 (d, *J* = 8.4 Hz, 2H), 7.75 - 7.68 (m, 3H), 7.64 (d, *J* = 8.3 Hz, 2H), 6.66 (d, *J* = 7.6 Hz, 1H), 6.61 (d, *J* = 8.9 Hz, 2H), 6.24 (t, *J* = 74.4 Hz, 1H), 5.59 (dd, *J* = 12.7, 6.2 Hz, 1H), 4.24 (d, *J* = 15.9 Hz, 1H), 4.18 - 4.12 (m, 4H), 3.69 - 3.64 (m, 1H), 3.54 (dd, *J* = 10.3, 5.9 Hz, 1H), 3.38 (dd, *J* = 10.3, 2.4 Hz, 1H), 3.18 - 3.02 (m, 4H), 2.41 - 2.34 (m, 1H), 2.11 (d, *J* = 13.7 Hz, 1H), 1.28 (t, *J* = 7.4 Hz, 3H).

**Example 38 *N*-((*S*)-2-cyano-1-(4-(ethylsulfonyl)phenyl)ethyl)-4-((2*S*,4*S*)-2-((difluoromethoxy) methyl)-4-((4-(trifluoromethyl)thiazol-2-yl)oxy)pyrrolidin-1-yl)benzamide**

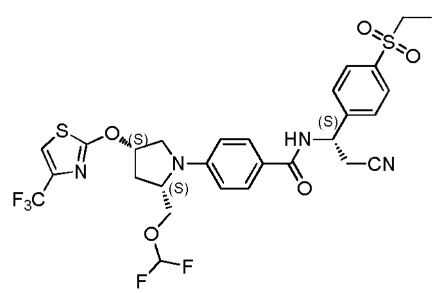

**Step 1: Synthesis of methyl 4-((2*S*,4*S*)-2-((difluoromethoxy)methyl)-4-hydroxypyrrolidin-1-yl)benzoate**

Using (2*S*,4*S*)-1-*tert*-butyl 2-methyl 4-hydroxypyrrolidine-1,2-dicarboxylate as raw material, the title compound was prepared according to the methods of step 1 to step 6 of Example 1 and a pale yellow liquid (4.80 g, 31%) was obtained.

**Step 2: Synthesis of methyl 4-((2*S*,4*S*)-2-((difluoromethoxy)methyl) -4-(trifluoromethyl)thiazol-2-yl)oxy)pyrrolidin-1-yl)benzoate**

Methyl 4-((2S,4S)-2-((difluoromethoxy)methyl)-4-hydroxypyrrolidin- 1 -yl)benzoate (200 mg, 0.66 mmol), 2-bromo-4-(trifluoromethyl)thiazole (231 mg, 1.00 mmol), $K_2CO_3$ (275 mg, 1.99 mmol) were added to DMF (4 mL), and the mixture was reacted at 100 °C for 24 h. The reaction solution was diluted with EtOAc (60 mL), washed with $H_2O$ (20 mL × 2) and saturated NaCl solution (20 mL) successively, dried over anhydrous $Na_2SO_4$, concentrated under reduced pressure, and the concentrated solution was separated by silica gel column chromatography (eluent: PE/EtOAc (v/v) = 4/1) to give colorless liquid (40 mg, 13%).
MS (ESI, pos.ion) m/z: 453.0 $[M+H]^+$.

**Step 3: Synthesis of 4-((2*S*,4*S*)-2-((difluoromethoxy)methyl)-4-(trifluoromethyl) thiazol-2-yl)oxy)pyrrolidin-1-yl)benzoic acid**

To a solution of methyl 4-((2*S*,4*S*)-2-((difluoromethoxy)methyl)-4-((4-(trifluoromethyl) thiazol-2-yl)oxy)pyrrolidin-1-yl)benzoate (30 mg, 0.07 mmol) in MeOH (0.5 mL) was added a solution of $LiOH \cdot H_2O$ (30 mg, 0.72 mmol) in $H_2O$ (0.5 mL). The mixture was stirred at room temperature for 16 h. The reaction solution was concentrated under reduced pressure, and HCl solution (1 mol/L) was added to the concentrated solution to adjust the pH to about 4. The resulting mixture was extracted with EtOAc (10 mL), washed with saturated NaCl (5 mL) solution, dried over anhydrous $Na_2SO_4$, concentrated under reduced pressure, and the concentrated solution was separated by silica gel column chromatography (eluent: PE/EtOAc (v/v) = 3/2) to give a white solid (20 mg, 69%).
MS (ESI, pos.ion) m/z: 439.1 $[M+H]^+$.

**Step 4: Synthesis of *N*-((*S*)-2-cyano-1-(4-(ethylsulfonyl)phenyl)ethyl)-4-((2*S*,4*S*) -2-((difluoromethoxy)methyl)-4-((4-(trifluoromethyl)thiazol-2-yl)oxy)pyrrolidin-1-yl)benzami de**

HATU (26 mg, 0.07 mmol), (*S*)-3-amino-3-(4-(ethylsulfonyl)phenyl)propionitrile hydrochloride (13 mg, 0.05 mmol), 4-((2*S*,4*S*)-2-((difluoromethoxy)methyl)-4-((4-(trifluoromethyl)thiazol-2-yl) oxy)pyrrolidin-1-yl)benzoic acid (20 mg , 0.05 mmol) and TEA (13 mg, 0.13 mmol) were successively added to DCM (1 mL), and the mixture was stirred at room temperature for 16 h. The reaction solution was concentrated under reduced pressure. The concentrated solution was diluted with DCM (20 mL), washed successively with HCl solution (10 mL, 1 mol/L), saturated $NaHCO_3$ solution (10 mL) and saturated NaCl solution (10 mL), dried over anhydrous $Na_2SO_4$, concentrated under reduced pressure, and the concentrated solution was separated by silica gel column chromatography (eluent: DCM/EtOAc (v/v) = 4/1) to give a white solid (25 mg, 83%).

MS (ESI, pos.ion) m/z: 659.2 $[M+H]^+$.
$^1H$ NMR (400 MHz, $CDCl_3$) $\delta$ (ppm): 7.91 (d, *J*= 8.3 Hz, 2H), 7.75 (d, *J*= 8.7 Hz, 2H), 7.65 (d, *J*= 8.2 Hz, 2H), 7.20 (s, 1H), 6.75 (d, *J* = 7.6 Hz, 1H), 6.66 (d, *J* = 8.8 Hz, 2H), 6.25 (t, *J* = 74.1 Hz, 1H), 5.81 (t, *J*= 4.6 Hz, 1H), 5.59 (dd, *J* = 12.5, 6.2 Hz, 1H), 4.24 - 4.14 (m, 2H), 3.90 - 3.83 (m, 2H), 3.74 (dd, *J*= 12.2, 4.8 Hz, 1H), 3.19 - 3.02 (m, 4H), 2.58 (d, *J* = 14.9 Hz, 1H), 2.50 - 2.41 (m, 1H), 1.28 (t, *J* = 7.5 Hz, 3H).

**Example 39 *N*-((*S*)-2-cyano-1-(4-(ethylsulfonyl)phenyl)ethyl)-4-((2*S*,4*S*)-2-((difluoromethoxy)methyl)-4-((4-(trifluoromethoxy)benzyl)oxy)pyrrolidin-1-yl)benzamide**

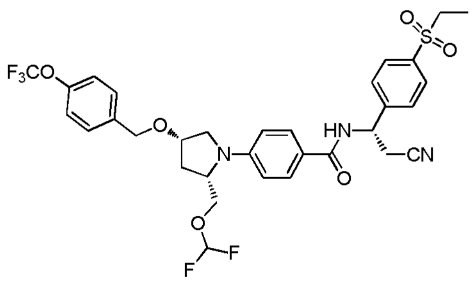

**Step 1: Synthesis of methyl 4-((2*S*,4*S*)-2-((difluoromethoxy)methyl)-4-(trifluoromethoxy)benzyl)oxy)pyrrolidin-1-yl)benzoate**

To a solution of methyl 4-((2*S*,4*S*)-2-((difluoromethoxy)methyl)-4-hydroxypyrrolidin-1-yl)benzoate (300 mg, 1.00 mmol) in DMF (4 mL) was added NaH (60 mg, 1.50 mmol, 60%) at 0 °C, and the mixture was transferred to room temperature for reaction for 30 min, then 1-(bromomethyl)-4-(trifluoromethoxy)benzene (0.2 mL, 1.25 mmol) was added. The reaction was continued for 14 h at room temperature. The reaction solution was diluted with EtOAc (40 mL), washed with $H_2O$ (15 mL × 2) and saturated NaCl solution (15 mL), dried over anhydrous $Na_2SO_4$, concentrated under reduced pressure, and the concentrated solution was separated by silica gel column chromatography (eluent: PE/EtOAc (v/v) = 6/1) to give a white solid (300 mg, 63%).
MS (ESI, pos.ion) m/z: 476.3 $[M+H]^+$.

**Step 2: Synthesis of 4-((2*S*,4*S*)-2-((difluoromethoxy)methyl)-4-(trifluoromethoxy)benzyl) oxy)pyrrolidin-1-yl)benzoic acid**

To a solution of methyl 4-((2*S*,4*S*)-2-((difluoromethoxy)methyl)-4-((4-(trifluoromethoxy)benzyl)oxy)pyrrolidin-1-yl)benzoate (300 mg, 0.63 mmol) in MeOH (2 mL) and THF (2 mL) was added a solution of $LiOH{\cdot}H_2O$ (160 mg, 3.81 mmol) in $H_2O$ (2 mL). The mixture was stirred at room temperature for 24 h. The reaction solution was concentrated under reduced pressure, and HCl solution (1 mol/L) was added to the concentrated solution to adjust the pH to about 4. The resulting mixture was extracted with EtOAc (30 mL), washed with saturated NaCl (10 mL) solution, dried over anhydrous $Na_2SO_4$, concentrated under reduced pressure, and the concentrated solution was separated by silica gel column chromatography (eluent: PE/EtOAc (v/v) = 3/2) to give a white solid (241 mg, 83%).
MS (ESI, pos.ion) m/z: 462.3 $[M+H]^+$.

**Step 3: Synthesis of *N*-((*S*)-2-cyano-1-(4-(ethylsulfonyl)phenyl)ethyl)-4-((2*S*,4*S*)-2-((difluoromethoxy)methyl)-4-((4-(trifluoromethoxy)benzyl)oxy)pyrrolidin-1-yl)benzamide**

HATU (260 mg, 0.68 mmol), (*S*)-3-amino-3-(4-(ethylsulfonyl)phenyl)propionitrile hydrochloride (160 mg, 0.58 mmol), 4-((2*S*,4*S*)-2-((difluoromethoxy)methyl) -4-((4-(trifluoromethoxy)benzyl)oxy)pyrrolidin-1-yl)benzoic acid (240 mg , 0.52 mmol) and TEA (0.20 mL, 1.44 mmol) were successively added to DCM (6 mL), and the mixture was stirred at room temperature for 24 h. The reaction solution was concentrated under reduced pressure. The concentrated solution was diluted with DCM (40 mL), washed successively with HCl solution (20 mL, 1 mol/L), saturated $NaHCO_3$ solution (20 mL) and saturated NaCl solution (20 mL), dried over anhydrous $Na_2SO_4$, concentrated under reduced pressure, and the concentrated solution was separated by silica gel column chromatography (eluent: DCM/EtOAc (v/v) = 4/1) to give a white solid (280 mg, 79%).

MS (ESI, pos.ion) m/z: 682.2 $[M+H]^+$.
$^1$H NMR (400 MHz, $CDCl_3$) δ (ppm): 7.90 (d, *J*= 8.3 Hz, 2H), 7.73 (d, *J*= 8.8 Hz, 2H), 7.64 (d, *J* = 8.3 Hz, 2H), 7.36 (d, *J* = 8.5 Hz, 2H), 7.20 (d, *J* = 8.1 Hz, 2H), 6.77 (d, *J* = 7.6 Hz, 1H), 6.62 (d, *J* = 8.9 Hz, 2H), 6.25 (t, *J* = 74.6 Hz, 1H), 5.59 (dd, *J* = 12.9, 6.2 Hz, 1H), 4.61 (d, *J* = 11.8 Hz, 1H), 4.53 (d, *J* = 11.8 Hz, 1H), 4.34 (t, *J* = 4.6 Hz, 1H), 4.15 - 4.08 (m, 2H), 3.98 (t, *J* = 10.7 Hz, 1H), 3.65 (d, *J* = 11.0 Hz, 1H), 3.48 (dd, *J* = 11.1, 4.9 Hz, 1H), 3.17 - 3.01 (m, 4H), 2.45 (d, *J* = 14.1 Hz, 1H), 2.19-2.09 (m, 1H), 1.28 (t, *J* = 7.5 Hz, 3H).

**Example 40 *N*-((*S*)-2-cyano-1-(4-(ethylsulfonyl)phenyl)ethyl)-4-((2*S*,4*S*) -4-((5-cyclopropylpyridin-2-yl)oxy)-2-((difluoromethoxy)methyl)pyrrolidin-1-yl)benzamide**

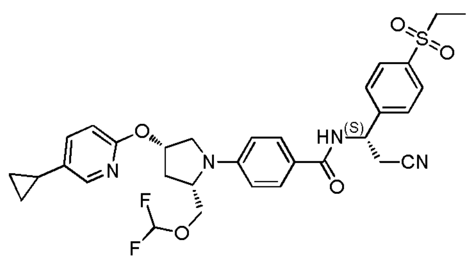

**Step 1: Synthesis of methyl 4-((2*S*,4*S*)-4-((5-bromopyridin-2-yl)oxy)-2 -((difluoromethoxy)methyl)pyrrolidin-1-yl)benzoate**

Methyl 4-((2*S*,4*S*)-2-((difluoromethoxy)methyl)-4-hydroxypyrrolidin-1-yl)benzoate (530 mg, 1.76 mmol)and 5-bromo-2-fluoro-pyridine (0.5 mL, 5 mmol) were dissolved in anhydrous THF (20 mL). Then *t*-BuOK (500 mg, 4.32 mmol) was added, and the reaction solution was stirred at room temperature for 12 h. The reaction solution was concentrated under reduced pressure. The concentrated solution was diluted with DCM (20 mL), washed successively with $Na_2CO_3$ solution (35 mL) and saturated NaCl solution (25 mL), dried over anhydrous $Na_2SO_4$, filtered and concentrated under reduced pressure, and the concentrated solution was separated by silica gel column chromatography (eluent: PE/EtOAc (v/v) = 10/1) to give a white solid (780 mg, 97%).
MS (ESI, pos.ion) m/z: 458.9 $[M+H]^+$.

**Step 2: Synthesis of methyl 4-((2*S*,4*S*)-4-((5-cyclopropylpyridin-2-yl)oxy)-2-((difluoromethoxy)methyl)pyrrolidin-1-yl)benzoate**

Methyl 4-((2*S*,4*S*)-4-((5-bromopyridin-2-yl)oxy)-2-((difluoromethoxy)methyl) pyrrolidin-1-yl)benzoate (100 mg, 0.22 mmol), cyclopropylboronic acid (50 mg, 0.58 mmol) and Pd(dppf)$Cl_2$ (20 mg, 0.027 mmol) were dissolved in 1,4-dioxane (20 mL), then $Cs_2CO_3$ (100 mg, 0.31 mmol) was added. The reaction solution was reacted at 100°C for 14 h under nitrogen protection. After the reaction was completed, the reaction solution was cooled to room temperature and filtered. The filtrate was concentrated under reduced pressure, then $NaHCO_3$ solution (20 mL) was added. The resulting mixture was extracted with DCM (34 mL). The aqueous phase was extracted with DCM (20 mL). The combined organic phases were dried over anhydrous $Na_2SO_4$, filtered and concentrated under reduced pressure. The concentrated solution was separated by silica gel column chromatography (eluent: PE/EtOAc (v/v) = 10/1) to give a white solid (77 mg, 84%).
MS (ESI, pos.ion) m/z: 419.2 $[M+H]^+$.

**Step 3: Synthesis of 4-((2*S*,4*S*)-4-((5-cyclopropylpyridin-2-yl)oxy)-2-((difluoromethoxy) methyl)pyrrolidin-1-yl)benzoic acid**

Methyl 4-((2*S*,4*S*)-4-((5-cyclopropylpyridin-2-yl)oxy)-2-((difluoromethoxy)methyl) pyrrolidin-1-yl)benzoate (77 mg, 0.18 mmol) was dissolved in MeOH (5 mL), then LiOH (190 mg, 4.44 mmol) was added. The mixture was stirred at room temperature for 22 h. After the reaction was completed, HCl solution (5.5 mL, 1.0 mol/L) was added to the reaction solution, the mixture was extracted with DCM (20 mL × 3). The organic phases were combined, washed with saturated NaCl solution (15 mL), dried over anhydrous $Na_2SO_4$, concentrated under reduced pressure, and the concentrated solution was separated by silica gel column chromatography (eluent: DCM/MeOH (v/v) = 10/1) to give a white solid (50 mg, 67 %).
MS (ESI, pos.ion) m/z: 405.1 $[M+H]^+$.

**Step 4: Synthesis of *N*-((*S*)-2-cyano-1-(4-(ethylsulfonyl)phenyl)ethyl)-4-((2*S*,4*S*) -4-((5-cyclopropylpyridin-2-yl)oxy)-2-((difluoromethoxy)methyl)pyrrolidin-1-yl)benzamide**

(*S*)-3-amino-3-(4-(ethylsulfonyl)phenyl)propionitrile hydrochloride (39 mg, 0.16 mmol), 4-((2*S*,4*S*)-4-((5-cyclopropylpyridin-2-yl)oxy)-2-((difluoromethoxy)methyl)pyrrolidin -1-yl)benzoic acid (60 mg, 0.15 mmol), HATU (62 mg, 0.16 mmol) and DIPEA (0.3 mL, 2 mmol) were dissolved in DCM (14 mL), and the reaction solution was stirred at room temperature for 16 h. The reaction solution was diluted with DCM (40 mL), washed successively with $NaHCO_3$ solution (30 mL) and saturated NaCl solution (40 mL). The organic phase was dried over anhydrous $Na_2SO_4$, concentrated under reduced pressure, and the concentrated solution was separated by silica gel column chromatography (eluent: PE/EtOAc (v/v) = 1/1) to give a white solid (60 mg, 65%).

MS (ESI, pos.ion) m/z: 625.2 $[M+H]^+$.
$^1$H NMR (400 MHz, $CDCl_3$) δ (ppm): 7.98 (s, 1H), 7.93 (d, *J* = 7.4 Hz, 2H), 7.69 (dd, *J* = 27.7, 7.5 Hz, 4H), 6.63 (s, 4H), 6.23 (t, *J* = 74.5 Hz, 1H), 5.66 (d, *J*= 59.3 Hz, 2H), 4.17 (s, 2H), 4.01 (d, *J* = 8.5 Hz, 1H), 3.70 (s, 2H), 3.23 - 2.93 (m, 4H), 2.43 (t, *J*= 14.5 Hz, 2H), 1.85 (s, 1H), 1.28 (t, *J* = 7.4 Hz, 3H), 0.95 (d, *J* = 6.8 Hz, 2H), 0.63 (s, 2H).

**Example 41 *N*-((*S*)-2-cyano-1-(4-(ethylsulfonyl)phenyl)ethyl)-4-((2*S*,4*S*)-2-((difluoromethoxy) methyl)-4-((5-methoxypyridin-2-yl)oxy)pyrrolidin-1-yl)benzamide**

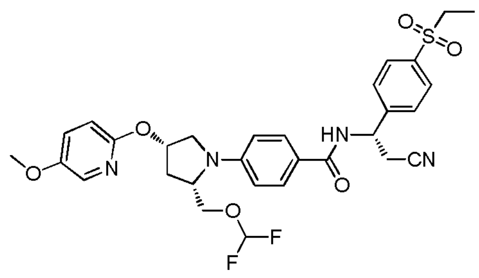

**Step 1: Synthesis of methyl 4-((2*S*,4*S*)-2-((difluoromethoxy)methyl) -4-((5-methoxypyridin-2-yl)oxy)pyrrolidin-1-yl)benzoate**

2-Fluoro-5-methoxypyridine (65 mg, 0.51 mmol), methyl 4-((2*S*,4*S*)-2-(difluoromethoxymethyl)-4-hydroxypyrrolidin-1-yl)benzoate (128 mg, 0.42 mmol), *t*-BuOK (71 mg, 0.64 mmol) were dissolved in THF (10 mL). The mixture was degassed and refilled with $N_2$, heated to 80 °C to reflux, and reacted for 10 h. The reaction was quenched by adding water. The resulting mixture was extracted with EtOAc (10 mL × 2), dried over anhydrous $Na_2SO_4$, concentrated and separated by silica gel column chromatography (eluent: PE/EtOAc (v/v) = 3/1) to give a white solid (0.12 g, 69%).
MS (ESI, pos.ion) m/z: 409 $[M+H]^+$.

**Step 2: Synthesis of 4-((2*S*,4*S*)-2-((difluoromethoxy)methyl)-4-((5-methoxypyridin -2-yl)oxy)pyrrol-1-yl)benzoic acid**

To a solution of methyl 4-((2*S*,4*S*)-2-((difluoromethoxy) methyl)-4-((5-methoxypyridin-2-yl)oxy)pyrrolidin-1-yl)benzoate (120 mg, 0.29 mmol) in MeOH (5 mL) was added a solution of LiOH (35 mg, 1.46 mmol) in $H_2O$ (5 mL). The mixture was stirred at room temperature for 24 h. Dilute hydrochloric acid was added to adjust the pH of the solution to be acidic. The resulting mixture was extracted with EtOAc (10 ml × 2). The organic phases were combined, dried over anhydrous $Na_2SO_4$, filtered, concentrated and separated by silica gel column chromatography (eluent: PE/EtOAc (v/v) = 1/1) to give a white solid (0.105 g, 91%).
MS (ESI, pos.ion) m/z: 395 $[M+H]^+$.

**Step 3: Synthesis of *N*-((*S*)-2-cyano-1-(4-(ethylsulfonyl)phenyl)ethyl)-4-((2*S*,4*S*)-2 -((difluoromethoxy)methyl)-4-((5-methoxypyridin-2-yl)oxy)pyrrolidin-1-yl)benzamide**

4-((2*S*,4*S*)-2-((Difluoromethoxy)methyl)-4-((5-methoxypyridin-2-yl)oxy)pyrrol-1-yl)b enzoic acid (20 mg, 0.051 mmol), (*S*)-3-amino-3-(4-(ethylsulfonyl)phenyl)propane cyanide (16 mg, 0.058 mmol) and HATU (24 mg, 0.064 mmol) were dissolved in DCM (25 mL), then TEA (0.016 mL, 0.12 mmol) was added and the mixture was stirred at room temperature for 24 h. The reaction was quenched by adding saturated $NH_4Cl$ solution. The resulting mixture was extracted with DCM (10 mL × 3). The organic phases were combined, dried over anhydrous $Na_2SO_4$, filtered, concentrated and separated by silica gel column chromatography (eluent: PE/EtOAc (v/v) = 1/1) to give a white solid (18 mg, 58%).

MS (ESI, pos.ion) m/z: 615 $[M+H]^+$.
$^1H$ NMR (400 MHz, $CDCl_3$) δ (ppm): 7.97 (d, *J* = 8.3 Hz, 2H), 7.84 (d, *J* = 3.0 Hz, 1H), 7.74 (d, *J* = 8.8 Hz, 2H), 7.68 (d, *J*= 8.3 Hz, 2H), 7.26 (dd, *J*= 9.0, 3.1 Hz, 1H), 6.69 (t, *J*= 9.4 Hz, 3H), 6.60 (d, *J* = 7.6 Hz, 1H), 6.26 (t, *J* = 74.5 Hz, 1H), 5.71 (s, 1H), 5.62 (dd, *J* = 12.4, 6.4 Hz, 1H), 4.29 - 4.12 (m, 2H), 4.03 (t, *J* = 9.3 Hz, 1H), 3.85 (s, 3H), 3.72 (s, 2H), 3.13 (dt, *J* = 7.1, 6.4 Hz, 4H), 2.54 - 2.34 (m, 2H), 1.36 - 1.27 (m, 3H).

**Example 42 *N*-((*S*)-2-cyano-1-(4-(ethylsulfonyl)phenyl)ethyl)-4-((2*S*,4*S*)-2-((difluoromethoxy) methyl)-4-((5-methylpyridin-2-yl)oxy)pyrrolidin-1-yl)benzamide**

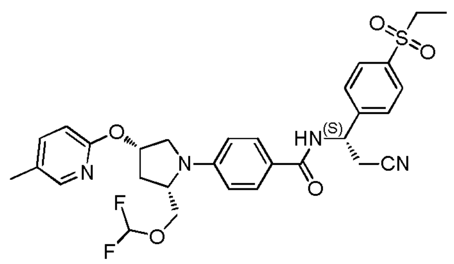

**Step 1: Synthesis of methyl 4-((2*S*,4*S*)-4-((5-bromopyridin-2-yl)oxy) -2-((difluoromethoxy)methyl)pyrrolidin-1-yl)benzoate**

Methyl 4-((2*S*,4*S*)-2-((difluoromethoxy)methyl)-4-hydroxypyrrolidin-1-yl)benzoate (530 mg, 1.76 mmol) and 5-bromo-2-fluoro-pyridine (0.5 mL, 5 mmol) were dissolved in anhydrous THF (20 mL). Then *t*-BuOK (500 mg, 4.32 mmol) was added, and the mixture was reacted at room temperature. After the reaction was completed, the reaction solution was concentrated under reduced pressure. The concentrated solution was diluted with DCM (20 mL), washed successively with $Na_2CO_3$ solution (35 mL) and saturated NaCl solution (25 mL), dried over anhydrous $Na_2SO_4$, filtered and concentrated under reduced pressure, and the concentrated solution was separated by silica gel column chromatography (eluent: PE/EtOAc (v/v) = 10/1) to give a white solid (780 mg, 97%).
MS (ESI, pos.ion) m/z: 458.9 $[M+H]^+$.

**Step 2: Synthesis of methyl 4-((2*S*,4*S*)-4-((5-methylpyridin-2-yl)oxy)-2-((difluoromethoxy)methyl)pyrrolidin-1-yl)benzoate**

Methyl 4-((2*S*,4*S*)-4-((5-bromopyridin-2-yl)oxy)-2-((difluoromethoxy)methyl) pyrrolidin-1-yl)benzoate (100 mg, 0.22 mmol), methylboronic acid (50 mg, 0.84 mmol) and $Pd(dppf)Cl_2$ (20 mg, 0.027 mmol) were dissolved in 1,4-dioxane (20 mL), then $Cs_2CO_3$ (100 mg, 0.31 mmol) was added. The reaction solution was reacted at 80°C for 16 h under nitrogen protection. After the reaction was completed, the reaction solution was cooled to room temperature and filtered. The filtrate was concentrated under reduced pressure, then $NaHCO_3$ solution (20 mL) was added. The resulting mixture was extracted with DCM (34 mL). The aqueous phase was extracted with DCM (20 mL). The combined organic phases were dried over anhydrous $Na_2SO_4$, filtered and concentrated under reduced pressure. The concentrated solution was separated by silica gel column chromatography (eluent: PE/EtOAc (v/v) = 3/1) to give a white solid (70 mg, 82%).
MS (ESI, pos.ion) m/z: 393.1 $[M+H]^+$.

**Step 3: Synthesis of 4-((2*S*,4*S*)-2-((difluoromethoxy)methyl)-4-((5-methylpyridin -2-yl)oxy)pyrrolidin-1-yl)benzoic acid**

To a solution of methyl 4-((2*S*,4*S*)-2-((difluoromethoxy)methyl)-4-((5-methylpyridin-2-yl)oxy)pyrrolidin-1-yl)benzoate (70 mg, 0.18 mmol) in MeOH (5 mL) was added LiOH (190 mg, 4.44 mmol). The mixture was reacted at room temperature for 19 h. HCl solution (1.5 mL, 0.1mol/L) was added to the reaction solution, then the mixture was extracted with DCM (20 mL × 3). The organic phases were combined, washed with saturated NaCl solution (15 mL), dried over anhydrous $Na_2SO_4$, concentrated under reduced pressure, and the concentrated solution was separated by silica gel column chromatography (eluent: DCM/MeOH (v/v) = 10/1) to give a white solid (30 mg, 44%).
MS (ESI, pos.ion) m/z: 379.2 $[M+H]^+$.

**Step 4: Synthesis of *N*-((*S*)-2-cyano-1-(4-(ethylsulfonyl)phenyl)ethyl)-4-((2*S*,4*S*)-2-((difluoromethoxy)methyl)-4-((5-methylpyridin-2-yl)oxy)pyrrolidin-1-yl)benzamide**

(*S*)-3-Amino-3-(4-(ethylsulfonyl)phenyl)propionitrile hydrochloride (36 mg, 0.15 mmol), 4-((2*S*,4*S*)-2-((difluoromethoxy)methyl)-4-((5-methylpyridin-2-yl)oxy)pyrrolidin-1-yl) benzoic acid (40 mg , 0.11 mmol), HATU (48 mg, 0.12 mmol) and DIPEA (0.3 mL, 2 mmol) were dissolved in DCM (12 mL), and the reaction solution was stirred at room temperature for 15 h. The reaction solution was diluted with DCM (40 mL), washed successively with $NaHCO_3$ solution (30 mL) and saturated NaCl solution (40 mL). The organic phase was dried over anhydrous $Na_2SO_4$, concentrated under reduced pressure, and the concentrated solution was separated by silica gel column chromatography (eluent: PE/EtOAc (v/v) = 1/1) to give a white solid (15 mg, 24%).

MS (ESI, pos.ion) m/z: 599.2 $[M+H]^+$.

$^1$H NMR (400 MHz, $CDCl_3$) δ (ppm): 7.97 (s, 1H), 7.91 (d, *J* = 8.3 Hz, 2H), 7.72 (d, *J* = 8.8 Hz, 2H), 7.65 (d, *J* = 8.2 Hz, 2H), 7.42 (dd, *J* = 8.4, 1.9 Hz, 1H), 6.70 (d, *J* = 7.5 Hz, 1H), 6.64 (d, *J* = 8.6 Hz, 3H), 6.23 (t, *J*= 74.5 Hz, 1H), 5.74 (s, 1H), 5.59 (dd, *J*= 12.6, 6.3 Hz, 1H), 4.21 - 4.13 (m, 2H), 4.01 (t, *J* = 9.7 Hz, 1H), 3.68 (d, *J* = 15.2 Hz, 2H), 3.17 - 3.03 (m, 4H), 2.45 (d, *J* = 14.2 Hz, 1H), 2.41 - 2.33 (m, 1H), 2.23 (d, *J*= 17.5 Hz, 3H), 1.27 (d, *J*= 7.4 Hz, 3H).

**Example 43 *N*-((*S*)-2-cyano-1-(4-(ethylsulfonyl)phenyl)ethyl)-4-((2*S*,4*S*)-2-((difluoromethoxy)methyl) -4-((pyridin-2-yl)oxy)pyrrolidin-1-yl)benzamide**

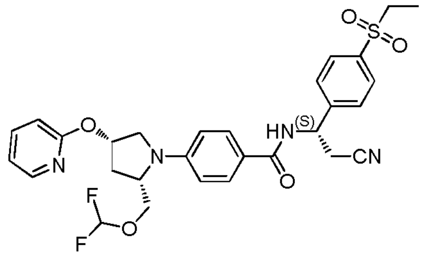

**Step 1: Synthesis of methyl 4-((2*S*,4*S*)-2-((difluoromethoxy)methyl) -4-((pyridin-2-yl)oxy)pyrrolidin-1-yl)benzoate**

Methyl 4-((2*S*,4*S*)-4-((5-bromopyridin-2-yl)oxy)-2-((difluoromethoxy)methyl) pyrrolidin-1-yl)benzoate (150 mg, 0.33 mmol), isopropylboronic acid (50 mg, 0.57 mmol) and Pd(dppf)$Cl_2$ (30 mg, 0.040 mmol) were dissolved in 1,4-dioxane (20 mL), then $Cs_2CO_3$ (120 mg, 0.37 mmol) was added. The reaction solution was reacted at 80°C for 14 h under nitrogen protection. The reaction solution was cooled to room temperature and filtered. The filtrate was concentrated under reduced pressure, then $NaHCO_3$ solution (20 mL) was added. The resulting mixture was extracted with DCM (34 mL). The aqueous phase was extracted with DCM (20 mL). The combined organic phases were dried over anhydrous $Na_2SO_4$, filtered and concentrated under reduced pressure. The concentrated solution was separated by silica gel column chromatography (eluent: PE/EtOAc (v/v) = 10/1) to give a white solid (80 mg, 64 %).

**Step 2: Synthesis of 4-((2*S*,4*S*)-2-((difluoromethoxy)methyl)-4-((pyridin-2-yl)oxy)pyrrolidin-1-yl)benzoic acid**

To a solution of methyl 4-((2*S*,4*S*)-2-((difluoromethoxy)methyl)-4-((pyridin-2-yl)oxy)pyrrolidin-1-yl)benzoate (80 mg, 0.21 mmol) in MeOH (5 mL) was added LiOH (90 mg, 2.10 mmol). The mixture was reacted at room temperature for 16 h. HCl solution (1.5 mL, 0.1mol/L) was added to the reaction solution, then the mixture was extracted with DCM (20 mL × 3). The organic phases were combined, washed with saturated NaCl solution (15 mL), dried over anhydrous $Na_2SO_4$, concentrated under reduced pressure, and the concentrated solution was separated by silica gel column chromatography (eluent: DCM/MeOH (v/v) = 10/1) to give a white solid (57 mg, 74%).
MS (ESI, pos.ion) m/z: 365.2 $[M+H]^+$.

**Step 3: Synthesis of *N*-((*S*)-2-cyano-1-(4-(ethylsulfonyl)phenyl)ethyl)-4-((2*S*,4*S*) -2-((difluoromethoxy)methyl)-4-(pyridin-2-yloxy)pyrrolidin-1-yl)benzamide**

(*S*)-3-Amino-3-(4-(ethylsulfonyl)phenyl)propionitrile hydrochloride (31 mg, 0.13 mmol), 4-((2*S*,4*S*)-2-((difluoromethoxy)methyl)-4-((pyridin-2-yl)oxy)pyrrolidin-1-yl)benzoic acid (50 mg, 0.14 mmol), HATU (62 mg, 0.16 mmol) and DIPEA (0.3 mL, 2 mmol) were dissolved in DCM (10 mL). The mixture was reacted at room temperature for 16 h. The reaction solution was diluted with DCM (40 mL), washed successively with $NaHCO_3$ solution (30 mL) and saturated NaCl solution (40 mL). The organic phase was dried over anhydrous $Na_2SO_4$, concentrated under reduced pressure, and the concentrated solution was separated by silica gel column chromatography (eluent: PE/EtOAc (v/v) = 1/1) to give a white solid (20 mg, 25%).

MS (ESI, pos.ion) m/z: 585.2 $[M+H]^+$.
$^1$H NMR (400 MHz, $CDCl_3$) δ (ppm): 8.17 (d, *J*= 3.8 Hz, 1H), 7.89 (d, *J*= 8.2 Hz, 2H), 7.73 (d, *J*= 8.7 Hz, 2H), 7.61 (dd, *J* = 20.4, 7.6 Hz, 3H), 6.94 - 6.87 (m, 1H), 6.79 (d, *J* = 7.6 Hz, 1H), 6.73 (d, *J* = 8.3 Hz, 1H), 6.64 (d, *J* = 8.7 Hz, 2H), 6.23 (t, *J* = 74.5 Hz, 1H), 5.78 (s, 1H), 5.59 (dd, *J* = 12.5, 6.1 Hz, 1H), 4.23 - 4.10 (m, 2H), 4.01 (t, *J* = 9.3 Hz, 1H), 3.71 (s, 2H), 3.17 - 3.01 (m, 4H), 2.47 (d, *J* = 14.3 Hz, 1H), 2.43 -2.34 (m, 1H), 1.27 (t, *J* = 7.4 Hz, 3H).

**Example 44 *N*-((*S*)-2-cyano-1-(4-(ethylsulfonyl)phenyl)ethyl)-4-((2*S*,4*S*)-2-((difluoromethoxy) methyl)-4-(4-(trifluoromethyl)-1*H*-imidazol-1-yl)pyrrolidin-1-yl)benzamide**

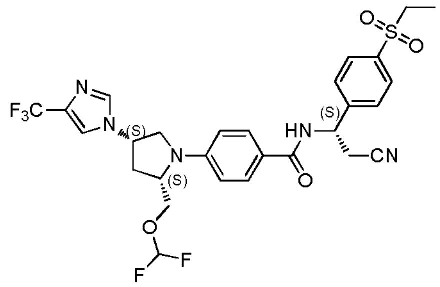

**Step 1: Synthesis of methyl 4-((2*S*,4*R*)-2-((difluoromethoxy)methyl)-4-((methylsulfonyl)oxy)pyrrolidin-1-yl)benzoate**

To a solution of methyl 4-((2*S*,4*R*)-2-((difluoromethoxy)methyl) -4-hydroxy-pyrrolidin-1-yl)benzoate (260 mg, 0.86 mmol) in DCM (15 mL) were added TEA (0.44 mL, 3.40 mmol) and DMAP (86 mg, 0.67 mmol). The mixture was cooled to 0°C, then methanesulfonyl chloride (0.2 mL, 3.00 mmol) was added. The resulting mixture was slowly cooled to room temperature, and continued stirring for 5 h. The resulting mixture was extracted with DCM (10 mL × 2). The organic phases were combined, dried over anhydrous $Na_2SO_4$, filtered, concentrated and separated by silica gel column chromatography (eluent: PE/EtOAc (v/v) = 3/1) to give colorless liquid (0.31 g, 95%).
MS (ESI, pos.ion) m/z: 380 $[M+H]^+$.

**Step 2: Synthesis of methyl 4-((2*S*,4*S*)-2-((difluoromethoxy)methyl) -4-(4-(trifluoromethyl)-1*H*-imidazol-1-yl)pyrrolidin-1-yl)benzoate**

Methyl 4-((2*S*,4*R*)-2-((difluoromethoxy)methyl)-4-((methylsulfonyl)oxy)pyrrolidin-1 -yl)benzoate (133 mg, 0.35 mmol), 4-(trifluoromethyl)-1*H*-imidazole (71 mg, 0.52 mmol) and $K_2CO_3$ (96 mg, 0.69 mmol) were dissolved in anhydrous DMF (15 mL), and the mixture was reacted at 80 °C for 5 h. The resulting mixture was cooled to room temperature, washed with saturated NaCl solution, extracted with EtOAc (10 mL × 2). The organic phases were combined, dried over anhydrous $Na_2SO_4$, filtered, concentrated and separated by silica gel column chromatography (eluent: PE/EtOAc (v/v) = 4/1) to give colorless liquid (0.13 g, 88%).
MS (ESI, pos.ion) m/z: 420 $[M+H]^+$.

**Step 3: Synthesis of 4-((2*S*,4*S*)-2-((difluoromethoxy)methyl)-4-(4-(trifluoromethyl)-1*H*-imidazol-1-yl)pyrrolidin-1-yl)benzoic acid**

To a solution of methyl 4-((2*S*,4*S*)-2-((difluoromethoxy)methyl)-4-(4-(trifluoromethyl)-1*H*-imidazol-1-yl)pyrrolidin-1-yl)benzoate (45 mg, 0.11 mmol) in MeOH (10 mL) was added LiOH (5 mg, 0.21 mmol). The mixture was reacted at room temperature for 12 h. Dilute hydrochloric acid was added to adjust the pH of the solution to be acidic. The resulting mixture was extracted with EtOAc (10 mL × 3). The organic phases were combined, dried over anhydrous $Na_2SO_4$, filtered, concentrated and separated by silica gel column chromatography (eluent: PE/EtOAc (v/v) = 1/1) to give a white solid (40 mg, 92%).
MS (ESI, pos.ion) m/z: 406 $[M+H]^+$.

**Step 4: Synthesis of *N*-((*S*)-2-cyano-1-(4-(ethylsulfonyl)phenyl)ethyl)-4-((2*S*,4*S*)-2-((difluoromethoxy)methyl)-4-(4-(trifluoromethyl)-1*H*-imidazol-1-yl)pyrrolidin-1-yl)benzamide**

4-((2*S*,4*S*)-2-((Difluoromethoxy)methyl)-4-(4-(trifluoromethyl)-1*H*-imidazol-1-yl)pyrr olidin-1-yl)benzoic acid (60 mg, 0.15 mmol), (*S*)-3-amino-3-(4-(ethylsulfonyl)phenyl)propionitrile hydrochloride (44 mg, 0.16 mmol) and HATU (68 mg, 0.18 mmol) were dissolved in DCM (25 mL), then TEA (0.035 mL, 0.27 mmol) was added and the mixture was reacted at room temperature for 12 h. The resulting mixture was washed with saturated $NH_4Cl$ solution, extracted with DCM (10 mL × 3). The organic phases were combined, dried over anhydrous $Na_2SO_4$, filtered, concentrated and separated by silica gel column chromatography (eluent: PE/EtOAc (v/v) = 1/1) to give a white solid (23 mg, 25%).

MS (ESI, pos.ion) m/z: 626 [M+H]+.
$^1$H NMR (400 MHz, $CDCl_3$) δ (ppm): 7.96 (d, *J* = 8.1 Hz, 2H), 7.79 (d, *J* = 8.6 Hz, 2H), 7.68 (d, *J* = 8.9 Hz, 2H), 7.55 (d, *J*= 8.3 Hz, 0.5H), 7.44 (s, 1H), 7.38 (m, 0.5 H), 7.15 (m, 0.5H), 6.75 (m, 0.5H), 6.70 (d, *J* = 8.5 Hz, 2H), 6.20 (t, *J* = 73.8 Hz, 1H), 5.62 (d, *J* = 5.9 Hz, 1H), 4.98 (dd, *J* = 25.4, 13.7 Hz, 1H), 4.91 - 4.82 (m, 1H), 4.33 (s, 1H), 4.08 (d, *J* = 9.8 Hz, 1H), 4.04 - 3.95 (m, 1H), 3.91 (dd, *J* = 10.4, 6.1 Hz, 1H), 3.86-3.77 (m, 1H), 3.25 - 3.04 (m, 3H), 3.00 - 2.87 (m, 1H), 2.54 - 2.41 (m, 1H), 1.38 (d, *J*= 12.3 Hz, 3H).

**Example 45 *N*-((*S*)-2-cyano-1-(4-(ethylsulfonyl)phenyl)ethyl)-4-((2*S*,4*S*)-2-((difluoromethoxy) methyl)-4-(4-(4-methyl-1*H*-imidazol-1-yl)phenoxy)pyrrolidin-1-yl)benzamide**

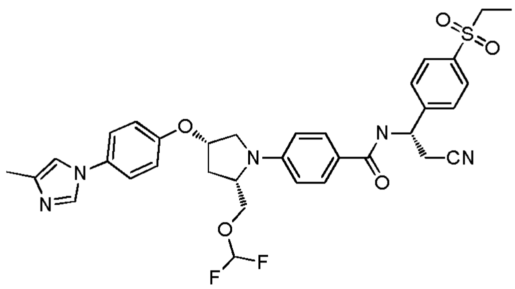

**Step 1: Synthesis of 1-(4-methoxyphenyl)-4-methyl-1*H*-imidazole**

To a solution of 4-methoxyphenylboronic acid (925 mg, 6.09 mmol) and 4-methylimidazole (700 mg, 8.53 mmol) in MeOH/$H_2O$ (6 mL/6 mL) were added $K_2CO_3$ (1.82 g, 12.77 mmol) and CuO (101 mg, 1.27 mmol). The mixture was reacted at room temperature for 24 h. The reaction solution was filtered through a celite pad, and the filtrate was concentrated under reduced pressure, diluted with EtOAc (40 mL), washed with saturated NaCl solution (20 mL), dried over anhydrous $Na_2SO_4$, and concentrated under reduced pressure. The concentrated solution was separated by silica gel column chromatography (eluent: DCM/EtOAc (v/v) = 3/2) to give pale yellow liquid (300 mg, 19%).
MS (ESI, pos.ion) m/z: 189.2 [M+H]+.

**Step 2: Synthesis of 4-(4-methyl-1*H*-imidazol-1-yl)phenol**

To a solution of 1-(4-methoxyphenyl)-4-methyl-1*H*-imidazole (300 mg, 1.59 mmol) in DCM (8 mL) was added $BBr_3$ (0.50 mL, 5.03 mmol) at -10 °C. The mixture was reacted at room temperature for 3 h. The reaction was quenched by adding MeOH (6 mL). The resulting mixture was concentrated under reduced pressure, diluted with EtOAc (50 mL), washed successively with $NaHCO_3$ solution (20 mL) and saturated NaCl solution (20 mL), dried over anhydrous $Na_2SO_4$, concentrated under reduced pressure, and the concentrated solution was separated by silica gel column chromatography (eluent: DCM/MeOH (v/v) = 20/1) to give a white solid (226 mg, 81%).
MS (ESI, pos.ion) m/z: 175.1 [M+H]+.

**Step 3: Synthesis of methyl 4-((2*S*,4*S*)-2-((difluoromethoxy)methyl)-4-(4-(4-methyl-1*H*-imidazol-1-yl)phenoxy)pyrrolidin-1-yl)benzoate**

$K_2CO_3$ (546 mg, 3.95 mmol), 4-(4-methyl-1*H*-imidazol-1-yl)phenol (220 mg, 1.26 mmol) and methyl 4-((2*S*,4*R*)-2-((difluoromethoxy)methyl)-4-((methylsulfonyl)oxy) pyrrolidin-1-yl)benzoate (500 mg, 1.32 mmol) was successively added to DMF (6 mL) solution. The mixture was reacted at 100 °C for 24 h. The reaction solution was cooled to room temperature, diluted with EtOAc (50 mL), washed with $H_2O$ (20 mL × 2) and saturated NaCl solution (20 mL) successively, dried over anhydrous $Na_2SO_4$, concentrated under reduced pressure, and the concentrated solution was separated by silica gel column chromatography (eluent: DCM/EtOAc (v/v) = 1/1) to give a white solid (400 mg, 66%).
MS (ESI, pos.ion) m/z: 175.1 [M+H]+.

**Step 4: Synthesis of 4-((2*S*,4*S*)-2-((difluoromethoxy)methyl)-4-(4-(4-methyl -1*H*-imidazol-1-yl)phenoxy)pyrrolidin-1-yl)benzoic acid**

To a solution of methyl 4-((2*S*,4*S*)-2-((difluoromethoxy)methyl) -4-(4-(4-methyl-1*H*-imidazol-1-yl)phenoxy)pyrrolidin-1-yl)benzoate (400 mg, 0.87 mmol) in MeOH (2 mL) and THF (2 mL) was added a solution of LiOH·$H_2O$ (220 mg, 5.24 mmol) in $H_2O$ (2 mL). The mixture was reacted at room temperature for 18 h. The reaction solution was concentrated under reduced pressure, and HCl solution (1 mol/L) was added to the concentrated solution to adjust the pH to about 4. EtOAc (30 mL) was added, and a large amount of white solid was precipitated. The mixture was filtered and dried to obtain a white solid (300 mg, 77%).
MS (ESI, pos.ion) m/z: 444.2 $[M+H]^+$.

**Step 5: Synthesis of *N*-((*S*)-2-cyano-1-(4-(ethylsulfonyl)phenyl)ethyl)-4-((2*S*,4*S*) -2-((difluoromethoxy)methyl)-4-(4-(4-methyl-1*H*-imidazol-1-yl)phenoxy)pyrrolidin-1-yl)benza mide**

HATU (385 mg, 1.01 mmol), (*S*)-3-amino-3-(4-(ethylsulfonyl)phenyl)propionitrile hydrochloride (204 mg, 0.74 mmol), 4-((2*S*,4*S*)-2-((difluoromethoxy)methyl)-4-(4-(4-methyl-1*H*-imidazol-1-yl)phenoxy)pyrrolidin-1-yl) benzoic acid (300 mg, 0.68 mmol) and TEA (0.3 mL, 2.16 mmol) were successively added to DCM (6 mL), and the mixture was reacted at room temperature for 20 h. The reaction solution was concentrated under reduced pressure. The concentrated solution was added with DCM (40 mL), washed successively with $NaHCO_3$ solution (20 mL) and saturated NaCl solution (20 mL), dried over anhydrous $Na_2SO_4$, concentrated under reduced pressure, and the concentrated solution was separated by silica gel column chromatography (eluent: DCM/MeOH (v/v) = 20/1) to give a white solid (250 mg, 56%).

MS (ESI, pos.ion) m/z: 664.1 $[M+H]^+$.
$^1$H NMR (400 MHz, $CDCl_3$) δ (ppm): 7.91 (d, *J*= 8.3 Hz, 2H), 7.75 (d, *J*= 8.8 Hz, 2H), 7.65 (d, *J*= 8.3 Hz, 3H), 7.30 (d, *J*= 8.9 Hz, 2H), 7.00 - 6.94 (m, 3H), 6.85 (d, *J*= 7.3 Hz, 1H), 6.65 (d, *J*= 8.8 Hz, 2H), 6.23 (t, *J* = 74.3 Hz, 1H), 5.59 (dd, *J* = 12.8, 6.1 Hz, 1H), 5.14 (t, *J* = 4.6 Hz, 1H), 4.24 - 4.14 (m, 2H), 3.99 (t, *J* = 9.2 Hz, 1H), 3.77 (d, *J* = 11.3 Hz, 1H), 3.69 (dd, *J* = 11.4, 4.7 Hz, 1H), 3.18 - 3.02 (m, 4H), 2.56 - 2.49 (m, 1H), 2.43 - 2.33 (m, 1H), 2.29 (s, 3H), 1.28 (t, *J*= 7.5 Hz, 3H).

**Example 46 *N*-((*S*)-2-cyano-1-(4-(ethylsulfonyl)phenyl)ethyl)-4-((2*S*,4*S*)-2-((difluoromethoxy) methyl)-4-(((1*r*,4*S*)-4-methoxycyclohexyl)amino)pyrrolidin-1-yl)benzamide**

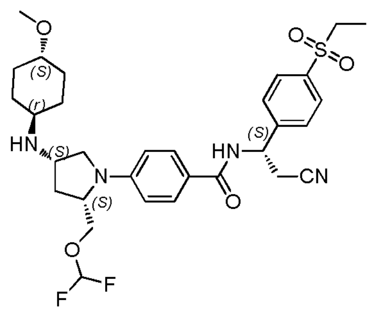

**Step 1: Synthesis of methyl 4-((2*S*,4*R*)-2-((difluoromethoxy)methyl) -4-(((trifluoromethyl)sulfonyl)oxy)pyrrolidin-1-yl)benzoate**

To a solution of methyl 4-((2*S*,4*R*)-2-((difluoromethoxy)methyl)-4-hydroxypyrrolidin-1-yl)benzoate (220 mg, 0.73 mmol) in DCM (5 mL) were added DIPEA(0.21 mL, 1.30 mmol) and $Tf_2O$ (0.16 mL, 0.95 mmol) successively. The mixture was reacted at -25°C for 4 h. The reaction solution was diluted with DCM (30 mL), washed with saturated $NH_4Cl$ (30 mL × 2) and saturated $NaHCO_3$ solution (30 mL) successively. The combined organic phases were dried over anhydrous $Na_2SO_4$, and concentrated under reduced pressure in an ice bath to obtain yellow color oil (280 mg, 88.5%).

**Step 2: Synthesis of methyl 4-((2*S*,4*S*)-2-((difluoromethoxy)methyl) -4-(((1*r*,4*S*)-4-methoxycyclohexyl)amino)pyrrolidin-1-yl)benzoate**

Methyl 4-((2*S*,4*R*)-2-((difluoromethoxy)methyl)-4-(((trifluoromethyl)sulfonyl)oxy) pyrrolidin-1-yl)benzoate (280 mg, 0.65 mmol), (1*r*,4*r*)-4-methoxycyclohexylamine (194 mg, 1.50 mmol) and $K_2CO_3$ (349 mg, 2.53 mmol) were dissolved in ACN (5 mL) and the mixture was reacted at 80 °C for 6 h. The reaction solution was concentrated under reduced pressure. The concentrated solution was extracted with saturated $NaHCO_3$ solution (20 mL) and DCM (30 mL × 2), dried over anhydrous $Na_2SO_4$, concentrated under reduced pressure, and the concentrated solution was separated by

silica gel column chromatography (eluent: DCM/MeOH (v/v) = 10/1) to give yellow oil (265 mg, 99.4%).
MS (ESI, pos.ion) m/z:413.3 [M+H]$^+$.

**Step 3: Synthesis of 4-((2*S*,4*S*)-2-((difluoromethoxy)methyl)-4-(((1*r*,4*S*)-4-methoxycyclohexyl) amino)pyrrolidin-1-yl)benzoic acid**

Methyl 4-((2*S*,4*S*)-2-((difluoromethoxy)methyl)-4-(((1*r*,4*S*)-4-methoxycyclohexyl) amino)pyrrolidin-1-yl)benzoate (265 mg, 0.64 mmol) and LiOH·$H_2O$ (296 mg, 7.05 mmol) were dissolved in THF/MeOH/$H_2O$ (2 mL/1 mL/1 mL). The mixture was reacted at room temperature for 24 h. The reaction solution was concentrated under reduced pressure, and 1.0 M HCl solution (10 mL) was added to the concentrated solution to adjust the pH to about 6. The resulting mixture was extracted with EtOAc (20 mL × 3) and DCM (20 mL × 3). The combined organic phases were dried over anhydrous $Na_2SO_4$, concentrated under reduced pressure, and the concentrated solution was separated by silica gel column chromatography (eluent: DCM/MeOH (v/v) = 7/1) to give a white solid (110 mg, 43.0 %).
MS (ESI, pos.ion) m/z:399.1 [M+H]$^+$.

**Step 4: Synthesis of *N*-((*S*)-2-cyano-1-(4-(ethylsulfonyl)phenyl)ethyl)-4-((2*S*,4*S*) -2-((difluoromethoxy)methyl)-4-(((1*r*,4*S*)-4-methoxycyclohexyl)amino)pyrrolidin-1-yl)benzami de**

4-((2*S*,4*S*)-2-((Difluoromethoxy)methyl)-4-(((1*r*,4*S*)-4-methoxycyclohexyl)amino)pyrr olidin-1-yl)benzoic acid (110 mg, 0.28 mmol), (*S*)-3-amino-3-(4-(ethylsulfonyl)phenyl)propionitrile hydrochloride (95 mg, 0.40 mmol), EDCI (163 mg, 0.85 mmol) and HOBT (123 mg, 0.91 mmol) were dissolved in DCM (5 mL), then DIPEA (0.25 mL, 1.50 mmol) was added and the mixture was reacted at room temperature for 20 h. The reaction solution was added with DCM (50 mL), washed with saturated $NH_4Cl$ (20 mL × 2) and saturated $NaHCO_3$ solution (20 mL) successively, dried over anhydrous $Na_2SO_4$, and concentrated under reduced pressure. The concentrated solution was separated by silica gel column chromatography (eluent: DCM/MeOH (v/v) = 10/1) to obtain a brown solid 60 mg, which was further separated by PTLC to obtain a brown solid ( 28 mg, 16.4%).

MS (ESI, pos.ion) m/z: 619.3 [M+H]$^+$.
$^1$H NMR (400 MHz, DMSO-$d_6$) δ (ppm): 8.87 (d, *J* = 8.2 Hz, 1H), 7.88 (d, *J* = 8.3 Hz, 2H), 7.78 (d, *J* = 8.6 Hz, 2H), 7.71 (d, *J* = 8.3 Hz, 2H), 6.88 (t, *J* = 76 Hz, 1H), 6.66 (s, 2H), 5.49 (dd, *J* = 15.6, 8.5 Hz, 1H), 4.08 (d, *J* = 5.9 Hz, 2H), 4.00 (d, *J* = 5.6 Hz, 1H), 3.50 (s, 2H), 3.47 (d, *J* = 4.3 Hz, 2H), 3.30 (d, *J* = 6.8 Hz, 2H), 3.27 (s, 1H), 3.25 (s, 1H), 3.22 (s, 3H), 3.15 (s, 1H), 3.13 (s, 1H), 3.08 (s, 1H), 2.03 - 1.86 (m, 6H), 1.31 (dd, *J* = 27.2, 12.1 Hz, 3H), 1.09 (t, *J* = 7.4 Hz, 3H).

**Example 47 *N*-((*S*)-2-cyano-1-(4-(ethylsulfonyl)phenyl)ethyl)-4-((2*S*,4*S*)-2 -((difluoromethoxy)methyl)-4-(4-(trifluoromethyl)phenoxy)pyrrolidin-1-yl)benzamide**

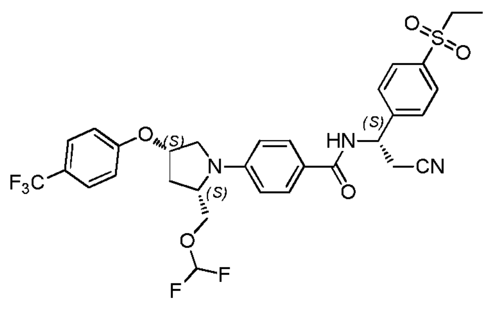

**Step 1: Synthesis of (2*S*,4*S*)-1-*tert*-butyl 2-methyl 4-(4-(trifluoromethyl)phenoxy)pyrrolidine-1,2-dicarboxylate**

(2*S*,4*R*)-1-*tert*-butyl 2-methyl 4-hydroxypyrrolidine-1,2-dicarboxylate (10.00 g, 40.77 mmol), 4-(trifluoromethyl)phenol (6.60 g, 40.71 mmol), $PPh_3$ (11.80 g, 44.99 mmol) were added to THF (120 mL). The mixture was transferred to 0 °C, and DIAD (11.0 mL, 55.87 mmol) was slowly added. After the addition was complete, the mixture was transferred to room temperature and reacted for 24 h. The reaction solution was concentrated under reduced pressure. The concentrated solution was diluted with methyl *tert*-butyl ether (80 mL) and stirred at -20 °C. A large amount of white insoluble solid was precipitated, filtered while cold, and the filter cake was washed with cold methyl *tert*-butyl ether. The filtrate

was concentrated under reduced pressure, and the concentrated solution was separated by silica gel column chromatography (eluent: PE/EtOAc (v/v) = 4/1) to obtain a white solid (13.56 g, 85%).
MS (ESI, pos.ion) m/z: 412.2 $[M+Na]^+$.

**Step 2: Synthesis of *tert-butyl* (2*S*,4*S*)-2-(hydroxymethyl) -4-(4-(trifluoromethyl)phenoxy)pyrrolidine-1-carboxylate**

To THF (200 mL) solution was added (2*S*,4*S*)-1-*tert*-butyl 2-methyl 4-(4-(trifluoromethyl)phenoxy)pyrrolidine-1,2-dicarboxylate (18.00 g, 46.22 mmol), then the mixture was transferred to 0 °C, and $LiBH_4$ (2.00 g, 91.80 mmol) was added slowly. After the addition was completed, the mixture was stirred at room temperature for 14 h. Saturated $NH_4Cl$ (60 mL) solution was added to the reaction solution to quench the reaction. The mixture was left standing for layers. The upper organic phase was separated, and the aqueous phase was extracted with EtOAc (30 mL × 2). The combined organic phases were dried over anhydrous $Na_2SO_4$, concentrated under reduced pressure, and the concentrated solution was separated by silica gel column chromatography (eluent: PE/EtOAc (v/v) = 3/1) to give a white solid (14.23 g, 85%).
MS (ESI, pos.ion) m/z: 306.2 $[M-56+H]^+$.

**Step 3: Synthesis of *tert*-butyl (2*S*,4*S*)-2-((difluoromethoxy)methyl)-4-(4-(trifluoromethyl)phenoxy)pyrrolidine-1-carboxylate**

To a solution of *tert*-butyl (2*S*,4*S*)-2-(hydroxymethyl)-4-(4-(trifluoromethyl)phenoxy)pyrrolidine-1-carboxylate (300 mg, 0.83 mmol) in DCM (1 mL) were sequentially added $H_2O$ (1 mL), KOAc (488 mg, 4.97 mmol) and $TMSCF_2Br$ (0.40 mL, 2.60 mmol). The mixture was reacted at room temperature for 24 h. The reaction solution was diluted with DCM (30 mL), washed with saturated NaCl solution (15 mL), dried over anhydrous $Na_2SO_4$, concentrated under reduced pressure, and the concentrated solution was separated by silica gel column chromatography (eluent: PE/EtOAc (v/v) = 4/1) to give colorless liquid (280 mg, 82%).
MS (ESI, pos.ion) m/z: 434.1 $[M+Na]^+$.

**Step 4: Synthesis of *tert-butyl* (2*S*,4*S*)-2-((difluoromethoxy)methyl) -4-(4-(trifluoromethyl)phenoxy)pyrrolidine**

To a solution of *tert-butyl* (2*S*,4*S*)-2-((difluoromethoxy)methyl)-4 -(4-(trifluoromethyl)phenoxy)pyrrolidine-1-carboxylate (280 mg, 0.68 mmol) in DCM (6 mL) was added a solution of HCl in methanol (2 mL, 20%). The mixture was stirred at room temperature for 24 h. The reaction solution was concentrated under reduced pressure. The concentrated solution was diluted with DCM (30 mL), washed successively with saturated $NaHCO_3$ solution (15 mL) and saturated NaCl solution (15 mL), dried over anhydrous $Na_2SO_4$, and concentrated under reduced pressure to give brown liquid (211 mg, 100%).
MS (ESI, pos.ion) m/z: 312.3 $[M+H]^+$.

**Step 5: Synthesis of methyl 4-((2*S*,4*S*)-2-((difluoromethoxy)methyl) -4-(4-(trifluoromethyl)phenoxy)pyrrolidin-1-yl)benzoate**

Under nitrogen protection, (2*S*,4*S*)-2-((difluoromethoxy)methyl)-4-(4-(trifluoromethyl) phenoxy)pyrrolidine (200 mg, 0.64 mmol), $Pd_2(dba)_3$ (58 mg, 0.06 mmol), 4,5-bis(diphenylphosphine)-9,9-dimethylxanthene (55 mg, 0.10 mmol), $Cs_2CO_3$ (418 mg, 1.28 mmol), methyl 4-iodobenzoate (252 mg, 0.96 mmol) were successively added to 1,4-dioxane (6 mL), and the mixture was reacted at 100 °C for 24 h. The reaction solution was cooled to room temperature, and concentrated under reduced pressure. The concentrated solution was diluted with DCM (50 mL), washed successively with $NaHCO_3$ solution (15 mL) and saturated NaCl solution (15 mL), dried over anhydrous $Na_2SO_4$, concentrated under reduced pressure, and the concentrated solution was separated by silica gel column chromatography (eluent: PE/EtOAc (v/v) = 5/1) to give yellow liquid (220 mg, 77%).
MS (ESI, pos.ion) m/z: 446.2 $[M+H]^+$.

**Step 6: Synthesis of 4-((2*S*,4*S*)-2-((difluoromethoxy)methyl)-4-(4-(trifluoromethyl) phenoxy)pyrrolidin-1-yl)benzoic acid**

To a solution of methyl 4-((2*S*,4*S*)-2-((difluoromethoxy)methyl) -4-(4-(trifluoromethyl)phenoxy)pyrrolidin-1-yl)benzoate (220 mg, 0.49 mmol) in MeOH (3 mL) and THF (3 mL) was added a solution of $LiOH{\cdot}H_2O$ (414 mg, 9.87 mmol) in $H_2O$ (2 mL). The mixture was stirred at room temperature for 16 h. The reaction solution was concentrated under reduced pressure, and HCl solution (1 mol/L) was added to the concentrated solution to adjust the pH to about 4. The resulting mixture was extracted with EtOAc (20 mL × 2), and the organic phases were combined, washed with

saturated NaCl (15 mL) solution, dried over anhydrous $Na_2SO_4$, concentrated under reduced pressure. The concentrated solution was separated by silica gel column chromatography (eluent: DCM/MeOH (v/v) = 20/1) to give a white solid (170 mg, 80%).
MS (ESI, pos.ion) m/z: 432.2 $[M+H]^+$.

**Step 7: Synthesis of *N*-((*S*)-2-cyano-1-(4-(ethylsulfonyl)phenyl)ethyl) -4-((2*S*,4*S*)-2-((difluoromethoxy)methyl)-4-(4-(trifluoromethyl)phenoxy)pyrrolidin-1-yl)benza mide**

EDCI (99 mg, 0.52 mmol), HOBT (70 mg, 0.52 mmol), (*S*)-3-amino-3-(4-(ethylsulfonyl)phenyl)propionitrile hydrochloride (124 mg, 0.45 mmol), 4-((2*S*,4*S*)-2-((difluoromethoxy)methyl)-4-(4-(trifluoromethyl)phenoxy)pyrrolidin-1-yl)benzoic acid (150 mg, 0.35 mmol) and TEA (105 mg, 1.04 mmol) were successively added to DCM (6 mL), and the mixture was stirred at room temperature for 22 h. The reaction solution was diluted with DCM (40 mL), washed successively with $NaHCO_3$ solution (15 mL) and saturated NaCl solution (15 mL), dried over anhydrous $Na_2SO_4$, concentrated under reduced pressure, and the concentrated solution was separated by silica gel column chromatography (eluent: DCM/EtOAc (v/v) = 4/1) to give a white solid (182 mg, 80%).

MS (ESI, pos.ion) m/z: 652.2 $[M+H]^+$.
$^1$H NMR (600 MHz, $CDCl_3$) δ (ppm): 7.93 (d, *J* = 8.3 Hz, 2H), 7.74 (d, *J* = 8.8 Hz, 2H), 7.65 (d, *J* = 8.3 Hz, 2H), 7.59 (d, *J* = 8.7 Hz, 2H), 6.97 (d, *J* = 8.6 Hz, 2H), 6.66 (d, *J* = 8.9 Hz, 2H), 6.62 (d, *J* = 7.6 Hz, 1H), 6.22 (t, *J* = 74.2 Hz, 1H), 5.59 (dd, *J* = 12.3, 6.6 Hz, 1H), 5.17 (t, *J* = 4.8 Hz, 1H), 4.24 - 4.20 (m, 1H), 4.17 (dd, *J* = 10.1, 4.3 Hz, 1H), 3.96 (t, *J* = 9.7 Hz, 1H), 3.76 (d, *J* = 11.4 Hz, 1H), 3.71 (dd, *J* = 11.4, 4.8 Hz, 1H), 3.16 (dd, *J* = 17.0, 6.5 Hz, 1H), 3.11 (t, *J* = 7.4 Hz, 2H), 3.06 (dd, *J* = 16.9, 4.9 Hz, 1H), 2.52 (d, *J* = 14.4 Hz, 1H), 2.42 - 2.36 (m, 1H), 1.29 (t, *J* = 7.4 Hz, 3H).

The target compounds in Table 2 could be prepared according to the method of Example 47 with suitable materials, for example:

1) the material (methyl 4-iodobenzoate) in step 5 of Example 47 was replaced with other reaction substrates, which were used to prepare the target compounds in Table 2 according to the methods of step 5 to step 7 of Example 47 with the intermediate ((2*S*,4*S*)-2-((difluoromethoxy)methyl)-4-(4-(trifluoromethyl)phenoxy)pyrrolidine) prepared in step 4; or
2) the material ((*S*)-3-amino-3-(4-(ethylsulfonyl)phenyl)propionitrile hydrochloride) in step 7 of Example 47 was replaced with other reaction substrates, which were used to prepare the target compounds in Table 2 according to the method of step 7 of Example 47 with the intermediate (4-((2*S*,4*S*)-2-((difluoromethoxy)methyl)-4-(4-(trifluoromethyl)phenoxy)pyrrolidin-1-yl)benzoic acid) in step 6; or
3) the material (4-(trifluoromethyl)phenol) in step 1 of Example 47 was replaced with other reaction substrates, which were used to prepare the target compounds in Table 2 according to the methods of step 1 to step 7 of Example 47 with another starting material ((2*S*,4*R*)-1-*tert*-butyl-2-methyl-4-hydroxypyrrolidine-1,2-dicarboxylate) in step 1; or
4) the reagent $TMSCF_2Br$ in step 3 of Example 47 was replaced with $TMSCF_3$, and optionally the material (methyl 4-iodobenzoate) in step 5 of Example 47 was replaced with other reaction substrates, which were used to prepare the target compounds in Table 2 according to the methods of step 3 to step 7 of Example 47 with the intermediate (*tert*-butyl (2*S*,4*S*)-2-(hydroxymethyl)-4-(4-(trifluoromethyl)phenoxypyrrolidine-1-carboxylate) prepared in step 2.

Table 2 The target compound prepared according to the synthetic method of Example 47

| Example No. | Target compound structure and name | Target compound characterization data |
|---|---|---|
| **Example 48** | 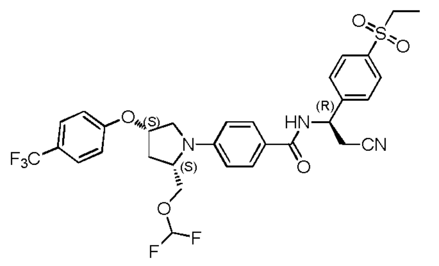 *N*-((*R*)-2-cyano-1 -(4-(ethylsulfonyl) phenyl)ethyl)-4-((2*S*,4*S*)-2-((difluor omethoxy)methyl)-4-(4-(trifluorom ethyl)phenoxy)pyrrolidin-1-yl)benz amide | pale yellow solid; MS (ESI, pos.ion) m/z: 652.2 $[M+H]^+$; $^1H$ NMR (400 MHz, $CDCl_3$) $\delta$ (ppm): 7.88 (d, *J* = 7.9 Hz, 2H), 7.74 (d, *J* = 8.8 Hz, 2H), 7.63 (d, *J* = 8.2 Hz, 2H), 7.58 (d, *J* = 8.7 Hz, 2H), 6.97 (d, *J* = 8.6 Hz, 2H), 6.88 (d, *J* = 7.7 Hz, 1H), 6.64 (d, *J* = 8.8 Hz, 2H), 6.22 (t, *J* = 74.3 Hz, 1H), 5.58 (dd, *J* = 13.1, 6.2 Hz, 1H), 5.17 (t, *J* = 4.5 Hz, 1H), 4.23 - 4.13 (m, 2H), 3.96 (t, *J* = 9.3 Hz, 1H), 3.75 (d, *J* = 11.3 Hz, 1H), 3.70 (dd, *J* = 11.5, 4.6 Hz, 1H), 3.16 - 3.01 (m, 4H), 2.51 (d, *J* = 14.4 Hz, 1H), 2.43 - 2.35 (m, 1H), 1.27 (t, *J* = 7.4 Hz, 3H). |
| **Example 49** | 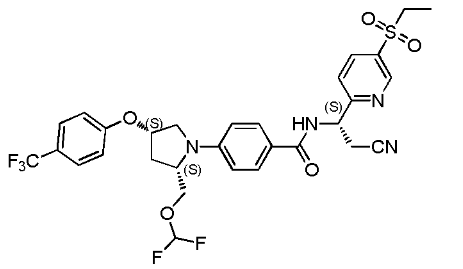 *N*-((*S*)-2-cyano-1-(5-(ethylsulfonyl) pyridin-2-yl)ethyl)-4-((2*S*,4*S*)-2-((d ifluoromethoxy)methyl)-4-(4-(triflu oromethyl)phenoxy)pyrrolidin-1-yl )benzamide | white solid; MS (ESI, pos.ion) m/z: 653.1 $[M+H]^+$; $^1H$ NMR (600 MHz, $CDCl_3$) $\delta$ (ppm): 8.78 (s, 1H), 8.04 (d, *J* = 0.8 Hz, 2H), 7.75 (d, *J*= 8.8 Hz, 2H), 7.58 (d, *J* = 8.6 Hz, 2H), 7.00 (dd, *J* = 7.6, 4.9 Hz, 1H), 6.97 (d, *J* = 8.6 Hz, 2H), 6.64 (d, *J* = 8.6 Hz, 2H), 6.28 (d, *J* = 74.2 Hz, 1H), 5.65 - 5.61 (m, 1H), 5.17 (t, *J* = 4.8 Hz, 1H), 4.23 - 4.20 (m, 1H), 4.16 (dd, *J* = 10.0, 4.3 Hz, 1H), 3.96 (t, *J* = 9.7 Hz, 1H), 3.75 (d, *J* = 11.4 Hz, 1H), 3.71 (dd, *J* = 11.4, 4.7 Hz, 1H), 3.39 (q, *J*= 7.4 Hz, 2H), 3.17 - 3.13 (m, 1H), 3.10 (dd, *J* = 17.0, 5.9 Hz, 1H), 2.51 (d, *J* = 14.4 Hz, 1H), 2.42 - 2.37 (m, 1H), 1.30 (t, *J* = 7.4 Hz, 3H). |
| **Example 50** | 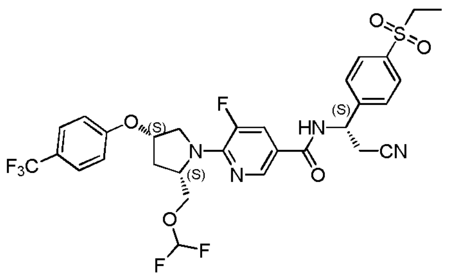 *N*-((*S*)-2-cyano-1-(4-(ethylsulfonyl) phenyl)ethyl)-6-((2*S*,4*S*)-2-((difluor omethoxy)methyl)-4-(4-(trifluorom ethyl)phenoxy)pyrrolidin-1-yl)-5-fl uoronicotinamide | white solid; MS (ESI, pos.ion) m/z: 671.2 $[M+H]^+$; $^1H$ NMR (400 MHz, $CDCl_3$) $\delta$ (ppm): 8.45 (s, 1H), 7.86 (d, *J* = 8.3 Hz, 2H), 7.70 (dd, *J*= 13.9, 1.6 Hz, 1H), 7.63 (d, *J* = 8.3 Hz, 2H), 7.57 (d, *J* = 8.6 Hz, 2H), 7.00 (d, *J*= 7.6 Hz, 1H), 6.96 (d, *J* = 8.6 Hz, 2H), 6.19 (t, *J* = 74.6 Hz, 1H), 5.59 (dd, *J* = 13.1, 6.2 Hz, 1H), 5.11 (s, 1H), 4.76 (s, 1H), 4.25 (dd, *J* = 9.3, 4.3 Hz, 1H), 4.12 (s, 2H), 3.98 (t, *J* = 9.4 Hz, 1H), 3.19 - 3.04 (m, 4H), 2.50 (d, *J* = 14.3 Hz, 1H), 2.39 - 2.32 (m, 1H), 1.27 (t, *J* = 7.4 Hz, 3H). |

(continued)

| Example No. | Target compound structure and name | Target compound characterization data |
|---|---|---|
| **Example 51** | 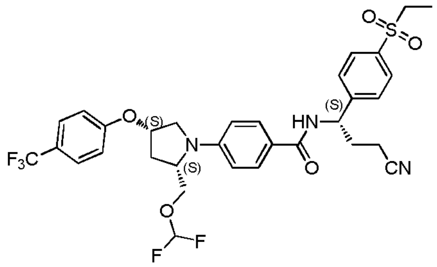 *N*-((*S*)-3-cyano-1-(4-(ethylsulfonyl) phenyl)propyl)-4-((2*S*,4*S*)-2-((diflu oromethoxy)methyl)-4-(4-(trifluoro methyl)phenoxy)pyrrolidin-1-yl)be nzamide | pale yellow solid; MS (ESI, pos.ion) m/z: 666.2 $[M+H]^+$; $^1$H NMR (400 MHz, $CDCl_3$) δ (ppm): 7.89 (d, *J* = 6.5 Hz, 2H), 7.77 (d, *J* = 8.7 Hz, 2H), 7.59 (t, *J* = 9.3 Hz, 4H), 6.99 (d, *J* = 8.6 Hz, 2H), 6.66 (d, *J* = 8.4 Hz, 3H), 6.24 (t, *J* = 74.3 Hz, 1H), 5.39 (d, *J* = 6.9 Hz, 1H), 5.19 (t, *J* = 4.5 Hz, 1H), 4.25 - 4.16 (m, 2H), 3.97 (t, *J* = 9.4 Hz, 1H), 3.78 (d, *J* = 11.4 Hz, 1H), 3.71 (dd, *J* = 11.5, 4.6 Hz, 1H), 3.12 (q, *J* = 7.4 Hz, 2H), 2.56 - 2.46 (m, 3H), 2.45 - 2.28 (m, 3H), 1.31 (t, *J* = 7.4 Hz, 3H). |
| **Example 52** | 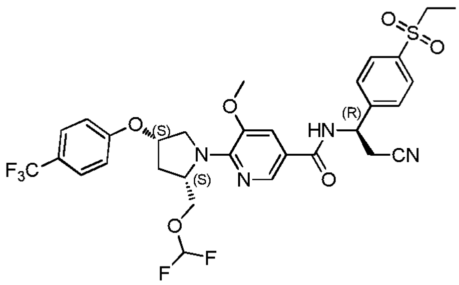 *N*-((*R*)-2-cyano-1 -(4-(ethylsulfonyl) phenyl)ethyl)-6-((2*S*,4*S*)-2-((difluor omethoxy)methyl)-4-(4-(trifluorom ethyl)phenoxy)pyrrolidin-1-yl)-5-m ethoxynicotinamide | pale yellow solid; MS (ESI, pos.ion) m/z: 683.3 $[M+H]^+$; $^1$H NMR (400 MHz, $CDCl_3$) δ (ppm): 8.22 (s, 1H), 7.89 (d, *J* = 8.3 Hz, 2H), 7.64 (d, *J* = 8.3 Hz, 2H), 7.56 (d, *J* = 8.6 Hz, 2H), 7.42 (d, *J* = 1.4 Hz, 1H), 6.96 (d, *J* = 8.6 Hz, 2H), 6.91 (d, *J* = 7.5 Hz, 1H), 6.18 (t, *J* = 74.8 Hz, 1H), 5.59 (dd, *J* = 13.2, 6.3 Hz, 1H), 5.04 (s, 1H), 4.90 (s, 1H), 4.24 (dd, *J* = 9.4, 4.2 Hz, 1H), 4.17 - 4.08 (m, 2H), 3.93 (t, *J* = 9.4 Hz, 1H), 3.82 (s, 3H), 3.18 - 3.04 (m, 4H), 2.44 (d, *J* = 14.1 Hz, 1H), 2.37 - 2.31 (m, 1H), 1.28 (t, *J*= 7.4 Hz, 3H). |
| **Example 53** | 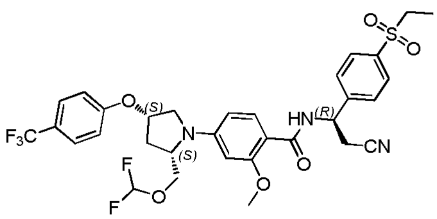 *N*-((*R*)-2-cyano-1 -(4-(ethylsulfonyl) phenyl)ethyl)-4-((2*S*,4*S*)-2-((difluor omethoxy)methyl)-4-(4-(trifluorom ethyl)phenoxy)pyrrolidin-1-yl)-2-m ethoxybenzamide | pale yellow solid; MS (ESI, pos.ion) m/z: 682.2 $[M+H]^+$; $^1$H NMR (400 MHz, $CDCl_3$) δ (ppm): 8.63 (d, *J* = 7.6 Hz, 1H), 8.05 (d, *J* = 8.8 Hz, 1H), 7.95 (d, *J* = 8.3 Hz, 2H), 7.64 (d, *J* = 8.3 Hz, 2H), 7.59 (d, *J* = 8.6 Hz, 2H), 6.98 (d, *J* = 8.6 Hz, 2H), 6.34 (dd, *J* = 8.9, 1.9 Hz, 1H), 6.24 (t, *J* = 74.4, Hz, 1H), 6.21 (s, 1H), 5.63 (dd, *J* = 11.1, 6.5 Hz, 1H), 5.18 (t, *J* = 4.3 Hz, 1H), 4.22 (t, *J* = 8.0 Hz, 2H), 4.01 (s, 3H), 3.95 (d, *J* = 11.2 Hz, 1H), 3.74 (dt, *J* = 11.4, 7.9 Hz, 2H), 3.20 - 3.08 (m, 3H), 2.97 (dd, *J* = 16.8, 4.2 Hz, 1H), 2.52 (d, *J* = 14.4 Hz, 1H), 2.42 (dd, *J* = 14.3, 5.4 Hz, 1H), 1.30 (t, *J*= 7.4 Hz, 3H). |

(continued)

| Example No. | Target compound structure and name | Target compound characterization data |
|---|---|---|
| Example 54 | 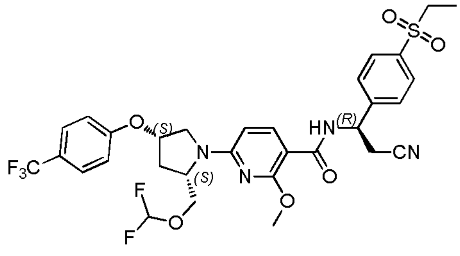 *N*-((*R*)-2-cyano-1 -(4-(ethylsulfonyl) phenyl)ethyl)-6-((2*S*,4*S*)-2-((difluor omethoxy)methyl)-4-(4-(trifluorom ethyl)phenoxy)pyrrolidin-1-yl)-2-m ethoxynicotinamide | pale yellow solid; MS (ESI, pos.ion) m/z: 683.2 [M+H] $^+$; $^1$H NMR (400 MHz, $CDCl_3$) δ (ppm): 8.53 (d, *J* = 7.5 Hz, 1H), 8.25 (d, *J* = 8.5 Hz, 1H), 7.96 (d, *J* = 8.3 Hz, 2H), 7.64 (d, *J* = 8.3 Hz, 2H), 7.59 (d, *J* = 8.6 Hz, 2H), 6.97 (d, *J* = 8.5 Hz, 2H), 6.21 (t, *J* = 74.4 Hz, 1H), 6.10 (d, *J* = 8.5 Hz, 1H), 5.59 (dd, *J* = 11.6, 6.3 Hz, 1H), 5.17 (s, 1H), 4.53 (d, *J* = 37.9 Hz, 2H), 4.09 (s, 3H), 3.92 (t, *J* = 9.6 Hz, 1H), 3.81 (s, 2H), 3.19 - 3.07 (m, 3H), 2.98 (dd, *J* = 16.8, 4.4 Hz, 1H), 2.55 (d, *J* = 14.5 Hz, 1H), 2.42 - 2.31 (m, 1H), 1.30 (t, *J* = 7.4 Hz, 3H). |
| Example 55 | 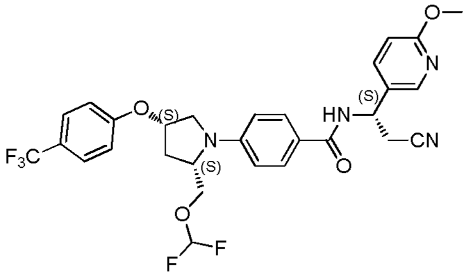 *N*-((*S*)-2-cyano-1-(6-methoxypyridi ne-3-yl)ethyl)-4-((2*S*,4*S*)-2-((difluo romethoxy)methyl)-4-(4-trifluorom ethyl)phenoxy)pyrrolidin-1-yl)benz amide | pale yellow solid; MS (ESI, pos.ion) m/z: 591.2 [M+H] $^+$; $^1$H NMR (400 MHz, $CDCl_3$) δ (ppm): 8.27 (d, *J* = 2.4 Hz, 1H), 7.70 (d, *J* = 8.8 Hz, 3H), 7.59 (d, *J* = 8.6 Hz, 2H), 6.97 (d, *J* = 8.5 Hz, 2H), 6.81 (d, *J* = 8.6 Hz, 1H), 6.64 (d, *J* = 8.8 Hz, 2H), 6.38 (d, *J* = 7.2 Hz, 1H), 6.21 (t, *J* = 74.3 Hz, 1H), 5.43 (dd, *J* = 11.3, 6.8 Hz, 1H), 5.17 (t, *J* = 4.7 Hz, 1H), 4.24 - 4.13 (m, 2H), 3.98 - 3.93 (m, 4H), 3.76 (d, *J* = 11.3 Hz, 1H), 3.70 (dd, *J* = 11.4, 4.7 Hz, 1H), 3.17 (dd, *J* = 16.8, 6.7 Hz, 1H), 3.02 (dd, *J* = 16.8, 4.4 Hz, 1H), 2.52 (d, *J* = 14.4 Hz, 1H), 2.42 - 2.34 (m, 1H). |
| Example 56 | 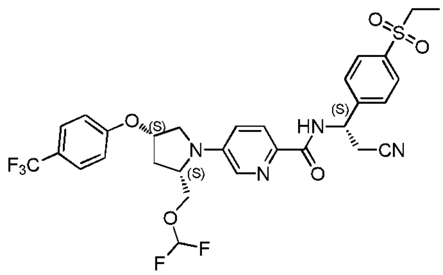 *N*-((*S*)-2-cyano-1-(4-(ethylsulfonyl) phenyl)ethyl)-5-((2*S*,4*S*)-2-((difluor omethoxy)methyl)-4-(4-(trifluorom ethyl)phenoxy)pyrrolidin-1-yl)picol inamide | pale yellow solid; MS (ESI, pos.ion) m/z: 653.2 [M+H] $^+$; $^1$H NMR (400 MHz, $CDCl_3$) δ (ppm): 8.43 (d, *J* = 8.1 Hz, 1H), 8.03 (d, *J* = 8.8 Hz, 1H), 8.00 (d, *J* = 2.7 Hz, 1H), 7.96 (d, *J* = 8.3 Hz, 2H), 7.68 (d, *J* = 8.3 Hz, 2H), 7.60 (d, *J* = 8.6 Hz, 2H), 7.02 (d, *J* = 2.8 Hz, 1H), 6.98 (d, *J* = 8.9 Hz, 2H), 6.23 (t, *J* = 73.9 Hz, 1H), 5.57 (dd, *J* = 13.8, 6.1 Hz, 1H), 5.20 (t, *J* = 4.7 Hz, 1H), 4.30 - 4.25 (m, 1H), 4.18 (dd, *J* = 10.1, 5.0 Hz, 1H), 3.99 (t, *J* = 9.1 Hz, 1H), 3.81 (d, *J* = 11.4 Hz, 1H), 3.72 (dd, *J* = 11.4, 4.8 Hz, 1H), 3.16 - 3.03 (m, 4H), 2.52 (d, *J* = 14.3 Hz, 1H), 2.45 - 2.38 (m, 1H), 1.29 (t, *J* = 7.4 Hz, 4H). |
| Example 57 | 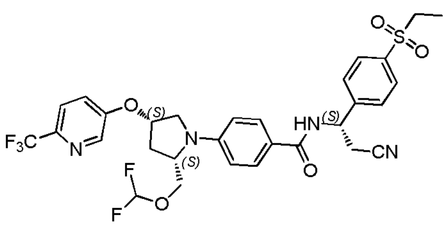 *N*-((*S*)-2-cyano-1-(4-(ethylsulfonyl) phenyl)ethyl)-4-((2*S*,4*S*)-2-((difluor omethoxy)methyl)-4-((6-(trifluoro methyl)pyridine-3-yl)oxy)pyrrolidi n-1-yl)benzamide | pale yellow solid; MS (ESI, pos.ion) m/z: 653.2 [M+H] $^+$; $^1$H NMR (400 MHz, $CDCl_3$) δ (ppm): 8.63 (d, *J* = 7.6 Hz, 1H), 8.05 (d, *J* = 8.8 Hz, 1H), 7.95 (d, *J* = 8.3 Hz, 2H), 7.64 (d, *J* = 8.3 Hz, 2H), 7.59 (d, *J* = 8.6 Hz, 2H), 6.98 (d, *J* = 8.6 Hz, 2H), 6.34 (dd, *J* = 8.9, 1.9 Hz, 1H), 6.24 (t, *J* = 74.4, Hz, 1H), 6.21 (s, 1H), 5.63 (dd, *J* = 11.1, 6.5 Hz, 1H), 5.18 (t, *J* = 4.3 Hz, 1H), 4.22 (t, *J* = 8.0 Hz, 2H), 4.01 (s, 3H), 3.95 (d, *J* = 11.2 Hz, 1H), 3.74 (dt, *J* = 11.4, 7.9 Hz, 2H), 3.20 - 3.08 (m, 3H), 2.97 (dd, *J* = 16.8, 4.2 Hz, 1H), 2.52 (d, *J* = 14.4 Hz, 1H), 2.42 (dd, *J* = 14.3, 5.4 Hz, 1H), 1.30 (t, *J* = 7.4 Hz, 3H). |

(continued)

| Example No. | Target compound structure and name | Target compound characterization data |
|---|---|---|
| **Example 58** | 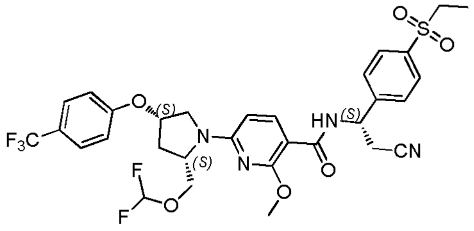 *N*-((*S*)-2-cyano-1-(4-(ethylsulfonyl) phenyl)ethyl)-6-((2*S*,4*S*)-2-((difluor omethoxy)methyl)-4-(4-(trifluorom ethyl)phenoxy)pyrrolidin-1-yl)-2-m ethoxynicotinamide | pale yellow solid; MS (ESI, pos.ion) m/z: 683.2 $[M+H]^+$; $^1H$ NMR (400 MHz, $CDCl_3$) δ (ppm): 8.53 (d, *J*= 7.5 Hz, 1H), 8.25 (d, *J* = 8.5 Hz, 1H), 7.96 (d, *J* = 8.3 Hz, 2H), 7.64 (d, *J* = 8.3 Hz, 2H), 7.59 (d, *J* = 8.6 Hz, 2H), 6.97 (d, *J* = 8.5 Hz, 2H), 6.21 (t, *J* = 74.4 Hz, 1H), 6.10 (d, *J* = 8.5 Hz, 1H), 5.59 (dd, *J* = 11.6, 6.3 Hz, 1H), 5.17 (s, 1H), 4.53 (d, *J*= 37.9 Hz, 2H), 4.09 (s, 3H), 3.92 (t, *J* = 9.6 Hz, 1H), 3.81 (s, 2H), 3.19 - 3.07 (m, 3H), 2.98 (dd, *J* = 16.8, 4.4 Hz, 1H), 2.55 (d, *J* = 14.5 Hz, 1H), 2.42 - 2.31 (m, 1H), 1.30 (t, *J* = 7.4 Hz, 3H). |
| **Example 59** | 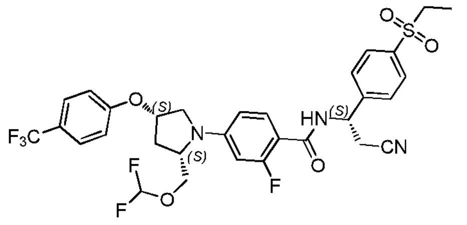 *N*-((*S*)-2-cyano-1-(4-(ethylsulfonyl) phenyl)ethyl)-4-((2*S*,4*S*)-2-((difluor omethoxy)methyl)-4-(4-(trifluorom ethyl)phenoxy)pyrrolidin-1-yl)-2-fl uorobenzamide | white solid; MS (ESI, pos.ion) m/z: 670.1 $[M+H]^+$; $^1H$ NMR (400 MHz, $CDCl_3$) δ (ppm): 8.02 - 7.88 (m, 3H), 7.65 (d, *J* = 8.2 Hz, 2H), 7.59 (d, *J* = 8.6 Hz, 2H), 7.20 (dd, *J* = 15.3, 7.4 Hz, 1H), 6.97 (d, *J* = 8.5 Hz, 2H), 6.51 (dd, *J* = 8.9, 1.9 Hz, 1H), 6.33 (d, *J* = 15.9 Hz, 1H), 6.22 (t, *J* = 74.0 Hz, 1H), 5.61 (d, *J* = 6.1 Hz, 1H), 5.17 (t, *J* = 4.1 Hz, 1H), 4.18 (dq, *J* = 13.8, 4.6 Hz, 2H), 3.97 (t, *J* = 9.1 Hz, 1H), 3.79 - 3.67 (m, 2H), 3.19 - 3.08 (m, 3H), 3.04 (dd, *J*= 16.9, 5.1 Hz, 1H), 2.51 (d, *J* = 14.4 Hz, 1H), 2.45 - 2.35 (m, 1H), 1.29 (t, *J* = 7.4 Hz, 3H). |
| **Example 60** | 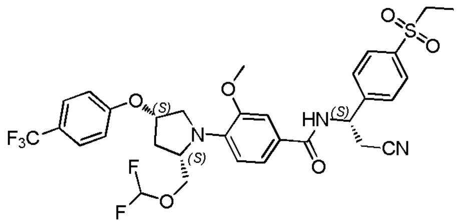 *N*-((*S*)-2-cyano-1-(4-(ethylsulfonyl) phenyl)ethyl)-4-((2*S*,4*S*)-2-((difluor omethoxy)methyl)-4-(4-(trifluorom ethyl)phenoxy)pyrrolidin-1-yl)-3-m ethoxybenzamide | white solid; MS (ESI, pos.ion) m/z: 682.2 $[M+H]^+$; $^1H$ NMR (400 MHz, $CDCl_3$) δ (ppm): 7.89 (d, *J* = 8.2 Hz, 2H), 7.64 (d, *J* = 8.2 Hz, 2H), 7.57 (d, *J* = 8.5 Hz, 2H), 7.41 (s, 1H), 7.30 (d, *J* = 8.3 Hz, 1H), 6.98 (d, *J* = 8.5 Hz, 2H), 6.84 (d, *J* = 7.6 Hz, 1H), 6.69 (d, *J* = 8.4 Hz, 1H), 6.12 (t, *J* = 74.5 Hz, 1H), 5.59 (dd, *J* = 12.8, 6.1 Hz, 1H), 5.05 (s, 1H), 4.66 (dd, *J* = 7.6, 3.9 Hz, 1H), 3.99 (dd, *J* = 9.8, 3.8 Hz, 1H), 3.86 (s, 4H), 3.83 - 3.76 (m, 1H), 3.71 (dd, *J* = 10.8, 5.9 Hz, 1H), 3.19 - 3.03 (m, 4H), 2.58 - 2.49 (m, 1H), 2.30 (d, *J* = 13.8 Hz, 1H), 1.27 (t, *J* = 7.5 Hz, 3H). |
| **Example 61** | 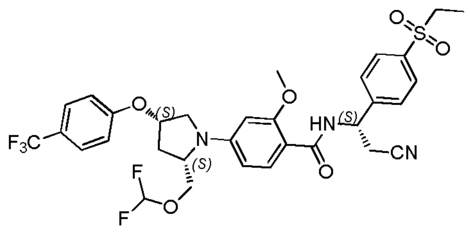 *N*-((*S*)-2-cyano-1-(4-(ethylsulfonyl) phenyl)ethyl)-4-((2*S*,4*S*)-2-((difluor omethoxy)methyl)-4-(4-(trifluorom ethyl)phenoxy)pyrrolidin-1-yl)-2-m ethoxybenzamide | white solid; MS (ESI, pos.ion) m/z: 682.2 $[M+H]^+$; $^1H$ NMR (400 MHz, $CDCl_3$) δ (ppm): 8.64 (d, *J* = 7.6 Hz, 1H), 8.05 (d, *J* = 8.8 Hz, 1H), 7.95 (d, *J* = 8.2 Hz, 2H), 7.64 (d, *J* = 8.2 Hz, 2H), 7.59 (d, *J* = 8.5 Hz, 2H), 6.97 (d, *J* = 8.5 Hz, 2H), 6.34 (d, *J* = 8.7 Hz, 1H), 6.24 (t, *J* = 74.4 Hz, 1H), 6.21 (s, 1H), 5.63 (dd, *J* = 11.2, 6.2 Hz, 1H), 5.18 (s, 1H), 4.23 (d, *J* = 7.7 Hz, 2H), 4.05 - 3.98 (m, 3H), 3.95 (d, *J* = 11.0 Hz, 1H), 3.80 - 3.69 (m, 2H), 3.20 - 3.07 (m, 3H), 2.97 (dd, *J* = 16.9, 4.0 Hz, 1H), 2.52 (d, *J* = 14.3 Hz, 1H), 2.42 (d, *J* = 5.8 Hz, 1H), 1.30 (t, *J* = 7.4 Hz, 3H). |

(continued)

| Example No. | Target compound structure and name | Target compound characterization data |
|---|---|---|
| **Example 62** | 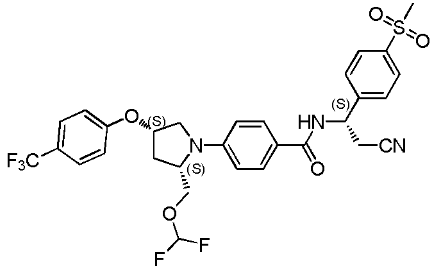 *N*-((*S*)-2-cyano-1-(4-(methylsulfon yl)phenyl)ethyl)-4-((2*S*,4*S*)-2-((difl uoromethoxy)methyl)-4-(4-(trifluor omethyl)phenoxy)pyrrolidin-1 -yl)b enzamide | pale yellow solid; MS (ESI, pos.ion) m/z: 638.1 $[M+H]^+$; $^1H$ NMR (400 MHz, $CDCl_3$) δ (ppm): 7.96 (d, *J* = 8.3 Hz, 2H), 7.73 (d, *J* = 8.8 Hz, 2H), 7.65 (d, *J* = 8.3 Hz, 2H), 7.59 (d, *J* = 8.6 Hz, 2H), 6.97 (d, *J* = 8.6 Hz, 2H), 6.69 (d, *J* = 7.6 Hz, 1H), 6.65 (d, *J* = 8.8 Hz, 2H), 6.22 (t, *J* = 74.2 Hz, 1H), 5.58 (dd, *J* = 12.6, 6.3 Hz, 1H), 5.17 (t, *J* = 4.6 Hz, 1H), 4.24 - 4.13 (m, 2H), 3.96 (t, *J* = 9.4 Hz, 1H), 3.76 (d, *J* = 11.4 Hz, 1H), 3.70 (dd, *J* = 11.4, 4.6 Hz, 1H), 3.15 (dd, *J* = 16.9, 6.4 Hz, 1H), 3.09 - 3.01 (m, 4H), 2.52 (d, *J* = 14.3 Hz, 1H), 2.44 - 2.34 (m, 1H). |
| **Example 63** | 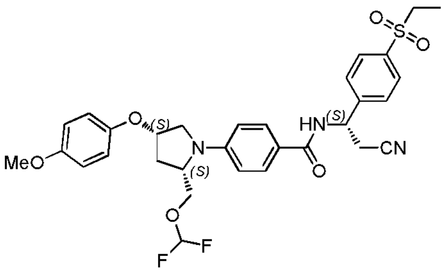 *N*-((*S*)-2-cyano-1-(4-(ethylsulfonyl) phenyl)ethyl)-4-((2*S*,4*S*)-2-((difluor omethoxy)methyl)-4-(4-methoxyph enoxy)pyrrolidin-1-yl)benzamide | white solid; MS (ESI, pos.ion) m/z: 614.15 $[M+H]^+$; $^1H$ NMR (400 MHz, $CDCl_3$) δ (ppm): 7.88 (d, *J* = 8.3 Hz, 2H), 7.76 (d, *J* = 8.7 Hz, 2H), 7.65 (d, *J* = 8.3 Hz, 2H), 7.11 (d, *J* = 7.7 Hz, 1H), 6.92 - 6.82 (m, 4H), 6.63 (d, *J* = 8.8 Hz, 2H), 6.24 (t, *J* = 74.6 Hz, 1H), 5.60 (dd, *J* = 13.4, 6.3 Hz, 1H), 5.03 (t, *J* = 4.6 Hz, 1H), 4.17 (d, *J* = 7.0 Hz, 2H), 4.02 (t, *J* = 10.7 Hz, 1H), 3.79 (s, 3H), 3.74 (d, *J* = 11.3 Hz, 1H), 3.61 (dd, *J* = 11.2, 4.8 Hz, 1H), 3.11 (dd, *J* = 14.9, 7.3 Hz, 4H), 2.49 (d, *J* = 14.2 Hz, 1H), 2.38 - 2.25 (m, 1H), 1.28 (t, *J* = 7.4 Hz, 3H). |
| **Example 64** | 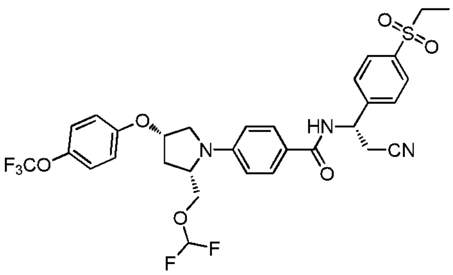 *N*-((*S*)-2-cyano-1-(4-(ethylsulfonyl) phenyl)ethyl)-4-((2*S*,4*S*)-2-((difluor omethoxy)methyl)-4-(4-(trifluorom ethoxy)phenoxy)pyrrolidin-1-yl)be nzamide | white solid; MS (ESI, pos.ion) m/z: 668.15 $[M+H]^+$; $^1H$ NMR (400 MHz, $CDCl_3$) δ (ppm): 7.92 (d, *J* = 8.1 Hz, 2H), 7.76 (d, *J* = 8.5 Hz, 2H), 7.66 (d, *J* = 8.1 Hz, 2H), 7.20 (d, *J* = 8.5 Hz, 2H), 6.92 (d, *J* = 8.9 Hz, 2H), 6.82 (d, *J* = 7.5 Hz, 1H), 6.66 (d, *J* = 8.5 Hz, 2H), 6.24 (t, *J* = 74.3 Hz, 1H), 5.61 (d, *J* = 6.4 Hz, 1H), 5.11 (s, 1H), 4.18 (dd, *J* = 22.0, 12.2 Hz, 2H), 3.99 (t, *J* = 9.2 Hz, 1H), 3.77 (d, *J* = 11.3 Hz, 1H), 3.69 (dd, *J* = 11.2, 4.4 Hz, 1H), 3.23 - 2.97 (m, 4H), 2.52 (d, *J* = 14.2 Hz, 1H), 2.39 (dd, *J* = 12.8, 6.5 Hz, 1H), 1.34 - 1.27 (m, 3H). |

(continued)

| Example No. | Target compound structure and name | Target compound characterization data |
|---|---|---|
| **Example 65** | 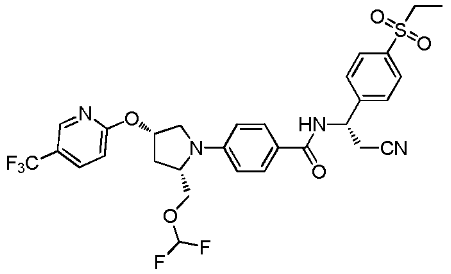<br>*N*-((*S*)-2-cyano-1-(4-(ethylsulfonyl) phenyl)ethyl)-4-((2*S*,4*S*)-2-((difluor omethoxy)methyl)-4-((5-(trifluoro methyl)pyridine-2-yl)oxy)pyrrolidi n-1-yl)benzamide | pale yellow solid; MS (ESI, pos.ion) m/z: 653.1 $[M+H]^+$; $^1H$ NMR (400 MHz, $CDCl_3$) δ (ppm): 8.48 (s, 1H), 7.90 (d, *J* = 8.1 Hz, 2H), 7.83 (d, *J* = 8.6 Hz, 1H), 7.76 (d, *J* = 8.6 Hz, 2H), 7.65 (d, *J* = 8.1 Hz, 2H), 6.87 (dd, *J* = 16.2, 8.2 Hz, 2H), 6.66 (d, *J* = 8.6 Hz, 2H), 6.26 (t, *J* = 74.3 Hz, 1H), 5.86 (s, 1H), 5.61 (dd, *J* = 12.8, 6.1 Hz, 1H), 4.18 (dd, *J* = 17.7, 7.5 Hz, 2H), 3.99 (t, *J* = 9.5 Hz, 1H), 3.82 - 3.67 (m, 2H), 3.18 - 3.03 (m, 4H), 2.46 (dt, *J* = 14.7, 10.8 Hz, 2H), 1.28 (d, *J* = 7.0 Hz, 3H). |
| **Example 66** | 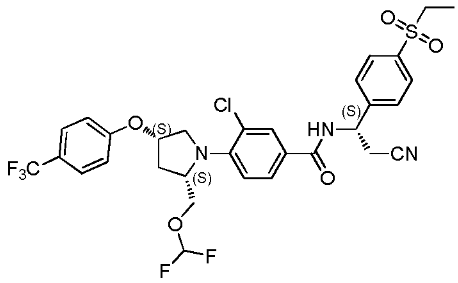<br>3-chloro-*N*-((*S*)-2-cyano-1-(4-(ethyl sulfonyl)phenyl)ethyl)-4-((2*S*,4*S*)-2 -((difluoromethoxy)methyl)-4-(4-(tr ifluoromethyl)phenoxy)pyrrolidin-1 -yl)benzamide | pale yellow solid; MS (ESI, pos.ion) m/z: 686.5 $[M+H]^+$; $^1H$ NMR (400 MHz, $CDCl_3$) δ (ppm): 7.89 (d, *J* = 8.3 Hz, 2H), 7.82 (d, *J* = 2.0 Hz, 1H), 7.65 (dd, *J*= 11.5, 5.1 Hz, 3H), 7.57 (d, *J* = 8.6 Hz, 2H), 6.99 (t, *J* = 7.6 Hz, 3H), 6.86 (d, *J* = 7.5 Hz, 1H), 6.10 (t, *J* = 74.3 Hz, 1H), 5.59 (dd, *J* = 13.0, 6.3 Hz, 1H), 5.06 - 4.99 (m, 1H), 4.69 - 4.63 (m, 1H), 3.99 (dd, *J* = 10.3, 5.3 Hz, 1H), 3.91 (dd, *J* = 10.2, 4.1 Hz, 1H), 3.79 (dd, *J*= 10.2, 6.7 Hz, 1H), 3.64 (dd, *J* = 10.2, 6.3 Hz, 1H), 3.20 - 3.04 (m, 4H), 2.73 - 2.64 (m, 1H), 2.27 - 2.21 (m, 1H), 1.28 (t, *J* = 7.4 Hz, 3H). |
| **Example 67** | 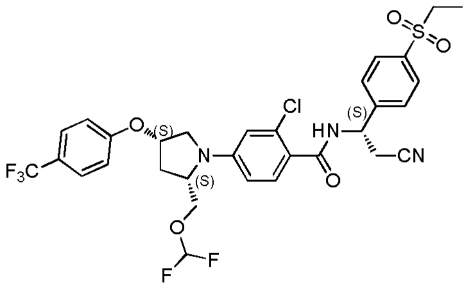<br>2-chloro-*N*-((*S*)-2-cyano-1-(4-(ethyl sulfonyl)phenyl)ethyl)-4-((2*S*,4*S*)-2 -((difluoromethoxy)methyl)-4-(4-(tr ifluoromethyl)phenoxy)pyrrolidin-1 -yl)benzamide | pale yellow solid; MS (ESI, pos.ion) m/z: 686.5 $[M+H]^+$; $^1H$ NMR (400 MHz, $CDCl_3$) δ (ppm): 7.97 (d, *J* = 8.3 Hz, 2H), 7.86 (d, *J* = 9.3 Hz, 1H), 7.67 (d, *J* = 8.3 Hz, 2H), 7.59 (d, *J* = 8.6 Hz, 2H), 7.37 (d, *J* = 7.3 Hz, 1H), 6.97 (d, *J* = 8.6 Hz, 2H), 6.62 (d, *J* = 7.4 Hz, 2H), 6.22 (t, *J* = 74.0 Hz, 1H), 5.60 (dd, *J*= 12.0, 6.4 Hz, 1H), 5.17 (t, *J* = 4.5 Hz, 1H), 4.22 - 4.12 (m, 2H), 3.97 (t, *J* = 9.4 Hz, 1H), 3.74 (d, *J* = 11.3 Hz, 1H), 3.69 (dd, *J* = 11.5, 4.7 Hz, 1H), 3.20 - 3.10 (m, 3H), 3.03 (dd, *J* = 16.9, 4.8 Hz, 1H), 2.51 (d, *J* = 14.4 Hz, 1H), 2.44 - 2.35 (m, 1H), 1.29 (d, *J* = 7.4 Hz, 3H) |

(continued)

| Example No. | Target compound structure and name | Target compound characterization data |
|---|---|---|
| **Example 68** | 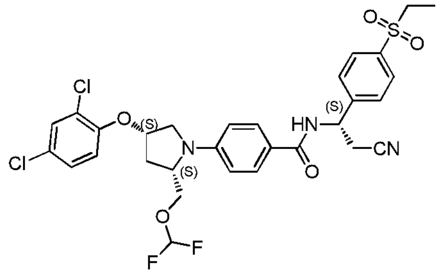 *N*-((*S*)-2-cyano-1-(4-(ethylsulfonyl) phenyl)ethyl)-4-((2*S*,4*S*)-4-(2,4-dic hlorophenoxy)-2-((difluoromethoxy )methyl)pyrrolidin-1-yl)benzamide | pale yellow solid; MS (ESI, pos.ion) m/z: 654.2 [M+H]$^+$; $^1$H NMR (400 MHz, $CDCl_3$) δ (ppm): 7.92 (d, *J* = 8.3 Hz, 2H), 7.74 (d, *J* = 8.8 Hz, 2H), 7.65 (d, *J* = 8.3 Hz, 2H), 7.32 (d, *J* = 8.3 Hz, 1H), 6.94 (dd, *J* = 11.1, 2.6 Hz, 2H), 6.70 (d, *J* = 7.6 Hz, 1H), 6.65 (d, *J* = 8.8 Hz, 2H), 6.24 (t, *J* = 74.6 Hz, 1H), 5.59 (dd, *J* = 12.6, 6.3 Hz, 1H), 5.14 (t, *J* = 4.6 Hz, 1H), 4.26 - 4.10 (m, 3H), 3.76 (d, *J* = 11.5 Hz, 1H), 3.67 (dd, *J* = 11.6, 4.5 Hz, 1H), 3.19 - 3.02 (m, 4H), 2.57 (d, *J* = 14.4 Hz, 1H), 2.45 - 2.36 (m, 1H), 1.28 (t, *J* = 7.4 Hz, 3H). |
| **Example 69** | 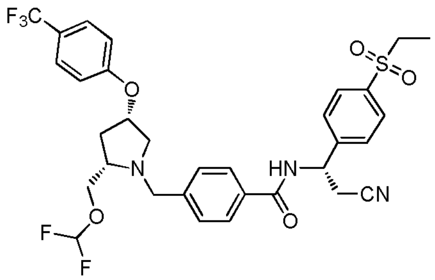 *N*-((*S*)-2-cyano-1-(4-(ethylsulfonyl) phenyl)ethyl)-4-((2*S*,4*S*)-2-((difluor omethoxy)methyl)-4-(4-(trifluorom ethyl)phenoxy)pyrrolidin-1-yl)meth yl)benzamide | white solid; MS (ESI, pos.ion) m/z: 666 [M+H]$^+$; $^1$H NMR (400 MHz, $CDCl_3$) δ (ppm): 7.98 - 7.86 (m, 2H), 7.80 (d, *J* = 8.1 Hz, 2H), 7.68 (d, *J* = 7.9 Hz, 2H), 7.47 (dd, *J* = 30.0, 8.0 Hz, 4H), 6.87 (d, *J* = 8.5 Hz, 2H), 6.25 (t, *J* = 74.8 Hz, 1H), 5.61 (d, *J* = 6.8 Hz, 1H), 4.81 (s, 1H), 4.19 (d, *J* = 13.8 Hz, 1H), 3.96 (ddd, *J* = 35.6, 10.1, 5.7 Hz, 2H), 3.57 - 3.45 (m, 1H), 3.11 (dd, *J* = 15.2, 6.8 Hz, 7H), 3.07 - 2.91 (m, 1H), 2.64 (dd, *J* = 10.8, 5.3 Hz, 1H), 2.58 - 2.46 (m, 1H), 2.06 (d, *J* = 7.7 Hz, 1H), 1.97 (dd, *J* = 13.9, 6.2 Hz, 2H), 0.88 (d, *J* = 7.0 Hz, 4H). |
| **Example 70** | 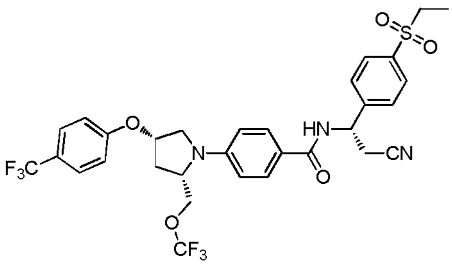 *N*-((*S*)-2-cyano-1-(4-(ethylsulfonyl) phenyl)ethyl)-4-((2*S*,4*S*)-2-((trifluo romethoxy)methyl)-4-(4-(trifluoro methyl)phenoxy)pyrrolidin-1-yl)be nzamide | white solid; MS (ESI, pos.ion) m/z = 670.1 [M+H]$^+$; $^1$H NMR (400 MHz, $CDCl_3$) δ (ppm): 7.93 (d, *J* = 8.3 Hz, 2H), 7.75 (d, *J* = 8.8 Hz, 2H), 7.65 (d, *J* = 8.3 Hz, 2H), 7.59 (d, *J* = 8.6 Hz, 2H), 6.97 (d, *J* = 8.5 Hz, 2H), 6.64 (d, *J* = 8.7 Hz, 3H), 5.60 (dd, *J* = 12.3, 6.4 Hz, 1H), 5.19 (t, *J* = 4.4 Hz, 1H), 4.30 - 4.21 (m, 2H), 4.11 (t, *J* = 9.3 Hz, 1H), 3.77 (d, *J* = 11.4 Hz, 1H), 3.71 (dd, *J*= 11.4, 4.6 Hz, 1H), 3.17 (dd, *J* = 15.8, 5.2 Hz, 1H), 3.12 (t, *J* = 7.4 Hz, 2H), 3.06 (dd, *J*= 17.0, 5.0 Hz, 1H), 2.53 (d, *J* = 14.5 Hz, 1H), 2.47 - 2.38 (m, 1H), 1.29 (t, *J* = 7.4 Hz, 4H). |

(continued)

| Example No. | Target compound structure and name | Target compound characterization data |
|---|---|---|
| **Example 71** | 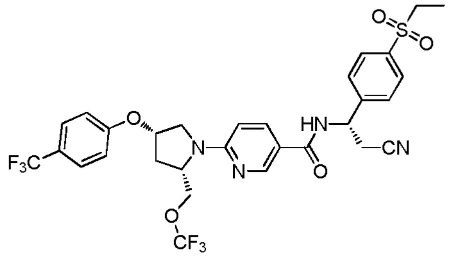<br>*N*-((*S*)-2-cyano-1-(4-(ethylsulfonyl) phenyl)ethyl)-6-((2*S*,4*S*)-2-((trifluo romethoxy)methyl)-4-(4-(trifluoro methyl)phenoxy)pyrrolidin-1-yl)nic otinamide | white solid; MS (ESI, pos.ion) m/z = 671.1 $[M+H]^+$; $^1$H NMR (400 MHz, $CDCl_3$) δ (ppm): 8.67 (d, *J* = 2.0 Hz, 1H), 7.95 (d, *J* = 7.6 Hz, 1H), 7.89 (d, *J* = 8.0 Hz, 2H), 7.64 (d, *J* = 8.3 Hz, 2H), 7.58 (d, *J* = 8.6 Hz, 2H), 6.96 (d, *J* = 8.5 Hz, 2H), 6.86 (d, *J* = 7.5 Hz, 1H), 6.43 (d, *J* = 8.9 Hz, 1H), 5.60 (dd, *J* = 12.9, 6.2 Hz, 1H), 5.18 (s, 1H), 4.61 (s, 1H), 4.48 - 4.42 (m, 1H), 4.08 (t, *J* = 9.2 Hz, 1H), 3.86 - 3.78 m, 2H), 3.20 - 3.04 (m, 4H), 2.54 (d, *J* = 14.5 Hz, 1H), 2.46 - 2.37 (m, 1H), 1.28 (t, *J* = 7.4 Hz, 3H). |
| **Example 72** | 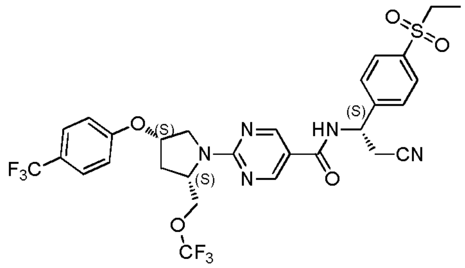<br>*N*-((*S*)-2-cyano-1-(4-(ethylsulfonyl) phenyl)ethyl)-2-((2*S*,4*S*)-2-((trifluo romethoxy)methyl)-4-(4-(trifluoro methyl)phenoxy)pyrrolidin-1-yl)py rimidine-5-carboxamide | white solid; MS (ESI, pos.ion) m/z: 672.1 $[M+H]^+$; $^1$H NMR (400 MHz, $CDCl_3$) δ (ppm): 8.82 (s, 2H), 7.79 (d, *J* = 8.2 Hz, 2H), 7.61 (d, *J* = 8.3 Hz, 2H), 7.57 (d, *J* = 8.7 Hz, 2H), 7.19 (d, *J* = 7.4 Hz, 1H), 6.95 (d, *J* = 8.5 Hz, 2H), 5.59 (dd, *J* = 13.3, 6.4 Hz, 1H), 5.14 (t, *J* = 4.3 Hz, 1H), 4.63 (s, 1H), 4.52 - 4.46 (m, 1H), 4.11 (t, *J* = 9.2 Hz, 1H), 4.03 (dd, *J* = 13.3, 4.6 Hz, 1H), 3.95 (d, *J* = 13.2 Hz, 1H), 3.19 - 3.06 (m, 4H), 2.57 (d, *J* = 14.5 Hz, 1H), 2.48 - 2.39 (m, 1H), 1.26 (t, *J* = 7.4 Hz, 3H). |
| **Example 73** | 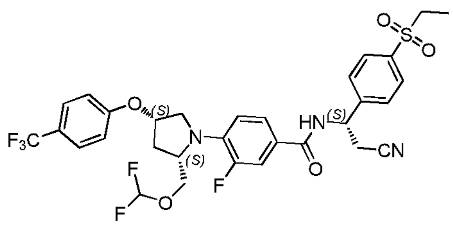<br>*N*-((*S*)-2-cyano-1-(4-(ethylsulfonyl) phenyl)ethyl)-4-((2*S*,4*S*)-2-((difluor omethoxy)methyl)-4-(4-(trifluorom ethoxy)phenoxy)pyrrolidin-1-yl)-3-fluorobenzamide | white solid; MS (ESI, pos.ion) m/z: 670.2 $[M+H]^+$; $^1$H NMR (400 MHz, $CDCl_3$) δ (ppm): 7.91 (d, *J* = 8.3 Hz, 2H), 7.64 (d, *J* = 8.2 Hz, 2H), 7.61 - 7.46 (m, 4H), 6.97 (d, *J* = 8.6 Hz, 2H), 6.70 (d, *J* = 8.2 Hz, 2H), 6.07 (d, *J* = 74.3 Hz, 1H), 5.58 (dd, *J* = 12.6, 6.3 Hz, 1H), 5.10 (s, 1H), 4.48 (s, 1H), 4.08 (dd, *J* = 9.9, 4.2 Hz, 1H), 3.94 (dd, *J* = 17.0, 7.9 Hz, 2H), 3.85 (dd, *J* = 11.0, 4.8 Hz, 1H), 3.21 - 3.02 (m, 4H), 2.43 (d, *J* = 4.2 Hz, 2H), 1.29 (t, *J* = 7.4 Hz, 3H). |

**Example 74 N-((*S*)-2-cyano-1-(4-(ethylsulfonyl)phenyl)ethyl)-4-((2*S*,4*R*)-2-((difluoromethoxy) methyl)-4-(4-(trifluoromethyl)phenoxy)pyrrolidin-1-yl)benzamide**

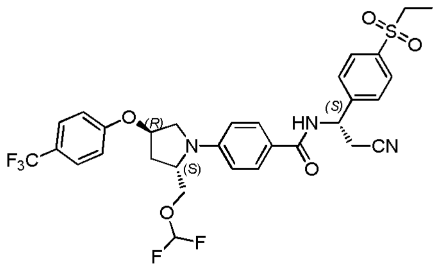

The starting material (2*S*,4*R*)-1-*tert*-butyl 2-methyl 4-hydroxypyrrolidine-1,2-dicarboxylate in step 1 of Example 47 was replaced with (2*S*,4*S*)-1-*tert*-butyl 2-methyl 4-hydroxypyrrolidine-1,2-dicarboxylate, and the title compound was prepared according to the methods of step 1 to step 7 of Example 47 as a white solid (70 mg, 58%). MS (ESI, pos.ion) m/z: 652.1 $[M+H]^+$.
$^1$H NMR(400 MHz, $CDCl_3$) δ (ppm): 7.91 (d, *J*= 7.5 Hz, 2H), 7.76 - 7.70 (d, *J*= 6.9 Hz, 2H), 7.64 (d, *J* = 6.9 Hz, 2H), 7.56 (d, *J* = 8.6 Hz, 2H), 6.95 (d, *J* = 8.5 Hz, 2H), 6.72 (t, *J* = 6.6 Hz, 1H), 6.62 (d, *J* = 8.7 Hz, 2H), 6.21 (t, *J* = 74.1 Hz, 1H), 5.59 (dd, *J* = 12.8, 6.2 Hz, 1H), 5.21 - 5.15 (m, 1H), 4.35 (d, *J* = 3.5 Hz, 1H), 4.02 - 3.96 (m, 2H), 3.92 (dd, *J* = 10.6, 5.8 Hz, 1H), 3.54 (dd, *J* = 10.6, 3.4 Hz, 1H), 3.18 - 3.02 (m, 4H), 2.58 - 2.44 (m, 2H), 1.28 (t, *J* = 7.4 Hz, 3H).

**Example 75 *N*-((*S*)-2-cyano-1-(4-(ethylsulfonyl)phenyl)ethyl)-4-((2*R*,4*S*) -2-(2-(difluoromethoxy)ethyl)-4-(4-(trifluoromethyl)phenoxy)pyrrolidin-1-yl)benzamide**

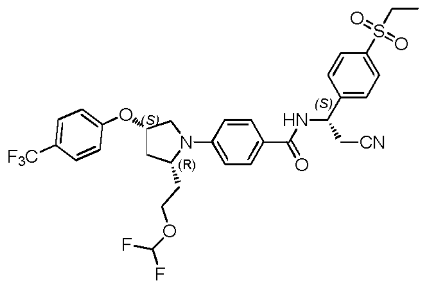

**Step** 1: **Synthesis of (2*S*,4*S*)-2-(hydroxymethyl)-4-(4-(trifluoromethyl)phenoxy)pyrrolidine**

To a solution of tert-butyl (2*S*,4*S*)-2-(hydroxymethyl)-4-(4-(trifluoromethyl) phenoxy)pyrrolidine-1-carboxylate (361 mg, 1.00mmol) in DCM (20 mL) was added a solution of HCl in methanol (0.5 mL, 4.0 M) at room temperature. The mixture was reacted for 5 h. Saturated $Na_2CO_3$ solution was added to adjust pH to weakly alkaline, and the resulting mixture was extracted with DCM (10 mL × 2). The organic phases were combined, dried over anhydrous $Na_2SO_4$, filtered and concentrated to give colorless liquid (253 mg, 97%).
MS (ESI, pos.ion) m/z: 262 $[M+H]^+$.

**Step 2: Synthesis of benzyl (2*S*,4*S*)-2-(hydroxymethyl)-4-(4-(trifluoromethyl) phenoxy)pyrrolidine-1-carboxylate**

To a solution of (2*S*,4*S*)-2-(hydroxymethyl)-4-(4-(trifluoromethyl)phenoxy)pyrrolidine (253 mg, 0.97 mmol) in THF (15 mL) were added 25 mL of saturated $NaHCO_3$ solution and benzyl chloroformate (171 mg, 1.00 mmol) in turn. The mixture was reacted at room temperature for 12 h. The reaction was quenched by adding water. The resulting mixture was extracted with EtOAc (20 mL × 2). The organic phases were combined, dried over anhydrous $Na_2SO_4$, concentrated and separated by silica gel column chromatography (eluent: PE/EtOAc (v/v) = 10/1) to give colorless liquid (378 mg, 97%).
MS (ESI, pos.ion) m/z: 396 $[M+H]^+$.

**Step 3: Synthesis of benzyl (2*S*,4*S*)-2-(((methylsulfonyl)oxy)methyl)-4-(4-(trifluoromethyl) phenoxy)pyrrolidine-1-carboxylate**

To a solution of TEA (0.90 mL, 6.46 mmol) and benzyl (2*S*,4*S*)-2-(hydroxymethyl)-4-(4-(trifluoromethyl)phenoxy)pyrrolidine-1-carboxylate (1.00 g, 2.53 mmol) in DCM (16 mL) was added MsCl (0.40 mL, 5.17 mmol) at 0°C. After

the addition was completed, the mixture was transferred and reacted at room temperature for 17 h. The reaction solution was added with saturated $NaHCO_3$ solution (15 mL) to quench the reaction. The resulting mixture was diluted with DCM (20 mL), extracted and separated. The organic phase was washed with saturated NaCl solution (15 mL), dried over anhydrous $Na_2SO_4$, and concentrated under reduced pressure to give pale yellow liquid (940 mg, 79%).
MS (ESI, pos.ion) m/z: 474.3 $[M+H]^+$.

**Step 4: Synthesis of benzyl (2*S*,4*S*)-2-(cyanomethyl)-4-(4-(trifluoromethyl) phenoxy)pyrrolidine-1-carboxylate**

Benzyl (2*S*,4*S*)-2-(((methylsulfonyl)oxy)methyl)-4-(4-(trifluoromethyl)phenoxy) pyrrolidine-1-carboxylate (900 mg, 1.90 mmol) and N(*n*-Bu)$_4$CN (765 mg, 2.85 mmol) were added to ACN (12 mL), and the mixture was reacted at 85°C for 8 h. The reaction solution was cooled to room temperature, concentrated under reduced pressure, and the concentrated solution was separated by silica gel column chromatography (eluent: PE/EtOAc (v/v) = 3/1) to give a white solid (552 mg, 72%).
MS (ESI, pos.ion) m/z: 405.2 $[M+H]^+$.

**Step 5: Synthesis of methyl 2-((2*S*,4*S*)-4-(4-(trifluoromethyl)phenoxy)pyrrolidin-2-yl)acetate**

To a solution of benzyl (2*S*,4*S*)-2-(cyanomethyl)-4-(4-(trifluoromethyl)phenoxy) pyrrolidine-1-carboxylate (550 mg, 1.36 mmol) in MeOH (4 mL) was added a solution of HCl in MeOH (8 mL, 20%). The mixture was stirred at 70°C for 20 h. The resulting mixture was concentrated under reduced pressure to give yellow liquid (300 mg, 73%).
MS (ESI, pos.ion) m/z: 304.3 $[M+H]^+$.

**Step 6: Synthesis of benzyl (2*S*,4*S*)-2-(2-methoxy-2-oxoethyl)-4-(4-(trifluoromethyl) phenoxy)pyrrolidine-1-carboxylate**

A solution of $NaHCO_3$ (443 mg, 5.27 mmol) in $H_2O$ (2 mL) was added to a solution of methyl 2-((2*S*,4*S*)-4-(4-(trifluoromethyl)phenoxy)pyrrolidin-2-yl)acetate (200 mg, 0.66 mmol) in THF (6 mL), then CbzCl (0.20 mL, 1.42 mmol) was added, and the mixture was reacted at room temperature for 17 h. The reaction solution was diluted with saturated $NaHCO_3$ solution (20 mL) and EtOAc (10 mL). The resulting mixture was left standing for layers. The upper organic phase was separated, and the aqueous phase was extracted with EtOAc (20 mL × 2). The combined organic phases were washed with saturated NaCl solution (15 mL), dried over anhydrous $Na_2SO_4$, concentrated under reduced pressure, and the concentrated solution was separated by silica gel column chromatography (eluent: PE/EtOAc (v/v) = 4/1) to give colorless liquid (250 mg, 87%).
MS (ESI, pos.ion) m/z: 338.3 $[M+H]^+$.

**Step 7: Synthesis of benzyl (2*R*,4*S*)-2-(2-(hydroxyethyl))-4-(4-(trifluoromethyl) phenoxy)pyrrolidine-1-carboxylate**

To THF (4 mL) was added benzyl (2*S*,4*S*)-2-(2-methoxy-2-oxoethyl) -4-(4-(trifluoromethyl)phenoxy)pyrrolidine-1-carboxylate (200 mg, 0.46 mmol), then the mixture was transferred to 0 °C, and $LiBH_4$ (40 mg, 1.84 mmol) was added. After the addition was completed, the mixture was reacted at room temperature for 12 h. Saturated $NH_4Cl$ (15 mL) solution was added to the reaction solution to quench the reaction. The mixture was left standing for layers. The organic phase was separated, and the aqueous phase was extracted with EtOAc (15 mL × 2). The combined organic phases were washed with saturated NaCl solution (15 mL), dried over anhydrous $Na_2SO_4$, concentrated under reduced pressure, and the concentrated solution was separated by silica gel column chromatography (eluent: PE/EtOAc (v/v) = 3/1) to give colorless liquid (85 mg, 45%).
MS (ESI, pos.ion) m/z: 410.3 $[M+H]^+$.

**Step 8: Synthesis of benzyl (2*R*,4*S*)-2-(2-(difluoromethoxy)ethyl) -4-(4-(trifluoromethyl)phenoxy)pyrrolidine-1-carboxylate**

To a solution of benzyl (2*R*,4*S*)-2-(2-(hydroxyethyl))-4-(4-(trifluoromethyl) phenoxy)pyrrolidine-1-carboxylate (80 mg, 0.20 mmol) in DCM (1 mL) were sequentially added $H_2O$ (1 mL), KOAc (230 mg, 2.34 mmol) and $TMSCF_2Br$ (0.30 mL, 1.93 mmol). The mixture was reacted at room temperature for 17 h. The reaction solution was diluted with DCM (30 mL), washed with saturated NaCl solution (15 mL), dried over anhydrous $Na_2SO_4$, concentrated under reduced pressure, and the concentrated solution was separated by silica gel column chromatography (eluent: PE/EtOAc (v/v) = 3/1) to give yellow liquid (65 mg, 72%).
MS (ESI, pos.ion) m/z: 460.3 $[M+H]^+$.

**Step 9: Synthesis of (2*R*,4*S*)-2-(2-(difluoromethoxy)ethyl)-4-(4-(trifluoromethyl)phenoxy)pyrrolidine**

To a solution of benzyl (2*R*,4*S*)-2-(2-(difluoromethoxy)ethyl)-4-(4-(trifluoromethyl)phenoxy)pyrrolidine-1-carboxylate (60 mg, 0.13 mmol) in MeOH (6 mL) was added Pd/C (20 mg, 10%) under hydrogen protection. The mixture was reacted at room temperature for 12 h. The reaction solution was filtered, and the filtrate was concentrated under reduced pressure to give colorless liquid (42 mg, 100%).
MS (ESI, pos.ion) m/z: 326.1 $[M+H]^+$.

**Step 10: Synthesis of methyl 4-((2*R*,4*S*)-2-(2-(difluoromethoxy)ethyl) -4-(4-(trifluoromethyl)phenoxy)pyrrolidin-1-yl)benzoate**

Under nitrogen protection, (2*R,*4*S*)-2-(2-(difluoromethoxy)ethyl)-4-(4-(trifluoromethyl) phenoxy)pyrrolidine (40 mg, 0.12 mmol), $Pd_2(dba)_3$ (11 mg, 0.01 mmol), 4,5-bisdiphenylphosphine-9,9-dimethylxanthene (10 mg, 0.02 mmol), $Cs_2CO_3$ (40 mg, 0.12 mmol), methyl 4-iodobenzoate (32 mg, 0.12 mmol) were successively added to 1,4-dioxane (3 mL), and the mixture was reacted at 100 °C for 12 h. The reaction solution was cooled to room temperature, and concentrated under reduced pressure. The concentrated solution was diluted with DCM (40 mL), washed successively with $NaHCO_3$ solution (15 mL) and saturated NaCl solution (15 mL), dried over anhydrous $Na_2SO_4$, concentrated under reduced pressure, and the concentrated solution was separated by silica gel column chromatography (eluent: PE/EtOAc (v/v) = 4/1) to give yellow liquid (35 mg, 62%).
MS (ESI, pos.ion) m/z: 460.3 $[M+H]^+$.

**Step 11: Synthesis of 4-((2*R*,4*S*)-2-(2-(difluoromethoxy)ethyl)-4-(4-(trifluoromethyl) phenoxy)pyrrolidin-1-yl)benzoic acid**

To a solution of methyl 4-((2*R*,4*S*)-2-(2-(difluoromethoxy)ethyl)-4-(4-(trifluoromethyl)phenoxy)pyrrolidin-1-yl)benzoate (40 mg, 0.09 mmol) in MeOH (4 mL) was added a solution of $LiOH \cdot H_2O$ (75 mg, 1.79 mmol) in $H_2O$ (1 mL). The mixture was reacted at room temperature for 24 h. The reaction solution was added with HCl solution (1 mol/L) to adjust the pH to about 4. The resulting mixture was extracted with EtOAc (20 mL × 2), and the organic phases were combined, washed with saturated NaCl (15 mL) solution, dried over anhydrous $Na_2SO_4$, concentrated under reduced pressure. The concentrated solution was separated by silica gel column chromatography (eluent: DCM/EtOAc (v/v) = 1/1) to give a white solid (20 mg, 52%).
MS (ESI, pos.ion) m/z: 446.0 $[M+H]^+$.

**Step 10: Synthesis of *N*-((*S*)-2-cyano-1-(4-(ethylsulfonyl)phenyl)ethyl)-4-((2*R*,4*S*) -2-(2-(difluoromethoxy)ethyl)-4-(4-(trifluoromethyl)phenoxy)pyrrolidin-1-yl)benzamide**

EDCI (29 mg, 0.15 mmol), HOBT (20 mg, 0.15 mmol), (*S*)-3-amino-3-(4-(ethylsulfonyl)phenyl)propionitrile hydrochloride (30 mg, 0.13 mmol), 4-((2*R*,4*S*)-2-(2-(difluoromethoxy)ethyl)-4-(4-(trifluoromethyl)phenoxy)pyrrolidin-1-yl)benzoic acid (45 mg, 0.10 mmol) and TEA (20 mg, 0.20 mmol) were successively added to DCM (4 mL), and the mixture was reacted at room temperature for 12 h. The reaction solution was diluted with DCM (30 mL), washed successively with $NaHCO_3$ solution (15 mL) and saturated NaCl solution (15 mL), dried over anhydrous $Na_2SO_4$, concentrated under reduced pressure, and the concentrated solution was separated by silica gel column chromatography (eluent: DCM/EtOAc (v/v) = 2/1) to give a white solid (20 mg, 30%).

MS (ESI, pos.ion) m/z: 657.1 $[M+H]^+$.
$^1H$ NMR (400 MHz, $CDCl_3$) δ (ppm): 7.95 (d, *J*= 8.2 Hz, 2H), 7.75 (d, *J*= 8.6 Hz, 2H), 7.68 (d, *J*= 8.2 Hz, 2H), 7.61 (d, *J* = 8.5 Hz, 2H), 6.98 (d, *J* = 8.4 Hz, 2H), 6.66 (d, *J* = 8.0 Hz, 1H), 6.63 (d, *J* = 8.6 Hz, 2H), 6.26 (t, *J*= 74.5 Hz, 1H), 5.62 (dd, *J*= 12.1, 6.1 Hz, 1H), 5.18 (s, 1H), 4.20 (t, *J*= 7.9 Hz, 1H), 4.04 - 3.99 (m, 1H), 3.97 - 3.88 (m, 1H), 3.75 (d, *J* = 2.3 Hz, 2H), 3.22 - 3.11 (m, 3H), 3.08 (dd, *J* = 17.0, 5.0 Hz, 1H), 2.48 - 2.40 (m, 1H), 2.36 (d, *J* = 14.0 Hz, 1H), 2.30 - 221 (m, 1H), 2.06 - 1.97 (m, 1H), 1.31 (t, *J* = 7.4 Hz, 3H).

**Example 76 *N*-((*S*)-2-cyano-1-(4-(ethylsulfonyl)phenyl)ethyl)-4-((2*R*,4*S*)-2-(2 -(trifluoromethoxy)ethyl) -4-(4-(trifluoromethyl)phenoxy)pyrrolidin-1-yl)benzamide**

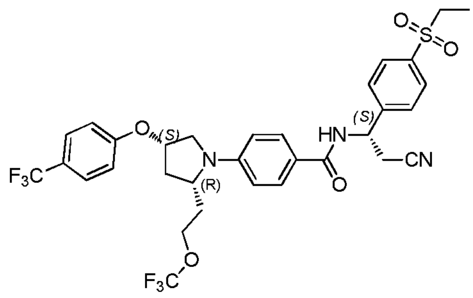

**Step 1: Synthesis of benzyl (2*R*,4*S*)-2-(2-(trifluoromethoxy)ethyl)-4-(4-(trifluoromethyl)phenoxy)pyrrolidine-1-carboxylate**

Under nitrogen protection, AgOTf (564 mg, 2.20 mmol), 1-chloromethyl-4-fluoro-1,4-diazobicyclo-2.2.2 octane bis(tetrafluoroborate) salt (389 mg, 1.10 mmol), KF (170 mg, 2.93 mmol) and (2*R*,4*S*)-benzyl 2-(2-(hydroxyethyl))-4-(4-(trifluoromethyl)phenoxy) pyrrolidine-1-carboxylate (300 mg, 0.73 mmol) were successively added to DCM (8 mL), then 2-fluoropyridine (0.21 mL, 2.40 mmol) and $TMSCF_3$ (0.31 mL, 2.10 mmol) were added, and the mixture was reacted at room temperature for 40 h. The reaction solution was filtered, and concentrated under reduced pressure. The concentrated solution was separated by silica gel column chromatography (eluent: PE/EtOAc (v/v) = 4/1) to give colorless liquid (80 mg, 23%).
MS (ESI, pos.ion) m/z: 478.1 $[M+H]^+$.

**Step 2: Synthesis of (2*R*,4*S*)-2-(2-(trifluoromethoxy)ethyl)-4-(4-(trifluoromethyl)phenoxy)pyrrolidine**

To a solution of benzyl (2*R*,4*S*)-2-(2-(trifluoromethoxy)ethyl)-4-(4-(trifluoromethyl) phenoxy)pyrrolidine-1-carboxylate (160 mg, 0.34 mmol) in MeOH (10 mL) was added Pd/C (30 mg, 10%) under hydrogen protection. The mixture was reacted at room temperature for 24 h. The reaction solution was filtered, and the filtrate was concentrated under reduced pressure to give colorless liquid (52 mg, 45%).
MS (ESI, pos.ion) m/z: 344.1 $[M+H]^+$.

**Step 3: Synthesis of methyl 4-((2*R*,4*S*)-2-(2-(trifluoromethoxy)ethyl) -4-(4-(trifluoromethyl)phenoxy)pyrrolidin-1-yl)benzoate**

Under nitrogen protection, (2*R*,4*S*)-2-(2-(trifluoromethoxy)ethyl)-4-(4-(trifluoromethyl) phenoxy)pyrrolidine (50 mg, 0.15 mmol), $Pd_2(dba)_3$ (26 mg, 0.03 mmol), 4,5-bisdiphenylphosphine-9,9-dimethylxanthene (25 mg, 0.04 mmol), $Cs_2CO_3$ (94 mg, 0.29 mmol), methyl 4-iodobenzoate (50 mg, 0.19 mmol) were successively added to 1,4-dioxane (4 mL), and the mixture was reacted at 100 °C for 17 h. The reaction solution was cooled to room temperature, and concentrated under reduced pressure. The concentrated solution was diluted with DCM (40 mL), washed successively with $NaHCO_3$ solution (15 mL) and saturated NaCl solution (15 mL), dried over anhydrous $Na_2SO_4$, concentrated under reduced pressure, and the concentrated solution was separated by silica gel column chromatography (eluent: PE/EtOAc (v/v) = 4/1) to give yellow liquid (56 mg, 80%).
MS (ESI, pos.ion) m/z: 478.0 $[M+H]^+$.

Step 4: Synthesis of **4-((2*R*,4*S*)-2-(2-(trifluoromethoxy)ethyl)-4-(4-(trifluoromethyl) phenoxy)pyrrolidin-1-yl)benzoic acid**

To a solution of methyl 4-((2*R*,4*S*)-2-(2-(trifluoromethoxy)ethyl)-4-(4-(trifluoromethyl)phenoxy)pyrrolidin-1-yl)benzoate (56 mg, 0.12 mmol) in MeOH (2 mL) and THF (2 mL) was added a solution of $LiOH \cdot H_2O$ (100 mg, 2.38 mmol) in $H_2O$ (1 mL). The mixture was stirred at room temperature for 24 h. The reaction solution was added with HCl solution (1 mol/L) to adjust the pH to about 4. The resulting mixture was extracted with EtOAc (20 mL × 2), and the organic phases were combined, washed with saturated NaCl (15 mL) solution, dried over anhydrous $Na_2SO_4$, concentrated under reduced pressure. The concentrated solution was separated by silica gel column chromatography (eluent: DCM/EtOAc (v/v) = 3/1) to give a white solid (31 mg, 52%).
MS (ESI, pos.ion) m/z: 461.1 $[M+H]^+$.

**Step 5: Synthesis of *N*-((*S*)-2-cyano-1-(4-(ethylsulfonyl)phenyl)ethyl)-4-((2*R*,4*S*) -2-(2-(trifluoromethoxy)ethyl)-4-(4-(trifluoromethyl)phenoxy)pyrrolidin-1-yl)benzamide**

EDCI (27 mg, 0.14 mmol), HOBT (19 mg, 0.14 mmol), (*S*)-3-amino-3-(4-(ethylsulfonyl)phenyl)propionitrile hydrochloride (23 mg, 0.08 mmol), 4-((2*R*,4*S*)-2-(2-(trifluoromethoxy)ethyl)-4-(4-(trifluoromethyl)phenoxy)pyrrolidin-1-yl)benzoic acid (31 mg, 0.07 mmol) and TEA (21 mg, 0.21 mmol) were successively added to DCM (4 mL), and the mixture was reacted at room temperature for 12 h. The reaction solution was diluted with DCM (30 mL), washed successively with $NaHCO_3$ solution (15 mL) and saturated NaCl solution (15 mL), dried over anhydrous $Na_2SO_4$, concentrated under reduced pressure, and the concentrated solution was separated by silica gel column chromatography (eluent: DCM/EtOAc (v/v) = 3/1) to give a yellow solid (25 mg, 55%).

MS (ESI, pos.ion) m/z: 684.3 $[M+H]^+$.
$^1H$ NMR (400 MHz, $CDCl_3$) $\delta$ (ppm): 7.93 (d, *J* = 8.1 Hz, 2H), 7.73 (d, *J* = 8.7 Hz, 2H), 7.65 (d, *J*= 8.0 Hz, 2H), 7.59 (d, *J* = 8.4 Hz, 2H), 6.96 (d, *J* = 8.5 Hz, 2H), 6.60 (d, *J* = 8.7 Hz, 3H), 5.62 - 5.57 (m, 1H), 5.18 (s, 1H), 4.22 - 4.10 (m, 2H), 4.06 - 3.99 (m, 1H), 3.74 (d, *J* = 2.4 Hz, 2H), 3.20 - 3.14 (m, 1H), 3.11 (q, *J* = 7.40 Hz, 2H), 3.06 (dd, *J* = 16.9, 4.9 Hz, 1H), 2.48 - 2.40 (m, 1H), 2.35 - 2.24 (m, 2H), 2.11 - 2.02 (m, 1H), 1.29 (t, *J*= 7.40 Hz, 3H).

**Example 77 *N*-((*R*)-2-cyano-1-(4-(ethylsulfonyl)phenyl)ethyl)-4-((2*S*,4*S*)-2-(ethoxymethyl)-4-(4 -(trifluoromethyl)phenoxy)-1-pyrrolidine)benzamide**

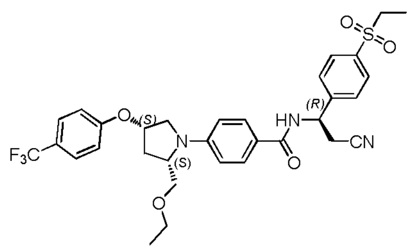

**Step 1: Synthesis of *tert*-butyl (2*S*,4*S*)-2-(ethoxymethyl)-4-(4-(trifluoromethyl) phenoxy)pyrrolidine-1-carboxylate**

To a solution of tert-butyl (2*S*,4*S*)-2-(hydroxymethyl)-4-(4-(trifluoromethyl)phenoxy) pyrrolidine-1-carboxylate (304 mg, 0.84 mmol) in DCM (5.0 mL) was added NaH (82 mg, 2.05 mmol, 60%) at -10°C. After 0.5 h of reaction, iodoethane (0.3 mL, 4.00 mmol) was added to the above solution, and the reaction was continued for 5 h. The reaction was quenched by adding water. The resulting mixture was extracted with EtOAc (5 mL × 3). The organic phase was washed with saturated NaCl solution, dried over anhydrous $Na_2SO_4$, filtered, concentrated and separated by silica gel column chromatography (eluent: PE/EtOAc (v/v) = 3/1) to give a white solid (235 mg, 72%).
MS (ESI, pos.ion) m/z: 390.4 $[M+H]^+$.

**Step 2: Synthesis of (2*S*,4S)-2-(ethoxymethyl)-4-(4-(trifluoromethyl)phenoxy)pyrrolidine**

To a solution of tert-butyl (2*S*,4*S*)-2-(ethoxymethyl)-4-(4-(trifluoromethyl)phenoxy) pyrrolidine-1-carboxylate (83 mg, 0.21 mmol) in DCM (3 mL) was added HCl (0.5 mL, 3.00 mmol, 20% aqueous solution) at room temperature. The mixture was reacted for 6 h. Saturated $Na_2CO_3$ solution was added to quench the reaction, and the resulting mixture was extracted with DCM (5 mL × 3). The organic phases were combined, dried over anhydrous $Na_2SO_4$, filtered and concentrated to give a white solid (62 mg, 100%).
MS (ESI, pos.ion) m/z: 290.5 $[M+H]^+$.

**Step 3: Synthesis of ethyl 4-((2*S*,4*S*)-2-(ethoxymethyl)-4-(4-(trifluoromethyl) phenoxy)pyrrolidin-1-yl)benzoate**

Under nitrogen protection, (2*S*,4*S*)-2-(ethoxymethyl)-4-(4-(trifluoromethyl)phenoxy)pyrrolidine (62 mg, 0.21 mmol), methyl 4-iodobenzoate (121 mg, 0.46 mmol), $PdOAc_2$ (10 mg, 0.045 mmol), $Cs_2CO_3$ (92 mg, 0.28 mmol), 4,5-bisdiphenylphosphine-9,9-dimethylxanthene (15 mg, 0.026 mmol) were successively added to 1,4-dioxane (5.0 mL) and the mixture was reacted at 100 °C for 12 h. The mixture was cooled to room temperature, and the reaction was quenched by adding water. The resulting mixture was extracted with EtOAc (5 mL × 3). The organic phases were combined, dried

over anhydrous $Na_2SO_4$, filtered, concentrated and separated by silica gel column chromatography (eluent: PE/EtOAc (v/v) = 5/1) to give a white solid (46 mg, 52%).
MS (ESI, pos.ion) m/z: 424.3 [M+H]+.

**Step 4: Synthesis of 4-((2*S*,4*S*)-2-(ethoxymethyl)-4-(4-(trifluoromethyl) phenoxy)pyrrolidin-1-yl)benzoic acid**

To a solution of ethyl 4-((2*S*,4*S*)-2-(ethoxymethyl)-4-(4-(trifluoromethyl)phenoxy) pyrrolidin-1-yl)benzoate (153 mg, 0.36 mmol) in MeOH (5 mL) were added $H_2O$ (3 mL) and LiOH (630 mg, 2.60 mmol). The mixture was reacted at room temperature for 12 h, and diluted hydrochloric acid solution was added to adjust the pH of the solution to acidity. The resulting mixture was extracted with EtOAc (5 mL × 3), and the organic phases were combined, dried over anhydrous $Na_2SO_4$, filtered, concentrated and separated by silica gel column chromatography (eluent: PE/EtOAc (v/v) = 2/1) to give a white solid (99 mg, 67%).
MS (ESI, pos.ion) m/z: 411 [M+H]+.

**Step 5: *N*-((*R*)-2-cyano-1-(4-(ethylsulfonyl)phenyl)ethyl)-4-((2*S*,4*S*)-2-(ethoxymethyl)-4-(4-(trifluoromethyl)phenoxy)-1-pyrrolidine)benzamide**

4-((2S,4S)-2-(Ethoxymethyl)-4-(4-(trifluoromethyl)phenoxy)pyrrolidin-1-yl)benzoic acid (115 mg, 0.28 mmol), (*R*)-3-amino-3-(4-(ethylsulfonyl)phenyl)propionitrile hydrochloride (85 mg, 0.31 mmol), HOBT (52 mg, 0.38 mmol) and EDCI (102 mg, 0.53 mmol) were dissolved in DCM (25 mL), then DIPEA (100 mg, 0.771 mmol) was added. After the mixture was reacted at room temperature for 12 h, water was added to quench the reaction. The resulting mixture was extracted with EtOAc (5 mL × 3), and the organic phases were combined, dried over anhydrous $Na_2SO_4$, filtered, concentrated and separated by silica gel column chromatography (eluent: PE/EtOAc (v/v) = 2/1) to give a white solid (65 mg, 37%).

MS (ESI, pos.ion) m/z: 630.1 [M+H]+.
$^1$H NMR (400 MHz, CDCl3) δ (ppm): 7.94 (d, *J* = 8.3 Hz, 2H), 7.72 (d, *J* = 8.7 Hz, 2H), 7.66 (d, *J* = 8.3 Hz, 2H), 7.58 (d, *J* = 8.6 Hz, 2H), 6.97 (d, *J* = 8.5 Hz, 2H), 6.67 (d, *J* = 8.8 Hz, 2H), 6.62 (d, *J* = 7.6 Hz, 1H), 5.60 (dd, *J* = 12.4, 6.2 Hz, 1H), 5.14 (t, *J* = 4.4 Hz, 1H), 3.73 (td, *J* = 8.9, 4.2 Hz, 3H), 3.56 - 3.40 (m, 3H), 3.22 - 3.00 (m, 4H), 2.51 (d, *J*= 14.2 Hz, 1H), 2.38 (td, *J* = 8.4, 4.2 Hz, 1H), 1.29 (t, *J* = 7.4 Hz, 3H), 1.16 (t, *J* = 7.0 Hz, 3H).

**Example 78 *N*-((*S*)-2-cyano-1-(4-(ethylsulfonyl)phenyl)ethyl)-4-((2S,4S)-4-((2,2 -difluorobenzo[d][1,3]dioxol-5-yl)oxy)-2-((difluoromethoxy)methyl)pyrrolidin-1-yl) -benzamide**

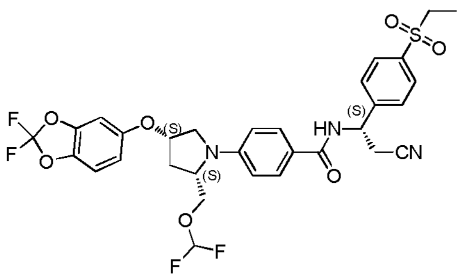

**Step 1: Synthesis of (2*S*,4*S*)-2-methyl 1-*tert*-butyl 4-((2,2-difluorobenzo[*d*][1,3]dioxol-5-yl)oxy) pyrrolidine-1,2-dicarboxylate**

(2*S*,4*R*)-1-*tert*-Butyl 2-methyl 4-hydroxypyrrolidine-1,2-dicarboxylate (2.00 g, 7.75 mmol), 2,2-difluorobenzo[d][1,3]dioxol-5-ol (1.50 g, 7.75 mmol), $PPh_3$ (2.35 g, 8.96 mmol) were added to THF (20 mL). The mixture was transferred to 0 °C, and DIAD (2.00 mL, 10.16 mmol) was slowly added. After the addition was complete, the mixture was transferred to room temperature and reacted for 24 h. The reaction solution was concentrated under reduced pressure. The concentrated solution was diluted with methyl *tert*-butyl ether (50 mL) and stirred at -20 °C. A large amount of white insoluble solid was precipitated, filtered while cold, and the filter cake was washed with cold methyl *tert*-butyl ether. The filtrate was concentrated under reduced pressure, and the crude product was separated by silica gel column chromatography (eluent: PE/EtOAc (v/v) = 4/1) to give yellow liquid (3.00 g, 96%).
MS (ESI, pos.ion) m/z: 346.2 [M-56+H]+.

**Step 2: Synthesis of *tert-butyl* (2*S*,4*S*)-2-(hydroxymethyl)-4-((2,2-difluorobenzo[*d*][1,3]dioxol-5-yl) oxy)pyrrolidine-1-dicarboxylate**

To THF (30 mL) solution was added (2S,4S)-2-methyl 1-tert-butyl 4-((2,2-difluorobenzo[*d*][1,3]dioxol-5-yl)oxy)pyrrolidine-1,2-dicarboxylate (3.30 g, 5.47 mmol), then the mixture was transferred to 0 °C, and $LiBH_4$ (325 mg, 14.92 mmol) was added slowly. After the addition was completed, the mixture was reacted at room temperature for 15 h. Saturated $NH_4Cl$ (20 mL) solution was added to the reaction solution to quench the reaction. The mixture was left standing for layers. The organic phase was separated, the aqueous phase was extracted with EtOAc (20 mL × 2). The combined organic phases were dried over anhydrous $Na_2SO_4$, concentrated under reduced pressure, and the concentrated solution was separated by silica gel column chromatography (eluent: PE/EtOAc (v/v) = 3/1) to give yellow liquid (2.60 g, 93%).
MS (ESI, pos.ion) m/z: 318.2 $[M-56+H]^+$.

**Step 3: Synthesis of *tert-butyl* (2*S*,4*S*)-2-((difluoromethoxy)methyl)-4-((2,2-difluorobenzo[*d*][1,3]dioxol -5-yl)oxy)pyrrolidine-1-dicarboxylate**

To a solution of *tert-butyl* (2S,4S)-2-hydroxymethyl-4-((2,2-difluorobenzo[d][1,3]dioxol-5-yl)oxy)pyrrolidine-1-carboxylate (2.50 g, 6.70 mmol) in DCM (12 mL) were sequentially added $H_2O$ (12 mL), KOAc (8.0 g, 80.30 mmol) and $TMSCF_2Br$ (8.50 mL, 55.00 mmol). The mixture was reacted at room temperature for 19 h. The reaction solution was diluted with DCM (50 mL), washed with saturated NaCl solution (20 mL), dried over anhydrous $Na_2SO_4$, concentrated under reduced pressure, and the concentrated solution was separated by silica gel column chromatography (eluent: PE/EtOAc (v/v) = 4/1) to give colorless liquid (2.25 g, 79%). MS (ESI, pos.ion) m/z: 446.1 $[M+Na]^+$.

**Step 4: Synthesis of (2*S*,4*S*)-2-(difluoromethoxy)methyl)-4-((2,2-difluorobenzo[d][1,3]dioxol -5-yl)oxy)pyrrolidine**

To a solution of *tert-butyl* (2*S*,4*S*)-2-((difluoromethoxy)methyl)-4-((2,2-difluorobenzo[*d*][1,3]dioxol-5-yl)oxy)pyrrolidine-1-carboxylate (2.20 g, 5.20 mmol) in DCM (10 mL) was added a solution of HCl in 1,4-dioxane (3.0 mL, 4 mol/L). The mixture was reacted at room temperature for 13 h. The reaction solution was concentrated under reduced pressure, and the concentrated solution was diluted with DCM (40 mL), washed successively with saturated $NaHCO_3$ solution (20 mL) and saturated NaCl solution (20 mL), dried over anhydrous $Na_2SO_4$, and concentrated under reduced pressure to give yellow liquid (1.52 g, 90%).
MS (ESI, pos.ion) m/z: 324.1 $[M+H]^+$.

**Step 5: Synthesis of methyl 4-((2S,4S)-2-(difluoromethoxy)methyl) -4-((2,2-difluorobenzo[*d*][1,3]dioxol-5-yl)oxy)pyrrolidin-1-yl)benzoate**

Under nitrogen protection, (2S,4S)-2-(difluoromethoxy)methyl)-4-((2,2-difluorobenzo[d][1,3]dioxol-5-yl)oxy)pyrrolidine (1.50 g, 4.64 mmol), $Pd_2(dba)_3$ (425 mg, 0.46 mmol), 4,5-bisdiphenylphosphine-9,9-dimethylxanthene (403 mg, 0.70 mmol), $Cs_2CO_3$ (2.27 g, 6.97 mmol), methyl 4-iodobenzoate (1.58 g, 6.03 mmol) were successively added to 1,4-dioxane (20 mL) and the mixture was reacted at 100 °C for 23 h. The reaction solution was cooled to room temperature, and concentrated under reduced pressure. The concentrated solution was diluted with DCM (60 mL), washed successively with $NaHCO_3$ solution (20 mL) and saturated NaCl solution (20 mL), dried over anhydrous $Na_2SO_4$, concentrated under reduced pressure, and the concentrated solution was separated by silica gel column chromatography (eluent: PE/EtOAc (v/v) = 4/1) to give yellow liquid (1.62 g, 76%).
MS (ESI, pos.ion) m/z: 458.1 $[M+H]^+$.

**Step 6: Synthesis of 4-((2S,4S)-2-(difluoromethoxy)methyl)-4-((2,2-difluorobenzo[*d*][1,3] dioxol-5-yl)oxy)pyrrolidin-1-yl)benzoic acid**

To a solution of methyl 4-((2S,4S)-2-((difluoromethoxy)methyl)-4-((2,2 -difluorobenzo[d][1,3]dioxol-5-yl)oxy)pyrrolidin-1-yl)benzoate (1.60 g, 3.50 mmol) in MeOH (8 mL) and THF (8 mL) was added $LiOH{\cdot}H_2O$ (3.00 g, 71.50 mmol) in $H_2O$ (8 mL). The mixture was reacted at room temperature for 15 h. The reaction solution was concentrated under reduced pressure, and HCl solution (3 mol/L) was added to the concentrated solution to adjust the pH to about 4. The resulting mixture was extracted with EtOAc (20 mL × 2), and the organic phases were combined, washed with saturated NaCl (15 mL) solution, dried over anhydrous $Na_2SO_4$, concentrated under reduced pressure. The concentrated solution was separated by silica gel column chromatography (eluent: DCM/EtOAc (v/v) = 5/1) to give a pale yellow solid (1.42 g, 91%).

MS (ESI, pos.ion) m/z: 444.1 $[M+H]^+$.

**Step 7: Synthesis of *N*-((*S*)-2-cyano-1-(4-(ethylsulfonyl)phenyl)ethyl)-4-((2*S*,4*S*) -2-((difluoromethoxy)methyl)-4-((2,2-difluorobenzo[*d*][1,3]dioxol-5-yl)oxy)pyrrolidin-1-yl)ben zamide**

EDCI (64 mg, 0.33 mmol), HOBT (45 mg, 0.33 mmol), (*S*)-3-amino-3-(4-(ethylsulfonyl)phenyl)propionitrile hydrochloride (70 mg, 0.25 mmol), 4-((2*S*,4*S*)-2-((difluoromethoxy)methyl)-4-((2,2-difluorobenzo[d][1,3]dioxol-5-yl)oxy)pyrrolidin-1-yl)benzoic acid (100 mg, 0.22 mmol) and TEA (70 mg, 0.69 mmol) were successively added to DCM (6 mL) and the mixture was reacted at room temperature for 17 h. The reaction solution was diluted with DCM (40 mL), washed successively with $NaHCO_3$ solution (15 mL) and saturated NaCl solution (15 mL), dried over anhydrous $Na_2SO_4$, concentrated under reduced pressure, and the concentrated solution was separated by silica gel column chromatography (eluent: DCM/EtOAc (v/v) = 3/1) to give a pale yellow solid (80 mg, 53%).

MS (ESI, pos.ion) m/z: 664.1 $[M+H]^+$.
$^1H$ NMR (400 MHz, CDCl3) $\delta$ (ppm): 7.91 (s, 2H), 7.74 (d, *J* = 8.7 Hz, 2H), 7.64 (d, *J* = 7.8 Hz, 2H), 6.98 (d, *J* = 8.7 Hz, 1H), 6.69 (d, *J* = 2.3 Hz, 1H), 6.64 (d, *J* = 8.2 Hz, 2H), 6.57 (dd, *J* = 8.7, 2.4 Hz, 1H), 6.23 (t, *J* = 74.3 Hz, 1H), 5.59 (dd, *J* = 12.3, 6.2 Hz, 1H), 5.03 (t, *J* = 4.6 Hz, 1H), 4.22 - 4.13 (m, 2H), 3.95 (t, *J* = 9.0 Hz, 1H), 3.74 (d, J = 11.3 Hz, 1H), 3.65 (dd, J = 11.3, 4.8 Hz, 1H), 3.16 - 3.02 (m, 4H), 2.49 (d, *J* = 14.3 Hz, 1H), 2.39 - 2.29 (m, 1H), 1.28 (t, *J* = 7.4 Hz, 3H).

**Example 79 *N*-((*S*)-2-cyano-1-(4-(ethylsulfonyl)phenyl)ethyl)-)-4-(1-(4-(trifluoromethyl) benzyl)pyrrolidin-2-yl)benzamide**

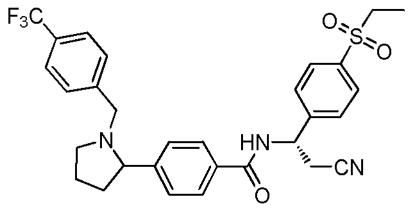

**Step 1: Synthesis of methyl 4-(4-(*tert*-butoxycarbonylamino)butyryl)benzoate**

4-Iodobenzoate (3.00 g, 11.00 mmol) was dissolved in THF (20 mL). After the mixture was cooled to -50°C, *i*-PrMgBr (11 mL, 11.00 mmol, 1 mol/L) was added, and the resulting mixture was reacted for 0.5 h, warmed to room temperature and continued to react for 0.5 h. The mixture was then cooled to -50°C, and *tert*-butyl 2-oxopyrrolidine-1-carboxylate (2.50 g, 13.00 mmol) was added. The resulting mixture was reacted for 0.5 h, warmed to room temperature and continued to react for 5 h. The reaction was quenched by adding water, and the mixture was extracted with EtOAc (30 mL × 2). The organic phases were combined, dried over anhydrous $Na_2SO_4$, filtered, concentrated and separated by silica gel column chromatography (eluent: PE/EtOAc (v/v) = 10/1) to give colorless oily liquid (2.00 g, 54%).
MS (ESI, pos.ion) m/z: 322 $[M+H]^+$.

**Step 2: Synthesis of methyl 4-(3,4-dihydro-2*H*-pyrrol-5-yl)benzoate**

To a solution of methyl 4-(4-((*tert*-butoxycarbonyl)amino)butyryl)benzoate (2.00 g, 6.20 mmol) in DCM (10 mL) was added trifluoroacetic acid (1.6 mL, 24.00 mmol), and the mixture was reacted at room temperature for 2 h. The reaction was quenched by adding water. The resulting mixture was extracted with EtOAc (30 mL × 2). The organic phases were combined, dried over anhydrous $Na_2SO_4$, filtered, concentrated and separated by silica gel column chromatography (eluent: PE/EtOAc (v/v) = 10/1) to give a white solid (1.10 g, 87%).
MS (ESI, pos.ion) m/z: 204 $[M+H]^+$.

**Step 3: Synthesis of methyl 4-(pyrrolidin-2-yl)benzoate**

Methyl 4-(3,4-dihydro-2H-pyrrol-5-yl)benzoate (2.23 g, 11.00 mmol) was dissolved in THF (12 mL) and MeOH (5 mL), and $NaBH_4$ (0.11 g, 2.90 mmol) was added slowly at room temperature. The mixture was reacted for 12 h. The reaction was quenched by adding water. The resulting mixture was extracted with EtOAc (30 mL × 2). The organic

phases were combined, dried over anhydrous $Na_2SO_4$, filtered and concentrated to give colorless liquid (1.12 g, 49%). MS (ESI, pos.ion) m/z: 206 $[M+H]^+$.

**Step 4: Synthesis of *tert*-butyl 2-(4-(methoxycarbonyl)phenyl)pyrrolidine-1-carboxylate**

To a solution of methyl 4-(pyrrolidin-2-yl)benzoate (2.25 g, 11.00 mmol) in DCM (10 mL) were added TEA (3.6 mL, 28.00 mmol) and $Boc_2O$ (2.87 g, 13.10 mmol), and the mixture was reacted at room temperature for 5 h. The reaction was quenched by adding water. The resulting mixture was extracted with EtOAc (30 mL × 2). The organic phases were combined, dried over anhydrous $Na_2SO_4$, filtered, concentrated and separated by silica gel column chromatography (eluent: PE/EtOAc (v/v) = 10/1) to give a white solid (1.05 g, 31%).
MS (ESI, pos.ion) m/z: 306 $[M+H]^+$.

**Step 5: Synthesis of 4-(1-((*tert*-butoxy)carbonyl)pyrrolidin-2-yl)benzoic acid**

tert-Butyl 2-(4-(methoxycarbonyl)phenyl)pyrrolidine-1-carboxylate (1.05 g, 3.44 mmol) was dissolved in MeOH (10 mL) and $H_2O$ (5 mL). The mixture was reacted at room temperature for 12 h, and dilute hydrochloric acid was added to adjust the pH to be acidic. The resulting mixture was extracted with EtOAc (30 mL × 2), and the organic phases were combined, dried over anhydrous $Na_2SO_4$, filtered, and concentrated to give a white solid (0.88 g, 88%).
MS (ESI, pos.ion) m/z: 292 $[M+H]^+$.

**Step 6: Synthesis of *tert*-butyl 2-(4-(((*S*)-2-cyano-1-(4-(ethylsulfonyl)phenyl)ethyl) carbamoyl)phenyl)pyrrolidine-1-carboxylate**

4-(1-((tert-butoxy)carbonyl)pyrrolidin-2-yl)benzoic acid (210 mg, 0.72 mmol), (*S*)-3-amino-3-(4-(ethylsulfonyl)phenyl)propionitrile hydrochloride (206 mg, 0.86 mmol) and HATU (332 mg, 0.86 mmol) were dissolved in DCM (12 mL), then TEA (0.22 mL, 1.70 mmol) was added. After the mixture was reacted at room temperature for 12 h, water was added to quench the reaction. The resulting mixture was extracted with EtOAc (10 mL × 2), and the organic phases were combined, dried over anhydrous $Na_2SO_4$, filtered, concentrated and separated by silica gel column chromatography (eluent: PE/EtOAc (v/v) = 1/1) to give a white solid (220 mg, 60%).
MS (ESI, pos.ion) m/z: 512 $[M+H]^+$.

**Step 7: Synthesis of *N*-((*S*)-2-cyano-1(4-(ethylsulfonyl)phenyl)ethyl)-4-(pyrrolidin-2-yl)benzamide**

To a solution of *tert*-butyl 2-(4-(((S)-2-cyano-1-(4-(ethylsulfonyl)phenyl)ethyl) carbamoyl)phenyl)pyrrolidine-1-carboxylate (150 mg, 0.29 mmol) in DCM (10 mL) was added TFA (0.10 mL, 1.30 mmol) slowly. The mixture was reacted at room temperature for 2 h, and saturated $Na_2CO_3$ solution was added to adjust the pH to neutral. The resulting mixture was extracted with DCM (10 mL × 2), and the organic phases were combined, dried over anhydrous $Na_2SO_4$, filtered, and concentrated under reduced pressure to give colorless liquid (120 mg, 99%).
MS (ESI, pos.ion) m/z: 412 $[M+H]^+$.

**Step 8: Synthesis of *N*-((S)-2-cyano-1-(4-(ethylsulfonyl)phenyl)ethyl) -4-4-(1-(4-(trifluoromethyl)benzyl)pyrrolidin-2-yl)benzamide**

To a solution of *N*-((*S*)-2-cyano-1-(4-(ethylsulfonyl)phenyl)ethyl) -4-(pyrrolidin-2-yl)benzamide (120 mg, 0.29 mmol) in MeOH (10 mL) were added 4-(trifluoromethyl)benzaldehyde (61 mg, 0.35 mmol) and $NaBH_3CN$ (7 mg, 0.11 mmol), and the mixture was reacted at room temperature for 12 h. The reaction was quenched by adding water. The resulting mixture was extracted with DCM (10 mL × 2). The organic phases were combined, dried over anhydrous $Na_2SO_4$, filtered, concentrated and separated by silica gel column chromatography (eluent: DCM/EtOAc (v/v) = 3/1) to give a white solid (84 mg, 51%).

MS (ESI, pos.ion) m/z: 570.1 $[M+H]^+$.
$^1H$ NMR (400 MHz, CDCl3) δ (ppm): 7.95 (d, *J* = 8.1 Hz, 2H), 7.80 (d, *J* = 8.0 Hz, 2H), 7.67 (d, *J* = 8.1 Hz, 2H), 7.54 (t, *J* = 8.5 Hz, 4H), 7.38 (d, *J* = 7.9 Hz, 2H), 6.96 (d, *J* = 7.6 Hz, 1H), 5.62 (dd, *J* = 12.6, 6.2 Hz, 1H), 3.79 (d, *J* = 13.5 Hz, 1H), 3.48 (t, *J* = 8.2 Hz, 1H), 3.25 - 3.03 (m, 7H), 2.31 - 2.17 (m, 4H), 1.30 (d, *J* = 7.4 Hz, 3H).

**Example 80 *N*-((*S*)-2-cyano-1-(4-(ethylsulfonyl)phenyl)ethyl)-4-((2S,4S)-2-(hydroxymethyl)-4-(4-(trifluoromethyl)phenoxy)pyrrolidin-1-yl)benzamide**

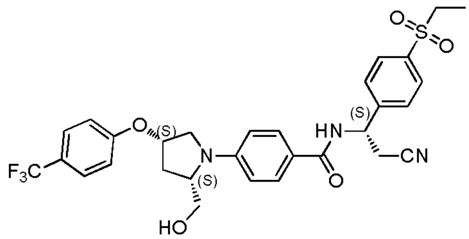

**Step 1: Synthesis of (2*S*,4*S*)-2-(hydroxymethyl)-4-(4-(trifluoromethyl)phenoxy)pyrrolidine**

To a solution of *tert*-butyl (4*S*,4*S*)-2-(hydroxymethyl)-4-(4-(trifluoromethyl)phenoxy) pyrrolidine-1-carboxylate (1.00 g, 2.77 mmol) in DCM (3 mL) was added a solution of HCl in 1,4-dioxane (4 mL, 4 mol/L). The mixture was reacted at room temperature for 12 h. The reaction solution was concentrated under reduced pressure to obtain a brown solid (600 mg, 83%).
MS (ESI, pos.ion) m/z: 262.2 $[M+H]^+$.

**Step 2: Synthesis of ethyl 4-((2S,4S)-2-(hydroxymethyl)-4-(4-trifluoromethyl) phenoxy)pyrrolidin-1-yl)benzoate**

Under nitrogen protection, (2S,4S)-2-hydroxymethyl)-4-(4-(trifluoromethyl) phenoxy)pyrrolidine (600 mg, 2.30 mmol), $Pd_2(dba)_3$ (210 mg, 0.23 mmol), 2-bicyclohexylphosphine-2',6'-diisopropoxybiphenyl (160 mg, 0.34 mmol), $Cs_2CO_3$ (748 mg, 2.30 mmol) and methyl 4-iodobenzoate (0.45 mL, 2.60 mmol) were successively added to 1,4-dioxane (10 mL), and the mixture was reacted at 100 °C for 24 h. The reaction solution was cooled to room temperature, and filtered through a celite pad. The filtrate was concentrated under reduced pressure, and the concentrated solution was separated by silica gel column chromatography (eluent: PE/EtOAc (v/v) = 3/1) to give yellow liquid (460 mg, 49%).
MS (ESI, pos.ion) m/z: 410.2 $[M+H]^+$.

**Step 3: Synthesis of 4-((2*S*,4*S*)-2-(hydroxymethyl)-4-(4-(trifluoromethyl)phenoxy) pyrrolidin-1-yl)benzoic acid**

To a solution of ethyl 4-((2S,4S)-2-(hydroxymethyl)-4-(4-(trifluoromethyl) phenoxy)pyrrolidin-1-yl)benzoate (460 mg, 1.16 mmol) in MeOH (2 mL) and THF (2 mL) was added a solution of $LiOH \cdot H_2O$ (488 mg, 11.63 mmol) in $H_2O$ (3 mL). The mixture was reacted at room temperature for 16 h. The reaction solution was concentrated under reduced pressure, and HCl solution (1 mol/L) was added to the concentrated solution to adjust the pH to about 4. The resulting mixture was extracted with EtOAc (20 mL × 2), and the organic phases were combined, washed with saturated NaCl (15 mL) solution, dried over anhydrous $Na_2SO_4$, concentrated under reduced pressure. The concentrated solution was separated by silica gel column chromatography (eluent: DCM/MeOH (v/v) = 20/1) to give a pale yellow solid (390 mg, 88%).
MS (ESI, pos.ion) m/z: 382.1 $[M+H]^+$.

**Step 4: Synthesis of *N*-((*S*)-2-cyano-1-(4-(ethylsulfonyl)phenyl)ethyl)-4-((2S,4S) -2-(hydroxymethyl)-4-(4-(trifluoromethyl)phenoxy)pyrrolidin-1-yl)benzamide**

HATU (180 mg, 0.47 mmol), (S)-3-amino-3-(4-(ethylsulfonyl)phenyl)propionitrile hydrochloride (82 mg, 0.34 mmol), 4-((2S,4S)-2-(hydroxymethyl)-4-(4-(trifluoromethyl) phenoxy)pyrrolidin-1-yl)benzoic acid (120 mg , 0.31 mmol) and TEA (95 mg, 0.94 mmol) were successively added to DCM (6 mL), and the mixture was reacted at room temperature for 18 h. The reaction solution was concentrated under reduced pressure, diluted with DCM (30 mL), washed successively with HCl solution (15 mL, 0.5 mol/L) and saturated NaCl solution (15 mL), dried over anhydrous $Na_2SO_4$, concentrated under reduced pressure. The concentrated solution was separated by silica gel column chromatography (eluent: DCM/EtOAc (v/v) = 1/1) to give a pale yellow solid (50 mg, 26%).

MS (ESI, pos.ion) m/z: 602.3 $[M+H]^+$.
$^1H$ NMR (400 MHz, $CDCl_3$) δ (ppm): 7.89 (d, *J*= 8.1 Hz, 2H), 7.70 (d, *J*= 8.7 Hz, 2H), 7.63 (d, *J*= 8.2 Hz, 2H), 7.57 (d, *J* = 8.6 Hz, 2H), 6.96 (d, *J* = 8.6 Hz, 2H), 6.93 (s, 1H), 6.65 (d, *J* = 8.5 Hz, 2H), 5.58 (dd, *J* = 13.3, 6.2 Hz, 1H), 5.13 (s, 1H), 4.10 (d, *J* = 3.8 Hz, 1H), 3.89 (dd, *J* = 10.8, 4.4 Hz, 1H), 3.79 - 3.71 (m, 2H), 3.68 (dd, *J* = 11.5, 4.8

Hz, 1H), 3.15 - 3.01 (m, 4H), 2.48 - 2.38 (m, 2H), 1.27 (t, *J*= 7.3 Hz, 3H).

**Example 81 *N*-((*S*)-2-cyano-1-(4-(ethylsulfonyl)phenyl)ethyl)-4-((2*S*,4*S*)-2-((difluoromethoxy) methyl)-4-(4-(trifluoromethyl)phenyl)pyrrolidin-1-yl)benzamide**

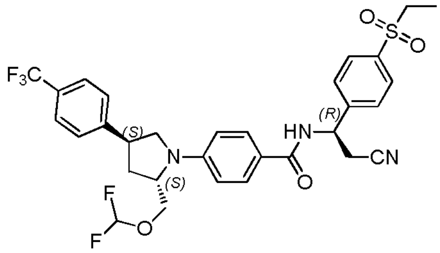

**Step 1: Synthesis of *(S)-1-tert-butyl* 2-methyl 4-(((trifluoromethyl)sulfonyl)oxy) -1H-pyrrole-1,2(2*H*,5*H*)-dicarboxylate**

To a solution of (*S*)-1-tert-butyl 2-methyl 4-oxopyrrolidine-1,2-dicarboxylate (19.91 g, 81.90 mmol) and DIPEA (23.1 mL, 139.77 mmol) in DCM (100 mL) was added $Tf_2O$ (18.0 mL, 106.99 mmol) dropwise at -10°C. After the drop was completed for 1 h, the mixture was reacted at room temperature for 8 h. After the reaction was completed, $H_2O$ (65 mL) was slowly added to the reaction solution to quench the reaction. The organic phase was extracted, and the aqueous phase was extracted with DCM (50 mL $\times$ 2). The combined organic phases were washed with saturated NaCl solution (60 mL), dried over anhydrous $Na_2SO_4$, concentrated under reduced pressure, and the concentrated solution was separated by silica gel column chromatography (eluent: PE/DCM (v/v) = 10/1) to give pale yellow transparent oil (29.00 g, 95%).

MS (ESI, pos.ion) m/z: 398.1 $[M+Na]^+$.
$^1H$ NMR (400 MHz, $CDCl_3$) $\delta$ (ppm): 5.74 (dd, *J*= 18.9, 1.7 Hz, 1H), 5.13 - 4.94 (m, 1H), 4.46 - 4.22 (m, 2H), 3.78 (s, 3H), 1.47 (d, *J*= 22.2 Hz, 9H).

**Step 2: Synthesis of (*S*)-*1*-tert-butyl 2-methyl 4-(4-(trifluoromethyl)phenyl) -1*H*-pyrrole-1,2(2*H*,5*H*)-dicarboxylate**

Under nitrogen protection, (4-(trifluoromethyl)phenyl)boronic acid (16.00 g, 84.20 mmol), *(S)-1-tert-butyl* 2-methyl 4-(((trifluoromethyl)sulfonyl)oxy)-1*H*-pyrrole -1,2(2*H*,5*H*)-dicarboxylate (29.00 g, 77.30 mmol) and $Pd(dppf)Cl_2$ (2.00 g, 2.65 mmol) were dissolved in 1,4-dioxane (80.0 mL), then $Cs_2CO_3$ (25.10 g, 77.00 mmol) was added, and the mixture was reacted at 100°C for 10 h. The reaction solution was cooled to room temperature, and filtered. The filtrate was concentrated under reduced pressure, and the concentrated solution was diluted with DCM (80 mL), washed successively with $NaHCO_3$ solution (40 mL) and saturated NaCl solution (50 mL), dried over anhydrous $Na_2SO_4$, concentrated under reduced pressure. The concentrated solution was separated by silica gel column chromatography (eluent: PE/EtOAc (v/v) = 5/1) to give a pale yellow solid (25.40 g, 88%).
MS (ESI, pos.ion) m/z: 394.2 $[M+Na]^+$.

**Step 3: Synthesis of *tert-butyl* (*S*)-2-(hydroxymethyl)-4-(4-(trifluoromethyl) phenyl)-2,5-dihydro-1*H*-pyrrole-1-carboxylate**

(*S*)-1-tert-Butyl 2-methyl 4-(4-(trifluoromethyl)phenyl)-1H-pyrrole-1,2(2H,5H)-dicarboxylate (7.10 g, 19.10 mmol) was dissolved in anhydrous THF (45 mL). The reaction solution was cooled to -10°C, then $LiBH_4$ (830 mg, 37.40 mmol) was added, and the mixture was reacted at room temperature for 8 h. Saturated $NH_4Cl$ (35 mL) solution was added dropwise to the reaction solution to quench the reaction. The mixture was concentrated under reduced pressure, and extracted with DCM (30 mL $\times$ 2). The combined organic phases were washed with saturated NaCl solution (30 mL), dried over anhydrous $Na_2SO_4$, concentrated under reduced pressure, and the concentrated solution was separated by silica gel column chromatography (eluent: PE/EtOAc (v/v) = 3/1) to give colorless transparent oil (4.80 g, 73%).
MS (ESI, pos.ion) m/z: 244.1 $[M-100+H]^+$.

**Step 4: Synthesis of *tert-butyl* (2*S*)-2-(hydroxymethyl)-4-(4-(trifluoromethyl)phenyl)pyrrolidine-1-carboxylate**

Under hydrogen protection, *tert*-butyl (*S*)-2-(hydroxymethyl)-4-(4-(trifluoromethyl) phenyl) -2,5-dihydro-1H-pyrrole-1-carboxylate (3.05 g, 8.88 mmol) was dissolved in EtOAc (30 mL). Pd/C (280 mg, 10%) was added, and the mixture was stirred at room temperature for 6 h. The resulting mixture was filtered through a celite pad. The filtrate was concentrated under reduced pressure, and the concentrated solution was separated by silica gel column chromatography (eluent: PE/EtOAc (v/v) = 5/1) to give colorless transparent liquid (3.00 g, 98%).
MS (ESI, pos.ion) m/z: 246.1 $[M-100+H]^+$.

**Step 5: Synthesis of ((2*S*)-4-(4-(trifluoromethyl)phenyl)pyrrolidin-2-yl)methanol**

tert-Butyl (2*S*)-2-(hydroxymethyl)-4-(4-(trifluoromethyl)phenyl)pyrrolidine -1-carboxylate (2.01 g, 5.40 mmol) was dissolved in DCM (30 mL) and TFA (25 mL). The mixture was reacted at room temperature for 5 h. The reaction solution was diluted with DCM (80 mL), then washed successively with saturated $NaHCO_3$ solution (50 mL) and saturated NaCl solution (30 mL), dried over anhydrous $Na_2SO_4$, and concentrated under reduced pressure to give pale yellow liquid (1.00 g, 68%).

**Step 6: Synthesis of methyl 4-((2*S*,4*S*)-2-(hydroxymethyl)-4-(4-(trifluoromethyl) phenyl)pyrrolidin-1-yl)benzoate**

Under nitrogen protection, ((2*S*)-4-(4-(trifluoromethyl)phenyl)pyrrolidin-2-yl)methanol (1.00 g, 4.08 mmol), methyl 4-iodobenzoate (1.46 g, 5.31 mmol), $Pd_2(dba)_3$ (373 mg, 0.41 mmol), 4,5-bisdiphenylphosphine-9,9-dimethylxanthene (354 mg, 0.61 mmol) and $Cs_2CO_3$ (2.66 g, 8.16 mmol) were dissolved in 1,4-dioxane (30 mL), and the mixture was reacted at 100°C for 16 h. The reaction solution was cooled to room temperature, and filtered. The filtrate was concentrated under reduced pressure. The concentrated solution was diluted with DCM (80 mL), washed successively with $NaHCO_3$ solution (20 mL) and saturated NaCl solution (20 mL), dried over anhydrous $Na_2SO_4$, concentrated under reduced pressure, and the concentrated solution was separated by silica gel column chromatography (eluent: PE/EtOAc (v/v) = 4/1) to give a brown solid (1.12 g, 71%).
MS (ESI, pos.ion) m/z: 394.1 $[M+H]^+$.

**Step 7: Synthesis of methyl 4-((2S)-2-((difluoromethoxy)methyl)-4-(4-(trifluoromethyl)phenyl)pyrrolidin-1-yl)benzoate**

Under nitrogen protection, to a solution of methyl 4-((2S)-2-(hydroxymethyl)-4-(4-(trifluoromethyl)phenyl)pyrrolidin-1-yl)benzoate (600 mg, 1.58 mmol) and KOAc (1.60 g, 16.10 mmol) in DCM/$H_2O$ (2.0 mL/2.0 mL) was added $TMSCF_2Br$ (2.0 mL, 12.80 mmol) dropwise. The mixture was reacted at room temperature for 15 h. The reaction solution was slowly poured into $H_2O$ (45 mL), then extracted with DCM (30 mL × 2). The combined organic phases were washed with saturated NaCl solution (30 mL × 2), dried over anhydrous $Na_2SO_4$, concentrated under reduced pressure, and the concentrated solution was separated by silica gel column chromatography (eluent: PE/EtOAc (v/v) = 3/1) to give brown oil (420 mg, 62%).
MS (ESI, pos.ion) m/z: 430.1 $[M+H]^+$.

**Step 8: Synthesis of 4-((2*S*,4*S*)-2-((difluoromethoxy)methyl)-4-(4-(trifluoromethyl) phenyl)pyrrolidin-1-yl)benzoic acid**

To a solution of methyl 4-((2S)-2-((difluoromethoxy)methyl)-4-(4-(trifluoromethyl) phenyl)pyrrolidin-1-yl)benzoate (420 mg, 0.98 mmol) in THF (10 mL) was added a solution of $LiOH \cdot H_2O$ (350 mg, 8.17 mmol) in $H_2O$ (3 mL). The mixture was heated to 55°C and reacted for 6 h. The reaction solution was cooled to room temperature, concentrated under reduced pressure, and HCl solution (1 mol/L) was dropwise added slowly to the concentrated solution at 0°C to adjust the pH to about 6. The resulting mixture was extracted with EtOAc (50 mL × 2), and the organic phase was washed with saturated NaCl (30 mL) solution, dried over anhydrous $Na_2SO_4$, concentrated under reduced pressure. The concentrated solution was separated by silica gel column chromatography (eluent: PE/EtOAc (v/v) = 1/1), and separated by chiral preparative chromatography (chromatographic column: Chiralpak AD-H (4.6mm*250mm, 5μm); mobile phase: *n*-hexane:ethanol = 20:80; isocratic elution; flow rate: 1 mL/min) to give a white solid (40 mg, 10%).
MS (ESI, pos.ion) m/z: 416.1 $[M+H]^+$.

**Step 9: Synthesis of *N*-((*S*)-2-cyano-1-(4-(ethylsulfonyl)phenyl)ethyl)-4-((2*S*,4*S*) -2-((difluoromethoxy)methyl)-4-(4-(trifluoromethyl)phenyl)pyrrolidin-1-yl)benzamide**

(*S*)-3-Amino-3-(4-(ethylsulfonyl)phenyl)propionitrile hydrochloride (40 mg, 0.17 mmol), 4-((2*S*,4*S*)-2-((difluoromethoxy)methyl)-4-(4-(trifluoromethyl)phenyl)pyrrolidin -1-yl)benzoic acid (60 mg , 0.14 mmol), HATU (80 mg, 0.21 mmol) and DIPEA (0.2 mL, 1 mmol) were dissolved in DCM (20 mL), and the mixture was reacted at room temperature for 14 h. The reaction solution was diluted with DCM (20 mL), washed successively with $NaHCO_3$ solution (20 mL) and saturated NaCl solution (10 mL). The organic phase was dried over anhydrous $Na_2SO_4$, concentrated under reduced pressure, and the concentrated solution was separated by silica gel column chromatography (eluent: PE/EtOAc (v/v) = 1/1) to give a white solid (50 mg, 55%).

MS (ESI, pos.ion) m/z: 636.2[M+H]$^+$.
$^1$H NMR (400 MHz, $CDCl_3$) $\delta$ (ppm): 7.93 (d, *J* = 8.3 Hz, 2H), 7.76 (d, *J* = 8.7 Hz, 2H), 7.64 (dd, *J* = 13.8, 8.2 Hz, 4H), 7.44 (d, *J* = 80 Hz, 2H), 6.74 - 6.69 (m, 1H), 6.67 (d, *J* = 8.8 Hz, 2H), 6.39 - 5.99 (m, 1H), 5.61 (dd, *J* = 12.6, 6.1 Hz, 1H), 4.29 (d, *J* = 4.3 Hz, 1H), 4.11 (dd, *J* = 10.2, 2.6 Hz, 1H), 3.94 - 3.83 (m, 2H), 3.59 (t, *J* = 9.5 Hz, 1H), 3.51 (dt, *J* = 17.2, 8.5 Hz, 1H), 3.11 (pd, *J* = 17.2, 5.8 Hz, 4H), 2.82 - 2.71 (m, 1H), 2.31 - 2.21 (m, 1H), 1.29 (t, *J* = 7.4 Hz, 3H).

**Example 82 *N*-((*S*)-2-cyano-1-(4-(ethylsulfonyl)phenyl)ethyl)-4-((*S*)-3-(4-(trifluoromethyl) phenoxy) pyrrolidin-1-yl)benzamide**

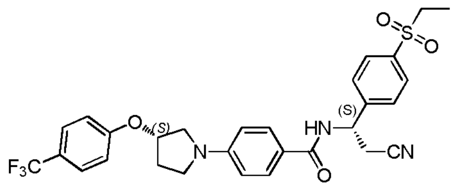

**Step 1: Synthesis of *tert-butyl* (*S*)-3-(4-(trifluoromethyl)phenoxy)pyrrolidine-1-carboxylate**

*tert*-Butyl (R)-3-hydroxypyrrolidine-1-carboxylate (1.00 g, 5.34 mmol), 4-(trifluoromethyl)phenol (865 mg, 5.34 mmol) and $PPh_3$ (2.00 g, 5.83 mmol) were added to THF (20 mL). The mixture was transferred to 0 °C, and DIAD (1.40 mL, 7.11 mmol) was slowly added. After the addition was complete, the mixture was transferred to room temperature and reacted for 21 h. The reaction solution was concentrated under reduced pressure. The concentrated solution was diluted with methyl *tert*-butyl ether (30 mL) and stirred at -20 °C. A large amount of white insoluble solid was precipitated, and filtered while cold. The filter cake was washed with cold methyl *tert*-butyl ether, and the filtrate was concentrated under reduced pressure. The concentrated solution was separated by silica gel column chromatography (eluent: PE/EtOAc (v/v) = 6/1) to obtain pale yellow liquid (1.76 g, 100%).
MS (ESI, pos.ion) m/z: 276.2 [M-56+H]$^+$.

**Step 2: Synthesis of (*S*)-3-(4-(trifluoromethyl)phenoxy)pyrrolidine**

To a solution of *tert-butyl* (*S*)-3-(4-(trifluoromethyl)phenoxy)pyrrolidine-1-carboxylate (800 mg, 2.42 mmol) in DCM (8 mL) was added a solution of HCl in methanol (2 mL, 20%). The mixture was reacted at room temperature for 9 h. The reaction solution was concentrated under reduced pressure to give light red liquid (558 mg, 100%).
MS (ESI, pos.ion) m/z: 232.2 [M+H]$^+$.

**Step 3: Synthesis of methyl (*S*)-4-(3-(4-(trifluoromethyl)phenoxy)pyrrolidin-1-yl)benzoate**

Under nitrogen protection, (*S*)-3-(4-(trifluoromethyl)phenoxy)pyrrolidine (600 mg, 2.60 mmol), $Pd_2(dba)_3$ (237 mg, 0.26 mmol), 4,5-bisdiphenylphosphine-9,9-dimethylxanthene (225 mg, 0.39 mmol), $Cs_2CO_3$ (1.26 g, 3.87 mmol) and methyl 4-iodobenzoate (884 mg, 3.37 mmol) were successively added to 1,4-dioxane (16 mL), and the mixture was reacted at 100 °C for 15 h. The reaction solution was cooled to room temperature, and filtered. The filtrate was concentrated under reduced pressure, and the concentrated solution was diluted with DCM (50 mL), washed successively with $NaHCO_3$ solution (15 mL) and saturated NaCl solution (15 mL), dried over anhydrous $Na_2SO_4$, concentrated under reduced pressure. The concentrated solution was separated by silica gel column chromatography (eluent: PE/EtOAc (v/v) = 4/1)

to give a yellow solid (850 mg, 89%).
MS (ESI, pos.ion) m/z: 366.3 $[M+H]^+$.

**Step 4: Synthesis of (*S*)-4-(3-(4-(trifluoromethyl)phenoxy)pyrrolidin-1-yl)benzoic acid**

To a solution of methyl (*S*)-4-(3-(4-(trifluoromethyl)phenoxy)pyrrolidin-1-yl)benzoate (850 mg, 2.33 mmol) in MeOH (6 mL) and THF (6 mL) was added a solution of $LiOH \cdot H_2O$ (2.00 g, 47.70 mmol) in $H_2O$ (6 mL). The mixture was reacted at room temperature for 19 h. The reaction solution was concentrated under reduced pressure, and HCl solution (3 mol/L) was added to the concentrated solution to adjust the pH to about 4. The resulting mixture was extracted with EtOAc

(30 mL × 2), and the organic phases were combined, washed with saturated NaCl (25 mL) solution, dried over anhydrous $Na_2SO_4$, concentrated under reduced pressure. The concentrated solution was separated by silica gel column chromatography (eluent: PE/EtOAc (v/v) = 2/1) to give a white solid (550 mg, 67%).
MS (ESI, pos.ion) m/z: 352.1 $[M+H]^+$.

**Step 5: Synthesis of *N*-((*S*)-2-cyano-1-(4-(ethylsulfonyl)phenyl)ethyl)-4-((*S*) -3-(4-(trifluoromethyl)phenoxy)pyrrolidin-1-yl)benzamide**

EDCI (65 mg, 0.34 mmol), HOBT (46 mg, 0.34 mmol), (S)-3-amino-3-(4-(ethylsulfonyl)phenyl)propionitrile hydrochloride (60 mg, 0.25 mmol), (S)-4-(3-(4-(trifluoromethyl)phenoxy)pyrrolidin-1-yl)benzoic acid (80 mg, 0.23 mmol) and TEA(46 mg, 0.45 mmol) were successively added to DCM (6 mL), and the mixture was reacted at room temperature for 18 h. The reaction solution was diluted with DCM (30 mL), washed successively with $NaHCO_3$ solution (15 mL) and saturated NaCl solution (15 mL), dried over anhydrous $Na_2SO_4$, concentrated under reduced pressure, and the concentrated solution was separated by silica gel column chromatography (eluent: DCM/EtOAc (v/v) = 5/1) to give a white solid (65 mg, 50%).

MS (ESI, pos.ion) m/z: 572.1 $[M+H]^+$.
$^1H$ NMR (400 MHz, DMSO-$d_6$) δ (ppm): 8.86 (*d, J* = 8.2 Hz, 1H), 7.88 (d, *J* = 8.3 Hz, 2H), 7.78 (d, *J* = 8.7 Hz, 2H), 7.71 (d, *J* = 8.3 Hz, 2H), 7.66 (*d, J* = 8.6 Hz, 2H), 7.17 (d, *J* = 8.6 Hz, 2H), 6.62 (d, *J* = 8.7 Hz, 2H), 5.49 (dd, *J* = 15.4, 8.0 Hz, 1H), 5.31 (s, 1H), 3.75 (*dd, J* = 11.7, 4.5 Hz, 1H), 3.47 (*d, J* = 11.4 Hz, 2H), 3.31 - 3.24 (m, 3H), 3.14 (d, *J* = 6.9 Hz, 2H), 2.41 - 2.32 (m, 1H), 2.27 - 2.20 (m, 1H), 1.09 (t, *J*= 7.3 Hz, 3H).

The material (methyl 4-iodobenzoate) in step 3 of Example 82 was replaced with other reaction substrates, which were used to prepare the target compounds in Table 3 according to the methods of step 3 to step 5 of Example 82 with the intermediate ((*S*)-3-(4-(trifluoromethyl)phenoxy)pyrrolidine) in step 2; or the material ((*S*)-3-amino-3-(4-(ethylsulfonyl)phenyl)propionitrile hydrochloride) in step 5 of Example 82 was replaced with other reaction substrates, which were used to prepare the target compounds in Table 3 according to the method of step 5 of Example 82 with the intermediate ((S)-3-(4-(trifluoromethyl)phenoxy)pyrrolidin-1-yl) in step 4.

Table 3 The target compound prepared according to the synthetic method of Example 82

| Example No. | Target compound structure and name | Target compound characterization data |
|---|---|---|
| **Example 83** | 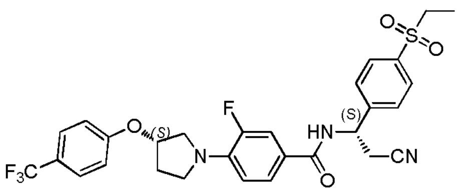 N-((S)-2-cyano-1-(4-(ethylsulfonyl) phenyl)ethyl)-3-fluoro-4-((S)-3-(4-( trifluoromethyl)phenoxy)pyrrolidin -1-yl)benzamide | white solid; MS (ESI, pos.ion) m/z: 590.3 $[M+H]^+$; $^1H$ NMR (400 MHz, $CDCl_3$) δ (ppm): 9.00 (d, *J* = 8.2 Hz, 1H), 7.88 (d, *J* = 8.3 Hz, 2H), 7.71 (d, *J* = 8.3 Hz, 2H), 7.68 - 7.61 (m, 4H), 7.16 (d, *J* = 8.6 Hz, 2H), 6.81 (t, *J*= 8.8 Hz, 1H), 5.49 (dd, *J* = 15.2, 8.1 Hz, 1H), 5.26 (s, 1H), 3.91 (d, *J* = 10.7 Hz, 1H), 3.57 (d, *J* = 10.4 Hz, 3H), 3.27 (*q, J* = 7.3 Hz, 2H), 3.15 - 3.11 (m, 2H), 2.34 - 2.27 (m, 1H), 2.22 - 2.14 (m, 1H), 1.09 (t, *J*= 7.4 Hz, 3H). |

(continued)

| Example No. | Target compound structure and name | Target compound characterization data |
|---|---|---|
| **Example 84** | 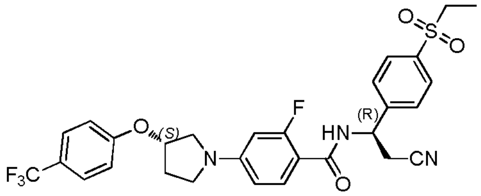 *N*-((*R*)-2-cyano-1-(4-(ethyl sulfonyl) phenyl)ethyl)-2-fluoro-4-((*S*)-3-(4-( trifluoromethyl)phenoxy)pyrrolidin -1-yl)benzamide | white solid; MS (ESI, pos.ion) m/z: 590.0 $[M+H]^+$; $^1H$ NMR (400 MHz, $CDCl_3$) δ (ppm): 7.99 (d, *J* = 8.3 Hz, 2H), 7.95 (d, *J* = 9.1 Hz, 1H), 7.68 (d, *J* = 8.3 Hz, 2H), 7.59 (d, *J* = 8.6 Hz, 2H), 7.20 (dd, *J* = 15.8, 7.3 Hz, 1H), 6.98 (d, *J* = 8.6 Hz, 2H), 6.44 (dd, *J* = 8.9, 2.0 Hz, 1H), 6.24 (dd, *J* = 15.9, 2.0 Hz, 1H), 5.63 (d, *J* = 6.2 Hz, 1H), 5.16 (s, 1H), 3.76 (dd, *J* = 11.3, 4.6 Hz, 1H), 3.63 - 3.52 (m, 3H), 3.21 - 3.11 (m, 3H), 3.06 (dd, *J* = 16.9, 5.0 Hz, 1H), 2.47 - 2.30 (m, 2H), 1.32 (t, *J* = 7.4 Hz, 3H). |
| **Example 85** | 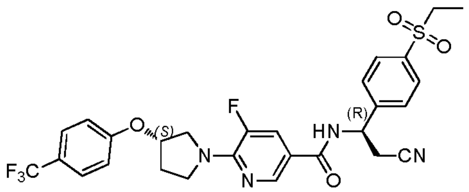 *N*-((R)-2-cyano-1-(4-(ethylsulfonyl) phenyl)ethyl)-5-fluoro-6-((S)-3-(4-(trifluoromethyl)phenoxy)pyrrolidin -1-yl)nicotinamide | white solid; MS (ESI, pos.ion) m/z: 591.2 $[M+H]^+$; $^1H$ NMR (400 MHz, DMSO-$d_6$) δ (ppm): 9.06 (d, *J* = 8.0 Hz, 1H), 8.49 (s, 1H), 7.89 (d, *J* = 8.4 Hz, 2H), 7.84 (s, 1H), 7.72 (d, *J*= 8.2 Hz, 2H), 7.67 (d, *J* = 8.6 Hz, 2H), 7.18 (d, *J* = 8.5 Hz, 2H), 5.50 (dd, *J* = 14.7, 8.3 Hz, 1H), 5.27 (s, 1H), 4.04 - 3.97 (m, 1H), 3.91-3.81 (m, 2H), 3.75 (dd, *J* = 17.6, 9.1 Hz, 1H), 3.32 - 3.22 (m, 2H), 3.20 - 3.08 (m, 2H), 2.36 - 2.18 (m, 2H), 1.09 (t, *J*= 7.3 Hz, 3H). |
| **Example 86** | 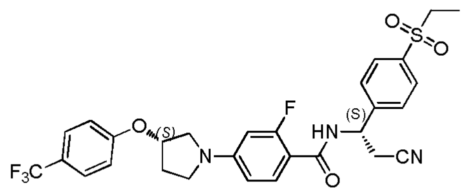 *N*-((*S*)-2-cyano-1-(4-(ethylsulfonyl) phenyl)ethyl)-2-fluoro-4-((S)-3-(4-( trifluoromethyl)phenoxy)pyrrolidin -1-yl)benzamide | white solid; MS (ESI, pos.ion) m/z: 590.1 $[M+H]^+$; $^1H$ NMR (400 MHz, $CDCl_3$) δ (ppm): 7.97 (d, *J* = 8.3 Hz, 2H), 7.93 (d, *J* = 9.1 Hz, 1H), 7.65 (d, *J* = 8.3 Hz, 2H), 7.57 (d, *J* = 8.6 Hz, 2H), 7.17 (dd, *J* = 15.9, 7.3 Hz, 1H), 6.96 (d, *J* = 8.6 Hz, 2H), 6.42 (dd, *J* = 8.9, 2.1 Hz, 1H), 6.21 (dd, *J* = 15.9, 2.0 Hz, 1H), 5.61 (d, *J* = 6.4 Hz, 1H), 5.14 (s, 1H), 3.74 (dd, *J* = 11.3, 4.6 Hz, 1H), 3.61 - 3.50 (m, 3H), 3.19 - 3.09 (m, 3H), 3.04 (dd, *J* = 16.9, 4.9 Hz, 1H), 2.45 - 2.29 (m, 2H), 1.30 (t, *J* = 7.4 Hz, 3H). |
| **Example 87** | 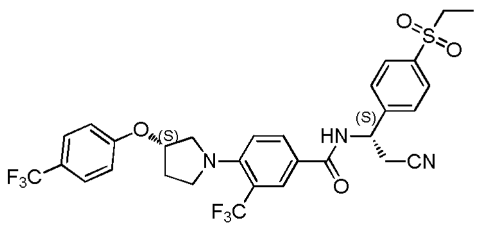 *N*-((*S*)-2-cyano-1-(4-(ethylsulfonyl) phenyl)ethyl)-3-(trifluoromethyl)-4 -((S)-3-(4-(trifluoromethyl)phenoxy )pyrrolidin-1-yl)benzamide | brown solid; MS (ESI, pos.ion) m/z: 640.4 $[M+H]^+$. $^1H$ NMR (400 MHz, $CDCl_3$) δ (ppm) 8.14 (s, 1H), 7.80 (dt, *J* = 20.3, 10.3 Hz, 4H), 7.61 (d, *J* = 8.1 Hz, 2H), 7.54 (d, *J* = 8.4 Hz, 2H), 6.95 (d, *J* = 8.4 Hz, 2H), 6.87 (d, *J* = 8.9 Hz, 1H), 5.61 (dd, *J* = 13.8, 6.7 Hz, 1H), 5.06 (s, 1H), 3.93 (dd, *J* = 10.9, 3.5 Hz, 1H), 3.73 (dd, *J* = 16.4, 8.9 Hz, 1H), 3.65 - 3.44 (m, 2H), 3.22 - 2.98 (m, 4H), 2.27 (d, *J* = 21.6 Hz, 2H), 1.23 (dd, *J* = 13.6, 6.4 Hz, 3H). |

(continued)

| Example No. | Target compound structure and name | Target compound characterization data |
|---|---|---|
| Example 88 | 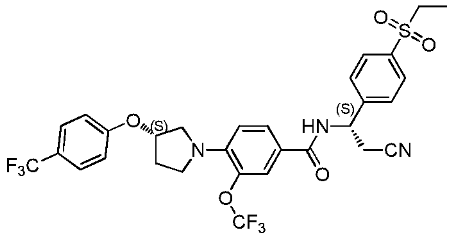 N-((S)-2-cyano-1-(4-(ethylsulfonyl) phenyl)ethyl)-3 -(trifluoromethoxy)-4-((S)-3-(4-(trifluoromethyl)phenox y)pyrrolidin-1-yl)benzamide | brown solid; MS (ESI, pos.ion) m/z: 655.9 $[M+H]^+$; $^1$H NMR (400 MHz, $CDCl_3$) δ 7.90 (t, *J* = 9.2 Hz, 3H), 7.78 - 7.59 (m, 4H), 7.53 (d, *J* = 8.6 Hz, 2H), 6.93 (d, *J* = 8.5 Hz, 2H), 6.71 (d, *J* = 8.7 Hz, 1H), 5.60 - 5.49 (m, 1H), 5.06 (s, 1H), 3.95 (dd, *J* = 11.6, 4.5 Hz, 1H), 3.74 - 3.62 (m, 2H), 3.57 (t, *J* = 7.9 Hz, 1H), 3.17 - 3.02 (m, 4H), 2.35 - 2.17 (m, 2H), 1.26 (t, *J* = 7.4 Hz, 3H). |

**Example 89 *N*-((*S*)-2-cyano-1-(4-(ethylsulfonyl)phenyl)ethyl)-4-((2S)-2-((difluoromethoxy) methyl)-4-((4-(trifluoromethyl)cyclohexyl)oxy)pyrrolidin-1-yl)benzamide**

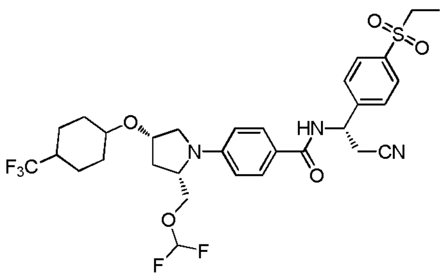

**Step 1: Synthesis of (2S,4S)-1-benzyl 2-methyl 4-hydroxypyrrolidine-1,2-dicarboxylate**

To a solution of (2*S*,4*S*)-1-*tert*-butyl 2-methyl 4-hydroxypyrrolidine-1,2-dicarboxylate (6.6 g, 27 mmol) in dichloromethane (25 mL, 390 mmol) was slowly added a solution of HCl in 1,4-dioxane (30 mL, 120 mmol) at 0°C, and the mixture was reacted at room temperature overnight, then the reaction was stopped. TLC monitored the disappearance of raw materials. The resulting mixture was concentrated under reduced pressure to give 4.9 g of methyl (2S,4S)-4-hydroxypyrrolidine-2-carboxylate hydrochloride as a white solid powder. To a solution of methyl (2S,4,S)-4-hydroxypyrrolidine-2-carboxylate hydrochloride (4.9 g, 27 mmol) and benzyl chloroformate (3.9 mL, 27 mmol) in THF (20 mL) and $H_2O$ (20 mL) was slowly added TEA (12 mL, 86.1 mmol) at 0°C. After 24 hours of reaction at room temperature, the reaction was stopped. The mixture was diluted with saturated $NH_4Cl$ solution (50 mL), and extracted with EtOAc (50 mL × 3). The combined organic phases were washed with saturated NaCl solution (20 mL), dried over anhydrous $Na_2SO_4$, concentrated under reduced pressure, and the concentrate was separated by silica gel column chromatography (eluent: PE/EtOAc (v/v) = 4/1) to give colorless oil ( 5.8 g, 77%).
$^1$H NMR (400 MHz, $CDCl_3$) δ (ppm): 7.49 - 7.30 (m, 5H), 5.14 (dt, *J*= 22.0, 12.4 Hz, 2H), 4.53 - 4.34 (m, 2H), 3.85 - 3.58 (m, 5H), 3.32 (dd, *J* = 62.2, 9.3 Hz, 1H), 2.35 (tdd, *J* = 14.3, 9.8, 4.6 Hz, 1H), 2.15 (dd, *J* = 13.9, 4.8 Hz, 1H)。

**Step 2: Synthesis of (2S,4S)-1-benzyl 2-methyl 4-(4-(trifluoromethyl)cyclohexyl)oxy) pyrrolidine-1,2-dicarboxylate**

To a solution of (2S,4S)-1-benzyl 2-methyl 4-hydroxypyrrolidine-1,2-dicarboxylate (2.40 g, 8.59 mmol) and TEA (1.4 mL, 10 mmol) in THF (25 mL) was added trimethylchlorosilane (1.2 mL, 9.2 mmol) at 0°C. After 1.5 hours of reaction at room temperature, the stirring was stopped. The mixture was filtered through a celite pad, washed with petroleum ether, and concentrated to obtain a pale yellow oily product (2S,4S)-1-benzyl 2-methyl 4-((trimethylsilyl)oxy)pyrrolidine-1,2-dicarboxylate. To a solution of (2S,4S)-1-benzyl-2-methyl-4-((trimethylsilyl)oxy)pyrrolidine-1,2-dicarboxylate and 4-trifluoromethylcyclohexanone (1.43 g, 8.61 mmol) in dichloromethane (30 mL, 468.0 mmol) were added triethylsilane

(1.52 mL, 9.42 mmol) and trimethylsilyl trifluoromethanesulfonate (700 μL, 4.168 mmol) in turn at -60°C. The mixture was warmed to 0°C and reacted for 12 hours, then the reaction was stopped. The resulting mixture was diluted with EtOAc (50 mL), and added with saturated $NaHCO_3$to adjust to neutral. The mixture was extracted with EtOAc (30 mL × 3), dried over anhydrous $Na_2SO_4$, separated by silica gel column chromatography (eluent: PE/EtOAc (v/v) = 8/1) to give colorless oil (442 mg, 12.0%).
MS (ESI, pos.ion) m/z: 430.3 $[M+H]^+$.

**Step 3: Synthesis of benzyl (2S,4S)-(hydroxymethyl) -4-((4-(trifluoromethyl)cyclohexyl)oxy)pyrrolidine-1-carboxylate**

To a solution of (2S,4S)-1-benzyl 2-methyl 4-((4-(trifluoromethyl)cyclohexyl)oxy)pyrrolidine-1,2-dicarboxylate (442 mg, 1.03 mmol) in THF (12 mL) was added lithium borohydride (30 mg, 1.38 mmol) at 0°C. The reaction was stopped after 12 hours of reaction at room temperature, and quenched by adding saturated $NH_4Cl$ solution (20 mL). The resulting mixture was extracted with EtOAc (30 mL × 3), and dried over anhydrous $Na_2SO_4$. The concentrated solution was separated by silica gel column chromatography (eluent: PE/EtOAc (v/v) = 2/1) to give the product (400 mg, 96.81%) as colorless oil.
MS (ESI, pos.ion) m/z: 402.1 $[M+H]^+$.

**Step 4: Synthesis of benzyl (2*S*,4*S*)-2-((difluoromethoxy)methyl)-4-((4-(trifluoromethyl)cyclohexyl)oxy)pyrrolidine-1-carboxylate**

To a solution of benzyl (2S,4S)-(hydroxymethyl)-4-((4-(trifluoromethyl) cyclohexyl)oxy)pyrrolidine-1-carboxylate (400 mg, 1.00 mmol) in DCM (5 mL) and $H_2O$ (6 mL) were added KOAc (450 mg, 4.585 mmol) and $TMSCF_2Br$ (520 μL, 3.34 mmol) at 0°C. The reaction was stopped after 24 hours of reaction at room temperature, and quenched by adding saturated $NH_4Cl$ solution (30 mL). The resulting mixture was extracted with EtOAc (30 mL × 3), dried over anhydrous $Na_2SO_4$, concentrated under reduced pressure. The concentrated solution was separated by silica gel column chromatography (eluent: PE/EtOAc (v/v) = 6/1) to give the product (367 mg, 81.59%) as pale yellow oil.
MS (ESI, pos.ion) m/z: 452.1 $[M+H]^+$.

**Step 5: Synthesis of methyl 4-((2S,4S)-2-((difluoromethoxy)methyl)-4-((4-(trifluoromethyl)cyclohexyl)oxy)pyrrolidin-1-yl)benzoate**

To a solution of benzyl (2S,4,S)-2-((difluoromethoxy)methyl)-4-((4-(trifluoromethyl) cyclohexyl)oxy)pyrrolidine-1-carboxylate (367 mg, 0.81 mmol) in MeOH (10 mL) was added Pd/C (56 mg, 10%). The mixture was reacted at room temperature for 3 hours under $H_2$ protection, filtered through a celite pad, washed with DCM, and concentrated to give a colorless oily product (2S,4S)-2-((difluoromethoxy)methyl)-4-((4-(trifluoromethyl)cyclohexyl)oxy)pyrrolidine (220 mg, 85.28%). To a mixture of (2S,4S)-2-((difluoromethoxy)methyl)-4-((4-(trifluoromethyl)cyclohexyl)oxy)pyrrolidine (220 mg, 0.69 mmol), $Pd_2(dba)_3$ (65 mg, 0.07 mmol), 2-bicyclohexylphosphine-2',6'-diisopropoxybiphenyl (40 mg, 0.08 mmol) and $Cs_2CO_3$ (360 mg, 1.08 mmol) were added 1,4-dioxane (6 mL) and methyl *p*-iodobenzoate (200 mg, 0.76 mmol) at room temperature. Under nitrogen protection, the mixture was reacted at 100°C for 18 hours, The reaction was quenched by adding saturated $NH_4Cl$ solution (30 mL). The resulting mixture was extracted with EtOAc (30 mL × 3), dried over anhydrous $Na_2SO_4$, and concentrated under reduced pressure. The concentrated solution was separated by silica gel column chromatography (eluent: PE/EtOAc (v/v) = 4/1) to give the product (217 mg, 69.33%) as pale yellow oil.
MS (ESI, pos.ion) m/z: 452.1 $[M+H]^+$.

**Step 6: Synthesis of *N*-((S)-2-cyano-1-(4-(ethylsulfonyl)phenyl)ethyl)-4-((2S)-2-((difluoromethoxy)methyl)-4-((4-(trifluoromethyl)cyclohexyl)oxy)pyrrolidin-1-yl)benzamide**

To a solution of methyl 4-((2*S*,4*S*)-2-((difluoromethoxy)methyl)-4-((4-(trifluoromethyl) cyclohexyl)oxy)pyrrolidin-1-yl)benzoate (217 mg, 0.48 mmol) in THF (4 mL) and MeOH (4 mL) were added $H_2O$ (0.5 mL) and NaOH (100 mg, 2.50 mmol) at room temperature. The mixture was reacted at 60°C for 24 h. The reaction was stopped, and the mixture was diluted with $H_2O$ (20 mL), then dilute hydrochloric acid was added to adjust pH to 6-7. The resulting mixture was extracted with DCM (30 mL × 3), dried over anhydrous $Na_2SO_4$, and concentrated to give a pale yellow solid product 4-((2*S*,4*S)*-2-((difluoromethoxy)methyl)-4-((4-(trifluoromethyl)cyclohexyl)oxy)pyrrolidin-1-yl)ben zoic acid (197 mg, 93.69%). To a mixture of 4-((2*S*,4*S*)-2-((Difluoromethoxy)methyl)-4-((4-(trifluoromethyl)cyclohexyl)oxy)pyrrolidin -1-yl)benzoic acid (68 mg, 0.15 mmol), (*S*)-3-amino-3-(4-(ethylsulfonyl)phenyl)propionitrile hydrochloride (52 mg, 0.19 mmol), HOBt (43 mg, 0.38 mmol) and EDCI (60 mg, 0.31 mmol) were added DCM (6 mL, 93.61 mmol) and TEA (70 μL, 0.50 mmol) at room temperature. After the mixture was reacted at room temperature for 24 h, the reaction was

stopped. The resulting mixture was diluted with DCM (30 mL), washed with saturated $NH_4Cl$ (15 mL), and dried over anhydrous $Na_2SO_4$, concentrated. The concentrated solution was separated by silica gel column chromatography (eluent: PE/EtOAc (v/v) = 4/1) to give a pale yellow solid (62 mg, 59.29%).

MS (ESI, pos.ion) m/z: 658.0 [M+H]$^+$.
$^1$H NMR (400 MHz, $CDCl_3$) $\delta$ (ppm): 7.87 (d, *J*= 8.3 Hz, 2H), 7.75 (d, *J*= 8.8 Hz, 2H), 7.64 (d, *J*= 8.3 Hz, 2H), 7.02 (d, *J* = 7.7 Hz, 1H), 6.61 (d, *J* = 8.8 Hz, 2H), 6.26 (t, *J* = 74.7 Hz, 1H), 5.59 (dd, *J* = 13.3, 6.3 Hz, 1H), 4.39 (t, *J* = 4.1 Hz, 1H), 4.18 - 4.04 (m, 2H), 3.98 (t, *J* = 10.8 Hz, 1H), 3.48 (dt, *J* = 10.9, 7.7 Hz, 2H), 3.37 (ddd, *J* = 14.2, 10.2, 3.9 Hz, 1H), 3.18 - 3.01 (m, 4H), 2.29 (d, *J* = 13.9 Hz, 1H), 2.12 (dd, *J*= 15.5, 9.5 Hz, 3H), 2.07 - 1.93 (m, 3H), 1.41 (dd, *J*= 17.6, 8.4 Hz, 2H), 1.28 (t, *J* = 7.4 Hz, 5H)。

**Example 90 2-((R)-2-(4-((2S,4S)-2-((difluoromethoxy)methyl)-4-(4-(trifluoromethyl)phenoxy) pyrrolidin-1-yl)benzoylamino)-2-(4-(ethylsulfonyl)phenyl)ethoxy)acetic acid**

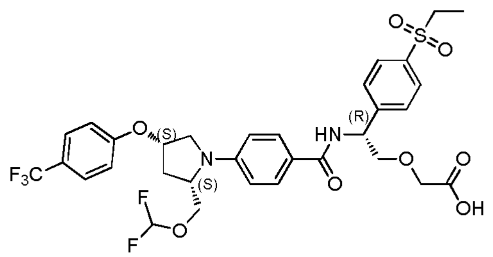

**Step 1: Synthesis of 4-((2*S*,4*S*)-2-((difluoromethoxy)methyl)-4-(4-trifluoromethyl) phenoxy)pyrrolidin-1-yl)-N-((R)-1-(4-(ethylsulfonyl)phenyl)-2-hydroxyethyl)benzamide**

(R)-2-Amino-2-(4-(ethylsulfonyl)phenyl)ethanol (38 mg, 0.17 mmol), 4-((2S,4S)-2-((difluoromethoxy)methyl)-4-(4-trifluoromethyl)phenoxy)pyrrolidin-1-yl)benzoic acid (60 mg , 0.14 mmol), EDCI (39 mg, 0.20 mmol), HOBT (28 mg, 0.21 mmol) and TEA (14 mg, 0.14 mmol) were dissolved in DCM (6 mL), and the mixture was reacted at room temperature for 5 h. The reaction solution was diluted with DCM (40 mL), washed successively with $NaHCO_3$ solution (15 mL) and saturated NaCl solution (15 mL), dried over anhydrous $Na_2SO_4$, concentrated under reduced pressure, and the concentrated solution was separated by silica gel column chromatography (eluent: DCM/MeOH (v/v) = 20/1) to give a white solid (55 mg, 62%).
MS (ESI, pos.ion) m/z = 643.1 [M+H]$^+$.

**Step 2: Synthesis of ethyl 2-((*R*)-2-(4-((2*S*,4*S*)-2-((difluoromethoxy) methyl)-4-(4-(trifluoromethyl)phenoxy)pyrrolidin-1-yl)benzoylamino)-2-(4-(ethylsulfonyl) phenyl)ethoxy)acetate**

To a solution of 4-((2S,4S)-2-((difluoromethoxy)methyl)-4-(4-trifluoromethyl) phenoxy)pyrrolidin-1-yl)-N((R)-1-(4-(ethylsulfonyl)phenyl)-2-hydroxyethyl)benzamide (1.01 g, 1.57 mmol) in DMF (5 mL) was added NaH (0.10 g, 3.00 mmol). The mixture was reacted at room temperature for 0.5 h, then ethyl 2-bromoacetate (0.2 mL, 2.00 mmol) was added, and the reaction was continued at room temperature for 4 h. The resulting mixture was washed with saturated NaCl, extracted with EtOAc (10 mL $\times$ 2), dried over anhydrous $Na_2SO_4$, concentrated and separated by silica gel column chromatography (eluent: DCM/EtOAc (v/v) = 5/1) to give a white solid (1.02 g, 89%).
MS (ESI, pos.ion) m/z: 729 [M+H]$^+$.

**Step 3: Synthesis of 2-((*R*)-2-(4-((2*S*,4*S*)-2-((difluoromethoxy)methyl)-4-(4-(trifluoromethyl) phenoxy)pyrrolidin-1-yl)benzoylamino)-2-(4-(ethylsulfonyl)phenyl)ethoxy)acetic acid**

To a solution of 2-((*R*)-2-(4-((2*S*,4*S*)-2-((difluoromethoxy)methyl)-4-(4-(trifluoromethyl)phenoxy)pyrrolidin-1-yl)benzoylamino)-2-(4-(ethylsulfonyl)phenyl)ethoxy)acet ic acid (120 mg, 0.16 mmol) in MeOH (5 mL) was added LiOH (0.11 g, 0.80 mmol). The mixture was reacted at room temperature for 12 h. Dilute hydrochloric acid was added to adjust the pH of the solution to be weakly acidic. The resulting mixture was extracted with EtOAc (10 mL $\times$ 2), dried over anhydrous $Na_2SO_4$, concentrated and separated by silica gel column chromatography (eluent: PE/EtOAc (v/v) = 1/1) to give a white solid (0.092 g, 79%).

MS (ESI, pos.ion) m/z: 701 [M+H]+.

$^1$H NMR (400 MHz, MeOD) δ (ppm): 7.89 (d, *J* = 8.7 Hz, 2H), 7.81 (d, *J* = 8.3 Hz, 2H), 7.58 (dd, *J* = 17.2, 8.4 Hz, 4H), 6.98 (d, *J* = 8.5 Hz, 2H), 6.65 (d, *J* = 8.8 Hz, 2H), 6.23 (t, *J* = 74.5 Hz, 1H), 5.22 (d, *J* = 32.7 Hz, 2H), 4.26 - 4.10 (m, 6H), 3.90 (dd, *J* = 35.6, 7.1 Hz, 3H), 3.78 - 3.65 (m, 2H), 3.09 (q, *J* = 7.4 Hz, 2H), 2.49 (d, *J* = 14.3 Hz, 1H), 2.39 (d, *J* = 7.6 Hz, 1H), 1.24 (t, *J* = 7.4 Hz, 3H).

**Example 91 2-((*R*)-2-(4-((2*S*,4*S*)-2-((difluoromethoxy)methyl)-4-(4-(trifluoromethyl)phenoxy) pyrrolidin-1-yl)benzoylamino)-2-(4-(ethylsulfonyl)phenyl)ethoxy)-2-methylpropionic acid**

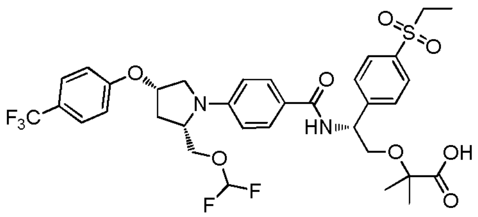

**Step 1: Synthesis of ethyl 2-((*R*)-2-(4-((2*S*,4*S*)-2-((difluoromethoxy) methyl)-4-(4-(trifluoromethyl)phenoxy)pyrrolidin-1-yl)benzoylamino)-2-(4-(ethylsulfonyl) phenyl)ethoxy)-2-methylpropionate**

To a solution of 4-((2S,4S)-2-((difluoromethoxy)methyl)-4-(4-trifluoromethyl) phenoxy)pyrrolidin-1-yl)-N((R)-1-(4-(ethylsulfonyl)phenyl)-2-hydroxyethyl)benzamide (1.01 g, 1.57 mmol) in DMF (5 mL) was added NaH (0.31 g, 7.85 mmol) at 0°C. The mixture was reacted for 0.5 h, then NaI (1.18 g, 7.87 mmol) and ethyl 2-bromo-2-methyl-propionate (1 mL, 7.7 mmol) were added. The temperature was raised to 80°C, and the reaction was continued for 24 h. The resulting mixture was washed with saturated NaCl, extracted with EtOAc (10 mL × 2), dried over anhydrous $Na_2SO_4$, concentrated and separated by silica gel column chromatography (eluent: DCM/EtOAc (v/v) = 5/1) to give a white solid (0.87 g, 73%).

MS (ESI, pos.ion) m/z: 757 [M+H]+.

**Step 2: Synthesis of 2-((R)-2-(4-((2S,4S)-2-((difluoromethoxy)methyl)-4-(4-(trifluoromethyl) phenoxy)pyrrolidin-1-yl)benzoylamino)-2-(4-(ethylsulfonyl)phenyl)ethoxy)-2-methylpropionic acid**

To a solution of ethyl 2-((*R*)-2-(4-((2*S*,4*S*)-2-((difluoromethoxy) methyl)-4-(4-(trifluoromethyl)phenoxy)pyrrolidin-1-yl)benzoylamino)-2-(4-(ethylsulfonyl) phenyl)ethoxy)-2-methylpropionate (200 mg, 0.26 mmol) in MeOH (5 mL) was added LiOH (12.7 mg, 0.530 mmol, 100%). The mixture was reacted at room temperature for 24 h. Dilute hydrochloric acid was added to adjust the pH of the solution to be weakly acidic. The resulting mixture was extracted with EtOAc (10 mL × 2), dried over anhydrous $Na_2SO_4$, concentrated and separated by silica gel column chromatography (eluent: DCM/MeOH (v/v) = 20/1) to give a white solid (0.10 g, 52%).

MS (ESI, pos.ion) m/z: 729 [M+H]+.

$^1$H NMR (400 MHz, $CD_3OD$) δ (ppm) 7.89 (dd, *J*= 15.0, 8.4 Hz, 4H), 7.65 (t, *J*= 8.5 Hz, 4H), 7.14 (d, *J* = 8.6 Hz, 2H), 6.76 (d, *J* = *8.6* Hz, 2H), 6.41 (t, *J* = 75.1 Hz, 1H), 5.31 (s, 1H), 5.16 (s, 1H), 4.30 - 4.10 (m, 2H), 3.97 (t, *J* = 9.5 Hz, 1H), 3.91 - 3.70 (m, 4H), 3.36 - 3.30 (m, 2H), 3.19 (q, *J* = 7.4 Hz, 2H), 1.44 (d, *J*= 5.0 Hz, 6H), 1.22 (t, *J*= 7.4 Hz, 3H).

**Example 92 (*S*)-3-(4-((2S,4S)-2-((difluoromethoxy)methyl)-4-(4-(trifluoromethyl)phenoxy) pyrrolidin -1-yl)benzoylamino)-3-(4-(ethylsulfonyl)phenyl)propionic acid**

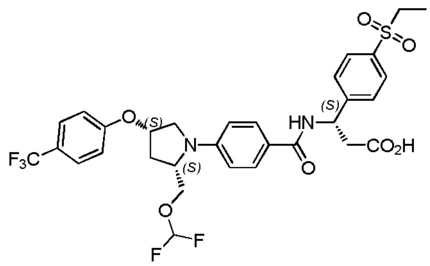

**Step 1: Synthesis of methyl (*S*)-3-(4-((2S,4S)-2-((difluoromethoxy)methyl) -4-(4-(trifluoromethyl)phenoxy)pyrrolidin-1-yl)benzoylamino)-3-(4-(ethylsulfonyl)phenyl)pro pionate**

4-((2*S*,4*S*)-2-((Difluoromethoxy)methyl)-4-(4-(trifluoromethyl)phenoxy)pyrrolidin-1-y l)benzoic acid (160 mg, 0.37 mmol), methyl (S)-3-amino-3-(4-(ethylsulfonyl)phenyl)propanoate (88 mg, 0.32 mmol), HATU (190 mg, 0.48 mmol) and DIPEA (0.1 mL, 0.6 mmol) were dissolved in DCM (20 mL). The mixture was reacted at room temperature for 30 h. The reaction solution was diluted with DCM (30 mL), washed successively with $NaHCO_3$ solution (15 mL) and saturated NaCl solution (15 mL), dried over anhydrous $Na_2SO_4$, concentrated under reduced pressure, and the concentrated solution was separated by silica gel column chromatography (eluent: DCM/EtOAc (v/v) = 1/1) to give a white solid (180 mg, 81%).

MS (ESI, pos.ion) m/z: 685.2 $[M+H]^+$.
$^1$H NMR (400 MHz, $CDCl_3$) $\delta$ (ppm): 7.88 (d, *J*= 8.3 Hz, 2H), 7.81 (d, *J*= 8.7 Hz, 2H), 7.68 (d, *J*= 8.0 Hz, 1H), 7.58 (dt, *J*= 17.2, 8.8 Hz, 4H), 7.00 (*d, J* = 8.6 Hz, 2H), 6.70 (d, *J*= 8.8 Hz, 2H), 6.25 (t, *J* = 74.2 Hz, 1H), 5.69 (dd, *J* = 13.0, 5.4 Hz, 1H), 5.20 (t, *J* = 4.6 Hz, 1H), 4.23 (td, *J* = 12.5, 4.1 Hz, 2H), 3.99 (t, *J* = 9.1 Hz, 1H), 3.75 (dt, *J* = 11.4, 8.0 Hz, 2H), 3.67 (s, 3H), 3.16 - 3.06 (m, 2H), 3.02 (d, *J*= 5.2 Hz, 2H), 2.55 (d, *J* = 14.3 Hz, 1H), 2.46 - 2.36 (m, 1H), 1.29 (t, *J* = 7.4 Hz, 3H).

**Step 2: Synthesis of (S)-3-(4-((2*S*,4*S*)-2-((difluoromethoxy)methyl)-4-(4-(trifluoromethyl) phenoxy)pyrrolidin-1-yl)benzoylamino)-3-(4-(ethylsulfonyl)phenyl)propionic acid**

To a solution of methyl (*S*)-3-(4-((2S,4S)-2-((difluoromethoxy)methyl)-4-(4-(trifluoromethyl)phenoxy)pyrrolidin-1-yl)benzoylamino)-3-(4-(ethylsulfonyl)phenyl)propionate (150 mg, 0.22 mmol) in MeOH (15 mL) were added LiOH (180 mg, 4.2 mmol) and $H_2O$ (3 mL) in turn. The mixture was reacted at room temperature for 18 h. HCl solution (2 mol/L) was added to the reaction solution to adjust the pH to about 5. The resulting mixture was concentrated under reduced pressure, and extracted with DCM (20 mL $\times$ 3). The organic phases were combined, washed with saturated NaCl (15 mL) solution, dried over anhydrous $Na_2SO_4$, and concentrated under reduced pressure. The concentrated solution was separated by silica gel column chromatography (eluent: DCM/MeOH (v/v) = 10/1) to give a pale yellow solid (100 mg, 68%).

MS (ESI, pos.ion) m/z: 671.1 $[M+H]^+$.
$^1$H NMR (400 MHz, $CDCl_3$) $\delta$ (ppm): 7.83 (d, *J* = 8.1 Hz, 2H), 7.74 (d, *J* = 8.4 Hz, 2H), 7.63 - 7.47 (m, 5H), 6.97 (d, *J* = 8.5 Hz, 2H), 6.64 (d, *J* = 8.6 Hz, 2H), 6.21 (t, *J* = 74.4 Hz, 1H), 5.68 (d, *J* = 7.3 Hz, 1H), 5.17 (s, 1H), 4.14 (dt, *J* = 14.1, 7.9 Hz, 2H), 3.95 (t, *J* = 9.3 Hz, 1H), 3.71 (dt, *J* = 11.5, 8.0 Hz, 2H), 3.13 - 2.99 (m, 4H), 2.51 (d, *J* = 14.4 Hz, 1H), 2.44 - 2.31 (m, 1H), 1.26 (t, *J* = 7.4 Hz, 3H).

**Example 93 4-((2*S*,4*S*)-2-((difluoromethoxy)methyl)-4-(4-trifluoromethyl)phenoxy)pyrrolidin -1-yl)-*N*-((*R*)-1-(4-(ethylsulfonyl)phenyl)-3-(hydroxyamino)-3-oxopropyl)benzamide**

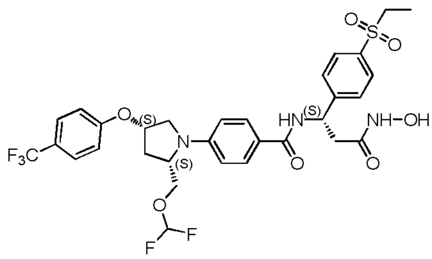

To a solution of $NH_2OH \cdot HCl$ (462 mg, 6.65 mmol) in MeOH (10 mL) was added KOH (745 mg, 13.28 mmol) at -10°C. The mixture was reacted for 0.5 h, then methyl (*S*)-3-(4-((2S,4S)-2-((difluoromethoxy)methyl)-4-(4-(trifluoromethylphenoxy)pyrrolidin-1-yl)benza mido)-3-(4-(ethylsulfonyl)phenyl)propionate (910 mg, 1.33 mmol)was added. The mixture was slowly returned to room temperature and reacted for 4 h. Dilute hydrochloric acid was added to adjust the pH of the solution to be weakly acidic. The resulting mixture was extracted with EtOAc, dried over anhydrous $Na_2SO_4$, filtered, concentrated under reduced pressure, and the concentrated solution was separated by silica gel column chromatography (eluent: DCM/MeOH (v/v) = 25/1) to give a white solid (0.40 g, 40%).

MS (ESI, pos.ion) m/z: 686 $[M+H]^+$.
$^1H$ NMR (400 MHz, $CD_3OD$) δ (ppm): 7.83 (d, *J* = 21.0 Hz, 5H), 7.63 (d, *J* = 8.2 Hz, 4H), 7.12 (d, *J* = 7.9 Hz, 2H), 6.71 (s, 2H), 6.40 (t, *J* = 75.2 Hz, 1H), 5.60 (s, 1H), 5.27 (s, 1H), 4.14 (t, *J* = 21.0 Hz, 2H), 3.95 (t, *J* = 9.0 Hz, 2H), 3.71 (s, 2H), 3.33 (d, *J* = 1.4 Hz, 1H), 3.16 (d, *J* = 5.9 Hz, 2H), 2.74 (s, 2H), 2.44 (s, 2H), 1.19 (s, 3H).

**Example 94 4-((2*S*,4*S*)-2-((difluoromethoxy)methyl)-4-(4-trifluoromethyl)phenoxy)pyrrolidin -1-yl)-N-((S)-1-(4-(ethylsulfonyl)phenyl)-3-(methylamino)-3-oxopropyl)benzamide**

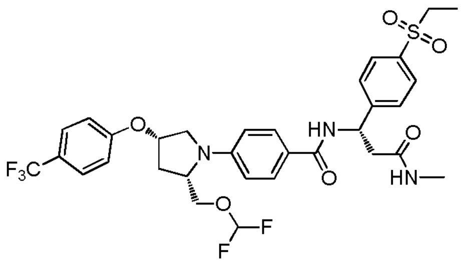

To a solution of (*S*)-3-(4-((2S,4S)-2-((difluoromethoxy)methyl)-4-(4-(trifluoromethyl) phenoxy)pyrrolidin-1-yl)benzoylamino)-3-(4-(ethylsulfonyl)phenyl)propionic acid (72 mg, 0.11 mmol), methylamine hydrochloride (14.4 mg, 0.21 mmol) and HATU (49 mg, 0.13 mmol) in DCM (25 mL) was added TEA (0.07 mL, 0.53 mmol). The mixture was reacted at room temperature for 12 h. The reaction was quenched by adding water. The resulting mixture was extracted with DCM (30 mL × 2). The organic phases were combined, dried over anhydrous $Na_2SO_4$, filtered, concentrated and separated by silica gel column chromatography (eluent: PE/EtOAc (v/v) = 1/1) to give a white solid (21 mg, 29%).

MS (ESI, pos.ion) m/z: 684 $[M+H]^+$.
$^1H$ NMR (400 MHz, $CDCl_3$) δ (ppm): 8.89 (d, *J* = 7.2 Hz, 1H), 7.83 (dd, *J* = 14.2, 8.5 Hz, 4H), 7.57 (dd, *J* = 33.3, 8.3 Hz, 4H), 7.00 (d, *J* = 8.5 Hz, 2H), 6.68 (d, *J* = 8.7 Hz, 2H), 6.20 (dd, *J* = 108.9, 39.6 Hz, 2H), 5.48 (d, *J* = 6.3 Hz, 1H), 5.19 (s, 1H), 4.32 - 4.12 (m, 2H), 3.99 (d, *J* = 8.9 Hz, 1H), 3.85 - 3.66 (m, 2H), 3.09 (dd, *J* = 14.8, 7.3 Hz, 2H), 2.88 (dd, *J* = 14.7, 4.7 Hz, 1H), 2.65 (dd, *J* = 11.9, 4.8 Hz, 4H), 2.53 (d, *J* = 14.4 Hz, 1H), 2.42 (d, *J* = 6.4 Hz, 1H), 1.28 (dd, *J* = 13.4, 6.6 Hz, 3H).

**Example 95 4-((2*S*,4*S*)-2-((difluoromethoxy)methyl)-4-(4-trifluoromethyl)phenoxy) pyrrolidin-1-yl)-*N*-((*S*)-3-(dimethylamino)-1-(4-(ethylsulfonyl)phenyl)-3-oxopropyl)benzamide**

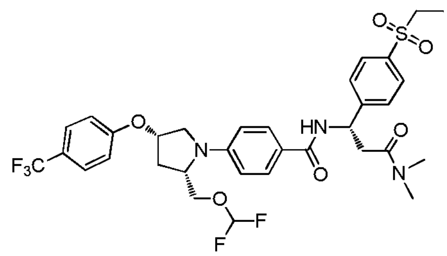

To a solution of (*S*)-3-(4-((2*S*,4*S*)-2-((difluoromethoxy)methyl)-4-(4-(trifluoromethyl) phenoxy)pyrrolidin-1-yl)benzoylamino)-3-(4-(ethylsulfonyl)phenyl)propionic acid (92 mg, 0.14 mmol), dimethylamine hydrochloride (22 mg, 0.27 mmol) and HATU (63 mg, 0.16 mmol) in DCM (25 mL) was added TEA (0.089 mL, 0.69 mmol). The mixture was reacted at room temperature for 12 h. The reaction was quenched by adding water. The resulting mixture was extracted with DCM (30 mL × 2). The organic phases were combined, dried over anhydrous $Na_2SO_4$, filtered, concentrated and separated by silica gel column chromatography (eluent: PE/EtOAc (v/v) = 1/1) to give a white solid (41 mg, 43%).

MS (ESI, pos.ion) m/z: 699 $[M+H]^+$.
$^1H$ NMR (400 MHz, $CDCl_3$) δ (ppm): 8.76 (d, *J* = 7.7 Hz, 1H), 7.85 (dd, *J* = 8.6, 2.1 Hz, 4H), 7.60 (dd, *J* = 8.1, 5.8 Hz, 4H), 7.00 (d, *J* = 8.6 Hz, 2H), 6.68 (d, *J* = 8.8 Hz, 2H), 6.25 (t, *J* = 74.3 Hz, 1H), 5.64 (dd, *J* = 8.0, 4.0 Hz, 1H), 5.19 (t, *J* = 4.6 Hz, 1H), 4.21 (dt, *J* = 11.1, 6.4 Hz, 2H), 3.98 (t, *J*= 9.3 Hz, 1H), 3.74 (dt, *J* = 11.4, 8.0 Hz, 2H), 3.16 - 3.02 (m, 3H), 2.97 - 2.87 (m, 4H), 2.85 (s, 3H), 2.53 (d, *J* = 14.4 Hz, 1H), 2.41 (td, *J* = 8.8, 4.3 Hz, 1H), 1.28 (dd, *J* = 8.6, 6.4 Hz, 3H).

**Example 96 methyl (*S*)-3-(4-(ethylsulfonyl)phenyl)-3-(4-((2*S*,4*S*)-2-((trifluoromethoxy) methyl)-4-(4- (trifluoromethyl)phenoxy)pyrrolidin-1-yl)benzamido)propionate**

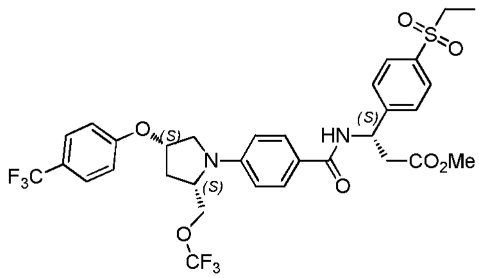

**Step** 1: **Synthesis of methyl (*S*)-3-amino-3-(4-(ethylsulfonyl)phenyl)propionate**

To a solution of benzyl (*S*)-(2-cyano-1-(4-(ethylsulfonyl)phenyl)ethyl)carbamate (750 mg, 2.01 mmol) in MeOH (5 mL) was slowly added a solution of 20% HCl in MeOH (10 mL) dropwise. The mixture was reacted at 80 °C for 12 h. The reaction solution was concentrated under reduced pressure. The concentrated solution was slowly added with $NaHCO_3$ solution (20 mL) and diluted with EtOAc (20 mL). The aqueous phase was extracted with EtOAc (20 mL). The combined organic phases were dried over anhydrous $Na_2SO_4$ and concentrated under reduced pressure. The concentrated solution was separated by silica gel column chromatography (eluent: DCM/MeOH (v/v) = 10/1) to give a white solid (282 mg, 52%).
MS (ESI, pos.ion) m/z: 272.1 $[M+H]^+$.

**Step 2: Synthesis of methyl (*S*)-3-(4-(ethylsulfonyl)phenyl)-3-(4-((2*S*,4*S*)-2-((trifluoromethoxy) methyl)-4-(4-(trifluoromethyl)phenoxy)pyrrolidin-1-yl)benzamido)propionate**

Methyl (*S*)-3-amino-3-(4-(ethylsulfonyl)phenyl)propionate (40 mg, 0.15 mmol), 4-((2*S*,4*S*)-2-((trifluoromethoxy)methyl)-4-(4-(trifluoromethyl)phenoxy)pyrrolidin-1-yl)benzoic acid (60 mg , 0.13 mmol), HATU (0.10 g, 0.26 mmol) and DIPEA (0.1 mL, 0.6 mmol) were dissolved in DCM (5 mL). The mixture was reacted at room temperature for 16 h. The reaction solution was poured into DCM (10 mL), and the resulting mixture was washed successively with HCl solution (0.5 mol/L, 20 mL), saturated $NaHCO_3$ solution (20 mL) and saturated NaCl solution (15 mL), dried over anhydrous

$Na_2SO_4$, filtered and concentrated under reduced pressure. The concentrated solution was separated by silica gel column chromatography (eluent: DCM/MeOH (v/v) = 15/1) to give a white solid product (70 mg, 75%).

MS (ESI, pos.ion) m/z: 703.2 $[M+H]^+$.
$^1H$ NMR (400 MHz, $CDCl_3$) δ (ppm): 7.86 (d, *J*= 8.3 Hz, 2H), 7.79 (d, *J*= 8.7 Hz, 2H), 7.68 (d, *J*= 7.4 Hz, 1H), 7.57 (dd, *J* = 18.7, 8.4 Hz, 4H), 6.97 (d, *J* = 8.6 Hz, 2H), 6.67 (d, *J* = 8.7 Hz, 2H), 5.66 (d, *J* = 7.7 Hz, 1H), 5.19 (t, *J* = 4.3 Hz, 1H), 4.30 - 4.21 (m, 2H), 4.11 (t, *J* = 9.5 Hz, 1H), 3.73 (dt, *J* = 11.4, 8.1 Hz, 2H), 3.65 (s, 3H), 3.09 (q, *J* = 7.4 Hz, 2H), 3.00 (d, *J* = 5.2 Hz, 2H), 2.54 (d, *J* = 14.4 Hz, 1H), 2.47 - 2.37 (m, 1H), 1.27 (t, *J* = 7.4 Hz, 3H).

**Example 97 methyl (*S*)-3-(4-((2*S*,4*S*)-2-((difluoromethoxy)methyl)-4-(4-(trifluoromethyl) phenoxy)pyrrolidin-1-yl)benzoylamino)-3-(4-(ethylsulfonyl)phenyl)propionate**

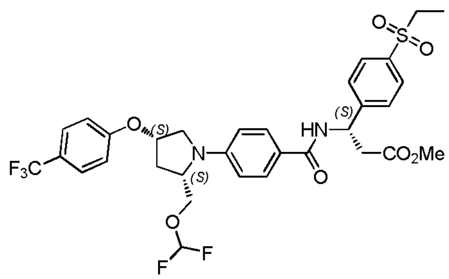

4-((2*S*,4*S*)-2-((Difluoromethoxy)methyl)-4-(4-(trifluoromethyl)phenoxy)pyrrolidin-1-y l)benzoic acid (160 mg, 0.37 mmol), methyl (*S*)-3-amino-3-(4-(ethylsulfonyl)phenyl)propanoate (88 mg, 0.32 mmol), HATU (190 mg, 0.48 mmol) and DIPEA (0.1 mL, 0.6 mmol) were dissolved in DCM (20 mL). The mixture was reacted at room temperature for 30 h. The reaction solution was poured into DCM (10 mL), and the resulting mixture was washed successively with HCl solution (15 mL, 0.5 mol/L), saturated $NaHCO_3$ solution (20 mL) and saturated NaCl solution (15 mL), dried over anhydrous $Na_2SO_4$, filtered and concentrated under reduced pressure. The concentrated solution was separated by silica gel column chromatography (eluent: DCM/EtOAc (v/v) = 1/1) to give a white solid (180 mg, 81%).

MS (ESI, pos.ion) m/z: 685.2 $[M+H]^+$.
$^1H$ NMR (400 MHz, $CDCl_3$) δ (ppm): 7.88 (d, *J*= 8.3 Hz, 2H), 7.81 (d, *J*= 8.7 Hz, 2H), 7.68 (d, *J*= 8.0 Hz, 1H), 7.58 (dt, *J*= 17.2, 8.8 Hz, 4H), 7.00 (d, *J* = 8.6 Hz, 2H), 6.70 (d, *J*= 8.8 Hz, 2H), 6.25 (t, *J* = 74.2 Hz, 1H), 5.69 (dd, *J* = 13.0, 5.4 Hz, 1H), 5.20 (t, *J* = 4.6 Hz, 1H), 4.23 (td, *J* = 12.5, 4.1 Hz, 2H), 3.99 (t, *J*= 9.1 Hz, 1H), 3.75 (dt, *J* = 11.4, 8.0 Hz, 2H), 3.67 (s, 3H), 3.16 - 3.06 (m, 2H), 3.02 (d, *J*= 5.2 Hz, 2H), 2.55 (d, *J* = 14.3 Hz, 1H), 2.46 - 2.36 (m, 1H), 1.29 (t, *J*= 7.4 Hz, 3H).

**Example 98 *N*-((*S*)-3-amino-1-(4-(ethylsulfonyl)phenyl)-3-oxopropyl)-4-((2*S*,4*S*)-2-((difluoromethoxy) methyl)-4-(4-(trifluoromethyl)phenoxy)pyrrolidin-1-yl)benzamide**

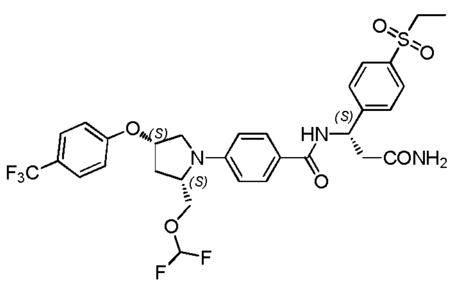

(*S*)-3-4-((2*S*,4*S*)-2-((Difluoromethoxy)methyl)-4-(4-(trifluoromethyl)phenoxy)pyrrolid in-1-yl)benzoylamino)-3-(4-(ethylsulfonyl)phenyl)propionic acid (50 mg, 0.075 mmol), $NH_4Cl$ (15 mg, 0.28 mmol), HATU (38 mg, 0.09 mmol) and DIPEA(0.2 mL, 1 mmol) were dissolved in DCM (5 mL). The mixture was reacted at room temperature for 18 h. The reaction solution was poured into DCM (10 mL), and the resulting mixture was washed successively with HCl solution

(0.5 mol/L, 20 mL), saturated $NaHCO_3$ solution (20 mL) and saturated NaCl solution (15 mL), dried over anhydrous $Na_2SO_4$, filtered and concentrated under reduced pressure. The concentrated solution was separated by silica gel column chromatography (eluent: DCM/EtOAc (v/v) = 1/1) to give a white solid product (20 mg, 40 %).

MS (ESI, pos.ion) m/z: 670.2 $[M+H]^+$.
$^1H$ NMR (400 MHz, $CDCl_3$) $\delta$ (ppm): 8.46 (d, *J* = 5.3 Hz, 1H), 7.79 (dd, *J* = 8.4, 2.2 Hz, 4H), 7.56 (dd, *J* = 18.9, 8.4 Hz, 4H), 6.97 (d, *J* = 8.5 Hz, 2H), 6.65 (d, *J* = 8.7 Hz, 2H), 6.22 (t, *J* = 74.4 Hz, 1H), 5.99 (s, 1H), 5.63 - 5.46 (m, 2H), 5.16 (d, *J* = 4.4 Hz, 1H), 4.18 (t, *J*= 8.5 Hz, 2H), 3.95 (t, *J* = 9.3 Hz, 1H), 3.71 (dt, *J* = 11.4, 7.9 Hz, 2H), 3.07 (q, *J* = 7.4 Hz, 2H), 2.83 (ddd, *J* = 41.2, 15.0, 4.7 Hz, 2H), 2.50 (d, *J* = 14.2 Hz, 1H), 2.37 (dt, *J* = 16.1, 7.3 Hz, 1H), 1.25 (t, *J* = 7.4 Hz, 3H).

**Example 99 methyl 2-((*S*)-3-(4-((2*S*,4*S*)-2-((difluoromethoxy)methyl)-4-(4-(trifluoromethyl) phenoxy) pyrrolidin-1-yl)benzoylamino)-3-(4-(ethylsulfonyl)phenyl)propionamido)acetate**

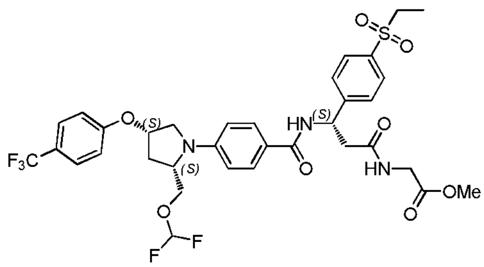

**Step 1: Synthesis of (*S*)-3-(((*tert*-butoxy)carbonyl)amino)-3-(4-(ethylsulfonyl)phenyl)propionic** acid

To a solution of methyl (*S*)-3-(((*tert*-butoxy)carbonyl)amino)-3-(4-(ethylsulfonyl)phenyl)propanoate (5.00 g, 13.00 mmol) in MeOH (15 mL) were added LiOH (1.00 g, 42.00 mmol) and $H_2O$ (8 mL). The mixture was reacted at room temperature for 12 h. Dilute hydrochloric acid was added to adjust the pH of the solution to be acidic. The resulting mixture was extracted with EtOAc (5 mL $\times$ 3). The organic phases were combined, dried over anhydrous $Na_2SO_4$, filtered, concentrated and separated by silica gel column chromatography (eluent: PE/EtOAc (v/v) = 1/1) to give a white solid (4.04 g, 87%).
MS (ESI, pos.ion) m/z: 358.1 $[M+H]^+$.

**Step 2: Synthesis of methyl (*S*)-2-(3-(((*tert*-butoxy)carbonyl)amino) -3-(4-(ethylsulfonyl)phenyl)propionamido)acetate**

To a solution of (*S*)-3-(((*tert*-butoxy)carbonyl)amino)-3-(4-(ethylsulfonyl) phenyl)propionic acid (0.50 g, 1.40 mmol), HOBT (0.20 g, 1.50 mmol), EDCI (0.30 g, 1.60 mmol) and methyl 2-aminoacetate (0.12 g, 1.30 mmol) in DCM (10 mL) was added DIPEA (0.5 mL, 3.00 mmol). The mixture was reacted at room temperature for 12 h. The reaction was quenched by adding saturated $NH_4Cl$ solution (15 mL). The resulting mixture was extracted with DCM (10 mL $\times$ 2). The organic phases were combined, dried over anhydrous $Na_2SO_4$, filtered, concentrated and separated by silica gel column chromatography (eluent: DCM/EtOAc (v/v) = 5/1) to give a white solid (0.39 g, 65%).
MS (ESI, pos.ion) m/z: 429.5 $[M+H]^+$.

**Step 3: Synthesis of methyl (*S*)-2-(3-amino-3-(4-(ethylsulfonyl)phenyl)propionamido)acetate**

To a solution of methyl (*S*)-2-(3-(((*tert*-butoxy)carbonyl)amino)-3-(4-(ethylsulfonyl) phenyl)propionamido)acetate (140 mg, 0.32 mmol) in DCM (3 mL) was added TFA (1.2 mL, 3.2 mmol). The mixture was reacted at room temperature for 12 h. Saturated $Na_2CO_3$ solution (10 ml) was added to quench the reaction, and the resulting mixture was extracted with DCM (10 mL $\times$ 2). The organic phases were combined, dried over anhydrous $Na_2SO_4$, filtered and concentrated to give a white solid (94 mg, 90%).
MS (ESI, pos.ion) m/z: 329.3 $[M+H]^+$.

**Step 4: Synthesis of methyl 2-((*S*)-3-(4-((2*S*,4*S*)-2-((difluoromethoxy)methyl)-4-(4-(trifluoromethyl)phenoxy)pyrrolidin-1-)benzoylamino)-3-(4-(ethylsulfonyl)phenyl)propionami do)acetate**

4-((2*S*,4*S*)-2-((Difluoromethoxy)methyl)-4-(4-(trifluoromethyl)phenoxy)pyrrolidin-1-) benzoic acid (82 mg, 0.19 mmol), methyl (*S*)-2-(3-amino-3-(4-(ethylsulfonyl)phenyl) propionamido)acetate (68 mg, 0.21 mmol) and HATU (87 mg, 0.23 mmol) were dissolved in DCM (25 mL), then TEA (23 mg, 0.23 mmol) was added. The mixture was reacted at room temperature for 12 h. The reaction was quenched by adding saturated $NH_4Cl$ solution (15 mL). The resulting mixture was extracted with DCM (10 mL × 2). The organic phases were combined, dried over anhydrous $Na_2SO_4$, filtered, concentrated and separated by silica gel column chromatography (eluent: DCM/EtOAc (v/v) = 5/1) to give a white solid (99 mg, 70%).

MS (ESI, pos.ion) m/z: 742.5 $[M+H]^+$.
$^1H$ NMR (400 MHz, $CDCl_3$) δ (ppm): 8.53 (d, *J*= 7.5 Hz, 1H), 7.81 (d, *J*= 8.3 Hz, 4H), 7.59 (d, *J*= 8.6 Hz, 2H), 7.53 (d, *J* = 8.3 Hz, 2H), 6.98 (d, *J* = 8.6 Hz, 2H), 6.66 (d, *J* = 8.8 Hz, 2H), 6.48 (s, 1H), 6.23 (t, *J* = 74.3 Hz, 1H), 5.54 (dd, *J* = 12.1, 5.0 Hz, 1H), 5.17 (t, *J* = 4.6 Hz, 1H), 4.27 - 4.15 (m, 2H), 4.02 - 3.82 (m, 3H), 3.76 (d, *J* = 11.3 Hz, 1H), 3.70 (s, 3H), 3.07 (q, *J*= 7.4 Hz, 2H), 2.86 (ddd, *J*= 60.0, 15.0, 5.0 Hz, 2H), 2.49 (t, *J* = 17.7 Hz, 1H), 2.40 (dd, *J* = 12.9, 7.4 Hz, 1H), 1.26 (t, *J* = 7.4 Hz, 3H).

**Example 100 2-((*S*)-3-(4-((2*S*,4*S*)-2-((difluoromethoxy)methyl)-4-(4-(trifluoromethyl) phenoxy)pyrrolidin-1-)benzoylamino)-3-(4-(ethylsulfonyl)phenyl)propionamido)acetic acid**

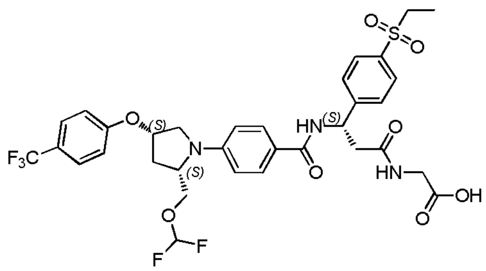

To a solution of methyl 2-((*S*)-3-(4-((2*S*,4,*S*)-2-((difluoromethoxy)methyl)-4-(4-(trifluoromethyl)phenoxy)pyrrolidin-1-)benzoylamino)-3-(4-(ethylsulfonyl)phenyl)propionamido)a cetate (80 mg, 0.11 mmol) in MeOH (5 mL) were added LiOH (10 mg, 0.42 mmol) and $H_2O$ (2 mL). The mixture was reacted at room temperature for 12 h. Dilute hydrochloric acid was added to adjust the pH of the solution to be acidic. The resulting mixture was extracted with EtOAc (5 mL × 3). The organic phases were combined, dried over anhydrous $Na_2SO_4$, filtered, concentrated and separated by silica gel column chromatography (eluent: PE/EtOAc (v/v) = 1/1) to give a white solid (55 mg, 71%).

MS (ESI, pos.ion) m/z: 728.3 $[M+H]^+$.
$^1H$ NMR (400 MHz, $CD_3OD$) δ (ppm): 7.85 (d, *J* = 8.3 Hz, 2H), 7.78 (d, *J* = 8.7 Hz, 2H), 7.66 (d, *J* = 8.3 Hz, 2H), 7.61 (d, *J* = 8.6 Hz, 2H), 7.11 (d, *J* = 8.6 Hz, 2H), 6.71 (d, *J* = 8.8 Hz, 2H), 6.38 (t, *J* = 75.1 Hz, 1H), 5.62 (d, *J* = 6.5 Hz, 1H), 5.28 (s, 1H), 4.22 (dd, *J* = 8.9, 4.4 Hz, 1H), 3.95 (d, *J* = 9.5 Hz, 1H), 3.87 - 3.61 (m, 5H), 3.32 (dd, *J* = 9.3, 7.7 Hz, 2H), 3.16 (q, *J* = 7.4 Hz, 2H), 2.88 (t, *J* = 7.2 Hz, 2H), 2.44 (s, 2H), 1.24 (t, *J* = 7.1 Hz, 3H).

**Example 101 4-((2*S*,4*S*)-2-((difluoromethoxy)methyl)-4-(4-trifluoromethyl)phenoxy) pyrrolidin-1-yl)-*N*-((*R*)-2-(2-(dimethylamino)ethoxy)-1-(4-(ethylsulfonyl)phenyl)ethyl) benzamide**

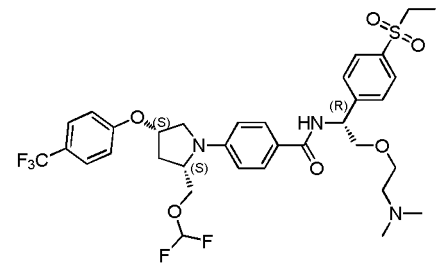

**Step 1: Synthesis of 4-((2*S*,4*S*)-2-((difluoromethoxy)methyl)-4-(4-(trifluoromethyl)phenoxy) pyrrolidin-1-yl)-*N*-((*R*)-1-(4-(ethylsulfonyl)phenyl)-2-hydroxyethyl)benzamide**

HATU (458 mg, 1.20 mmol), (R)-2-amino-2-(4-(ethylsulfonyl)phenyl)ethanol (233 mg, 1.01 mmol), 4-((2*S*,4*S*)-2-((difluoromethoxy)methyl)-4-(4-(trifluoromethyl)phenoxy) pyrrolidin-1-yl)benzoic acid (400 mg, 0.93 mmol) and TEA(187 mg, 1.85 mmol) were successively added to DCM (8 mL) and the mixture was reacted at room temperature for 22 h. The reaction solution was diluted with DCM (40 mL), washed successively with HCl solution (15 mL, 0.5 mol/L) and saturated NaCl solution (15 mL), dried over anhydrous $Na_2SO_4$, concentrated under reduced pressure, and the concentrated solution was separated by silica gel column chromatography (eluent: DCM/EtOAc (v/v) = 1/1) to give a pale yellow solid (430 mg, 72%).
MS (ESI, pos.ion) m/z: 643.2 $[M+H]^+$.

**Step 2: Synthesis of 4-((2*S*,4*S*)-2-((difluoromethoxy)methyl)-4-(4-trifluoromethyl) phenoxy)pyrrolidin-1-yl)-*N*-((*R*)-2-(2-(dimethylamino)ethoxy)-1-(4-(ethylsulfonyl)phenyl)ethyl )benzamide**

To a solution of 4-((2*S*,4*S*)-2-((difluoromethoxy)methyl)-4-(4-(trifluoromethyl) phenoxy)pyrrolidin-1-yl)-*N*((*R*)-1-(4-(ethylsulfonyl)phenyl)-2-hydroxyethyl)benzamide (430 mg, 0.67 mmol) and 2-bromo-*N,N*-dimethylethylamine hydrobromide (171 mg, 0.73 mmol) in DMF (3 mL) was added NaH (53 mg, 1.32 mmol, 60%) at -10°C. After the addition was completed, the reaction was carried out at room temperature for 2 h. The reaction was quenched by adding $H_2O$ (15 mL). The resulting mixture was extracted with EtOAc (40 mL). The organic phase was washed with $H_2O$ (15 mL) and saturated NaCl solution (15 mL), dried over anhydrous $Na_2SO_4$, concentrated under reduced pressure, and the concentrated solution was separated by silica gel column chromatography (eluent: DCM/MeOH (v/v) = 10/1) to give a pale yellow solid (70 mg, 15%).

MS (ESI, pos.ion) m/z: 714.3 $[M+H]^+$.
$^1$H NMR (400 MHz, $CDCl_3$) δ (ppm): 7.84 (d, *J* = 8.3 Hz, 4H), 7.60 (t, *J* = 9.0 Hz, 4H), 6.97 (d, *J* = 8.5 Hz, 2H), 6.65 (d, *J* = 8.7 Hz, 2H), 6.22 (t, *J* = 74.3 Hz, 1H), 5.37 (d, *J* = 5.9 Hz, 1H), 5.17 (t, *J* = 4.6 Hz, 1H), 4.24 - 4.16 (m, 2H), 3.96 (t, *J* = 9.1 Hz, 1H), 3.86 - 3.80 (m, 2H), 3.76 (d, *J*= 11.3 Hz, 1H), 3.70 (dd, *J* = 11.3, 4.6 Hz, 1H), 3.65 - 3.57 (m, 2H), 3.07 (q, *J* = 7.4 Hz, 2H), 2.56 - 2.50 (m, 3H), 2.42 - 2.36 (m, 1H), 2.28 (s, 6H), 1.27 (t, *J*= 7.4 Hz, 3H).

**Example 102 (*R*)-2-(4-((2*S*,4*S*)-2-((difluoromethoxy)methyl)-4-(4-(trifluoromethyl) phenoxy)pyrrolidin-1-yl)benzoylamino)-2-(4-(ethylsulfonyl)phenyl)acetic acid**

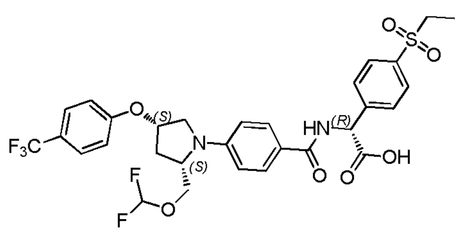

To a solution of 4-((2*S*,4*S*)-2-((difluoromethoxy)methyl)-4-(4-(trifluoromethyl) phenoxy((*R*)-1-(4-(ethylsulfonyl)phenyl)-2-hydroxyethyl)benzamide (300 mg, 0.47 mmol) and $RuCl_3$ (44 mg, 0.21 mmol) in ACN (10 mL) were added $NaIO_4$ (300 mg, 1.39 mmol) and $H_2O$ (10 mL) in turn. The mixture was reacted at room temperature for 22 h. HCl solution (15 mL, 0.1mol/L) was added to the reaction solution. The mixture was extracted with DCM (20 mL × 3), and the organic

phases were combined, washed with saturated NaCl solution (15 mL), dried over anhydrous $Na_2SO_4$, concentrated under reduced pressure. The concentrated solution was separated by silica gel column chromatography (eluent: DCM/EtOAc (v/v) = 1/1) to give a yellow solid (80 mg, 26%).

MS (ESI, pos.ion) m/z: 657.2 $[M+H]^+$.
$^1H$ NMR (400 MHz, $CD_3OD$) δ (ppm): 7.98 (d, *J* = 8.4 Hz, 2H), 7.89 (d, *J* = 8.3 Hz, 2H), 7.69 (d, *J* = 8.3 Hz, 2H), 7.60 (dd, *J* = 16.8, 7.9 Hz, 4H), 7.25 (d, *J* = 8.6 Hz, 2H), 6.30 (t, *J* = 74.8, 1H), 5.31 (dt, *J* = 12.6, 7.2 Hz, 2H), 4.71 - 4.65 (m, 1H), 3.99 (dd, *J* = 11.0, 3.8 Hz, 1H), 3.93 (dd, *J* = 7.6, 4.6 Hz, 3H), 3.19 (q, *J* = 7.4 Hz, 2H), 2.97 (dt, *J* = 13.5, 7.9 Hz, 1H), 2.21 (dt, *J* = 13.6, 6.9 Hz, 1H), 1.21 (t, *J* = 7.4 Hz, 3H).

**Example 103 4-((2*S*,4*S*)-2-((difluoromethoxy)methyl)-4-(4-trifluoromethyl)phenoxy) pyrrolidin-1-yl) -*N*-((*R*)-2-(dimethylamino)-1-(4-(ethylsulfonyl)phenyl)ethyl)benzamide**

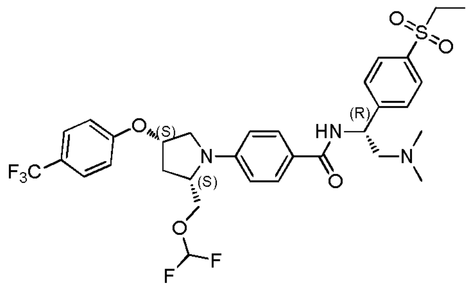

**Step 1: Synthesis of (*R*)-2-(((*tert*-butoxy)carbonyl)amino)-2-(4-(ethylsulfonyl)phenyl)ethyl methanesulfonate**

Under nitrogen protection, to a solution of TEA (1.0 mL, 7.19 mmol) and *tert*-butyl (*R*)-(1-(4-(ethylsulfonyl)phenyl)-2-hydroxyethyl)carbamate (1.20 g, 3.46 mmol) in DCM (16 mL) was added MsCl (0.42 mL, 5.46 mmol). The mixture was reacted at room temperature for 16 h. The reaction solution was diluted with DCM (20 mL), washed successively with $NaHCO_3$ solution (15 mL) and saturated NaCl solution (15 mL), dried over anhydrous $Na_2SO_4$, concentrated under reduced pressure, and the concentrated solution was separated by silica gel column chromatography (eluent: DCM/EtOAc (v/v) = 5/1) to give a pale yellow solid (1.40 g, 94%).
MS (ESI, pos.ion) m/z: 352.1 $[M-56+H]^+$.

**Step 2: Synthesis of *tert*-butyl (*R*)-(2-(dimethylamino)-1-(4-(ethylsulfonyl) phenyl)ethyl)carbamate**

To a solution of dimethylamine in THF (16.0 mL, 32.0 mmol, 2 mol/L) was added (*R*)-2-(((*tert*-butoxy)carbonyl)amino)-2-(4-(ethylsulfonyl)phenyl)ethyl methanesulfonate (1.40 g, 3.44 mmol). The mixture was reacted at 70 °C for 3 h. The reaction solution was cooled to room temperature, concentrated under reduced pressure, and the concentrated solution was separated by silica gel column chromatography (eluent: DCM/MeOH (v/v) = 10/1) to give a pale yellow solid (134 mg, 11%).
MS (ESI, pos.ion) m/z: 357.2 $[M+H]^+$.

**Step 3: Synthesis of (*R*)-1-(4-(ethylsulfonyl)phenyl)-$N^2$,$N^2$-dimethylethane-1,2-diamine dihydrochloride**

To a solution of *tert*-butyl (*R*)-(2-(dimethylamino)-1-(4-(ethylsulfonyl)phenyl) ethyl)carbamate (130 mg, 0.36 mmol) in DCM (4 mL) was added a solution of HCl in 1,4-dioxane (1.0 mL, 4.0 mmol, 4 mol/L). The mixture was reacted at room temperature for 4 h. The reaction solution was concentrated under reduced pressure to give a white solid (120 mg, 100%).
MS (ESI, pos.ion) m/z: 257.2 $[M+H]^+$.

**Step 4: Synthesis of 4-((2*S*,4*S*)-2-((difluoromethoxy)methyl)-4-(4-trifluoromethyl)phenoxy) pyrrolidin-1-yl)-*N*-((*R*)-2-(dimethylamino)-1-(4-(ethylsulfonyl)phenyl)ethyl)benzamide**

HATU (158 mg, 0.42 mmol), (*R*)-1-(4-(ethylsulfonyl)phenyl)-$N^2$,$N^2$-dimethylethane-1,2-diamine dihydrochloride (120 mg, 0.36 mmol), 4-((2*S*,4*S*)-2-((difluoromethoxy)methyl)-4-(4-(trifluoromethyl) phenoxy)pyrrolidin-1-yl)benzoic acid

(120 mg, 0.28 mmol) and TEA (112 mg, 1.11 mmol) were successively added to DCM (6 mL), and the mixture was reacted at room temperature for 16 h. The reaction solution was diluted with DCM (30 mL), washed successively with $NaHCO_3$ solution (15 mL) and saturated NaCl solution (15 mL), dried over anhydrous $Na_2SO_4$, concentrated under reduced pressure, and the concentrated solution was separated by silica gel column chromatography (eluent: DCM/MeOH (v/v) = 10/1) to give a pale yellow solid (80 mg, 43%).

MS (ESI, pos.ion) m/z: 670.2 $[M+H]^+$.
$^1H$ NMR (400 MHz, $CDCl_3$) $\delta$ (ppm): 7.85 (d, *J*= 8.3 Hz, 2H), 7.80 (d, *J*= 8.7 Hz, 2H), 7.59 (d, *J*= 8.6 Hz, 2H), 7.54 (d, *J* = 8.2 Hz, 2H), 7.36 (s, 1H), 6.97 (d, *J* = 8.6 Hz, 2H), 6.67 (d, *J* = 8.8 Hz, 2H), 6.23 (t, *J* = 74.3 Hz, 1H), 5.18 (t, *J* = 4.6 Hz, 1H), 5.05 - 4.98 (m, 1H), 4.25 - 4.16 (m, 2H), 3.96 (t, *J*= 9.6 Hz, 1H), 3.77 (d, *J*= 11.3 Hz, 1H), 3.70 (dd, *J*= 11.4, 4.6 Hz, 1H), 3.08 (q, *J*= 7.4 Hz, 2H), 2.72 - 2.64 (m, 1H), 2.55 - 2.49 (m, 2H), 2.43 - 2.34 (m, 1H), 2.30 (s, 6H), 1.28 (t, *J* = 7.4 Hz, 3H).

**Example 104 4-((2*S*,4*S*)-2-((difluoromethoxy)methyl)-4-(4-trifluoromethyl)phenoxy)pyrrolidin -1-yl)-*N*-((*R*)-1-(4-(ethylsulfonyl)phenyl)-2-(2-methoxyethoxy)ethyl)benzamide**

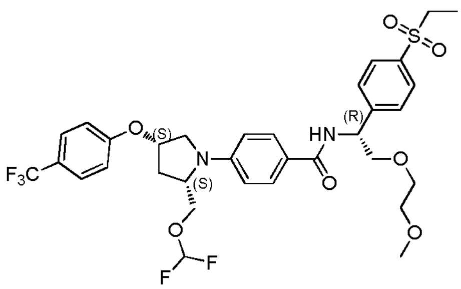

To a solution of 4-((2*S*,4*S*)-2-((difluoromethoxy)methyl)-4-(4-(trifluoromethyl) phenoxy((*R*)-1-(4-(ethylsulfonyl)phenyl)-2-hydroxyethyl)benzamide (120 mg, 0.19 mmol) in DMF (3 mL) was added *t*-BuOK (90 mg, 0.48 mmol). After 20 min, 1-bromo-2-methoxyethane (1.0 mL) was added. The mixture was reacted at room temperature for 20 h. The reaction solution was diluted with EtOAc (30 mL), washed successively with $H_2O$ (10 mL × 2) and saturated NaCl solution (15 mL), dried over anhydrous $Na_2SO_4$, concentrated under reduced pressure, and the concentrated solution was separated by silica gel column chromatography (eluent: DCM/EtOAc (v/v) = 5/1) to give a pale yellow solid (30 mg, 23%).

MS (ESI, pos.ion) m/z: 701.2 $[M+H]^+$.
$^1H$ NMR (400 MHz, $CDCl_3$) $\delta$ (ppm): 7.89 (d, *J* = 7.8 Hz, 2H), 7.84 (d, *J* = 8.2 Hz, 2H), 7.64 (s, 4H), 7.02 (d, *J* = 8.1 Hz, 2H), 6.70 (d, *J* = 8.3 Hz, 2H), 6.27 (t, *J* = 74.2 Hz, 1H), 5.40 (s, 1H), 5.22 (s, 1H), 4.30 - 4.19 (m, 2H), 4.00 (t, *J* = 9.2 Hz, 1H), 3.92 (s, 2H), 3.76 (dd, *J* = 15.2, 11.3 Hz, 2H), 3.70 (s, 2H), 3.59 (s, 2H), 3.42 (s, 3H), 3.12 (q, *J* = 7.4 Hz, 2H), 2.56 (d, *J* = 14.2 Hz, 1H), 2.48 - 2.38 (m, 1H), 1.31 (t, *J* = 7.4 Hz, 3H).

**Example 105 (*S*)-4-(4-((2*S*,4*S*)-2-((difluoromethoxy)methyl)-4-(4-(trifluoromethyl)phenoxy) pyrrolidin-1-yl)benzoylamino)-4-(4-(ethylsulfonyl)phenyl)butyric acid**

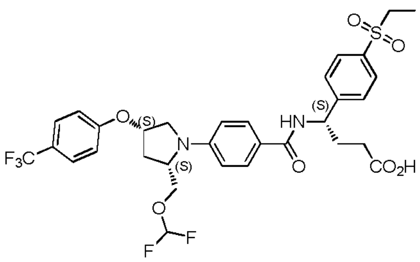

**Step 1: Synthesis of methyl (*S*)-4-(4-((2*S*,4*S*)-2-((difluoromethoxy)methyl) -4-(4-(trifluoromethyl)phenoxy)pyrrolidin-1-yl)benzoylamino)-4-(4-(ethylsulfonyl)phenyl) butyrate**

EDCI (66 mg, 0.34 mmol), HOBT (46 mg, 0.34 mmol), methyl (*S*)-4-amino-4-(4-(ethylsulfonyl)phenyl)butyrate (72 mg, 0.25 mmol), 4-((2*S*,4*S*)-2-((difluoromethoxy)methyl)-4-(4-(trifluoromethyl)phenoxy)pyrrolidin-1-yl)benzoic acid (100 mg, 0.23 mmol) and TEA (46 mg, 0.45 mmol) were successively added to DCM (4 mL) and the mixture was reacted at room temperature for 14 h. The reaction solution was diluted with DCM (30 mL), washed successively with $NaHCO_3$ solution (15 mL) and saturated NaCl solution (15 mL), dried over anhydrous $Na_2SO_4$, concentrated under reduced pressure, and the concentrated solution was separated by silica gel column chromatography (eluent: DCM/EtOAc (v/v) = 2/1) to give a white solid (110 mg, 68%).
MS (ESI, pos.ion) m/z: 699.2 $[M+H]^+$.

**Step 2: Synthesis of (*S*)-4-(4-((2*S*,4*S*)-2-((difluoromethoxy)methyl)-4-(4-(trifluoromethyl) phenoxy)pyrrolidin-1-yl)benzoylamino)-4-(4-(ethylsulfonyl)phenyl)butyric acid**

To a solution of methyl (*S*)-4-(4-((2*S*,4,*S*)-2-((difluoromethoxy)methyl)-4-(4-(trifluoromethyl)phenoxy)pyrrolidin-1-yl)benzoylamino)-4-(4-(ethylsulfonyl)phenyl)butyrate (80 mg, 0.11 mmol) in MeOH (3 mL) and THF (3 mL) was added a solution of $LiOH{\cdot}H_2O$ (120 mg, 2.86 mol) in $H_2O$ (3 mL). The mixture was reacted at room temperature for 12 h. The reaction solution was concentrated under reduced pressure, and HCl solution (1 mol/L) was added to the concentrated solution to adjust the pH to about 4. The resulting mixture was extracted with EtOAc (20 mL $\times$ 2), and the organic phases were combined, washed with saturated NaCl (15 mL) solution, dried over anhydrous $Na_2SO_4$, concentrated under reduced pressure. The concentrated solution was separated by silica gel column chromatography (eluent: DCM/MeOH (v/v) = 10/1) to give a white solid (50 mg, 64%).

MS (ESI, pos.ion) m/z: 685.1 $[M+H]^+$.
$^1H$ NMR (400 MHz, $CDCl_3$) $\delta$ (ppm): 7.85 (d, *J* = 8.2 Hz, 2H), 7.72 (d, *J* = 8.7 Hz, 2H), 7.58 (d, *J* = 8.6 Hz, 2H), 7.54 (d, *J* = 8.2 Hz, 2H), 7.10 (d, *J* = 7.4 Hz, 1H), 6.96 (d, *J* = 8.6 Hz, 2H), 6.61 (d, *J* = 8.7 Hz, 2H), 6.21 (t, *J* = 74.3 Hz, 1H), 5.25 (dd, *J* = 13.5, 7.7 Hz, 1H), 5.16 (t, *J* = 4.6 Hz, 1H), 4.21 - 4.12 (m, 2H), 3.94 (t, *J* = 9.7 Hz, 1H), 3.73 (d, *J* = 11.4 Hz, 1H), 3.67 (dd, *J* = 11.4, 4.6 Hz, 1H), 3.08 (q, *J* = 7.4 Hz, 2H), 2.53-2.48 (m, 3H), 2.42 - 2.34 (m, 1H), 2.25 - 2.16 (m, 2H), 1.28 (d, *J* = 7.4 Hz, 3H).

**Example 106 (*S*)-5-(4-((2*S*,4*S*)-2-((difluoromethoxy)methyl)-4-(4-(trifluoromethyl)phenoxy) pyrrolidin-1-yl)benzoylamino)-5-(4-(ethylsulfonyl)phenyl)valeric acid**

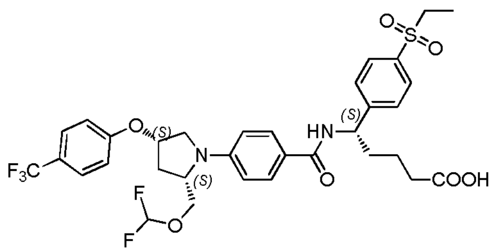

**Step 1: Synthesis of 4-((2*S*,4*S*)-2-((difluoromethoxy)methyl)-4-(4-(trifluoromethyl)phenoxy) pyrrolidin-1-yl)-*N*-((*S*)-1-(4-(ethylsulfonyl)phenyl)-3-hydroxypropyl)benzamide**

(*S*)-3-Amino-3-(4-(ethylsulfonyl)phenyl)propan-1-ol (29 mg, 0.12 mmol), 4-((2*S*,4*S*)-2-((difluoromethoxy)methyl)-4-(4-trifluoromethyl)phenoxy)pyrrolidin-1-yl)benzoic acid (40 mg, 0.09 mmol), EDCI (26 mg, 0.14 mmol), HOBT (18 mg, 0.13 mmol) and TEA (28 mg, 0.28 mmol) were dissolved in DCM (3 mL), and the mixture was stirred at room temperature for 18 h. The reaction solution was concentrated under reduced pressure, and the crude product was separated by silica gel column chromatography (eluent: DCM/MeOH (v/v) = 20/1) to give a white solid (49 mg, 80%).
MS (ESI, pos.ion) m/z: 657.3 $[M+H]^+$.

**Step 2: Synthesis of 4-((2*S*,4*S*)-2-((difluoromethoxy)methyl)-4-(4-(trifluoromethyl) phenoxy)pyrrolidin-1-yl)-*N*-((*S*)-1-(4-(ethylsulfonyl)phenyl)-3-oxopropyl)benzamide**

To a solution of 4-((2*S*,4*S*)-2-((difluoromethoxy)methyl)-4-(4-(trifluoromethyl)phenoxy)pyrrolidin-1-yl)-*N*-((*S*)-1-(4-(ethylsulfonyl)phenyl)-3-hydroxypropyl) benzamide (500 mg, 0.76 mmol) in DCM (10 mL) was slowly added DMP (600 mg, 1.40 mmol). The mixture was reacted at room temperature for 5 h. The reaction solution was filtered, and $Na_2S_2O_3$ solution (25 mL) was added to the filtrate under an ice bath, then the resulting mixture was extracted with DCM (25 mL × 2). The organic phase was dried over anhydrous $Na_2SO_4$, concentrated under reduced pressure. The concentrated solution was separated by silica gel column chromatography (eluent: DCM/EtOAc (v/v) = 10/1) to give a white solid (150 mg, 30%).
MS (ESI, pos.ion) m/z: 655.1 $[M+H]^+$.

**Step 3: Synthesis of methyl (*S*,*E*)-5-(4-((2*S*,4*S*)-2-((difluoromethoxy)methyl)-4-(4-(trifluoromethyl)phenoxy)pyrrolidin-1-yl)benzoylamino)-5-(4-(ethylsulfonyl)phenyl)pent-2-en oate**

To a solution of 4-((2*S*,4*S*)-2-((difluoromethoxy)methyl)-4-(4-(trifluoromethyl) phenoxy)pyrrolidin-1-yl)-N-((*S*)-1-(4-(ethylsulfonyl)phenyl)-3-oxypropyl)benzamide (152 mg, 0.23 mmol) in anhydrous THF (10 mL) was added methyl 2-(triphenylphosphoranylidene)acetate (200 mg, 0.60 mmol) under anhydrous and anoxic conditions at -20°C. The mixture was reacted at -20°C for 3 h. Then the mixture was slowly warmed to room temperature and the reaction was continued for 14 h. The reaction solution was slowly added with $NaHCO_3$ solution (20 mL) and diluted with DCM (50 mL). The aqueous phase was extracted with DCM (20 mL). The combined organic phases were dried over anhydrous $Na_2SO_4$, filtered and concentrated under reduced pressure. The concentrated solution was separated by silica gel column chromatography (eluent: PE/EtOAc (v/v) = 10/1) to give a white solid (120 mg, 73%).
MS (ESI, pos.ion) m/z: 711.1 $[M+H]^+$.

**Step 3: Synthesis of methyl (*S*)-5-(4-((2*S*,4*S*)-2-((difluoromethoxy) methyl)-4-(4-(trifluoromethyl) phenoxy)pyrrolidin-1-yl)benzoylamino)-5-(4-(ethylsulfonyl) phenyl)valerate**

To a solution of methyl (*S*,*E*)-5-(4-((2*S*,4*S*)-2-((difluoromethoxy)methyl)-4-(4-(trifluoromethyl)phenoxy)pyrrolidin-1-yl)benzoylamino)-5-(4-(ethylsulfonyl)phenyl)pent-2-enoate (120 mg, 0.17 mmol) in MeOH (20 mL) was added 10% palladium on carbon (100 mg). The mixture was reacted at room temperature under hydrogen protection. The reaction solution was filtered through a celite pad. The filtrate was concentrated under reduced pressure, dried *in vacuo,* and the concentrated solution was separated by silica gel column chromatography (eluent: PE/EtOAc (v/v) = 2/1) to give a white solid (60 mg, 50%).
MS (ESI, pos.ion) m/z: 713.3 $[M+1]^+$.

**Step 4: Synthesis of (*S*)-5-(4-((2*S*,4*S*)-2-((difluoromethoxy)methyl)-4-(4-(trifluoromethyl) phenoxy)pyrrolidin-1-yl)benzoylamino)-5-(4-(ethylsulfonyl)phenyl)valeric acid**

To a solution of methyl (*S*)-5-(4-((2*S*,4*S*)-2-((difluoromethoxy)methyl)-4-(4 -(trifluoromethyl)phenoxy)pyrrolidin-1-yl)benzoylamino)-5-(4-(ethylsulfonyl)phenyl)valerate (60 mg, 0.08 mmol) in MeOH (5 mL) were added $LiOH{\cdot}H_2O$ (30 mg, 0.70 mmol) and $H_2O$ (3 mL) in turn. The mixture was reacted at room temperature for 10 h. HCl solution (15 mL, 0.1mol/L) was added to the reaction solution. The mixture was extracted with DCM (20 mL × 3). The organic phases were combined, washed with saturated NaCl solution (15 mL), dried over anhydrous $Na_2SO_4$, concentrated under reduced pressure, and the concentrated solution was separated by silica gel column chromatography (eluent: DCM/MeOH (v/v) = 10/1) to give a white solid (30 mg, 51%).

MS (ESI, pos.ion) m/z: 699.2 $[M+H]^+$.
$^1$H NMR (400 MHz, $CDCl_3$) $\delta$ (ppm): 7.83 (d, *J*= 8.1 Hz, 2H), 7.74 (d, *J*= 8.4 Hz, 2H), 7.63 - 7.47 (m, 5H), 6.97 (d, *J* = 8.5 Hz, 2H), 6.64 (d, *J* = 8.6 Hz, 2H), 6.21 (t, *J* = 74.4 Hz, 1H), 5.68 (d, *J* = 7.3 Hz, 1H), 5.17 (s, 1H), 4.14 (dt, *J* = 14.1, 7.9 Hz, 2H), 3.95 (t, *J* = 9.3 Hz, 1H), 3.71 (dt, *J* = 11.5, 8.0 Hz, 2H), 3.13 - 2.99 (m, 4H), 2.51 (d, *J* = 14.4 Hz, 1H), 2.44 - 2.31 (m, 1H), 1.26 (t, *J* = 7.4 Hz, 3H).

**Example 107 (5R)-1-(4-(((*R*)-2-cyano-1-(4-(ethylsulfonyl)phenyl)ethyl)carbamoyl) phenyl)-*N,N*-dimethyl-5-(4-(trifluoromethyl)phenyl)piperidine-2-carboxamide / (5*S*)-1-(4-(((*R*)-2-cyano-1-(4-(ethylsulfonyl)phenyl)ethyl)carbamoyl)phenyl)-*N,N*-dimethyl-5-(4 -(trifluoromethyl)phenyl)piperidine-2-carboxamide**

107-1: 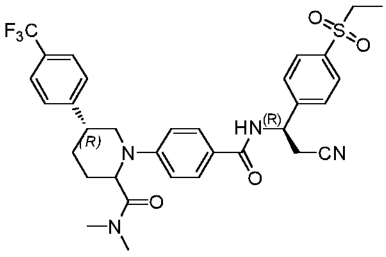 or 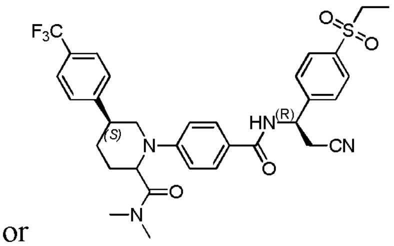

107-2: 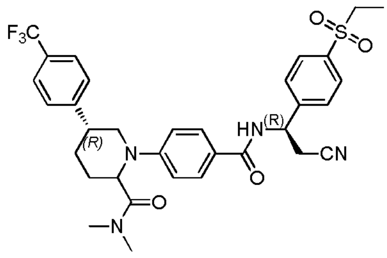 or 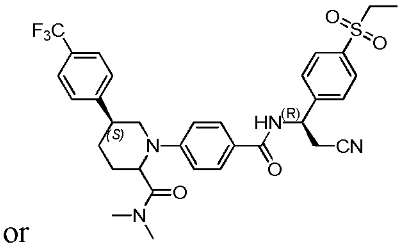

**Step** 1: **Synthesis of methyl (2*S*,5*S*)-5-hydroxypiperidine-2-carboxylate**

(2*S*,5*S*)-5-Hydroxypiperidine-2-carboxylic acid (20.40 g, 141 mmol) was dissolved in MeOH (200 mL), then $SOCl_2$ (18 mL, 248 mmol) was slowly added dropwise under an ice bath. The mixture was stirred at room temperature for 22 h. The resulting mixture was concentrated under reduced pressure to give a white solid (21 g, 94%).
MS (ESI, pos.ion) m/z: 160.2 $[M+H]^+$.

**Step 2: Synthesis of (2*S*,5*S*)-1-*tert*-butyl 2-methyl 5-hydroxypiperidine-1,2-dicarboxylate**

To a solution of methyl (2S,5,*S*)-5-hydroxypiperidine-2-carboxylate (21.10 g, 131.93 mmol) in THF (240 mL) was added a solution of $K_2CO_3$ (46.60 g, 337 mmol) in $H_2O$ (120 mL) in an ice bath. After the reaction solution was evenly mixed, $Boc_2O$ (45 mL, 200 mmol) was slowly added dropwise in an ice bath, and the mixture was reacted at room temperature for 24 h. The resulting mixture was extracted with EtOAc (200 mL × 3). The organic phases were combined, dried over anhydrous $Na_2SO_4$, and concentrated under reduced pressure to obtain yellow oil (33 g, 96%).
MS (ESI, pos.ion) m/z: 282.3 $[M+Na]^+$.

**Step 3: Synthesis of (*S*)-1-*tert*-butyl 2-methyl 5-oxopiperidine-1,2-dicarboxylate**

(2*S*,5*S*)-1-*tert*-butyl 2-methyl 5-hydroxypiperidine-1,2-dicarboxylate (32.01 g, 123.41 mmol) was dissolved in DCM (400 mL), and DMP (105.01 g, 247.60 mmol) was added in batches under ice bath. The mixtrue was reacted at room temperature for 21 h. The mixture was filtered through a celite pad, and saturated $NaHCO_3$ solution (600 mL) was added to the reaction solution to adjust the pH of the system to about 8.The resulting mixture was extracted with DCM (200 mL × 3), dried over anhydrous $Na_2SO_4$, concentrated under reduced pressure, and the concentrated solution was separated by silica gel column chromatography (eluent: PE/EtOAc (v/v) = 3/1) to give yellow oil (30 g, 94%).
MS (ESI, pos.ion) m/z: 280.1 $[M+Na]^+$.

**Step 4: Synthesis of (*S*)-1-*tert*-butyl 2-methyl 5-bromo-3,4-dihydropyridine -1,2(2*H*)-dicarboxylate**

To a solution of (S)-1-*tert*-butyl 2-methyl 5-oxopiperidine-1,2-dicarboxylate (5.10 g, 20.01 mmol) and $P(OPh_3)$ (18.08 g, 58.27 mmol) in DCM (50 mL) was added TEA(14 mL, 101.10 mmol) at -20°C. then $Br_2$ (2.8 mL, 55.03 mmol) was diluted with DCM (25 mL), and the mixture was slowly added dropwise to the reaction system. The addition was completed within 40 min, and the reaction was carried out at room temperature for 16 h. The reaction solution was added with DCM (80 mL), washed successively with saturated $NaHCO_3$ solution (150 mL × 2) and saturated NaCl solution (100 mL × 2), dried over anhydrous $Na_2SO_4$, concentrated under reduced pressure, and the concentrated solution was separated by silica gel column chromatography (eluent: PE/EtOAc (v/v) = 10/1) to give yellow oil (3.77 g, 59%).
MS (ESI, pos.ion) m/z: 222.0 $[M+H]^+$.

**Step 5: Synthesis of 5-(4-(trifluoromethyl)phenyl)-3,4-dihydropyridine-1,2-(2*H*)-dicarboxylic acid-1-*tert*-butyl-2-methyl ester**

Under nitrogen protection, (S)-1-*tert*-butyl 2-methyl 5-bromo-3,4-dihydropyridine-1,2(2*H*)-dicarboxylate (3.77 g, 11.80 mmol), (4-(trifluoromethyl)phenyl)boronic acid (3.57 g, 18.80 mmol), Pd(dppf)$Cl_2$ (887 mg, 1.21 mmol) and $Cs_2CO_3$ (7.63 g, 23.40 mmol) were dissolved in 1,4-dioxane (30 mL). The mixture was reacted at 100°C for 12 h. The resulting mixture was filtered through a celite pad. The filtrate was concentrated under reduced pressure, and the concentrated solution was separated by silica gel column chromatography (eluent: PE/EtOAc (v/v) = 10/1) to give yellow oil (2.62 g, 58%).
MS (ESI, pos.ion) m/z: 408.2 $[M+Na]^+$.

**Step 6: Synthesis of 2-(4-(trifluoromethyl)phenyl)piperidine-1,2-dicarboxylic acid-1-*tert*-butyl-2-methyl ester**

Under hydrogen protection, 5-(4-(trifluoromethyl)phenyl)-3,4-dihydropyridine-1,2(2*H*) -dicarboxylic acid-1-*tert*-butyl-2-methyl ester (2.62 g, 6.80 mmol) and Pd/C (4.41 g, 41.40 mmol) were dissolved in MeOH/THF (15 mL/15 mL), and the mixture was reacted at room temperature for 16 h. The reaction solution was filtered through a celite pad, and the filtrate was concentrated under reduced pressure to give colorless oil (2.40 g, 91%).
MS (ESI, pos.ion) m/z: 410.2 $[M+Na]^+$.

**Step 7: Synthesis of methyl 5-(4-(trifluoromethyl)phenyl)piperidine-2-carboxylate**

To a solution of 2-(4-(trifluoromethyl)phenyl)piperidine-1,2-dicarboxylate-1-*tert*-butyl-2-methyl ester (4.05 g, 10.50 mmol) in DCM (20 mL) ) was slowly added dropwise TFA (6.2 mL, 83 mmol) in an ice bath. The mixture was reacted at room temperature for 6 h. Saturated $NaHCO_3$ solution (80 mL) was added to the reaction solution to adjust the pH of the system to about 8. The resulting mixture was extracted with DCM (100 mL × 2), dried over anhydrous $Na_2SO_4$, concentrated under reduced pressure to give colorless oil (1.69 g, 56%).
MS (ESI, pos.ion) m/z: 288.0$[M+H]^+$.

**Step 8: Synthesis of methyl 1-(4-cyanophenyl)-5-(4-(trifluoromethyl) phenyl)piperidine-2-carboxylate**

Under nitrogen protection, methyl 5-(4-(trifluoromethyl)phenyl)piperidine -2-carboxylate (1.69 g, 5.88 mmol), 4-bromobenzonitrile (1.48 g, 8.13 mmol), $Pd_2(dba)_3$ (549 mg, 0.60 mmol), 2-bicyclohexylphosphine-2',6'-diisopropoxybiphenyl (1.08 g, 2.31 mmol) and $Cs_2CO_3$ (5.79 g, 17.80 mmol) were dissolved in toluene (20 mL), and the mixture was reacted at 100°C for 16 h. The resulting mixture was filtered through a celite pad. The filtrate was concentrated under reduced pressure, and the concentrated solution was separated by silica gel column chromatography (eluent: PE/EtOAc (v/v) = 5/1) to give yellow oil (1.80 g, 79%).
MS (ESI, pos.ion) m/z: 389.1 $[M+H]^+$.

**Step 9: Synthesis of 1-(4-cyanophenyl)-5-(4-(trifluoromethyl)phenyl)piperidine-2-carboxylic acid**

Methyl 1-(4-cyanophenyl)-5-(4-(trifluoromethyl)phenyl)piperidine-2-carboxylate (628 mg, 1.62 mmol) and Li-OH.$H_2O$ (215 mg, 5.12 mmol) were dissolved in MeOH/THF/H2O (3 mL/6 mL/2 mL) and the mixture was reacted at room temperature for 24 h. 1.0 M HCl solution (30 mL) was added to the reaction solution to adjust the pH to about 4.The resulting mixture was extracted with EtOAc (50 mL × 2), and the organic phase was dried over anhydrous $Na_2SO_4$, concentrated under reduced pressure. The concentrated solution was separated by silica gel column chromatography (eluent: PE/EtOAc (v/v) = 1/1) to give yellow oil (417 mg, 69%).
MS (ESI, pos.ion) m/z: 375.0 $[M+H]^+$.

**Step 10: Synthesis of 1-(4-cyanophenyl)--*N*,*N*-dimethyl-5-(4-(trifluoromethyl) phenyl)piperidine-2-carboxamide**

1-(4-Cyanophenyl)-5-(4-(trifluoromethyl)phenyl)piperidine-2-carboxylic acid (417 mg, 1.11 mmol), N,N-dimethylmethylamine (249 mg, 3.05 mmol), EDCI (459 mg, 2.40 mmol) and HOBT (320 mg, 2.37 mmol) were dissolved in DCM (6 mL), then TEA (0.8 mL, 6 mmol) was added, and the mixture was reacted at room temperature for 17 h. The reaction solution was added with DCM (80 mL), washed with saturated NaCl solution (40 mL × 2), dried over anhydrous $Na_2SO_4$, concentrated under reduced pressure, and the concentrated solution was separated by silica gel column chromatography (eluent: PE/EtOAc (v/v) = 2/1) to give a yellow solid (374 mg, 84%).
MS (ESI, pos.ion) m/z:402.3 $[M+H]^+$.

**Step 11: Synthesis of 4-(2-(dimethylcarbamoyl)-5-(4-(trifluoromethyl) phenyl)piperidin-1-yl)benzoic acid**

1-(4-cyanophenyl)-*N*,*N*-dimethyl-5-(4-(trifluoromethyl)phenyl)piperidine-2-carboxami de (374 mg, 0.93 mmol) and $CH_3ONa$ (5.20 g, 96 mmol) were dissolved in EtOH (8 mL), and the mixture was reacted at 100°C for 22 h. 3.0 M HCl solution (20 mL) was added to the reaction solution to adjust the pH to about 3.The resulting mixture was extracted with EtOAc (40 mL × 2), and the organic phase was dried over anhydrous $Na_2SO_4$, concentrated under reduced pressure. The concentrated solution was separated by silica gel column chromatography (eluent: DCM/MeOH (v/v) = 8/1) to give a yellow solid 360 mg, which was further performed chiral resolution to give yellow solids (63 mg, 16 %) and (32 mg, 8%). MS (ESI, pos.ion) m/z:421.3 $[M+H]^+$.

**Step 12**: **Synthesis of (5*R*)-1-(4-(((*R*)-2-cyano-1-(4-(ethylsulfonyl)phenyl)ethyl)carbamoyl) phenyl)-*N,N*-dimethyl-5-(4-(trifluoromethyl)phenyl)piperidine-2-carboxamide / (5*S*)-1-(4-(((*R*)-2-cyano-1-(4-(ethylsulfonyl)phenyl)ethyl)carbamoyl)phenyl)-*N,N*-dimethyl-5-(4 -(trifluoromethyl)phenyl)piperidine-2-carboxamide**

4-(2-(Dimethylcarbamoyl)-5-(4-(trifluoromethyl)phenyl)piperidin-1-yl)benzoic acid (63 mg, 0.15 mmol), (*R*)-3-amino-3-(4-(ethylsulfonyl)phenyl)propionitrile hydrochloride (64 mg, 0.23 mmol), EDCI (89 mg, 0.46 mmol) and HOBT (65 mg, 0.48 mmol) were dissolved in DCM (5 mL), then TEA (0.17 mL, 1.2 mmol) was added and the mixture was reacted at room temperature for 19 h. The reaction solution was added with DCM (50 mL), washed with saturated NaCl solution (30 mL × 2), dried over anhydrous $Na_2SO_4$, concentrated under reduced pressure, and the concentrated solution was separated by silica gel column chromatography (eluent: PE/EtOAc (v/v) = 1/2) to give a yellow solid 107-1: (17 mg, 18%) and a yellow solid 107-2: (25 mg, 26%).

MS (ESI, neg.ion) m/z: 639.2 $[M-H]^-$.

**107-1:**

$^1H$ NMR (400 MHz, DMSO-$d_6$) δ (ppm) : 8.95 (d, *J* = 8.2 Hz, 1H), 7.89 (d, *J* = 7.6 Hz, 2H), 7.77 (d, *J* = 8.7 Hz, 2H), 7.72 (d, *J* = 8.1 Hz, 2H), 7.66 (d, *J* = 8.1 Hz, 2H), 7.55 (d, *J* = 8.0 Hz, 2H), 6.94 (d, *J* = 8.7 Hz, 2H), 5.50 (dd, *J* = 15.0, 7.0 Hz, 1H), 4.83 (s, 1H), 3.96 (dd, *J* = 12.5, 4.3 Hz, 1H), 3.73 (dd, *J* = 12.3, 5.0Hz, 1H), 3.51 (s, 1H), 3.31 - 3.26 (m, 3H), 3.16 - 3.11 (m, 4H), 2.79 (s, 3H), 2.04 - 1.92 (m, 2H), 1.80 (s, 2H), 1.09 (t, *J*= 7.3 Hz, 3H).

**107-2:**

$^1H$ NMR (400 MHz, DMSO-$d_6$) δ (ppm) : 8.94 (d, *J* = 8.1 Hz, 1H), 7.88 (d, *J* = 7.2 Hz, 2H), 7.78 - 7.68 (m, 6H), 7.56 (d, *J* = 8.0 Hz, 2H), 6.97 (d, *J* = 8.9 Hz, 2H), 5.49 (dd, *J* = 15.3, 7.7 Hz, 1H), 5.20 (d, *J* = 4.8 Hz, 1H), 3.78 (dd, *J* = 11.3, 4.4 Hz, 1H), 3.59 (t, *J* = 11.8 Hz, 1H), 3.31 - 3.26 (m, 2H), 3.19 - 3.08 (m, 5H), 3.02 - 2.94 (m, 1H), 2.81 (s, 3H), 2.10 (d, *J* = 12.9 Hz, 1H), 2.04 - 1.96 (m, 1H), 1.83 (d, *J*= 14.9 Hz, 2H), 1.09 (t, *J*= 7.3 Hz, 3H).

**Example 108**

**108-1:**

***N*-((*R*)-2-cyano-1-(4-(ethylsulfonyl)phenyl)ethyl)-4-((2*S*,5*R*)-2-(ethoxymethyl)-5-(4-(trifluorom ethyl) phenyl)piperidin-1-yl)benzamide**

**108-2:**

***N*-((*R*)-2-cyano-1-(4-(ethylsulfonyl)phenyl)ethyl)-4-((2*R*,5*S*)-2-(ethoxymethyl)-5-(4-(trifluorom ethyl) phenyl)piperidin-1-yl)benzamide**

108-1: 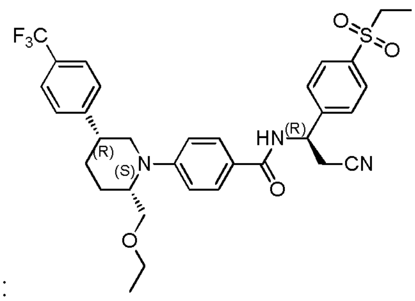 and 108-2: 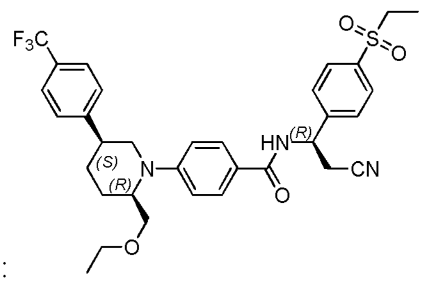

**Step 1: Synthesis of 4-(2-(hydroxymethyl)-5-(4-(trifluoromethyl)phenyl) piperidin-1-yl)benzonitrile**

To a solution of methyl 1-(4-cyanophenyl)-5-(4-(trifluoromethyl)phenyl) piperidine-2-carboxylate (107 mg, 0.28 mmol) in THF (6 mL) was slowly added $LiBH_4$ (22 mg, 1.01 mmol) in an ice bath. The mixture was reacted at room temperature for 18 h. The reaction solution was added with $NaHCO_3$ (20 mL) to quench the reaction. The resulting mixture was concentrated under reduced pressure, extracted with DCM (30 mL × 2), dried over anhydrous $Na_2SO_4$, concentrated under reduced pressure, and the concentrated solution was separated by silica gel column chromatography (eluent: PE/EtOAc (v/v) = 2/1) to give colorless oil (47 mg, 47%).
MS (ESI, pos.ion) m/z: 361.1 $[M+H]^+$.

**Step 2: Synthesis of 4-(2-(ethoxymethyl)-5-(4-(trifluoromethyl)phenyl) piperidin-1-yl)benzonitrile**

4-(2-(Hydroxymethyl)-5-(4-(trifluoromethyl)phenyl)piperidin-1-yl)benzonitrile (213 mg, 0.59 mmol), NaOH (111 mg, 2.78 mmol) and iodoethane (0.2 mL, 3 mmol) were dissolved in DMF (1 mL), and the mixture was reacted at 70°C for 15 h. The reaction solution was added with saturated NaCl solution (20 mL), extracted with EtOAc (40 mL × 2), dried over anhydrous $Na_2SO_4$, concentrated under reduced pressure, and the concentrated solution was separated by silica gel column chromatography (eluent: PE/EtOAc (v/v) = 5/1) to give a yellow solid (190 mg, 83%).
MS (ESI, pos.ion) m/z: 389.1 $[M+H]^+$.

**Step 3: Synthesis of 4-((2*S*,5*R*)-2-(ethoxymethyl)-5-(4-(trifluoromethyl)phenyl) piperidin-1-yl)benzoic acid / 4-((2*R*,5*S*)-2-(ethoxymethyl)-5-(4-(trifluoromethyl)phenyl) piperidin-1-yl)benzoic acid**

4-(2-(Ethoxymethyl)-5-(4-(trifluoromethyl)phenyl)piperidin-1-yl)benzonitrile (190 mg, 0.49 mmol) and $CH_3ONa$ (2.70 g, 50 mmol) were dissolved in EtOH (8 mL), and the mixture was reacted at 100°C for 18 h. 1.0 M HCl solution (20 mL) was added to the reaction solution to adjust the pH about 3. The resulting mixture was extracted with EtOAc (50 mL × 2), dried over anhydrous $Na_2SO_4$, concentrated under reduced pressure, and the concentrated solution was separated by silica gel column chromatography (eluent: PE/EtOAc (v/v) = 2/1) to give yellow oil 190 mg, which was performed further chiral resolution (chiral OA, *i*-PrOH/DCM = 20/1) to give yellow solids 4-((2*S*,5*R*)-2-(ethoxymethyl)-5-(4-(trifluoromethyl)phenyl)piperidin-1-yl)benzoic acid (111 mg, 56%) and 4-((2*R*,5*S*)-2-(ethoxymethyl)-5-(4-(trifluoromethyl)phenyl)piperidin-1-yl)benzoic acid (74 mg, 37%).
MS (ESI, pos.ion) m/z: 408.1 $[M+H]^+$.

**Step 4: Synthesis of *N*-((*R*)-2-cyano-1-(4-(ethylsulfonyl)phenyl)ethyl)-4-((2*S*,5*R*)-2 -(ethoxymethyl)-5-(4-(trifluoromethyl)phenyl)piperidin-1-yl)benzamide (108-1) / *N*-((*R*)-2-cyano-1-(4-(ethylsulfonyl)phenyl)ethyl)-4-((2*R*,5*S*)-2-(ethoxymethyl)-5-(4-(trifluorom ethyl)phenyl)piperidin-1-yl)benzamide (108-2)**

4-((2*S*,5*R*)-2-(Ethoxymethyl)-5-(4-(trifluoromethyl)phenyl)piperidin-1-yl)benzoic acid (111 mg, 0.27 mmol), (*R*)-3-amino-3-(4-(ethylsulfonyl)phenyl)propionitrile hydrochloride (88 mg, 0.32 mmol), EDCI (170 mg, 0.89 mmol) and HOBT (120 mg, 0.89 mmol) were dissolved in DCM (5 mL), then TEA (0.32 mL, 2.3 mmol) was added and the mixture was reacted at room temperature for 12 h. The reaction solution was added with DCM (60 mL), washed with saturated NaCl solution (30 mL × 2), dried over anhydrous $Na_2SO_4$, concentrated under reduced pressure, and the concentrated solution was separated by silica gel column chromatography (eluent: PE/EtOAc (v/v) = 1/2) to give compound 108-1 as a yellow solid (121 mg, 71%).

Using 4-((2*R*,5*S*)-2-(ethoxymethyl)-5-(4-(trifluoromethyl)phenyl)piperidin-1-yl) benzoic acid as raw material, compound 108-2 was prepared by the above method as a yellow solid (110 mg, 60%).

MS (ESI, pos.ion) m/z: 628.2 $[M+H]^+$.
**108-1:**
$^1$H NMR (400 MHz, DMSO-$d_6$) δ (ppm): 8.92 (d, *J* = 8.1 Hz, 1H), 7.89 (d, *J* = 7.6 Hz, 2H), 7.77 (d, *J* = 7.8 Hz, 2H), 7.74 - 7.68 (m, 4H), 7.64 (d, *J* = 7.8 Hz, 2H), 7.00 (d, *J* = 8.2 Hz, 2H), 5.51 (d, *J* = 7.2 Hz, 1H), 4.29 (s, 1H), 3.72 (dd, *J* = 20.2, 11.0 Hz, 2H), 3.45 (d, *J* = 5.2 Hz, 3H), 3.28 (dd, *J* = 14.2, 7.0 Hz, 2H), 3.14 (d, *J* = 6.5 Hz, 2H), 3.11 - 3.04 (m, 1H), 2.91 (t, *J* = 11.2 Hz, 1H), 2.04 - 1.90 (m, 2H), 1.80 (t, *J* = 15.3 Hz, 2H), 1.13 - 1.05 (m, 6H).
**108-2:**
$^1$H NMR (400 MHz, DMSO-$d_6$) δ (ppm): 8.92 (d, *J* = 7.9 Hz, 1H), 7.89 (d, *J*= 7.9 Hz, 2H), 7.77 (d, *J* = 8.3 Hz, 2H), 7.74 - 7.68 (m, 4H), 7.64 (d, *J* = 7.7 Hz, 2H), 7.01 (d, *J* = 8.4 Hz, 2H), 5.51 (dd, *J* = 14.6, 7.4 Hz, 1H), 4.29 (s, 1H), 3.72 (dd, *J* = 21.2, 11.8 Hz, 2H), 3.52 - 3.43 (m, 3H), 3.28 (dd, *J* = 14.6, 7.4 Hz, 2H), 3.15 (d, *J* = 6.6 Hz, 2H), 3.12 - 3.04 (m, 1H), 2.92 (t, *J* = 10.8 Hz, 1H), 1.98 (dd, *J* = 22.5, 15.2 Hz, 2H), 1.79 (t, *J* = 15.9 Hz, 2H), 1.12 - 1.05 (m, 6H).

**Example 109**

**109-1 : *N*-((*R*)-2-cyano-1-(4-(ethylsulfonyl)phenyl)ethyl)-4-((2*S*,5*R*)-2-((cyclopropylmethoxy)methyl) -5-(4-(trifluoromethyl)phenyl)piperidin-1-yl)benzamide**

**109-2: *N*-((*R*)-2-cyano-1-(4-(ethylsulfonyl)phenyl)ethyl)-4-((2*R*,5*S*)-2-((cyclopropylmethoxy)methyl)** -5-(**4-(trifluoromethyl)phenyl)piperidin-1-yl)benzamide**

109-1: 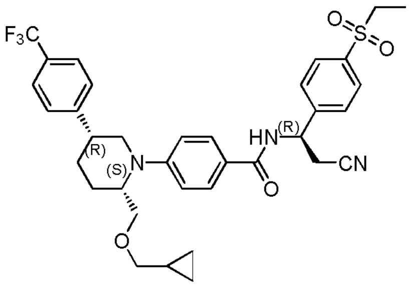 and 109-2: 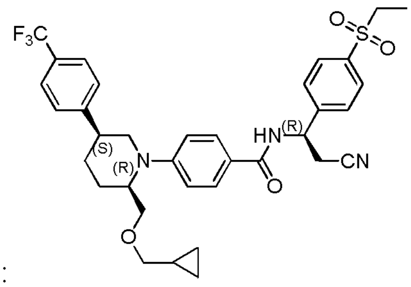

The raw material iodoethane in step 2 of Example 108 was replaced with bromomethylcyclopropane, which was used to prepare the title compounds with the intermediate 4-(2-(hydroxymethyl)-5-(4-(trifluoromethyl)phenyl)piperidin-1-yl)benzonitrile according to the methods of step 2 to step 4 in Example 108. All of the title compounds were white solids, 109-1: (80 mg, 87%); 109-2: (75 mg, 79%).

MS (ESI, pos.ion) m/z: 654.1 $[M+H]^+$.
**109-1 :**
$^1$H NMR (400 MHz, DMSO-$d_6$) δ (ppm): 8.91 (d, *J* = 7.7 Hz, 1H), 7.89 (d, *J* = 7.6 Hz, 2H), 7.77 (d, *J* = 8.3 Hz, 2H), 7.75 - 7.68 (m, 4H), 7.64 (d, *J* = 7.7 Hz, 2H), 7.00 (d, *J* = 8.2 Hz, 2H), 5.50 (dd, *J* = 14.8, 7.3 Hz, 1H), 4.30 (s, 1H), 3.78 - 3.66 (m, 2H), 3.50 (dd, *J* = 9.4, 4.1 Hz, 1H), 3.27 (dd, *J* = 10.4, 7.4 Hz, 4H), 3.11 (dd, *J* = 26.0, 9.6 Hz, 3H), 2.93 (d, *J* = 11.1 Hz, 1H), 2.15 - 1.91 (m, 3H), 1.80 (t, *J* = 16.2 Hz, 2H), 1.09 (t, *J* = 7.1 Hz, 3H), 0.43 (d, *J* = 7.2 Hz, 2H), 0.14 (d, *J* = 3.4 Hz, 2H).
**109-2:**
$^1$H NMR (400 MHz, DMSO-$d_6$) δ (ppm): 8.91 (d, *J* = 8.3 Hz, 1H), 7.89 (d, *J* = 8.1 Hz, 2H), 7.77 (d, *J* = 8.7 Hz, 2H), 7.71 (dd, *J* = 8.1, 4.0 Hz, 4H), 7.64 (d, *J* = 8.0 Hz, 2H), 7.00 (d, *J* = 8.7 Hz, 2H), 5.50 (dd, *J* = 15.2, 7.9 Hz, 1H), 4.30 (s, 1H), 3.78 - 3.65 (m, 2H), 3.50 (dd, *J* = 9.1, 4.3 Hz, 1H), 3.27 (dd, *J* = 9.9, 7.2 Hz, 4H), 3.11 (dd, *J* = 25.8, 10.5 Hz, 3H), 2.92 (t, *J* = 11.7 Hz, 1H), 2.09 - 1.89 (m, 3H), 1.80 (dd, *J* = 20.8, 11.9 Hz, 2H), 1.09 (t, *J* = 7.3 Hz, 3H), 0.44 (d, *J* = 7.8 Hz, 2H), 0.14 (d, *J* = 4.2 Hz, 2H).

**Example 110**

**110-1: *N*-((*R*)-2-cyano-1-(4-(ethylsulfonyl)phenyl)ethyl)-4-((2*S*,5*R*)-2-((1,1-difluoropropoxy)methyl) -5-(4-(trifluoromethyl)phenyl)piperidin-1-yl)benzamide**

**110-2: *N*-((*R*)-2-cyano-1-(4-(ethylsulfonyl)phenyl)ethyl)-4-((2*R*,5*S*)-2-((1,1-difluoropropoxy)methyl)-5 -(4-(trifluoromethyl)phenyl)piperidin-1-yl)benzamide**

110-1: 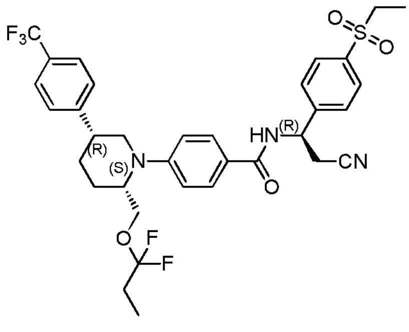 and 110-2: 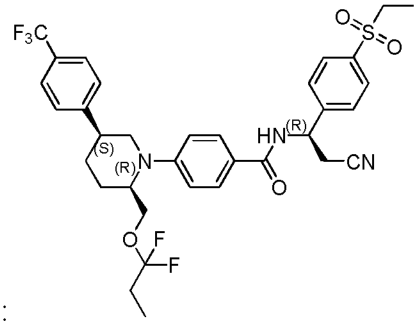

**Step 1: Synthesis of 4-(2-(hydroxymethyl)-5-(4-(trifluoromethyl)phenyl)piperidin-1-yl)benzoic acid**

4-(2-(Hydroxymethyl)-5-(4-(trifluoromethyl)phenyl)piperidin-1-yl)benzonitrile (511 mg, 1.42 mmol) and $CH_3ONa$ (7.50 g, 140 mmol) ) were dissolved in EtOH (10 mL), and the mixture was reacted at 100°C for 18 h. 1.0 M HCl solution (100 mL) was added to the reaction solution to adjust the pH to about 3. The resulting mixture was extracted with EtOAc (150 mL × 2), dried over anhydrous $Na_2SO_4$, concentrated under reduced pressure to give yellow oil (522 mg, 97%).
MS (ESI, pos.ion) m/z: 380.1 $[M+H]^+$.

**Step 2: Synthesis of methyl 4-(2-(hydroxymethyl)-5-(4-(trifluoromethyl) phenyl)piperidin-1-yl)benzoate**

4-(2-(Hydroxymethyl)-5-(4-(trifluoromethyl)phenyl)piperidin-1-yl)benzoic acid (522 mg, 1.38 mmol), MeOH (0.3 mL, 7 mmol), HOBT (586 mg, 4.34 mmol) and EDCI (798 mg, 4.16 mmol) were dissolved in DCM (6 mL), then TEA (1.6 mL, 12 mmol) was added, and the mixture was reacted at room temperature for 18 h. The reaction solution was added with DCM (80 mL), washed with saturated NaCl solution (40 mL × 2), dried over anhydrous $Na_2SO_4$, concentrated under reduced pressure, and the concentrated solution was separated by silica gel column chromatography (eluent: PE/EtOAc (v/v) = 2/1) to give a yellow solid (357 mg, 66%).
MS (ESI, pos.ion) m/z:394.1 $[M+H]^+$.

**Step 3: Synthesis of methyl 4-(2-(((1,1-difluoroallyl)oxy)methyl)-5-(4-(trifluoromethyl) phenyl)piperidin-1-yl)benzoate**

To a solution of methyl 4-(2-(hydroxymethyl)-5-(4-(trifluoromethyl)phenyl) piperidin-1-yl)benzoate (337 mg, 0.86 mmol), NaOH (125 mg, 3.13 mmol) and $Bu_4NBr$ (839 mg, 2.60 mmol) in DCM (5 mL) was added dropwise 3-bromo-3,3-difluoro-prop-1-ene (0.35 mL, 3.40 mmol). The mixture was reacted at room temperature for 48 h. TLC monitored that there was not much raw material remaining. The reaction solution was added with saturated NaCl (20 mL) solution, extracted with EtOAc (30 mL × 2), dried over anhydrous $Na_2SO_4$, concentrated under reduced pressure, and the concentrated solution was separated by silica gel column chromatography (eluent: PE/EtOAc (v/v) = 1/5) to give colorless oil (229 mg, 57%).
MS (ESI, pos.ion) m/z:470.1 $[M+H]^+$.

**Step 4: Synthesis of methyl 4-(2-((1,1-difluoropropoxy)methyl)-5-(4-(trifluoromethyl) phenyl)piperidin-1-yl)benzoate**

Under hydrogen protection, methyl 4-(2-(((1,1-difluoroallyl)oxy)methyl)-5-(4-(trifluoromethyl)phenyl)piperidin-1-yl)benzoate (229 mg, 0.49 mmol) and Pd/C (254 mg, 2.39 mmol) were dissolved in MeOH/THF (5 mL/5 mL), and the mixture was reacted at room temperature for 16 h. The reaction solution was filtered through a celite pad, and the filtrate was concentrated under reduced pressure to give colorless oil (229 mg, 99%).
MS (ESI, pos.ion) m/z: 472.2 $[M+H]^+$.

**Step 5: Synthesis of 4-((2*S*,5*R*)-2-((1,1-difluoropropoxy)methyl) -5-(4-(trifluoromethyl)phenyl)piperidin-1-yl)benzoic acid / 4-((2*R*,5*S*) -2-((1,1-difluoropropoxy)methyl)-5-(4-(trifluoromethyl)phenyl)piperidin -1-yl)benzoic acid**

Methyl 4-(2-((1,1-difluoropropoxy)methyl)-5-(4-(trifluoromethyl)phenyl)piperidin -1-yl)benzoate (229 mg, 0.49 mmol) and $LiOH{\cdot}H_2O$ (141 mg, 3.36 mmol) were dissolved in MeOH/THF/$H_2O$ (1 mL/2 mL/0.6 mL) and the mixture was reacted at room temperature for 24 h. 3.0 M HCl solution (8 mL) was added to the reaction solution to adjust the pH to

about 4. The resulting mixture was concentrated under reduced pressure, extracted with EtOAc (30 mL × 2), dried over anhydrous $Na_2SO_4$, concentrated under reduced pressure, and the concentrated solution was separated by silica gel column chromatography (eluent: PE/EtOAc (v/v) = 1/2) to give a yellow solid 112 mg, which was further performed chiral resolution (chiral OA column, mobile phase i-PrOH/DCM = 20/1) to give a white solid A: 4-((2*S*,5*R*)-2-((1,1-difluoropropoxy)methyl)-5-(4-(trifluoromethyl)phenyl) piperidin-1-yl)benzoic acid (45 mg, 20 %) and a white solid B: 4-((2*R*,5*S*)-2-((1,1-difluoropropoxy)methyl)-5-(4-(trifluoromethyl)phenyl)piperidin-1-yl)benzoic acid (26 mg, 12 %).
MS (ESI, pos.ion) m/z: 458.1 $[M+H]^+$.

**Step 6: Synthesis of *N*-((*R*)-2-cyano-1-(4-(ethylsulfonyl)phenyl)ethyl)-4-((2*S*,5*R*) -2-((1,1-difluoropropoxy)methyl)-5-(4-(trifluoromethyl)phenyl)piperidin-1-yl)benzamide / *N*-((*R*)-2-cyano-1-(4-(ethylsulfonyl)phenyl)ethyl)-4-((2*R*,5*S*)-2-((1,1-difluoropropoxy)methyl)-5 -(4-(trifluoromethyl)phenyl)piperidin-1-yl)benzamide**

4-((2*S*,5*R*)-2-((1,1-Difluoropropoxy)methyl)-5-(4-(trifluoromethyl)phenyl)piperidin-1-yl)benzoic acid (45 mg, 0.098 mmol), (*R*)-3-amino-3-(4-(ethylsulfonyl)phenyl)propionitrile hydrochloride (38 mg, 0.14 mmol), EDCI (130 mg, 0.68 mmol) and HOBT (110 mg, 0.81 mmol) were dissolved in DCM (5 mL), then TEA (0.16 mL, 1.20 mmol) was added and the mixture was reacted at room temperature for 18 h. The reaction solution was added with DCM (60 mL), washed with saturated NaCl solution (30 mL × 2), dried over anhydrous $Na_2SO_4$, concentrated under reduced pressure, and the concentrated solution was separated by silica gel column chromatography (eluent: PE/EtOAc (v/v) = 1/2) to give compound 110-1 as a white solid (30 mg, 45%).

Using 4-((2*R*,5*S*)-2-((1,1-Difluoropropoxy)methyl)-5-(4-(trifluoromethyl)phenyl) piperidin-1-yl)benzoic acid as raw material, compound 110-2 was prepared by the above method as a white solid (28 mg, 42%).

MS (ESI, pos.ion) m/z: 678.2 $[M+H]^+$.
**110-1:**
$^1$H NMR (400 MHz, DMSO-$d_6$) δ (ppm): 8.93 (d, *J* = 8.0 Hz, 1H), 7.89 (d, *J* = 8.2 Hz, 2H), 7.78 (d, *J* = 7.7 Hz, 2H), 7.74 - 7.69 (m, 4H), 7.66 (d, *J* = 8.3 Hz, 2H), 7.01 (d, *J* = 8.8 Hz, 2H), 5.51 (dd, *J* = 15.3, 7.7 Hz, 1H), 4.44 - 4.37 (m, 1H), 4.18 - 4.12 (m, 1H), 4.03 (dd, *J* = 10.1, 6.3 Hz, 1H), 3.70 (dd, *J* = 13.2, 2.8 Hz, 1H), 3.28 (dd, *J* = 14.7, 7.4 Hz, 2H), 3.17 - 3.09 (m, 3H), 2.94 (dd, *J* = 12.9, 7.6 Hz, 1H), 1.97 (dd, *J*= 19.3, 8.4 Hz, 2H), 1.93 - 1.81 (m, 4H), 1.09 (t, *J*= 7.3 Hz, 3H), 0.86 (t, *J* = 7.5 Hz, 3H).
**110-2:**
$^1$H NMR (400 MHz, DMSO-$d_6$) δ (ppm): 8.93 (d, *J* = 8.3 Hz, 1H), 7.88 (d, *J* = 8.2 Hz, 2H), 7.77 (d, *J* = 8.5 Hz, 2H), 7.71 (d, *J* = 8.9 Hz, 4H), 7.65 (d, *J* = 8.2 Hz, 2H), 7.01 (d, *J* = 8.8 Hz, 2H), 5.50 (dd, *J* = 15.2, 7.8 Hz, 1H), 4.40 (s, 1H), 4.18 - 4.09 (m, 1H), 4.03 (dd, *J* = 10.1, 6.5 Hz, 1H), 3.69 (d, *J* = 9.7 Hz, 1H), 3.27 (t, *J* = 7.5 Hz, 2H), 3.18 - 3.06 (m, 3H), 2.93 (s, 1H), 1.97 (dd, *J* = 18.3, 8.4 Hz, 2H), 1.88 (dd, *J* = 18.7, 7.6 Hz, 3H), 1.80 (d, *J* = 10.5 Hz, 1H), 1.09 (t, *J* = 7.3 Hz, 3H), 0.86 (t, *J* = 7.5 Hz, 3H).

**Example 111**

**111-1: *N*-((*S*)-2-cyano-1-(4-(ethylsulfonyl)phenyl)ethyl)-4-((2*S*,5*S*)-2-((difluoromethoxy)methyl)-5-(4-(trifluoromethyl)phenyl)piperidin-1-yl)-2-fluorobenzamide**

**111-2: *N*-((*S*)-2-cyano-1-(4-(ethylsulfonyl)phenyl)ethyl)-4-((2*S*,5*R*)-2-((difluoromethoxy)methyl)-5-(4-(trifluoromethyl)phenyl)piperidin-1-yl)-2-fluorobenzamide**

111-1: 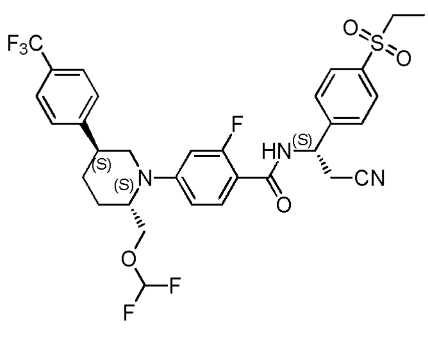 and 111-2: 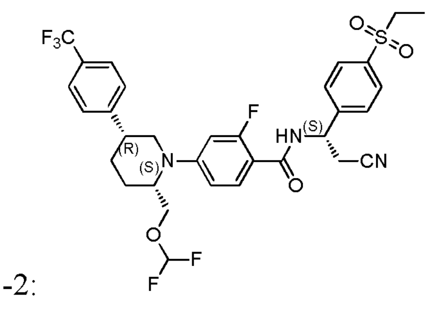

**Step 1: Synthesis of *tert*-butyl (S)-5-bromo-2-(hydroxymethyl)-3,4-dihydropyridine-1(2*H*)-carboxylate**

To a solution of (*S*)-1-*tert*-butyl 2-methyl 5-bromo-3,4-dihydropyridine -1,2(2*H*)-dicarboxylate (1.60 g, 5.00 mmol) in THF (8 mL) ) was slowly added $LiBH_4$ (342 mg, 15.70 mmol) in batches under an ice bath, and the mixture was reacted at room temperature for 16 h. Saturated $NaHCO_3$ solution (50 mL) was added to the reaction solution to quench the reaction. The resulting mixture was concentrated under reduced pressure, extracted with DCM (80 mL × 2), dried over anhydrous $Na_2SO_4$, concentrated under reduced pressure, and the concentrated solution was separated by silica gel column chromatography (eluent: PE/EtOAc (v/v) = 3/1) to give colorless oil (450 mg, 31%).
MS (ESI, pos.ion) m/z:302.1 $[M+H-56]^+$.

**Step 2: Synthesis of *tert*-butyl (*S*)-2-(hydroxymethyl)-5-(4-(trifluoromethyl)phenyl)-3,4-dihydropyridine-1(2*H*)-carboxylate**

Under nitrogen protection, *tert*-butyl (2*S*)-5-bromo-2-(hydroxymethyl)-3,4-dihydro-2*H*-pyridine-1-carboxylate (376 mg, 1.27 mmol), (4-(trifluoromethyl)phenyl)boronic acid (395 mg, 2.08 mmol), Pd(dppf)$Cl_2$ (128 mg, 0.17 mmol) and $Cs_2CO_3$ (468 mg, 1.44 mmol) were dissolved in 1,4-dioxane (8 mL). The mixture was heated to 90°C and reacted for 12 h. The resulting mixture was filtered through a celite pad. The filtrate was concentrated under reduced pressure, and the concentrated solution was separated by silica gel column chromatography (eluent: PE/EtOAc (v/v) = 3/1) to give yellow oil (224 mg, 49 %).
MS (ESI, pos.ion) m/z: 380.1 $[M+Na]^+$.

**Step 3: Synthesis of *tert*-butyl (2*S*)-2-(hydroxymethyl)-5-(4-(trifluoromethyl) phenyl)piperidine-1-carboxylate**

Under hydrogen protection, *tert*-butyl (*S*)-2-(hydroxymethyl)-5-(4-(trifluoromethyl)phenyl)-3,4-dihydropyridine-1(2*H*)-dicarboxylate (2.60 g, 7.30 mmol) and Pd/C (3.70 g, 35.00 mmol) were dissolved in MeOH/THF (10 mL/10 mL), and the mixture was reacted at room temperature for 24 h. The reaction solution was filtered through a celite pad, and the filtrate was concentrated under reduced pressure to give colorless oil (2.58 g, 99%).
MS (ESI, pos.ion) m/z: 382.2 $[M+Na]^+$.

**Step 4: Synthesis of *tert*-butyl (2*S*)-2-((difluoromethoxy)methyl)-5-(4-(trifluoromethyl) phenyl)piperidine-1-carboxylate**

To a solution of *tert*-butyl (2*S*)-2-(hydroxymethyl)-5-(4-(trifluoromethyl) phenyl)piperidine-1-carboxylate(2.58 g, 7.18 mmol) in DCM/$H_2O$ ( 5.9 mL/5.9 mL) was added KOAc (4.25 g, 43.30 mmol), then (bromo(difluoro)methyl)-trimethylsilane (3.4 mL, 22 mmol) was slowly added dropwise, and the mixture was reacted at room temperature for 17 h . Saturated $NaHCO_3$ solution (50 mL) was added to the reaction solution to quench the reaction. The resulting mixture was extracted with DCM (80 mL × 2), dried over anhydrous $Na_2SO_4$, concentrated under reduced pressure, and the concentrated solution was separated by silica gel column chromatography (eluent: PE/EtOAc (v/v) = 8/1) to give colorless oil (2.10 g, 71%).
MS (ESI, pos.ion) m/z: 432.2 $[M+Na]^+$.

**Step 5: Synthesis of (2*S*)-2-((difluoromethoxy)methyl)-5-(4-(trifluoromethyl)phenyl)piperidine**

To a solution of *tert*-butyl (2*S*)-2-((difluoromethoxy)methyl)-5-(4-(trifluoromethyl)phenyl)piperidine-1-carboxylate (2.10 g, 5.10 mmol) in EtOAc (5 mL) was slowly added dropwise HCl.EtOAc (9 mL, 27.00 mmol, 3 mol/L) under an ice bath, and the mixture was reacted at room temperature for 18 h . The mixture was concentrated under reduced pressure to remove part of HCl and EtOAc, then saturated $NaHCO_3$ solution (50 mL) was added to adjust the pH of the system to about 8. The resulting mixture was extracted with DCM (80 mL × 2), dried over anhydrous $Na_2SO_4$, concentrated under reduced pressure to give colorless oil (1.50 g, 95%).
MS (ESI, pos.ion) m/z: 310.1 $[M+H]^+$.

Step 6: Synthesis of methyl **4-((2S)-2-((difluoromethoxy)methyl)-5-(4-(trifluoromethyl) phenyl)piperidin-1-yl)-2-fluorobenzoate**

Under nitrogen protection, (2*S*)-2-((difluoromethoxy)methyl)-5-(4-(trifluoromethyl) phenyl)piperidine (214 mg, 0.69 mmol), methyl 2-fluoro-4-iodo-benzoate (730 mg, 2.61 mmol), $Pd_2(dba)_3$ (62 mg, 0.068 mmol), 2-bicyclohexylphosphine-2',6'-diisopropoxybiphenyl (65 mg, 0.14 mmol) and $CS_2CO_3$ (472 mg, 1.45 mmol) were dissolved in toluene (5 mL), and the mixture was reacted at 110°C for 17 h. The resulting mixture was filtered through a celite pad. The filtrate

was concentrated under reduced pressure, and the concentrated solution was separated by silica gel column chromatography (eluent: PE/EtOAc (v/v) = 7/1) to give yellow oil (300 mg, 94%).
MS (ESI, pos.ion) m/z:462.1 [M+H]+.

Step 7: Synthesis of **4-((2*S*)-2-((difluoromethoxy)methyl)-5-(4-(trifluoromethyl) phenyl)piperidin-1-yl)-2-fluorobenzoic acid**

Methyl 4-((2S)-2-((difluoromethoxy)methyl)-5-(4-(trifluoromethyl)phenyl)piperidin-1-yl)-2-fluorobenzoate (300 mg, 0.65 mmol) and LiOH·$H_2O$ (278 mg, 6.61 mmol) were dissolved in THF/MeOH/$H_2O$ (2 mL/1 mL/0.8 mL), and the mixture was reacted at room temperature for 13 h. 1.0 M HCl solution (5 mL) was added to the reaction solution to adjust the pH to about 4. The resulting mixture was extracted with EtOAc (20 mL × 2), dried over anhydrous $Na_2SO_4$, concentrated under reduced pressure, and the concentrated solution was separated by silica gel column chromatography (eluent: PE/EtOAc (v/v) = 3/1) to give yellow oil (272 mg, 94 %), which was further separated by preparative chiral resolution (chiral OA column, *i*-PrOH/DCM = 20/1) to give yellow solids A: 4-((2*S*,5*S*)-2-((difluoromethoxy)methyl)-5-(4-(trifluoromethyl)phenyl)piperidin-1-yl)-2-fluorobenzo ic acid (130 mg) and B: 4-((2*S*,5*R*)-2-((difluoromethoxy)methyl)-5-(4-(trifluoromethyl) phenyl)piperidin-1-yl)-2-fluorobenzoic acid (60 mg).
MS (ESI, pos.ion) m/z:448.1 [M+H]+.

Step 8: Synthesis of ***N*-((*S*)-2-cyano-1-(4-(ethylsulfonyl)phenyl)ethyl)-4-((2*S*,5*S*)-2 -((difluoromethoxy)methyl)-5-(4-(trifluoromethyl)phenyl)piperidin-1-yl)-2-fluorobenzamide / *N*-((*S*)-2-cyano-1-(4-(ethylsulfonyl)phenyl)ethyl)-4-((2*S*,5*R*)-2-((difluoromethoxy)methyl)-5-(4-(trifluoromethyl)phenyl)piperidin-1-yl)-2-fluorobenzamide**

4-((2*S*,5*S*)-2-((difluoromethoxy)methyl)-5-(4-(trifluoromethyl)phenyl)piperidin-1-yl)-2-fluorobenzoic acid (130 mg, 0.29 mmol), (*S*)-3-amino-3-(4-(ethylsulfonyl)phenyl)propionitrile hydrochloride (99 mg, 0.36 mmol), EDCI (169 mg, 0.88 mmol) and HOBT (136 mg, 1.01 mmol) were dissolved in DCM (5 mL), then TEA (0.24 mL, 1.70 mmol) was added and the mixture was stirred at room temperature for 20 h. The reaction solution was added with DCM (50 mL), washed with saturated NaCl solution (20 mL × 2), dried over anhydrous $Na_2SO_4$, concentrated under reduced pressure, and the concentrated solution was separated by silica gel column chromatography (eluent: PE/EtOAc (v/v) = 1/2) to give compound 111-1 as a white solid (160 mg, 82%).
Using 4-((2S,5*R*)-2-((difluoromethoxy)methyl)-5-(4-(trifluoromethyl)phenyl) piperidin-1-yl)-2-fluorobenzoic acid as raw material, compound 111-2 was prepared by the above method as a white solid (142 mg, 70%).

MS (ESI, pos.ion) m/z: 668.1 [M+H]+.
**111-1:**
$^1$H NMR (400 MHz, DMSO-$d_6$) δ (ppm): 8.72 (dd, *J* = 8.0, 3.3 Hz, 1H), 7.90 (d, *J* = 8.2 Hz, 2H), 7.74 (d, *J* = 8.2 Hz, 2H), 7.64 (d, *J* = 8.1 Hz, 2H), 7.57 (t, *J* = 9.0 Hz, 1H), 7.52 (d, *J* = 8.0 Hz, 2H), 6.91 - 6.84 (m, 2H), 6.67 (t, *J* = 67.6 Hz, 1H), 5.52 (q, *J* = 7.7 Hz, 1H), 4.28 (s, 1H), 4.11 - 4.03 (m, 1H), 3.95 (dd, *J* = 10.1, 5.4 Hz, 1H), 3.91 - 3.82 (m, 1H), 3.47 (dd, *J* = 13.1, 4.1 Hz, 1H), 3.33 - 3.26 (m, 3H), 3.17 (d, *J*= 7.6 Hz, 2H), 2.19 (dt, *J* = 12.5, 5.6 Hz, 1H), 1.86 - 1.68 (m, 3H), 1.11 (t, *J* = 7.3 Hz, 3H).
**111-2:**
$^1$H NMR (400 MHz, DMSO-$d_6$) δ (ppm): 8.68 (dd, *J* = 7.9, 3.7 Hz, 1H), 7.90 (d, *J* = 8.2 Hz, 2H), 7.77 - 7.69 (m, 4H), 7.66 (d, *J* = 8.2 Hz, 2H), 7.55 (t, *J* = 9.1 Hz, 1H), 6.85 (t, *J* = 10.3 Hz, 2H), 6.70 (t, *J* = 67.6 Hz, 1H), 5.51 (dd, *J* = 15.1, 7.8 Hz, 1H), 4.43 (d, *J* = 4.1 Hz, 1H), 4.20 - 4.05 (m, 2H), 3.74 (dd, *J* = 12.6, 2.9 Hz, 1H), 3.30 - 3.26 (m, 2H), 3.16 (dd, *J* = 15.1, 8.4 Hz, 3H), 2.91 (t, *J* = 10.9 Hz, 1H), 2.02 - 1.91 (m, 2H), 1.85 (dd, *J* = 20.6, 10.6 Hz, 2H), 1.10 (t, *J* = 7.3 Hz, 3H).

**Example 112**

**112-1 *N*-((*S*)-2-cyano-1-(4-(ethylsulfonyl)phenyl)ethyl)-4-((2*S*,5*S*)-5-(2,2-difluorobenzo[1,3]dioxol -5-yl)-2-((difluoromethoxy)methyl)piperidin-1-yl)benzamide**

**112-2 *N*-((*S*)-2-cyano-1-(4-(ethylsulfonyl)phenyl)ethyl)-4-((2*S*,5*R*)-5-(2,2-difluorobenzo[1,3]dioxol-5-yl)-2-((difluoromethoxy)methyl)piperidin-1-yl)benzamide**

112-1: 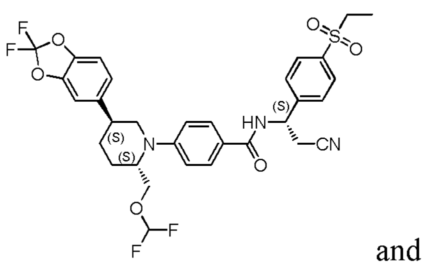 and 112-2: 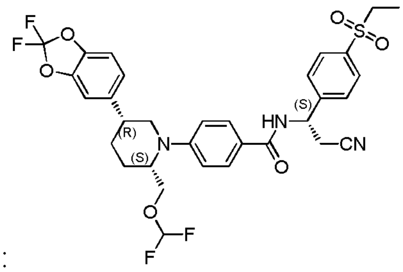

The raw material (4-(trifluoromethyl)phenyl)boronic acid in step 2 of Example 111 was replaced with (2,2-difluoro-1,3-benzodioxol-5-yl)boronic acid, and the raw material methyl 2-fluoro-4-iodo-benzoate in step 6 was replaced with methyl 4-iodobenzoate. They were used to prepare the title compounds with the intermediate *tert-butyl* (2S)-5-bromo-2-(hydroxymethyl)-3,4-dihydro-2*H*-pyridine-1-carboxylate according to the methods of step 2 to step 8 in Example 111. All of the title compounds were white solids, 112-1: (220 mg, 87%); 112- 2: (102 mg, 80%).

MS (ESI, pos.ion) m/z: 662.2 $[M+H]^+$.

**112-1:**

$^1$H NMR (400 MHz, DMSO-$d_6$) δ (ppm): 8.99 (d, *J* = 8.1 Hz, 1H), 7.89 (d, *J* = 8.1 Hz, 2H), 7.82 (d, *J* = 8.5 Hz, 2H), 7.73 (d, *J* = 8.0 Hz, 2H), 7.35 (s, 1H), 7.29 (d, *J* = 8.2 Hz, 1H), 7.14 (d, *J* = 8.3 Hz, 1H), 7.08 (d, *J* = 8.6 Hz, 2H), 6.66 (t, *J* = 75.9 Hz, 1H), 5.52 (dd, *J* = 15.0, 7.9 Hz, 1H), 4.13 (d, *J* = 0.9 Hz, 1H), 4.01 (dd, *J* = 17.3, 7.7 Hz, 1H), 3.87 (dd, *J* = 9.8, 4.3 Hz, 1H), 3.67 (dd, *J* = 12.5, 4.2 Hz, 1H), 3.41 (s, 1H), 3.28 (dd, *J* = 14.6, 7.2 Hz, 2H), 3.23 - 3.07 (m, 3H), 2.12 (dd, *J* = 13.8, 9.3 Hz, 1H), 1.88 (dd, *J* = 12.6, 8.3 Hz, 1H), 1.82 - 1.64 (m, 2H), 1.09 (t, *J* = 7.2 Hz, 3H).

**112-2:**

$^1$H NMR (400 MHz, DMSO-$d_6$) δ (ppm): 8.94 (d, *J* = 7.9 Hz, 1H), 7.89 (d, *J* = 7.8 Hz, 2H), 7.78 (d, *J* = 8.3 Hz, 2H), 7.72 (d, *J* = 7.8 Hz, 2H), 7.58 (s, 1H), 7.36 (d, *J* = 8.1 Hz, 1H), 7.25 (d, *J* = 8.2 Hz, 1H), 7.02 (d, *J* = 8.4 Hz, 2H), 6.68 (t, *J* = 76.0 Hz, 1H), 5.51 (dd, *J* = 14.9, 7.6 Hz, 1H), 4.40 (s, 1H), 4.18 (t, *J* = 8.9 Hz, 1H), 4.06 - 3.97 (m, 1H), 3.67 (d, *J* = 11.0 Hz, 1H), 3.28 (dd, *J* = 14.6, 7.2 Hz, 2H), 3.15 (d, *J* = 6.7 Hz, 2H), 3.12 - 3.04 (m, 1H), 2.86 (s, 1H), 2.00 - 1.89 (m, 2H), 1.80 (dd, *J* = 36.3, 13.4 Hz, 2H), 1.09 (t, *J* = 7.2 Hz, 3H).

**Example 113**

**113-1: *N*-((*S*)-2-cyano-1-(4-(ethylsulfonyl)phenyl)ethyl)-4-((2*S*,5*S*)-2-((difluoromethoxy)methyl)-5-(4-(trifluoromethyl)phenyl)piperidin-1-yl)-3-fluorobenzamide**

**113-2: *N*-((*S*)-2-cyano-1-(4-(ethylsulfonyl)phenyl)ethyl)-4-((2*S*,5*R*)-2-((difluoromethoxy)methyl) -5-(4-(trifluoromethyl)phenyl)piperidin-1-yl)-3-fluorobenzamide**

113-1: 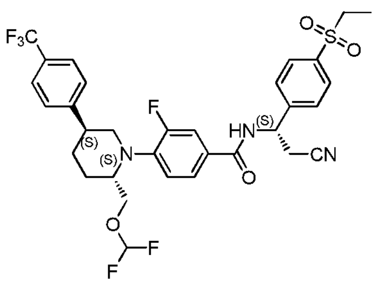 and 113-2: 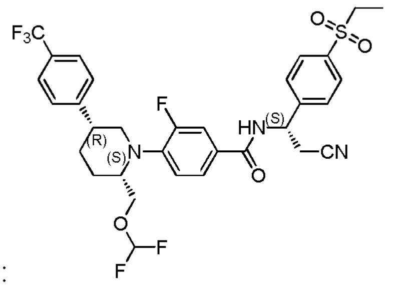

The raw material methyl 2-fluoro-4-iodo-benzoate in step 6 of Example 111 was replaced with methyl 3-fluoro-4-iodo-benzoate, which was used to prepare the title compounds with the intermediate (2*S*)-2-((difluoromethoxy)methyl)-5-(4-(trifluoromethyl)phenyl)pipenyl)piperidine according to the methods of step 6 to step 8 in Example 111. All of the title compounds were white solids, 113-1: (52 mg, 54%); 113-2: (96 mg, 71%).

MS (ESI, pos.ion) m/z: 668.2 $[M+H]^+$.

**113-1:**

$^1$H NMR (400 MHz, DMSO-$d_6$) δ (ppm): 9.25 (d, *J* = 8.0 Hz, 1H), 7.90 (d, *J* = 8.2 Hz, 2H), 7.77 - 7.68 (m, 4H), 7.65 (d, *J* = 8.0 Hz, 2H), 7.54 (d, *J* = 8.0 Hz, 2H), 7.45 (t, *J* = 8.4 Hz, 1H), 6.55 (t, *J* = 75.8 Hz, 1H), 5.52 (dd, *J* = 14.6, 7.8 Hz, 1H), 3.85 (dd, *J* = 10.1, 3.9 Hz, 1H), 3.74 (dd, *J* = 10.1, 6.1 Hz, 1H), 3.57 (s, 1H), 3.30 (d, *J* = 7.3 Hz, 2H), 3.26 (s, 1H), 3.20 - 3.12 (m, 2H), 3.12 - 3.02 (m, 2H), 2.03 (t, *J* = 13.9 Hz, 2H), 1.80 - 1.61 (m, 2H), 1.09 (t, *J* = 7.3 Hz, 3H).

**113-2:**

$^1$H NMR (400 MHz, DMSO-$d_6$) δ (ppm): 9.12 (d, *J* = 8.0 Hz, 1H), 7.89 (d, *J* = 8.1 Hz, 2H), 7.72 (d, *J* = 8.4 Hz, 3H), 7.70 - 7.63 (m, 5H), 7.14 (t, *J* = 8.7 Hz, 1H), 6.59 (t, *J* = 75.9 Hz, 1H), 5.51 (dd, *J* = 14.7, 7.9 Hz, 1H), 4.14 (d, *J* = 6.2 Hz, 2H), 4.10 (s, 1H), 3.41 (s, 1H), 3.28 (dd, *J* = 14.6, 7.2 Hz, 3H), 3.16 (t, *J* = 10.6 Hz, 2H), 3.06 - 2.97 (m, 1H), 2.02 - 1.89 (m, 3H), 1.80 (d, *J* = 9.3 Hz, 1H), 1.09 (t, *J* = 7.3 Hz, 3H).:

**Example 114**

**114-1: *N*-((*S*)-2-cyano-1-(4-(ethylsulfonyl)phenyl)ethyl)-4-((2*S*,5*S*)-2-((difluoromethoxy)methyl) -5-(4-fluorophenyl)piperidin-1-yl)benzamide**

**114-2: *N*-((*S*)-2-cyano-1-(4-(ethylsulfonyl)phenyl)ethyl)-4-((2*S*,5*R*)-2-((difluoromethoxy) methyl)-5-(4-fluorophenyl)piperidin-1-yl)benzamide**

114-1: 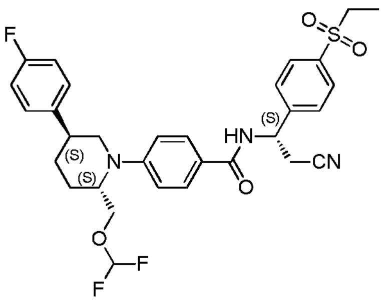 and 114-2: 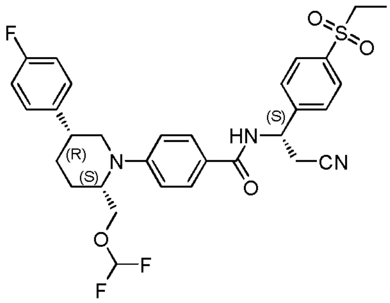

The raw material (4-(trifluoromethyl)phenyl)boronic acid in step 2 of Example 111 was replaced with 4-fluorophenylboronic acid, and the raw material methyl 2-fluoro-4-iodo-benzoate in step 6 was replaced with methyl 4-iodobenzoate. They were used to prepare the title compounds with the intermediate tert-butyl (2S)-5-bromo-2-(hydroxymethyl)-3,4-dihydro-2*H*-pyridine-1-carboxylate according to the methods of step 2 to step 8 in Example 111. All of the title compounds were white solids, 114-1: (127 mg, 74%); 114- 2: (100 mg, 70%).

MS (ESI, pos.ion) m/z: 600.3 $[M+H]^+$.

**114-1:**

$^1$H NMR (400 MHz, DMSO-$d_6$) δ (ppm): 8.98 (d, *J* = 8.2 Hz, 1H), 7.89 (d, *J* = 8.2 Hz, 2H), 7.81 (d, *J* = 8.6 Hz, 2H), 7.72 (d, *J* = 8.2 Hz, 2H), 7.38 - 7.27 (m, 2H), 7.08 (dd, _*J* = 15.5, 8.6 Hz, 4H), 6.66 (t, *J* = 75.9 Hz, 1H), 5.51 (dd, *J* = 15.0, 8.0 Hz, 1H), 4.14 (s, 1H), 4.05 - 3.95 (m, 1H), 3.87 (dd, *J*= 10.0, 4.5 Hz, 1H), 3.68 (dd, *J* = 12.7, 4.4 Hz, 1H), 3.43 (s, 1H), 3.30 - 3.25 (m, 2H), 3.18 - 3.11 (m, 3H), 2.11 (s, 1H), 1.86 (s, 1H), 1.72 (d, *J* = 9.3 Hz, 2H), 1.09 (t, *J* = 7.3 Hz, 3H).

**114-2:**

$^1$H NMR (400 MHz, DMSO-$d_6$) δ (ppm): 8.94 (d, *J* = 8.2 Hz, 1H), 7.89 (d, *J* = 8.2 Hz, 2H), 7.78 (d, *J* = 8.8 Hz, 2H), 7.72 (d, *J* = 8.2 Hz, 2H), 7.45 (dd, *J* = 8.2, 5.8 Hz, 2H), 7.17 (t, *J* = 8.8 Hz, 2H), 7.01 (d, *J* = 8.8 Hz, 2H), 6.68 (t, *J* = 76.0 Hz, 1H), 5.51 (dd, *J* = 15.2, 7.9 Hz, 1H), 4.41 (s, 1H), 4.20 - 4.13 (m, 1H), 4.00 (dd, *J*= 10.1, 6.3 Hz, 1H), 3.66 (d, *J*= 9.6 Hz, 1H), 3.30 - 3.25 (m, 2H), 3.17 - 3.12 (m, 2H), 3.07 (t, *J* = 12.4 Hz, 1H), 2.82 (t, *J* = 10.9 Hz, 1H), 1.97 (d, *J* = 10.8 Hz, 1H), 1.87 (d, *J*= 9.1 Hz, 2H), 1.76 (d, *J*= 9.9 Hz, 1H), 1.09 (t, *J*= 7.3 Hz, 3H).

**Example 115**

**115-1: *N*-((*S*)-2-cyano-1-(4-(ethylsulfonyl)phenyl)ethyl)-4-((2*S*,5*R*)-2-((difluoromethoxy) methyl)-5-phenylpiperidin-1-yl)benzamide**

**115-2: *N*-((*S*)-2-cyano-1-(4-(ethylsulfonyl)phenyl)ethyl)-4-((2*S*,5*S*)-2-((difluoromethoxy)methyl) -5-phenylpiperidin-1-yl)benzamide**

115-1: 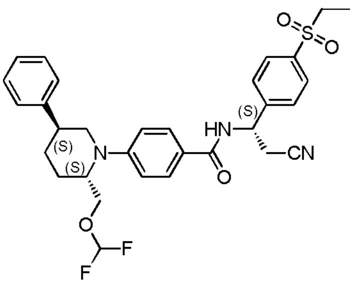 and 115-2: 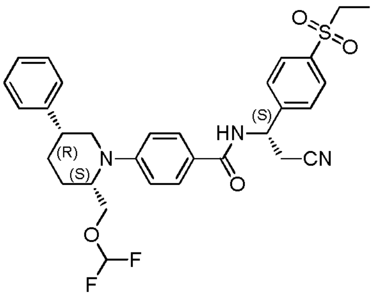

The raw material (4-(trifluoromethyl)phenyl)boronic acid in step 2 of Example 111 was replaced with 4,4,5,5-tetramethyl-2-phenyl-1,3,2-dioxaborolane, and the raw material methyl 2-fluoro-4-iodo-benzoate in step 6 was replaced with methyl 4-iodobenzoate. They were used to prepare the title compounds with the intermediate *tert-butyl* (2*S*)-5-bromo-2-(hydroxymethyl)-3,4-dihydro-2*H*-pyridine-1-carboxylate according to the methods of step 2 to step 8 in Example 111. The title compounds were 115-1: a yellow solid (63 mg, 63%) and 115-2: a white solid (36 mg, 36%).

MS (ESI, pos.ion) m/z: 582.3 $[M+H]^+$.

**115-1:**

$^1$H NMR (400 MHz, DMSO-$d_6$) δ (ppm): 8.94 (d, *J* = 8.2 Hz, 1H), 7.89 (d, *J* = 8.2 Hz, 2H), 7.78 (d, *J* = 8.7 Hz, 2H), 7.72 (d, *J* = 8.2 Hz, 2H), 7.40 (d, *J* = 7.3 Hz, 2H), 7.35 (t, *J* = 7.5 Hz, 2H), 7.25 (t, *J* = 7.1 Hz, 1H), 7.01 (d, *J* = 8.8 Hz, 2H), 6.69 (t, *J* = 76.0 Hz, 1H), 5.50 (dd, *J* = 15.3, 8.0 Hz, 1H), 4.41 (s, 1H), 4.21 - 4.12 (m, 1H), 4.01 (dd, *J* = 10.1, 6.2 Hz, 1H), 3.67 (d, *J* = 9.8 Hz, 1H), 3.29 - 3.25 (m, 2H), 3.14 (d, *J* = 9.3 Hz, 2H), 3.08 (d, *J* = 12.4 Hz, 1H), 2.83 - 2.75 (m, 1H), 2.04 - 1.92 (m, 2H), 1.91 - 1.85 (m, 1H), 1.81 - 1.74 (m, 1H), 1.09 (t, *J* = 7.3 Hz, 3H).

**115-2:**

$^1$H NMR (400 MHz, DMSO-$d_6$) δ (ppm): 8.98 (d, *J* = 8.1 Hz, 1H), 7.89 (d, *J* = 8.2 Hz, 2H), 7.80 (d, *J* = 8.5 Hz, 2H), 7.72 (d, *J* = 8.2 Hz, 2H), 7.28 (dd, *J* = 18.1, 7.4 Hz, 4H), 7.17 (t, *J* = 6.8 Hz, 1H), 7.06 (d, *J* = 8.7 Hz, 2H), 6.66 (t, *J* = 75.9 Hz, 1H), 5.51 (dd, *J* = 14.9, 7.8 Hz, 1H), 4.13 (s, 1H), 4.04 - 3.97 (m, 1H), 3.88 (dd, *J* = 9.9, 4.6 Hz, 1H), 3.70 (dd, *J* = 12.7, 4.4 Hz, 1H), 3.28 (dd, *J* = 14.7, 7.3 Hz, 3H), 3.18 - 3.10 (m, 3H), 2.12 (s, 1H), 1.88 (d, *J* = 5.2 Hz, 1H), 1.74 (s, 2H), 1.09 (t, *J* = 7.3 Hz, 3H).

**Example 116**

**116-1: Synthesis of 4-((2*S*,5*S*)-5-(4-chlorophenyl)-2-((difluoromethoxy)methyl) piperidin-1-yl)-*N*-((*S*)-2-cyano-1-(4-(ethylsulfonyl)phenyl)ethyl)benzamide**

**116-2: Synthesis of 4-((2*S*,5*R*)-5-(4-chlorophenyl)-2-((difluoromethoxy)methyl) piperidin-1-yl)-*N*-((*S*)-2-cyano-1-(4-(ethylsulfonyl)phenyl)ethyl)benzamide**

116-1: 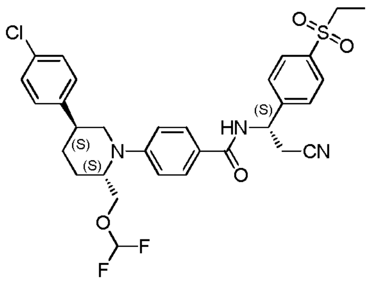 and 116-2: 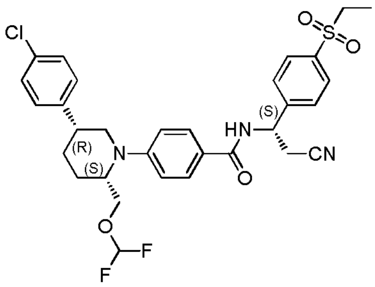

The raw material (4-(trifluoromethyl)phenyl)boronic acid in step 2 of Example 111 was replaced with 4-chlorophenylboronic acid, and the raw material methyl 2-fluoro-4-iodo-benzoate in step 6 was replaced with methyl 4-iodobenzoate. They were used to prepare the title compounds with the intermediate tert-butyl (2S)-5-bromo-2-(hydroxymethyl)-3,4-dihydro-2*H*-pyridine-1-carboxylate according to the methods of step 2 to step 8 in Example 111. All of the title compounds were white solids, 116-1: (113 mg, 72%); 116- 2: (94 mg, 64%).

MS (ESI, pos.ion) m/z: 617.2 $[M+H]^+$.

**116-1:**

$^1$H NMR (400 MHz, DMSO-$d_6$) δ (ppm): 8.98 (d, *J* = 8.2 Hz, 1H), 7.89 (d, *J* = 8.1 Hz, 2H), 7.81 (d, *J* = 8.5 Hz, 2H), 7.72 (d, *J* = 8.1 Hz, 2H), 7.32 (s, 4H), 7.06 (d, *J* = 8.7 Hz, 2H), 6.66 (t, *J* = 75.9 Hz, 1H), 5.51 (dd, *J* = 15.2, 8.0 Hz, 1H), 4.14 (s, 1H), 4.01 (dd, *J* = 16.3, 6.9 Hz, 1H), 3.87 (dd, *J* = 10.0, 4.6 Hz, 1H), 3.69 (dd, *J* = 12.7, 4.3 Hz, 1H), 3.43 (s, 1H), 3.30 - 3.25 (m, 2H), 3.19 - 3.12 (m, 3H), 2.18 - 2.06 (m, 1H), 1.91 - 1.81 (m, 1H), 1.77- 1.67 (m, 2H), 1.09 (t, *J* = 7.3 Hz, 3H).

**116-2:**

$^1$H NMR (400 MHz, DMSO-$d_6$) δ (ppm): 8.94 (d, *J* = 8.2 Hz, 1H), 7.89 (d, *J* = 8.2 Hz, 2H), 7.78 (d, *J* = 8.7 Hz, 2H), 7.71 (d, *J* = 8.2 Hz, 2H), 7.45 (d, *J* = 8.4 Hz, 2H), 7.40 (d, *J* = 8.4 Hz, 2H), 7.01 (d, *J* = 8.8 Hz, 2H), 6.68 (t, *J* = 76.0 Hz, 1H), 5.50 (dd, *J* = 15.3, 8.0 Hz, 1H), 4.40 (s, 1H), 4.20 - 4.12 (m, 1H), 4.00 (dd, *J* = 10.2, 6.3 Hz, 1H), 3.66 (d, *J* = 9.7 Hz, 1H), 3.28 (dd, *J* = 14.5, 7.1 Hz, 2H), 3.18 - 3.12 (m, 2H), 3.07 (t, *J* = 12.4 Hz, 1H), 2.87 - 2.77 (m, 1H), 2.03 - 1.89 (m, 2H), 1.87 (d, *J* = 8.9 Hz, 1H), 1.76 (d, *J* = 9.8 Hz, 1H), 1.09 (t, *J* = 7.3 Hz, 3H).

**Example 117 *N*-((*S*)-2-cyano-1-(4-(ethylsulfonyl)phenyl)ethyl)-4-((3'*S*,6'*S*)-6'-((difluoromethoxy)methyl)-4-(trifluoromethyl)-(1,3'-bipiperidin)-1'-yl)benzamide / *N*-((*S*)-2-cyano-1-(4-(ethylsulfonyl)phenyl)ethyl)-4-((3'*R*,6'*S*)-6'-((difluoromethoxy)methyl) -4-(trifluoromethyl)-(1,3'-bipiperidin )-1'-yl)benzamide**

117-1: 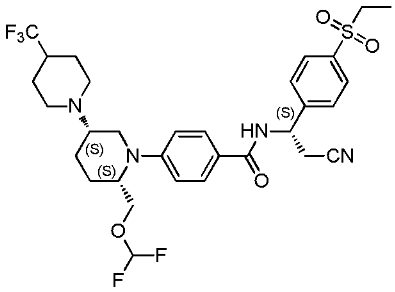 or 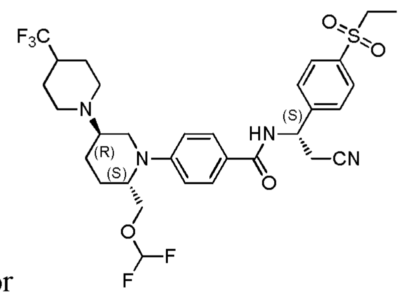

117-2: 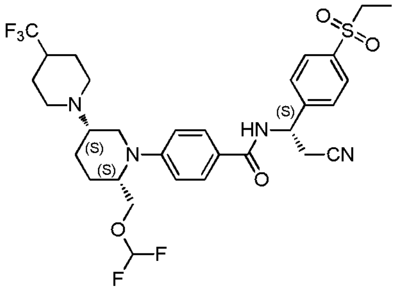 or 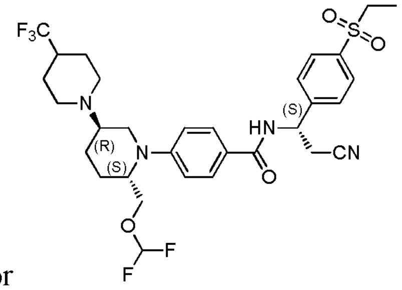

**Step 1: Synthesis of (2*S*,5*S*)-1-*tert*-butyl2-methyl 5-(benzyloxy)piperidine-1,2-dicarboxylate**

(2S,5*S*)-1-*tert*-butyl 2-methyl 5-hydroxypiperidine-1,2-dicarboxylate (18.1 g, 69.80 mmol) was dissolved in DCM/cyclohexane (20 mL/40 mL), and benzyltrichloroacetimide (24 mL, 129.20 mmol) was added under an ice bath, then TBSOTf (2 mL) was added dropwise. The mixtrue was reacted at room temperature for 48 h. The reaction solution was added with saturated $NaHCO_3$ solution (150 mL), extracted with EtOAc (200 mL × 2), dried over anhydrous $Na_2SO_4$, concentrated under reduced pressure, and the concentrated solution was separated by silica gel column chromatography (eluent: PE/EtOAc (v/v) = 6/1) to give yellow oil (24 g, 98.4%).
MS (ESI, pos.ion) m/z: 250.2 $[M+H-100]^+$.

**Step 2: Synthesis of *tert-butyl* (2*S*,5*S*)-5-(benzyloxy)-2-(hydroxymethyl) piperidine-1-carboxylate**

To a solution of (2*S*,5*S*)-1-*tert*-butyl 2-methyl 5-(benzyloxy)piperidine-1,2-dicarboxylate (20 g, 57.24 mmol) in THF (40 mL) ) was slowly added $LiBH_4$ (2.50 g, 110.00 mmol) in batches under an ice bath, and the mixture was reacted at room temperature for 17 h. The reaction solution was added with saturated $NaHCO_3$ solution (200 mL), extracted with EtOAc (300 mL × 2), dried over anhydrous $Na_2SO_4$, concentrated under reduced pressure, and the concentrated solution was separated by silica gel column chromatography (eluent: PE/EtOAc (v/v) = 3/1) to give colorless oil (13 g, 71%).
MS (ESI, pos.ion) m/z: 344.2 $[M+Na]^+$.

Step 3: Synthesis of ***tert-butyl* (2*S*,5*S*)-5-(benzyloxy)-2-((difluoromethoxy) methyl)piperidine-1-carboxylate**

To a solution of *tert-butyl* (2*S*,5*S*)-5-(benzyloxy)-2-(hydroxymethyl) piperidine-1-carboxylate (13 g, 40.45 mmol) in DCM/$H_2O$ (36 mL/ 36 mL) was added KOAc (23.90 g, 244.00 mmol), then (bromo(difluoro)methyl)-trimethyl-silane (25 mL, 160.80 mmol) was slowly added dropwise under an ice bath, and the mixture was stirred at room temperature for 48 h. Saturated $NaHCO_3$ solution (150 mL) was added to the reaction solution to quench the reaction. The resulting mixture was extracted with DCM (200 mL × 2), dried over anhydrous $Na_2SO_4$, concentrated under reduced pressure, and the concentrated solution was separated by silica gel column chromatography (eluent: PE/EtOAc (v/v) = 6/1) to give colorless oil (12 g, 80%).
MS (ESI, pos.ion) m/z: 272.2 $[M+H-100]^+$.

**Step 4: Synthesis of (2*S*,5*S*)-5-(benzyloxy)-2-((difluoromethoxy)methyl)piperidine**

To a solution of *tert-butyl* (2*S*,5*S*)-5-(benzyloxy)-2-((difluoromethoxy) methyl)piperidine -1-carboxylate (12 g, 32.31 mmol) in EtOAc (10 mL) was slowly added dropwise HCl in EtOAc (40 mL, 120 mmol, 3 mol/L) under an ice bath, and the mixture was reacted at room temperature for 13 h . The mixture was distilled under reduced pressure to remove part of HCl and EtOAc, then saturated $NaHCO_3$ solution (150 mL) was added to adjust the pH of the system to about 8. The resulting mixture was extracted with DCM (200 mL × 2), dried over anhydrous $Na_2SO_4$, concentrated under reduced pressure to give yellow oil (8.2 g, 94%).
MS (ESI, pos.ion) m/z: 272.1 $[M+H]^+$.

**Step 5: Synthesis of methyl 4-((2*S*,5*S*)-5-(benzyloxy)-2-((difluoromethoxy) methyl)piperidin-1-yl)benzoate**

Under nitrogen protection, (2*S*,5*S*)-5-(benzyloxy)-2-((difluoromethoxy) methyl)piperidine (8.2 g, 30.00 mmol), methyl 4-iodobenzoate (12.10 g, 46.20 mmol), $Pd_2(dba)_3$ (2.75 g, 3.00 mmol), 2-bicyclohexylphosphine-2',6'-diisopropoxybiphenyl (2.80 g, 6.00 mmol) and $CS_2CO_3$ (20.20 g, 62.00 mmol) were dissolved in toluene (50 mL), and the mixture was reacted at 110°C for 16 h. The resulting mixture was filtered through a celite pad. The filtrate was concentrated under reduced pressure, and the concentrated solution was separated by silica gel column chromatography (eluent: PE/EtOAc (v/v) = 5/1) to give yellow oil (11.5 g, 94%).
MS (ESI, pos.ion) m/z: 406.1 $[M+H]^+$.

**Step 6: Synthesis of methyl 4-((2*S*,5*S*)-2-((difluoromethoxy)methyl) -5-hydroxypiperidin-1-yl)benzoate**

Under hydrogen protection, methyl 4-((2*S*,5*S*)-5-(benzyloxy)-2-((difluoromethoxy) methyl) piperidin-1-yl)benzoate (5 g, 12.33 mmol) and $Pd(OH)_2/C$ (5.20 g, 3.70 mmol) were dissolved in MeOH (20 mL), and the mixture was reacted at 60°C for 36 h. The resulting mixture was filtered through a celite pad. The filtrate was concentrated under reduced pressure, and the concentrated solution was separated by silica gel column chromatography (eluent: PE/EtOAc (v/v) = 3/1) to give colorless oil (2.2 g, 57%).
MS (ESI, pos.ion) m/z: 316.1 $[M+H]^+$.

**Step 7: Synthesis of methyl 4-((2*S*,5*S*)-2-((difluoromethoxy)methyl)-5-((methylsulfonyl)oxy) piperidine-1-yl)benzoate**

To a solution of methyl 4-((2*S*,5*S*)-2-((difluoromethoxy)methyl)-5-hydroxypiperidine-1-yl)benzoate (308 mg, 0.98 mmol) in DCM ( 4 mL) were added TEA (0.27 mL, 1.90 mmol) and MsCl (0.08 mL, 1.00 mmol) successively. The mixture was reacted at room temperature for 5 h. The reaction solution was added with saturated $NaHCO_3$ solution (30 mL) to adjust the pH to about 8. The resulting mixture was extracted with DCM (50 mL × 2), dried over anhydrous $Na_2SO_4$, concentrated under reduced pressure, and the concentrated solution was separated by silica gel column chromatography (eluent: PE/EtOAc (v/v) = 3/1) to give yellow oil (248 mg, 65%).
MS (ESI, pos.ion) m/z: 394.1 $[M+H]^+$.

**Step 8: Synthesis of methyl 4-((6'S)-6'-((difluoromethoxy)methyl)-4-(trifluoromethyl) -(1,3'-dipiperidin)-1'-yl)benzoate**

Methyl 4-((2*S*,5*S*)-2-((difluoromethoxy)methyl)-5-((methylsulfonyl)oxy) piperidin-1-yl)benzoate (330 mg, 0.84 mmol), 4-(trifluoromethyl)piperidine (78 mg, 0.52 mmol), NaI (78 mg, 0.52 mmol) and $K_2CO_3$ (233 mg, 1.69 mmol) were dissolved in ACN (8 mL), and the mixture was reacted under microwave at 120 °C for 3 h. The mixture was concentrated under reduced pressure. The residue was added with saturated $NaHCO_3$ solution (40 mL), extracted with DCM (60 mL × 2), dried over anhydrous $Na_2SO_4$, concentrated under reduced pressure, and the concentrated solution was separated by silica gel column chromatography (eluent: PE/EtOAc (v/v) = 3/1) to give yellow oil (111 mg, 29%).
MS (ESI, pos.ion) m/z: 451.1 $[M+H]^+$.

**Step 9: Synthesis of 4-((6'S)-6'-((difluoromethoxy)methyl)-4-(trifluoromethyl) -(1,3'-dipiperidin) -1'-yl)benzoic acid**

Methyl 4-((6'*S*)-6'-((difluoromethoxy)methyl)-4-(trifluoromethyl)-(1,3'-dipiperidin) -1'-yl)benzoate (170 mg, 0.38 mmol) and LiOH·$H_2O$ (193 mg, 4.60 mmol) were dissolved in THF/MeOH/$H_2O$ (2 mL/1 mL/0.7 mL), and the mixture was reacted at room temperature for 48 h. 1.0 M HCl solution (5 mL) was added to the reaction solution to adjust the pH to about 4. The resulting mixture was extracted with EtOAc (20 mL × 2), dried over anhydrous $Na_2SO_4$, concentrated under reduced pressure, and the concentrated solution was separated by silica gel column chromatography (eluent: PE/EtOAc (v/v) = 2/1) to give yellow oil (132 mg, 80%),
MS (ESI, pos.ion) m/z: 437.1 $[M+H]^+$.

**Step 10: Synthesis of *N*-((*S*)-2-cyano-1-(4-(ethylsulfonyl)phenyl)ethyl)-4-((3'*S*,6'*S*)-6'-((difluoromethoxy)methyl)-4-(trifluoromethyl)-(1,3'-bipiperidin)-1'-yl)benzamide / *N*-((*S*)-2-cyano-1-(4-(ethylsulfonyl)phenyl)ethyl)-4-((3'*R*,6'*S*)-6'-((difluoromethoxy)methyl)-4-( trifluoromethyl)-(1,3'-bipiperidin)-1'-yl)benzamide**

**4-((6'*S*)-6'-((Difluoromethoxy)methyl)-4-(trifluoromethyl)-(1,3'-dipiperidin)-1'-yl)benz** oic acid (132 mg, 0.30 mmol), (*S*)-3-amino-3-(4-(ethylsulfonyl)phenyl)propionitrile hydrochloride (115 mg, 0.42 mmol), EDCI (219 mg, 1.14 mmol) and HOBT (137 mg, 1.01 mmol) were dissolved in DCM (5 mL), then TEA (0.3 mL, 2.00 mmol) was added and the mixture was reacted at room temperature for 12 h. The reaction solution was added with DCM (50 mL), washed with saturated $NaHCO_3$ (20 mL × 2) and saturated NaCl (20 mL), dried over anhydrous $Na_2SO_4$, concentrated under reduced pressure, and the concentrated solution was separated by silica gel column chromatography (eluent: PE/EtOAc (v/v) = 1/2) to give compound 117-1 as a white solid (71 mg, 36 %), and compound 117-2 as a yellow solid (21 mg, 11 %).

MS (ESI, pos.ion) m/z: 657.4 $[M+H]^+$.
**117-1:**
$^1$H NMR (400 MHz, DMSO-$d_6$) δ (ppm): 8.87 (d, *J* = 8.2 Hz, 1H), 7.89 (d, *J* = 8.1 Hz, 2H), 7.79 (d, *J* = 8.5 Hz, 2H), 7.72 (d, *J* = 8.1 Hz, 2H), 6.68 (t, *J* = 42.4 Hz, 3H), 5.50 (dd, *J* = 15.3, 8.3 Hz, 1H), 4.04 (s, 1H), 3.97-3.83 (m, 3H), 3.31- 3.25 (m, 2H), 3.20-3.06 (m, 3H), 2.87 (d, *J*= 11.1 Hz, 1H), 2.48 (s, 1H), 2.38 (dd, *J*= 13.0, 8.0 Hz, 1H), 2.25 (d, *J*= 9.0 Hz, 1H), 2.11-1.90 (m, 6H), 1.77 (t, *J* = 11.4 Hz, 2H), 1.54-1.41 (m, 2H), 1.10 (t, *J* = 7.3 Hz, 3H).
**117-2:**
$^1$H NMR (400 MHz, DMSO-$d_6$) δ (ppm): 8.92 (d, *J* = 8.3 Hz, 1H), 7.89 (d, *J* = 8.2 Hz, 2H), 7.78 (d, *J* = 8.6 Hz, 2H), 7.72 (d, *J* = 8.2 Hz, 2H), 6.98 (d, *J* = 8.8 Hz, 2H), 6.66 (t, *J* = 76.0 Hz, 1H), 5.50 (dd, *J* = 15.4, 8.2 Hz, 1H), 4.28 (s, 1H), 4.04-3.95 (m, 1H), 3.93-3.82 (m, 1H), 3.68 (d, *J* = 9.8 Hz, 1H), 3.30-3.24 (m, 2H), 3.20-3.11 (m, 2H), 3.10-2.99 (m, 2H), 2.94 (t, *J* = 11.9 Hz, 1H), 2.39-2.18 (m, 3H), 2.10-1.54 (m, 7H), 1.43 (d, *J*= 12.1 Hz, 2H), 1.10 (t, *J*= 7.3 Hz, 3H).

**Example 118** *N*-((*S*)-2-cyano-1-(4-(ethylsulfonyl)phenyl)ethyl)-4-((2*S*,5*S*)-2-**((difluoromethoxy)methyl)-5-(4-(trifluoromethyl)phenoxy)piperidin-1-yl)benzamide**

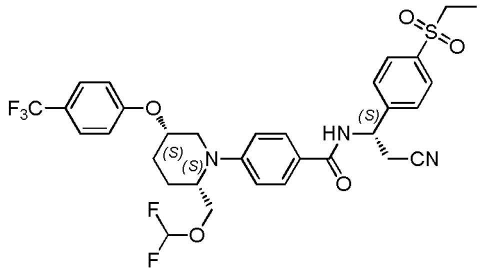

**Step 1: Synthesis of tert-butyl (2*S*,4*S*)-2-((difluoromethoxy)methyl)-5-(4-(trifluoromethyl) phenoxy)piperidine-1-carboxylate**

To a solution of tert-butyl (2*S*,5*S*)-2-(hydroxymethyl)-5-(4-(trifluoromethyl)phenoxy) piperidine-1-carboxylate (236 mg, 0.63 mmol) and $TMSCF_2Br$ (0.50 mL, 3.20 mmol) in DCM/$H_2O$ (2 mL/2 mL) was added KOAc (250 mg, 2.55 mmol). The mixture was reacted at room temperature for 12 h. Then $TMSCF_2Br$ (0.50 mL, 3.20 mmol) was added and the reaction was continued for 6 h. The reaction solution was added with DCM (80 mL), washed with saturated $NaHCO_3$ solution (20 mL × 2), dried over anhydrous $Na_2SO_4$, concentrated under reduced pressure, and the concentrated solution was separated by silica gel column chromatography (eluent: PE/DCM/EtOAc (v/v/v) = 10/1/1) to give a pale yellow solid (197 mg, 74%).
MS (ESI, pos.ion) m/z: 448.1 $[M+Na]^+$.

**Step 2: Synthesis of (2*S*,4*S*)-2-((difluoromethoxy)methyl)-5-(4-(trifluoromethyl)phenoxy)piperidine**

*tert*-Butyl (2*S*,5*S*)-2-((difluoromethoxy)methyl)-5-(4-(trifluoromethyl)phenoxy) piperidine-1-carboxylate (197 mg, 0.46 mmol) was dissolved in a solution of HCl in MeOH (10 mL, 20%). The mixture was stirred at room temperature for 12 h. Saturated $Na_2CO_3$ solution (40 mL) was added to the reaction solution. The resulting mixture was extracted with DCM (30 mL × 3), and the organic phases were combined and dried over anhydrous $Na_2SO_4$, concentrated under reduced pressure to give pale yellow oil (150 mg, 100%).
MS (ESI, pos.ion) m/z: 326.2 $[M+H]^+$.

**Step 3: Synthesis of methyl 4-((2*S*,5*S*)-2-((difluoromethoxy)methyl)-5-(4-(trifluoromethyl) phenoxy)piperidin-1-yl)benzoate**

Under nitrogen protection, (2*S*,5*S*)-2-((difluoromethoxy)methyl)-5-(4-(trifluoromethyl) phenoxy)piperidine (150 mg, 0.46 mmol), methyl 4-iodobenzoate (360 mg, 1.37 mmol), $Pd_2(dba)_3$ (21 mg, 0.02 mmol), 4,5-bisdiphenylphosphine-9,9-dimethylxanthene (20 mg, 0.04 mmol) and $CS_2CO_3$ (323 mg, 0.98 mmol) were dissolved in 1,4-dioxane (5 mL), and the mixture was reacted at 100°C for 22 h. The reaction solution was cooled to room temperature, and added with $NaHCO_3$ (50 mL) to quench the reaction. The aqueous phase was extracted with DCM (40 mL × 3), and the organic phases were combined and dried over anhydrous $Na_2SO_4$, concentrated under reduced pressure. The concentrated solution was separated by silica gel column chromatography (eluent: PE/DCM/EtOAc (v/v/v) = 10/1/1) to give a yellow solid (69 mg, 33%).
MS (ESI, pos.ion) m/z: 460.2 $[M+H]^+$.

**Step 4: Synthesis of 4-((2*S*,5*S*)-2-((difluoromethoxy)methyl)-5-(4-(trifluoromethyl)phenoxy) piperidin-1-yl)benzoic acid**

To a solution of methyl 4-((2S,5S)-2-((difluoromethoxy)methyl)-5-(4-(trifluoromethyl) phenoxy)piperidin-1-yl)benzoate (69 mg, 0.15 mmol) in THF/MeOH (1 mL/1 mL) was added $LiOH \cdot H_2O$ (134 mg, 3.19 mmol). The mixture was stirred at room temperature for 12 h. $LiOH \cdot H_2O$ (1.20 g, 28.6 mmol) was added, and the reaction was continued for 6 h at room temperature. DCM (80 mL) and $H_2O$ (40 mL) were added to the reaction solution, and HCl solution (1 mol/L) was added dropwise to adjust the pH to about 3. The mixture was separated for layers. The aqueous phase was extracted with DCM (40 mL × 2), and the organic phases were combined and dried over anhydrous $Na_2SO_4$ and

concentrated under reduced pressure to give a yellow solid (60 mg, 90%).
MS (ESI, pos.ion) m/z: 445.9 $[M+H]^+$.

**Step 5: Synthesis of *N*-((*S*)-2-cyano-1-(4-(ethylsulfonyl)phenyl)ethyl)-4-((2*S*,5*S*)-2-((difluoromethoxy)methyl)-5-(4-(trifluoromethyl)phenoxy)piperidin-1-yl)benzamide**

4-((2*S*,5*S*)-2-((difluoromethoxy)methyl)-5-(4-(trifluoromethyl)phenoxy)piperidin-1-yl) benzoic acid (70 mg, 0.16 mmol), (*S*)-3-amino-3-(4-(ethylsulfonyl)phenyl)propionitrile (50 mg, 0.21 mmol), EDCI (107 mg, 0.56 mmol) and HOBT (101 mg, 0.75 mmol) were dissolved in DCM (5 mL), then TEA (0.12 mL, 0.86 mmol) was added and the mixture was reacted at room temperature for 8 h. The reaction solution was added with DCM (60 mL), washed with saturated NaCl solution (30 mL × 2), dried over anhydrous $Na_2SO_4$, concentrated under reduced pressure, and the concentrated solution was separated by silica gel column chromatography (eluent: PE/EtOAc (v/v) = 1/1) to give a white solid (69 mg, 66%).

MS (ESI, pos.ion) m/z: 666.3 $[M+H]^+$.
$^1$H NMR (400 MHz, DMSO-$d_6$) δ (ppm): 8.96 (d, *J* = 7.9 Hz, 1H), 7.89 (d, *J* = 8.0 Hz, 2H), 7.80 (d, *J* = 8.4 Hz, 2H), 7.72 (d, *J* = 8.0 Hz, 2H), 7.66 (d, *J* = 8.4 Hz, 2H), 7.21 (d, *J* = 8.4 Hz, 2H), 7.05 (d, *J* = 8.6 Hz, 2H), 6.68 (t, *J* = 75.9 Hz, 1H), 5.51 (dd, *J* = 15.0, 7.7 Hz, 1H), 4.63 (dd, *J* = 8.6, 4.3 Hz, 1H), 4.34 (s, 1H), 4.11 - 4.04 (m, 1H), 3.98 (t, *J* = 8.1 Hz, 2H), 3.31 - 3.25 (m, 2H), 3.19 - 3.13 (m, 2H), 3.08 (d, *J* = 11.2 Hz, 1H), 2.14 - 2.06 (m, 1H), 1.93 (s, 2H), 1.73 (dd, *J* = 17.9, 9.1 Hz, 1H), 1.10 (t, *J* = 7.2 Hz, 3H).

**Example 119 (*S*)-*N*-(2-cyano-1-(4-(ethylsulfonyl)phenyl)ethyl)-4-(4-((difluoromethoxy)methyl) -1-(4-(trifluoromethyl)benzyl)-1,2,5,6-tetrahydropyridin-3-yl)benzamide**

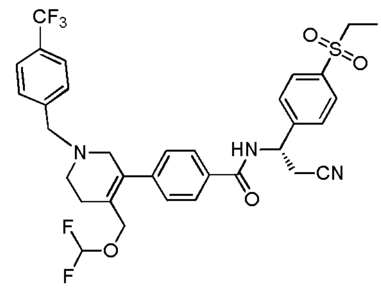

**Step 1: Synthesis of 1-*tert*-butyl 4-ethyl 3-(((trifluoromethyl)sulfonyl)oxy)-5,6-dihydropyridine -1,4(2*H*)-dicarboxylate**

To a solution of **1-*tert*-butyl** 4-ethyl 3-oxopiperidine-1,4-dicarboxylate (5.00 g, 18.43 mmol) in DCM (20 mL) was added DIPEA (6.09 mL, 36.80 mmol). The mixture was reacted at room temperature for 5 minutes, then cooled to 0°C, and trifluoromethanesulfonic anhydride (4.1 mL, 24.00 mmol) was slowly added dropwise. The reaction was continued for 3 h. The resulting mixture was washed with saturated NaCl, extracted with DCM (10 ml × 2), dried over anhydrous $Na_2SO_4$, concentrated and separated by silica gel column chromatography (eluent: PE/EtOAc (v/v) = 5/1) to give yellow liquid (6.80 g, 91%).
MS (ESI, pos.ion) m/z: 404 $[M+H]^+$.

**Step 2: Synthesis of 1-tert-butyl 4-ethyl 3-(4,4,5,5-tetramethyl-1,3,2-dioxaboro-2-yl)-5,6-dihydropyridine-1,4(2*H*)-dicarboxylate**

1-*tert*-Butyl 4-ethyl 3-(((trifluoromethyl)sulfonyl)oxy)-5,6-dihydropyridine-1,4(2*H*)-dicarboxylate (1.41 g, 3.50 mmol), 4,4,4',4',5,5,5',5'-octamethyl-2,2'-bis(1,3,2-dioxoborate) (0.98 g, 3.90 mmol), KOAc (1.03 g, 10.50 mmol) and dppf-$PdCl_2$ (0.29 g, 0.35 mmol) were dissolved in 1,4-dioxane (10 mL). The reaction mixture was degassed and refilled with nitrogen for three times, then heated to 80 °C and reacted for 12 h. The resulting mixture was washed with saturated NaCl, extracted with EtOAc (10 mL × 2), dried over anhydrous $Na_2SO_4$, concentrated and separated by silica gel column chromatography (eluent: PE/EtOAc (v/v) = 5/1) to give yellow liquid (1.12 g, 83%). MS (ESI, pos.ion) m/z: 382 $[M+H]^+$.

**Step 3: Synthesis of 1-*tert*-butyl 4-ethyl 3-(4-cyanophenyl)-5,6-dihydropyridine-1,4(2*H*)-dicarboxylate**

1-*tert*-Butyl 4-ethyl 3-(4,4,5,5-tetramethyl-1,3,2-dioxaboro-2-yl)-5,6-dihydropyridine-1,4(2*H*)-dicarboxylate (1.21 g, 3.17 mmol), dppf-$PdCl_2$ (0.28 g, 0.34 mmol), 4-bromophenylacetonitrile (0.93 g, 5.10 mmol) and $CS_2CO_3$ (2.21 g, 6.78 mmol) were dissolved in 1,4-dioxane (8 mL). The reaction mixture was degassed and refilled with nitrogen for three times, then heated to 90 °C and reacted for 24 h. The resulting mixture was washed with saturated NaCl, extracted with EtOAc (10 mL × 2), dried over anhydrous $Na_2SO_4$, concentrated and separated by silica gel column chromatography (eluent: PE/EtOAc (v/v) = 5/1) to give a yellow solid (0.85 g, 75%).
MS (ESI, pos.ion) m/z: 357 $[M+H]^+$.

**Step 4: Synthesis of *tert-butyl* 3-(4-cyanophenyl)-4-(hydroxymethyl) -5,6-dihydropyridine-1(2*H*)-carboxylate**

1-*tert*-Butyl 4-ethyl 3-(4-cyanophenyl)-5,6-dihydropyridine-1,4(2*H*)-dicarboxylate (0.78 g, 2.20 mmol) was dissolved in THF (8 mL), the reaction solution was cooled to 0°C, $LiBH_4$ (95 mg, 4.36 mmol) was added, and the mixture was warmed to room temperature and reacted for 4 h. The reaction was quenched by adding water. The resulting mixture was washed with saturated NaCl, extracted with EtOAc (10 mL × 2), dried over anhydrous $Na_2SO_4$, concentrated and separated by silica gel column chromatography (eluent: PE/EtOAc (v/v) = 5/1) to give a white solid (0.41 g, 59%). MS (ESI, pos.ion) m/z: 315 $[M+H]^+$.

**Step 5: Synthesis of *tert*-butyl 3-(4-cyanophenyl)-4-((difluoromethoxy)methyl) -5,6-dihydropyridine-1(2*H*)-carboxylate**

To a solution of *tert-butyl* 3-(4-cyanophenyl)-4-(hydroxymethyl)-5,6-dihydropyridine -1(2*H*)-carboxylate (330 mg, 1.05 mmol) in DCM (5 mL) were added KOAc (0.61 g, 6.20 mmol) and $H_2O$ (5 mL), then (bromo(difluoro)methyl)-trimethyl-silane (0.4 mL, 3.00 mmol) was added. The mixture was reacted at room temperature for 24 h, then washed with saturated NaCl, extracted with DCM (10 mL × 2), dried over anhydrous $Na_2SO_4$, concentrated and separated by silica gel column chromatography (eluent: PE/EtOAc (v/v) = 5/1) to obtain a white solid (0.26 g, 69%).
MS (ESI, pos.ion) m/z: 365 $[M+H]^+$.

**Step 6: Synthesis of 4-(4-((difluoromethoxy)methyl)-1,2,5,6-tetrahydropyridin -3-yl)benzonitrile**

To a solution of tert-butyl 3-(4-cyanophenyl)-4-((difluoromethoxy)methyl)-5,6-dihydropyridine-1(2*H*)-carboxylate (0.15 g, 0.41 mmol ) in DCM (25 mL) was added HCl (0.23 mL, 0.92 mmol, 4.0 mol/L), and the mixture was reacted at room temperature for 2 h. The resulting mixture was washed with saturated $Na_2CO_3$, extracted with DCM (10 mL × 2), dried over anhydrous $Na_2SO_4$, concentrated and separated by silica gel column chromatography (eluent: DCM/EtOAc (v/v) = 5/1) to give a white solid (0.10 g, 97%).
MS (ESI, pos.ion) m/z: 265 $[M+H]^+$.

**Step 7: Synthesis of 4-(4-((difluoromethoxy)methyl)-1-(4-(trifluoromethyl)benzyl)-1,2,5,6-tetrahydropyridin-3-yl)benzonitrile**

*tert*-Butyl 3-(4-cyanophenyl)-4-((difluoromethoxy)methyl)-5,6-dihydropyridine -1(2*H*)-carboxylate (0.11 g, 0.42 mmol ), $K_2CO_3$ (0.17 g, 1.20 mmol) and 1-(bromomethyl)-4-(trifluoromethyl)benzene (0.11 g, 0.46 mmol) were dissolved in ACN (25 mL), and the mixture was reacted at room temperature for 8 h. The resulting mixture was washed with saturated NaCl, extracted with EtOAc (8 ml × 2), dried over anhydrous $Na_2SO_4$, concentrated and separated by silica gel column chromatography (eluent: PE/EtOAc (v/v) = 5/1) to give a white solid (0.1 g, 57%).
MS (ESI, pos.ion) m/z: 423 $[M+H]^+$.

**Step 8: Synthesis of 4-(4-((difluoromethoxy)methyl)-1-(4-(trifluoromethyl)benzyl) -1,2,5,6-tetrahydropyridin-3-yl)benzoic acid**

To a solution of 4-(4-((difluoromethoxy)methyl)-1-(4-(trifluoromethyl)benzyl) -1,2,5,6-tetrahydropyridin-3-yl)benzonitrile (110 mg, 0.26 mmol) in MeOH (10 mL) was added a solution of KOH (584 mg, 10.41 mmol) in $H_2O$ (2 mL), and the mixture was reacted at 80°C for 24 h. Dilute hydrochloric acid was added to adjust the pH of the solution to be weakly acidic. The resulting mixture was extracted with EtOAc (10 mL × 2), dried over anhydrous $Na_2SO_4$, concentrated and separated by silica gel column chromatography (eluent: PE/EtOAc (v/v) = 5/1) to give a white solid (0.058 g, 50%).
MS (ESI, pos.ion) m/z: 442 $[M+H]^+$.

**Step 9: Synthesis of (*S*)-*N*-(2-cyano-1-(4-(ethylsulfonyl)phenyl)ethyl)-4-(4-((difluoromethoxy) methyl)-1-(4-(trifluoromethyl)benzyl)-1,2,5,6-tetrahydropyridin-3-yl)benzamide**

4-(4-((Difluoromethoxy)methyl)-1-(4-(trifluoromethyl)benzyl)-1,2,5,6-tetrahydropyrid in-3-yl)benzoic acid (100 mg, 0.23 mmol), (*S*)-3-amino-3-(4-(ethylsulfonyl)phenyl)propionitrile hydrochloride (68 mg, 0.25 mmol) and HATU (104 mg, 0.27 mmol) were dissolved in DCM (25 mL), then TEA (0.068 mL, 0.52 mmol) was added and the mixture was reacted at room temperature for 24 h. The resulting mixture was washed with saturated $NH_4Cl$, extracted with DCM (10 mL × 2), dried over anhydrous $Na_2SO_4$, concentrated and separated by silica gel column chromatography (eluent: DCM/EtOAc (v/v) = 5/1) to give a white solid (0.12 g, 75%).

MS (ESI, pos.ion) m/z: 662 $[M+H]^+$.
$^1$H NMR (600 MHz, $CDCl_3$) δ (ppm): 7.97 (d, *J* = 7.6 Hz, 2H), 7.82 (d, *J* = 7.4 Hz, 2H), 7.69 (d, *J*= 7.8 Hz, 2H), 7.61 (d, *J* = 7.8 Hz, 2H), 7.52 (d, *J* = 7.7 Hz, 2H), 7.36 - 7.24 (m, 2H), 6.88 (s, 1H), 6.18 (t, *J*= 74.5 Hz, 1H), 5.63 (s, 1H), 4.19 (s, 2H), 3.73 (s, 2H), 3.28 - 3.19 (m, 2H), 3.13 (ddd, *J* = 21.7, 15.9, 6.1 Hz, 3H), 2.75 (s, 2H), 2.43 (s, 2H), 0.96 - 0.84 (m, 3H).

**Example 120 *N*-((*S*)-2-cyano-1-(4-(ethylsulfonyl)phenyl)ethyl)-4-(1-(4-(trifluoromethyl) benzyl)piperidin-2-yl)benzamide**

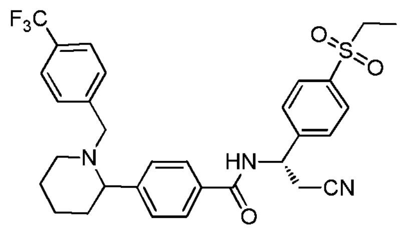

**Step 1: Synthesis of methyl 4-(5-(((*tert*-butoxy)carbonyl)amino)valeryl)benzoate**

To a solution of methyl 4-iodobenzoate (4.60 g, 18.00 mmol) in THF (20 mL) was added *i*-PrMgBr (17 mL, 17.00 mmol) at -50°C. The mixture was reacted for 1 h, then tert-butyl 2-oxopiperidine-1-carboxylate (2.90 g, 15.00 mmol) was added. The mixture was slowly returned to room temperature and reacted for 3 h. Dilute hydrochloric acid was added to adjust the pH of the solution to be acidic. The resulting mixture was extracted with EtOAc (25 mL × 2). The organic phases were combined, dried over anhydrous $Na_2SO_4$, concentrated under reduced pressure, and the concentrated solution was separated by silica gel column chromatography (eluent: PE/EtOAc (v/v) = 10/1) to give colorless liquid (2.75 g, 56%).
MS (ESI, pos.ion) m/z: 358 $[M+Na]^+$.

**Step 2: Synthesis of methyl 4-(3,4,5,6-tetrahydropyridin-2-yl)benzoate**

To a solution of methyl 4-(5-(((*tert*-butoxy)carbonyl)amino)valeryl)benzoate (2.57 g, 7.66 mmol) in DCM (10 mL) was added TAF (2.85 mL, 38.40 mmol). The mixture was reacted at room temperature for 1 h. The reaction solution was diluted with DCM (30 mL), washed successively with saturated $NaHCO_3$ solution (15 mL) and saturated NaCl solution (15 mL), dried over anhydrous $Na_2SO_4$, concentrated under reduced pressure, and the concentrated solution was separated by silica gel column chromatography (eluent: PE/EtOAc (v/v) = 10/1) to give a white solid (1.63 g, 98%).
MS (ESI, pos.ion) m/z: 218 $[M+H]^+$.

**Step 3: Synthesis of methyl 4-(piperidin-2-yl)benzoate**

Methyl 4-(3,4,5,6-tetrahydropyridin-2-yl)benzoate (2.23 g, 11.00 mmol) was dissolved in THF (12 mL) and MeOH (5 mL) at room temperature, then $NaBH_4$ (0.11 g, 2.90 mmol) was slowly added. The mixture was reacted for 2 h, and water was added to quench the reaction. The resulting mixture was extracted with EtOAc (15 mL × 2), and the combined organic phases were dried over anhydrous $Na_2SO_4$, and concentrated under reduced pressure to give colorless liquid (2.25 g, 99%). MS (ESI, pos.ion) m/z: 220 $[M+H]^+$.

**Step 4: Synthesis of *tert*-butyl 2-(4-(methoxycarbonyl)phenyl)piperidine-1-carboxylate**

To a solution of methyl 4-(piperidin-2-yl)benzoate (1.69 g, 7.71 mmol) in DCM (5 mL) were added TEA (2 mL, 15.00 mmol) and $Boc_2O$ (1.70 g, 7.71 mmol) in turn, and the mixture was reacted at room temperature for 5 h. The reaction solution was diluted with DCM (30 mL), washed successively with saturated $NH_4Cl$ solution (15 mL) and saturated NaCl solution (15 mL), dried over anhydrous $Na_2SO_4$, concentrated under reduced pressure, and the concentrated solution was separated by silica gel column chromatography (eluent: PE/EtOAc (v/v) = 5/1) to give colorless liquid (1.98 g, 80.4%).
MS (ESI, pos.ion) m/z: 320 $[M+H]^+$.

**Step 5: Synthesis of 4-(1-((*tert*-butoxy)carbonyl)piperidin-2-yl)benzoic acid**

To a solution of tert-butyl 2-(4-(methoxycarbonyl)phenyl)piperidine-1-carboxylate (1.98 g, 6.20 mmol) in THF (10 mL) and MeOH (10 mL) was added a solution of LiOH (1.48 g, 61.80 mmol) in $H_2O$ (2 mL). The mixture was reacted at room temperature for 12 h. The reaction solution was added with HCl solution (2 mol/L) to adjust the pH to about 5. The resulting mixture was extracted with EtOAc (25 mL $\times$ 2), and the organic phases were combined, washed with saturated NaCl (20 mL) solution, dried over anhydrous $Na_2SO_4$, concentrated under reduced pressure. The concentrated solution was separated by silica gel column chromatography (eluent: DCM/MeOH (v/v) = 20/1) to give a white solid (1.66 g, 88%).
MS (ESI, pos.ion) m/z: 306 $[M+H]^+$.

**Step 6: Synthesis of *tert-butyl* 2-(4-(((*S*)-2-cyano-1-(4-(ethylsulfonyl)phenyl)ethyl)carbamoyl) phenyl)piperidine-1-carboxylate**

4-(1-((*tert*-Butoxy)carbonyl)piperidin-2-yl)benzoic acid (120 mg, 0.39 mmol), (*S*)-3-amino-3-(4-(ethylsulfonyl)phenylpropionitrile hydrochloride (112 mg, 0.49 mmol) and HATU (181 mg, 0.47 mmol) were dissolved in DCM (12 mL), then TEA (0.12 mL, 0.92 mmol) was added and the mixture was reacted at room temperature for 24 h. The reaction solution was diluted with DCM (30 mL), washed successively with $NaHCO_3$ solution (15 mL) and saturated NaCl solution (15 mL), dried over anhydrous $Na_2SO_4$, concentrated under reduced pressure, and the concentrated solution was separated by silica gel column chromatography (eluent: DCM/EtOAc (v/v) = 1/1) to give a white solid (120 mg, 59%).
MS (ESI, pos.ion) m/z: 526 $[M+H]^+$.

**Step 7: Synthesis of *N*-((*S*)-2-cyano-1-(4-(ethylsulfonyl)phenyl) ethyl)-4-(piperidin-2-yl)benzamide**

To a solution of *tert-butyl* 2-(4-(((*S*)-2-cyano-1-(4-(ethylsulfonyl)phenyl) ethyl)carbamoyl)phenyl)piperidine-1-carboxylate (120 mg, 0.23 mmol) in DCM (10 mL) was added TFA (0.24 mL, 3.20 mmol) slowly. The mixture was reacted at room temperature for 3 h. The reaction solution was diluted with DCM (30 mL), then washed successively with saturated $NaHCO_3$ solution (15 mL) and saturated NaCl solution (15 mL), dried over anhydrous $Na_2SO_4$, and concentrated under reduced pressure to give colorless liquid (94 mg, 97%).
MS (ESI, pos.ion) m/z: 426 $[M+H]^+$.

**Step 8: Synthesis of *N*-((*S*)-2-cyano-1-(4-(ethylsulfonyl)phenyl)ethyl) -4-(1-(4-(trifluoromethyl)benzyl)piperidin-2-yl)benzamide**

To a solution of *N*-((*S*)-2-cyano-1-(4-(ethylsulfonyl)phenyl) ethyl)-4-(piperidin-2-yl)benzamide (97 mg, 0.23 mmol) in DMF (10 mL) were added 1-(bromomethyl)-4-(trifluoromethyl)benzene (65 mg, 0.28 mmol) and $K_2CO_3$ (15 mg, 0.24 mmol), and the mixture was reacted at room temperature for 12 h. The reaction solution was diluted with DCM (30 mL), then washed successively with saturated $NaHCO_3$ solution and saturated NaCl solution, dried over anhydrous $Na_2SO_4$, and concentrated under reduced pressure to give a white solid (44 mg, 33%).

MS (ESI, pos.ion) m/z: 584 $[M+H]^+$.
$^1H$ NMR (400 MHz, $CDCl_3$) $\delta$ (ppm): 7.94 (d, *J*= 7.1 Hz, 2H), 7.80 (d, *J*= 8.2 Hz, 2H), 7.67 (d, *J*= 8.1 Hz, 2H), 7.57 (d, *J* = 7.8 Hz, 2H), 7.53 (d, *J* = 8.0 Hz, 2H), 7.39 (d, *J* = 7.9 Hz, 2H), 6.82 (s, 1H), 5.62 (dd, *J* = 12.9, 6.1 Hz, 1H), 3.72 (t, *J* = 6.9 Hz, 1H), 3.32 - 3.03 (m, 5H), 2.96 (d, *J* = 14.1 Hz, 2H), 1.77 (m, 8H), 1.30 (d, *J*= 7.2 Hz, 3H).

**Example 121**

**121-1: *N*-((*S*)-2-cyano-1-(4-(ethylsulfonyl)phenyl)ethyl)-4-((2*S*,5*S*)-2-((difluoromethoxy)methyl) -5-(4-(trifluoromethyl)phenyl)piperidin-1-yl)benzamide**

**121-2: *N*-((*S*)-2-cyano-1-(4-(ethylsulfonyl)phenyl)ethyl)-4-((2*S*,5*R*)-2-((difluoromethoxy)methyl)-5-(4 -(trifluoromethyl)phenyl)piperidin-1-yl)benzamide**

121-1: 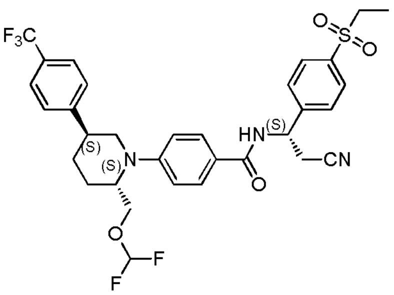 and121-2: 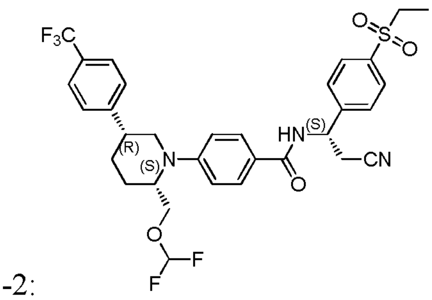

The raw material methyl 2-fluoro-4-iodo-benzoate in step 6 of Example 111 was replaced with methyl 4-iodobenzoate, which was used to prepare the title compounds with the intermediate (2*S*)-2-((difluoromethoxy)methyl)-5-(4-(trifluoromethyl)phenyl)piperidine according to the methods of step 6 to step 8 in Example 111. All of the title compounds were white solids, 121-1: (216 mg, 74%); 121-2: (73 mg, 59%).

MS (ESI, pos.ion) m/z: 650.2 $[M+H]^+$.

**121-1:**

$^1$H NMR (400 MHz, DMSO-$d_6$) $\delta$ (ppm): 8.99 (d, *J*= 8.1 Hz, 1H), 7.89 (d, *J*= 8.0 Hz, 2H), 7.82 (d, *J* = 8.0 Hz, 2H), 7.73 (d, *J* = 7.9 Hz, 2H), 7.64 (d, *J* = 7.8 Hz, 2H), 7.53 (d, *J* = 7.8 Hz, 2H), 7.08 (d, *J* = 8.5 Hz, 2H), 6.67 (t, *J* = 75.8 Hz, 1H), 5.52 (dd, *J* = 14.3, 7.5 Hz, 1H), 4.18 (s, 1H), 4.09 - 3.99 (m, 1H), 3.88 (dd, *J* = 9.7, 4.2 Hz, 1H), 3.77 (dd, *J* = 12.5, 3.6 Hz, 1H), 3.44 (s, 1H), 3.31 (s, 1H), 3.29 - 3.24 (m, 2H), 3.16 (d, *J* = 5.2 Hz, 2H), 2.17 (s, 1H), 1.89 - 1.69 (m, 3H), 1.10 (t, *J* = 7.2 Hz, 3H).

**121-2:**

$^1$H NMR (400 MHz, DMSO-$d_6$) $\delta$ (ppm): 8.95 (d, *J* = 8.1 Hz, 1H), 7.89 (d, *J* = 8.2 Hz, 2H), 7.79 (d, *J* = 8.7 Hz, 2H), 7.75 - 7.69 (m, 4H), 7.66 (d, *J* = 8.1 Hz, 2H), 7.03 (d, *J* = 8.8 Hz, 2H), 6.69 (t, *J* = 76.0 Hz, 1H), 5.51 (dd, *J* = 15.2, 8.0 Hz, 1H), 4.42 (s, 1H), 4.22 - 4.14 (m, 1H), 4.02 (dd, *J* = 10.0, 6.2 Hz, 1H), 3.71 (d, *J* = 9.9 Hz, 1H), 3.31 - 3.25 (m, 2H), 3.14 (t, *J* = 9.6 Hz, 3H), 2.93 (t, *J* = 11.1 Hz, 1H), 1.97 (t, *J* = 10.7 Hz, 2H), 1.84 (dd, *J* = 35.0, 11.8 Hz, 2H), 1.09 (t, *J* = 7.3 Hz, 3H).

**Example 122 *N*-((*S*)-2-cyano-1-(4-(ethylsulfonyl)phenyl)ethyl)-4-((*S*)-2-((difluoromethoxy) methyl)-4-(4-(trifluoromethyl)benzyl)piperazin-1-yl)benzamide**

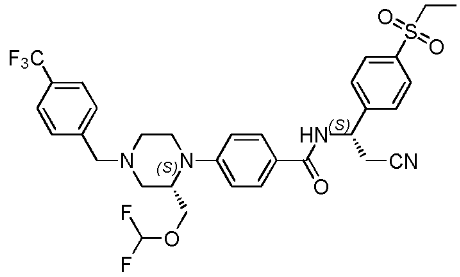

**Step 1: Synthesis of** (*S*)-4-benzyl **1-*tert*-butyl 2-(hydroxymethyl)piperazine-1,4-dicarboxylate**

To a solution of (*S*)-4-benzyl **1-*tert*-butyl** 2-methylpiperazine-1,2,4-tricarboxylate (5.1 g, 13.00 mmol) in THF

(15 mL) was slowly added $LiBH_4$ (700 mg, 32.14 mmol) in batches under an ice bath, and the mixture was reacted at room temperature for 24 h. The reaction solution was added with saturated $NaHCO_3$ solution (100 mL) to quench the reaction. The resulting mixture was concentrated under reduced pressure, extracted with DCM (120 mL × 2), dried over anhydrous $Na_2SO_4$, concentrated under reduced pressure, and the concentrated solution was separated by silica gel column chromatography (eluent: PE/EtOAc (v/v) = 1/1) to give colorless oil (4.6 g, 97%).
MS (ESI, pos.ion) m/z: 373.2 $[M+Na]^+$.

**Step 2: Synthesis of (*S*)-4-benzyl 1-*tert*-butyl 2-((difluoromethoxy)methyl) piperazine-1,4-dicarboxylate**

To a solution of (*S*)-4-benzyl **1-*tert*-butyl** 2-(hydroxymethyl)piperazine-1,4-dicarboxylate (4.6 g, 13.00 mmol) in DCM/$H_2O$ (18 mL/18 mL) was added KOAc (7.70 g, 78.00 mmol), then (bromo(difluoro)methyl)-trimethyl-silane (8.2 mL, 53.00 mmol) was slowly added dropwise under an ice bath, and the mixture was reacted at room temperature for 18 h. Saturated $NaHCO_3$ solution (60 mL) was added to the reaction solution to quench the reaction. The resulting mixture was extracted with DCM (80 mL × 2), dried over anhydrous $Na_2SO_4$, concentrated under reduced pressure, and the concentrated solution was separated by silica gel column chromatography (eluent: PE/EtOAc (v/v) = 6/1) to give colorless oil (3.59 g, 68%).
MS (ESI, pos.ion) m/z: 423.3 $[M+Na]^+$.

**Step 3: Synthesis of benzyl (*S*)-3-((difluoromethoxy)methyl)piperazine-1-carboxylate**

To a solution of (*S*)-4-benzyl 1-*tert*-butyl 2-((difluoromethoxy)methyl)piperazine -1,4-dicarboxylate (2.10 g, 5.20 mmol) in EtOAc (5 mL) was added HCl, then EtOAc (6 mL, 18 mmol, 3 mol/L) under an ice bath, and the mixture was reacted at room temperature for 24 h . The mixture was distilled under reduced pressure to remove part of HCl and EtOAc, then saturated $NaHCO_3$ solution (50 mL) was added to adjust the pH of the system to about 8. The resulting mixture was extracted with DCM (80 mL × 2), dried over anhydrous $Na_2SO_4$, concentrated under reduced pressure to give yellow oil (1.45 g, 92%).
MS (ESI, pos.ion) m/z: 301.2 $[M+H]^+$.

**Step 4: Synthesis of benzyl (*S*)-3-((difluoromethoxy)methyl)-4-(4-(methoxycarbonyl) phenyl)piperazine-1-carboxylate**

Under nitrogen protection, benzyl (*S*)-3-((difluoromethoxy)methyl) piperazine-1-carboxylate (550 mg, 1.83 mmol), methyl 4-iodobenzoate (665 mg, 2.54 mmol), $Pd_2(dba)_3$ (158 mg, 0.17 mmol), 2-bicyclohexylphosphine-2',6'-diisopropoxybiphenyl (197 mg, 0.42 mmol) and $Cs_2CO_3$ (1.06 g, 3.25 mmol) were dissolved in toluene (10 mL), and the mixture was reacted at 110°C for 19 h. The resulting mixture was filtered through a celite pad. The filtrate was concentrated under reduced pressure, and the concentrated solution was separated by silica gel column chromatography (eluent: PE/EtOAc (v/v) = 3/1) to give yellow oil (778 mg, 98%).
MS (ESI, pos.ion) m/z: 435.1 $[M+H]^+$.

**Step 5: Synthesis of methyl (*S*)-4-(2-((difluoromethoxy)methyl)piperazin-1-yl)benzoate**

Under hydrogen atmosphere, benzyl (S)-3-((difluoromethoxy)methyl)-4-(4-(methoxycarbonyl)phenyl)piperazine-1-carboxylate (778 mg, 1.79 mmol) and Pd/C (1.06 g, 0.99 mmol) were dissolved in MeOH/THF (5 mL/5 mL), and the mixture was reacted at room temperature for 12 h. The reaction solution was filtered through a celite pad, and the filtrate was concentrated under reduced pressure to give yellow oil (455 mg, 85%).
MS (ESI, pos.ion) m/z: 301.2 $[M+H]^+$.

Step 6: Synthesis of methyl **(*S*)-4-(2-((difluoromethoxy)methyl)-4-(4-(trifluoromethyl) benzyl)piperazin-1-yl)benzoate**

To a solution of methyl (*S*)-4-(2-((difluoromethoxy)methyl)piperazin-1-yl)benzoate (203 mg, 0.68 mmol) and 4-(trifluoromethyl)benzaldehyde (0.12 mL, 0.88 mmol) in EtOH/THF (3 mL/3 mL) were added AcOH (0.08 mL, 1.00 mmol) and STAB (299 mg, 1.41 mmol), and the mixture was reacted at room temperature for 18 h. Then STAB (2eq 300mg) was added and the mixture was reacted at 60°C for 15h. The reaction solution was added with saturated $NaHCO_3$ solution (30 mL) to adjust the pH to about 8. The resulting mixture was extracted with EtOAc (50 mL × 2), dried over anhydrous $Na_2SO_4$, concentrated under reduced pressure, and the concentrated solution was separated by silica gel column chromatography (eluent: PE/EtOAc (v/v) = 3/1) to give yellow oil (133 mg, 43 %).
MS (ESI, pos.ion) m/z: 459.3 $[M+H]^+$.

Step 7: Synthesis of **(*S*)-4-(2-((difluoromethoxy)methyl)-4-(4-(trifluoromethyl)benzyl)piperazin-1-yl)benzoic acid**

Methyl (S)-4-(2-((difluoromethoxy)methyl)-4-(4-(trifluoromethyl)benzyl)piperazin -1-yl)benzoate (133 mg, 0.29 mmol) and $LiOH{\cdot}H_2O$ (162 mg, 3.86 mmol) were dissolved in THF/MeOH/$H_2O$ (2 mL/1 mL/0.7 mL), and the mixture was reacted at room temperature for 22 h. 1.0 M HCl solution (5 mL) was added to the reaction solution to adjust the pH to about 4. The resulting mixture was extracted with EtOAc (20 mL × 2), dried over anhydrous $Na_2SO_4$, concentrated under reduced pressure, and the concentrated solution was separated by silica gel column chromatography (eluent: PE/EtOAc (v/v) = 3/1) to give a white solid (62 mg, 48 %),
MS (ESI, pos.ion) m/z: 445.2 $[M+H]^+$.

Step 8: Synthesis of ***N*-((*S*)-2-cyano-1-(4-(ethylsulfonyl)phenyl)ethyl) -4-((*S*)-2-((difluoromethoxy) methyl)-4-(4-(trifluoromethyl)benzyl)piperazin-1-yl)benzamide**

(S)-4-(2-((Difluoromethoxy)methyl)-4-(4-(trifluoromethyl)benzyl)piperazin-1-yl)benz oic acid (62 mg, 0.14 mmol), (*S*)-3-amino-3-(4-(ethylsulfonyl)phenyl)propionitrile hydrochloride (63 mg, 0.23 mmol), EDCI (90 mg, 0.47 mmol) and HOBT (57 mg, 0.42 mmol) were dissolved in DCM (5 mL), then TEA (0.16 mL, 1.20 mmol) was added and the mixture was reacted at room temperature for 17 h. The reaction solution was added with DCM (50 mL), washed with saturated $NaHCO_3$ solution (20 mL) and saturated NaCl solution (20 mL), dried over anhydrous $Na_2SO_4$, concentrated under reduced pressure, and the concentrated solution was separated by silica gel column chromatography (eluent: PE/EtOAc (v/v) = 1/2) to give a pale yellow solid (32 mg, 35 %).

MS (ESI, pos.ion) m/z: 665.3 $[M+H]^+$.
$^1H$ NMR (400 MHz, DMSO-$d_6$) δ (ppm): 8.94 (d, *J* = 8.3 Hz, 1H), 7.89 (d, *J* = 8.0 Hz, 2H), 7.79 (d, *J* = 8.5 Hz, 2H), 7.76 - 7.67 (m, 4H), 7.59 (d, *J* = 7.9 Hz, 2H), 6.98 (d, *J* = 8.7 Hz, 2H), 6.61 (t, *J* = 76.0 Hz, 1H), 5.50 (dd, *J* = 15.3, 8.3 Hz, 1H), 4.23 (dd, *J* = 17.4, 8.6 Hz, 2H), 3.78 (d, *J* = 4.7 Hz, 1H), 3.71 (d, *J* = 13.6 Hz, 1H), 3.58 (d, *J* = 13.9 Hz, 2H), 3.26 (d, *J* = 7.3 Hz, 2H), 3.17 - 3.13 (m, 2H), 3.08 (d, *J* = 9.3 Hz, 1H), 2.94 (d, *J* = 10.3 Hz, 2H), 2.28 - 2.21 (m, 2H), 1.09 (t, *J* = 7.1 Hz, 3H).

**Example 123 *N*-((*S*)-2-cyano-1-(4-(ethylsulfonyl)phenyl)ethyl)-4-((*S*)-4-(4-(difluoromethoxy) benzyl) -2-((difluoromethoxy)methyl)piperazin-1-yl)benzamide**

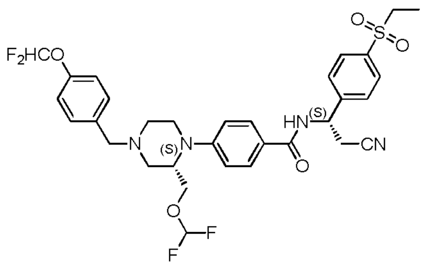

The raw material 4-(trifluoromethyl)benzaldehyde in step 5 of Example 122 was replaced with 4-(difluoromethoxy)benzaldehyde, which was reacted with the intermediate methyl (*S*)-4-(2-((difluoromethoxy)methyl)piperazin-1-yl)benzoate obtained in step 4 according to the methods of step 5 to step 8 in Example 122 to prepare the title compound as a white solid (180 mg, 63%).

MS (ESI, pos.ion) m/z: 663.3 $[M+H]^+$.
$^1H$ NMR (400 MHz, DMSO-$d_6$) δ (ppm): 8.95 (d, *J* = 7.8 Hz, 1H), 7.89 (d, *J* = 7.6 Hz, 2H), 7.80 (d, *J* = 8.0 Hz, 2H), 7.72 (d, *J* = 7.7 Hz, 2H), 7.40 (d, *J* = 7.5 Hz, 2H), 7.22 (s, 1H), 7.15 (d, *J* = 7.7 Hz, 2H), 6.98 (d, *J* = 8.1 Hz, 2H), 6.61 (t, *J* = 75.7 Hz, 1H), 5.51 (dd, *J* = 14.0, 7.2 Hz, 1H), 4.22 (dd, *J* = 21.3, 12.5 Hz, 2H), 3.77 (d, *J* = 4.3 Hz, 1H), 3.59 (d, *J*= 12.9 Hz, 2H), 3.48 (d, *J*= 13.3 Hz, 1H), 3.31 - 3.25 (m, 2H), 3.15 (d, *J* = 5.5 Hz, 2H), 3.10-3.03 (m, 1H), 2.92 (dd, *J* = 18.9, 11.1 Hz, 2H), 2.19 (dd, *J* = 23.4, 10.7 Hz, 2H), 1.09 (t, *J* = 6.9 Hz, 3H).

**Example 124 (*S*)-*N*-(2-cyano-1-(4-(ethylsulfonyl)phenyl)ethyl)-4-((4-(cyclopentanecarbonyl)piperazin -1-yl)methyl) benzamide**

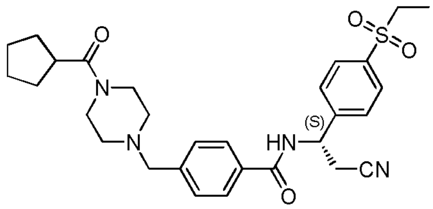

**Step 1: Synthesis of *tert*-butyl 4-(4-(methoxycarbonyl)benzyl)piperazine-1-carboxylate**

To a solution of tert-butyl piperazine-1-carboxylate (1.12 g, 6.01 mmol) and methyl 4-formylbenzoate (1.15 g, 7.01 mmol) in EtOH/THF (8 mL/8 mL) were added AcOH (1.5 mL, 26 mmol) and STAB (5.70 g, 27.00 mmol), and the mixture was reacted at 60°C for 22 h. The reaction solution was added with saturated $NaHCO_3$ solution (80 mL) to adjust the pH to about 8. The resulting mixture was extracted with EtOAc (100 mL × 2), dried over anhydrous $Na_2SO_4$, concentrated under reduced pressure, and the concentrated solution was separated by silica gel column chromatography (eluent: PE/EtOAc (v/v) = 3/1) to give yellow oil (1.8 g, 90%).
MS (ESI, pos.ion) m/z: 335.1 $[M+H]^+$.

**Step 2: Synthesis of methyl 4-(piperazin-1-ylmethyl)benzoate**

To a solution of tert-butyl 4-(4-(methoxycarbonyl)benzyl)piperazine-1-carboxylate (1.80 g, 5.40 mmol) in DCM (5 mL) was added a solution of HCl in 1,4-dioxane (13 mL, 52.00 mmol, 4 mol/L) under an ice bath, and the mixture was reacted at room temperature for 15 h. The mixture was distilled under reduced pressure to remove part of the solvent, and a solid was precipitated. The mixture was suction filtered, and the filter cake was washed with DCM (10 mL × 2) and dried to obtain a brown solid (959 mg, 76%).
MS (ESI, pos.ion) m/z: 235.1 $[M+H]^+$.

**Step 3: Synthesis of methyl 4-((4-(cyclopentanecarbonyl)piperazine-1-yl)methyl)benzoate**

Methyl 4-(piperazin-1-ylmethyl)benzoate (959 mg, 4.09 mmol), cyclopentanecarboxylic acid (0.6 mL, 6 mmol), EDCI (1.52 g, 7.93 mmol) and HOBT (1.12 g, 8.29 mmol) were dissolved in DCM (15 mL), then DIPEA (3.5 mL, 21.00 mmol) was added, and the mixture was reacted at room temperature for 13 h. The reaction solution was added with DCM (50 mL), washed successively with saturated $NH_4Cl$ solution (30 mL × 2) and saturated $NaHCO_3$ solution (30 mL × 2), dried over anhydrous $Na_2SO_4$, concentrated under reduced pressure, and the concentrated solution was separated by silica gel column chromatography (eluent: PE/EtOAc (v/v) = 1/1) to give yellow oil (1.3 g, 96%).
MS (ESI, pos.ion) m/z: 331.2 $[M+H]^+$.

**Step 4: Synthesis of 4-((4-(cyclopentanecarbonyl)piperazine-1-yl)methyl)benzoic acid**

Methyl 4-((4-(cyclopentanecarbonyl)piperazin-1-yl)methyl)benzoate (1.30 g, 3.90 mmol) and $LiOH{\cdot}H_2O$ (1.80 g, 43.00 mmol) were dissolved in THF/MeOH/$H_2O$ (8 mL/4 mL/4 mL), and the mixture was reacted at room temperature for 20 h. The mixture was concentrated under reduced pressure, and 1.0 M HCl solution (20 mL) was added to the reaction solution to adjust the pH to about 4. A white solid was precipitated. The resulting mixture was filtered with suction to give a white solid (1.15 g, 92%).
MS (ESI, pos.ion) m/z: 317.2 $[M+H]^+$.

**Step 5: Synthesis of (*S*)-*N*-(2-cyano-1-(4-(ethylsulfonyl)phenyl)ethyl)-4-((4-(cyclopentanecarbonyl) piperazin-1-yl)methyl) benzamide**

4-((4-(cyclopentanecarbonyl)piperazin-1-yl)methyl)benzoic acid (156 mg, 0.49 mmol), (*S*)-3-amino-3-(4-(ethylsulfonyl)phenyl)propionitrile hydrochloride (125 mg, 0.52 mmol), EDCI (186 mg, 0.97 mmol) and HOBT (130 mg, 0.96 mmol) were dissolved in DCM (8 mL), then DIPEA (0.6 mL, 4.00 mmol) was added and the mixture was reacted at room temperature for 14 h. The reaction solution was added with DCM (50 mL), washed with $NaHCO_3$ solution (20 mL) and

saturated NaCl solution (20 mL), dried over anhydrous $Na_2SO_4$, concentrated under reduced pressure, and the concentrated solution was separated by silica gel column chromatography (eluent: DCM/MeOH (v/v) = 15/1) to give a white solid (170 mg, 64%).

MS (ESI, pos.ion) m/z: 537.2 $[M+H]^+$.
$^1$H NMR (400 MHz, DMSO-$d_6$) $\delta$ (ppm): 9.24 (d, *J* = 8.0 Hz, 1H), 7.89 (t, *J* = 9.1 Hz, 4H), 7.75 (d, *J* = 7.9 Hz, 2H), 7.46 (d, *J* = 8.0 Hz, 2H), 5.55 (dd, *J* = 14.8, 7.4 Hz, 1H), 3.56 (s, 2H), 3.49 (d, *J* = 4.7 Hz, 4H), 3.29 (dd, *J* = 14.7, 7.3 Hz, 2H), 3.17 (d, *J* = 10.7 Hz, 2H), 2.94 (dd, *J* = 15.2, 7.3 Hz, 1H), 2.34 (d, *J* = 20.1 Hz, 4H), 1.73 (d, *J* = 7.7 Hz, 2H), 1.68 - 1.56 (m, 4H), 1.55 - 1.47 (m, 2H), 1.10 (t, *J* = 7.3 Hz, 3H).

**Example 125 (*S*)-*N*-(2-cyano-1-(4-(ethylsulfonyl)phenyl)ethyl)-4-(1-(4-(trifluoromethyl) benzyl)piperazin-2-yl)benzamide**

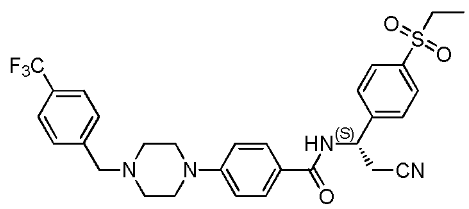

**Step 1: Synthesis of *tert*-butyl 4-(4-(methoxycarbonyl)phenyl)piperazine-1-carboxylate**

Under nitrogen protection, tert-butyl piperazine-1-carboxylate (8.2 g, 44.00 mmol), methyl 4-iodobenzoate (16.90 g, 64.50 mmol), $Pd_2(dba)_3$ (3.88 g, 4.24 mmol), 2-bicyclohexylphosphine-2',6'-diisopropoxybiphenyl (4.20 g, 9.00 mmol) and $Cs_2CO_3$ (27.90 g, 85.60 mmol) were dissolved in toluene (80 mL), and the mixture was reacted at 110°C for 19 h. The resulting mixture was filtered through a celite pad. The filtrate was concentrated under reduced pressure, and the concentrated solution was separated by silica gel column chromatography (eluent: PE/EtOAc (v/v) = 3/1) to give a yellow solid (10 g, 71%).
MS (ESI, pos.ion) m/z: 321.1 $[M+H]^+$.

**Step 2: Synthesis of methyl 4-(piperazin-1-yl)benzoate**

To a solution of tert-butyl 4-(4-(methoxycarbonyl)phenyl)piperazine-1-carboxylate (3 g, 9.36 mmol) in DCM (8 mL) was added a solution of HCl in 1,4-dioxane (16 mL, 48.00 mmol, 3 mol/L) under an ice bath, and the mixture was reacted at room temperature for 13 h. The mixture was distilled under reduced pressure to remove part of the solvent. A solid was precipitated. The mixture was filtered with suction, and the filter cake was washed with DCM (10 mL $\times$ 2) and added with saturated $NaHCO_3$ solution (120 mL) to adjust the pH of the system to about 8. The resulting mixture was extracted with DCM (150 mL $\times$ 2), dried over anhydrous $Na_2SO_4$, concentrated under reduced pressure to a brown solid (1.9 g, 92%).
MS (ESI, pos.ion) m/z: 221.2 $[M+H]^+$.

**Step 3: Synthesis of methyl 4-(4-(4-(trifluoromethyl)benzyl)piperazin-1-yl)benzoate**

To a solution of methyl 4-(piperazin-1-yl)benzoate (1.04 g, 4.72 mmol) and 4-(trifluoromethyl)benzaldehyde (0.8 mL, 6.00 mmol) in THF/EtOH (6 mL/6 mL) were added AcOH (1.3 mL, 23.00 mmol) and STAB (4.80 g, 23.00 mmol), and the mixture was reacted at 60°C for 18 h. The reaction solution was added with saturated $NaHCO_3$ solution (60 mL) to adjust the pH to about 8. The resulting mixture was extracted with EtOAc (100 mL $\times$ 2), dried over anhydrous $Na_2SO_4$, concentrated under reduced pressure, and the concentrated solution was separated by silica gel column chromatography (eluent: PE/EtOAc (v/v) = 3/1) to give a brown solid (1.6 g, 90%).
MS (ESI, pos.ion) m/z: 379.3 $[M+H]^+$.

**Step 4: Synthesis of 4-(4-(4-(trifluoromethyl)benzyl)piperazin-1-yl)benzoic acid**

Methyl 4-(4-(4-(trifluoromethyl)benzyl)piperazin-1-yl)benzoate (1.60 g, 4.20 mmol) and LiOH·$H_2O$ (1.82 g, 43.40 mmol) were dissolved in THF/MeOH/$H_2O$ (8 mL/4 mL/4 mL), and the mixture was reacted at room temperature for 48

h. The mixture was concentrated under reduced pressure, and 1.0 M HCl solution (20 mL) was added to the reaction solution to adjust the pH to about 4. A brown solid was precipitated. The resulting mixture was filtered with suction and dried to give a brown solid (1.45 g, 94%).
MS (ESI, pos.ion) m/z: 365.4 $[M+H]^+$.

**Step 5: Synthesis of (*S*)-*N*-(2-cyano-1-(4-(ethylsulfonyl)phenyl)ethyl) -4-(1-(4-(trifluoromethyl)benzyl)piperazin-2-yl)benzamide**

4-(4-(4-(trifluoromethyl)benzyl)piperazin-1-yl)benzoic acid (122 mg, 0.33 mmol), (*S*)-3-amino-3-(4-(ethylsulfonyl)phenyl)propionitrile hydrochloride (91 mg, 0.38 mmol), EDCI (147 mg, 0.77 mmol) and HOBT (103 mg, 0.76 mmol) were dissolved in DCM (5 mL), then DIPEA(0.45 mL, 2.70 mmol) was added and the mixture was reacted at room temperature for 17 h. The reaction solution was added with DCM (50 mL), washed with saturated $NaHCO_3$ solution (20 mL × 2) and saturated NaCl solution (20 mL), dried over anhydrous $Na_2SO_4$, concentrated under reduced pressure, and the concentrated solution was separated by silica gel column chromatography (eluent: DCM/MeOH (v/v) = 15/1) to give a white solid (128 mg, 65%).

MS (ESI, pos.ion) m/z: 585.7 $[M+H]^+$.
$^1H$ NMR (400 MHz, DMSO-$d_6$) δ (ppm): 8.95 (d, *J* = 8.2 Hz, 1H), 7.89 (d, *J* = 8.2 Hz, 2H), 7.79 (d, *J* = 8.6 Hz, 2H), 7.72 (d, *J* = 8.2 Hz, 4H), 7.59 (d, *J* = 7.8 Hz, 2H), 6.99 (d, *J* = 8.7 Hz, 2H), 5.51 (dd, *J* = 15.5, 8.5 Hz, 1H), 3.64 (s, 2H), 3.29 (t, *J* = 10.3 Hz, 6H), 3.18 - 3.11 (m, 2H), 2.52 (s, 4H), 1.09 (t, *J*= 7.3 Hz, 3H).

**Example 126 (*S*)-*N*-(2-cyano-1-(4-(ethylsulfonyl)phenyl)ethyl)-4-(3-(4-(trifluoromethyl) phenoxy) azetidin-1-yl)benzamide**

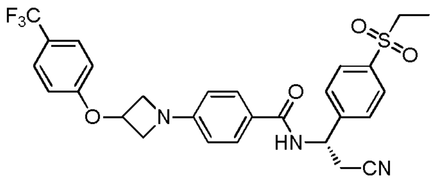

**Step 1: Synthesis of *tert*-butyl 3-(4-(trifluoromethyl)phenoxy)azetidine-1-carboxylate**

To a solution of 4-(trifluoromethyl)phenol (2.00 g, 12.3 mmol) in anhydrous THF (45 mL) were added tert-butyl 3-hydroxyazetidine-1-carboxylate (2.11 g, 12.2 mmol) and $PPh_3$ (3.80 g, 14.3 mmol). The mixture was cooled to 0°C and then DIAD (4.10 mL, 20.0 mmol) was added dropwise in 30 minutes. The reaction solution was reacted at room temperature for 18 h. The reaction solution was concentrated under reduced pressure, and methyl tert-butyl ether (80 mL) was slowly added to the reaction solution. The mixture was cooled to -15 °C and stirred, and a white solid was precipitated. The resulting mixture was filtered, the filtrate was concentrated. The concentrated solution was diluted with DCM (80 mL), washed with $NaHCO_3$ solution (100 mL), dried over anhydrous $Na_2SO_4$, concentrated under reduced pressure, and the concentrated solution was separated by silica gel column chromatography (eluent: PE/EtOAc (v/v) = 4/1) to give colorless transparent liquid (780 mg, 20%).
MS (ESI, pos.ion) m/z: 262.1 $[M-56+H]^+$.

**Step 2: Synthesis of 3-(4-(trifluoromethyl)phenoxy)azetidine**

To a solution of *tert*-butyl 3-(4-(trifluoromethyl)phenoxy)azetidine-1-carboxylate (780 mg, 2.46 mmol) in DCM (15 mL) was added TFA (6.6 mL, 89 mmol). The reaction solution was stirred and reacted at room temperature for 8 h. The reaction solution was diluted with DCM (20 mL), washed successively with saturated $NaHCO_3$ solution (40 mL) and saturated NaCl solution (40 mL), dried over anhydrous $Na_2SO_4$, filtered, concentrated under reduced pressure, and the concentrated solution was separated by silica gel column chromatography (eluent: DCM/MeOH (v/v) = 10/1) to give colorless liquid (400 mg, 75%).
MS (ESI, pos.ion) m/z: 218.1 $[M+H]^+$.

**Step 3: Synthesis of methyl 4-(3-(4-(trifluoromethyl)phenoxy)azetidin-1-yl)benzoate**

To a solution of methyl 4-iodobenzoate (800 mg, 3.05 mmol) and 3-(4-(trifluoromethyl)phenoxy)azetidine (400 mg, 1.84 mmol) in toluene (50 mL, 468 mmol) were added $Cs_2CO_3$ (900 mg, 2.76 mmol), 4,5-bisdiphenylphosphine-9,9-dimethylxanthene (60 mg, 0.10 mmol) and $Pd_2(dba)_3$ (95 mg, 0.10 mmol). The reaction solution was reacted at 95°C for 14 h under nitrogen protection. The reaction solution was cooled and filtered, the filtrate was concentrated. The mixture was slowly added with $NaHCO_3$ solution (30 mL) and diluted with DCM (30 mL). The aqueous phase was extracted with DCM (20 mL). The combined organic phases were dried over anhydrous $Na_2SO_4$, concentrated under reduced pressure. The concentrated solution was separated by silica gel column chromatography (eluent: PET/EtOAc (v/v) = 4/1) to give a yellow solid (250 mg, 39%).
MS (ESI, pos.ion) m/z: 352.1 $[M+H]^+$.

**Step 4: Synthesis of methyl 4-(3-(4-(trifluoromethyl)phenoxy)azetidin-1-yl)benzoic acid**

To a solution of methyl 4-(3-(4-(trifluoromethyl)phenoxy)azetidin-1-yl)benzoate (250 mg, 0.71 mmol) in THF (15 mL, 184 mmol) were added $H_2O$ (1 mL) and LiOH(230 mg, 5.37 mmol). The reaction solution was stirred and reacted at room temperature for 16 h. The reaction solution was concentrated under reduced pressure, and the resulting mixture was added with HCl solution (19 mL, 1.0 mol/L), extracted with DCM (10 mL × 3). The organic phases were combined, washed with saturated NaCl solution (15 mL), dried over anhydrous $Na_2SO_4$, filtered and concentrated under reduced pressure to give a crude product was a white solid (200 mg, 83%).
MS (ESI, pos.ion) m/z: 338.1 $[M+H]^+$.

**Step 5: Synthesis of (*S*)-*N*-(2-cyano-1-(4-(ethylsulfonyl)phenyl)ethyl)-4-(3-(4-(trifluoromethyl) phenoxy)azetidin-1-yl)benzamide**

(*S*)-3-Amino-3-(4-(ethylsulfonyl)phenyl)propionitrile hydrochloride (75 mg, 0.315 mmol), 4-(3-(4-(trifluoromethyl)phenoxy)azetidin-1-yl)benzoic acid (65 mg , 0.19 mmol), HATU (100 mg, 0.255 mmol) and DIPEA (0.2 mL, 1 mmol) were dissolved in DCM (20 mL), and the reaction solution was stirred at room temperature for 16 h. The reaction solution was diluted with DCM (30 mL), washed successively with HCl solution (20 mL, 0.1 mol/L), saturated $NaHCO_3$ solution (20 mL, 1 mol/L) and saturated NaCl solution (15 mL), dried over anhydrous $Na_2SO_4$, filtered and concentrated under reduced pressure, and the concentrated solution was separated by silica gel column chromatography (eluent: DCM/EtOAc (v/v) = 1/1) to give a white solid (53 mg, 49%).

MS (ESI, pos.ion) m/z: 558.1 $[M+H]^+$.
$^1H$ NMR (400 MHz, $CDCl_3$) $\delta$ (ppm): 7.96 (d, *J* = 8.2 Hz, 2H), 7.68 (dd, *J* = 18.2, 8.4 Hz, 4H), 7.58 (d, *J* = 8.5 Hz, 2H), 6.87 (d, *J* = 8.4 Hz, 2H), 6.52 (d, *J* = 7.5 Hz, 1H), 6.46 (d, *J* = 8.5 Hz, 2H), 5.63 - 5.55 (m, 1H), 5.16 (s, 1H), 4.46 - 4.37 (m, 2H), 4.02 (dd, *J* = 8.5, 3.8 Hz, 2H), 3.22 - 3.01 (m, 4H), 1.30 (t, *J* = 7.4 Hz, 3H).

**Example 127 (*S*)-*N*-(2-cyano-1-(4-(ethylsulfonyl)phenyl)ethyl)-4-(2-(4-(trifluoromethyl)phenoxy)thiazol-4-yl)benzamide**

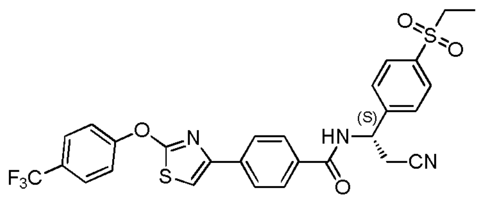

**Step 1: Synthesis of 4-bromo-2-(4-(trifluoromethyl)phenoxy)thiazole**

Under nitrogen protection, 2,4-dibromothiazole (1.80 g, 7.41 mmol), 4-(trifluoromethyl)phenol (1.00 g, 6.17 mmol) and $K_2CO_3$ (2.56 g, 18.50 mmol) were dissolved in DMF (15 mL). The mixture was reacted at 120 °C for 22 h. The reaction solution was cooled to room temperature, diluted with EtOAc (120 mL), washed with $H_2O$ (40 mL × 2) and saturated NaCl solution (40 mL × 2) successively, dried over anhydrous $Na_2SO_4$, concentrated under reduced pressure, and the concentrated solution was separated by silica gel column chromatography (eluent: PE/DCM/EtOAc (v/v/v) =

20/1/1) to give a pale yellow solid (1.51 g, 76%).
MS (ESI, pos.ion) m/z: 361.1 [M+Na]+.

**Step 2: Synthesis of methyl 4-(2-(4-(trifluoromethyl)phenoxy)thiazol-4-yl)benzoate**

Under nitrogen protection, 4-((methoxy)carbonyl)phenylboronic acid (590 mg, 3.28 mmol), 4-bromo-2-(4-(trifluoromethyl)phenoxy)thiazole (700 mg, 2.16 mmol), Pd(dppf)$Cl_2$ (163 mg, 0.22 mmol) and $Cs_2CO_3$ (1.26 g, 3.87 mmol) were dissolved in 1,4-dioxane (10 mL). The mixture was heated to 100°C and reacted for 16 h. The reaction solution was cooled to room temperature, and concentrated under reduced pressure. The residue was diluted with DCM (40 mL), washed successively with $NaHCO_3$ solution (15 mL) and saturated NaCl solution (15 mL), dried over anhydrous $Na_2SO_4$, concentrated under reduced pressure, and the concentrated solution was separated by silica gel column chromatography (eluent: PE/DCM/EtOAc (v/v/v) = 6/1/1) to give a white solid (750 mg, 92%).
MS (ESI, pos.ion) m/z: 380.0 [M+H]+.

**Step 3: Synthesis of 4-(2-(4-(trifluoromethyl)phenoxy)thiazol-4-yl)benzoic acid**

To a solution of methyl 4-(2-(4-(trifluoromethyl)phenoxy)thiazol-4-yl)benzoate (700 mg, 1.85 mmol) in THF/MeOH (2 mL/6 mL) was added a solution of LiOH·$H_2O$ (1.55 g, 36.90 mmol) in $H_2O$ (6 mL). The mixture was reacted at room temperature for 24 h. The reaction solution was concentrated under reduced pressure, and the remaining solution was added with concentrated HCl solution (1 mol/L) to adjust the pH to 4. The resulting mixture was extracted with EtOAc (20 mL × 2), and the combined organic phases were washed with saturated NaCl solution (15 mL), dried over anhydrous $Na_2SO_4$, and concentrated under reduced pressure to give a yellow solid (563 mg, 84%). MS (ESI, pos.ion) m/z: 366.1 [M+H]+.

**Step 4: Synthesis of (*S*)-*N*-(2-cyano-1-(4-(ethylsulfonyl)phenyl)ethyl)-4-(2-(4-(trifluoromethyl) phenoxy)thiazol-4-yl)benzamide**

4-(2-(4-(trifluoromethyl)phenoxy)thiazol-4-yl)benzoic acid (80 mg, 0.22 mmol), (*S*)-3-amino-3-4-(ethylsulfonyl)phenyl)propionitrile (57 mg, 0.24 mmol), EDCI (62 mg, 0.32 mmol) and HOBT (44 mg, 0.33 mmol) were dissolved in DCM (6 mL), then TEA (44 mg, 0.43 mmol) was added and the mixture was reacted at room temperature for 16 h. The reaction solution was concentrated under reduced pressure, and the concentrated solution was separated by silica gel column chromatography (eluent: DCM/EtOAc (v/v) = 5/1) to give a white solid (95 mg, 74%).

MS (ESI, pos.ion) m/z: 586.0 [M+H]+.
$^1$H NMR (400 MHz, $CDCl_3$) δ (ppm): 7.87 - 7.79 (m, 6H), 7.66 (d, *J* = 8.7 Hz, 2H), 7.63 (d, *J* = 8.2 Hz, 2H), 7.43 (d, *J* = 8.4 Hz, 2H), 7.25 (d, *J* = 6.0 Hz, 1H), 7.18 (s, 1H), 5.54 (t, *J* = 6.6 Hz, 1H), 3.11 - 3.01 (m, 4H), 1.22 (t, *J* = 7.4 Hz, 3H).

**Example 128 *N*-((*S*)-2-cyano-1-(4-ethylsulfonyl)phenyl)ethyl)-4-((S)-2-((difluoromethoxy)methyl) pyrrolidin-1-yl)benzamide**

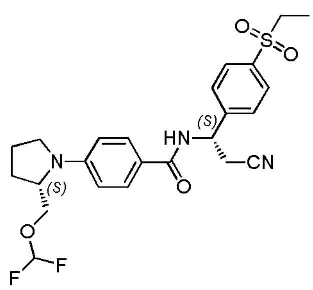

**Step 1: Synthesis of *tert-butyl* (*S*)-2-((difluoromethoxy)methyl)pyrrolidine-1-carboxylate**

To a solution of tert-butyl (2S)-2-(hydroxymethyl)pyrrolidine-1-carboxylate (3.00 g, 15.00 mmol) in DCM (5 mL) were added KOAc (8.80 g, 90.00 mmol) and $H_2O$ (5 mL), then (bromo(difluoro)methyl)-trimethylsilane (12.00 g, 59.10 mmol) was added. The mixture was reacted at room temperature for 12 h . The reaction was quenched by adding saturated NaCl solution (20 mL). The resulting mixture was extracted with DCM (20 mL × 2). The organic phases were

combined, dried over anhydrous $Na_2SO_4$, filtered, concentrated and separated by silica gel column chromatography (eluent: PE/EtOAc (v/v) = 10/1) to give colorless oily liquid (2.80 g, 75%).
MS (ESI, pos.ion) m/z: 252.3 $[M+H]^+$.

**Step 2: Synthesis of (*S*)-2-((difluoromethoxy)methyl)pyrrolidine**

To a solution of tert-butyl (2S)-2-(difluoromethoxymethyl)pyrrolidine-1-carboxylate (1.50 g, 6.00 mmol) in DCM (5 mL) was added HCl (11 mL, 59.00 mmol4.0 M methanol solution) at room temperature. The mixture was reacted for 5 h. Saturated $Na_2CO_3$ solution (10 mL) was added to quench the reaction, and the resulting mixture was extracted with DCM (10 mL × 2). The organic phases were combined, dried over anhydrous $Na_2SO_4$, filtered and concentrated to give colorless oily liquid (0.82 g, 90%).
MS (ESI, pos.ion) m/z: 152.6 $[M+H]^+$.

**Step 3: Synthesis of methyl (*S*)4-(2-((difluoromethoxy)methyl)pyrrolidin-1-yl)benzoate**

Under nitrogen protection, (2*S*)-2-(difluoromethoxymethyl)pyrrolidine (0.20 g, 1.29 mmol), methyl 4-iodobenzoate (0.51 g, 1.93 mmol), $Pd(dba)_2$ (74 mg, 0.13 mmol), $Cs_2CO_3$ (0.63 g, 1.90 mmol), 4,5-bisdiphenylphosphine-9,9-dimethylxanthene (112 mg, 0.20 mmol) were added to 1,4-dioxane (5 mL), and the mixture was reacted at 100 °C for 24 h. The mixture was cooled to room temperature, and the reaction was quenched by adding saturated NaCl solution (20 mL). The resulting mixture was extracted with EtOAc (20 mL × 2). The organic phases were combined, dried over anhydrous $Na_2SO_4$, filtered, concentrated and separated by silica gel column chromatography (eluent: PE/EtOAc (v/v) = 5/1) to give a white solid (0.18 g, 49%).
MS (ESI, pos.ion) m/z: 286.4 $[M+H]^+$.

**Step 4: Synthesis of (*S*)-4-(2-((difluoromethoxy)methyl)pyrrolidin-1-yl)benzoic acid**

To a solution of methyl (*S*)-4-(2-((difluoromethoxy)methyl)pyrrolidin-1-yl)benzoate (153 mg, 0.54 mmol) in MeOH (5 mL) were added $H_2O$ (3 mL) and LiOH (114 mg, 4.74 mmol). The mixture was reacted at room temperature for 12 h, and diluted hydrochloric acid solution was added to adjust the pH of the solution to acidity. The resulting mixture was extracted with EtOAc (5 mL × 3), and the organic phases were combined, dried over anhydrous $Na_2SO_4$, filtered, concentrated and separated by silica gel column chromatography (eluent: PE/EtOAc (v/v) = 2/1) to give a white solid (130 mg, 89%).
MS (ESI, pos.ion) m/z: 272.2 $[M+H]^+$.

**Step 5: Synthesis of *N*-((*S*)-2-cyano-1-(4-ethylsulfonyl)phenyl)ethyl)-4-((*S*)-2-((difluoromethoxy)methyl)pyrrolidin-1-yl)benzamide**

((1*S*)-2-cyano-1-(4-(ethylsulfonyl)phenyl)ethyl)ammonium chloride (96 mg, 0.35 mmol), 4-((2*S*)-2-(difluoromethoxymethyl)pyrrolidin-1-yl)benzoic acid (95 mg , 0.35 mmol) and HATU (0.16 g, 0.42 mmol) were dissolved in DCM (5 mL), then TEA (0.14 mL, 1.10 mmol) was added, and the mixture was reacted at room temperature for 12 h. The reaction was quenched by adding saturated $NH_4Cl$ solution (15 mL). The resulting mixture was extracted with DCM (10 mL × 2). The organic phases were combined, dried over anhydrous $Na_2SO_4$, filtered, concentrated and separated by silica gel column chromatography (eluent: DCM/EtOAc (v/v) = 5/1) to give a white solid (104 mg, 61%).

MS (ESI, pos.ion) m/z: 492.2 $[M+H]^+$.
$^1H$ NMR (400 MHz, $CDCl_3$) $\delta$ (ppm): 7.86 (d, *J*= 8.3 Hz, 2H), 7.73 (d, *J*= 8.7 Hz, 2H), 7.63 (d, *J*= 8.3 Hz, 2H), 7.03 (d, *J* = 7.7 Hz, 1H), 6.59 (d, *J* = 8.8 Hz, 2H), 6.23 (t, *J* = 74.5 Hz, 1H), 5.58 (dd, *J* = 13.5, 6.3 Hz, 1H), 4.02 (s, 1H), 3.93 (dd, *J* = 10.2, 3.4 Hz, 1H), 3.70 - 3.59 (m, 1H), 3.56 - 3.44 (m, 1H), 3.21 (d, *J*= 8.1 Hz, 1H), 3.09 (dt, *J*= 9.5, 6.2 Hz, 4H), 2.14 - 2.05 (m, 4H), 1.26 (t, *J*= 7.4 Hz, 3H).

**Example 129 *N*-((*S*)-2-cyano-1-(4-(ethylsulfonyl)phenyl)ethyl)-4-((2*S*,4*S*)-2-((difluoromethoxy)methyl) -4-methoxypyrrolidin-1-yl)benzamide**

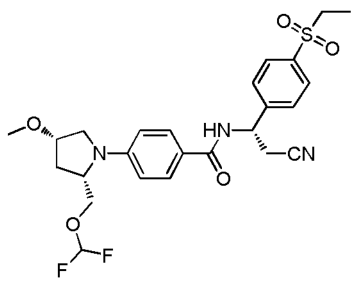

**Step 1: Synthesis of (2*S*,4*S*)-1-*tert*-butyl 2-methyl 4-((*tert*-butyldimethylsilyl) oxy)pyrrolidine-1,2-dicarboxylate**

(2*S*,5*S*)-1-*tert*-butyl 2-methyl 4-hydroxypyrrolidine-1,2-dicarboxylate (7.00 g, 29.00 mmol) and imidazole (2.30 g, 34.00 mmol) were dissolved in DCM (20 mL), then *tert*-butylchlorodimethylsilane (4.70 g, 31.00 mmol) was added. The mixture was reacted at room temperature for 5 h. The reaction was quenched by adding water. The resulting mixture was extracted with DCM (30 mL × 2). The organic phases were combined, dried over anhydrous $Na_2SO_4$, filtered, concentrated and separated by silica gel column chromatography (eluent: PE/EtOAc (v/v) = 10/1) to give colorless oily liquid (9.20 g, 90%).
MS (ESI, pos.ion) m/z: 360 $[M+H]^+$.

**Step 2: Synthesis of *tert-butyl* (2*S*,4*S*)-4-((*tert*-butyldimethylsilyl)oxy)-2-(hydroxymethyl)pyrrolidine-1-carboxylate**

To a solution of (2*S*,4*S*)-1-*tert*-butyl 2-methyl 4-((*tert*-butyldimethylsilyl)oxy)pyrrolidine-1,2-dicarboxylate (9.20 g, 26.00 mmol) in THF (50 mL) was added $LiBH_4$ (1.40 g, 64.00 mmol) at -10°C. The mixture was stirred for 5 minutes, then warmed to room temperature and reacted for 2 h. Water was added to quench the reaction. The resulting mixture was extracted with EtOAc (30 mL × 2), and the organic phases were combined, dried over anhydrous $Na_2SO_4$, filtered, concentrated and separated by silica gel column chromatography (eluent: PE/EtOAc (v/v) = 3/1) to give colorless liquid (8.5 g, 99%).
MS (ESI, pos.ion) m/z: 332 $[M+H]^+$.

**Step 3: Synthesis of *tert-butyl* (2*S*,4*S*)-4-((*tert*-butyldimethylsilyl)oxy)-2-((difluoromethoxy)methyl)pyrrolidine-1-carboxylate**

To a solution of *tert*-butyl (2*S*,4*S*)-4-((*tert*-butyldimethylsilyl)oxy)-2-(hydroxymethyl)pyrrolidine-1-carboxylate (8.50 g, 26.00 mmol) in DCM (20 mL) were added KOAc (15.00 g, 152.80 mmol) and $H_2O$ (20 mL), then [Bromo(difluoro)methyl]-trimethyl-silane (16 mL, 102.90 mmol) was added. The mixture was reacted at room temperature for 12 h . The reaction was quenched by adding water. The resulting mixture was extracted with DCM (30 mL × 2). The organic phases were combined, dried over anhydrous $Na_2SO_4$, filtered, concentrated and separated by silica gel column chromatography (eluent: PE/EtOAc (v/v) = 10/1) to give colorless liquid (8.30 g, 85%). MS (ESI, pos.ion) m/z: 382 $[M+H]^+$.

**Step 4: Synthesis of *tert*-butyl (2*S*,4*S*)-2-((difluoromethoxy)methyl) -4-hydroxypyrrolidine-1-carboxylate**

To a solution of *tert*-butyl (2*S*,4*S*)-4-((*tert*-butyldimethylsilyl)oxy)-2-((difluoromethoxy)methyl)pyrrolidine-1-carboxylate (1.80 g, 4.70 mmol) in THF (10 mL) was added tetrabutylammonium fluoride (5.70 mL, 5.70 mmol, 1 mol/L). The mixture was reacted at room temperature for 4 h. The reaction was quenched by adding water. The resulting mixture was extracted with EtOAc (10 mL × 2). The organic phases were combined, dried over anhydrous $Na_2SO_4$, filtered, concentrated and separated by silica gel column chromatography (eluent: PE/EtOAc (v/v) = 5/1) to give colorless liquid (1.20 g, 95%).
MS (ESI, pos.ion) m/z: 268 $[M+H]^+$.

**Step 5: Synthesis of *tert-butyl* (2*S*,4*S*)-2-((difluoromethoxy)methyl) -4-methoxypyrrolidine-1-carboxylate**

To a solution of tert-butyl (2S,4S)-2-((difluoromethoxy)methyl)-4-hydroxypyrrolidine -1-carboxylate (500 mg, 1.87 mmol) and NaI (0.13 g, 0.90 mmol) in DMF (5 mL) was added NaH (91 mg, 2.26 mmol). The mixture was reacted at room temperature for 0.5 h, then iodomethane (0.15 mL, 2.40 mmol) was added, and the reaction was continued for 5 h. The reaction was quenched by adding water. The resulting mixture was extracted with EtOAc (10 mL × 2). The organic phases were combined, dried over anhydrous $Na_2SO_4$, filtered, concentrated and separated by silica gel column

chromatography (eluent: PE/EtOAc (v/v) = 4/1) to give colorless liquid (0.48 g, 91%).
MS (ESI, pos.ion) m/z: 282 $[M+H]^+$.

**Step 6: Synthesis of *tert-butyl* (2*S*,4*S*)-2-((difluoromethoxy)methyl)-4-methoxypyrrolidine**

To a solution of *tert-butyl* (2S,4S)-2-((difluoromethoxy)methyl)-4-methoxypyrrolidine-1-carboxylate (500 mg, 1.78 mmol) in DCM (5 mL) was added TAF (1.32 mL, 17.80 mmol). The mixture was reacted at room temperature for 2 h . Saturated $Na_2CO_3$ solution was added to adjust pH to weakly alkaline, and the resulting mixture was extracted with DCM (10 mL × 2). The organic phases were combined, dried over anhydrous $Na_2SO_4$, filtered and concentrated to give colorless liquid (0.30 g, 93%).
MS (ESI, pos.ion) m/z: 182 $[M+H]^+$.

**Step 7: Synthesis of methyl 4-((2*S*,4*S*)-2-((difluoromethoxy)methyl) -4-methoxypyrrolidin-1-yl)benzoate**

Under nitrogen protection, (2S,4S)-2-((difluoromethoxy)methyl)-4-methoxypyrrole (0.32 g, 1.77 mmol), methyl 4-iodobenzoate (0.70 g, 2.70 mmol), $Pd(dba)_2$ (102 mg, 0.18 mmol), $Cs_2CO_3$ (0.87 g, 2.70 mmol), 4,5-bisdiphenylphosphine-9,9-dimethylxanthene (154 mg, 0.27 mmol) were added to 1,4-dioxane (5 mL), and the mixture was reacted at 100°C for 12 h. The reaction solution was cooled to room temperature, filtered, concentrated under reduced pressure, and the concentrated solution was separated by silica gel column chromatography (eluent: PE/EtOAc (v/v) = 5/1) to give a yellow solid (0.15 g, 26%).
MS (ESI, pos.ion) m/z: 316 $[M+H]^+$.

**Step 8: Synthesis of 4-((2*S*,4*S*)-2-((difluoromethoxy)methyl)-4-methoxypyrrolidin-1-yl)benzoic acid**

To a solution of methyl 4-((2*S*,4*S*)-2-((difluoromethoxy)methyl)-4-methoxypyrrolidin -1-yl)benzoate (153 mg, 0.49 mmol) in MeOH (5 mL) were added $H_2O$ (3 mL) and LiOH (0.063 g, 2.6 mmol). The mixture was reacted at room temperature for 12 h. The reaction was quenched by adding water. The resulting mixture was extracted with EtOAc (10 mL × 2). The organic phases were combined, dried over anhydrous $Na_2SO_4$, filtered, concentrated and separated by silica gel column chromatography (eluent: DCM/EtOAc (v/v) = 4/1) to give a white solid (105 mg, 72%).
MS (ESI, pos.ion) m/z: 302 $[M+H]^+$.

**Step 9: Synthesis of *N*-((*S*)-2-cyano-1-(4-(ethylsulfonyl)phenyl)ethyl)-4-((2*S*,4*S*)-2-((difluoromethoxy)methyl)-4-methoxypyrrolidin-1-yl)benzamide**

4-((2*S*,4*S*)-2-((difluoromethoxy)methyl)-4-methoxypyrrolidin-1-yl)benzoic acid (100 mg, 0.33 mmol), (*S*)-3-amino-3-(4-(ethylsulfonyl)phenyl)propionitrile (79 mg, 0.33 mmol) and HATU (153 mg, 0.40 mmol) were dissolved in DCM (25 mL), then TEA (0.1 mL, 0.80 mmol) was added and the mixture was reacted at room temperature for 12 h. The reaction was quenched by adding saturated ammonium chloride. The resulting mixture was extracted with DCM (10 mL × 2). The organic phases were combined, dried over anhydrous $Na_2SO_4$, filtered, concentrated and separated by silica gel column chromatography (eluent: DCM/EtOAc (v/v) = 4/1) to give a white solid (52 mg, 30%).

MS (ESI, pos.ion) m/z: 522 $[M+H]^+$.
$^1$H NMR (400 MHz, $CDCl_3$) δ (ppm): 7.89 (d, *J* = 8.2 Hz, 2H), 7.73 (t, *J* = 12.8 Hz, 2H), 7.65 (d, *J* = 8.1 Hz, 2H), 7.00 (d, *J* = 7.3 Hz, 1H), 6.62 (d, *J* = 8.6 Hz, 2H), 6.26 (t, *J* = 74.7 Hz, 1H), 5.60 (d, *J* = 6.8 Hz, 1H), 4.10 (dd, *J* = 20.4, 5.7 Hz, 3H), 3.91 (t, *J* = 10.6 Hz, 1H), 3.59 (d, *J* = 11.0 Hz, 1H), 3.44 (dd, *J* = 11.1, 4.7 Hz, 1H), 3.38 (s, 3H), 3.16 - 3.05 (m, 4H), 2.38 (d, *J* = 14.1 Hz, 1H), 2.21 - 2.03 (m, 1H), 1.80 (s, 2H), 1.28 (t, *J* = 7.3 Hz,3H).

**Example 130 *N*-((*S*)-2-cyano-1-(4-(ethylsulfonyl)phenyl)ethyl)-4-((2*S*,4*S*)-4-(difluoromethoxy) -2-((difluoromethoxy)methyl)pyrrolidin-1-yl)benzamide**

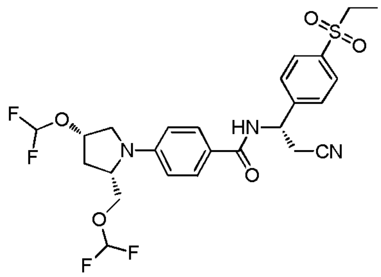

**Step 1: Synthesis of *tert*-butyl (2*S*,4*S*)-4-(difluoromethoxy)-2-((difluoromethoxy) methyl)pyrrolidine-1-carboxylate**

To a solution of *tert*-butyl (2*S*,4*S*)-2-((difluoromethoxy)methyl)-4-hydroxypyrrolidine -1-carboxylate (500 mg, 1.87 mmol) in DCM (20 mL) were added KOAc (1.10 g, 11.20 mmol) and $H_2O$ (5 mL), then (bromo(difluoro)methyl)-trimethylsilane (1.20 mL, 7.70 mmol) was added. The mixture was reacted at room temperature for 12 h. The reaction was quenched by adding water. The resulting mixture was extracted with DCM (30 mL × 2). The organic phases were combined, dried over anhydrous $Na_2SO_4$, filtered, concentrated and separated by silica gel column chromatography (eluent: PE/EtOAc (v/v) = 5/1) to give colorless oily liquid (0.52 g, 88%).
MS (ESI, pos.ion) m/z: 318 $[M+H]^+$.

**Step 2: Synthesis of (2*S*,4*S*)-4-(difluoromethoxy)-2-((difluoromethoxy)methyl)pyrrolidine**

To a solution of tert-butyl (2S,4S)-4-(difluoromethoxy)-2-((difluoromethoxy)methyl) pyrrolidine-1-carboxylate (324 mg, 1.02 mmol) in DCM (8 mL) was added TFA (0.76 mL, 10.00 mmol). The mixture was reacted at room temperature for 3 h . Saturated $Na_2CO_3$ solution was added to adjust pH to weakly alkaline, and the resulting mixture was extracted with DCM (10 mL × 2). The organic phases were combined, dried over anhydrous $Na_2SO_4$, filtered and concentrated to give colorless liquid (0.20 g, 90%).
MS (ESI, pos.ion) m/z: 218 $[M+H]^+$.

**Step 3: Synthesis of methyl 4-((2*S*,4*S*)-4-(difluoromethoxy)-2-((difluoromethoxy) methyl)pyrrolidin-1-yl)benzoate**

Under nitrogen protection, (2*S*,4*S*)-4-(difluoromethoxy)-2-((difluoromethoxy) methyl)pyrrolidine (0.20 g, 0.92 mmol), methyl 4-iodobenzoate (0.40 g, 1.50 mmol), $Pd(dba)_2$ (59 mg, 0.10 mmol), $Cs_2CO_3$ (0.50 g, 2.00 mmol), 4,5-bisdiphenylphosphine-9,9-dimethylxanthene (89 mg, 0.15 mmol) were added to 1,4-dioxane (5 mL), and the mixture was reacted at 100°C for 12 h. The reaction solution was cooled to room temperature, filtered, concentrated under reduced pressure, and the concentrated solution was separated by silica gel column chromatography (eluent: PE/EtOAc (v/v) = 5/1) to give a yellow solid (0.18 g, 56%).
MS (ESI, pos.ion) m/z: 352 $[M+H]^+$.

**Step 4: Synthesis of 4-((2*S*,4*S*)-4-(difluoromethoxy)-2-((difluoromethoxy) methyl)pyrrolidin-1-yl)benzoic acid**

To a solution of methyl **4-((2*S*,4*S*)-4-(difluoromethoxy)-2-((difluoromethoxy)** methyl)pyrrolidin-1-yl)benzoate (153 mg, 0.44 mmol) in MeOH (5 mL) were added $H_2O$ (3 mL) and LiOH (0.063 g, 2.60 mmol). The mixture was reacted at room temperature for 12 h. Water was added to quench the reaction. Dilute hydrochloric acid was added to adjust the pH of the solution to be weakly acidic. The resulting mixture was extracted with EtOAc (10 mL × 2). The organic phases were combined, dried over anhydrous $Na_2SO_4$, filtered, concentrated and separated by silica gel column chromatography (eluent: DCM/EtOAc (v/v) = 4/1) to give a white solid (115 mg, 78%).
MS (ESI, pos.ion) m/z: 338 $[M+H]^+$.

**Step 5: Synthesis of *N*-((*S*)-2-cyano-1-(4-(ethylsulfonyl)phenyl)ethyl) -4-((2*S*,4*S*)-4-(difluoromethoxy)-2-((difluoromethoxy)methyl)pyrrolidin-1-yl)benzamide**

4-((2*S*,4*S*)-4-(difluoromethoxy)methyl)-2-((difluoromethoxy)methyl)pyrrolidin-1-yl)b enzoic acid (100 mg, 0.30 mmol), (*S*)-3-amino-3-(4-(ethylsulfonyl)phenyl)propionitrile hydrochloride (70 mg, 0.29 mmol) and HATU (136 mg, 0.35 mmol) were dissolved in DCM (25 mL), then TEA (0.1 mL, 0.8 mmol) was added and the mixture was reacted at room temperature for 12 h. The reaction was quenched by adding saturated ammonium chloride. The resulting mixture was

extracted with DCM (10 mL × 2). The organic phases were combined, dried over anhydrous $Na_2SO_4$, filtered, concentrated and separated by silica gel column chromatography (eluent: PE/EtOAc (v/v) = 1/1) to give a white solid (98 mg, 59%).

MS (ESI, pos.ion) m/z: 558 $[M+H]^+$.
$^1H$ NMR (400 MHz, $CDCl_3$) δ (ppm): 7.87 (d, *J*= 7.8 Hz, 2H), 7.76 (d, *J*= 8.4 Hz, 2H), 7.64 (d, *J*= 7.9 Hz, 2H), 7.17 (s, 1H), 6.62 (d, *J* = 8.3 Hz, 2H), 6.29 (d, *J* = 19.9 Hz, 2H), 5.59 (d, *J* = 6.5 Hz, 1H), 5.05 (s, 1H), 4.11 (d, *J* = 7.7 Hz, 2H), 3.90 (d, *J* = 8.8 Hz, 1H), 3.64 (dd, *J* = 26.9, 7.9 Hz, 2H), 3.11 (dd, *J*= 15.4, 7.5 Hz, 4H), 1.27 (t, *J*= 7.0 Hz, 3H).

**Example 131 *N*-((*S*)-2-cyano-1-(4-(ethylsulfonyl)phenyl)ethyl)-4-((2*S*,4*R*)-2-((difluoromethoxy)methyl) -4-hydroxypyrrolidin-1-yl)benzamide**

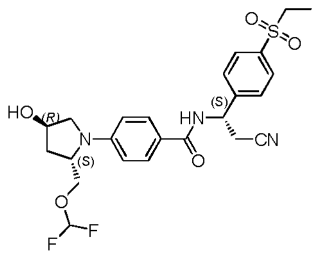

HATU (172 mg, 0.45 mmol), (*S*)-3-amino-3-(4-(ethylsulfonyl)phenyl)propionitrile hydrochloride (105 mg, 0.38 mmol), 4-((2*S*,4*R*)-2-((difluoromethoxy)methyl) -4-hydroxypyrrolidin-1-yl)benzoic acid (100 mg, 0.35 mmol) and TEA (105 mg, 1.04 mmol) were successively added to DCM (5 mL), and the mixture was reacted at room temperature for 20 h. The reaction solution was diluted with DCM (30 mL), washed successively with HCl solution (15 mL, 0.5 mol/L) and saturated NaCl solution (15 mL), dried over anhydrous $Na_2SO_4$, concentrated under reduced pressure, and the concentrated solution was separated by silica gel column chromatography (eluent: DCM/EtOAc (v/v) = 1/1) to give a pale yellow solid (125 mg, 71%).

MS (ESI, pos.ion) m/z: 508.1 $[M+H]^+$.
$^1H$ NMR (400 MHz, $CDCl_3$) δ (ppm): 7.85 (d, *J*= 8.3 Hz, 2H), 7.69 (d, *J*= 8.7 Hz, 2H), 7.60 (d, *J* = 8.3 Hz, 2H), 7.53 (d, *J* = 7.8 Hz, 1H), 6.53 (d, *J* = 8.7 Hz, 2H), 6.15 (t, *J*= 74.4 Hz, 1H), 5.53 (q, *J* = 6.5 Hz, 1H), 4.63 - 4.55 (m, 1H), 4.16 (s, 1H), 3.91 (dd, *J* = 10.2, 2.8 Hz, 1H), 3.77 (dd, *J* = 10.2, 6.5 Hz, 1H), 3.64 (dd, *J* = 10.1, 5.7 Hz, 1H), 3.21 - 3.15 (m, 1H), 3.08 (q, *J* = 7.4 Hz, 2H), 3.03 (d, *J* = 6.3 Hz, 2H), 2.29-2.22 (m, 1H), 2.17-2.09 (m, 1H), 1.24 (t, *J* = 7.4 Hz, 3H).

**Example 132 4-(2,5-bis((difluoromethoxy)methyl)pyrrolidin-1-yl)-*N*-((*S*)-2-cyano -1-(4-(ethylsulfonyl) phenyl)ethyl)benzamide**

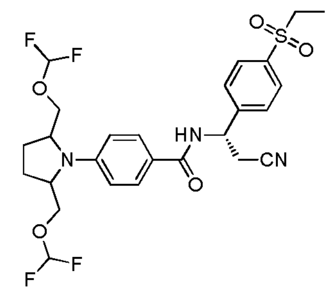

**Step 1: Synthesis of *tert-butyl* 2,5-bis(hydroxymethyl)pyrrolidine-1-carboxylate**

*tert*-Butyl 2,5-di(ethoxyformyl)pyrrolidine-1-carboxylate (2.0 g, 6.30 mmol) was dissolved in THF (10 mL). The reaction solution was cooled to -10°C, then $LiBH_4$ (0.28 g, 13.00 mmol) was added, and the mixture was warmed to

room temperature and reacted for 4 h. The reaction was quenched by adding water. The resulting mixture was extracted with EtOAc (30 mL × 2). The organic phases were combined, dried over anhydrous $Na_2SO_4$, filtered and concentrated to give colorless oily liquid (1.50 g, 99%).
MS (ESI, pos.ion) m/z: 232 $[M+H]^+$.

**Step 2: Synthesis of *tert*-butyl 2,5-bis((difluoromethoxy)methyl)pyrrolidine-1-carboxylate**

To a solution of tert-butyl 2,5-bis(hydroxymethyl)pyrrolidine-1-carboxylate (1.50 g, 6.50 mmol) in DCM (5 mL) were added KOAc (7.40 g, 75.00 mmol) and $H_2O$ (5 mL), then (bromo(difluoro)methyl)-trimethylsilane (7.80 mL, 50.00 mmol) was added. The mixture was reacted at room temperature for 12 h. The reaction was quenched by adding water. The resulting mixture was extracted with DCM (30 ml × 2). The organic phases were combined, dried over anhydrous $Na_2SO_4$, filtered, concentrated and separated by silica gel column chromatography (eluent: PE/EtOAc (v/v) = 5/1) to give colorless liquid (2.10 g, 98%).
MS (ESI, pos.ion) m/z: 332 $[M+H]^+$.

**Step 3: Synthesis of 2,5-bis((difluoromethoxy)methyl)pyrrolidine**

To a solution of tert-butyl 2,5-bis((difluoromethoxy)methyl)pyrrolidine-1-carboxylate (2.10 g, 6.30 mmol) in DCM (10 mL) was added TFA (2.80 mL, 31.00 mmol). The mixture was reacted at room temperature for 4 h. Saturated $Na_2CO_3$ solution was added to adjust pH to weakly alkaline, and the resulting mixture was extracted with DCM (10 mL × 2). The organic phases were combined, dried over anhydrous $Na_2SO_4$, filtered and concentrated to give colorless liquid (1.40 g, 96%).
MS (ESI, pos.ion) m/z: 232 $[M+H]^+$.

**Step 4: Synthesis of methyl 4-(2,5-bis((difluoromethoxy)methyl)pyrrolidin-1-yl)benzoate**

Under nitrogen protection, 2,5-bis((difluoromethoxy)methyl)pyrrolidine (470 mg, 2.03 mmol), methyl 4-iodobenzoate (0.80 g, 3.10 mmol), $Pd(dba)_2$ (117 mg, 0.20 mmol), $Cs_2CO_3$ (1.00 g, 3.10 mmol), 4,5-bisdiphenylphosphine-9,9-dimethylxanthene (176 mg, 0.30 mmol) were added to 1,4-dioxane (5 mL), and the mixture was reacted at 110 °C for 12 h. The reaction solution was cooled to room temperature, filtered, concentrated under reduced pressure, and the concentrated solution was separated by silica gel column chromatography (eluent: PE/EtOAc (v/v) = 5/1) to give a yellow solid (0.18 g, 25%).
MS (ESI, pos.ion) m/z: 366 $[M+H]^+$.

**Step 5: Synthesis of 4-(2,5-bis((difluoromethoxy)methyl)pyrrolidin-1-yl)benzoic acid**

To a solution of methyl 4-(2,5-bis((difluoromethoxy)methyl)pyrrolidin-1-yl)benzoate (83 mg, 0.23mmol) in MeOH (5 mL) were added $H_2O$ (3 mL) and LiOH (13 mg, 0.56 mmol). The mixture was reacted at room temperature for 24 h. Water was added to quench the reaction. Dilute hydrochloric acid was added to adjust the pH of the solution to be weakly acidic. The resulting mixture was extracted with EtOAc (10 mL × 2). The organic phases were combined, dried over anhydrous $Na_2SO_4$, filtered, concentrated and separated by silica gel column chromatography (eluent: DCM/EtOAc (v/v) = 4/1) to give a white solid (76 mg, 95%).
MS (ESI, pos.ion) m/z: 352 $[M+H]^+$.

**Step 6: Synthesis of 4-(2,5-bis((difluoromethoxy)methyl)pyrrolidin-1-yl) -*N*-((*S*)-2-cyano-1-(4-(ethylsulfonyl)phenyl)ethyl)benzamide**

4-(2,5-Bis((difluoromethoxy)methyl)pyrrolidin-1-yl)benzoic acid (85 mg, 0.24 mmol), (*S*)-3-amino-3-(4-(ethylsulfonyl)phenylpropionitrile hydrochloride (69 mg, 0.29 mmol) and HATU (111 mg, 0.29 mmol) were dissolved in DCM (25 mL), then TEA (0.072 mL, 0.55 mmol) was added and the mixture was reacted at room temperature for 12 h. The reaction was quenched by adding saturated ammonium chloride. The resulting mixture was extracted with DCM (10 mL × 2). The organic phases were combined, dried over anhydrous $Na_2SO_4$, filtered, concentrated and separated by silica gel column chromatography (eluent: PE/EtOAc (v/v) = 1/1) to give a white solid (98 mg, 71%). MS (ESI, pos.ion) m/z: 572 $[M+H]^+$.
$^1H$ NMR (400 MHz, $CDCl_3$) δ (ppm): 7.89 (d, *J* = 8.2 Hz, 2H), 7.75 (d, *J* = 8.7 Hz, 2H), 7.64 (d, *J* = 8.2 Hz, 2H), 6.91 (d, *J* = 7.7 Hz, 1H), 6.71 (d, *J* = 8.8 Hz, 2H), 6.24 (t, *J* = 74.3 Hz, 2H), 5.59 (dd, *J* = 13.1, 6.2 Hz, 1H), 4.02 (dd, *J* = 17.9, 7.6 Hz, 4H), 3.78 (dd, *J* = 9.8, 7.2 Hz, 2H), 3.21 - 3.01 (m, 4H), 2.25 - 2.10 (m, 2H), 2.09 - 1.98 (m, 2H), 1.28 (t, *J* = 7.4 Hz, 3H).

**Example 133 (*S*)-*N*-(2-cyano-1-(4-(ethylsulfonyl)phenyl)ethyl)-4-((5-fluoro-1*H*-indazol-1-yl) methyl)benzamide**

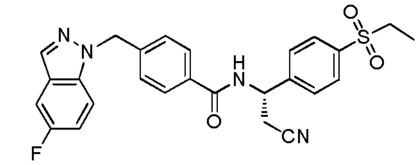

**Step 1: Synthesis of methyl 4-((5-fluoro-1*H*-indazol-1-yl)methyl)benzoate and methyl 4-((5-fluoro-2*H*-indazol-2-yl)methyl)benzoate**

To a solution of 5-fluoro-1*H*-indazole (310 mg, 2.28 mmol) and $K_2CO_3$ (930 mg, 5.19 mmol) in ACN (5 mL, 95.7 mmol) was added methyl 4-(bromomethyl)benzoate (550 mg, 2.40 mmol). The mixture was reacted at room temperature for 13 h. After the reaction was completed, the reaction solution was diluted with DCM (20 mL), washed successively with $Na_2CO_3$ solution (20 mL) and saturated NaCl solution (15 mL), dried over anhydrous $Na_2SO_4$, filtered and concentrated under reduced pressure. The concentrated solution was separated by silica gel column chromatography (eluent: PE/EtOAc (v/v) = 1/1) to give methyl 4-((5-fluoro-1*H*-indazol-1-yl)methyl)carboxylate (330 mg, 48%) as pale yellow solid and methyl 4-((5-fluoro-2*H*-indazol-2-yl)methyl)carboxylate (320 mg, 47%) as a pale yellow solid.
MS (ESI, pos.ion) m/z: 285.1 $[M+H]^+$, 285.1 $[M+H]^+$.

**Step 2: Synthesis of 4-((5-fluoro-1*H*-indazol-1-yl)methyl)benzoic acid**

To a solution of methyl 4-((5-fluoro-1*H*-indazol-1-yl)methyl)benzoate (200 mg, 0.70 mmol) in MeOH (5 mL) was added LiOH (90 mg, 2.10 mmol). The mixture was reacted at room temperature for 10 h. After the reaction was completed, HCl solution (15 mL, 0.1mol/L) was added to the reaction solution. The mixture was extracted with DCM (20 mL × 3). The organic phases were combined, washed with saturated NaCl solution (15 mL), dried over anhydrous $Na_2SO_4$, concentrated under reduced pressure, and the concentrated solution was separated by silica gel column chromatography (eluent: DCM/MeOH (v/v) = 10/1) to give a white solid (160 mg, 84 %).

**Step 3: (*S*)-*N*-(2-cyano-1-(4-(ethylsulfonyl)phenyl)ethyl)-4-((5-fluoro-1*H*-indazol-1-yl) methyl)benzamide**

(*S*)-3-Amino-3-(4-(ethylsulfonyl)phenyl)propionitrile hydrochloride (80 mg, 0.34 mmol), 4-((5-fluoro-1*H*-indazol-1-yl)methyl)benzoic acid (81 mg , 0.30 mmol), HATU (151 mg, 0.39 mmol) and DIPEA(0.3 mL, 2 mmol) were dissolved in DCM (10 mL), and the reaction solution was stirred at room temperature for 16 h. The reaction solution was diluted with DCM (50 mL), washed successively with $NaHCO_3$ solution (33 mL) and saturated NaCl solution (45 mL), dried over anhydrous $Na_2SO_4$, concentrated under reduced pressure, and the concentrated solution was separated by silica gel column chromatography (eluent: PE/EtOAc (v/v) = 1/1) to give a white solid (130 mg, 88.43%).

MS (ESI, pos.ion) m/z: 491.2 $[M+H]^+$.
$^1H$ NMR (400 MHz, $CDCl_3$) δ (ppm): 8.01 (s, 1H), 7.90 (d, *J* = 8.2 Hz, 2H), 7.77 (d, *J* = 8.1 Hz, 2H), 7.65 (d, *J* = 8.2 Hz, 2H), 7.37 (dd, *J* = 8.6, 2.1 Hz, 1H), 7.24 (t, *J* = 6.3 Hz, 2H), 7.12 (td, *J* = 8.9, 2.1 Hz, 1H), 6.98 (d, *J* = 7.7 Hz, 1H), 5.62 (s, 2H), 5.58 (d, *J* = 7.2 Hz, 1H), 3.10 (dd, *J*= 13.3, 7.4 Hz, 4H), 1.27 (t, *J* = 7.4 Hz, 4H).

**Example 134 (*S*)-*N*-(2-cyano-1-(4-(ethylsulfonyl)phenyl)ethyl)-4-((5-fluoro-2*H*-indazol-2-yl) methyl)benzamide**

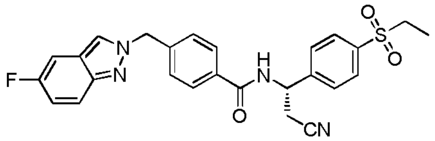

According to the method of step 1 of Example 135, the intermediate methyl 4-((5-fluoro-2*H*-indazol-2-yl)methyl)benzoate was prepared, which was used to prepare the title compound as a white solid according to the methods

of step 2 to step 3.

MS (ESI, pos.ion) m/z: 491.1 $[M+H]^+$.
$^1H$ NMR (400 MHz, DMSO-$d_6$) δ (ppm): 9.26 (d, *J* = 8.2 Hz, 1H), 8.49 (s, 1H), 7.88 (dd, *J* = 8.1, 2.5 Hz, 4H), 7.72 (d, *J*= 8.2 Hz, 2H), 7.66 (dd, *J*= 9.2, 4.7 Hz, 1H), 7.44 (d, *J* = 7.7 Hz, 3H), 7.14 (td, *J*= 9.3, 2.3 Hz, 1H), 5.72 (s, 2H), 5.52 (dd, *J*= 14.8, 8.3 Hz, 1H), 3.27 (q, *J*= 7.3 Hz, 2H), 3.20 - 3.12 (m, 2H), 1.08 (t, *J* = 7.3 Hz, 3H).

**Example 135 (*S*)-*N*-(2-cyano-1-(4-(ethylsulfonyl)phenyl)ethyl)-4-(3,4-dihydroquinolin -1(2*H*)-yl)benzamide**

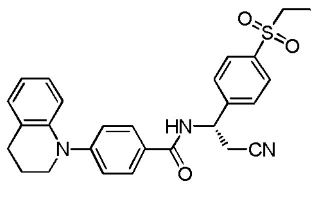

**Step 1: Synthesis of methyl 4-(3,4-dihydroquinolin-1(2*H*)-yl)benzoate**

Under nitrogen protection, 1,2,3,4-tetrahydroquinoline (0.20 g, 1.46 mmol), methyl 4-iodobenzoate (0.51 g, 1.93 mmol), $Pd(dba)_2$ (74 mg, 0.13 mmol), $Cs_2CO_3$ (0.63 g, 1.90 mmol), 4,5-bisdiphenylphosphine-9,9-dimethylxanthene (112 mg, 0.19 mmol) were added to 1,4-dioxane (5 mL), and the mixture was reacted at 100 °C for 12 h. The reaction solution was cooled to room temperature, filtered, and concentrated under reduced pressure. The concentrated solution was separated by silica gel column chromatography (eluent: PE/EtOAc (v/v) = 5/1) to give a yellow solid (0.18 g, 47%).
MS (ESI, pos.ion) m/z: 268 $[M+H]^+$.

**Step 2: Synthesis of 4-(3,4-dihydroquinolin-1(2*H*)-yl)benzoic acid**

To a solution of methyl 4-(3,4-dihydroquinolin-1(2*H*)-yl)benzoate (163 mg, 0.61 mmol) in MeOH (5 mL) were added $H_2O$ (3 mL) and LiOH (13 mg, 0.6 mmol). The mixture was reacted at room temperature for 12 h. Water was added to quench the reaction. Dilute hydrochloric acid was added to adjust the pH of the solution to be weakly acidic. The resulting mixture was extracted with EtOAc (10 mL × 2). The organic phases were combined, dried over anhydrous $Na_2SO_4$, filtered, concentrated and separated by silica gel column chromatography (eluent: DCM/EtOAc (v/v) = 4/1) to give a white solid (140 mg, 91%).
MS (ESI, pos.ion) m/z: 254 $[M+H]^+$.

**Step 3: Synthesis of (*S*)-*N*-(2-cyano-1-(4-(ethylsulfonyl)phenyl)ethyl)-4-(3,4-dihydroquinolin-1(2*H*)-yl)benzamide**

4-(3,4-dihydroquinolin-1(2*H*)-yl)benzoic acid (67 mg, 0.26 mmol), (*S*)-3-amino-3-(4-(ethylsulfonyl)phenylpropionitrile hydrochloride (75 mg, 0.31 mmol) and HATU (121 mg, 0.32 mmol) were dissolved in DCM (12 mL), then TEA (0.08 mL, 0.6 mmol) was added and the mixture was reacted at room temperature for 12 h. The reaction was quenched by adding saturated ammonium chloride. The resulting mixture was extracted with DCM (10 mL × 2). The organic phases were combined, dried over anhydrous $Na_2SO_4$, filtered, concentrated and separated by silica gel column chromatography (eluent: DCM/EtOAc (v/v) = 4/1) to give a white solid (25 mg, 20%).

MS (ESI, pos.ion) m/z: 474 $[M+H]^+$.
$^1H$ NMR (400 MHz, $CDCl_3$) δ (ppm): 7.90 (d, *J*= 8.3 Hz, 2H), 7.77 (d, *J*= 8.7 Hz, 2H), 7.67 (d, *J* = 8.3 Hz, 2H), 7.23 (d, *J* = 8.7 Hz, 2H), 7.16 - 6.98 (m, 4H), 6.87 (t, *J* = 7.2 Hz, 1H), 5.63 (dd, *J* = 13.4, 6.3 Hz, 1H), 3.67 (s, 2H), 3.12 (d, *J* = 7.4 Hz, 4H), 2.09 - 1.96 (m, 2H), 1.29 (t, *J* = 7.3 Hz, 4H).

**Example 136 (*S*)-*N*-(2-cyano-1-(4-(ethylsulfonyl)phenyl)ethyl)-4-(3,4-dihydroisoquinolin -2(1*H*)-yl)benzamide**

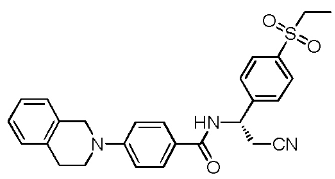

The raw material 1,2,3,4-tetrahydroquinoline in step 1 of Example 137 was replaced with 1,2,3,4-tetrahydroisoquinoline, which was used with another raw material methyl 4-iodobenzoate according to the methods of step 1 to step 3 of Example 137 to prepare the title compound as a white solid.

MS (ESI, pos.ion) m/z: 474 $[M+H]^+$.
$^1$H NMR (400 MHz, $CDCl_3$) δ (ppm): 7.86 (d, *J*= 8.3 Hz, 2H), 7.78 (d, *J*= 8.9 Hz, 2H), 7.64 (d, *J*= 8.3 Hz, 2H), 7.33 (d, *J* = 7.8 Hz, 1H), 7.20 (td, *J* = 9.1, 4.4 Hz, 4H), 6.86 (d, *J* = 8.9 Hz, 2H), 5.60 (d, *J*= 7.2 Hz, 1H), 4.48 (s, 2H), 3.62 (t, *J*= 5.9 Hz, 2H), 3.09 (dd, *J*= 10.3, 6.9 Hz, 4H), 2.98 (t, *J* = 5.8 Hz, 2H), 2.81 (s, 4H), 1.26 (dd, *J* = 9.6, 5.2 Hz, 3H).

**Example 137 (*S*)-*N*-(4-((2-cyano-1-(4-(ethylsulfonyl)phenyl)ethyl)carbamoyl)phenyl)-*N*-(2-(difluoromethoxy)ethyl)-4-(trifluoromethyl)benzamide**

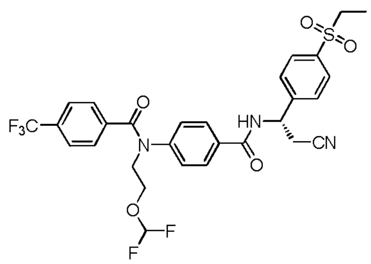

**Step 1: Synthesis of 2-(difluoromethoxy)ethylamine**

To a solution of ***tert*-butyl** (2-(difluoromethoxy)ethyl)carbamate (800 mg, 3.79 mmol) in DCM (5 mL) was added TFA (1.6 mL, 21 mmol). The reaction solution was reacted at room temperature for 4 h. The reaction solution was concentrated under reduced pressure. The concentrated solution was diluted with DCM (10 mL), washed successively with saturated $Na_2CO_3$ solution (30 mL) and saturated NaCl solution (15 mL), dried over anhydrous $Na_2SO_4$, filtered and concentrated under reduced pressure to give colorless liquid (420 mg, 100%).

**Step 2: Synthesis of methyl 4-((2-(difluoromethoxy)ethyl)amino)benzoate**

To a solution of methyl 4-iodobenzoate (1.20 g, 4.58 mmol) and 2-(difluoromethoxy)ethylamine (410 mg, 3.69 mmol) in 1,4-dioxane (38 mL) were added $Cs_2CO_3$ (2.25 g, 6.90 mmol), 4,5-bisdiphenylphosphine-9,9-dimethylxanthene (200 mg, 0.33 mmol) and $Pd_2(dba)_3$ (300 mg, 0.32 mmol). Under nitrogen protection, the mixture was reacted at 100°C for 13 h. The reaction solution was added with $NaHCO_3$ solution (40 mL) and diluted with DCM (20 mL). The aqueous phase was extracted with DCM (20 mL). The combined organic phases were dried over anhydrous $Na_2SO_4$, filtered and concentrated under reduced pressure. The concentrated solution was separated by silica gel column chromatography (eluent: PE/EtOAc (v/v) = 2/1) to give a pale yellow solid (320 mg, 35%).
MS (ESI, pos.ion) m/z: 246.1 $[M+H]^+$.

**Step 3: Synthesis of methyl 4-(*N*-(2-(difluoromethoxy)ethyl)-4-(trifluoromethyl)benzamido)benzoate**

Methyl 4-((2-(difluoromethoxy)ethyl)amino)benzoate (240 mg, 0.98 mmol) and $K_2CO_3$ (590 mg, 3.29 mmol) were dissolved in DMF (10 mL). 4-(Trifluoromethyl)benzoyl chloride (300 mg, 1.44 mmol) was added dropwise to the reaction solution at room temperature, and the mixture was reacted at 75°C for 10 h. The reaction solution was diluted with DCM (10 mL), washed successively with saturated $Na_2CO_3$ solution (10 mL) and saturated NaCl solution (15 mL), dried over anhydrous $Na_2SO_4$, filtered and concentrated under reduced pressure. The concentrated solution was separated by silica gel column chromatography (eluent: PE/EtOAc (v/v) = 1/1) to give a pale yellow solid (300 mg, 74%).

MS (ESI, pos.ion) m/z: 418.1 [M+H]+.

**Step 4: Synthesis of 4-(*N*-(2-(difluoromethoxy)ethyl)-4-(trifluoromethyl)benzamido)benzoic acid**

To a solution of methyl 4-(*N*-(2-(difluoromethoxy)ethyl)-4-(trifluoromethyl)benzamido)benzoate (300 mg, 0.72 mmol) in THF (5 mL) was added $H_2O$ (1 mL), then LiOH (100 mg, 2.34 mmol) was slowly added. The reaction solution was reacted at room temperature for 8 h. The resulting mixture was added with HCl solution (9 mL, 0.1 mol/L), and extracted with DCM (10 mL × 3). The organic phases were combined, washed with saturated NaCl solution (15 mL), dried over anhydrous $Na_2SO_4$, filtered and concentrated under reduced pressure to give a crude product was a white solid (220 mg, 76%).

**Step 5: Synthesis of (*S*)-*N*-(4-((2-cyano-1-(4-(ethylsulfonyl)phenyl)ethyl)carbamoyl)phenyl) -*N*-(2-(difluoromethoxy)ethyl)-4-(trifluoromethyl)benzamide**

(*S*)-3-Amino-3-(4-(ethylsulfonyl)phenyl)propionitrile (89 mg, 0.37 mmol), 4-(*N*-(2-(difluoromethoxy)ethyl)-4-(trifluoromethyl)benzamido)benzoic acid (60 mg, 0.15 mmol), HATU (150 mg, 0.38 mmol) and DIPEA (0.2 mL, 1 mmol) were dissolved in DCM (20 mL), and the reaction solution was stirred at room temperature for 13 h. The reaction solution was diluted with DCM (50 mL), washed successively with HCl solution (15 mL, 0.1 mol/L), saturated $NaHCO_3$ solution (15 mL) and saturated NaCl solution (15 mL), dried over anhydrous $Na_2SO_4$, filtered and concentrated under reduced pressure. The concentrated solution was separated by silica gel column chromatography (eluent: DCM/EtOAc (v/v) = 1/1) to give a white solid(50 mg, 54%).

MS (ESI, pos.ion) m/z: 624.2 [M+H]+.
$^1$H NMR (400 MHz, $CDCl_3$) δ (ppm): 7.93 (d, *J*= 8.2 Hz, 2H), 7.71 (d, *J*= 8.4 Hz, 2H), 7.64 (d, *J* = 8.2 Hz, 2H), 7.45 (dd, *J* = 21.2, 8.2 Hz, 4H), 7.18 (d, *J* = 8.4 Hz, 2H), 6.71 (d, *J* = 7.3 Hz, 1H), 6.23 (t, *J* = 74.1 Hz, 1H), 5.56 (dd, *J* = 12.5, 6.3 Hz, 1H), 4.18 (s, 4H), 3.22 - 3.02 (m, 4H), 1.26 (t, *J*= 7.5 Hz, 3H).

**Example 138 (*S*)-1-(2-cyano-1-(4-(ethylsulfonyl)phenyl)ethyl)-3-(4-(1,1,1,3,3,3-hexafluoro-2-hydroxypropan-2-yl)phenyl)urea**

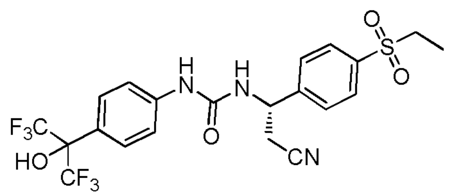

**Step 1: Synthesis of phenyl (4-(1,1,1,3,3,3-hexafluoro-2-hydroxypropan -2-yl)phenyl)carbamate**

To a solution of 2-(4-aminophenyl)-1,1,1,3,3,3-hexafluoro-2-propanol (500 mg, 1.93 mmol) and pyridine (0.2 mL, 2.50 mmol) in EtOAc (10 mL) was slowly added dropwise phenyl chloroformate (0.27 mL, 2.12 mmol). The mixture was reacted for 8 h at room temperature. The reaction solution was filtered, and the filtrate was concentrated under reduced pressure to give a white solid (731 mg, 100%).
MS (ESI, pos.ion) m/z: 380.2 [M+H]+.

**Step 2: Synthesis of (*S*)-1-(2-cyano-1-(4-(ethylsulfonyl)phenyl)ethyl)-3-(4-(1,1,1,3,3,3 -hexafluoro-2-hydroxypropan-2-yl)phenyl)urea**

Under nitrogen protection, to a solution of phenyl 4-(1,1,1,3,3,3-hexafluoro-2-hydroxypropan-2-yl)phenyl)carbamate (350 mg, 0.92 mmol) in ACN (8 mL) were added TEA (0.4 mL, 2.88 mmol) and (*S*)-3-amino-3-(4-(ethylsulfonyl)phenyl)propionitrile hydrochloride (280 mg, 1.02 mmol). The mixture was reacted at 85°C for 5 h. The reaction solution was concentrated under reduced pressure, and the concentrated solution was separated by silica gel column chromatography (eluent: PE/EtOAc (v/v) = 3/2) to give a white solid (400 mg, 83%).

MS (ESI, pos.ion) m/z: 523.7 [M+H]+.

$^1$H NMR (400 MHz, $CDCl_3$) δ (ppm): 7.86 (d, *J* = 8.3 Hz, 2H), 7.57 (dd, *J* = 8.3, 3.8 Hz, 4H), 7.39 (d, *J* = 8.8 Hz, 2H), 5.27 (dd, *J* = 6.5, 4.4 Hz, 1H), 3.10 (q, *J* = 7.4 Hz, 2H), 3.01 (dd, *J* = 16.9, 5.9 Hz, 1H), 2.88 (d, *J* = 5.4 Hz, 1H), 1.24 (d, *J* = 7.4 Hz, 3H).

**Example 139 (*S*)-1-(2-cyano-1-(4-(ethylsulfonyl)phenyl)ethyl)-3-(3-fluoro-5-(trifluoromethyl) phenyl)urea**

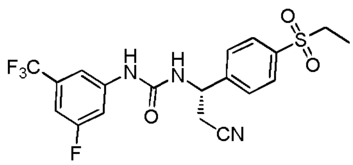

**Step 1: Synthesis of phenyl (3-fluoro-5-(trifluoromethyl)phenyl)carbamate**

To a solution of 3-fluoro-5-(trifluoromethyl)aniline (500 mg, 2.79 mmol) and pyridine (0.3 mL, 3.73 mmol) in EtOAc (10 mL) was slowly added dropwise phenyl chloroformate (0.4 mL, 3.19 mmol). The mixture was reacted for 12 h at room temperature. The reaction solution was filtered, and the filtrate was concentrated under reduced pressure. The concentrated solution was separated by silica gel column chromatography (eluent: PE/EtOAc (v/v) = 5/1) to give colorless liquid (800 mg, 96%).
MS (ESI, pos.ion) m/z: 300.2 $[M+H]^+$.

**Step 2: Synthesis of (*S*)-1-(2-cyano-1-(4-(ethylsulfonyl)phenyl)ethyl)-3-(3-fluoro -5-(trifluoromethyl)phenyl)urea**

Under nitrogen protection, to a solution of (3-fluoro-5-(trifluoromethyl)phenyl)carbamate (200 mg, 0.67 mmol) in ACN (8 mL) were added TEA (0.3 mL, 2.16 mmol) and (*S*)-3-amino-3-(3-(ethylsulfonyl)phenyl)propionitrile hydrochloride (202 mg, 0.74 mmol). The mixture was reacted at 85°C for 16 h. The reaction solution was concentrated under reduced pressure, and the concentrated solution was separated by silica gel column chromatography (eluent: DCM/EtOAc (v/v) = 4/1) to give a white solid (250 mg, 84%).

MS (ESI, pos.ion) m/z: 444.0 $[M+H]^+$.
$^1$H NMR (400 MHz, $CDCl_3$) δ (ppm): 7.80 (d, *J* = 8.3 Hz, 2H), 7.69 (s, 1H), 7.59 (d, *J* = 8.3 Hz, 2H), 7.51 (d, *J* = 10.5 Hz, 1H), 7.39 (s, 1H), 6.95 (d, *J* = 8.1 Hz, 1H), 6.20 (d, *J* = 7.5 Hz, 1H), 5.33 (dd, *J* = 12.8, 6.6 Hz, 1H), 3.22 (q, *J* = 7.4 Hz, 2H), 3.09 (dd, *J* = 16.9, 6.7 Hz, 1H), 3.01 (dd, *J* = 16.9, 5.1 Hz, 1H), 1.34 (t, *J* = 7.4 Hz, 3H).

**Example 140 3-(4-ethylsulfonyl)phenyl)-*N*-(4-(1,1,1,3,3,3-hexafluoro-2-hydroxypropan-2-yl)phenyl) oxetane-3-carboxamide**

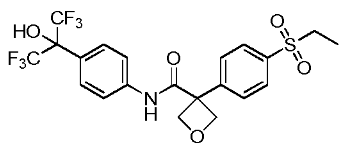

**Step 1: Synthesis of 3-(4-bromophenyl)-*N*-(4-(1,1,1,3,3,3-hexafluoro-2-hydroxypropan -2-yl)phenyl)oxetane-3-carboxamide**

2-(4-Aminophenyl)-1,1,1,3,3,3-hexafluoropropan-2-ol (100 mg, 0.39 mmol), 3-(4-bromophenyl)oxetane-3-carboxylic acid (109 mg, 0.42 mmol) and HATU (105 mg, 0.27 mmol) were dissolved in DMF (25 mL), then TEA (0.17 mL, 1.3 mmol) was added, and the mixture was reacted at room temperature for 12 h. The resulting mixture was washed with saturated ammonium chloride, extracted with EtOAc (10 mL × 2), dried over anhydrous $Na_2SO_4$, concentrated and separated by silica gel column chromatography (eluent: DCM/EtOAc (v/v) = 3/1) to give pale yellow liquid (0.028 g, 15%).
MS (ESI, pos.ion) m/z: 498 $[M+H]^+$.

**Step 2: Synthesis of 3-(4-bromophenyl)-*N*-(4-(2-((*tert*-butyldimethylsilyl)oxy) -1,1,1,3,3,3-hexafluoropropane-2-yl)phenyl)oxetane-3-carboxamide**

3-(4-Bromophenyl)-*N*-(4-(1,1,1,1,3,3,3-hexafluoro-2-hydroxypropan-2-yl)phenyl)oxetan e-3-carboxamide (28 mg, 0.056 mmol), *tert*-butylchlorodimethylsilane (33 mg, 0.22 mmol) and DMAP (10 mg, 0.082 mmol) were dissolved in DCM (25 mL), then TEA (0.24 mL, 1.80 mmol) was added, and the mixture was reacted at room temperature for 3 h. The resulting mixture was washed with saturated ammonium chloride, extracted with DCM (10 ml × 2), dried over anhydrous $Na_2SO_4$, concentrated and separated by silica gel column chromatography (eluent: DCM/EtOAc (v/v) = 8/1) to give a white solid (0.030 g, 87%).
MS (ESI, pos.ion) m/z: 613 $[M+H]^+$.

**Step 3: Synthesis of 3-(4-ethylsulfonyl)phenyl)-*N*-(4-(1,1,1,3,3,3-hexafluoro - 2-hydroxypropan-2-yl)phenyl)oxetane-3-carboxamide**

3-(4-Bromophenyl)-*N*-(4-(2-((*tert*-butyldimethylsilyl)oxy)-1,1,1,3,3,3-hexafluoroprop ane-2-yl)phenyl)oxetane-3-carboxamide (45 mg, 0.073 mmol), sodium ethylsulfinate (0.017 g, 0.15 mmol), (2*S*,4*R*)-*N*-(2,6-dimethylphenyl)-4-hydroxypyrrolidine-2-carboxamide (2.0 mg, 0.0076 mmol), potassium phosphate (19 mg, 0.088 mmol) and iodide (1.4 mg, 0.0074 mmol) were dissolved in DMSO (8 mL). The reaction mixture was degassed and refilled with nitrogen, and then reacted at 100°C for 24 h. The resulting mixture was washed with saturated NaCl, extracted with EtOAc (10 ml × 2), dried over anhydrous $Na_2SO_4$, concentrated and separated by silica gel column chromatography (eluent: DCM/EtOAc (v/v) = 3/1) to obtain a white solid (0.012 g, 32%).

MS (ESI, pos.ion) m/z: 512 $[M+H]^+$.
$^1H$ NMR (400 MHz, $CDCl_3$) δ (ppm): 7.93 - 7.82 (m, 3H), 7.68 (d, *J*= 8.6 Hz, 2H), 7.66 - 7.57 (m, 4H), 5.39 (d, *J* = 6.3 Hz, 2H), 5.03 (d, *J* = 6.3 Hz, 2H), 3.15 (q, *J* = 7.4 Hz, 2H), 1.38 - 1.23 (m, 3H).

**Example 141 (*R*)-*N*-(2-cyano-1-(4-(ethylsulfonyl)phenyl)ethyl)-4-(1,1,1,3,3,3-hexafluoro-2 -hydroxyprop-2-yl)benzamide**

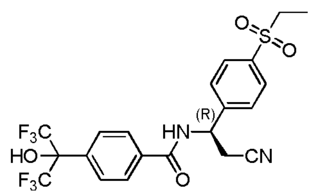

EDCI (100 mg, 0.52 mmol), HOBT (70 mg, 0.52 mmol), (*R*)-3-amino-3-(4-(ethylsulfonyl)phenyl)propionitrile hydrochloride (114 mg, 0.41 mmol), 4-(1,1,1,3,3,3-hexafluoro-2-hydroxypropan-2-yl)benzoic acid (100 mg , 0.35 mmol) and TEA (70 mg, 0.69 mmol) were successively added to DCM (6 mL), and the mixture was reacted at room temperature for 16 h. The reaction solution was concentrated under reduced pressure, and the concentrated solution was separated by silica gel column chromatography (eluent: DCM/EtOAc (v/v) = 2/1) to give a white solid (160 mg, 91%).

MS (ESI, pos.ion) m/z: 509.2 $[M+H]^+$.
$^1H$ NMR (400 MHz, DMSO-$d_6$) δ (ppm): 9.42 (d, *J* = 8.2 Hz, 1H), 8.97 (s, 1H), 8.00 (d, *J* = 8.4 Hz, 2H), 7.89 (d, *J* = 8.2 Hz, 2H), 7.83 (d, *J* = 8.2 Hz, 2H), 7.75 (d, *J* = 8.2 Hz, 2H), 5.54 (dd, *J* = 14.5, 8.5 Hz, 1H), 3.28 (t, *J* = 7.4 Hz, 2H), 3.23 - 3.10 (m, 2H), 1.09 (t, *J* = 7.3 Hz, 3H).

**Example 142 (*R*)-*N*-(2-cyano-1-(4-(ethylsulfonyl)phenyl)ethyl)-3,5-difluoro -4-(trimethylsilyl)benzamide**

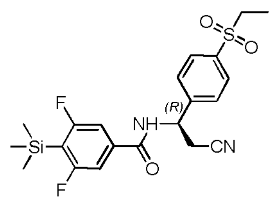

**Step 1: Synthesis of 3,5-difluorobenzoic acid**

3,5-Difluorobenzonitrile (5.00 g, 36.00 mmol) and *t*-BuOK (8.60 g, 77.00 mmol) were added to *i*-PrOH (35 mL) and the mixture was reacted at room temperature for 24 h. Dilute hydrochloric acid was added to adjust the pH of the solution to be acidic. The resulting mixture was extracted with EtOAc (25 mL × 3). The organic phases were combined, dried over anhydrous $Na_2SO_4$, filtered, concentrated and separated by silica gel column chromatography (eluent: DCM/EtOAc (v/v) = 1/1) to give a white solid (3.87 g, 68%).
MS (ESI, pos.ion) m/z: 159.3 $[M+H]^+$.

**Step 2: Synthesis of 3,5-difluoro-4-(trimethylsilyl)benzoic acid**

To a solution of 3,5-difluorobenzoic acid (2.50 g, 18.00 mmol) in THF (20 mL) was added LDA (9.9 mL, 20 mmol, 2 mol/L) dropwise at -78°C. The mixture was reacted for 1 h, then TMSCl (2.3 mL, 27 mmol) was added, and the mixture was slowly returned to room temperature and reacted for 5 h. The reaction was quenched by adding saturated NaCl solution (20 mL). The resulting mixture was extracted with EtOAc (10 mL × 2). The organic phases were combined, dried over anhydrous $Na_2SO_4$, filtered, concentrated and separated by silica gel column chromatography (eluent: PE/EtOAc (v/v) = 3/1) to give a white solid (1.06 g, 28%).
MS (ESI, pos.ion) m/z: 231.2 $[M+H]^+$.

**Step 3: Synthesis of (*R*)-*N*-(2-cyano-1-(4-(ethylsulfonyl)phenyl)ethyl)-3,5-difluoro -4-(trimethylsilyl)benzamide**

((1*R*)-2-cyano-1-(4-(ethylsulfonyl)phenyl)ethyl)ammonium chloride (53 mg, 0.20 mmol), 3,5-difluoro-4-(trimethylsilyl)benzoic acid (35 mg , 0.26 mmol), HOBT (35 mg, 0.26 mmol) and EDCI (45 mg, 0.23 mmol) were dissolved in DCM (5 mL), then DIPEA (78 mg, 0.60 mmol) was added, and the mixture was reacted at room temperature for 12 h. The reaction was quenched by adding saturated $NH_4Cl$ solution (15 mL). The resulting mixture was extracted with DCM (10 mL × 2). The organic phases were combined, dried over anhydrous $Na_2SO_4$, filtered, concentrated and separated by silica gel column chromatography (eluent: DCM/EtOAc (v/v) = 5/1) to give a white solid (55 mg, 61%).

MS (ESI, pos.ion) m/z: 451.6 $[M+H]^+$.
$^1H$ NMR (400 MHz, $CDCl_3$) δ (ppm): 7.77 (d, *J*= 8.2 Hz, 2H), 7.61 (d, *J*= 8.2 Hz, 2H), 7.52 (d, *J*= 7.8 Hz, 1H), 7.32 (d, *J*= 7.2 Hz, 2H), 5.60 (q, *J*= 6.6 Hz, 1H), 3.19 - 2.99 (m, 4H), 1.25 (t, *J*= 6.2 Hz, 3H), 0.38 (s, 9H).

**Example 143 (*S*)-*N*-(2-cyano-1-(4-(ethylsulfonyl)phenyl)ethyl)-4-(1,1,1,3,3,3-hexafluoro-2 - hydroxypropan-2-yl)benzamide**

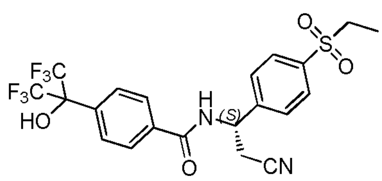

(*S*)-3-amino-3-(4-(ethylsulfonyl)phenyl)propionitrile hydrochloride (90 mg, 0.38 mmol), 4-(1,1,1,3,3,3-hexafluoro-2-hydroxypropan-2-yl)benzoic acid (160 mg, 0.56 mmol) and HATU (300 mg, 0.76 mmol) were dissolved in DCM (12 mL), then TEA (0.3 mL, 2 mmol) was added. The mixture was reacted at room temperature for 12 h. The reaction solution was diluted with DCM (10 mL), washed successively with HCl solution (10 mL, 0.1 mol/L), saturated $NaHCO_3$ solution (10 mL, 1 mol/L) and saturated NaCl solution (10 mL), dried over anhydrous $Na_2SO_4$, filtered and concentrated under reduced pressure. The concentrated solution was separated by silica gel column chromatography (eluent:

DCM/EtOAc (v/v) = 2/1) to give a white solid (99 mg, 52%).

MS (ESI, pos.ion) m/z: 509.1 [M+H]+.
$^1$H NMR (600 MHz, $CD_3OD$) δ (ppm): 7.95 (dd, *J* = 11.9, 8.5 Hz, 4H), 7.90 - 7.86 (m, 2H), 7.74 (d, *J*= 8.3 Hz, 2H), 5.67 - 5.56 (m, 1H), 3.20 (dt, *J* = 8.5, 6.4 Hz, 4H), 1.20 (t, *J* = 7.4 Hz, 3H).

**Example 144 (*S*)-*N*-(2-cyano-1-(4-(ethylsulfonyl)phenyl)ethyl)-4-(trifluoromethyl)benzamide**

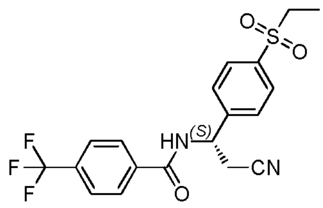

(*S*)-3-Amino-3-(4-(ethylsulfonyl)phenyl)propionitrile hydrochloride (80 mg, 0.34 mmol), 4-(trifluoromethyl)benzoic acid (60 mg , 0.32 mmol), HATU (140 mg, 0.36 mmol) and DIPEA (0.2 mL, 1 mmol) were dissolved in DCM (20 mL), and the reaction solution was stirred at room temperature. The reaction solution was diluted with DCM (50 mL), washed successively with HCl solution (15 mL, 0.1 mol/L), saturated $NaHCO_3$ solution (15 mL) and saturated NaCl solution (15 mL), dried over anhydrous $Na_2SO_4$, filtered and concentrated under reduced pressure. The concentrated solution was separated by silica gel column chromatography (eluent: DCM/EtOAc (v/v) = 1/1) to give a white solid(53 mg, 41%).

MS (ESI, pos.ion) m/z: 411.1 [M+H]+.
$^1$H NMR (400 MHz, $CDCl_3$) δ (ppm): 8.52 (d, *J* = 7.6 Hz, 1H), 7.91 (dd, *J* = 24.0, 8.1 Hz, 4H), 7.64 (dd, *J* = 18.1, 8.0 Hz, 4H), 5.55 (d, *J* = 5.9 Hz, 1H), 3.07 (q, *J* = 7.4 Hz, 2H), 2.84 (s, 2H), 1.24 (t, *J* = 7.3 Hz, 3H).

**Example 145 4-((2-(difluoromethoxy)ethyl)amino)-*N*-(4-(ethylsulfonyl)benzyl)benzamide**

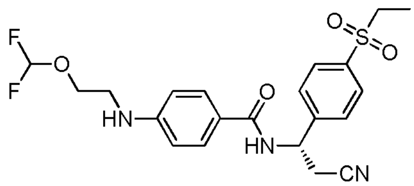

4-((2-(difluoromethoxy)ethyl)amino)benzoic acid (60 mg, 0.26 mmol), (*S*)-3-amino-3-(4-(ethylsulfonyl)phenylpropionitrile hydrochloride (130 mg, 0.55 mmol), HATU (150 mg, 0.38 mmol) and DIPEA(0.2 mL, 1 mmol) were dissolved in DCM (20 mL), and the mixture was reacted at room temperature for 16 h. The reaction solution was diluted with DCM (50 mL), washed successively with HCl solution (15 mL, 0.1 mol/L), saturated $NaHCO_3$ solution (15 mL) and saturated NaCl solution (15 mL), dried over anhydrous $Na_2SO_4$, filtered and concentrated under reduced pressure. The concentrated solution was separated by silica gel column chromatography (eluent: DCM/EtOAc (v/v) = 1/1) to give a white solid (30 mg, 26%).

MS (ESI, pos.ion) m/z: 413.2 [M+H]+.
$^1$H NMR (400 MHz, $CD_3OD$) δ (ppm): 7.87 (d, *J* = 8.3 Hz, 2H), 7.67 (dd, *J* = 14.3, 8.5 Hz, 4H), 6.61 (d, *J* = 8.7 Hz, 2H), 6.25 (t, *J*= 74.6 Hz, 1H), 5.53 (t, *J* = 7.1 Hz, 1H), 3.99 (t, *J* = 5.5 Hz, 2H), 3.42 (t, *J* = 5.5 Hz, 2H), 3.11 (q, *J*= 7.5 Hz, 2H), 3.09 - 2.97 (m, 2H), 1.24 (t, *J* = 7.5 Hz, 3H).

**Example 146 *N*-((*R*)-2-ethylsulfonyl-1-(4-(ethylsulfonyl)phenyl)ethyl)-4-((2*S*,4*S*) -2-((difluoromethoxy) methyl)-4-(4-(trifluoromethyl)phenoxy)pyrrolidin-1-yl)benzamide**

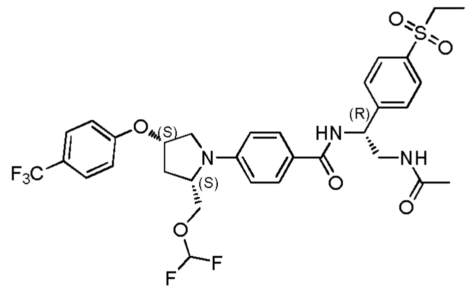

**Step 1: Synthesis of *tert-butyl* (*R*)-(1-(4-(ethylsulfonyl)phenyl)-2-hydroxyethyl)carbamate**

To a solution of (R)-2-amino-2-(4-(ethylsulfonyl)phenyl)ethanol (1.50 g, 6.54 mmol) in THF (15 mL) was added saturated $NaHCO_3$ solution (15 mL), and then $(BOC)_2O$ (2.40 mL, 13.33 mmol) was added slowly. The mixture was reacted at room temperature for 17 h. The reaction solution was diluted with saturated $NaHCO_3$ solution (10 mL) and EtOAc (30 mL). The resulting mixture was left standing for layers and separated, and the aqueous phase was extracted with EtOAc (20 mL × 2). The combined organic phases were washed with saturated NaCl solution (30 mL), dried over anhydrous $Na_2SO_4$, concentrated under reduced pressure, and the concentrated solution was separated by silica gel column chromatography (eluent: DCM/EtOAc (v/v) = 3/2) to give a pale yellow solid (1.30 g, 60%).
MS (ESI, pos.ion) m/z: 274.2 $[M-56+H]^+$.

**Step 2: Synthesis of (*R*)-2-((*tert*-butoxycarbonyl)amino)-2-(4-(ethylsulfonyl)phenyl)ethyl methanesulfonate**

To a solution of *tert*-butyl (*R*)-(1-(4-(ethylsulfonyl)phenyl)-2-hydroxyethyl)carbamate (1.30 g, 3.95 mmol) in DCM (16 mL) were added MsCl (0.43 mL, 5.55 mmol), TEA (1.10 mL, 7.91 mmol) and DMAP (50 mg, 0.41 mmol) in turn. The mixture was reacted at room temperature for 1 h. The reaction solution was concentrated under reduced pressure, and the concentrated solution was separated by silica gel column chromatography (eluent: DCM/EtOAc (v/v) = 4/1) to give a white solid (1.40 g, 87%).
MS (ESI, pos.ion) m/z: 308.0 $[M-100+H]^+$.

**Step 3: Synthesis of *tert*-butyl (*R*)-(2-azido-1-(4-(ethylsulfonyl)phenyl)ethyl)carbamate**

To a solution of (*R*)-2-((*tert*-butoxycarbonyl)amino)-2-(4-(ethylsulfonyl)phenyl)ethyl methanesulfonate (1.10 g, 2.70 mmol) in DMF (14 mL) was added $NaN_3$ (877 mg, 13.49 mmol). The mixture was reacted at 80 °C for 6 h. The reaction solution was diluted with EtOAc (50 mL), then washed with $H_2O$ (20 mL × 2) and saturated NaCl solution (20mL), dried over anhydrous $Na_2SO_4$, and concentrated under reduced pressure to give a white solid (900 mg, 94%).
MS (ESI, pos.ion) m/z: 270.1 $[M-56+H]^+$.

**Step 4: Synthesis of *tert*-butyl (*R*)-(2-amino-1-(4-(ethylsulfonyl)phenyl)ethyl)carbamate**

*tert*-Butyl (*R*)-(2-azido-1-(4-(ethylsulfonyl)phenyl)ethyl)carbamate (900 mg, 2.54 mmol) and $PPh_3$ (1.33 g, 5.07 mmol) were added to THF/$H_2O$ (6 mL/3 mL), and the mixture was reacted at 50 °C for 15 h. The reaction solution was diluted with EtOAc (40 mL), washed with $H_2O$ (15 mL) and saturated NaCl solution (15 mL), dried over anhydrous $Na_2SO_4$, and concentrated under reduced pressure. The concentrated solution was separated by silica gel column chromatography (eluent: DCM/MeOH (v/v) = 10/1) to give a white solid (580 mg, 70%).
MS (ESI, pos.ion) m/z: 329.1 $[M+H]^+$.

**Step 5: Synthesis of *tert*-butyl (*R*)-(2-acetylamino-1-(4-(ethylsulfonyl)phenyl)ethyl)carbamate**

HATU (350 mg, 0.92 mmol), *tert*-butyl (*R*)-(2-amino-1-(4-(ethylsulfonyl)phenyl) ethyl)carbamate (200 mg, 0.61 mmol), acetic acid (45 mg, 0.75 mmol) and TEA(184 mg, 1.82 mmol) were successively added to DCM (4 mL), and the mixture was reacted at room temperature for 15 h. The reaction solution was concentrated under reduced pressure. The residue was diluted with DCM (40 mL), washed successively with HCl solution (20 mL, 1 mol/L), saturated $NaHCO_3$ solution (20 mL, 1 mol/L) and saturated NaCl solution (20 mL), dried over anhydrous $Na_2SO_4$, and concentrated under reduced pressure. The concentrated solution was separated by silica gel column chromatography (eluent: DCM/EtOAc (v/v) = 1/1) to give a white solid (196 mg, 87%).
MS (ESI, pos.ion) m/z: 315.1 $[M-56+H]^+$.

**Step 6: Synthesis of (*R*)-*N*-(2-amino-2-(4-(ethylsulfonyl)phenyl)ethyl)acetamide hydrochloride**

To a solution of *tert*-butyl (*R*)-(2-acetylamino-1-(4-(ethyl sulfonyl)phenyl) ethyl)carbamate (196 mg, 0.53 mmol) in DCM (4 mL) was slowly added a solution of HCl in 1,4-dioxane (0.50 mL, 4 mol/L). The mixture was reacted at room temperature for 22 h. The reaction solution was concentrated under reduced pressure to give a white solid (143 mg, 88%).
MS (ESI, pos.ion) m/z: 271.1 $[M+H]^+$.

**Step 7: Synthesis of *N*-((*R*)-2-acetylamino-1-(4-(ethylsulfonyl)phenyl)ethyl)-4-((2*S*,4*S*)-2-((difluoromethoxy)methyl)-4-(4-(trifluoromethyl)phenoxy)pyrrolidin-1-yl)benzamide**

HATU (224 mg, 0.59 mmol), (*R*)-*N*-(2-amino-2-(4-(ethylsulfonyl)phenyl) ethyl)acetamide hydrochloride (140 mg, 0.46 mmol), 4-((2*S*,4*S*)-2-((difluoromethoxy)methyl)-4-(4-(trifluoromethyl) phenoxy)pyrrolidin-1-yl)benzoic acid (170 mg , 0.39 mmol) and TEA (120 mg, 1.19 mmol) were successively added to DCM (6 mL), and the mixture was stirred at room temperature for 22 h. The reaction solution was concentrated under reduced pressure. The residue was diluted with DCM (30 mL), washed successively with HCl solution (15 mL, 0.5 mol/L), saturated $NaHCO_3$ solution (15 mL) and saturated NaCl solution (15 mL), dried over anhydrous $Na_2SO_4$, and concentrated under reduced pressure. The concentrated solution was separated by silica gel column chromatography (eluent: DCM/MeOH (v/v) = 20/1) to give a white solid(200 mg, 74%).

MS (ESI, pos.ion) m/z: 684.2 $[M+H]^+$.
$^1H$ NMR (400 MHz, $CDCl_3$) δ (ppm): 8.45 (d, *J*= 5.3 Hz, 1H), 7.84 (d, *J*= 8.3 Hz, 2H), 7.81 (d, *J*= 8.8 Hz, 2H), 7.58 (d, *J* = 8.6 Hz, 2H), 7.50 (d, *J* = 8.3 Hz, 2H), 6.97 (d, *J* = 8.6 Hz, 2H), 6.67 (d, *J* = 8.8 Hz, 2H), 6.31 (t, *J* = 6.2 Hz, 1H), 6.22 (t, *J* = 74.3 Hz, 1H), 5.17 (t, *J* = 4.8 Hz, 1H), 5.13 (dd, *J* = 10.8, 5.4 Hz, 1H), 4.23 - 4.15 (m, 2H), 3.96 (t, *J* = 9.6 Hz, 1H), 3.76 (d, *J* = 11.4 Hz, 1H), 3.70 (dd, *J* = 11.4, 4.8 Hz, 1H), 3.64 (t, *J* = 6.8 Hz, 2H), 3.08 (q, *J* = 7.4 Hz, 2H), 2.51 (d, *J* = 14.4 Hz, 1H), 2.40 - 2.35 (m, 1H), 1.63 (s, 3H), 1.28 (t, *J* = 7.4 Hz, 3H).

**Example 147 methyl ((*R*)-2-(4-((2*S*,4*S*)-2-((difluoromethoxy)methyl)-4-(4-(trifluoromethyl) phenoxy)pyrrolidin-1-yl)benzoylamino)-2-(4-(ethylsulfonyl)phenyl)ethyl)carbamate**

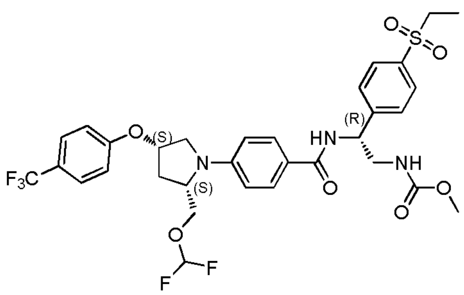

**Step 1: Synthesis of (*R*)-1-*tert*-butyl 2-methyl (1-(4-(ethylsulfonyl)phenyl)ethane-1,2-diyl)dicarbamate**

HATU (350 mg, 0.92 mmol), *tert*-butyl (*R*)-(2-amino-1-(4-(ethylsulfonyl)phenyl)ethyl)carbamate (200 mg, 0.61 mmol), methyl chloroformate (86 mg, 0.91 mmol) and TEA (184 mg, 1.82 mmol) were successively added to DCM (4 mL), and the mixture was reacted at room temperature for 5 h. The reaction solution was concentrated under reduced

pressure, and the concentrated solution was separated by silica gel column chromatography (eluent: DCM/EtOAc (v/v) = 1/1) to give a white solid (150 mg, 64%).
MS (ESI, pos.ion) m/z: 287.1 [M-100+H]$^+$.

**Step 2: Synthesis of methyl (*R*)-(2-amino-2-(4-(ethylsulfonyl)phenyl)ethyl)carbamate hydrochloride**

To a solution of (*R*)-1-*tert*-butyl 2-methyl (1-(4-(ethylsulfonyl)phenyl)ethane -1,2-diyl)dicarbamate (150 mg, 0.39 mmol) in DCM (3 mL) was slowly added a solution of HCl in 1,4-dioxane (0.40 mL, 4 mol/L). The mixture was reacted at room temperature for 22 h. The reaction solution was concentrated under reduced pressure to give a white solid (125 mg, 100%).
MS (ESI, pos.ion) m/z: 287.4 [M+H]$^+$.

**Step 3: Synthesis of methyl ((*R*)-2-(4-((2*S*,4*S*)-2-((difluoromethoxy)methyl)-4-(4-(trifluoromethyl)phenoxy)pyrrolidin-1-yl)benzoylamino)-2-(4-(ethylsulfonyl)phenyl)ethyl)car bamate**

HATU (185 mg, 0.49 mmol), methyl (*R*)-(2-amino-2-(4-(ethylsulfonyl) phenyl)ethyl)carbamate hydrochloride (140 mg, 0.46 mmol), 4-((2*S*,4*S*)-2-((difluoromethoxy)methyl)-4-(4-(trifluoromethyl) phenoxy)pyrrolidin-1-yl)benzoic acid (140 mg, 0.32 mmol) and TEA (100 mg, 0.99 mmol) were successively added to DCM (6 mL), and the mixture was reacted at room temperature for 21 h. The reaction solution was concentrated under reduced pressure. The residue was diluted with DCM (30 mL), washed successively with HCl solution (15 mL, 0.5 mol/L), saturated $NaHCO_3$ solution (15 mL) and saturated NaCl solution (15 mL), dried over anhydrous $Na_2SO_4$, and concentrated under reduced pressure. The concentrated solution was separated by silica gel column chromatography (eluent: DCM/EtOAc (v/v) = 1/1) to give a white solid (150 mg, 66%).

MS (ESI, pos.ion) m/z: 700.9 [M+H]$^+$.
$^1$H NMR (400 MHz, $CDCl_3$) δ (ppm): 8.07 (d, *J*= 5.4 Hz, 1H), 7.84 (d, *J*= 8.3 Hz, 2H), 7.81 (d, *J* = 8.6 Hz, 2H), 7.58 (d, *J* = 8.6 Hz, 2H), 7.51 (d, *J* = 8.2 Hz, 2H), 6.97 (d, *J* = 8.6 Hz, 2H), 6.67 (d, *J* = 8.7 Hz, 2H), 6.22 (t, *J* = 74.3 Hz, 1H), 5.26 (t, *J* = 6.2 Hz, 1H), 5.20 - 5.15 (m, 2H), 4.23 - 4.20 (m, 1H), 4.17 (dd, *J* = 9.9, 4.1 Hz, 1H), 3.96 (t, *J* = 9.6 Hz, 1H), 3.76 (d, *J* = 11.3 Hz, 1H), 3.72 - 3.68 (m, 4H), 3.59 (t, *J* = 7.0 Hz, 2H), 3.08 (q, *J* = 7.4 Hz, 2H), 2.52 (d, *J* = 14.4 Hz, 1H), 2.41 - 2.35 (m, 1H), 1.27 (t, *J* = 7.4 Hz, 3H).

**Example 148 4-((2*S*,4*S*)-2-((difluoromethoxy)methyl)-4-(4-trifluoromethyl)phenoxy) pyrrolidin-1-yl)-*N*-((*R*)-1-(4-(ethylsulfonyl)phenyl)-2-formamidoethyl)benzamide**

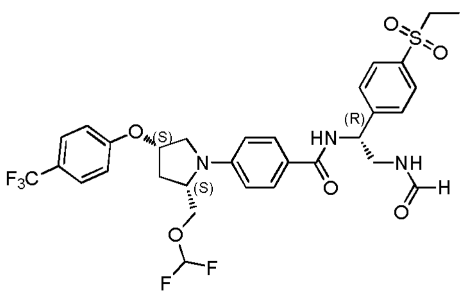

**Step** 1: **Synthesis of *tert-butyl* (*R*)-(1-(4-(ethylsulfonyl)phenyl)-2-formamidoethyl)carbamate**

*tert*-Butyl (*R*)-(2-amino-1-(4-(ethylsulfonyl)phenyl)ethyl)carbamate (200 mg, 0.61 mmol) was added to methyl formate (4 mL), and the mixture was reacted at 40 °C for 21 h. The reaction solution was concentrated under reduced pressure to give colorless liquid (200 mg, 92%). MS (ESI, pos.ion) m/z: 301.4 [M-56+H]$^+$.

**Step 2: Synthesis of (*R*)-*N*-(2-amino-2-(4-(ethylsulfonyl)phenyl)ethyl)formamide hydrochloride**

To a solution of *tert*-butyl (*R*)-(1-(4-(ethylsulfonyl)phenyl)-2-carboxamidoethyl)carbamate (200 mg, 0.56 mmol) in DCM (4 mL) was slowly added a solution of HCl in 1,4-dioxane (0.60 mL, 4 mol/L). The mixture was reacted at room temperature for 22 h. The reaction solution was concentrated under reduced pressure to give a white solid (160 mg, 98%).

MS (ESI, pos.ion) m/z: 257.4 $[M+H]^+$.

**Step 3: Synthesis of 4-((2*S*,4*S*)-2-((difluoromethoxy)methyl)-4-(4-(trifluoromethyl)phenoxy) pyrrolidin-1-yl)-*N*-((*R*)-1-(4-(ethylsulfonyl)phenyl)-2-carboxamidoethyl)benzamide**

HATU (240 mg, 0.63 mmol), (*R*)-*N*-(2-amino-2-(4-(ethylsulfonyl)phenyl) ethyl)formamide hydrochloride (140 mg, 0.48 mmol), 4-((2*S*,4*S*)-2-((difluoromethoxy)methyl)-4-(4-(trifluoromethyl) phenoxy)pyrrolidin-1-yl)benzoic acid (180 mg, 0.42 mmol) and TEA (130 mg, 1.28 mmol) were successively added to DCM (6 mL), and the mixture was reacted at room temperature for 22 h. The reaction solution was concentrated under reduced pressure. The residue was diluted with DCM (30 mL), washed successively with HCl solution (15 mL, 0.5 mol/L), saturated $NaHCO_3$ solution (15 mL) and saturated NaCl solution (15 mL), dried over anhydrous $Na_2SO_4$, and concentrated under reduced pressure. The concentrated solution was separated by silica gel column chromatography (eluent: DCM/MeOH (v/v) = 20/1) to give a white solid (200 mg, 72%).

MS (ESI, pos.ion) m/z: 670.9 $[M+H]^+$.
$^1$H NMR (400 MHz, $CDCl_3$) $\delta$ (ppm): 8.20 (d, *J*= 5.7 Hz, 1H), 8.15 (s, 1H), 7.83 (d, *J*= 8.2 Hz, 2H), 7.79 (d, *J* = 8.7 Hz, 2H), 7.58 (d, *J* = 8.5 Hz, 2H), 7.50 (d, *J* = 8.2 Hz, 2H), 6.97 (d, *J* = 8.5 Hz, 2H), 6.66 (d, *J* = 8.7 Hz, 2H), 6.60 (d, *J*= 6.5 Hz, 1H), 6.22 (t, *J*= 74.3 Hz, 1H), 5.17 (s, 2H), 4.24 - 4.15 (m, 2H), 3.96 (t, *J*= 9.2 Hz, 1H), 3.78 - 3.62 (m, 4H), 3.08 (q, *J*= 7.4 Hz, 2H), 2.51 (d, *J* = 14.3 Hz, 1H), 2.42 - 2.33 (m, 1H), 1.27 (t, *J* = 7.4 Hz, 3H).

**Example 149 (*R*)-2-(4-((2S,4S)-2-((difluoromethoxy)methyl)-4-(4-(trifluoromethyl) phenoxy)pyrrolidin -1-yl)benzoylamino)-2-(4-(ethylsulfonyl)phenyl)ethyl carbamate**

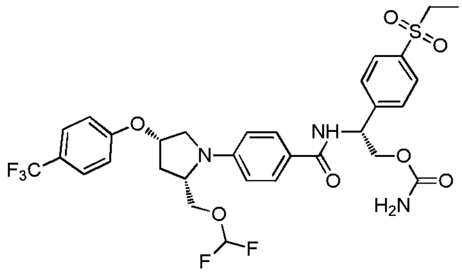

**Step** 1: **Synthesis of benzyl ((*R*)-2-(carbamoyloxy)-1-(4-(ethylsulfonyl)phenyl)ethyl)carbamate**

Benzyl (*R*)-(1-(4-(ethylsulfonyl)phenyl)-2-hydroxyethyl)carbamate (300 mg, 0.83 mmol) and sodium isocyanate (80 mg, 1.23 mmol) were dissolved in DCM (10 mL). The mixtrue was cooled to 0°C, then methanesulfonic acid (0.16 mL, 2.5 mmol) was added. The mixture was slowly returned to room temperature and reacted for 12 h. The resulting mixture was concentrated under reduced pressure. The concentrated solution was added with saturated $NaHCO_3$ solution (20 mL), extracted with DCM (30 mL $\times$ 2), dried over anhydrous $Na_2SO_4$, and concentrated under reduced pressure. The concentrated solution was separated by silica gel column chromatography (eluent: DCM/MeOH (v/v) = 20/1) to give a white solid (247 mg, 74%).
MS (ESI, pos.ion) m/z:407.2 $[M+H]^+$.

**Step 2: Synthesis of (*R*)-2-amino-2-(4-(ethylsulfonyl)phenyl)ethyl carbamate**

To a solution of (*R*)-benzylethyl 2-(carbamoyloxy)-1-(4-(ethylsulfonyl) phenyl)carbamate (245 mg, 0.60 mmol) in MeOH (10 mL) was added Pd/C (120 mg) under hydrogen atmosphere. The mixture was reacted at room temperature for 12 h, filtered to remove the remaining Pd/C, and directly concentrated to obtain a colorless liquid (0.15 g, 91%).
MS (ESI, pos.ion) m/z:273.1 $[M+H]^+$.

**Step 3: Synthesis of (*R*)-2-(4-((2*S*,4*S*)-2-((difluoromethoxy)methyl)-4-(4-(trifluoromethyl) phenoxy)pyrrolidin-1-yl)benzoylamino)-2-(4-(ethylsulfonyl)phenyl)ethyl carbamate**

4-((2*S*,4*S*)-2-((Difluoromethoxy)methyl)-4-(4-(trifluoromethyl)phenoxy)pyrrolidin-1-y l)benzoic acid (93 mg, 0.22 mmol), (*R*)-2-amino-2-(4-ethylsulfonylphenyl)ethyl carbamate (65 mg, 0.24 mmol) and HATU (99 mg, 0.26 mmol)

were dissolved in DCM (25 mL), then TEA (0.064 mL, 0.49 mmol) was added. The mixture was reacted at room temperature for 12 h. The reaction solution was washed successively with saturated $NH_4Cl$ solution (20 mL × 2) and saturated $NaHCO_3$ solution (20 mL), dried over anhydrous $Na_2SO_4$, and concentrated under reduced pressure. The concentrated solution was separated by silica gel column chromatography (eluent: DCM/MeOH (v/v) = 20/1) to give a white solid (82 mg, 55%).

MS (ESI, pos.ion) m/z:686.3 $[M+H]^+$.
$^1$H NMR (400 MHz, $CDCl_3$) δ (ppm):7.81 (dd, *J* = 18.1, 8.5 Hz, 5H), 7.58 (dd, *J* = 11.0, 8.7 Hz, 4H), 6.98 (d, *J*= 8.6 Hz, 2H), 6.64 (d, *J*= 8.8 Hz, 2H), 6.24 (t, *J*= 74.4 Hz, 1H), 5.40 (dd, *J*= 10.0, 7.3 Hz, 1H), 5.18 (t, *J* = 4.3 Hz, 1H), 5.09 (s, 1H), 4.52 (dd, *J* = 11.9, 7.9 Hz, 1H), 4.38 - 4.26 (m, 1H), 4.26 - 4.12 (m, 2H), 3.96 (t, *J* = 9.1 Hz, 1H), 3.71 (dt, *J* = 11.4, 7.9 Hz, 2H), 3.08 (q, *J* = 7.4 Hz, 2H), 2.51 (d, *J*= 14.3 Hz, 1H), 2.41 (dd, *J* = 8.2, 5.4 Hz, 1H), 1.26 (t, *J*= 7.4 Hz, 3H).

**Example 150 (*R*)-2-(4-((2*S*,4*S*)-2-((difluoromethoxy)methyl)-4-(4-(trifluoromethyl) phenoxy)pyrrolidin -1-yl)benzoylamino)-2-(4-(ethylsulfonyl)phenyl)ethyl methylcarbamate**

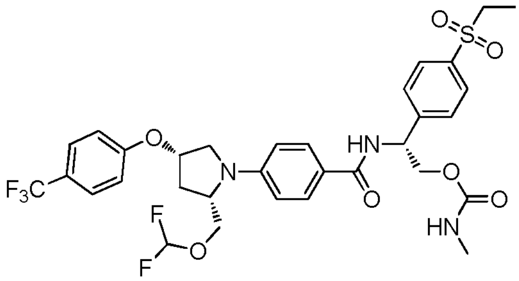

**Step 1: Synthesis of benzyl (*R*)-(1-(4-(ethylsulfonyl)phenyl)-2-(methylcarbamoyloxy)ethyl)carbamate**

To a mixture of benzyl (*R*)-(1-(4-(ethylsulfonyl)phenyl)-2-hydroxyethyl)carbamate (400 mg, 1.12 mmol) and bis(1*H*-imidazol-1-yl)methanone (357 mg, 2.20 mmol) was added THF (10 ml) under $N_2$ protection, and the mixture was reacted at room temperature for 4 h. Methylamine hydrochloride (149 mg, 2.21 mmol) and TEA (0.3 mL, 2.21 mmol) were added, and the mixture was reacted at room temperature for 10 h. The resulting mixture was washed with saturated $NH_4Cl$ solution, and extracted with EtOAc (10 mL × 3). The organic phases were combined, dried over anhydrous $Na_2SO_4$, filtered, concentrated and separated by silica gel column chromatography (eluent: DCM/MeOH (v/v) = 50/1) to give a white solid (86.2 mg, 19%).
MS (ESI, pos.ion) m/z: 421 $[M+H]^+$.

**Step 2: Synthesis of (*R*)-2-amino-2-(4-(ethylsulfonyl)phenyl)ethyl methylcarbamate**

To benzyl (*R*)-(1-(4-(ethylsulfonyl)phenyl)-2-(methylcarbamoyloxy)ethyl)carbamate (86 mg, 0.21 mmol) were added Pd/C (100 mg) and methanol (10 ml) under $H_2$ protection, and the mixture was reacted at room temperature for 16 h. The mixture was filtered with suction through a celite pad and concentrated to give a milky yellow solid (58.0 mg, 99%).
MS (ESI, pos.ion) m/z: 287 $[M+H]^+$.

**Step 3: Synthesis of (*R*)-2-(4-((2*S*,4*S*)-2-((difluoromethoxy)methyl)-4-(4-(trifluoromethyl) phenoxy)pyrrolidin-1-yl)benzoylamino)-2-(4-(ethylsulfonyl)phenyl)ethyl methylcarbamate**

4-((2*S*,4*S*)-2-((Difluoromethoxy)methyl)-4-(4-(trifluoromethyl)phenoxy)pyrrolidin-1-y l)benzoic acid (78 mg, 0.18 mmol), (*R*)-2-amino-2-(4-(ethylsulfonyl)phenyl)ethyl methylcarbamate (60 mg, 0.21 mmol) and HATU (80 mg, 0.21 mmol) were added to DCM (10 mL) and TEA (36 mg, 0.36 mmol) under $N_2$ protection. The mixture was reacted at room temperature for 7 h. The resulting mixture was washed with saturated $NH_4Cl$ solution, and extracted with DCM (10 mL × 3). The organic phases were combined, dried over anhydrous $Na_2SO_4$, filtered, concentrated and separated by silica gel column chromatography (eluent: DCM/EtOAc (v/v) = 5/1) to give a white solid (15 mg, 12%).

MS (ESI, pos.ion) m/z: 700 $[M+H]^+$.
$^1$H NMR (600 MHz, $CDCl_3$) δ (ppm): 7.86 (d, *J*= 8.4 Hz, 3H), 7.81 (d, *J*= 8.7 Hz, 2H), 7.60 (d, *J*= 8.6 Hz, 2H), 7.57

(d, *J* = 8.2 Hz, 2H), 6.99 (d, *J* = 8.6 Hz, 2H), 6.68 (d, *J* = 8.7 Hz, 2H), 6.24 (t, *J* = 74.3 Hz, 1H), 5.37 (dd, *J*= 11.0, 6.0 Hz, 1H), 5.19 (t, *J*= 4.7 Hz, 1H), 4.98 (d, *J*= 4.6 Hz, 1H), 4.54 (dd, *J* = 12.1, 8.1 Hz, 1H), 4.32 (dd, *J* = 12.2, 3.2 Hz, 1H), 4.21 (ddd, *J* = 14.0, 9.3, 4.3 Hz, 2H), 3.98 (t, *J* = 9.6 Hz, 1H), 3.78 (d, *J* = 11.3 Hz, 1H), 3.72 (dd, *J* = 11.3, 4.8 Hz, 1H), 3.10 (q, *J* = 7.4 Hz, 2H), 2.82 (s, 3H), 2.53 (d, *J* = 14.4 Hz, 1H), 2.46 - 2.37 (m, 1H), 1.30 - 1.25 (m, 3H).

**Example 151 *N*-((*S*)-2-cyano-1-(4-(ethylsulfonyl)phenyl)ethyl)-4-((2S,4s)-2-((3,3,3-d3-methoxy) methyl)-4-(4-(trifluoromethyl)phenoxy)pyrrolidin-1-yl)benzamide**

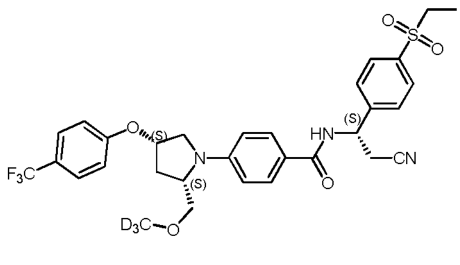

**Step 1: Synthesis of ethyl 4-((2*S*,4*S*)-2-(hydroxymethyl)-4-(4-(trifluoromethyl)phenoxy) pyrrolidin-1-yl)benzoate**

4-((2*S*,4*S*)-2-((Difluoromethoxy)methyl)-4-(4-(trifluoromethyl)phenoxy)pyrrolidin-1-y l)benzoic acid (2.02 g, 4.68 mmol) was dissolved in a solution of HCl in ethanol (10 mL, 4.0 M). The reaction mixture was reacted at 65°C for 8 h. After the reaction was completed, the reaction solution was cooled to room temperature and concentrated under reduced pressure, then $NaHCO_3$ solution (50 mL) was added. The resulting mixture was extracted with DCM (30 mL). The combined organic phases were dried over anhydrous $Na_2SO_4$, filtered and concentrated under reduced pressure. The crude product was separated by silica gel column chromatography (eluent: PE/EtOAc (v/v) = 1/1) to give a white solid (980 mg, 51%).
MS (ESI, pos.ion) m/z = 410.2 $[M+H]^+$.

**Step 2: Synthesis of ethyl 4-((2*S*,4*S*)-2-(((methylsulfonyl)oxy)methyl)-4-(4-trifluoromethyl)phenoxy)pyrrolidin-1-yl)benzoate**

Ethyl 4-((2*S*,4*S*)-2-(hydroxymethyl)-4-(4-(trifluoromethyl)phenoxy)pyrrolidin-1-yl) benzoate (500 mg, 1.22 mmol ) and TEA (0.40 mL, 2.7 mmol) were dissolved in DCM (10 mL, 156 mmol), then MsCl (0.2 mL, 3 mmol) was added dropwise. The reaction solution was stirred at room temperature for 10 h. After the reaction was completed, the mixture was concentrated under reduced pressure, and the crude product was separated by silica gel column chromatography (eluent: DCM/EtOAc (v/v) = 5/1) to give pale yellow liquid (500 mg, 84%).
MS (ESI, pos.ion) m/z = 488.1 $[M+H]^+$.

**Step 3: Synthesis of ethyl 4-((2*S*,4*S*)-2-(3,3,3-*d*3-methoxymethyl)-4-(4-(trifluoromethyl) phenoxy)pyrrolidin-1-yl)benzoate**

Ethyl 4-((2*S*,4*S*)-2-(((methylsulfonyl)oxy)methyl)-4-(4-(trifluoromethyl)phenoxy) pyrrolidin-1-yl)benzoate (800 mg, 1.64 mmol) was dissolved in $CD_3OD$ (0.2 mL, 5 mmol). Then NaH (70 mg, 1.76 mmol) was added, and the reaction solution was stirred at room temperature. After the reaction was completed, the stirring was stopped, and the reaction solution was concentrated under reduced pressure. The residue was diluted with DCM (20 mL), and quenched by adding HCl solution (15 mL, 0.1 mol/L). The organic phase was washed with saturated NaCl solution (15 mL), dried over anhydrous $Na_2SO_4$, filtered and concentrated under reduced pressure. The crude product was separated and purified by silica gel column chromatography (eluent: PET/EtOAc (v/v) = 1/1) to give a white solid (500 mg, 75%).
MS (ESI, pos.ion) m/z: 427.2 $[M+H]^+$.

**Step 4: Synthesis of 4-((2*S*,4*S*)-2-((3,3,3-*d*3-methoxy)methyl)-4-(4-(trifluoromethyl) phenoxy)pyrrolidin-1-yl)benzoic acid**

To a solution of methyl 4-((2*S*,4*S*)-2-((3,3,3-*d*3-methoxy)methyl)-4-(4-(trifluoromethyl) phenoxy)pyrrolidin-1-yl)benzoate (500 mg, 1.18 mmol) in MeOH (10 mL) and $H_2O$ (2 mL) was added LiOH (150 mg, 3.5 mmol). The mixture was reacted at room temperature. The reaction solution was added with HCl solution (2.0 mol/L) to adjust the pH of the

solution to about 5. The resulting mixture was extracted with EtOAc (25 mL × 2). The organic phases were combined, washed with saturated NaCl (20 mL) solution, dried over anhydrous $Na_2SO_4$, concentrated under reduced pressure, and the crude product was separated by silica gel column chromatography (eluent: DCM/MeOH (v/v) = 10/1) to give a brown solid (400 mg, 82%).
MS (ESI, pos.ion) m/z: 399.1 $[M+H]^+$.

**Step 5: Synthesis of *N*-((*S*)-2-cyano-1-(4-(ethylsulfonyl)phenyl)ethyl)-4-((2*S*,4*S*)-2-((3,3,3-*d*3-methoxy)methyl)-4-(4-(trifluoromethyl)phenoxy)pyrrolidin-1-yl)benzamide**

4-((2*S*,4*S*)-2-((3,3,3-*d*3-methoxy)methyl)-4-(4-(trifluoromethyl)phenoxy)pyrrolidin-1 - yl)benzoic acid (105 mg, 0.26 mmol), (*S*)-3-amino-3-(4-(ethylsulfonyl)phenyl)propionitrile (76 mg, 0.32 mmol), HATU (128 mg, 0.33 mmol) and DIPEA (0.1 mL, 0.56 mmol) were dissolved in DCM (15 mL). The mixture was stirred at room temperature for 15 h. The reaction solution was diluted with DCM (15 mL). The resulting mixture was washed successively with HCl solution (20 mL, 0.1 mol/L), saturated $NaHCO_3$ solution (20 mL) and saturated NaCl solution (15 mL), dried over anhydrous $Na_2SO_4$, filtered and concentrated under reduced pressure. The crude product was separated by silica gel column chromatography (eluent: DCM/EtOAc (v/v) = 1/1) to give a white solid (45 mg, 27%).

MS (ESI, pos.ion) m/z: 619.2 $[M+H]^+$.
$^1H$ NMR (600 MHz, $CDCl_3$) $\delta$ (ppm): 7.80 (d, *J*= 8.2 Hz, 2H), 7.75 (d, *J*= 8.8 Hz, 2H), 7.58 (d, *J* = 8.8 Hz, 2H), 7.49 (d, *J* = 8.2 Hz, 2H), 6.97 (d, *J* = 8.6 Hz, 2H), 6.66 (d, *J* = 8.7 Hz, 2H), 5.14 (t, *J* = 4.9 Hz, 1H), 4.71 (d, *J* = 14.6 Hz, 2H), 4.13 - 4.10 (m, 1H), 3.75 - 3.62 (m, 3H), 3.46 (t, *J* = 9.1 Hz, 1H), 3.10 (q, *J*= 7.4 Hz, 2H), 3.06 (d, *J*= 4.6 Hz, 1H), 2.47 (t, *J* = 11.0 Hz, 1H), 2.36 (m, 1H), 1.25 (t, *J* = 7.4 Hz, 3H).

**Example 152 4-((2*S*,4*S*)-4-(4-(1*H*-pyrazol-1-yl)phenoxy)-2-((difluoromethoxy)methyl) pyrrolidin -1-yl)-*N*-((*S*)-2-cyano-1-(4-(ethylsulfonyl)phenyl)ethyl)benzamide**

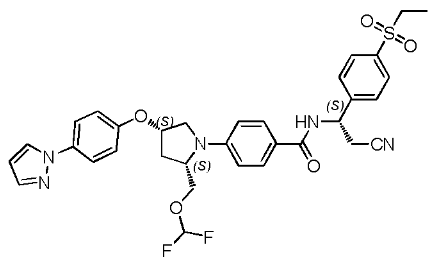

**Step 1: Synthesis of 4-(1*H*-pyrazol-1-yl)phenol**

To a solution of 1-(4-methoxyphenyl)-1*H*-pyrazole (700 mg, 4.02 mmol) in DCM (10 mL) was added $BBr_3$ (1.20 mL, 12.08 mmol) at -10 °C. The mixture was stirred at room temperature for 26 h. The reaction solution was added MeOH to quench the reaction. The reaction solution was concentrated under reduced pressure, diluted with EtOAc (50 mL), washed with $NaHCO_3$ solution (20 mL) and saturated NaCl solution (20 mL), dried over anhydrous $Na_2SO_4$, concentrated under reduced pressure, and the concentrated solution was separated by silica gel column chromatography (eluent: DCM/MeOH (v/v) = 20/1) to give a pale yellow solid (598 mg, 93%).
MS (ESI, pos.ion) m/z: 161.1 $[M+H]^+$.

**Step 2: Synthesis of methyl 4-((2*S*,4*R*)-2-((difluoromethoxy)methyl)-4-((methylsulfonyl)oxy)pyrrolidin-1-yl)benzoate**

Methyl 4-((2*S*,4*R*)-2-((difluoromethoxy)methyl)-4-hydroxypyrrolidin-1-yl)benzoate (200 mg, 0.66 mmol), MsCl (0.10 mL, 1.29 mmol), DMAP (10 mg, 0.08 mmol) and TEA (0.30 mL, 2.16 mmol) were added to DCM (4 mL). The mixture was stirred at room temperature for 23 h. The reaction solution was concentrated under reduced pressure, and the concentrated solution was separated by silica gel column chromatography (eluent: PE/EtOAc (v/v) = 3/2) to give pale yellow liquid (233 mg, 93%).
MS (ESI, pos.ion) m/z: 380.1 $[M+H]^+$.

**Step 3: Synthesis of methyl 4-((2*S*,4*S*)-4-(4-(1*H*-pyrazol-1-yl)phenoxy)-2-((difluoromethoxy)methyl)pyrrolidin-1-yl)benzoate**

Methyl 4-((2*S*,4*R*)-2-((difluoromethoxy)methyl)-4-((methylsulfonyl)oxy)pyrrolidin -1-yl)benzoate (230 mg, 0.61 mmol), 4-(1*H*-pyrazol-1-yl)phenol (116 mg, 0.72 mmol) and $K_2CO_3$ (251 mg, 1.82 mmol) were added to DMF (6mL), and the mixture was reacted at 100 °C for 16 h. The reaction solution was cooled to room temperature, diluted with EtOAc (50 mL), washed with $H_2O$ (25 mL × 2) and saturated NaCl solution (25 mL), dried over anhydrous $Na_2SO_4$, concentrated under reduced pressure, and the concentrated solution was separated by silica gel column chromatography (eluent: PE/EtOAc (v/v) = 2/1) to give a white solid (166 mg, 62%).
MS (ESI, pos.ion) m/z: 444.1 $[M+H]^+$.

**Step 4: Synthesis of 4-((2*S*,4*S*)-4-(4-(1*H*-pyrazol-1-yl)phenoxy)-2-((difluoromethoxy) methyl)pyrrolidin-1-yl)benzoic acid**

To a solution of methyl 4-((2*S*,4,*S*)-4-(4-(1*H*-pyrazol-1-yl)phenoxy)-2 -((difluoromethoxy)methyl)pyrrolidin-1-yl)benzoate (160 mg, 0.36 mmol) in MeOH (2 mL) and THF (2 mL) were added a solution of LiOH (86 mg, 3.59 mmol) in $H_2O$ (1 mL). The mixture was stirred at 50°C for 16 h. The reaction solution was concentrated under reduced pressure, the remaining solution was added with HCl solution (1 mol/L) to adjust the pH of the solution to about 4. The resulting mixture was extracted with EtOAc (30 mL), washed with saturated NaCl (10 mL) solution, dried over anhydrous $Na_2SO_4$, concentrated under reduced pressure, and the concentrated solution was separated by silica gel column chromatography (eluent: DCM/EtOAc (v/v) = 3/2) to give a white solid (120 mg, 77%).
MS (ESI, pos.ion) m/z: 430.2 $[M+H]^+$.

**Step 5: Synthesis of 4-((2*S*,4*S*)-4-(4-(1*H*-pyrazol-1-yl)phenoxy)-2-((difluoromethoxy)methyl)pyrrolidin-1-yl)-*N*-((S)-2-cyano-1-(4-(ethylsulfonyl)phenyl) ethyl)benzamide**

HATU (140 mg, 0.37 mmol), (*S*)-3-amino-3-(4-(ethylsulfonyl)phenyl)propionitrile hydrochloride (84 mg, 0.31 mmol), 4-((2*S*,4*S*)-4-(4-(1*H*-pyrazol-1-yl)phenoxy)-2-((difluoromethoxy) methyl)pyrrolidin-1-yl)benzoic acid (120 mg, 0.28 mmol) and TEA (90 mg, 0.89 mmol) were successively added to DCM (6 mL), and the mixture was stirred at room temperature for 20 h. The reaction solution was concentrated under reduced pressure. The residue was diluted with DCM (40 mL), washed successively with HCl solution (15 mL, 1 mol/L), saturated $NaHCO_3$ solution (15 mL) and saturated NaCl solution (15 mL), dried over anhydrous $Na_2SO_4$, and concentrated under reduced pressure. The concentrated solution was separated by silica gel column chromatography (eluent: DCM/EtOAc (v/v) = 1/1) to give a white solid(150 mg, 83%).

MS (ESI, pos.ion) m/z: 650.2 $[M+H]^+$.
$^1$H NMR (400 MHz, $CDCl_3$) δ (ppm): 7.94 (d, *J*= 8.2 Hz, 2H), 7.85 (d, *J*= 2.0 Hz, 1H), 7.78 - 7.69 (m, 3H), 7.68 - 7.59 (m, 4H), 6.98 (d, *J*= 8.9 Hz, 2H), 6.66 (d, *J*= 8.8 Hz, 2H), 6.59 (d, *J*= 7.6 Hz, 1H), 6.46 (s, 1H), 6.23 (t, *J* = 74.3 Hz, 1H), 5.60 (dd, *J* = 12.0, 6.3 Hz, 1H), 5.15 (t, *J* = 4.6 Hz, 1H), 4.24 - 4.15 (m, 2H), 4.00 (t, *J* = 9.6 Hz, 1H), 3.78 (d, *J* = 11.3 Hz, 1H), 3.68 (dd, *J* = 11.3, 4.7 Hz, 1H), 3.21 - 3.02 (m, 4H), 2.54 (d, *J*= 14.5 Hz, 1H), 2.41 - 2.32 (m, 1H), 1.29 (t, *J*= 7.4 Hz, 3H).

**Example 153 (*R*)-2-(4-((2*S*,4*S*)-4-((5-chloropyridin-2-yl)oxy)-2-((difluoromethoxy)methyl) pyrrolidin-1-yl) benzoylamino)-2-(4-(ethylsulfonyl)phenyl)ethyl carbamate**

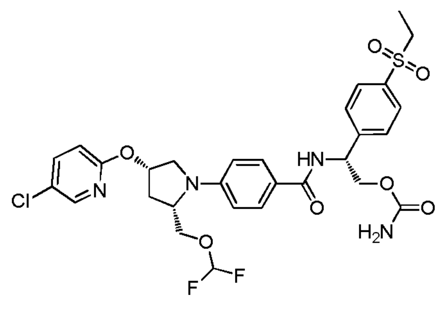

**Step 1: Synthesis of methyl 4-((2*S*,4*S*)-4-((5-chloropyridin-2-yl)oxy) -2-((difluoromethoxy)methyl)pyrrolidin-1-yl)benzoate**

5-Chloro-2-fluoro-pyridine (262 mg, 2.0 mmol), methyl 4-((2*S*,4*S*)-2-((difluoromethoxy)methyl) -4-hydroxypyridin-1-yl)benzoate (500 mg, 1.66 mmol) and *t*-BuOK (280 mg, 2.50 mmol) were dissolved in THF (10 mL). The mixture was degassed and refilled with $N_2$, and stirred at 80 °C for 5 h. The resulting mixture was washed with saturated $NH_4Cl$ solution, and extracted with EtOAc (10 mL × 3). The organic phases were combined, dried over anhydrous $Na_2SO_4$, filtered, concentrated and separated by silica gel column chromatography (eluent: DCM/EA (v/v) = 50/1) to give a white solid (520 mg, 76%).
MS (ESI, pos.ion) m/z: 413 $[M+H]^+$.

**Step 2: Synthesis of 4-((2*S*,4*S*)-4-((5-chloropyridin-2-yl)oxy)-2-((difluoromethoxy) methyl)pyrrolidin-1-yl)benzoic acid**

To a solution of methyl 4-((2*S*,4*S*)-4-((5-chloropyridin-2-yl)oxy)-2-((difluoromethoxy) methyl)pyrrolidin-1-yl)benzoate (300 mg, 0.73 mmol) in MeOH (5 mL) was added a solution of LiOH (87 mg, 3.63 mmol) in $H_2O$ (5 mL). The mixture was stirred at room temperature for 12 h. Dilute hydrochloric acid was added to adjust the pH of the solution to be weakly acidic. The resulting mixture was extracted with EtOAc (10 mL × 3). The organic phases were combined, dried over anhydrous $Na_2SO_4$, filtered, concentrated and separated by silica gel column chromatography (eluent: DCM/EA (v/v) = 5/1) to give a white solid (270 mg, 93%).
MS (ESI, pos.ion) m/z: 399 $[M+H]^+$.

**Step 3: Synthesis of (*R*)-2-(4-((2*S*,4*S*)-4-((5-chloropyridin-2-yl)oxy)-2-((difluoromethoxy) methyl)pyrrolidin-1-yl)benzoylamino)-2-(4-(ethylsulfonyl)phenyl)ethyl carbamate**

4-((2*S*,4*S*)-4-((5-Chloropyridin-2-yl)oxy)-2-((difluoromethoxy)methyl)pyrrolidin-1-yl) benzoic acid (80 mg, 0.20 mmol), (R)-2-amino-2-(4-(ethylsulfonyl)phenyl)ethyl carbamate (60 mg, 0.22 mmol) and HATU (92 mg, 0.24 mmol) were dissolved in DCM (25 mL), then TEA (0.064 mL, 0.49 mmol) was added. The mixture was stirred at room temperature for 24 h. The resulting mixture was washed with saturated $NH_4Cl$ solution, and extracted with DCM (10 mL × 3). The organic phases were combined, dried over anhydrous $Na_2SO_4$, filtered, concentrated and separated by silica gel column chromatography (eluent: DCM/EtOAc (v/v) = 5/1) to give a white solid (82 mg, 63%).

MS (ESI, pos.ion) m/z: 653 $[M+H]^+$.
$^1H$ NMR (400 MHz, $CDCl_3$) δ (ppm): 8.13 (d, *J*= 2.4 Hz, 1H), 7.85 (d, *J*= 8.2 Hz, 2H), 7.78 (d, *J*= 8.7 Hz, 2H), 7.66 (d, *J* = 6.3 Hz, 1H), 7.57 (d, *J* = 8.2 Hz, 3H), 6.72 (d, *J* = 8.8 Hz, 1H), 6.66 (d, *J* = 8.7 Hz, 2H), 6.25 (t, *J* = 74.4 Hz, 1H), 5.74 (s, 1H), 5.42 (s, 1H), 4.93 (s, 2H), 4.54 (dd, *J* = 11.9, 7.9 Hz, 1H), 4.33 (dd, *J* = 12.0, 3.3 Hz, 1H), 4.26 - 4.07 (m, 3H), 3.71 (d, *J* = 2.4 Hz, 2H), 3.09 (q, *J*= 7.4 Hz, 2H), 2.52 - 2.32 (m, 2H), 1.36 - 1.22 (m, 3H).

**Example 154 (*R*)-2-(4-((2*S*,4*S*)-2-((difluoromethoxy)methyl)-4-(4-(trifluoromethyl) phenoxy)pyrrolidin -1-yl)benzoylamino)-2-(4-(ethylsulfonyl)phenyl)ethyl dimethylcarbamate**

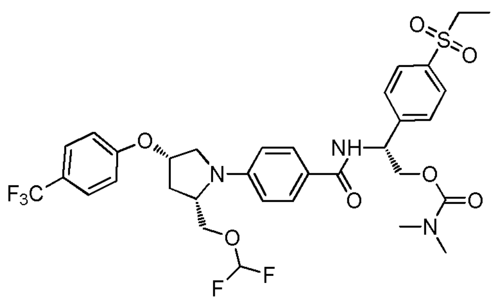

**Step 1: Synthesis of (*R*)-2-(((benzyloxy)carbonyl)amino)-2-(4-(ethylsulfonyl)phenyl)ethyl dimethylcarbamate**

To a mixture of benzyl (*R*)-(1-(4-(ethylsulfonyl)phenyl)-2-hydroxyethyl)carbamate (400 mg, 1.10 mmol) and bis(1*H*-imidazol-1-yl)methanone (357 mg, 2.20 mmol) was added THF (10 mL) under $N_2$ protection, and the mixture

was reacted at room temperature for 16 h. Dimethylamine hydrochloride (359 mg, 4.40 mmol) and TEA (445 mg, 4.40 mmol) were added, and the mixture was reacted at room temperature for 8 h. The resulting mixture was washed with saturated $NH_4Cl$ solution, extracted with EtOAc (10 ml × 3). The organic phases were combined, dried over anhydrous $Na_2SO_4$, filtered, concentrated and separated by silica gel column chromatography (eluent: DCM/EtOAc (v/v) = 5/1) to give a yellow solid (107 mg, 25%).
MS (ESI, pos.ion) m/z: 435 $[M+H]^+$.

**Step 2: Synthesis of (*R*)-2-amino-2-(4-(ethylsulfonyl)phenyl)ethyl dimethylcarbamate**

To (*R*)-2-(((benzyloxy)carbonyl)amino)-2-(4-(ethylsulfonyl)phenyl)ethyl dimethylcarbamate (111 mg, 0.26 mmol) were added Pd/C (100 mg) and methanol (10 mL) under $H_2$ protection, and the mixture was reacted at room temperature for 12 h. The mixture was filtered with suction through a celite pad and concentrated to give a yellow solid (24 mg, 30%).
MS (ESI, pos.ion) m/z: 435 $[M+H]^+$.

**Step 3: Synthesis of (*R*)-2-(4-((2*S*,4*S*)-2-((difluoromethoxy)methyl)-4-(4-(trifluoromethyl) phenoxy)pyrrolidin-1-yl)benzoylamino)-2-(4-(ethylsulfonyl)phenyl)ethyl dimethylcarbamate**

4-((2*S*, 4*S*)-2-((Difluoromethoxy)methyl)-4-(4-(trifluoromethyl)phenoxy)pyrrolidin-1-y l)benzoic acid (28 mg, 0.07 mmol), ((*R*)-2-amino-2-(4-(ethylsulfonyl)phenyl)ethyl dimethylcarbamate (24mg, 0.08 mmol) and HATU (31 mg, 0.08 mmol) were dissolved in DCM (10 mL), then TEA (0.02 mL, 0.14 mmol) was added. The mixture was reacted at room temperature for 12 h. The resulting mixture was washed with saturated $NH_4Cl$ solution, and extracted with DCM (10 mL × 3). The organic phases were combined, dried over anhydrous $Na_2SO_4$, filtered, concentrated and separated by silica gel column chromatography (eluent: DCM/EtOAc (v/v) = 5/1) to give a white solid (4.8 mg, 10%).

MS (ESI, pos.ion) m/z: 714.9 $[M+H]^+$.
$^1H$ NMR (400 MHz, $CDCl_3$) δ (ppm): 7.86 (d, *J* = 8.4 Hz, 3H), 7.81 (d, *J* = 8.7 Hz, 2H), 7.60 (d, *J* = 8.6 Hz, 2H), 7.57 (d, *J* = 8.2 Hz, 2H), 6.99 (d, *J* = 8.6 Hz, 2H), 6.68 (d, *J* = 8.7 Hz, 2H), 6.24 (t, *J* = 74.3 Hz, 1H), 5.37 (dd, *J* = 11.0, 6.0 Hz, 1H), 5.19 (t, *J* = 4.7 Hz, 1H), 4.98 (d, *J* = 4.6 Hz, 1H), 4.54 (dd, *J* = 12.1, 8.1 Hz, 1H), 4.32 (dd, *J* = 12.2, 3.2 Hz, 1H), 4.21 (ddd, *J* = 14.0, 9.3, 4.3 Hz, 2H), 3.98 (t, *J* = 9.6 Hz, 1H), 3.78 (d, *J* = 11.3 Hz, 1H), 3.72 (dd, *J* = 11.3, 4.8 Hz, 1H), 3.10 (q, *J* = 7.4 Hz, 2H), 2.91 (s, 3H), 2.82 (s, 3H), 2.53 (d, *J* = 14.4 Hz, 1H), 2.46 - 2.37 (m, 1H), 1.30 - 1.25 (m, 3H).

**Example 155 (*R*)-2-(4-((2*S*,4*S*)-2-((difluoromethoxy)methyl)-4-((5-(trifluoromethoxy)pyridin -2-yl)oxy)pyrrolidin-1-yl)benzoylamino)-2-(4-(ethylsulfonyl)phenyl)ethyl methylcarbamate**

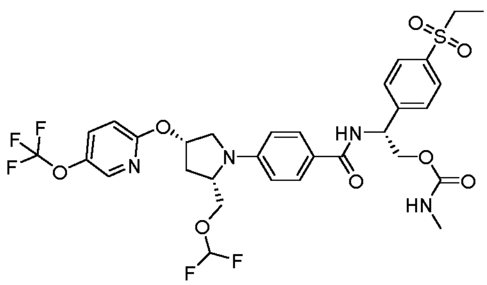

4-((2*S*,4*S*)-2-((Difluoromethoxy)methyl)-4-((5-(tiifluoromethoxy)pyridin-2-yl)oxy)pyr rolidin-1-yl)benzoic acid (45 mg, 0.19 mmol) prepared according to the method of the present invention, (*R*)-2-amino-2-(4-(ethylsulfonyl)phenyl)ethyl methylcarbamate (35 mg, 0.12 mmol) and HATU (88 mg, 0.23 mmol) were dissolved in DCM (10 mL), then TEA (0.032 mL, 0.2 mmol) was added. The mixture was reacted at room temperature for 11 h. The reaction was quenched by adding saturated $NH_4Cl$ solution (15 ml). The resulting mixture was extracted with DCM (20 ml × 3). The organic phases were combined, dried over anhydrous $Na_2SO_4$, concentrated under reduced pressure and the concentrated solution was separated by silica gel column chromatography (eluent: DCM/EtOAc (v/v) = 10/1) to give a white solid (26 mg, 35%).

MS (ESI, pos.ion) m/z: 717 $[M+H]^+$.

$^1H$ NMR (400 MHz, $CDCl_3$) δ (ppm): 8.13 (d, *J* = 2.4 Hz, 1H), 7.88 (d, *J* = 8.2 Hz, 2H), 7.79 (t, *J* = 9.6 Hz, 3H), 7.57 (d, *J* = 8.2 Hz, 2H), 7.52 (d, *J* = 8.9 Hz, 1H), 6.79 (d, *J* = 9.0 Hz, 1H), 6.69 (d, *J* = 8.7 Hz, 2H), 6.27 (t, *J* = 74.3 Hz, 1H), 5.77 (s, 1H), 5.38 (s, 2H), 4.81 (s, 1H), 4.55 (dd, *J* = 12.1, 8.1 Hz, 1H), 4.33 (dd, *J* = 12.2, 3.0 Hz, 1H), 4.19 (dd, *J* = 12.9, 5.0 Hz, 2H), 4.00 (t, *J* = 9.1 Hz, 1H), 3.74 (d, *J* = 2.4 Hz, 2H), 3.10 (q, *J* = 7.4 Hz, 2H), 2.83 (d, *J* = 4.8 Hz, 3H), 2.45 (dt, *J* = 15.0, 10.5 Hz, 2H), 2.14 - 1.92 (m, 1H), 1.32 - 1.26 (m, 3H).

**Example 156 (*R*)-2-(4-((2*S*,4*S*)-2-((difluoromethoxy)methyl)-4-((5-(trifluoromethoxy) pyridin-2-yl)oxy) pyrrolidin-1-yl)benzoylamino)-2-(4-(ethylsulfonyl)phenyl)ethyl carbamate**

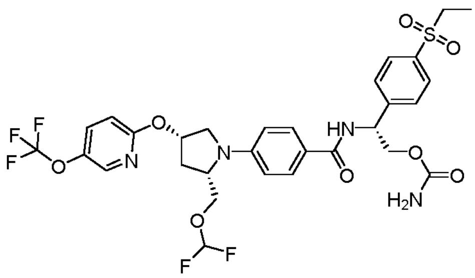

(*R*)-2-Amino-2-(4-(ethylsulfonyl)phenyl)ethyl carbamate (76 mg, 0.28 mmol), 4-((2*S*,4*S*)-2-((difluoromethoxy)methyl)-4-((5-(trifluoromethoxy)pyridin-2-yl)oxy)pyrrolidin-1-yl)b enzoic acid (104 mg, 0.23 mmol) and HATU (106 mg, 0.28 mmol) were dissolved in DCM (10 mL), then TEA (0.5 ml, 3.86 mmol) was added dropwise, and the mixture was reacted at room temperature for 16 h. The reaction was quenched by adding saturated $NH_4Cl$ solution (15 ml). The resulting mixture was extracted with DCM (20 mL × 3). The organic phases were combined, dried over anhydrous $Na_2SO_4$, concentrated under reduced pressure, and the concentrated solution was separated by silica gel column chromatography (eluent: DCM/EtOAc (v/v) = 5/1) to give a white solid (77 mg, 47%).

MS (ESI, pos.ion) m/z: 703 $[M+H]^+$.
$^1H$ NMR (400 MHz, $CDCl_3$) δ (ppm): 8.13 (d, *J*= 2.3 Hz, 1H), 7.88 (d, *J*= 8.2 Hz, 2H), 7.79 (d, *J*= 8.7 Hz, 2H), 7.63 (d, *J* = 6.0 Hz, 1H), 7.58 (d, *J* = 8.2 Hz, 2H), 7.52 (d, *J* = 9.1 Hz, 1H), 6.79 (d, *J* = 9.0 Hz, 1H), 6.68 (d, *J* = 8.7 Hz, 2H), 6.26 (t, *J* = 74.3 Hz, 1H), 5.77 (s, 1H), 5.42 (s, 1H), 4.82 (s, 2H), 4.57 (dd, *J* = 12.1, 8.0 Hz, 1H), 4.35 (dd, *J* = 12.0, 3.2 Hz, 1H), 4.19 (dd, *J* = 10.6, 4.0 Hz, 2H), 4.00 (t, *J* = 9.5 Hz, 1H), 3.73 (d, *J* = 2.5 Hz, 2H), 3.10 (q, *J* = 7.4 Hz, 2H), 2.51 - 2.37 (m, 2H), 1.32 - 1.18 (m, 3H).

**Example 157 4-((2S,4S)-4-(4-(2*H*-1,2,3-triazol-2-yl)phenoxy)-2-((difluoromethoxy)methyl) pyrrolidin -1-yl)-*N*-((S)-2-cyano-1-(4-(ethylsulfonyl)phenyl)ethyl)benzamide**

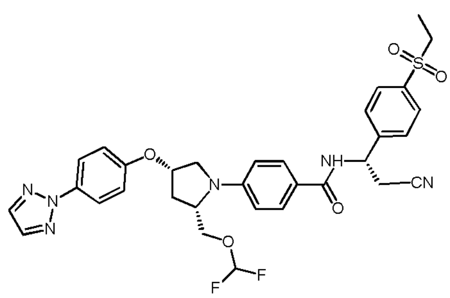

**Step 1: Synthesis of 4-(1*H*-1,2,3-triazol-2-yl)phenol**

4-Iodophenol (2.00 g, 9.10 mmol), 2*H*-triazole (0.94 g, 14 mmol), CuO (0.072 g, 0.91 mmol), iron triacetylacetonate (0.96 g, 2.70 mmol) and $Cs_2CO_3$ (5.90 g, 18.00 mmol) were dissolved in DMF (20 mL), and the mixture was heated to 90°C and reacted for 20 h. The reaction solution was cooled to room temperature, filtered, and concentrated under reduced pressure. The concentrated solution was separated by silica gel column chromatography (eluent: PE/EA

(v/v) = 5/1) to give a white solid (500 mg, 34%).
MS (ESI, pos.ion) m/z: 162.08 [M+H]$^+$.

**Step 2: Synthesis of methyl 4-((2*S*,4*S*)-4-(4-(2*H*-1,2,3-triazol-2-yl)phenoxy) -2-((difluoromethoxy)methyl)pyrrolidin-1-yl)benzoate**

4-(2*H*-1,2,3-Triazol-2-yl)phenol (66 mg, 0.41 mmol), $K_2CO_3$ (100 mg, 0.70 mmol) and methyl 4-((2*S*,4R)-2-((difluoromethoxy)methyl)-4-((methylsulfonyl)oxy)pyrrolidin-1-yl)benzoate (130 mg, 0.34 mmol) were dissolved in DMF (10 mL) solution. The mixture was reacted at 60 °C for 5 h. The resulting mixture was washed with saturated $NH_4Cl$ solution, and extracted with EtOAc (10 mL × 3). The organic phases were combined, dried over anhydrous $Na_2SO_4$, filtered, concentrated and separated by silica gel column chromatography (eluent: PE/EtOAc (v/v) = 5/1) to give a white solid (80 mg, 50%).
MS (ESI, pos.ion) m/z: 445.2 [M+H]$^+$.

**Step 3: Synthesis of 4-((2S,4S)-4-(4-(2*H*-1,2,3-triazol-2-yl)phenoxy)-2-((difluoromethoxy) methyl)pyrrolidin-1-yl)benzoic acid**

To a solution of methyl 4-((2*S*,4*S*)-4-(4-(2*H*-1,2,3-triazol-2-yl)phenoxy) -2-((difluoromethoxy)methyl)pyrrolidin-1-yl)benzoate (25 mg, 0.056 mmol) in MeOH (5 mL) was added LiOH (7.2 mg, 0.30 mmol). The mixture was stirred at room temperature for 24 h. Dilute hydrochloric acid was added to adjust the pH of the solution to be weakly acidic. The resulting mixture was extracted with EtOAc (10 mL × 3). The organic phases were combined, dried over anhydrous $Na_2SO_4$, filtered, concentrated and separated by silica gel column chromatography (eluent: DCM/EtOAc (v/v) = 5/1) to give a white solid (23 mg, 94%).
MS (ESI, pos.ion) m/z: 431.5 [M+H]$^+$.

**Step 4: Synthesis of 4-((2*S*,4*S*)-4-(4-(2*H*-1,2,3-triazol-2-yl)phenoxy)-2-((difluoromethoxy) methyl)pyrrolidin-1-yl)-*N*-((S)-2-cyano-1-(4-(ethylsulfonyl)phenyl)ethyl)benzamide**

4-((2*S*,4*S*)-4-4-(2*H*-1,2,3-Triazol-2-yl)phenoxy)-2-((difluoromethoxy)methyl)pyrrolidi n-1-yl)benzoic acid (100 mg, 0.23 mmol), (*S*)-3-amino-3-(4-(ethylsulfonyl)phenyl)propionitrile (65 mg, 0.27 mmol) and HATU (107 mg, 0.28 mmol) were dissolved in DCM (25 mL), then TEA (0.069 mL, 0.53 mmol) was added. The mixture was stirred at room temperature for 24 h. The resulting mixture was washed with saturated $NH_4Cl$ solution, extracted with DCM (10 mL × 3). The organic phases were combined, dried over anhydrous $Na_2SO_4$, filtered, concentrated and separated by silica gel column chromatography (eluent: DCM/EtOAc (v/v) = 5/1) to give a white solid (82 mg, 54%).

MS (ESI, pos.ion) m/z: 651 [M+H]$^+$.
$^1$H NMR (400 MHz, $CDCl_3$) δ (ppm): 8.04 (d, *J* = 8.9 Hz, 2H), 7.93 (d, *J* = 8.2 Hz, 2H), 7.81 (s, 2H), 7.77 (d, *J* = 8.6 Hz, 2H), 7.67 (d, *J* = 8.2 Hz, 2H), 7.02 (d, *J* = 8.9 Hz, 2H), 6.82 (d, *J* = 7.5 Hz, 1H), 6.67 (d, *J* = 8.7 Hz, 2H), 6.25 (t, *J* = 74.3 Hz, 1H), 5.61 (dd, *J* = 12.6, 6.1 Hz, 1H), 5.19 (s, 1H), 4.20 (d, *J*= 7.2 Hz, 2H), 4.03 (d, *J*= 9.1 Hz, 1H), 3.80 (d, *J*= 11.3 Hz, 1H), 3.71 (dd, *J*= 11.3, 4.6 Hz, 1H), 3.12 (dd, *J*= 12.7, 6.0 Hz, 4H), 2.56 (d, *J* = 14.3 Hz, 1H), 2.46 - 2.34 (m, 1H), 1.30 (t, *J* = 7.4 Hz, 3H).

**Example 158 4-((2*S*,4*S*)-4-(5-(1*H*-pyrazol-1-yl)pyridin-2-yl)oxy)-2-((difluoromethoxy) methyl)pyrrolidin -1-yl)-*N*-((S)-2-cyano-1-(4-(ethylsulfonyl)phenyl)ethyl)benzamide**

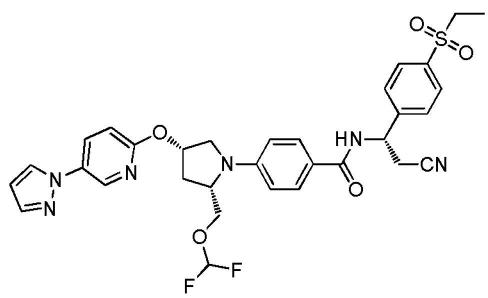

**Step 1: Synthesis of methyl 4-((2*S*,4*S*)-2-((difluoromethoxy)methyl)-4-((5-iodopyridin-2-yl) oxy)pyrrolidin-1-yl)benzoate**

Methyl 4-((2*S*, 4*R*)-2-((difluoromethoxy)methyl)-4-((methylsulfonyl)oxy)pyrrolidin -1-yl)benzoate (350 mg, 0.92 mmol) prepared according to the method of the present invention, 2-hydroxy-5-iodopyridine (225 mg, 1.02 mmol) and $K_2CO_3$ (255 mg, 1.85 mmol) were added to DMF (8 mL) and the mixture was reacted at 80 °C for 16 h. The reaction solution was cooled to room temperature, diluted with EtOAc (50 mL), washed with $H_2O$ (20 mL × 2) and saturated NaCl solution (20 mL), dried over anhydrous $Na_2SO_4$, concentrated under reduced pressure, and the concentrated solution was separated by silica gel column chromatography (eluent: PE/EtOAc (v/v) = 4/1) to give pale yellow liquid (386 mg, 83%).
MS (ESI, pos.ion) m/z: 505.0 $[M+H]^+$.

**Step 2: Synthesis of methyl 4-((2*S*,4*S*)-4-(5-(1H-pyrazol-1-yl)pyridin-2-yl)oxy)-2-((difluoromethoxy) methyl)pyrrolidin-1-yl)benzoate**

Methyl 4-((2*S*,4*S*)-2-((difluoromethoxy)methyl)-4-((5-iodopyridin-2-yl)oxy)pyrrolidin -1-yl)benzoate (380 mg, 0.75 mmol), 1*H*-pyrazole (60 mg, 0.88 mmol), CuI (11 mg, 0.14 mmol), (*S*)-2-methylproline methyl ester hydrochloride (17 mg, 0.15 mmol) and $K_2CO_3$ (162 mg, 1.14 mmol) were added to DMSO (4 mL) under nitrogen protection, and the mixture was reacted at 100 °C for 23 h. The reaction solution was cooled to room temperature, diluted with EtOAc (50 mL), washed with $H_2O$ (20 mL × 2) and saturated NaCl solution (20 mL), dried over anhydrous $Na_2SO_4$, concentrated under reduced pressure, and the concentrated solution was separated by silica gel column chromatography (eluent: PE/EtOAc (v/v) = 3/2) to give pale yellow liquid (223 mg, 67%).
MS (ESI, pos.ion) m/z: 445.2 $[M+H]^+$.

**Step 3: Synthesis of 4-((2*S*,4*S*)-4-((5-(1*H*-pyrazol-1-yl)pyridin-2-yl)oxy) -2-((difluoromethoxy)methyl) pyrrolidin-1-yl)benzoic acid**

To a solution of methyl 4-((2*S*,4*S*)-4-(5-(1*H*-pyrazol-1-yl)pyridin-2-yl)oxy)-2-((difluoromethoxy)methyl)pyrrolidin-1-yl)benzoate (223 mg, 0.50 mmol) in MeOH (2 mL) and THF (2 mL) was added a solution of LiOH (120 mg, 5.01 mmol) in $H_2O$ (1 mL). The mixture was stirred at 50°C for 12 h. The reaction solution was concentrated under reduced pressure, and the remaining liquid was added with concentrated HCl solution (1 mol/L) to adjust the pH to 4. The resulting mixture was extracted with EtOAc (20 mL), washed with saturated NaCl solution (10 mL), dried over anhydrous $Na_2SO_4$, and concentrated under reduced pressure to give a pale yellow solid (188 mg, 87%).
MS (ESI, pos.ion) m/z: 431.3 $[M+H]^+$.

**Step 4: Synthesis of 4-((2*S*,5*S*)-4-((5-(1H-pyrazol-1-yl)pyridin-2-yl)oxy)-2-((difluoromethoxy) methyl)pyrrolidin-1-yl)-*N*-((S)-2-cyano-1-(4-(ethylsulfonyl)phenyl)ethyl)benzamide**

HATU (238 mg, 0.63 mmol), (*S*)-3-amino-3-(4-(ethylsulfonyl)phenyl)propionitrile hydrochloride (126 mg, 0.46 mmol), 4-((2S,4S)-4-((5-(1*H*-pyrazol-1-yl)pyridin-2-yl)oxy)-2-((difluoromethoxy) methyl)pyrrolidin-1-yl)benzoic acid (180 mg , 0.42 mmol) and TEA (130 mg, 1.28 mmol) were successively added to DCM (6 mL), and the mixture was stirred at room temperature for 21 h. The reaction solution was concentrated under reduced pressure. The residue was diluted with DCM (40 mL), washed successively with HCl solution (15 mL, 1 mol/L), saturated $NaHCO_3$ solution (15 mL) and saturated NaCl solution (15 mL), dried over anhydrous $Na_2SO_4$, and concentrated under reduced pressure. The concentrated solution was separated by silica gel column chromatography (eluent: DCM/EtOAc (v/v) = 1/1) to give a white solid (230 mg, 85%).

MS (ESI, pos.ion) m/z: 651.4 $[M+H]^+$.
$^1H$ NMR (400 MHz, $CDCl_3$) δ (ppm): 8.48 (d, *J* = 2.5 Hz, 1H), 7.96 (dd, *J* = 8.8, 2.7 Hz, 1H), 7.91 (d, *J*= 8.3 Hz, 2H), 7.84 (d, *J*= 2.2 Hz, 1H), 7.76 - 7.70 (m, 3H), 7.65 (d, *J*= 8.2 Hz, 2H), 6.84 (d, *J* = 8.8 Hz, 1H), 6.73 (d, *J* = 7.3 Hz, 1H), 6.65 (d, *J* = 8.8 Hz, 2H), 6.49 (d, *J* = 1.8 Hz, 1H), 6.24 (t, *J* = 74.4 Hz, 1H), 5.81 (s, 1H), 5.59 (dd, *J* = 12.7, 6.2 Hz, 1H), 4.24 - 4.15 (m, 2H), 4.01 (t, *J* = 9.1 Hz, 1H), 3.74 (s, 2H), 3.18 - 3.02 (m, 4H), 2.50 (d, *J* = 14.4 Hz, 1H), 2.45 - 2.38 (m, 1H), 1.27 (d, *J* = 7.4 Hz, 3H).

**Example 159 *N*-((*S*)-2-cyano-1-(4-(ethylsulfonyl)phenyl)ethyl)-4-((2*S*,4*S*)-2-((difluoromethoxy)methyl)-4-(4-(4-methyl-1*H*-pyrazol-1-yl)phenoxy)pyrrolidin-1-yl) benzamide**

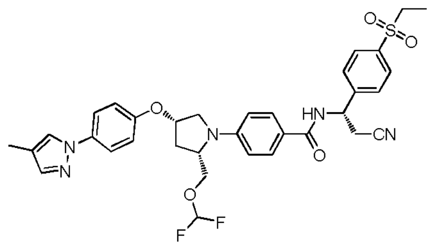

**Step 1: Synthesis of methyl 4-((2*S*,4*S*)-2-((difluoromethoxy)methyl)-4-(4-iodophenoxy) pyrrolidin-1-yl)benzoate**

Methyl 4-((2*S*,4*R*)-2-((difluoromethoxy)methyl)-4-((methyl sulfonyl)oxy)pyrrolidin-1-yl)benzoate (1.80 g, 4.74 mmol), 4-iodophenol (1.25 g, 5.68 mmol) and $K_2CO_3$ (1.31 g, 9.48 mmol) were added to DMF (16 mL), and the mixture was reacted at 100 °C for 12 h. The reaction solution was cooled to room temperature, diluted with EtOAc (80 mL), washed with $H_2O$ (40 mL × 2) and saturated NaCl solution (40 mL), dried over anhydrous $Na_2SO_4$, and concentrated under reduced pressure. The concentrated solution was separated by silica gel column chromatography (eluent: PE/EtOAc (v/v) = 4/1) to give a pale yellow solid (1.44 g, 60%).
MS (ESI, pos.ion) m/z: 504.2 $[M+H]^+$.

**Step 2: Synthesis of methyl 4-((2*S*,4*S*)-2-((difluoromethoxy)methyl)-4-(4-(4-methyl -1*H*-pyrazol-1-yl)phenoxy)pyrrolidin-1-yl)benzoate**

Methyl 4-((2*S*,4*S*)-2-((difluoromethoxy)methyl)-4-(4-iodophenoxy)pyrrolidin-1-yl)benzoate (250 mg, 0.50 mmol), 4-methyl-1*H*-pyrazole (44 mg, 0.54 mmol), CuI (7 mg, 0.09 mmol), (*S*)-2-methylproline methyl ester hydrochloride (11 mg, 0.10 mmol) and $K_2CO_3$ (106 mg, 0.74 mmol) were added to DMSO (4 mL) under nitrogen protection, and the mixture was reacted at 100 °C for 23 h. The reaction solution was cooled to room temperature, diluted with EtOAc (50 mL), washed with $H_2O$ (20 mL × 2) and saturated NaCl solution (20 mL), dried over anhydrous $Na_2SO_4$, and concentrated under reduced pressure. The concentrated solution was separated by silica gel column chromatography (eluent: PE/EtOAc (v/v) = 2/1) to give a white solid (135 mg, 59%).
MS (ESI, pos.ion) m/z: 458.2 $[M+H]^+$.

**Step 3: Synthesis of 4-((2*S*,4*S*)-2-((difluoromethoxy)methyl)-4-(4-(4-methyl-1H-pyrazol -1-yl)phenoxy)pyrrolidin-1-yl)benzoic acid**

To a solution of methyl 4-((2*S*,4*S*)-2-((difluoromethoxy)methyl)-4-(4-(4-methyl -1*H*-pyrazol-1-yl)phenoxy)pyrrolidin-1-yl)benzoate (130 mg, 0.28 mmol) in MeOH (2 mL) and THF (2 mL) was added a solution of LiOH (70 mg, 2.92 mmol) in $H_2O$ (1 mL). The mixture was reacted at 50°C for 16 h. The reaction solution was concentrated under reduced pressure, and the remaining liquid was added with concentrated HCl solution (1 mol/L) to adjust the pH to 4. The resulting mixture was extracted with EtOAc (30 mL), washed with saturated NaCl solution (10 mL), dried over anhydrous $Na_2SO_4$, and concentrated under reduced pressure to give a white solid (120 mg, 95%).
MS (ESI, pos.ion) m/z: 444.2 $[M+H]^+$.

**Step 4: Synthesis of *N*-((S)-2-cyano-1-(4-(ethylsulfonyl)phenyl)ethyl)-4-((2*S*,4*S*)-2-((difluoromethoxy)methyl)-4-(4-(4-methyl-1*H*-pyrazol-1-yl)phenoxy)pyrrolidin-1-yl)benzamid** e

HATU (133 mg, 0.35 mmol), (*S*)-3-amino-3-(4-(ethylsulfonyl)phenyl)propionitrile hydrochloride (90 mg, 0.33 mmol), 4-((2*S*,4*S*)-2-((difluoromethoxy)methyl)-4-(4-(4-methyl-1*H*-pyrazol-1-yl)phenoxy)pyrrolidin-1-yl)benzoic acid (120 mg , 0.27 mmol) and TEA (82 mg, 0.81 mmol) were successively added to DCM (6 mL), and the mixture was stirred at room temperature for 18 h. The reaction solution was concentrated under reduced pressure. The residue was diluted with DCM (40 mL), washed successively with HCl solution (15 mL, 1 mol/L), saturated $NaHCO_3$ solution (15 mL) and saturated NaCl solution (15 mL), dried over anhydrous $Na_2SO_4$, and concentrated under reduced pressure. The concentrated solution was separated by silica gel column chromatography (eluent: DCM/EtOAc (v/v) = 3/2) to give a white solid (140 mg, 78%).

MS (ESI, pos.ion) m/z: 664.2 $[M+H]^+$.
$^1H$ NMR (400 MHz, $CDCl_3$) δ (ppm): 7.93 (d, *J*= 8.3 Hz, 2H), 7.74 (d, *J*= 8.7 Hz, 2H), 7.65 (d, *J*= 8.3 Hz, 2H), 7.62 (s, 1H), 7.58 (d, *J* = 8.9 Hz, 2H), 7.50 (s, 1H), 6.95 (d, *J* = 8.9 Hz, 2H), 6.65 (d, *J* = 8.8 Hz, 3H), 6.22 (t, *J* = 74.4 Hz, 1H), 5.59 (dd, *J* = 12.5, 6.1 Hz, 1H), 5.13 (t, *J* = 4.5 Hz, 1H), 4.23 - 4.14 (m, 2H), 4.00 (t, *J* = 9.4 Hz, 1H), 3.77 (d,

*J* = 11.3 Hz, 1H), 3.67 (dd, *J* = 11.4, 4.8 Hz, 1H), 3.19 - 3.02 (m, 4H), 2.53 (d, *J* = 14.1 Hz, 1H), 2.39 - 2.32 (m, 1H), 2.16 (s, 3H), 1.29 (t, *J* = 7.4 Hz, 3H).

**Example 160 4-((2*S*,4*S*)-4-((5-chloropyridin-2-yl)oxy)-2-((difluoromethoxy)methyl)pyrrolidin -1-yl)-N-((*S*)-2-cyano-1-(4-(ethylsulfonyl)phenyl)ethyl)benzamide**

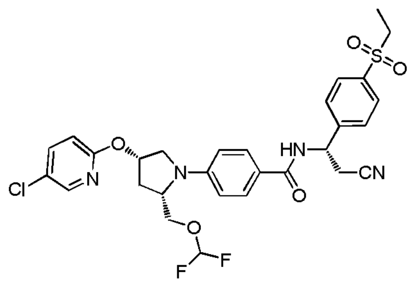

4-((2S,4S)-4-((5-chloropyridin-2-yl)oxy)-2-((difluoromethoxy)methyl)pyrrolidin-1-yl) benzoic acid (80 mg, 0.20 mmol), (*S*)-3-amino-3-(4-(ethylsulfonyl)phenyl)propionitrile (60 mg, 0.25 mmol) and HATU (92 mg, 0.24 mmol) were dissolved in DCM (25 mL), then TEA (0.064 mL, 0.49 mmol) was added and the mixture was stirred at room temperature for 24 h. The resulting mixture was washed with saturated $NH_4Cl$ solution, and extracted with DCM (10 mL × 3). The organic phases were combined, dried over anhydrous $Na_2SO_4$, filtered, concentrated and separated by silica gel column chromatography (eluent: DCM/EtOAc (v/v) = 5/1) to give a white solid (87 mg, 66%).

MS (ESI, pos.ion) m/z: 619 $[M+H]^+$.
$^1$H NMR (400 MHz, $CDCl_3$) δ (ppm): 8.13 (d, *J*= 2.4 Hz, 1H), 7.85 (d, *J*= 8.2 Hz, 2H), 7.78 (d, *J*= 8.7 Hz, 2H), 7.66 (d, *J*= 6.3 Hz, 1H), 7.57 (d, *J* = 8.2 Hz, 3H), 6.72 (d, *J* = 8.8 Hz, 1H), 6.66 (d, *J* = 8.7 Hz, 2H), 6.25 (t, *J* = 74.4 Hz, 1H), 5.74 (s, 1H), 5.42 (s, 1H), 4.54 (dd, *J* = 11.9, 7.9 Hz, 1H), 4.33 (dd, *J* = 12.0, 3.3 Hz, 1H), 4.26 - 4.07 (m, 3H), 3.71 (d, *J* = 2.4 Hz, 2H), 3.09 (q, *J* = 7.4 Hz, 2H), 2.52 - 2.32 (m, 2H), 1.36 - 1.22 (m, 3H).

**Example 161 *N*-((*S*)-2-cyano-1-(4-(ethylsulfonyl)phenyl)ethyl)-4-((2*S*,4*S*)-2-((difluoromethoxy)methyl)-4-(4-(4-fluoro-1*H*-pyrazol-1-yl)phenoxy)pyrrolidin-1-yl)** benzamide

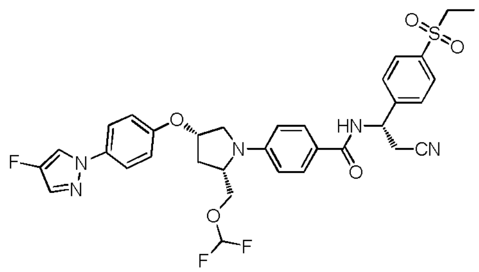

**Step 1: Synthesis of 4-((2*S*,4*S*)-2-((difluoromethoxy)methyl)-4-(4-iodophenoxy)pyrrolidin -1-yl)benzoic acid**

To a solution of methyl 4-((2*S*,4*S*)-2-((difluoromethoxy)methyl)-4-(4 -(4-iodophenoxy)pyrrolidin-1-yl)benzoate (500 mg, 0.99 mmol) in MeOH (2 mL) and THF (2 mL) was added a solution of LiOH (142 mg, 5.93 mmol) in $H_2O$ (1 mL). The mixture was reacted at 50°C for 16 h. The reaction solution was concentrated under reduced pressure, and the remaining liquid was added with concentrated HCl solution (1 mol/L) to adjust the pH to 4. The resulting mixture was extracted with EtOAc (30 mL), washed with saturated NaCl solution (15 mL), dried over anhydrous $Na_2SO_4$, and concentrated under reduced pressure to give a white solid (470 mg, 97%).
MS (ESI, pos.ion) m/z: 490.0 $[M+H]^+$.

**Step 2: Synthesis of *N*-((*S*)-2-cyano-1-(4-(ethylsulfonyl)phenyl)ethyl)-4-((2*S*,4*S*)-2 -((difluoromethoxy)methyl)-4-(4-iodophenoxy)pyrrolidin-1-yl)benzamide**

HATU (474 mg, 1.25 mmol), (*S*)-3-amino-3-(4-(ethylsulfonyl)phenyl)propionitrile hydrochloride (320 mg, 1.16 mmol), 4-((2*S*,4*S*)-2-((difluoromethoxy)methyl)-4-(4-iodophenoxy)pyrrolidin-1-yl)benzoic acid (470 mg, 0.96 mmol) and TEA (0.40 mL, 2.88 mmol) were successively added to DCM (6 mL), and the mixture was stirred at room temperature for 16 h. The reaction solution was concentrated under reduced pressure. The residue was diluted with DCM (40 mL), washed successively with HCl solution (15 mL, 1 mol/L), saturated $NaHCO_3$ solution (15 mL) and saturated NaCl solution (15 mL), dried over anhydrous $Na_2SO_4$, and concentrated under reduced pressure. The concentrated solution was separated by silica gel column chromatography (eluent: DCM/EtOAc (v/v) = 4/1) to give a pale yellow solid (630 mg, 92%). MS (ESI, pos.ion) m/z: 710.1 $[M+H]^+$.

**Step 3: Synthesis of *N*-((*S*)-2-cyano-1-(4-(ethylsulfonyl)phenyl)ethyl)-4-((2*S*,4*S*) -2-((difluoromethoxy)methyl)-4-(4-(4-fluoro-1*H*-pyrazol-1-yl)phenoxy)pyrrolidin-1-yl)benzam ide**

*N*-((*S*)-2-cyano-1-(4-(ethylsulfonyl)phenyl)ethyl)-4-((2*S*,4*S*)-2-((difluoromethoxy)met hyl)-4-(4-iodophenoxy)pyrrolidin-1-yl)benzamide (300 mg, 0.42 mmol), 4-fluoro-1*H*-pyrazole (54 mg, 0.63 mmol), CuI (16 mg, 0.20 mmol), (*S*)-2-methylproline methyl ester hydrochloride (24 mg, 0.21 mmol) and $K_2CO_3$ (120 mg, 0.84 mmol) were added to DMSO (4 mL) under nitrogen protection, and the mixture was reacted at 100 °C for 16 h. The reaction solution was cooled to room temperature, diluted with EtOAc (50 mL), washed with $H_2O$ (20 mL × 2) and saturated NaCl solution (20 mL), dried over anhydrous $Na_2SO_4$, and concentrated under reduced pressure. The concentrated solution was separated by silica gel column chromatography (eluent: DCM/EtOAc (v/v) = 2/1) to give a white solid (200 mg, 71%).

MS (ESI, pos.ion) m/z: 668.6 $[M+H]^+$.
$^1H$ NMR (400 MHz, $CDCl_3$) δ (ppm): 7.92 (d, *J* = 8.3 Hz, 2H), 7.76 - 7.71 (m, 3H), 7.65 (d, *J* = 8.2 Hz, 2H), 7.56 (d, *J* = 8.8 Hz, 3H), 6.97 (d, *J* = 8.9 Hz, 2H), 6.69 (d, *J* = 7.9 Hz, 1H), 6.65 (d, *J* = 8.7 Hz, 2H), 6.22 (t, *J* = 74.3 Hz, 1H), 5.60 (dd, *J* = 12.3, 6.0 Hz, 1H), 5.14 (t, *J* = 4.4 Hz, 1H), 4.23 - 4.14 (m, 2H), 3.99 (t, *J* = 9.3 Hz, 1H), 3.77 (d, *J* = 11.2 Hz, 1H), 3.68 (dd, *J* = 11.3, 4.6 Hz, 1H), 3.19 - 3.03 (m, 4H), 2.52 (d, *J* = 14.4 Hz, 1H), 2.41 - 2.32 (m, 1H), 1.28 (t, *J* = 7.4 Hz, 3H).

**Example 162 *N*-((*S*)-2-cyano-1-(4-(ethylsulfonyl)phenyl)ethyl)-4-((2*S*,4*S*)-2-((difluoromethoxy) methyl)-4-(4-(3-fluoro-1*H*-pyrazol-1-yl)phenoxy)pyrrolidin-1-yl)benzamide**

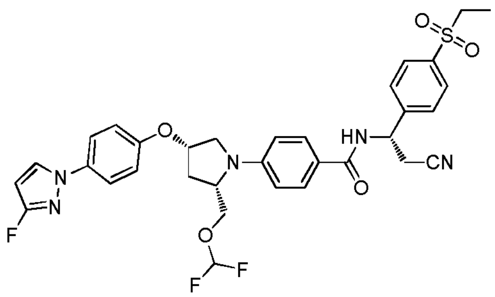

*N*-((*S*)-2-cyano-1-(4-(ethylsulfonyl)phenyl)ethyl)-4-((2*S*,4*S*)-2-((difluoromethoxy)met hyl)-4-(4-iodophenoxy)pyrrolidin-1-yl)benzamide (200 mg, 0.28 mmol), 3-fluoro-1*H*-pyrazole (50 mg, 0.58 mmol), CuI (22 mg, 0.28 mmol), (*S*)-2-methylproline methyl ester hydrochloride (32 mg, 0.28 mmol) and $K_2CO_3$ (80 mg, 0.56 mmol) were added to DMSO (4 mL) under nitrogen protection, and the mixture was reacted at 100 °C for 23 h. The reaction solution was cooled to room temperature, diluted with EtOAc (50 mL), washed with $H_2O$ (20 mL × 2) and saturated NaCl solution (20 mL), dried over anhydrous $Na_2SO_4$, and concentrated under reduced pressure. The concentrated solution was separated by silica gel column chromatography (eluent: DCM/EtOAc (v/v) = 3/1) to give a white solid (60 mg, 32%).

MS (ESI, pos.ion) m/z: 668.2 $[M+H]^+$.
$^1H$ NMR (400 MHz, $CDCl_3$) δ (ppm): 7.95 (d, J = 8.3 Hz, 2H), 7.73 (d, J = 8.7 Hz, 2H), 7.66 (d, J = 8.2 Hz, 3H), 7.54 (d, J = 9.0 Hz, 2H), 6.96 (d, J = 9.0 Hz, 2H), 6.66 (d, J = 8.8 Hz, 2H), 6.57 (d, J = 7.5 Hz, 1H), 6.22 (t, J = 74.3 Hz, 1H), 6.00 (dd, J = 5.8, 2.5 Hz, 1H), 5.60 (dd, J = 12.3, 6.3 Hz, 1H), 5.14 (t, J = 4.7 Hz, 1H), 4.24 - 4.15 (m, 2H), 3.99

(t, J = 9.4 Hz, 1H), 3.77 (d, J = 11.4 Hz, 1H), 3.68 (dd, J = 11.3, 4.8 Hz, 1H), 3.21 - 3.02 (m, 4H), 2.53 (d, J = 14.5 Hz, 1H), 2.41 - 2.33 (m, 1H), 1.29 (t, J = 7.4 Hz, 3H).

Example 163 N-((S)-2-cyano-1-(4-(ethylsulfonyl)phenyl)ethyl)-4-((2S,4S)-2-((difluoromethoxy)methyl)-4-(4-(3-methyl-1H pyrazol-1-yl)phenoxy)pyrrolidin-1-yl) benzamide

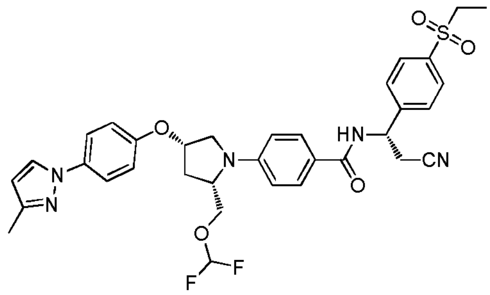

N-((S)-2-cyano-1-(4-(ethylsulfonyl)phenyl)ethyl)-4-((2S,4S)-2-((difluoromethoxy)met hyl)-4-(4-iodophenoxy)pyrrolidin-1-yl)benzamide (100 mg, 0.14 mmol), 3-methyl-1H-pyrazole (23 mg, 0.28 mmol), CuI (11 mg, 0.14 mmol), (S)-2-methylproline methyl ester hydrochloride (16 mg, 0.14 mmol) and $K_2CO_3$ (60 mg, 0.42 mmol) were added to DMSO (4 mL) under nitrogen protection, and the mixture was reacted at 100 °C for 23 h. The reaction solution was cooled to room temperature, diluted with EtOAc (50 mL), washed with $H_2O$ (20 mL × 2) and saturated NaCl solution (20 mL), dried over anhydrous $Na_2SO_4$, and concentrated under reduced pressure. The concentrated solution was separated by silica gel column chromatography (eluent: DCM/EtOAc (v/v) = 1/1) to give a pale yellow solid (12 mg, 13%).

MS (ESI, pos.ion) m/z: 664.3 $[M+H]^+$.
$^1$H NMR (400 MHz, $CDCl_3$) $\delta$ (ppm): 7.95 (d, J = 8.3 Hz, 2H), 7.73 (d, J = 8.7 Hz, 3H), 7.66 (d, J = 8.2 Hz, 2H), 7.58 (d, J = 8.9 Hz, 2H), 6.95 (d, J = 8.9 Hz, 2H), 6.66 (d, J = 8.7 Hz, 2H), 6.56 (d, J = 7.5 Hz, 1H), 6.23 (t, J = 74.4 Hz, 1H), 6.22 (s, 1H),5.60 (dd, J = 12.0, 6.4 Hz, 1H), 5.14 (t, J = 4.7 Hz, 1H), 4.23 - 4.15 (m, 2H), 4.00 (t, J = 10.1 Hz, 1H), 3.77 (d, J = 11.3 Hz, 1H), 3.67 (dd, J = 11.3, 4.8 Hz, 1H), 3.20 - 3.03 (m, 4H), 2.53 (d, J = 14.4 Hz, 1H), 2.37 (s, 4H), 1.30 (t, J = 7.4 Hz, 3H).

**Example 164 (R)-2-(6-((2S,4S)-4-((5-chloropyridin-2-yl)oxy)-2-((difluoromethoxy)methyl) pyrrolidin-1-yl)-5-fluoronicotinamido)-2-(4-(ethylsulfonyl)phenyl)ethyl carbamate**

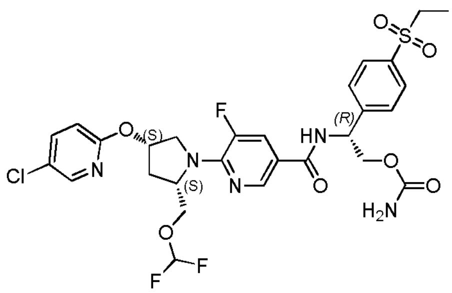

**Step 1: Synthesis of (2S,4R)-1-benzyl 2-methyl 4-((methylsulfonyl)oxy)pyrrolidine-1,2-dicarboxylate**

To a solution of (2S,4R)-1-benzyl 2-methyl 4-hydroxypyrrolidine-1,2-dicarboxylate (10.00 g, 34.73 mmol) in DCM (120 mL) were added MsCl (4.0 mL, 51.64 mmol), TEA (9.65 mL, 69.43 mmol) and DMAP (424 mg, 3.47 mmol) in turn. The mixture was stirred at room temperature for 21 h. The reaction solution was washed successively with $H_2O$ (80 mL × 2) and saturated NaCl solution (80 mL), dried over anhydrous $Na_2SO_4$, and concentrated under reduced pressure to give brown liquid (12.4 g, 100%).
MS (ESI, pos.ion) m/z: 358.6 $[M+H]^+$.

**Step 2: Synthesis of benzyl (2S,4R)-2-(hydroxymethyl)-4-((methylsulfonyl) oxy)pyrrolidine-1-carboxylate**

To a solution of (2S,4R)-1-benzyl 2-methyl 4-((methylsulfonyl)oxy)pyrrolidine -1,2-dicarboxylate (12.0 g , 33.58 mmol) in THF (100 mL) was added $LiBH_4$ (1.21 g, 55.56 mmol), and the mixture was reacted for 6 h at room temperature. Under vigorous stirring, the reaction solution was slowly poured into cooled saturated $NH_4Cl$ solution (100 mL) to quench the reaction. The mixture was concentrated under reduced pressure, and the remaining aqueous phase was extracted with EtOAc (80 mL × 2), washed with saturated NaCl solution (60 mL), dried over anhydrous $Na_2SO_4$, concentrated under reduced pressure to give pale yellow liquid (11.1 g, 100%).
MS (ESI, pos.ion) m/z: 330.0 $[M+H]^+$.

**Step 3: Synthesis of benzyl (2S,4R)-2-((difluoromethoxy)methyl) -4-((methylsulfonyl)oxy)pyrrolidin-1-carboxylate**

To a solution of benzyl (2S,4R)-2-(hydroxymethyl)-4-((methylsulfonyl)oxy)pyrrolidine -1-carboxylate (11.00 g, 33.39 mmol) in DCM (30 mL) was added a solution of KOAc (16.40 g, 167.11 mmol) in $H_2O$ (30 mL), then $TMSCF_2Br$ (16.0 mL, 102.89 mmol) was added under an ice-water bath, and the mixture was heated to room temperature and stirred for 14 h. The reaction solution was added with DCM (100 mL) and $H_2O$ (100 mL). The organic phase was separated and washed with saturated NaCl solution (60 mL), dried over anhydrous $Na_2SO_4$, concentrated under reduced pressure, and the crude product was separated by silica gel column chromatography (eluent: PE/EtOAc (v/v) = 1/1) to give pale yellow liquid (9.0 g, 71%).
MS (ESI, pos.ion) m/z: 380.1 $[M+H]^+$.

**Step 4: Synthesis of benzyl (2S,4S)-4-((5-chloropyridin-2-yl)oxy)-2-((difluoromethoxy) methyl)pyrrolidin-1-carboxylate**

Benzyl (2S,4R)-2-((difluoromethoxy)methyl)-4-((methylsulfonyl)oxy) pyrrolidin-1-carboxylate (2.00 g, 5.27 mmol), 5-chloropyridin-2-ol (820 mg, 6.33 mmol) and $K_2CO_3$ (1.46 g, 10.56 mmol) were added to DMF (20 mL), and the mixture was reacted at 60 °C for 7 h. The reaction solution was diluted with EtOAc (60 mL), washed with $H_2O$ (30 mL × 2) and saturated NaCl solution (30 mL), dried over anhydrous $Na_2SO_4$, and concentrated under reduced pressure. The concentrated solution was separated by silica gel column chromatography (eluent: PE/EtOAc (v/v) = 4/1) to give pale yellow liquid (1.06 g, 49%).
MS (ESI, pos.ion) m/z: 413.3 $[M+H]^+$.

**Step 5: Synthesis of 5-chloro-2-(((3S,5S)-5-((difluoromethoxy)methyl)pyrrolidin-3-yl)oxy)pyridine**

To a solution of benzyl (2S,4R)-4-((5-chloropyridin-2-yl)oxy)-2-((difluoromethoxy) methyl)pyrrolidine-1-carboxylate (1.00 g, 2.42 mmol) in DCM (20 mL) was added trimethylsilane (0.75 mL, 5.30 mmol). The mixture was stirred at room temperature for 2 h, then TEA (0.88 mL, 6.33 mmol) was added. The reaction solution was stirred for 15 min. The reaction solution was concentrated under reduced pressure, diluted with EtOAc (50 mL), washed with saturated $NaHCO_3$ solution (20 mL) and saturated NaCl solution (20 mL), dried over anhydrous $Na_2SO_4$, and concentrated under reduced pressure to give light brown liquid (620 mg, 92%).
MS (ESI, pos.ion) m/z: 279.3 $[M+H]^+$.

**Step 6: Synthesis of methyl 6-((2S,4S)-4-((5-chloropyridin-2-yl)oxy)-2-((difluoromethoxy) methyl)pyrrolidin-1-yl)5-fluoronicotinate**

5-Chloro-2-((3S,5S)-5-((difluoromethoxy)methyl)pyrrolidin-3-yl)oxy)pyridine (600 mg, 2.15 mmol), $K_2CO_3$ (500 mg, 3.62 mmol) and methyl 6-chloro-5-fluoronicotinate (490 mg, 2.58 mmol) were added to DMF (8 mL) in turn, and the mixture was reacted at 100 °C for 24 h. The reaction solution was diluted with EtOAc (50 mL), washed successively with $H_2O$ (20 mL × 2) and saturated NaCl solution (20 mL), dried over anhydrous $Na_2SO_4$, and concentrated under reduced pressure. The concentrated solution was separated by silica gel column chromatography (eluent: PE/EtOAc (v/v) = 4/1) to give pale yellow liquid (700 mg, 75%).
MS (ESI, pos.ion) m/z: 432.5 $[M+H]^+$.

**Step 7: Synthesis of 6-((2S,4S)-4-((5-chloropyridin-2-yl)oxy)-2-((difluoromethoxy) methyl)pyrrolidin-1-yl)5-fluoronicotinic acid**

To a solution of methyl 6-((2S,4S)-4-((5-chloropyridin-2-yl)oxy)-2-((difluoromethoxy) methyl)pyrrolidin-1-yl)5-fluoronicotinate (700 mg, 1.62 mmol) in MeOH (3 mL) and THF (3 mL) was added a solution of LiOH (310 mg, 12.94 mmol) in $H_2O$ (2 mL). The mixture was stirred at 50°C for 16 h. The reaction solution was concentrated under reduced pressure, and the remaining solution was added with HCl solution (1 mol/L) to adjust the pH of the solution to about 4. The resulting mixture was extracted with EtOAc (30 mL), washed with saturated NaCl (15 mL) solution, dried over anhydrous $Na_2SO_4$, and concentrated under reduced pressure. The concentrated solution was separated by silica gel column chromatography (eluent: DCM/MeOH (v/v) = 20/1) to give pale yellow liquid (600 mg, 89%).
MS (ESI, pos.ion) m/z: 418.5 $[M+H]^+$.

**Step 8: Synthesis of (R)-2-(6-((2S,4S)-4-((5-chloropyridin-2-yl)oxy)-2-((difluoromethoxy) methyl)pyrrolidin-1-yl)-5-fluoronicotinylamino)-2-(4-(ethylsulfonyl)phenyl)ethyl carbamate**

HATU (410 mg, 1.08 mmol), (R)-2-amino-2-(4-(ethylsulfonyl)phenyl)ethyl carbamate (215 mg, 0.79 mmol), 6-((2S,4S)-4-((5-chloropyridin-2-yl)oxy)-2-((difluoromethoxy)methyl)pyrrolidin-1-yl)-5-fluoronicotinic acid (300 mg, 0.72 mmol) and TEA (218 mg, 2.15 mmol) were successively added to DCM (8 mL), and the mixture was stirred at room temperature for 18 h. The reaction solution was concentrated under reduced pressure. The residue was diluted with DCM (40 mL), washed successively with HCl solution (15 mL, 1 mol/L), saturated $NaHCO_3$ solution (15 mL) and saturated NaCl solution (15 mL), dried over anhydrous $Na_2SO_4$, and concentrated under reduced pressure. The concentrated solution was separated by silica gel column chromatography (eluent: DCM/MeOH (v/v) = 20/1) to give a white solid(420 mg, 87%).

MS (ESI, pos.ion) m/z: 672.1 $[M+H]^+$.
$^1$H NMR (400 MHz, $CDCl_3$) $\delta$ (ppm): 8.45 (s, 1H), 8.10 (d, J = 2.2 Hz, 1H), 7.84 (t, J = 9.2 Hz, 3H), 7.65 (d, J = 13.9 Hz, 1H), 7.55 (d, J = 8.0 Hz, 3H), 6.70 (d, J = 8.7 Hz, 1H), 6.21 (t, J = 74.8 Hz, 1H), 5.65 (s, 1H), 5.38 (s, 1H), 4.97 (s, 2H), 4.73 (s, 1H), 4.55 (dd, *J*= 12.0, 8.0 Hz, 1H), 4.31 (dd, J = 12.1, 30 Hz, 1H), 4.24 (dd, J = 9.1, 4.0 Hz, 1H), 4.14 - 4.03 (m, 2H), 3.99 (t, J = 9.3 Hz, 1H), 3.09 (q, J = 7.4 Hz, 2H), 2.43 (d, J = 14.1 Hz, 1H), 2.39 - 2.31 (m, 1H), 1.27 (t, J = 7.4 Hz, 3H).

**Example 165 6-((2S,4S)-2-((difluoromethoxy)methyl)-4-(5-trifluoromethoxy)pyridin -2-yl)oxy)pyrrolidin-1-yl)-N-((R)-1-(4-(ethylsulfonyl)phenyl)-2-(3-methylureido)ethyl) nicotinamide**

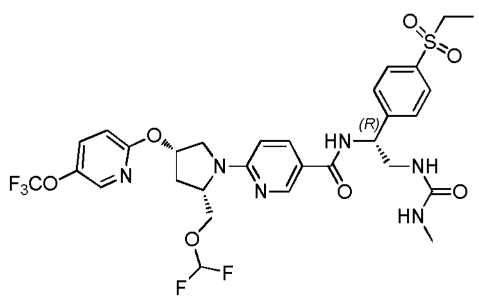

**Step 1: Synthesis of benzyl (R)-(1-(4-(ethylsulfonyl)phenyl)-2-(3-methylureido) ethyl)carbamate**

Benzyl (R)-(2-amino-1-(4-(ethylsulfonyl)phenyl)ethyl)carbamate (300 mg, 0.83 mmol), N-methyl-1H-imidazole-1-carboxamide (160 mg, 1.28 mmol) and TEA (167 mg, 1.65 mmol) were successively added to DCM (6 mL), and the mixture was reacted at room temperature for 24 h. The reaction solution was concentrated under reduced pressure, and the concentrated solution was separated by silica gel column chromatography (eluent: DCM/MeOH (v/v) = 10/1) to give a white solid (310 mg, 89%).
MS (ESI, pos.ion) m/z: 420.2 $[M+H]^+$.

**Step 2: Synthesis of (R)-1-(2-amino-2-(4-(ethylsulfonyl)phenyl)ethyl)-3-methylurea**

To a solution of benzyl (R)-(1-(4-(ethylsulfonyl)phenyl)-2-(3-methylureido) ethyl)carbamate (310 mg, 0.74 mmol) in MeOH (6 mL) was added Pd/C (40 mg, 10%) slowly. The mixture was degassed and refilled with hydrogen, and was reacted at room temperature for 24 h. The reaction solution was filtered through a celite pad, and the filtrate was concentrated under reduced pressure to give a white solid (200 mg, 95%).

MS (ESI, pos.ion) m/z: 286.2 [M+H]$^+$.

**Step 3: Synthesis of 6-((2S,4S)-2-((difluoromethoxy)methyl)-4-(5-trifluoromethoxy)pyridin -2-yl)oxy)pyrrolidin-1-yl)-N-((R)-1-(4-(ethylsulfonyl)phenyl)-2-(3-methylureido)ethyl)nicotina mide**

HATU (230 mg, 0.60 mmol), (R)-1-(2-amino-2-(4-(ethylsulfonyl)phenyl)ethyl) -3-methylurea (140 mg, 0.49 mmol), 6-((2S,4S)-2-((difluoromethoxy)methyl)-4-((5 -(trifluoromethoxy)pyridin-2-yl)oxy)pyrrolidin-1-yl)nicotinic acid (180 mg, 0.40 mmol) and TEA (121 mg, 1.20 mmol) were successively added to DCM (4 mL), and the mixture was reacted at room temperature for 18 h. The reaction solution was concentrated under reduced pressure. The residue was diluted with DCM (40 mL), washed successively with HCl solution (15 mL, 1 mol/L), saturated $NaHCO_3$ solution (15 mL) and saturated NaCl solution (15 mL), dried over anhydrous $Na_2SO_4$, and concentrated under reduced pressure. The concentrated solution was separated by silica gel column chromatography (eluent: DCM/MeOH (v/v) = 10/1) to give a white solid(220 mg, 77%).

MS (ESI, pos.ion) m/z: 717.2 [M+H]$^+$.
$^1$H NMR (400 MHz, $CDCl_3$) $\delta$ (ppm): 9.18 (d, J= 4.6 Hz, 1H), 8.74 *(d, J* = 1.9 Hz, 1H), 8.10 (d, J= 2.3 Hz, 1H), 7.98 (dd, J = 8.9, 2.2 Hz, 1H), 7.80 (d, J = 8.3 Hz, 2H), 7.49 (dd, J = 10.3, 5.4 Hz, 3H), 6.77 *(d, J* = 9.0 Hz, 1H), 6.44 (d, *J*= 8.5 Hz, 1H), 6.25 (t, *J*= 74.9 Hz, 1H), 5.72 (s, 1H), 5.47 (t, J = 5.9 Hz, 1H), 4.90 (d, J = 4.4 Hz, 2H), 4.53 (s, 1H), 4.32 (dd, J = 9.3, 4.2 Hz, 1H), 4.00 (t, J = 9.3 Hz, 1H), 3.86 (dd, J = 12.2, 4.9 Hz, 1H), 3.76 (d, J = 12.2 Hz, 1H), 3.61 (dd, J = 14.5, 2.7 Hz, 1H), 3.47- 3.35 (m, 1H), 3.07 (q, J = 7.3 Hz, 2H), 2.68 (d, J = 4.7 Hz, 3H), 2.49 (d, J = 14.2 Hz, 1H), 2.45 - 2.35 (m, 1H), 1.27 (t, *J*= 7.4 Hz, 3H).

**Example 166 6-((2S,4S)-2-((difluoromethoxy)methyl)-4-(5-trifluoromethoxy)pyridin-2-yl)oxy) pyrrolidin-1-yl)-N-((R)-1-(4-(ethylsulfonyl)phenyl)-2-ureidoethyl)nicotinamide**

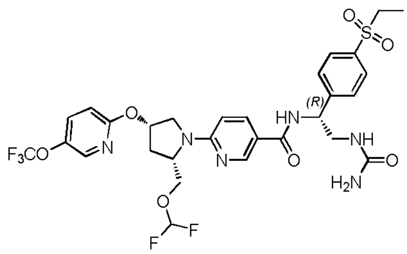

**Step 1: Synthesis of benzyl (R)-(1-(4-(ethylsulfonyl)phenyl)-2-hydroxyethyl)carbamate**

To a solution of (R)-2-amino-2-(4-(ethylsulfonyl)phenyl)ethanol (1.50 g, 6.54 mmol) in THF (15 mL) was added saturated $NaHCO_3$ solution (15 mL), then benzyl chloroformate (2.27 g, 13.33 mmol) was added slowly. The mixture was stirred at room temperature for 17 h. The reaction solution was concentrated under reduced pressure. The remaining solution was added with saturated $NaHCO_3$ solution (20 mL) and EtOAc (50 mL), and the liquid was extracted and separated. The organic phase was washed with saturated NaCl solution (20 mL), dried over anhydrous $Na_2SO_4$, concentrated under reduced pressure, and the concentrated solution was separated by silica gel column chromatography (eluent: DCM/EtOAc (v/v) = 3/2) to give a pale yellow solid (1.43 g, 60%).
MS (ESI, pos.ion) m/z: 364.2 [M+H]$^+$.

**Step 2: Synthesis of (R)-2-(((benzyloxy)carbonyl)amino)-2-(4-(ethylsulfonyl)phenyl)ethyl methanesulfonate**

To a solution of benzyl (R)-(1-(4-(ethylsulfonyl)phenyl)-2-hydroxyethyl)carbamate (1.80 g, 4.95 mmol) in DCM (16 mL) were added MsCl (0.55 mL, 7.11 mmol), TEA (1.40 mL, 10.07 mmol) and DMAP (61 mg, 0.50 mmol) in turn. The mixture was stirred at room temperature for 3 h. The reaction solution was concentrated under reduced pressure. The concentrated solution was diluted with DCM (50 mL), washed successively with HCl solution (20 mL, 0.5 mol/L), saturated $NaHCO_3$ solution (20 mL) and saturated NaCl solution (20 mL), dried over anhydrous $Na_2SO_4$, and concentrated under reduced pressure to give a pale yellow solid (2.16 g, 99%).
MS (ESI, pos.ion) m/z: 442.1 [M+H]$^+$.

**Step 3: Synthesis of benzyl (R)-(2-azido-1-(4-(ethylsulfonyl)phenyl)ethyl)carbamate**

To a solution of (R)-2-(((benzyloxy)carbonyl)amino)-2-(4-(ethylsulfonyl)phenyl)ethyl methanesulfonate (2.10 g, 4.76 mmol) in DMF (16 mL) was added $NaN_3$ (1.43 g, 22.00 mmol). The mixture was reacted at 80 °C for 12 h. The reaction solution was diluted with EtOAc (80 mL), washed with $H_2O$ (30 mL × 2) and saturated NaCl solution (30 mL), dried over anhydrous $Na_2SO_4$, and concentrated under reduced pressure. The concentrated solution was separated by silica gel column chromatography (eluent: PE/EtOAc (v/v) = 1/1) to give a white solid (1.40 g, 76%).
MS (ESI, pos.ion) m/z: 411.1 $[M+Na]^+$.

**Step 4: Synthesis of benzyl (R)-(2-amino-1-(4-(ethylsulfonyl)phenyl)ethyl)carbamate**

Benzyl (R)-(2-azido-1-(4-(ethylsulfonyl)phenyl)ethyl)carbamate (1.40 g, 3.60 mmol) and $PPh_3$ (1.90 g, 7.24 mmol) were added to THF/$H_2O$ (12 mL/4 mL), and the mixture was reacted at 70 °C for 24 h. The reaction solution was concentrated under reduced pressure, diluted with EtOAc (40 mL), washed with $H_2O$ (15 mL) and saturated NaCl solution (15 mL), dried over anhydrous $Na_2SO_4$, and concentrated under reduced pressure. The concentrated solution was separated by silica gel column chromatography (eluent: DCM/MeOH (v/v) = 10/1) to give a white solid (1.20 mg, 92%).
MS (ESI, pos.ion) m/z: 363.1 $[M+H]^+$.

**Step 5: Synthesis of benzyl (R)-(1-(4-(ethylsulfonyl)phenyl)-2-ureidoethyl)carbamate**

Benzyl (R)-(2-amino-1-(4-(ethylsulfonyl)phenyl)ethyl)carbamate (200 mg, 0.55 mmol), phenyl carbamate (80 mg, 0.57 mmol) and TEA (111 mg, 1.10 mmol) were successively added to THF (4 mL), and the mixture was reacted at room temperature for 11 h under nitrogen protection. The reaction solution was concentrated under reduced pressure, and the concentrated solution was separated by silica gel column chromatography (eluent: DCM/MeOH (v/v) = 10/1) to give a white solid (220 mg, 98%).
MS (ESI, pos.ion) m/z: 406.2 $[M+H]^+$.

**Step 6: Synthesis of (R)-1-(2-amino-2-(4-(ethylsulfonyl)phenyl)ethyl)urea**

To a solution of benzyl (R)-(1-(4-(ethylsulfonyl)phenyl)-2-ureidoethyl)carbamate (220 mg, 0.54 mmol) in MeOH (6 mL) was added Pd/C (40 mg, 10%) slowly. The mixture was degassed and refilled with hydrogen, and was reacted at room temperature for 24 h. The reaction solution was filtered through a celite pad, and the filtrate was concentrated under reduced pressure to give a white solid (147 mg, 100%).
MS (ESI, pos.ion) m/z: 272.2 $[M+H]^+$.

**Step 7: Synthesis of 6-((2S,4S)-2-((difluoromethoxy)methyl)-4-(5-trifluoromethoxy) pyridin-2-yl)oxy)pyrrolidin-1-yl)-N-((R)-1-(4-(ethylsulfonyl)phenyl)-2-ureidoethyl)nicotinami de**

HATU (230 mg, 0.60 mmol), (R)-1-(2-amino-2-(4-(ethylsulfonyl)phenyl)ethyl)urea (140 mg, 0.46 mmol), 6-((2S,4S)-2-((difluoromethoxy)methyl)-4-((5-(trifluoromethoxy)pyridin-2-yl)oxy)pyrrolidin -1-yl)nicotinic acid (180 mg , 0.40 mmol) and TEA (121 mg, 1.20 mmol) were successively added to DCM (4 mL), and the mixture was reacted at room temperature for 18 h. The reaction solution was concentrated under reduced pressure. The residue was diluted with DCM (40 mL), washed successively with HCl solution (15 mL, 0.5 mol/L), saturated $NaHCO_3$ solution (15 mL) and saturated NaCl solution (15 mL), dried over anhydrous $Na_2SO_4$, and concentrated under reduced pressure. The concentrated solution was separated by silica gel column chromatography (eluent: DCM/MeOH (v/v) = 10/1) to give a white solid(180 mg, 64%). MS (ESI, pos.ion) m/z: 703.3 $[M+H]^+$.
$^1H$ NMR (400 MHz, $CDCl_3$) δ (ppm): 9.01 (d, J = 4.2 Hz, 1H), 8.71 (s, 1H), 8.10 (d, J = 2.4 Hz, 1H), 7.94 (dd, J = 8.9, 1.9 Hz, 1H), 7.80 (d, J = 8.2 Hz, 2H), 7.49 (d, J = 8.1 Hz, 3H), 6.77 (d, J = 9.0 Hz, 1H), 6.42 (d, J = 8.5 Hz, 1H), 6.24 (, J = 74.9 Hz, 1H), 5.73 (d, J = 5.0 Hz, 2H), 4.97 (s, 1H), 4.77 (s, 2H), 4.52 (s, 1H), 4.30 (dd, J = 9.2, 4.1 Hz, 1H), 3.99 (t, J = 9.4 Hz, 1H), 3.85 (dd, J = 12.2, 4.8 Hz, 1H), 3.75 (d, J = 12.2 Hz, 1H), 3.62 (d, J = 17.3 Hz, 1H), 3.49 - 3.39 (m, 1H), 3.08 (q, J = 7.4 Hz, 2H), 2.49 (d, J = 14.3 Hz, 1H), 2.44 - 2.36 (m, 1H), 1.27 (t, J = 7.3 Hz, 3H).

**Example 167 (R)-2-(4-((2S,4S)-4-((5-(1H-pyrazol-1-yl)pyridine-2-yl)oxy)-2-((difluoromethoxy)methyl)pyrrolidin-1-yl)benzoylamino)-2-(4-(ethylsulfonyl)benzyl)ethyl carbamate**

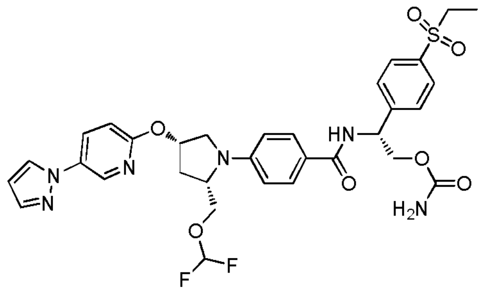

**Step 1: Synthesis of benzyl (R)-(2-(carbamoyloxy)-1-(4-(ethylsulfonyl)phenyl)ethyl)carbamate**

To a suspension of benzyl (R)-(1-(4-(ethylsulfonyl)phenyl)-2-hydroxyethyl)carbamate (2.00 mg, 5.50 mmol) in ACN (20 mL) was added chlorosulfonyl isocyanate (1.0 mL, 11.49 mmol) at -15°C. After 10 min, $H_2O$ (20 mL) was added, and the mixture was moved to 60°C and reacted for 5 h. The reaction solution was adjusted to pH 9 by adding saturated $NaHCO_3$ solution. The mixture was concentrated under reduced pressure, and the remaining aqueous phase was extracted with EtOAc (40 mL × 2). The organic phase was washed with saturated NaCl solution (30 mL), dried over anhydrous $Na_2SO_4$, and concentrated under reduced pressure to give pale yellow liquid (2.24 g, 100%).
MS (ESI, pos.ion) m/z: 407.1 $[M+H]^+$.

**Step 2: Synthesis of (R)-2-amino-2-(4-(ethylsulfonyl)phenyl)ethyl carbamate**

To a solution of benzyl (R)-(2-(carbamoyloxy)-1-(4-(ethylsulfonyl) phenyl)ethyl)carbamate (2.24 g, 5.51 mmol) in MeOH (16 mL) was added Pd/C (200 mg, 10%). The mixture was degassed and refilled with hydrogen, and was stirred at room temperature for 16 h. The reaction solution was filtered through a celite pad, and the filtrate was concentrated under reduced pressure to give a white solid (1.50 g, 100%).
MS (ESI, pos.ion) m/z: 273.2 $[M+H]^+$.

**Step 3: Synthesis of (R)-2-(4-((2S,4S)-4-((5-(1H-pyrazol-1-yl)pyridine-2-yl)oxy)-2-((difluoromethoxy)methyl)pyrrolidin-1-yl)benzoylamino)-2-(4-(ethylsulfonyl)benzyl)ethyl carbamate**

HATU (265 mg, 0.70 mmol), (R)-2-amino-2-(4-(ethylsulfonyl)phenyl)ethyl carbamate (151 mg, 0.55 mmol), 4-((2S,4S)-4-((5-(1H-pyrazol-1-yl)pyridin-2-yl) oxy)-2-((difluoromethoxy)methyl)pyrrolidin -1-yl)benzoic acid (200 mg, 0.46 mmol) and TEA (141 mg, 1.39 mmol) were successively added to DCM (6 mL), and the mixture was stirred at room temperature for 17 h. The reaction solution was concentrated under reduced pressure. The residue was diluted with DCM (40 mL), washed successively with HCl solution (15 mL, 1 mol/L), saturated $NaHCO_3$ solution (15 mL) and saturated NaCl solution (15 mL), dried over anhydrous $Na_2SO_4$, and concentrated under reduced pressure. The concentrated solution was separated by silica gel column chromatography (eluent: DCM/MeOH (v/v) = 20/1) to give a white solid (290 mg, 91%).

MS (ESI, pos.ion) m/z: 685.1 $[M+H]^+$.
$^1H$ NMR (400 MHz, $CDCl_3$) $\delta$ (ppm): 8.48 (d, *J* = 2.6 Hz, 1H), 7.96 (dd, J = 8.8, 2.7 Hz, 1H), 7.84 (dd, J = 5.4, 2.7 Hz, 3H), 7.77 (d, J = 8.7 Hz, 2H), 7.74 (d, J = 1.3 Hz, 1H), 7.63 (d, J = 6.3 Hz, 1H), 7.55 (d, J = 8.2 Hz, 2H), 6.84 (d, J = 8.8 Hz, 1H), 6.65 (d, J = 8.8 Hz, 2H), 6.49 (t, J = 2.0 Hz, 1H), 6.24 (t, J = 74.4 Hz, 1H), 5.80 (s, 1H), 5.40 (s, 1H), 4.90 (s, 2H), 4.53 (dd, J = 12.0, 8.0 Hz, 1H), 4.32 (dd, J = 12.1, 3.4 Hz, 1H), 4.22 - 4.15 (m, 2H), 4.00 (t, J = 10.6 Hz, 1H), 3.73 (s, 2H), 3.07 (q, J = 7.4 Hz, 2H), 2.49 *(d, J* = 14.4 Hz, 1H), 2.45 - 2.36 (m, 1H), 1.26 (t, *J*= 7.4 Hz, 3H).

**Example 168 (R)-2-(6-((2S,4S)-4-((5-(1H-pyrazol-1-yl)pyridine-2-yl)oxy)-2-((difluoromethoxy)methyl) pyrrolidin-1-yl)nicotinylamino)-2-(4-(ethylsulfonyl)phenyl)ethyl carbamate**

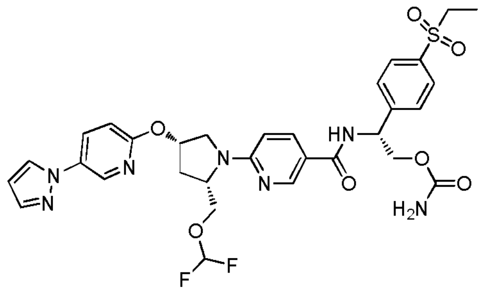

**Step 1: Synthesis of *tert-butyl* (2S,4R)-2-((difluoromethoxy)methyl)-4-((methylsulfonyl)oxy)pyrrolidin-1-carboxylate**

To a solution of benzyl (2S,4R)-2-((difluoromethoxy)methyl)-4-((methylsulfonyl) oxy)pyrrolidine-1-carboxylate (1.00 g, 2.64 mmol) and $(Boc)_2O$ (1.20 mL, 5.27 mmol) in EtOAc (6 mL) was added Pd/C (200 mg, 10%). The reaction mixture was degassed and refilled with hydrogen and stirred at room temperature for 16 h. The reaction solution was filtered through a celite pad, and the filtrate was concentrated under reduced pressure to give colorless liquid (910 mg, 100%).
MS (ESI, pos.ion) m/z: 290.4 $[M-56+H]^+$.

**Step 2: Synthesis of *tert-butyl* (2S,4S)-2-((difluoromethoxy)methyl)-4-((5-iodopyridin-2-yl) oxy)pyrrolidin-1-carboxylate**

tert-Butyl (2S,4R)-2-((difluoromethoxy)methyl)-4-((methylsulfonyl)oxy)pyrrolidin-1-carboxylate (900 mg, 2.61 mmol), 2-hydroxy-5-iodopyridine (691 mg, 3.13 mmol) and $K_2CO_3$ (720 mg, 5.21 mmol) were added to DMF (8 mL), and the mixture was reacted at 100 °C for 14 h. The reaction solution was cooled to room temperature, diluted with EtOAc (50 mL), washed with $H_2O$ (20 mL × 2) and saturated NaCl solution (20 mL), dried over anhydrous $Na_2SO_4$, and concentrated under reduced pressure. The concentrated solution was separated by silica gel column chromatography (eluent: PE/EtOAc (v/v) = 4/1) to give pale yellow liquid (877 mg, 72%).
MS (ESI, pos.ion) m/z: 415.0 $[M-56+H]^+$.

**Step 3: Synthesis of *tert-butyl* (2S,4S)-4-(5-(1H-pyrazol-1-yl)pyridin-2-yl)oxy)-2-((difluoromethoxy)methyl)pyrrolidin-1-carboxylate**

tert-Butyl (2S,4S)-2-((difluoromethoxy)methyl)-4-((5-iodopyridin-2-yl)oxy) pyrrolidin-1-carboxylate (850 mg, 1.81 mmol), 1H-pyrazole (135 mg, 1.99 mmol), CuI (28 mg, 0.35 mmol), (S)-2-methylproline methyl ester hydrochloride (41 mg, 0.36 mmol) and $K_2CO_3$ (386 mg, 2.71 mmol) were added to DMSO (6 mL) under nitrogen protection, and the mixture was reacted at 100 °C for 16 h. The reaction solution was cooled to room temperature, diluted with EtOAc (40 mL), washed with $H_2O$ (20 mL × 2) and saturated NaCl solution (20 mL), dried over anhydrous $Na_2SO_4$, and concentrated under reduced pressure. The concentrated solution was separated by silica gel column chromatography (eluent: PE/EtOAc (v/v) = 2/1) to give colorless liquid (466 mg, 63%).
MS (ESI, pos.ion) m/z: 355.1 $[M-56+H]^+$.

**Step 4: Synthesis of 2-(((3S,5S)-5-((difluoromethoxy)methyl)pyrrolidin -3-yl)oxy)-5-(1H-pyrazol-1-yl)pyridine**

To a solution of *tert-butyl* (2S,4S)-4-((5-(1H-pyrazol-1-yl)pyridin-2-yl)oxy)-2 -((difluoromethoxy)methyl)pyrrolidine-1-carboxylate (460 mg, 1.12 mmol) in DCM (3 mL) was added a solution of HCl in 1,4-dioxane (2.0 mL, 4 mol/L). The mixture was stirred at room temperature for 16 h. The reaction solution was concentrated under reduced pressure to give a white solid (347 mg, 100%).
MS (ESI, pos.ion) m/z: 311.1 $[M+H]^+$.

**Step 5: Synthesis of methyl 6-((2S,4S)-4-(5-(1H-pyrazol-1-yl)pyridin-2-yl)oxy) -2-((difluoromethoxy)methyl)pyrrolidin-1-yl)nicotinate**

2-(((3S,5S)-5-((difluoromethoxy)methyl)pyrrolidin-3-yl)oxy)-5-(1H-pyrazol-1-yl)pyri dine (340 mg, 1.10 mmol), methyl 6-fluoronicotinate (204 mg, 1.32 mmol) and $K_2CO_3$ (302 mg, 2.19 mmol) were added to DMF (8 mL), and the mixture was reacted at 100 °C for 24 h. The reaction solution was cooled to room temperature, diluted with EtOAc (50

mL), washed with $H_2O$ (20 mL × 2) and saturated NaCl solution (20 mL), dried over anhydrous $Na_2SO_4$, and concentrated under reduced pressure. The concentrated solution was separated by silica gel column chromatography (eluent: PE/EtOAc (v/v) = 2/1) to give pale yellow liquid (240 mg, 49%).
MS (ESI, pos.ion) m/z: 446.1 $[M+H]^+$.

**Step 6: Synthesis of 6-((2S,4S)-4-(5-(1H-pyrazol-1-yl)pyridin-2-yl)oxy)-2-((difluoromethoxy) methyl)pyrrolidin-1-yl)nicotinic acid**

To a solution of methyl 6-((2S,4S)-4-(5-(1H-pyrazol-1-yl)pyridin-2-yl)oxy)-2-((difluoromethoxy)methyl)pyrrolidin-1-yl)nicotinate (240 mg, 0.54 mmol) in MeOH (2 mL) and THF (2 mL) was added a solution of LiOH (100 mg, 4.18 mmol) in $H_2O$ (1 mL). The mixture was reacted at 60°C for 3 h. The reaction solution was concentrated under reduced pressure, and the remaining liquid was added with concentrated HCl solution (1 mol/L) to adjust the pH to 4. The resulting mixture was extracted with EtOAc (30 mL), washed with saturated NaCl solution (15 mL), dried over anhydrous $Na_2SO_4$, and concentrated under reduced pressure to give a white solid (207 mg, 89%).
MS (ESI, pos.ion) m/z: 432.1 $[M+H]^+$.

**Step 7: Synthesis of (R)-2-(6-((2S,4S)-4-((5-(1H-pyrazol-1-yl)pyridine-2-yl) oxy)-2-((difluoromethoxy)methyl)pyrrolidin-1-yl)nicotinylamino)-2-(4-(ethylsulfonyl)phenyl) ethyl carbamate**

HATU (264 mg, 0.69 mmol), (R)-2-amino-2-(4-(ethylsulfonyl)phenyl)ethyl carbamate (151 mg, 0.55 mmol), 6-((2S,4S)-4-((5-(1H-pyrazol-1-yl)pyridin-2-yl)oxy)-2-((difluoromethoxy)methyl)pyrrolidin -1-yl)nicotinic acid (200 mg, 0.46 mmol) and TEA (140 mg, 1.38 mmol) were successively added to DCM (6 mL), and the mixture was stirred at room temperature for 21 h. The reaction solution was concentrated under reduced pressure. The residue was diluted with DCM (40 mL), washed successively with HCl solution (15 mL, 1 mol/L), saturated $NaHCO_3$ solution (15 mL) and saturated NaCl solution (15 mL), dried over anhydrous $Na_2SO_4$, and concentrated under reduced pressure. The concentrated solution was separated by silica gel column chromatography (eluent: DCM/MeOH (v/v) = 20/1) to give a white solid(270 mg, 85%).

MS (ESI, pos.ion) m/z: 686.2 $[M+H]^+$.
$^1H$ NMR (400 MHz, $CDCl_3$) δ (ppm): 8.69 (s, 1H), 8.46 (d, J = 2.4 Hz, 1H), 7.98 - 7.90 (m, 2H), 7.85 (d, J = 8.2 Hz, 3H), 7.73 (d, J = 5.6 Hz, 2H), 7.56 (d, J = 8.2 Hz, 2H), 6.84 (d, J = 8.9 Hz, 1H), 6.48 (s, 1H), 6.44 (d, J = 3.8 Hz, 1H), 6.24 (t, J = 74.8 Hz, 1H), 5.78 (s, 1H), 5.40 (s, 1H), 4.98 (s, 2H), 4.54 (dd, J = 11.8, 7.8 Hz, 2H), 4.35 - 4.29 (m, 2H), 4.01 (t, J = 9.3 Hz, 1H), 3.87 (dd, J = 12.2, 4.8 Hz, 1H), 3.78 (d, J = 12.1 Hz, 1H), 3.08 (q, J = 7.4 Hz, 2H), 2.52 (d, J = 14.3 Hz, 1H), 2.45 - 2.36 (m, 1H), 1.27 *(t, J* = 7.4 Hz, 3H).

**Example 169 N-(Dideuterium(4-(ethylsulfonyl)phenyl)methyl)-4-((2S,4S)-2-((difluoromethoxy)methyl)-4-((5-(trifluoromethoxy)pyridin-2-yl)oxy)pyrrolidin-1-yl) benzamide**

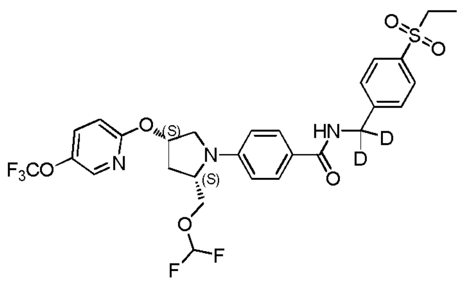

**Step 1: Synthesis of 4-(ethylsulfonyl) benzonitrile**

Sodium ethylsulfinate (1.52 g, 13.09 mmol), 4-iodobenzonitrile (1.00 g, 4.37 mmol), CuI (83 mg, 0.44 mmol), $K_3PO_4$ (1.02 g, 4.81 mmol) and (2S,4R)-4-hydroxy-N-m-methylpyrrolidine-2-carboxamide (108 mg, 0.44 mmol) were successively added to anhydrous DMSO (10 mL) under nitrogen protection, and the mixture was reacted at 100 °C for 22 h. The reaction solution was diluted with EtOAc (60 mL), and the insoluble solids were removed by filtration. The filtrate was washed successively with $H_2O$ (30 mL × 2) and saturated NaCl solution (30 mL), dried over anhydrous $Na_2SO_4$, and concentrated under reduced pressure. The crude product was separated by silica gel column chromatography (eluent: PE/EtOAc (v/v) = 3/1) to give a white solid (700 mg, 82%).
MS (ESI, pos.ion) m/z: 196.1 $[M+H]^+$.

**Step 2: Synthesis of dideuterium (4-(ethylsulfonyl)phenyl)methanamine**

To a solution of 4-(ethylsulfonyl)benzonitrile (280 mg, 1.43 mmol) in THF (8 mL) was added $LiAlD_4$ (120 mg, 2.86 mmol). The mixture was reacted at 45 °C for 18 h. NaOH solution (10 mL, 1 M) was added to the reaction solution to quench the reaction. The reaction solution was concentrated under reduced pressure, extracted with EtOAc (40 mL), washed with saturated NaCl solution (20 mL), dried over anhydrous $Na_2SO_4$, and concentrated under reduced pressure. The crude product was separated by silica gel column chromatography (eluent: DCM/MeOH (v/v) = 10/1) to give yellow liquid (58 mg, 20%).
MS (ESI, pos.ion) m/z: 202.2 $[M+H]^+$.

**Step 3: Synthesis of N-(Dideuterium(4-(ethylsulfonyl)phenyl)methyl) -4-((2S,4S)-2-((difluoromethoxy)methyl)-4-((5-(trifluoromethoxy)pyridin-2-yl)oxy) pyrrolidin-1-yl)benzamide**

HATU (128 mg, 0.33 mmol), dideuterium(4-(ethylsulfonyl)phenyl)methanamine (58 mg, 0.29 mmol), 4-((2S,4S)-2-((difluoromethoxy)methyl)-4-((5-(trifluoromethoxy) pyridin-2-yl)oxy)pyrrolidin-1-yl)benzoic acid (100 mg , 0.22 mmol) and TEA (45 mg, 0.44 mmol) were successively added to DCM (4 mL), and the mixture was reacted at room temperature for 23 h. The reaction solution was concentrated under reduced pressure. The residue was diluted with DCM (40 mL), washed successively with HCl solution (15 mL, 1 mol/L), saturated $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/EtOAc (v/v) = 4/1) to give a white solid (50 mg, 35%).

MS (ESI, pos.ion) m/z: 632.1 $[M+H]^+$.
$^1H$ NMR (400 MHz, $CDCl_3$) $\delta$ (ppm): 8.11 (s, 1H), 7.80 (d, J = 6.9 Hz, 2H), 7.75 (d, J = 8.6 Hz, 2H), 7.50 (d, J = 7.6 Hz, 3H), 6.77 (d, J = 8.9 Hz, 1H), 6.65 (d, J = 8.5 Hz, 3H), 6.24 (t, J = 74.4 Hz, 1H), 5.75 (s, 1H), 4.24 - 4.12 (m, 2H), 3.98 (t, J = 9.3 Hz, 1H), 3.72 (d, J = 2.2 Hz, 2H), 3.08 (q, *J*= 7.0 Hz, 2H), 2.49 - 2.36 (m, 2H), 1.25 (t, *J*= 7.0 Hz, 3H).

**Example 170 (R)-2-(4-((2S,4S)-4-((5-(2H-1,2,3-triazol-2-yl)pyridine-2-yl)oxy)-2 -((difluoromethoxy)methyl)pyrrolidin-1-yl)benzoylamino)-2-(4-(ethylsulfonyl)phenyl)ethyl carbamate**

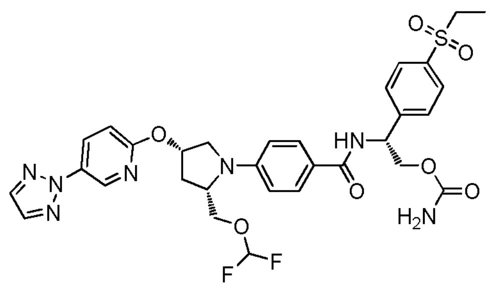

**Step 1: Synthesis of 5-(1H-1,2,3-triazol-2-yl)pyridin-2-ol**

5-Iodopyridin-2-ol (2.00 g, 9.10 mmol), 2H-triazole (0.94 g, 14.00 mmol), CuO (0.072 g, 0.91 mmol), iron triacetylacetonate (0.96 g, 2.70 mmol) and $Cs_2CO_3$ (5.90 g, 18.00 mmol) were dissolved in DMF (20 mL), and the mixture was heated to 90°C and reacted for 20 h. The reaction solution was cooled to room temperature, filtered, and concentrated under reduced pressure. The concentrated solution was separated by silica gel column chromatography (eluent: PE/EtOAc (v/v) = 5/1) to give a white solid (500 mg, 34%).
MS (ESI, pos.ion) m/z: 162.08 $[M+1]^+$.

**Step 2: Synthesis of methyl 4-((2S,4S)-4-(5-(2H-1,2,3-triazol-2-yl)pyridin-2-yl)oxy) -2-((difluoromethoxy)methyl)pyrrolidin-1-yl)benzoate**

5-(2H-1,2,3-Triazol-2-yl)pyridin-2-ol (66 mg, 0.41 mmol), $K_2CO_3$ (100 mg, 0.70 mmol) and methyl 4-((2S,4R)-2-((difluoromethoxy)methyl)-4-((methylsulfonyl)oxy) pyrrolidin-1-yl)benzoate (130 mg, 0.34 mmol) were dissolved in DMF

(10 mL) solution. The mixture was reacted at 60°C for 5 h. The resulting mixture was washed with saturated $NH_4Cl$ solution, and extracted with EtOAc (10 mL × 3). The organic phases were combined, dried over anhydrous $Na_2SO_4$, filtered, concentrated and separated by silica gel column chromatography (eluent: PE/EtOAc (v/v) = 5/1) to give a white solid (80 mg, 50%).
MS: (ESI, pos.ion) m/z: 445.2 $[M+1]^+$.

**Step 3: Synthesis of 4-((2S,4S)-4-((5-(2H-1,2,3-triazol-2-yl)pyridin-2-yl)oxy)-2-((difluoromethoxy)methyl)pyrrolidin-1-yl)benzoic acid**

To a solution of methyl 4-((2S,4S)-4-(5-(2H-1,2,3-triazol-2-yl)pyridin-2-yl)oxy) -2-((difluoromethoxy)methyl)pyrrolidin-1-yl)benzoate (25 mg, 0.056 mmol) in MeOH (5 mL) was added LiOH (7.2 mg, 0.30 mmol). The mixture was stirred at room temperature for 24 h. Dilute hydrochloric acid was added to adjust the pH of the solution to be weakly acidic. The resulting mixture was extracted with EtOAc (10 mL × 3). The organic phases were combined, dried over anhydrous $Na_2SO_4$, filtered, concentrated and separated by silica gel column chromatography (eluent: DCM/EtOAc (v/v) = 5/1) to give a white solid (23 mg, 94%).
MS: (ESI, pos.ion) m/z: 431.5 $[M+1]^+$.

**Step 4: Synthesis of (R)-2-(4-((2S,4S)-4-((5-(2H-1,2,3-triazol-2-yl)pyridine-2-yl)oxy)-2-((difluoromethoxy)methyl)pyrrolidin-1-yl)benzoylamino)-2-(4-(ethylsulfonyl)phenyl)ethyl carbamate**

4-((2S,4S)-4-((5-(2H-1,2,3-Triazol-2-yl)pyridin-2-yl)oxy)-2-((difluoromethoxy)methyl )pyrrolidin-1-yl)benzoic acid (100 mg, 0.23 mmol), (R)-2-amino-2-(4-(ethylsulfonyl)phenyl)ethyl carbamate (65 mg, 0.27 mmol) and HATU (107 mg, 0.28 mmol) were dissolved in DCM (25 mL), then TEA (0.069 mL, 0.53 mmol) was added. The mixture was stirred at room temperature for 24 h. The resulting mixture was washed with saturated $NH_4Cl$ solution, extracted with DCM (10 mL × 3). The organic phases were combined, dried over anhydrous $Na_2SO_4$, filtered, concentrated and separated by silica gel column chromatography (eluent: DCM/EtOAc (v/v) = 5/1) to give a white solid (82 mg, 54%).

MS: (ESI, pos.ion) m/z: 686.5 $[M+H]^+$.
$^1H$ NMR (400 MHz, $CDCl_3$) δ (ppm): 8.90 (d, *J* = 2.3 Hz, 1H), 8.30 (dd, J = 8.9, 2.5 Hz, 1H), 7.85 (d, J = 9.4 Hz, 4H), 7.78 (d, J = 8.6 Hz, 2H), 7.70 (d, J = 6.4 Hz, 1H), 7.60 (d, J = 8.1 Hz, 2H), 6.88 (d, J = 8.9 Hz, 1H), 6.68 (d, J = 8.7 Hz, 2H), 6.27 (t, *J*= 74.5 Hz, 1H), 5.84 (s, 1H), 5.42 (d, J = 2.5 Hz, 1H), 5.02 (s, 2H), 4.53 (dd, *J*= 11.8, 7.6 Hz, 1H), 4.35 (dd, *J*= 11.9, 3.2 Hz, 1H), 4.26 - 4.14 (m, 2H), 4.02 (t, J = 9.5 Hz, 1H), 3.76 (s, 2H), 3.10 (dd, J = 14.9, 7.4 Hz, 2H), 2.52 (d, J = 14.3 Hz, 1H), 2.48 - 2.39 (m, 1H), 1.30 (d, *J*= 7.3 Hz, 3H).

**Example 171 6-((2S,4S)-4-((5-chloropyridin-2-yl)oxy)-2-((difluoromethoxy)methyl) pyrrolidin-1-yl) -N-((R)-1-(4-(ethylsulfonyl)phenyl)-2-ureidoethyl)nicotinamide**

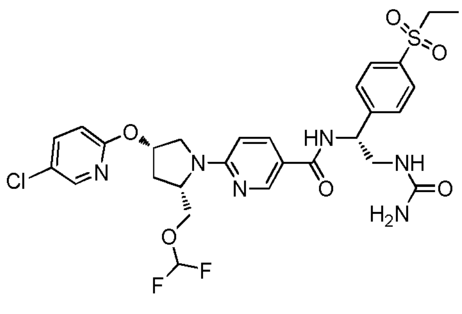

**Step 1: Synthesis of methyl 6-((2S,4S)-4-((5-chloropyridin-2-yl)oxy)-2-((difluoromethoxy) methyl)pyrrolidin-1-yl)nicotinate**

5-Chloro-2-((3S,5S)-5-((difluoromethoxy)methyl)pyrrolidin-3-yl)oxy)pyridine (500 mg, 1.79 mmol), $K_2CO_3$ (743 mg, 5.38 mmol) and methyl 6-fluoronicotinate (370 mg, 2.39 mmol) were added to DMF (8 mL) in turn, and the mixture was reacted at 100 °C for 24 h. The reaction solution was diluted with EtOAc (50 mL), washed successively with $H_2O$ (20 mL × 2) and saturated NaCl solution (20 mL), dried over anhydrous $Na_2SO_4$, and concentrated under reduced pressure. The crude product was separated by silica gel column chromatography (eluent: PE/EtOAc (v/v) = 4/1) to give

pale yellow liquid (580 mg, 78%).
MS (ESI, pos.ion) m/z: 414.1 [M+H]+.

**Step 2: Synthesis of 6-((2S,4S)-4-((5-chloropyridin-2-yl)oxy)-2-((difluoromethoxy)methyl) pyrrolidin-1-yl)nicotinic acid**

To a solution of methyl 6-((2S,4S)-4-((5-chloropyridin-2-yl)oxy)-2-((difluoromethoxy) methyl)pyrrolidin-1-yl)nicotinate (580 mg, 1.40 mmol) in MeOH (3 mL) and THF (3 mL) was added a solution of LiOH (268 mg, 11.19 mmol) in $H_2O$ (2 mL). The mixture was stirred at 50°C for 2 h. The reaction solution was concentrated under reduced pressure, and the remaining solution was added with HCl solution (1 mol/L) to adjust the pH of the solution to about 4. The resulting mixture was extracted with EtOAc (30 mL), washed with saturated NaCl (15 mL) solution, dried over anhydrous $Na_2SO_4$, and concentrated under reduced pressure. The crude product was separated by silica gel column chromatography (eluent: DCM/MeOH (v/v) = 20/1) to give a white solid (430 mg, 77%).
MS (ESI, pos.ion) m/z: 400.6 [M+H]+.

**Step 3: Synthesis of 6-((2S,4S)-4-((5-chloropyridin-2-yl)oxy)-2-((difluoromethoxy)methyl) pyrrolidin-1-yl)-N-((R)-1-(4-(ethylsulfonyl)phenyl)-2-ureidoethyl)nicotinamide**

HATU (142 mg, 0.37 mmol), (R)-1-(2-amino-2-(4-(ethylsulfonyl)phenyl)ethyl)urea (190 mg, 0.33 mmol), 6-((2S,4S)-4-((5-chloropyridin-2-yl)oxy)-2-((difluoromethoxy)methyl)pyrrolidin-1-yl)nicotinic acid (100 mg, 0.29 mmol) and TEA (75 mg, 0.74 mmol) were successively added to DCM (4 mL), and the mixture was stirred at room temperature for 24 h. The reaction solution was concentrated under reduced pressure. The residue was diluted with DCM (40 mL), washed successively with HCl solution (15 mL, 1 mol/L), saturated $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 10/1) to give a white solid (120 mg, 73%).

MS (ESI, pos.ion) m/z: 653.2 [M+H]+.
$^1$H NMR (400 MHz, $CDCl_3$) δ (ppm): 9.00 (d, *J*= 4.0 Hz, 1H), 8.70 (d, *J*= 1.7 Hz, 1H), 8.10 (d, *J*= 2.3 Hz, 1H), 7.92 (dd, J = 8.8, 2.1 Hz, 1H), 7.80 (d, J = 8.3 Hz, 2H), 7.55 (dd, J = 8.8, 2.6 Hz, 1H), 7.48 (d, J = 8.2 Hz, 2H), 6.70 (d, J = 8.8 Hz, 1H), 6.41 (d, J = 9.2 Hz, 1H), 6.24 (t, J = 74.9 Hz, 1H), 5.78 (t, J = 5.9 Hz, 1H), 5.69 (s, 1H), 4.95 (s, 1H), 4.79 (s, 2H), 4.50 (s, 1H), 4.30 (dd, J = 9.3, 4.2 Hz, 1H), 3.98 (t, J = 9.4 Hz, 1H), 3.83 (dd, J = 12.2, 4.8 Hz, 1H), 3.73 (d, J = 12.1 Hz, 1H), 3.61 (d, *J*= 14.5 Hz, 1H), 3.48 - 3.39 (m, 1H), 3.07 (q, *J*= 7.4 Hz, 2H), 2.47 (d, *J*= 14.2 Hz, 1H), 2.43 - 2.34 (m, 1H), 1.26 (t, J = 7.4 Hz, 3H).

**Example 172 (S)-3-(4-((2S,4S)-4-((5-(1H-pyrazol-1-yl)pyridine-2-yl)oxy)-2-((difluoromethoxy)methyl)pyrrolidin-1-yl)benzamido)-3-(4-(ethylsulfonyl)phenyl)propionic acid**

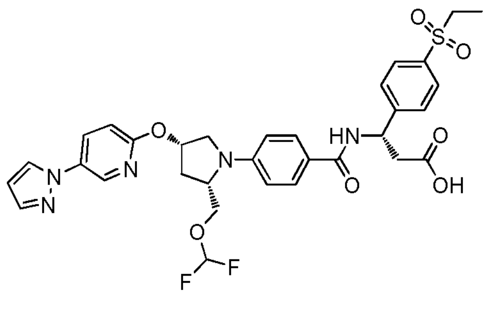

**Step 1: Synthesis of methyl (S)-3-(4-((2S,4S)-4-((5-(1H-pyrazol-1-yl)pyridine-2-yl)oxy)-2-((difluoromethoxy)methyl)pyrrolidin-1-yl)benzoylamino)-3-(4-(ethylsulfonyl)phenyl)propiona te**

HATU (240 mg, 0.63 mmol), methyl (S)-3-amino-3-(4-(ethylsulfonyl)phenyl)propionate hydrochloride (167 mg, 0.54 mmol), 4-((2S,4S)-4-((5-(1H-pyrazol-1-yl)pyridin-2-yl)oxy)-2-((difluoromethoxy)methyl)pyrrolidin -1-yl)benzoic acid (180 mg , 0.42 mmol) and TEA (130 mg, 1.28 mmol) were successively added to DCM (6 mL), and the mixture was stirred at room temperature for 17 h. The reaction solution was concentrated under reduced pressure. The residue was diluted with DCM (40 mL), washed successively with HCl solution (15 mL, 1 mol/L), saturated $NaHCO_3$ solution (15 mL)

and saturated NaCl solution (15 mL), dried over anhydrous $Na_2SO_4$, and concentrated under reduced pressure. The crude product was separated by silica gel column chromatography (eluent: DCM/EtOAc (v/v) = 1/1) to give a white solid (250 mg, 87%).
MS (ESI, pos.ion) m/z: 684.1 $[M+H]^+$.

**Step 2: Synthesis of (S)-3-(4-((2S,4S)-4-((5-(1H-pyrazol-1-yl)pyridine-2-yl)oxy)-2-((difluoromethoxy)methyl)pyrrolidin-1-yl)benzoylamino)-3-(4-(ethylsulfonyl)phenyl)propionic acid**

To a solution of methyl (S)-3-(4-((2S,4S)-4-(5-(1H-pyrazol-1-yl)pyridin-2-yl)oxy) -2-((difluoromethoxy)methyl)pyrrolidin-1-yl)benzamido)-3-(4-(ethylsulfonyl)phenyl)propanoate (250 mg, 0.37 mmol) in MeOH (2 mL) and THF (2 mL) was added a solution of LiOH (90 mg, 3.76 mmol) in $H_2O$ (1 mL). The mixture was stirred at 60°C for 17 h. The reaction solution was concentrated under reduced pressure. The remaining solution was added with HCl solution (1 mol/L) to adjust the pH of the solution to about 4. The resulting mixture was extracted with EtOAc (30 mL), washed with saturated NaCl (15 mL) solution, dried over anhydrous $Na_2SO_4$, concentrated under reduced pressure, and the crude product was separated by silica gel column chromatography (eluent: DCM/MeOH (v/v) = 20/1) to give a white solid (150 mg, 61%).

MS (ESI, pos.ion) m/z: 670.4 $[M+H]^+$.
$^1H$ NMR (400 MHz, $CDCl_3$) δ (ppm): 8.47 (d, J = 2.6 Hz, 1H), 7.94 (dd, J = 8.8, 2.7 Hz, 1H), 7.83 (dd, J = 7.2, 5.5 Hz, 3H), 7.74 (d, J = 8.4 Hz, 3H), 7.62 (d, J = 7.8 Hz, 1H), 7.55 (d, J = 8.1 Hz, 2H), 6.84 (d, J = 8.8 Hz, 1H), 6.63 (d, J = 8.6 Hz, 2H), 6.50 (t, J = 1.9 Hz, 1H), 6.24 (t, J = 74.4 Hz, 1H), 5.77 (s, 1H), 5.65 (s, 1H), 4.22-4.13 (m, 2H), 3.99 (t, J = 9.9 Hz, 1H), 3.71 (s, 2H), 3.07 (q, J = 7.4 Hz, 2H), 3.00 (s, 2H), 2.48 (d, J = 14.4 Hz, 1H), 2.44 - 2.35 (m, 1H), 1.25 (t, J = 7.4 Hz, 3H).

**Example 173 (R)-2-(6-((2S,4S)-4-((5-(2H-1,2,3-triazol-2-yl)pyridine-2-yl)oxy)-2 -((difluoromethoxy) methyl)pyrrolidin-1-yl)nicotinylamino)-2-(4-(ethylsulfonyl)phenyl)ethyl carbamate**

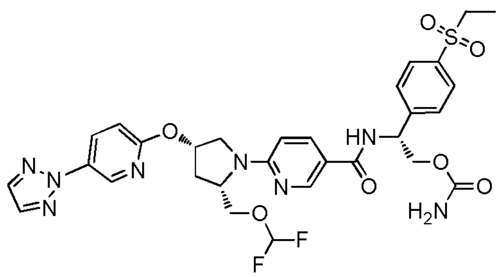

**Step 1: Synthesis of benzyl (2S,4S)-4-((5-(2H-1,2,3-triazol-2-yl)pyridin-2-yl)oxy)-2 -((difluoromethoxy)methyl)pyrrolidin-1-carboxylate**

To a 50 mL flask were added 5-(2H-1,2,3-triazol-2-yl)pyridin-2-ol (0.26 g, 1.58 mmol), $K_2CO_3$ (0.34 g, 2.50 mmol), benzyl (2S,4R)-2-((difluoromethoxy)methyl)-4-((methylsulfonyl)oxy)pyrrolidin-1-carboxylate (0.60 g, 1.60 mmol) and DMF (4 mL) successively. The mixture was stirred at 80 °C for 5 h. The mixture was cooled to room temperature and extracted with EtOAc. The combined organic phases were washed with water and saturated NaCl solution, dried over anhydrous $Na_2SO_4$, concentrated under reduced pressure, and the concentrated solution was separated by silica gel column chromatography (eluent: PE/EtOAc (v/v) = 3/1) to give colorless transparent oil (0.28 g, 40%).
MS (ESI, pos.ion) m/z: 446.2 $[M+H]^+$.

**Step 2: Synthesis of 2-(((3S,5S)-5-((difluoromethoxy)methyl)pyrrolidin-3-yl)oxy) -5-(2H-1,2,3-triazol-2-yl)pyridine**

To a 100 mL flask were added benzyl (2S,4S)-4-((5-(2H-1,2,3-triazol-2-yl)pyridin-2-yl) oxy)-2-((difluoromethoxy)methyl)pyrrolidine-1-carboxylate (0.28 g, 0.63 mmol), Pd/C (0.061 g) and MeOH (4 mL) successively. The reaction mixture was degassed and refilled with $H_2$ and was reacted overnight at room temperature. The resulting mixture was diluted with EtOAc, filtered to remove insolubles, and concentrated under reduced pressure to give colorless transparent oil (0.17 g, 99%).

**Step 3: Synthesis of methyl 6-((2S,4S)-4-((5-(2H-1,2,3-triazol-2-yl)pyridin-2-yl) oxy)-2-((difluoromethoxy)methyl)pyrrolidin-1-yl)nicotinate**

To a 100 mL flask were added 2-(((3S,5S)-5-((difluoromethoxy)methyl) pyrrolidin-3-yl)oxy)-5-(2H-1,2,3-triazol-2-yl)pyridine (0.17 g, 0.55 mmol), $K_2CO_3$ (0.17 g, 1.20 mmol), DMF (4 mL) and methyl 6-fluoropyridine-3-carboxylate (0.13 g, 0.84 mmol) successively and the mixture was reacted at 60 °C for 6 h. The mixture was cooled to room temperature and extracted with EtOAc. The combined organic phases were washed with water and saturated NaCl solution, dried over anhydrous $Na_2SO_4$, concentrated under reduced pressure, and the concentrated solution was separated by silica gel column chromatography (eluent: PE/EtOAc (v/v) = 1/1) to give colorless transparent oil (0.156 g, 64%).
MS (ESI, pos.ion) m/z: 447.1 $[M+H]^+$.

**Step 4: Synthesis of 6-((2S,4S)-4-((5-(2H-1,2,3-triazol-2-yl)pyridin-2-yl)oxy)-2-((difluoromethoxy)methyl)pyrrolidin-1-yl)nicotinic acid**

To a 100 mL flask were added methyl 6-((2S,4S)-4-((5 -(2H-1,2,3-triazol-2-yl)pyridin -2-yl)oxy)-2-((difluoromethoxy)methyl)pyrrolidine-1-yl)nicotinate (0.16 g, 0.35 mmol), LiOH (0.12 g, 2.90 mmol), MeOH (3.5 mL) and $H_2O$ (1 mL) successively. The mixture was reacted at room temperature for 5 h. The pH was adjusted with dilute hydrochloric acid. The resulting mixture was extracted with EtOAc, and the combined organic phases were washed with water and saturated NaCl solution, dried over anhydrous $Na_2SO_4$, concentrated under reduced pressure to give a white solid (0.11 g, 70.8%).
MS (ESI, pos.ion) m/z: 433.3 $[M+H]^+$.

**Step 5: Synthesis of (R)-2-(6-((2S,4S)-4-((5-(2H-1,2,3-triazol-2-yl)pyridine-2-yl)oxy) -2-((difluoromethoxy)methyl)pyrrolidin-1-yl)nicotinylamino)-2-(4-(ethylsulfonyl)phenyl)ethyl carbamate**

To a 25 mL flask were added 6-((2S,4S)-4-((5-(2H-1,2,3-triazol-2-yl)pyridin-2-yl) oxy)-2-((difluoromethoxy)methyl)pyrrolidine-1-yl)nicotinic acid (0.037 g, 0.086 mmol), DIPEA (0.023 g, 0.18 mmol), DMF (1.0 mL), HATU (0.038 g, 0.10 mmol) and (2R)-2-amino-2-(4-(ethylsulfonyl)phenyl)ethylcarbamate (0.027 g, 0.099 mmol) successively. The mixture was reacted at room temperature overnight. The mixture was extracted with EtOAc, and the combined organic phases were washed with water, saturated $NaHCO_3$ solution and saturated NaCl solution, dried over anhydrous $Na_2SO_4$, concentrated under reduced pressure. The concentrated solution was separated by silica gel column chromatography (eluent: PE/EtOAc (v/v) = 20/1) to give a white solid (0.014 g, 24%).

MS (ESI, pos.ion) m/z: 687.2 $[M+H]^+$.
$^1H$ NMR (400 MHz, $CDCl_3$) $\delta$ (ppm): 8.88 (s, 1H), 8.70 (s, 1H), 8.29 (d, J = 5.9 Hz, 1H), 7.93 (s, 2H), 7.84 (d, J = 6.3 Hz, 2H), 7.75 (s, 1H), 7.57 (d, J = 4.6 Hz, 2H), 6.87 (d, J = 7.1 Hz, 1H), 6.44 (s, 1H), 5.82 (s, 1H), 5.41 (s, 1H), 5.00 (s, 2H), 4.55 (br, 2H), 4.34 (br, 2H), 4.02 (br, 1H), 3.84 (dd, *J*= 36.3, 12.3 Hz, 2H), 3.09 (d, *J*= 5.0 Hz, 2H), 2.60 - 2.40 (m, 2H), 1.27 (t, *J*= 4.4 Hz, 3H).

**Example 174 (S)-3-(6-((2S,4S)-4-((5-(2H-1,2,3-triazol-2-yl)pyridine-2-yl)oxy) -2-((difluoromethoxy)methyl)pyrrolidin-1-yl)nicotinylamino)-3-(4-(ethylsulfonyl)phenyl) propionic acid**

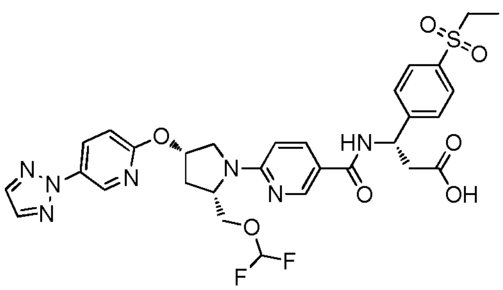

**Step 1: Synthesis of methyl (S)-3-(6-((2S,4S)-4-((5-(2H-1,2,3-triazol-2-yl)pyridine-2-yl) oxy)-2-((difluoromethoxy)methyl)pyrrolidin-1-yl)nicotinylamino)-3-(4-(ethylsulfonyl)phenyl)p ropionate**

To a 50 mL flask were added 6-((2S,4S)-4-((5-(2H-1,2,3-triazol-2-yl)pyridin -2-yl)oxy)-2-((difluoromethoxy)methyl)pyrrolidine-1-yl)nicotinic acid (0.070 g, 0.16 mmol), methyl (3S)-3-amino-3-(4-(ethylsulfonyl)phenyl)propanoate hydrochloride (0.056 g, 0.18 mmol), DIPEA (0.064 g, 0.50 mmol), DMF (2.5 mL) and HATU (0.075 g, 0.20 mmol) successively. The mixture was reacted at room temperature overnight. The resulting mixture was extracted with EtOAc. The combined organic phases were washed with water, saturated $NaHCO_3$ solution and saturated NaCl solution, dried over anhydrous $Na_2SO_4$, concentrated under reduced pressure to give a white solid (0.10 g, 90%).
MS (ESI, pos.ion) m/z: 686.2 $[M+H]^+$.

**Step 2: Synthesis of (S)-3-(6-((2S,4S)-4-((5-(2H-1,2,3-triazol-2-yl)pyridine-2-yl)oxy) -2-((difluoromethoxy)methyl)pyrrolidin-1-yl)nicotinylamino)-3-(4-(ethylsulfonyl)phenyl)propi onic acid**

To a 100 mL flask were added methyl (S)-3-(6-((2S,4S)-4-((5-(2H-1,2,3-triazol-2-yl) pyridin-2-yl)oxy)-2-((difluoromethoxy)methyl)pyrrolidine-1-yl)nicotinylamino)-3-(4-(ethylsulfonyl )phenyl)propanoate (0.10 g, 0.15 mmol), LiOH (0.044 g, 1.00 mmol), MeOH (3.0 mL) and $H_2O$ (0.6 mL) successively. The mixture was reacted at room temperature for 5 h. The pH was adjusted to 8 with dilute hydrochloric acid. The resulting mixture was extracted with EtOAc, and the combined organic phases were washed with water and saturated NaCl solution, dried over anhydrous $Na_2SO_4$, concentrated under reduced pressure. The concentrated solution was separated by silica gel column chromatography (eluent: DCM/MeOH (v/v) = 1/20) to give a white solid (0.060 g, 61%).
MS (ESI, pos.ion) m/z: 672.1 $[M+H]^+$.

**Example 175 2-((R)-2-(6-((2S,4S)-4-((5-(1H-pyrazol-1-yl)pyridine-2-yl)oxy) -2-((difluoromethoxy)methyl)pyrrolidin-1-yl)nicotinylamino)-2-(4-(ethylsulfonyl)phenyl) ethoxy)acetic acid**

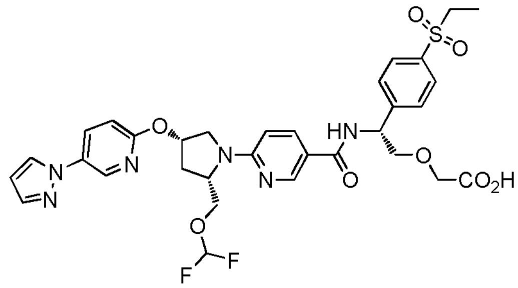

**Step 1: Synthesis of 6-((2S,5S)-4-((5-(1H-pyrazol-1-yl)pyridin-2-yl)oxy)-2-((difluoromethoxy) methyl)pyrrolidin-1-yl)-N-((R)-1-(4-(ethylsulfonyl)phenyl)-2-hydroxyethyl)nicotinamide**

HATU (264 mg, 0.69 mmol), (R)-2-amino-2-(4-(ethylsulfonyl)phenyl)ethanol (140 mg, 0.61 mmol), 6-((2S,4S)-4-((5-(1H-pyrazol-1-yl)pyridin-2-yl)oxy)-2-((difluoromethoxy) methyl)pyrrolidin-1-yl)nicotinic acid (200 mg , 0.46 mmol) and TEA (140 mg, 1.38 mmol) were successively added to DCM (6 mL), and the mixture was reacted at room temperature for 17 h. The reaction solution was concentrated under reduced pressure. The residue was diluted with DCM (40 mL), washed successively with HCl solution (15 mL, 1 mol/L), saturated $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (220 mg, 74%).
MS (ESI, pos.ion) m/z: 643.2 $[M+H]^+$.

**Step 2: Synthesis of ethyl 2-((R)-2-(6-((2S,4S)-4-((5-(1H-pyrazol-1-yl)pyridine-2-yl)oxy)-2 -((difluoromethoxy)methyl)pyrrolidin-1-yl)nicotinylamino)-2-(4-(ethylsulfonyl)phenyl)ethoxy) acetate**

To a solution of Rhodium(II) acetate dimer (20 mg, 0.05 mmol) and6-((2S,4S)-4-((5-(1H-pyrazol-1-yl)pyridin-2-yl)oxy)-2-((difluoromethoxy)methyl)pyrrolidine-1-yl)-N-((R)-1-(4-(ethylsulfonyl)phenyl)-2-hydroxyethyl)nicotinamide (220 mg, 0.34 mmol) in DCM (4 mL) was added a solution of ethyl 2-diazoacetate (0.40 mL, 3.80 mmol) in DCM (0.5 mL). The mixture was stirred at room temperature for 18 h. The reaction solution was diluted with DCM (30 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/EtOAc (v/v) = 1/2) to give a pale yellow solid (115 mg, 46%).
MS (ESI, pos.ion) m/z: 729.2 $[M+H]^+$.

**Step 3: Synthesis of 2-((R)-2-(6-((2S,4S)-4-((5-(1H-pyrazol-1-yl)pyridine-2-yl)oxy)-2-((difluoromethoxy)methyl)pyrrolidin-1-yl)nicotinylamino)-2-(4-(ethylsulfonyl)phenyl)ethoxy)a cetic acid**

To a solution of ethyl 2-((R)-2-(6-(2S,4S)-4-(5-(1H-pyrazol-1-yl)pyridin-2-yl)oxy)-2 -((difluoromethoxy)methyl)pyrrolidin-1-yl)nicotinamido)-2-(4-(ethylsulfonyl)phenyl)ethoxy)acetat e (115 mg, 0.16 mmol) in MeOH (2 mL) and THF (2 mL) was added a solution of LiOH (40 mg, 1.67 mmol) in $H_2O$ (1 mL). The mixture was stirred at room temperature for 16 h. The reaction solution was concentrated under reduced pressure, and the remaining solution was added with HCl solution (1 mol/L) to adjust the pH of the solution to about 4. The resulting mixture was extracted with EtOAc (30 mL), washed with saturated NaCl (15 mL) solution, dried over anhydrous $Na_2SO_4$, and concentrated under reduced pressure. The crude product was separated by silica gel column chromatography (eluent: DCM/MeOH (v/v) = 20/1) to give a white solid (40 mg, 36%).

MS (ESI, pos.ion) m/z: 701.2 $[M+H]^+$.
$^1H$ NMR (400 MHz, DMSO-$d_6$) δ (ppm): 8.89 (s, 1H), 8.69 (d, *J*= 2.5 Hz, 1H), 8.48 (d, *J*= 2.2 Hz, 1H), 8.20 (dd, J = 8.9, 2.8 Hz, 2H), 7.82 (d, *J*= 8.2 Hz, 2H), 7.78 (s, 1H), 7.60 (d, *J*= 8.1 Hz, 2H), 7.01 (d, J = 8.9 Hz, 1H), 6.72 (t, J = 76.0 Hz, 1H), 6.62 (d, J = 8.9 Hz, 1H), 6.57 (s, 1H), 5.05 (s, 1H), 4.49 (s, 1H), 4.29 (s, 1H), 3.94 (t, J = 9.0 Hz, 1H), 3.87 (d, J = 7.2 Hz, 1H), 3.78 - 3.67 (m, 5H), 3.29 - 3.24 (m, 2H), 2.31 (d, J = 14.4 Hz, 1H), 2.03 - 1.96 (m, 1H), 1.11 (t, J = 7.3 Hz, 3H).

**Example 176 (R)-2-(4-(Ethylsulfonyl)phenyl)-2-(6-((2S,4S)-2-(fluoromethyl)-4-((5-(trifluoromethoxy) pyridin-2-yl)oxy)pyrrolidin-1-yl)nicotinamido)ethyl carbamate**

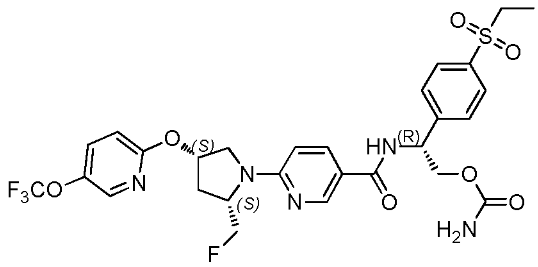

**Step 1: Synthesis of (2S,4S)-benzyl 2-(hydroxymethyl)-4-((5-(trifluoroethoxy)pyridin -2-yl)oxy)pyrrolidine-1-carboxylate**

Benzyl (2S,4R)-2-(hydroxymethyl)-4-((methylsulfonyl)oxy)pyrrolidin-1-carboxylate (1.10 g, 3.34 mmol), 5-(trifluoromethoxy)pyridin-2-ol (720 mg, 4.02 mmol) and $K_2CO_3$ (923 mg, 6.68 mmol) were added to DMF (10 mL), and the mixture was reacted at 80 °C for 11 h. The reaction solution was cooled to room temperature, diluted with EtOAc (50 mL), washed successively with $H_2O$ (20 mL × 2) and saturated NaCl solution (20 mL), dried over anhydrous $Na_2SO_4$, and concentrated under reduced pressure. The crude product was separated by silica gel column chromatography (eluent: PE/EtOAc (v/v) = 3/2) to give pale yellow liquid (957 mg, 70%).
MS (ESI, pos.ion) m/z: 413.1 $[M+H]^+$.

**Step 2: Synthesis of benzyl (2S,4S)-2-(((methylsulfonyl)oxy)methyl)-4-((5-(trifluoromethoxy) pyridin-2-yl)oxy)pyrrolidine-1-carboxylate**

Benzyl (2S,4S)-2-(hydroxymethyl)-4-((5-(trifluoromethoxy)pyridin-2-yl)oxy) pyrrolidine-1- carboxylate (950 mg, 2.30 mmol), MsCl (0.27 mL, 3.50 mmol), DMAP (30 mg, 0.25 mmol) and TEA (0.65 mL, 4.70 mmol) were added to DCM (12 mL). The mixture was reacted at room temperature for 6 h. The reaction solution was concentrated under reduced pressure, and the crude product was separated by silica gel column chromatography (eluent: PE/EtOAc (v/v) = 3/2) to give colorless liquid (1.05 g, 93%).
MS (ESI, pos.ion) m/z: 491.1 $[M+H]^+$.

**Step 3: Synthesis of benzyl (2S,4S)-2-(fluoromethyl)-4-((5-(trifluoromethoxy)pyridin-2-yl) oxy)pyrrolidine-1-carboxylate**

To a solution of benzyl (2S,4S)-2-(((methylsulfonyl)oxy)methyl)-4-((5-(trifluoromethoxy) pyridin-2-yl)oxy)pyrrolidine-1-carboxylate (1.00 g, 2.04 mmol) in THF (8 mL) was added $Bu_4NF$ (4.10 mL, 1 mol/L). The mixture was stirred at 75°C for 16 h. The reaction solution was concentrated under reduced pressure. The concentrated solution was diluted with EtOAc, washed successively with HCl solution (1 M), saturated $Na_2CO_3$ solution and saturated NaCl solution, dried over anhydrous $Na_2SO_4$, and concentrated under reduced pressure. The concentrated solution was separated by silica gel column chromatography (eluent: PE/EtOAc (v/v) = 4/1) to give colorless liquid (480 mg, 57%).
MS (ESI, pos.ion) m/z: 415.1 $[M+H]^+$.

**Step 4: Synthesis of 2-(((3S,5S)-5-(fluoromethyl)pyrrolidin-3-yl)oxy) -5-(trifluoromethoxy)pyridine**

To a solution of benzyl (2S,4S)-2-(fluoromethyl)-4-((5-(trifluoromethoxy)pyridin-2-yl) oxy)pyrrolidine-1-carboxylate (480 mg, 1.16 mmol) in MeOH (8 mL) was added Pd/C (50 mg, 10 %) under hydrogen protection. The mixture was reacted at room temperature for 5 h. The reaction solution was filtered through a celite pad, and concentrated under reduced pressure to give pale yellow liquid (300 mg, 92%).
MS (ESI, pos.ion) m/z: 281.1 $[M+H]^+$.

**Step 5: Synthesis of methyl 6-((2S,4S)-2-(fluoromethyl)-4-((5-(trifluoromethoxy) pyridin-2-yl)oxy)pyrrolidin-1-yl)nicotinate**

2-(((3S, 5)S)-5-(fluoromethyl)pyrrolidin-3-yl)oxy)-5-(trifluoromethoxy)pyridine (300 mg, 1.07 mmol), methyl 6-fluoronicotinate (250 mg, 1.61 mmol) and $K_2CO_3$ (300 mg, 2.17 mmol) were added to DMF (6 mL), and the mixture was reacted at 100 °C for 12 h. The reaction solution was cooled to room temperature, diluted with EtOAc (50 mL), washed successively with $H_2O$ (20 mL × 2) and saturated NaCl solution (20 mL), dried over anhydrous $Na_2SO_4$, and concentrated under reduced pressure. The concentrated solution was separated by silica gel column chromatography (eluent: PE/EtOAc (v/v) = 4/1) to give pale yellow liquid (321 mg, 72%).
MS (ESI, pos.ion) m/z: 416.1 $[M+H]^+$.

**Step 6: Synthesis of 6-((2S,4S)-2-(fluoromethyl)-4-((5-(trifluoromethoxy)pyridin-2-yl)oxy) pyrrolidin-1-yl)nicotinic acid**

To a solution of methyl 6-((2S,4S)-2-(fluoromethyl)-4-((5-(trifluoromethoxy)pyridin -2-yl)oxy)pyrrolidin-1-yl)nicotinate (321 mg, 0.77 mmol) in MeOH (2 mL) and THF (2 mL) was added a solution of LiOH (150 mg, 6.26 mmol) in $H_2O$ (1 mL). The mixture was stirred at 50°C for 2 h. The reaction solution was concentrated under reduced pressure, and the remaining solution was added with HCl solution (1 mol/L) to adjust the pH of the solution to about 4. The resulting mixture was extracted with EtOAc (30 mL), washed with saturated NaCl (15 mL) solution, dried over anhydrous $Na_2SO_4$, and concentrated under reduced pressure. The concentrated solution was separated by silica gel column chromatography (eluent: DCM/EtOAc (v/v) = 3/2) to give a white solid (176 mg, 57%).
MS (ESI, pos.ion) m/z: 402.1 $[M+H]^+$.

**Step 7: Synthesis of (*R*)-2-(4-(Ethylsulfonyl)phenyl)-2-(6-((2*S*,4*S*)-2-(fluoromethyl)-4-((5-(trifluoromethoxy)pyridin-2-yl)oxy)pyrrolidin-1-yl)nicotinamido)ethyl carbamate**

HATU (241 mg, 0.63 mmol), TEA (128 mg, 1.26 mmol), 6-((2*S*,4*S*)-2-(fluoromethyl)-4-((5-(trifluoromethoxy)pyridin-2-yl)oxy)pyrrolidin-1-yl)nicotinic acid (170 mg, 0.42 mmol) and (R)-2-amino-2-(4-(ethylsulfonyl)phenyl)ethyl carbamate (140 mg, 0.51 mmol) were successively added to DCM (6 mL), and the mixture was reacted at room temperature for 16 h. The reaction solution was concentrated under reduced pressure. The residue was diluted with DCM (40 mL), washed successively with HCl solution (15 mL, 1 mol/L), saturated $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (220 mg, 79%).

MS (ESI, pos.ion) m/z: 656.1 $[M+H]^+$.
$^1$H NMR (400 MHz, $CDCl_3$) δ (ppm): 8.68 (d, *J* = 2.0 Hz, 1H), 8.10 (d, *J* = 2.6 Hz, 1H), 7.92 (dd, *J* = 8.9, 2.4 Hz, 1H), 7.86 (d, *J* = 8.3 Hz, 2H), 7.73 (d, *J* = 6.2 Hz, 1H), 7.56 (d, *J* = 8.2 Hz, 2H), 7.50 (dd, *J* = 8.9, 2.1 Hz, 1H), 6.77 (d, *J* = 9.0 Hz, 1H), 6.47 (d, *J* = 8.9 Hz, 1H), 5.72 (s, 1H), 5.41- 5.37 (m, 1H), 4.98 - 4.72 (m, 3H), 4.62 - 4.43 (m, 3H), 4.32 (dd, *J* = 12.1, 3.3 Hz, 1H), 3.87 (dd, *J* = 12.2, 4.9 Hz, 1H), 3.76 (d, *J* = 12.2 Hz, 1H), 3.09 (q, ‘ = 7.4 Hz,

2H), 2.46 (s, 2H), 1.28 (t, $J$ = 7.4 Hz, 3H).

**Example 177 (2*R*)-2-(4-(Ethylsulfonyl)phenyl)-2-(2-(2-(3-fluorophenyl)pyrrolidin -1-yl)pyrimidine-5-carboxamido)ethyl carbamate**

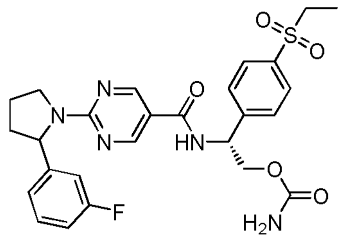

**Step 1: Synthesis of ethyl 2-(2-(3-fluorophenyl)pyrrolidin-1-yl)pyrimidine-5-carboxylate**

To a 50 mL flask were added a solution of 2-(3-fluorophenyl)pyrrolidine (0.41 g, 2.45 mmol) in DMF (5.5 mL), $K_2CO_3$ (0.41 g, 2.95 mmol) and ethyl 2-chloropyrimidine-5-carboxylate (0.50 g, 2.7 mmol) in turn. The mixture was reacted overnight at 50°C. The mixture was cooled to room temperature and extracted with EtOAc. The combined organic phases were washed with $H_2O$ and saturated NaCl solution, dried over anhydrous $Na_2SO_4$, concentrated under reduced pressure, and the concentrated solution was separated by silica gel column chromatography (eluent: PE/EtOAc (v/v) = 1/5) to give colorless transparent oil (0.56 g, 72%).
MS (ESI, pos.ion) m/z: 316.1 $[M+H]^+$.

**Step 2: Synthesis of 2-(2-(3-fluorophenyl)pyrrolidin-1-yl)pyrimidine-5-carboxylic acid**

To a 100 mL flask were added ethyl 2-(2-(3-fluorophenyl)pyrrolidin-1-yl)pyrimidine-5-carboxylate (0.562 g, 1.78 mmol), LiOH (0.747 g, 17.8 mmol), EtOH (8 mL) and $H_2O$ (2 mL) successively. The mixture was reacted overnight at room temperature. The pH was adjusted with dilute hydrochloric acid to about 5, and a white precipitate was precipitated. The resulting mixture was extracted with EtOAc. The combined organic phases were washed with $H_2O$ and saturated NaCl solution, dried over anhydrous $Na_2SO_4$, concentrated under reduced pressure to give a white solid (0.50 g, 98%).
MS (ESI, pos.ion) m/z: 288.1 $[M+H]^+$.

**Step 3: Synthesis of (2*R*)-2-(4-(Ethylsulfonyl)phenyl)-2-((2-(2-(3-fluorophenyl)pyrrolidin -1-yl)pyrimidine-5-carboxamido)ethyl carbamate**

To a 100 mL flask were added 2-(2-(3-fluorophenyl)pyrrolidin-1-yl)pyrimidine-5-carboxylic acid (0.087 g, 0.30 mmol), DIPEA (0.10 mL, 0.61 mmol), DMF (3.5 mL) and HATU (0.13 g, 0.35 mmol) successively. After the mixture was uniformly stirred, (2*R*)-2-amino-2-(4-(ethylsulfonyl)phenyl)ethyl carbamate (0.085 g, 0.31 mmol) was added, and the mixture was reacted at room temperature overnight. The mixture was extracted with EtOAc. The combined organic phases were successively washed with $H_2O$, saturated $NaHCO_3$ solution and saturated NaCl solution, dried over anhydrous $Na_2SO_4$, and concentrated under reduced pressure. The concentrated solution was separated by silica gel column chromatography (eluent: DCM/EtOAc (v/v) = 4/1) to give a white solid (0.14 g, 86%).

MS (ESI, pos.ion) m/z: 542.1 $[M+H]^+$.
$^1H$ NMR (400 MHz, $CDCl_3$) $\delta$ (ppm): 8.83 (s, 1H), 8.63 (s, 1H), 7.86 (d, $J$ = 6.7 Hz, 2H), 7.72 (d, $J$ = 2.4 Hz, 1H), 7.53 (d, $J$ = 6.5 Hz, 2H), 7.21 (s, 1H), 6.98 - 6.87 (m, 2H), 6.83 (d, $J$ = 8.7 Hz, 1H), 5.35 (br, 2H), 4.84 (br, 2H), 4.58 - 4.45 (m, 1H), 4.28 (d, $J$= 11.2 Hz, 1H), 3.94 (s, 1H), 3.80 (s, 1H), 3.16 - 3.00 (m, 2H), 2.48 - 2.35 (m, 1H), 2.09 - 1.95 (m, 3H), 1.27 (br, 3H).

**Example 178 (3*S*)-3-(4-(Ethylsulfonyl)phenyl)-3-(2-(2-(3-fluorophenyl)pyrrolidin -1-yl)pyrimidine-5-carboxamido)propionic acid**

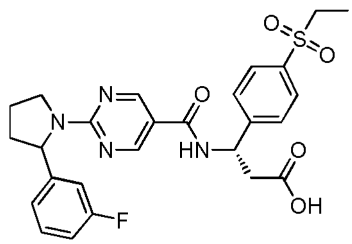

**Step 1: Synthesis of methyl (3*S*)-3-(4-(Ethylsulfonyl)phenyl)-3-(2-(2-(3-fluorophenyl)pyrrolidin-1-yl) pyrimidine-5-carboxamido)propionate**

To a 50 mL flask were added 2-(2-(3-fluorophenyl)pyrrolidin-1-yl)pyrimidine-5-carboxylic acid (0.082 g, 0.29 mmol), DIPEA (0.12 mL, 0.70 mmol) and DMF (3.0 mL) successively. After dissolving, HATU (0.13 g, 0.35 mmol) was added. After the mixture was uniformly stirred, methyl (2*S*)-3-amino-3-(4-(ethylsulfonyl)phenyl)propionate hydrochloride (0.086 g, 0.28 mmol) was added, and the mixture was reacted at room temperature overnight. The resulting mixture was extracted with EtOAc. The combined organic phases were washed with $H_2O$, saturated $NaHCO_3$ solution and saturated NaCl solution, dried over anhydrous $Na_2SO_4$, concentrated under reduced pressure to give crude product (210 mg, 96%).
MS (ESI, pos.ion) m/z: 541.3 $[M+H]^+$.

**Step 2: Synthesis of (3*S*)-3-(4-(Ethylsulfonyl)phenyl)-3-(2-(2-(3-fluorophenyl)pyrrolidin-1-yl) pyrimidine-5-carboxamido]propionic acid**

To a 100 mL flask were added methyl (3*S*)-3-(4-(ethylsulfonyl)phenyl)-3-(2-(2-(3-fluorophenyl)pyrrolidin-1-yl)pyrimidine-5-carboxamid o)propionate (0.14 g, 0.26 mmol), LiOH $H_2O$ (0.080 g, 1.90 mmol), MeOH (3.5 mL) and $H_2O$ (0.7 mL) successively. The mixture was reacted at room temperature for 5 h. The pH was adjusted to about 5 with dilute hydrochloric acid. The resulting mixture was extracted with EtOAc, and the combined organic phases were washed with $H_2O$ and saturated NaCl solution, dried over anhydrous $Na_2SO_4$, and concentrated under reduced pressure. The concentrated solution was separated by silica gel column chromatography (eluent: DCM/MeOH (v/v) = 1/20) to give a white solid (0.050 g, 27%).

MS (ESI, pos. ion) m/z: 527.05 $[M+H]^+$.
$^1H$ NMR (600 MHz, $CDCl_3$) $\delta$ (ppm): 9.06 (s, 1H), 8.80 (s, 1H), 8.58 (s, 1H), 7.82 (s, 2H), 7.54 (s, 2H), 7.21 (s, 1H), 6.88 - 6.78 (m, 3H), 5.60 (s, 1H), 5.32 (s, 1H), 3.89 (s, 1H), 3.70 (s, 1H), 3.55 - 3.37 (m, 2H), 3.09 (br, 2H), 2.74 (s, 1H), 2.39 (s, 1H), 2.01 (s, 2H), 1.26 (br, 3H).

**Example 179 (2*R*)-2-(2-(4,4-difluoro-2-(3-fluorophenyl)pyrrolidin-1-yl)** pyrimidine-5-carboxamido) -2-(4-(ethylsulfonyl)phenyl)ethyl carbamate

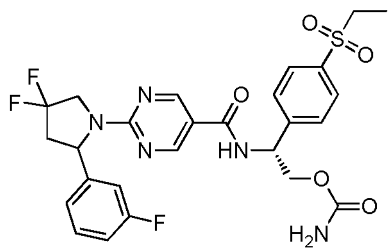

**Step** 1: **Synthesis of *tert-butyl* 3,3-difluoropyrrolidine-1-carboxylate**

To a 100 mL flask was added 3,3-difluoropyrrolidine hydrochloride (3.52 g, 24.50 mmol), which was dissolved in MeOH (51 mL). Then $K_2CO_3$ (6.74 g, 48.80 mmol) was added under an ice bath. After the mixture was uniformly stirred, di-*tert*-butyl dicarbonate (6.4 mL, 28.00 mmol) was added dropwise slowly, and the mixture was moved to room temperature to react overnight. The insolubles were removed by filtration, then the mixture was diluted with water and extracted with EtOAc. The combined organic phases were washed successively with $H_2O$ and saturated NaCl solution,

dried over anhydrous $Na_2SO_4$, and concentrated under reduced pressure. The concentrated solution was separated by silica gel column chromatography (eluent: PE/EtOAc (v/v) = 1/10) to give colorless transparent liquid (4.50 g, 89%).
MS (ESI, pos.ion) m/z: 208.1 $[M+H]^+$.

**Step 2: Synthesis of *tert*-butyl 4,4-difluoro-2-oxo-pyrrolidine-1-carboxylate**

To a 250 mL flask was added tert-butyl 3,3-difluoropyrrolidine-1-carboxylate (3.80 g, 18.3 mmol), which was dissolved in a mixed solvent of EtOAc (80 mL) and $H_2O$ (80 mL). Then ruthenium trichloride (0.23 g, 1.1 mmol) was added, and sodium periodate (15 g, 70.130 mmol) was slowly added in batches under an ice bath, and the reaction was stirred at room temperature for 24 h. The mixture was diluted with $H_2O$ (80mL) and extracted with EtOAc, and the combined organic phases were washed with saturated NaCl solution and added with IPA (40 mL). The mixture was stirred for 3 h, then dried over anhydrous $Na_2SO_4$, concentrated under reduced pressure, and the concentrated solution was separated by silica gel column chromatography (eluent: PE/EtOAc (v/v) = 1/8) to give pale yellow oil (2.41 g, 59.4%).
MS (ESI, pos.ion) m/z: 288.1 $[M+H]^+$.

**Step 3: Synthesis of *tert*-butyl 4,4-difluoro-2-(3-fluorophenyl)-2-hydroxy-pyrrolidine-1-carboxylate**

To a 500 mL two-necked flask was added *tert*-butyl 4,4-difluoro-2-oxo-pyrrolidine-1-carboxylate (2.40 g, 10.80 mmol), then anhydrous THF (41 mL) was added under nitrogen protection. Bromo-(3-fluorophenyl)magnesium (16 mL, 1 mol/L) was slowly added dropwise at -20°C for about 10 min, and the mixture was continued to stir for 10 min, and then moved to room temperature to react for 4 h. Methanol (50 mL) was added under an ice bath to quench the reaction. The mixture was stirred for 1 h, concentrated under reduced pressure to remove the solvent, and extracted with EtOAc. The mixture was extracted with EtOAc. The combined organic phases were washed saturated NaCl solution and saturated $NaHCO_3$ solution, dried over anhydrous $Na_2SO_4$, concentrated under reduced pressure, and the concentrated solution was separated by silica gel column chromatography (eluent: PE/EtOAc (v/v) = 4/1) to give a white solid (0.56 g, 16%).
MS (ESI, pos.ion) m/z: 262.2 $[M-55]^+$.

**Step 4: Synthesis of 3,3-difluoro-5-(3-fluorophenyl)-2,4-dihydropyrrole**

To a 100 mL flask was added a solution of *tert*-butyl 4,4-difluoro-2-(3-fluorophenyl)-2-hydroxy-pyrrolidine-1-carboxylate (0.47 g, 1.50 mmol) in DCM (8 mL). Then TFA (4.5 mL, 61 mmol) was added dropwise. The mixture was reacted at room temperature for 10 h. The mixture was concentrated under reduced pressure to remove the solvent to give brown oil (0.30 g, 100%).

**Step 5: Synthesis of 4,4-difluoro-2-(3-fluorophenyl)pyrrolidine**

To a 100 mL flask was added a solution of 3,3-difluoro-5-(3-fluorophenyl)-2,4-dihydropyrrole (0.30 g, 1.5 mmol) in EtOH (9 mL). Then glacial acetic acid (1.70 mL, 29.7 mmol) was added, and the mixture was stirred for 30 min. Finally sodium cyanoborohydride (0.28 g, 4.5 mmol) was added in batches under an ice bath, and the mixture was moved to room temperature and reacted for 2 h. The mixture was neutralized by adding saturated $NaHCO_3$ solution and extracted with EtOAc. The combined organic phases were washed with saturated NaCl solution, dried over anhydrous $Na_2SO_4$, concentrated under reduced pressure, and the concentrated solution was separated by silica gel column chromatography (eluent: PE/EtOAc (v/v) = 1/1) to give brown oil (0.174 g, 57%).
MS (ESI, pos.ion) m/z: 202.15 $[M+H]^+$.

**Step 6: Synthesis of ethyl 2-(4,4-difluoro-2-(3-fluorophenyl)pyrrolidin-1-yl) pyrimidine-5-carboxylate**

To a 100 mL flask were added a solution of 4,4-difluoro-2-(3-fluorophenyl)pyrrolidine (0.17 g, 0.85 mmol) in DMF (2.5 mL), $K_2CO_3$ (0.15 g, 1.08 mmol), ethyl 2-chloropyrimidine-5-carboxylate (0.18 g, 0.95 mmol) in turn. The mixture was reacted overnight at 50°C. The mixture was cooled to room temperature and extracted with EtOAc. The combined organic phases were washed with $H_2O$ and saturated NaCl solution, dried over anhydrous $Na_2SO_4$, concentrated under reduced pressure, and the concentrated solution was separated by silica gel column chromatography (eluent: PE/EtOAc (v/v) = 1/4) to give pale yellow oil (0.24 g, 81%).
MS (ESI, pos.ion) m/z: 352.15 $[M+H]^+$.

**Step 7: Synthesis of 2-(4,4-difluoro-2-(3-fluorophenyl)pyrrolidin-1-yl)pyrimidine-5-carboxylic acid**

To a 100 mL flask were added ethyl 2-(4,4-difluoro-2-(3-fluorophenyl)pyrrolidin-1-yl)pyrimidine-5-carboxylate

(0.24 g, 0.68 mmol), LiOH (0.275 g, 6.55 mmol), EtOH (3.5 mL) and $H_2O$ (0.9 mL) successively. The mixture was reacted at room temperature for 8 h. The pH was adjusted with dilute hydrochloric acid, and a precipitate was precipitated. The resulting mixture was extracted with EtOAc, and the combined organic phases were washed with saturated NaCl solution, dried over anhydrous $Na_2SO_4$, concentrated under reduced pressure to give a pale red solid (0.23 g, 100%).
MS (ESI, pos.ion) m/z: 324.2 $[M+H]^+$.

**Step 8: Synthesis of (2*R*)-2-(2-(4,4-difluoro-2-(3-fluorophenyl)pyrrolidin-1-yl)pyrimidine-5-carboxamido)-2-(4-(ethylsulfonyl)phenyl)ethyl carbamate**

To a 100 mL flask were added 2-(4,4-difluoro-2-(3-fluorophenyl)pyrrolidin-1-yl) pyrimidine-5-carboxylic acid (0.082 g, 0.25 mmol) and DIPEA (0.085 mL, 0.51 mmol) successively, which were dissolved in DMF (3 mL). Then HATU (0.10 g, 0.27 mmol) was added. After the mixture was uniformly stirred, (2*R*)-2-amino-2-(4-(ethylsulfonyl)phenyl)ethyl carbamate (0.068 g, 0.25 mmol) was added, and the mixture was reacted at room temperature overnight. The mixture was extracted with EtOAc. The combined organic phases were washed with $H_2O$, saturated $NaHCO_3$ solution and saturated NaCl solution, dried over anhydrous $Na_2SO_4$, and concentrated under reduced pressure. The concentrated solution was separated by silica gel column chromatography (eluent: DCM/EtOAc (v/v) = 4/1) to give a white solid (0.090 g, 61%).

MS (ESI, pos. ion) m/z: 578.2 $[M+H]^+$.
$^1$H NMR (400 MHz, $CDCl_3$) $\delta$ (ppm): 8.75 (d, *J* = 49.3 Hz, 2H), 7.83 (br, 3H), 7.51 (s, 2H), 7.31 (s, 1H), 6.98 - 6.94 (m, 3H), 5.40 - 5.53 (m, 2H), 4.87 (br, 2H), 4.50 (br, 1H), 4.33 - 4.22 (m, 1H), 4.20 - 4.06 (m, 1H), 3.97 - 3.95 (m, 1H), 3.06 (br, 2H), 2.49 (br, 2H), 1.25 (br, 3H).

**Example 180 (2*R*)-2-(5-chloro-6-(2-(3-fluorophenyl)pyrrolidin-1-yl) nicotinamido)-2-(4-(ethylsulfonyl) phenyl)ethyl carbamate**

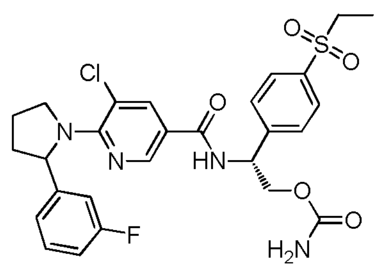

**Step 1: Synthesis of methyl 5-chloro-6-(2-(3-fluorophenyl)pyrrolidin-1-yl] pyridine-3-carboxylate**

To a 100 mL flask were added methyl 5,6-dichloropyridine-3-carboxylate (0.51 g, 2.45 mmol), $K_2CO_3$ (0.51 g, 3.70 mmol), 2-(3-fluorophenyl)pyrrolidine (0.49 g, 2.94 mmol) and DMF (5 mL) successively. The mixture was reacted at 70°C overnight. The mixture was cooled to room temperature and extracted with EtOAc. The combined organic phases were washed with $H_2O$ and saturated NaCl solution, dried over anhydrous $Na_2SO_4$, concentrated under reduced pressure, and the concentrated solution was separated by silica gel column chromatography (eluent: PE/EtOAc (v/v) = 1/4) to give colorless transparent oil (0.75 g, 91%).
MS (ESI, pos.ion) m/z: 335.1 $[M+H]^+$.

**Step 2: Synthesis of 5-chloro-6-(2-(3-fluorophenyl)pyrrolidin-1-yl)pyridine-3-carboxylic acid**

To a 100 mL flask were added methyl 5-chloro-6-(2-(3-fluorophenyl)pyrrolidin-1-yl)pyridine-3-carboxylate (0.75 g, 2.2 mmol) and LiOH (0.952 g, 22.7 mmol) successively, which were dissolved in a mixed solvent of MeOH (10 mL) and $H_2O$ (4 mL). The mixture was reacted at room temperature for 7 h. The pH was adjusted with dilute hydrochloric acid to 6-7. The resulting mixture was extracted with EtOAc, and the combined organic phases were washed with $H_2O$ and saturated NaCl solution, dried over anhydrous $Na_2SO_4$, concentrated under reduced pressure to give a white solid (0.65 g, 90%).
MS (ESI, pos.ion) m/z: 321.1 $[M+H]^+$.

**Step 3: Synthesis of (2R)-2-(5-chloro-6-(2-(3-fluorophenyl)pyrrolidin-1-yl)nicotinamido) -2-(4-(ethylsulfonyl)phenyl)ethyl carbamate**

To a 100 mL flask was added a solution of 5-chloro-6-(2-(3-fluorophenyl)pyrrolidin-1-yl)pyridine-3-carboxylic acid (0.080 g, 0.25 mmol) in DMF (2.5 mL, 0.61 mmol), then DIPEA (0.080 mL, 0.48 mmol) was added dropwise, and HATU (0.10 g, 0.27 mmol) was added with stirring. After the mixture was uniformly stirred, (2*R*)-2-amino-2-(4-(ethylsulfonyl)phenyl)ethyl carbamate (0.069 g, 0.25 mmol) was added, and the mixture was reacted at room temperature overnight. The mixture was extracted with EtOAc. The combined organic phases were washed with $H_2O$ and saturated NaCl solution, dried over anhydrous $Na_2SO_4$, concentrated under reduced pressure, and the concentrated solution was separated by silica gel column chromatography (eluent: DCM/EtOAc (v/v) = 3/2) to give a white solid (0.10 g, 70%).

MS (ESI, pos.ion) m/z: 575.1 $[M+H]^+$.
$^1H$ NMR (400 MHz, $CDCl_3$) $\delta$ (ppm): 8.41 (d, *J* = 5.1 Hz, 1H), 7.91 (s, 1H), 7.82 (d, *J* = 7.0 Hz, 2H), 7.71 (d, *J* = 6.0 Hz, 1H), 7.52 (d, *J* = 6.8 Hz, 2H), 7.21 (dd, *J* = 13.6, 6.1 Hz, 1H), 7.00 (d, *J* = 7.2 Hz, 1H), 6.94 - 6.82 (m, 2H), 5.53 (dd, *J* = 13.0, 6.4 Hz, 1H), 5.36 (s, 1H), 4.94 (s, 2H), 4.53 - 4.46 (m, 1H), 4.33 - 4.18 (m, 2H), 3.88 - 3.77 (m, 1H), 3.07 (q, *J* = 14.6, 7.2 Hz, 2H), 2.44 - 2.38 (m, 1H), 2.08 - 1.98 (m, 1H), 1.98 - 1.82 (m, 2H), 1.25 (t, *J*= 7.3 Hz, 3H).

**Example 181 2-(4-(ethylsulfonyl)phenyl)-3-((4-(1,1,1,3,3,3-hexafluoro-2-hydroxypropan-2-yl) phenyl)amino)-3-oxopropyl carbamate**

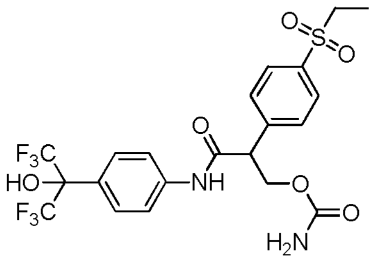

**Step 1: Synthesis of 3-(carbamoyloxy)-2-(4-(ethylsulfonyl)phenyl)propionic acid**

To a mixture of 2-(4-(ethylsulfonyl)phenyl)-3-hydroxypropionic acid (400 mg, 1.55 mmol) in ACN (6 mL) was added chlorosulfonyl isocyanate (0.55 mL, 6.30 mmol) at -15°C. After 10 min, $H_2O$ (6 mL) was added, and the mixture was reacted at 60°C for 16 h. The reaction solution was added with saturated $Na_2CO_3$ solution to adjust pH to 5. The resulting mixture was concentrated under reduced pressure. The aqueous phase was extracted with EtOAc (50 mL), washed with saturated NaCl solution (15 mL), dried over anhydrous $Na_2SO_4$, concentrated under reduced pressure, and the concentrated solution was separated by silica gel column chromatography (eluent: DCM/MeOH (v/v) = 10/1) to give a white solid (400 mg, 86%).
MS (ESI, pos.ion) m/z: 302.1 $[M+H]^+$.

**Step 2: Synthesis of 2-(4-(ethylsulfonyl)phenyl)-3-((4-(1,1,1,3,3,3-hexafluoro-2-hydroxypropan -2-yl)phenyl)amino)-3-oxopropyl carbamate**

HATU (567 mg, 1.49 mmol), DIPEA (386 mL, 2.99 mmol), 3-(carbamoyloxy)-2-(4-(ethylsulfonyl)phenyl)propionic acid (310 mg, 1.20 mmol), 2-(4-aminophenyl)-1,1,1,3,3,3-hexafluoropropan-2-ol (300 mg, 1.00 mmol) were successively added to DCM (6 mL), and the mixture was reacted at room temperature for 18 h. The reaction solution was concentrated under reduced pressure, diluted with DCM (40 mL), washed successively with HCl solution (15 mL, 1 mol/L) and saturated NaCl solution (15 mL), dried over anhydrous $Na_2SO_4$, concentrated under reduced pressure, and the concentrated solution was separated by silica gel column chromatography (eluent: DCM/MeOH (v/v) = 20/1) to give a white solid (63 mg, 12%).

MS (ESI, pos.ion) m/z: 499.1 $[M-44+H]^+$.
$^1H$ NMR (400 MHz, $CDCl_3$) $\delta$ (ppm): 7.83 (d, *J*= 8.3 Hz, 2H), 7.54 (d, *J*= 8.3 Hz, 2H), 7.46 (d, *J* = 8.5 Hz, 2H), 6.58 (d, *J* = 8.9 Hz, 2H), 3.89 - 3.78 (m, 2H), 3.43 (dd, *J*= 13.3, 4.6 Hz, 1H), 3.11 (s, 2H), 1.24 (t, *J* = 7.4 Hz, 3H).

**Example 182 2-(4-((cyclopropylmethyl)sulfonyl)phenyl)-3-((4-(1,1,1,3,3,3-hexafluoro-2-hydroxypropan-2-yl)phenyl)amino)-3-oxopropyl carbamate**

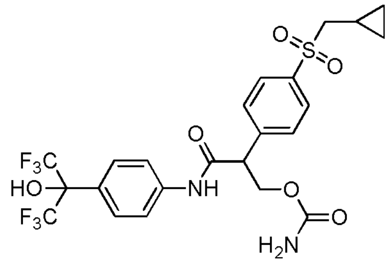

**Step** 1: **Synthesis of methyl 2-(4-mercaptophenyl)acetate**

To a solution of 2-(4-mercaptophenyl)acetic acid (2.58 g, 15.30 mmol) in MeOH (30 mL) was added $H_2SO_4$ (0.80 mL, 15.00 mmol), and the mixture was reacted at 70°C for 23 h. The reaction solution was concentrated under reduced pressure, diluted with EtOAc (60 mL), neutralized and washed with saturated $NaHCO_3$ solution (20 mL), then washed with saturated NaCl solution (20 mL), dried over anhydrous $Na_2SO_4$, concentrated under reduced pressure, and the concentrated solution was separated by silica gel column chromatography (eluent: PE/EtOAc (v/v) = 5/1) to give pale yellow liquid (2.51 g, 90%).
MS (ESI, pos.ion) m/z: 183.1 $[M+H]^+$.

**Step 2: Synthesis of methyl 2-(4-((cyclopropylmethyl)thio)phenyl)acetate**

Methyl 2-(4-mercaptophenyl)acetate (2.50 g, 13.70 mmol), (bromomethyl)cyclopropane (2.70 mL, 27.80 mmol), $K_2CO_3$ (3.80 g, 27.49 mmol) were added to ACN (40 mL), and the mixture was reacted at 80°C for 16 h. The reaction solution was filtered, and the filtrate was concentrated under reduced pressure. The concentrated solution was separated by silica gel column chromatography (eluent: PE/EtOAc (v/v) = 5/1) to give colorless liquid (2.75 g, 85%).
MS (ESI, pos.ion) m/z: 237.2 $[M+H]^+$.

**Step 3: Synthesis of methyl 2-(4-((cyclopropylmethyl)sulfonyl)phenyl)acetate**

To a solution of methyl 2-(4-((cyclopropylmethyl)thio)phenyl)acetate (2.30 g, 9.73 mmol) in MeOH (30 mL) was added a solution of potassium peroxomonosulfonate (12.00 g, 19.52 mmol) in $H_2O$ (30 mL) at 0°C. After the addition was completed, the mixture was reacted at room temperature for 20 h. Saturated $Na_2SO_3$ solution was added to the reaction solution, and no blue color was observed with starch KI test paper. The aqueous phase was extracted with EtOAc, and the organic phase was washed with saturated NaCl solution, dried over anhydrous $Na_2SO_4$, concentrated under reduced pressure. The concentrated solution was separated by silica gel column chromatography (eluent: PE/EtOAc (v/v) = 1/1) to give a white solid (1.34 g, 51%).
MS (ESI, pos.ion) m/z: 269.1 $[M+H]^+$.

**Step 4: Synthesis of 2-(4-((cyclopropylmethyl)sulfonyl)phenyl)acetic acid**

To a solution of methyl 2-(4-((cyclopropylmethyl)sulfonyl)phenyl)acetate (1.50 g, 5.59 mmol) in MeOH (6 mL) and THF (6 mL) was added a solution of LiOH (803 mg, 33.53 mmol) in $H_2O$ (6 mL). The mixture was stirred at 50°C for 18 h. The reaction solution was concentrated under reduced pressure, and the remaining liquid was added with concentrated HCl solution (1 mol/L) to adjust the pH to 4. The resulting mixture was extracted with EtOAc (50 mL), washed with saturated NaCl solution (30 mL), dried over anhydrous $Na_2SO_4$, and concentrated under reduced pressure to give a white solid (1.38 g, 97%).
MS (ESI, pos.ion) m/z: 255.1 $[M+H]^+$.

**Step 5: Synthesis of 2-(4-((cyclopropylmethyl)sulfonyl)phenyl)-3-hydroxypropionic acid**

To a solution of 2-(4-((cyclopropylmethyl)sulfonyl)phenyl)acetic acid (1.38 g, 5.43 mmol) in THF (30 mL) was added *i*-PrMgCl (8.0 mL, 2 mol/L) at -20°C under nitrogen protection. The mixture was reacted for about 10 min, and

moved to room temperature to react for 1 h, then moved to -20°C. Paraformaldehyde (815 mg, 27.14 mmol) was added, and the resulting mixture was moved to room temperature for 16 h after 30 min. The reaction solution was added $H_2O$ (10 mL) to quench the reaction, and HCl solution (1 mol/L) was added to adjust pH = 3. The mixture was concentrated under reduced pressure. The aqueous phase was extracted with EtOAc (50 mL), and the organic phase was washed with saturated NaCl solution (20 mL), dried over anhydrous $Na_2SO_4$, concentrated under reduced pressure. The concentrated solution was separated by silica gel column chromatography (eluent: DCM/EtOAc (v/v) = 1/1) to give pale yellow liquid (970 mg, 63%).
MS (ESI, pos.ion) m/z: 285.1 $[M+H]^+$.

**Step 6: Synthesis of 3-(carbamoyloxy)-2-(4-((cyclopropylmethyl)sulfonyl)phenyl)propionic acid**

To a solution of 2-(4-((cyclopropylmethyl)sulfonyl)phenyl)-3-hydroxypropionic acid (650 mg, 2.29 mmol) in ACN (8 mL) was added chlorosulfonyl isocyanate (0.60 mL, 6.86 mmol) at -15°C. After 10 min, $H_2O$ (4 mL) was added, and the mixture was reacted at 60°C for 16 h. The reaction solution was added with saturated $Na_2CO_3$ solution to adjust pH to 5. The resulting mixture was concentrated under reduced pressure, and the aqueous phase was extracted with EtOAc (50 mL), washed with saturated NaCl solution (20 mL), dried over anhydrous $Na_2SO_4$, concentrated under reduced pressure. The concentrated solution was separated by silica gel column chromatography (eluent: DCM/EtOAc (v/v) = 1/1) to give colorless liquid (468 mg, 63%).
MS (ESI, pos.ion) m/z: 328.0 $[M+H]^+$.

**Step 7: Synthesis of 2-(4-((cyclopropylmethyl)sulfonyl)phenyl)-3-((4-(1,1,1,3,3,3-hexafluoro-2-hydroxypropan-2-yl)phenyl)amino)-3-oxopropyl carbamate**

HATU (801 mg, 2.11 mmol), DIPEA (544 mg, 4.21 mmol), 3-(carbamoyloxy)-2-(4-((cyclopropylmethyl)sulfonyl)phenyl)propionic acid (460 mg, 1.41 mmol) and 2-(4-aminophenyl)-1,1,1,3,3,3-hexafluoropropan-2-ol (436 mg, 1.68 mmol) were successively added to DCM (8 mL) and the mixture was reacted at room temperature for 20 h. The reaction solution was concentrated under reduced pressure. The residue was diluted with DCM (40 mL), washed successively with HCl solution (15 mL, 1 mol/L), saturated $NaHCO_3$ solution (15 mL) and saturated NaCl solution (15 mL), dried over anhydrous $Na_2SO_4$, and concentrated under reduced pressure. The concentrated solution was separated by silica gel column chromatography (eluent: DCM/MeOH (v/v) = 20/1) to give a white solid(80 mg, 10%).

MS (ESI, pos.ion) m/z: 525.0 $[M-44+H]^+$.
$^1H$ NMR (400 MHz, DMSO-$d_6$) δ (ppm): 8.23 (s, 1H), 7.83 (dd, *J* = 16.8, 8.3 Hz, 2H), 7.63 (d, *J* = 8.1 Hz, 3H), 7.34 (d, *J* = 8.4 Hz, 2H), 7.05 (s, 1H), 6.66 (d, *J* = 8.8 Hz, 2H), 6.14 (t, *J* = 5.8 Hz, 1H), 3.94 - 3.88 (m, 1H), 3.76 - 3.67 (m, 1H), 3.31 - 3.28 (m, 1H), 3.23 (d, *J*= 7.1 Hz, 2H), 0.88 - 0.82 (m, 1H), 0.47- 0.43 (m, 2H), 0.15 - 0.11 (m, 2H).

**Example 183 (*R*)-2-(6-((2*S*,4*S*)-4-((5-chloropyridin-2-yl)oxy)-2-((difluoromethoxy) methyl)pyrrolidin-1-yl)nicotinamido)-2-(4-(ethylsulfonyl)phenyl)ethyl carbamate**

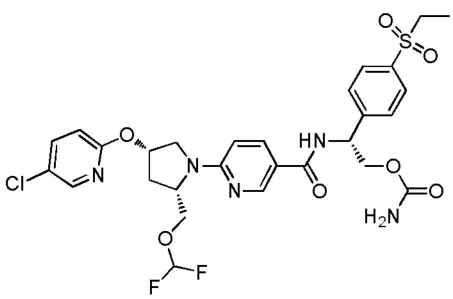

HATU (356 mg, 0.94 mmol), (R)-2-amino-2-(4-(ethylsulfonyl)phenyl)ethyl carbamate (187 mg, 0.69 mmol), 6-((2*S*,4*S*)-4-((5-chloropyridin-2-yl)oxy)-2-((difluoromethoxy) methyl)pyrrolidin-1-yl)nicotinic acid (250 mg , 0.63 mmol) and TEA (126 mg, 1.25 mmol) were successively added to DCM (6 mL), and the mixture was stirred at room temperature for 14 h. The reaction solution was concentrated under reduced pressure, and the residue was added with DCM (40 mL), washed successively with HCl solution (15 mL, 1 mol/L), saturated $NaHCO_3$ solution (15 mL) and saturated NaCl solution (15 mL), dried over anhydrous $Na_2SO_4$, concentrated under reduced pressure. The crude product was separated

by silica gel column chromatography (eluent: DCM/MeOH (v/v) = 20/1) to give a white solid (370 mg, 90%).

MS (ESI, pos.ion) m/z: 654.1 $[M+H]^+$.
$^1$H NMR (400 MHz, $CDCl_3$) $\delta$ (ppm): 8.68 (d, *J*= 2.0 Hz, 1H), 8.10 (d, *J*= 2.5 Hz, 1H), 7.92 (dd, *J* = 8.9, 2.3 Hz, 1H), 7.86 (d, *J* = 8.3 Hz, 2H), 7.71 (d, *J* = 6.2 Hz, 1H), 7.55 (dd, *J* = 8.5, 3.8 Hz, 3H), 6.70 (d, *J* = 8.8 Hz, 1H), 6.42 (d, *J* = 8.8 Hz, 1H), 6.24 (t, *J* = 74.8 Hz, 1H), 5.70 (t, J = 4.7 Hz, 1H), 5.40 (s, 1H), 4.91 (s, 2H), 4.57 - 4.49 (m, 2H), 4.32 (dd, *J* = 11.6, 3.1 Hz, 2H), 3.98 (t, *J* = 9.4 Hz, 1H), 3.84 (dd, *J* = 12.1, 4.9 Hz, 1H), 3.74 (d, *J* = 12.2 Hz, 1H), 3.09 (q, *J* = 7.4 Hz, 2H), 2.48 (d, *J*= 14.3 Hz, 1H), 2.42 - 2.34 (m, 1H), 1.27 (t, *J* = 7.4 Hz, 3H).

**Biological activity test**

**Biological Example 1 Fluorescence resonance energy transfer (FRET) test**

**1) Test method**

(1) Preparation of RORyt test buffer and 10 mM of DTT

100 mL of 1x Basic test Buffer (HEPES (pH 7.4), 100 mM of NaCl, 0.01% BSA) was prepared, and 154.25 mg of DTT was added. The mixture was well mixed.

(2) Preparation of compound gradient concentration

a. Standard compound was prepared: Standard compound was diluted with 100% DMSO to 2.5 mM, then diluted 3-fold with 11 serial dilutions to a final concentration of 42.34 nM;
b. Test compound was prepared referring to standard compound.

(3) Preparation of 1x protein solution mixture

a. Preparation of the desired amount of 2x B-RORγt LBD/SA-APC protein mixture: The concentration of B-RORγt LBD was 40 nM, and the concentration of SA-APC was 20 nM. They were mixed by gently inversion, and incubated at room temperature for 15 minutes. Then 400 nM of biotin was added. The mixture was mixed by gently inversion, and incubated at room temperature for 10 minutes;
b. Preparation of the desired amount of 2x Biotin-SRC1/SA-eu protein mixture: The concentration of Bioin-SRC1 was 40 nM, and the concentration of SA-eu was 20 nM. They were mixed by gently inversion, and incubated at room temperature for 15 minutes. Then 200 nM biotin was added. The mixture was mixed by gently inversion, and incubated at room temperature for 10 minutes;
c. The protein mixtures prepared in step a and step b were mixed at a ratio of 1:1, and incubated at room temperature for 5 minutes;
d. 25 μL of the mixture from step c was added to the 384-well plate containing the test compound;
e. The mixture was centrifuged at 1000 rpm for one minute;
f. The mixture was incubated for 1 hour at room temperature.

(4) Data collection and calculation

After incubation at room temperature for 1 hour, the fluorescence values at 665 nm and 615 nm were measured with an EnVision plate reader, and the inhibition rate was calculated. The final $IC_{50}$ values obtained are shown in Table 1;

$$\text{Inhibition rate (\%)} = [(\text{X-Min})/(\text{Max-Min})] \times 100\%$$

X is the fluorescence value ratio of "665 nm/615 nm" of the test compound; Min is the average value of the fluorescence value ratio of "665 nm/615 nm" of the DMSO blank control; Max is the average value of the fluorescence value ratio of "665 nm/615 nm" ot the 10 μM SRC.

**2) Test results**

Table 1 Evaluation of the compounds of the present invention on inhibitory activity of RORyt

| Example No. | $IC_{50}$ (nM) | Example No. | $IC_{50}$ (nM) |
|---|---|---|---|
| Example 5 | 36.1 | Example 7 | 57.5 |
| Example 8 | 18.8 | Example 9 | 25.8 |
| Example 10 | 19 | Example 11 | 27 |
| Example 12 | 21.5 | Example 13 | 31.4 |
| Example 15 | 78.68 | Example 17 | 14.88 |
| Example 18 | 25.9 | Example 20 | 30 |
| Example 21 | 2 | Example 22 | 9 |
| Example 24 | 19 | Example 25 | 66 |
| Example 26 | 23 | Example 28 | 24 |
| Example 31 | 59.84 | Example 32 | 61.1 |
| Example 39 | 67.47 | Example 40 | 21 |
| Example 41 | 17 | Example 42 | 18 |
| Example 45 | 43 | Example 47 | 10 |
| Example 48 | 8 | Example 49 | 28 |
| Example 50 | 16 | Example 51 | 14 |
| Example 57 | 41 | Example 59 | 47 |
| Example 60 | 81 | Example 62 | 22 |
| Example 63 | 7 | Example 64 | 11 |
| Example 66 | 33 | Example 70 | 26 |
| Example 72 | 34 | Example 73 | 8 |
| Example 74 | 70 | Example 75 | 10 |
| Example 76 | 18 | Example 77 | 7 |
| Example 78 | 12 | Example 81 | 44 |
| Example 83 | 91 | Example 89 | 14 |
| Example 90 | 9 | Example 92 | 21 |
| Example 93 | 13 | Example 94 | 31 |
| Example 95 | 77 | Example 96 | 31 |
| Example 97 | 21 | Example 98 | 21 |
| Example 99 | 25 | Example 100 | 14 |
| Example 102 | 4 | Example 103 | 60 |
| Example 104 | 12 | Example 106 | 52 |
| Example 105 | 7 | Example 109-1 | 7 |
| Example 108-2 | 24 | Example 110-2 | 7 |
| Example 110-1 | 19 | Example 112-2 | 13 |
| Example 111-1 | 16 | Example 114-2 | 23 |

(continued)

| Example No. | $IC_{50}$ (nM) | Example No. | $IC_{50}$ (nM) |
|---|---|---|---|
| Example 113-2 | 13 | Example 117-2 | 26 |
| Example 115-2 | 26 | Example 131 | 120 |
| Example 122-2 | 19 | Example 146 | 27 |
| Example 143 | 104 | Example 148 | 15.2 |
| Example 147 | 18.2 | Example 150 | 10.0 |
| Example 149 | 7.7 | Example 152 | 13.2 |
| Example 151 | 8.7 | Example 154 | 47 |
| Example 153 | 5 | Example 156 | 3.1 |
| Example 155 | 11 | Example 158 | 7.4 |
| Example 157 | 6.9 | Example 160 | 5.6 |
| Example 159 | 10.2 | Example 162 | 9.9 |
| Example 161 | 10.4 | Example 164 | 3.1 |
| Example 163 | 5.1 | Example 169 | 6 |
| Example 168 | 8 | Example 172 | 23.5 |
| Example 170 | 9 | Example 175 | 39.2 |
| Example 173 | 3.8 | Example 177 | 27.43 |
| Example 176 | 7.3 | Example 179 | 9.4 |
| Example 178 | 46.16 | Example 183 | 10 |
| Example 182 | 55.8 | / | / |

Conclusion: The experimental results show that the compounds of the present invention have good inhibitory activity on RORγt.

**Biological example 2 Pharmacokinetic evaluation**

**1) Test method:**

The ICR mice were weighed after an overnight fast for 15 hours, and then randomly divided into groups according to body weight. The test compounds were formulated in a vehicle of 10% DMSO + 10% kolliphor HS15 + 80% Saline. For the test group administered intravenously (indicated by *i.v.*), the test animals were administered 1 mg/kg of the test compound; for the test group administered by oral administration (indicated by *p.o.*), the test animals were administered 5 mg/kg of the test compound. Then, at time points 0, 0.083 (intravenous injection group only), 0.25, 0.5, 1.0, 2.0, 5.0, 7.0 and 24 h, venous blood (approximately 0.2 mL) was collected and placed in an EDTAK2 anticoagulation tube, then centrifuged at 11,000 rpm for 2 min. Plasma was collected and stored at -20°C or -70°C until LC/MS/MS analysis. The drug concentration in plasma was measured at each time point, and the pharmacokinetic parameters were calculated according to the drug concentration-time curve.

**2) Experimental results:**

The pharmacokinetic properties of the compounds of the present invention were tested by the above experiments. The experimental results show that the compounds of the present invention have good pharmacokinetic characteristics in ICR mice. The specific experimental results are shown in Table 2 and Table 3.

Table 2 Pharmacokinetic parameters of some compounds of the present invention in ICR mice

| Example No. | Drug-delivery way | $T_{1/2}$ (h) | $C_{max}$ (μM) | $AUC_{last}$ (h*μM) |
|---|---|---|---|---|
| Example 22 | *p.o.* | 3.6 | 4.8 | 37.0 |
| Example 47 | *p.o.* | 11.1 | 2.4 | 23.5 |
| Example 64 | *p.o.* | 13.2 | 3.33 | 47.2 |
| Example 65 | *p.o.* | 7.9 | 7.0 | 78.0 |

Table 3 Oral bioavailability of some compounds of the present invention in ICR mice

| Example No. | Example 22 | Example 47 | Example 64 | Example 65 |
|---|---|---|---|---|
| Oral bioavailability (F) | 80% | 66% | 91% | 82% |

**Biological example 3 Pharmacodynamic study on IMQ-induced mouse psoriasis model**

**1) Test method:**

Female c57bl/6 mice with a weight of 18-20 g were selected. After 7 days of adaptive feeding, they were randomly divided into normal group, model group and each compound group, with 4 mice in normal group and 10 mice in each other group. Administration method: The oral administration group was given by gavage. Modeling method: The modeling agent was evenly applied to the back and the inner and outer sides of the mice's right ear for 10 days. The state of the animals was observed daily and weighed every three days. After the experiment, the right ear of each animal was cut off and an 8 mm ear punch was used to obtain the ear piece at a fixed position and weighed. The ear piece was stored in liquid nitrogen, and then 500 μL of normal saline was added to homogenize it with a homogenizer. After centrifugation, the supernatant was taken and the IL-17 concentration in the supernatant was detected.

**2) Experimental results:**

It can be seen from the results that, compared with the model group, the compounds of the present invention can significantly reduce the ear weight and the secretion of the inflammatory factor IL-17 in the ear in the IMQ-induced mouse psoriasis model. Specifically, Example 47 and Example 64 can significantly reduce the ear weight of mice ($P < 0.05$); Example 47, Example 64 and Example 22 can significantly reduce the secretion of IL-17 in the ear of mice ($P < 0.05$); specific results are shown in Table 4 and Table 5.

Table 4 Effects of some compounds of the present invention on the ear weight of mice

| Group | Average ear weight (mg) | SD | Inhibition rate% |
|---|---|---|---|
| Normal group | 13.03 | 1.17 | - |
| Model group | 28.12 | 3.28 | - |
| Example 22 90mg/kg | 28.12 | 4.11 | 2% |
| Example 47 90mg/kg | 25.20* | 0.99 | 19% |
| Example 64 90mg/kg | 24.84* | 1.72 | 22% |

(Remark: * means P<0.05, ** means P<0.01)

Table 5 Inhibitory effect of some compounds of the present invention on the inflammatory factor IL-17 in the ear in IMQ-induced mouse psoriasis model

| Group | IL-17 | | |
|---|---|---|---|
| | Concentration (pg/μg protein) | SD | Inhibition rate |
| Normal group | 0.00 | 2.60 | - |
| Model group | 29.78 | 15.19 | - |
| Example 22 90mg/kg | 17.85 * | 6.58 | 40.08% |

(continued)

| Group | IL-17 | | |
|---|---|---|---|
| | Concentration (pg/μg protein) | SD | Inhibition rate |
| Example 47 90mg/kg | 13.29 ** | 5.38 | 55.38% |
| Example 64 90mg/kg | 8.20 ** | 2.31 | 72.47% |
| (Remark: * means P<0.05, ** means P<0.01) | | | |

**Biological example 4 Pharmacodynamic study on LPS-induced mouse blood inflammatory factor IL-17 model**

**1) Test method:**

BALB/c mice with a weight of 22-26 g were selected. After 7 days of adaptive feeding, they were randomly divided into normal group, model group and each compound group. The specific operation followed the time flow: On the first day, after the animals were weighed and divided into groups, the vehicle was administered for the first time about 16 hours before modeling. The next day, compound was administered 1 h before modeling. The modeling method was to inject 100 μl of 1 mg/ml LPS through the tail vein. Orbital blood was collected 4 hours after modeling. On the third day, IL-17 in plasma was detected.

**2) Experimental results:**

It can be seen from the results that the compounds of the present invention can reduce the LPS-induced mouse blood inflammatory factor IL-17. Specifically, Example 47, Example 64 and Example 65 can significantly reduce LPS-induced mouse blood inflammatory factor IL-17 ($P < 0.05$); Example 22 has a certain tendency to inhibit LPS-induced mouse blood inflammatory factor IL-17. The specific results are shown in Tables 6-8.

Table 6 Inhibitory effect of some compounds of the present invention on LPS-induced mouse blood inflammatory factor IL-17

| Group | IL-17 (pg/ml) | SD | Inhibition rate% |
|---|---|---|---|
| Normal group | -2.87 ** | 0.31 | - |
| Model group | 141.35 | 0.50 | - |
| Example 47 15mg/kg | 104.98 * | 0.67 | 25.22% |

Table 7 Inhibitory effect of some compounds of the present invention on LPS-induced mouse blood inflammatory factor IL-17

| Group | IL-17 (pg/ml) | SD | Inhibition rate% |
|---|---|---|---|
| Normal group | -4.95 ** | 0.48 | - |
| Model group | 174.69 | 1.09 | - |
| Example 47 30mg/kg | 83.18 * | 0.52 | 51% |
| Example 64 30mg/kg | 58.48 ** | 0.43 | 65% |
| Example 65 30mg/kg | 61.71 ** | 0.58 | 63% |

Table 8 Inhibitory effect of some compounds of the present invention on LPS-induced mouse blood inflammatory factor IL-17

| Group | IL-17(pg/ml) | SD | Inhibition rate% |
|---|---|---|---|
| Normal group | -5.99 ** | 0.46 | - |
| Model group | 81.33 | 0.43 | - |
| Example 47 15mg/kg | 56.69 ** | 0.48 | 28.21% |

(continued)

| Group | IL-17(pg/ml) | SD | Inhibition rate% |
|---|---|---|---|
| Example 22 15mg/kg | 72.12 | 0.51 | 10.55% |
| (Remark: * means P<0.05, ** means P<0.01) | | | |

**Biological example 5 Stability of the compounds of the present invention in human and rat liver microsomes**

**1) Test method:**

Human or rat liver microsomes were incubated in double wells in polypropylene tubes. A typical incubation mixture included human or rat liver microsomes (final concentration: 0.5 mg protein/mL), target compound (final concentration: 1.5 μM), and K-phosphate buffered solution (containing 1.0 nM EDTA, 100 mM, pH=7.4) in a total volume of 30 μL. Compounds were dissolved in DMSO and diluted with K-phosphate buffered solution to give a final working solution concentration of 30 μM. After 10 min of pre-incubation, 25 μL of test compound or positive control solution was transferred to the microsomal solution. The mixture was mixed well and 30 μL of the mixture was immediately pipetted, and ice-cold acetonitrile was added, then 15 μL of NADPH was added as To. 15 μL of NADPH (final concentration: 2 mM). The mixture for the enzymatic reaction, and the entire experiment was carried out in an incubation tube at 37°C. At various time points (0, 20 and 60 min), the reaction was stopped by adding 150 μL of acetonitrile (with internal standard). The protein was removed by centrifugation at 4000 rpm for 10 min, and the supernatant was collected and analyzed by LC-MS/MS.

For each reaction, the concentration of compound in the incubation of human or rat liver microsomes was plotted (expressed as a percentage) as a percentage relative to the zero time point to infer *in vitro* metabolic half-life and *in vivo* intrinsic hepatic clearance rate. The specific experimental results are shown in Table 9.

**2) Experimental results:**

Table 9 Experimental results of the stability of some compounds of the present invention in human and rat liver microsomes

| Example No. | Human | | Rat | |
|---|---|---|---|---|
| | Half-life $t_{1/2}$ (min) | Clearance rate (ml/min/kg) | Half-life $t_{1/2}$ (min) | Clearance rate (ml/min/kg) |
| Example 22 | 239 | 7.3 | Not metabolized | / |
| Example 47 | 210 | 8.3 | 1341 | 1.9 |
| Example 64 | Not metabolized | / | Not metabolized | / |
| Example 65 | 80.5 | 21.6 | Not metabolized | / |

Conclusion: It can be seen from the results that the compounds of the present invention have a long half-life and low clearance rate in human and rat liver microsomes, that is, the compounds of the present invention have better stability in human and rat liver microsomes.

Finally, it should be noted that there are other ways of implementing the invention. Accordingly, the embodiments of the present invention will be described as examples, but are not limited to the content described in the present invention, and may also be modifications made within the scope of the present invention or equivalents added in the claims. All publications or patents cited herein are incorporated herein by reference.

## Claims

**1.** A compound having Formula (I), or a stereoisomer, a geometric isomer, a tautomer, an *N*-oxide, a hydrate, a solvate, a metabolite, an ester, a pharmaceutically acceptable salt or a prodrug thereof,

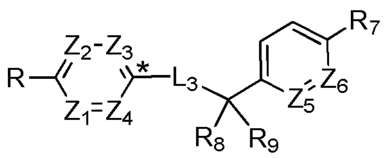

(I),

wherein,

R is $R_0$, $-(CH_2)_m$-B-$L_1$-**A or -$L_2$-G;
$Z_1$ is $CR_1$ or N; $Z_2$ is $CR_2$ or N; $Z_3$ is $CR_3$ or N; $Z_4$ is $CR_4$ or N; $Z_5$ is $CR_5$ or N; $Z_6$ is $CR_6$ or N;
each of $R_0$, $R_1$, $R_2$, $R_3$, $R_4$, $R_5$ and $R_6$ is independently H, deuterium, F, Cl, Br, I, $C_{1-6}$ alkyl, $C_{1-6}$ haloalkyl, $C_{1-6}$ alkoxy, hydroxy-substituted $C_{1-6}$ alkyl, hydroxy-substituted $C_{1-6}$ haloalkyl, -Si($C_{1-6}$ alkyl)$_3$, $C_{1-6}$ haloalkoxy or -N($R_dR_e$);
$R_7$ is -S(=O)$_2$-$C_{1-6}$ alkyl, -S(=O)$_2$-$C_{1-6}$ alkoxy, -S(=O)$_2$-$C_{1-6}$ alkylamino, -S(=O)$_2$-$C_{1-6}$ haloalkyl, -S(=O)$_2$-$C_{3-8}$ cycloalkyl, -S(=O)$_2$-$C_{1-6}$ alkylene-$C_{3-8}$ cycloalkyl, -S(=O)-$C_{1-6}$ alkyl, -S(=O)$_2$H, -COOH, -C(=O)-N($R_gR_h$), -N($R_g$)-C(=O)-$C_{1-6}$ alkyl, -C(=O)-O-$C_{1-6}$ alkyl, $C_{1-6}$ alkyl, $C_{1-6}$ alkoxy, $C_{1-6}$ haloalkyl or $C_{3-8}$ cycloalkyl;
each $R_g$ and $R_h$ is independently H, deuterium or $C_{1-6}$ alkyl;
each of A and G is independently $C_{3-8}$ cycloalkyl, $C_{6-10}$ aryl, 5- to 10-membered heteroaryl or 5- to 10-membered heterocyclyl; wherein, each of A and G is independently and optionally substituted with 1, 2, 3, 4 or 5 $R_a$;
B is 4- to 10-membered heterocyclyl or thiazolyl; wherein, the 4- to 10-membered heterocyclyl is optionally substituted with 1, 2, 3, 4 or 5 $R_b$;
each $R_a$ and $R_b$ is independently deuterium, F, Cl, Br, I, -OH, -CN, $-NH_2$, $-NO_2$, -COOH, oxo, $C_{1-6}$ alkyl, $C_{1-6}$ haloalkyl, $C_{1-6}$ alkoxy, $C_{1-6}$ haloalkoxy, -$C_{1-6}$ alkylene-O-$C_{1-6}$ alkyl, $C_{3-8}$ cycloalkyl, $C_{6-10}$ aryl, 5- to 10-membered heterocyclyl, 5- to 10-membered heteroaryl or -C(=O)-N($R_dR_e$); wherein, each of the $C_{1-6}$ alkyl, $C_{1-6}$ haloalkyl, $C_{1-6}$ alkoxy, $C_{1-6}$ haloalkoxy, -$C_{1-6}$ alkylene-O-$C_{1-6}$ alkyl, $C_{3-8}$ cycloalkyl, $C_{6-10}$ aryl, 5- to 10-membered heterocyclyl and 5- to 10-membered heteroaryl is independently and optionally substituted with 1, 2 or 3 $R_c$;
each $R_c$ is independently deuterium, F, Cl, Br, I, -OH, -CN, $-NH_2$, $C_{1-6}$ alkyl, $C_{1-6}$ alkoxy, $C_{1-6}$ haloalkyl, $C_{1-6}$ haloalkoxy, $C_{3-8}$ cycloalkyl, 5- to 10-membered heterocyclyl, $C_{6-10}$ aryl or 5- to 10-membered heteroaryl;
$R_8$ is H, deuterium, -OH, -CN, $-NH_2$, $-NO_2$, -COOH, $C_{1-6}$ alkyl, $C_{1-6}$ alkoxy, $C_{1-6}$ haloalkyl, cyano-substituted $C_{1-6}$ alkyl, carboxy-substituted $C_{1-6}$ alkyl, -$C_{1-6}$ alkylene-O-$C_{1-6}$ alkyl, -$C_{1-6}$ alkylene-C(=O)-O-$C_{1-6}$ alkyl, -$C_{1-6}$ alkylene-C(=O)-N($R_dR_e$), -$C_{1-6}$ alkylene-OC(=O)-N($R_dR_e$), -$C_{1-6}$ alkylene-N($R_f$)-C(=O)-N($R_dR_e$), -$C_{1-6}$ alkylene-N($R_dR_e$) or -N($R_f$)-C(=O)-$C_{1-6}$ alkyl;
$R_9$ is deuterium, -OH, -CN, $-NH_2$, $-NO_2$, -COOH, $C_{1-6}$ alkoxy, $C_{1-6}$ haloalkyl, cyano-substituted $C_{1-6}$ alkyl, carboxy-substituted $C_{1-6}$ alkyl, -$C_{1-6}$ alkylene-O-$C_{1-6}$ alkyl, -$C_{1-6}$ alkylene-C(=O)-O-$C_{1-6}$ alkyl, -$C_{1-6}$ alkylene-C(=O)-N($R_dR_e$), -$C_{1-6}$ alkylene-OC(=O)-N($R_dR_e$), -$C_{1-6}$ alkylene-N($R_f$)-C(=O)-N($R_dR_e$), -$C_{1-6}$ alkylene-N($R_dR_e$) or -N($R_f$)-C(=O)-$C_{1-6}$ alkyl;
wherein, each of the $C_{1-6}$ alkyl, $C_{1-6}$ alkoxy, $C_{1-6}$ haloalkyl, cyano-substituted $C_{1-6}$ alkyl, carboxy-substituted $C_{1-6}$ alkyl, -$C_{1-6}$ alkylene-O-$C_{1-6}$ alkyl and -$C_{1-6}$ alkylene-C(=O)-O-$C_{1-6}$ alkyl described in $R_8$ and $R_9$ is independently and optionally substituted with 1, 2, 3 or 4 substituents selected from deuterium, F, Cl, Br, I, -OH, -CN, $-NH_2$, -COOH, -N($R_dR_e$), $C_{1-6}$ alkyl, $C_{1-6}$ haloalkyl, $C_{1-6}$ alkoxy or $C_{1-6}$ haloalkoxy;
or, $R_8$ and $R_9$ together with the carbon atom to which they are attached, form $C_{3-8}$ cycloalkyl or 3- to 8-membered heterocyclyl; wherein, each of the $C_{3-8}$ cycloalkyl and 3- to 8-membered heterocyclyl is independently and optionally substituted with 1, 2, 3 or 4 substituents selected from deuterium, F, Cl, Br, I, -OH, -CN, $-NH_2$, -COOH, -N($R_dR_e$), $C_{1-6}$ alkyl, $C_{1-6}$ haloalkyl, $C_{1-6}$ alkoxy or $C_{1-6}$ haloalkoxy;
each $R_d$ and $R_e$ is independently H, deuterium, -OH, $C_{1-6}$ alkyl, -C(=O)H, -C(=O)-O-$C_{1-6}$ alkyl, -C(=O)-$C_{1-6}$ alkyl, -$C_{1-6}$ alkylene-C(=O)-O-$C_{1-6}$ alkyl or -$C_{1-6}$ alkylene-O-$C_{1-6}$ alkyl; wherein, each of the $C_{1-6}$ alkyl, -C(=O)-O-$C_{1-6}$ alkyl, -C(=O)-$C_{1-6}$ alkyl, -$C_{1-6}$ alkylene-C(=O)-O-$C_{1-6}$ alkyl and -$C_{1-6}$ alkylene-O-$C_{1-6}$ alkyl is independently and optionally substituted with 1, 2, 3 or 4 substituents selected from deuterium, F, Cl, Br, I, -OH, -CN, $-NH_2$ or -COOH;
$L_1$ is a bond, **-O-, **-C(=O)-, **-NH-, **-$CH_2$-, **-$C_{1-6}$ alkylene-O-, **-O-$C_{1-6}$ alkylene-, **-C(=O)-N($R_f$)-, **-N($R_f$)-C(=O)-, **-N($R_f$)-$C_{1-6}$ alkylene- or **-$C_{1-6}$ alkylene-N($R_f$)-; wherein, each of the **-$CH_2$-, **-$C_{1-6}$ alkylene-O-, **-O-$C_{1-6}$ alkylene-, **-N($R_f$)-$C_{1-6}$ alkylene- and **-$C_{1-6}$ alkylene-N($R_f$)- is independently and optionally substituted with 1, 2, 3 or 4 substituents selected from deuterium, oxo, halogen, $C_{1-6}$ alkyl or $C_{1-6}$ haloalkyl;
$L_2$ is a bond, -O-, -C(=O)-, -NH-, -$CH_2$-, -$C_{1-6}$ alkylene-O-, -O-$C_{1-6}$ alkylene-, -C(=O)-N($R_f$)-, -N($R_f$)-C(=O)-, -N($R_f$)-$C_{1-6}$ alkylene- or -$C_{1-6}$ alkylene-N($R_f$)-; wherein, each of the -$CH_2$-, -$C_{1-6}$ alkylene-O-, -O-$C_{1-6}$ alkylene-, -N($R_f$)-$C_{1-6}$ alkylene- and -$C_{1-6}$ alkylene-N($R_f$)- is independently and optionally substituted with 1, 2, 3 or 4 substituents selected from deuterium, oxo, halogen, $C_{1-6}$ alkyl or $C_{1-6}$ haloalkyl;
$L_3$ is *-S(=O)$_2$-NH-, *-NH-S(=O)$_2$-, *-S(=O)-NH-, *-NH-S(=O)-, *-C(=O)NH- or *-NHC(=O)-;

each $R_f$ is independently H, deuterium, $C_{1-6}$ alkyl, -$C_{1-6}$ alkylene-O-$C_{1-6}$ alkyl, -$C_{1-6}$ alkylene-(5- to 10-membered heterocyclyl), -$C_{1-6}$ alkylene-$C_{3-8}$ cycloalkyl, $C_{3-8}$ cycloalkyl, 3- to 8-membered heterocyclyl, -C(=O)-(3- to 8-membered heterocyclyl) or -C(=O)-$C_{3-8}$ cycloalkyl; wherein, each of the $C_{1-6}$ alkyl, -$C_{1-6}$ alkylene-O-$C_{1-6}$ alkyl, -$C_{1-6}$ alkylene-(5- to 10-membered heterocyclyl), -$C_{1-6}$ alkylene-$C_{3-8}$ cycloalkyl, $C_{3-8}$ cycloalkyl, 3- to 8-membered heterocyclyl, -C(=O)-(3- to 8-membered heterocyclyl) and -C(=O)-$C_{3-8}$ cycloalkyl is independently and optionally substituted with 1, 2 or 3 substituents selected from deuterium, F, Cl, Br, I, -OH, -CN, -$NH_2$ or -COOH;
m is 0, 1 or 2.

**2.** The compound of claim 1, wherein each of $R_0$, $R_1$, $R_2$, $R_3$, $R_4$, $R_5$ and $R_6$ is independently H, deuterium, F, Cl, Br, I, $C_{1-4}$ alkyl, $C_{1-4}$ haloalkyl, $C_{1-4}$ alkoxy, hydroxy-substituted $C_{1-4}$ alkyl, hydroxy-substituted $C_{1-4}$ haloalkyl, -Si($C_{1-4}$ alkyl)$_3$, $C_{1-4}$ haloalkoxy or -N($R_dR_e$).

**3.** The compound of any one of claims 1 to 2, wherein $R_7$ is -S(=O)$_2$-$C_{1-4}$ alkyl, -S(=O)$_2$-$C_{1-4}$ alkoxy, -S(=O)$_2$-$C_{1-4}$ alkylamino, -S(=O)$_2$-$C_{1-4}$ haloalkyl, -S(=O)$_2$-$C_{3-6}$ cycloalkyl, -S(=O)$_2$-$C_{1-4}$ alkylene-$C_{3-6}$ cycloalkyl, -S(=O)-$C_{1-4}$ alkyl, -S(=O)$_2$H, -COOH, -C(=O)-N($R_gR_h$), -N($R_g$)-C(=O)-$C_{1-4}$ alkyl, -C(=O)-O-$C_{1-4}$ alkyl, $C_{1-4}$ alkyl, $C_{1-4}$ alkoxy, $C_{1-4}$ haloalkyl or $C_{3-6}$ cycloalkyl;
each $R_g$ and $R_h$ is independently H, deuterium or $C_{1-4}$ alkyl.

**4.** The compound of any one of claims 1 to 3, wherein each of A and G is independently cyclopropyl, cyclobutyl, cyclopentyl, cyclohexyl, $C_{6-10}$ aryl, 5- to 10-membered heteroaryl or 5- to 7-membered heterocyclyl; wherein, each of A and G is independently and optionally substituted with 1, 2, 3, 4 or 5 $R_a$.

**5.** The compound of any one of claims 1 to 4, wherein B is 4- to 7-membered heterocyclyl; wherein, the 4- to 7-membered heterocyclyl is optionally substituted with 1, 2, 3, 4 or 5 $R_b$.

**6.** The compound of any one of claims 1 to 5, wherein each $R_a$ and $R_b$ is independently deuterium, F, Cl, Br, I, -OH, -CN, -$NH_2$, -$NO_2$, -COOH, oxo, $C_{1-4}$ alkyl, $C_{1-4}$ haloalkyl, $C_{1-4}$ alkoxy, $C_{1-4}$ haloalkoxy, -$C_{1-4}$ alkylene-O-$C_{1-4}$ alkyl, $C_{3-6}$ cycloalkyl, $C_{6-10}$ aryl, 5- to 7-membered heterocyclyl, 5- to 7-membered heteroaryl or -C(=O)-N($R_dR_e$); wherein, each of the $C_{1-4}$ alkyl, $C_{1-4}$ haloalkyl, $C_{1-4}$ alkoxy, $C_{1-4}$ haloalkoxy, -$C_{1-4}$ alkylene-O-$C_{1-4}$ alkyl, $C_{3-6}$ cycloalkyl, $C_{6-10}$ aryl, 5- to 7-membered heterocyclyl and 5- to 7-membered heteroaryl is independently and optionally substituted with 1, 2 or 3 $R_c$;
each $R_c$ is independently deuterium, F, Cl, Br, I, -OH, -CN, -$NH_2$, $C_{1-4}$ alkyl, $C_{1-4}$ alkoxy, $C_{1-4}$ haloalkyl, $C_{1-4}$ haloalkoxy, $C_{3-6}$ cycloalkyl, 5- to 7-membered heterocyclyl, $C_{6-10}$ aryl or 5- to 7-membered heteroaryl.

**7.** The compound of any one of claims 1 to 6, wherein $R_5$ is H, deuterium, -OH, -CN, -$NH_2$, -$NO_2$, -COOH, $C_{1-4}$ alkyl, $C_{1-4}$ alkoxy, $C_{1-4}$ haloalkyl, cyano-substituted $C_{1-4}$ alkyl, carboxy-substituted $C_{1-4}$ alkyl, -$C_{1-4}$ alkylene-O-$C_{1-4}$ alkyl, -$C_{1-4}$ alkylene-C (=O)-O-$C_{1-4}$ alkyl, -$C_{1-4}$ alkylene-C(=O)-N($R_dR_e$), -$C_{1-4}$ alkylene-OC(=O)-N($R_dR_e$), -$C_{1-4}$ alkylene-N($R_f$)-C(=O)-N($R_dR_e$), -$C_{1-4}$ alkylene-N($R_dR_e$) or -N($R_f$)-C(=O)- $C_{1-4}$ alkyl;

$R_9$ is deuterium, -OH, -CN, -$NH_2$, -$NO_2$, -COOH, $C_{1-4}$ alkoxy, $C_{1-4}$ haloalkyl, cyano-substituted $C_{1-4}$ alkyl, carboxy-substituted $C_{1-4}$ alkyl, -$C_{1-4}$ alkylene-O-$C_{1-4}$ alkyl, -$C_{1-4}$ alkylene-C(=O)-O-$C_{1-4}$ alkyl, -$C_{1-4}$ alkylene-C(=O)-N($R_dR_e$), -$C_{1-4}$ alkylene-OC(=O)-N($R_dR_e$), -$C_{1-4}$ alkylene-N($R_f$)-C(=O)-N($R_dR_e$), -$C_{1-4}$ alkylene-N($R_dR_e$) or -N($R_f$)-C(=O)-$C_{1-4}$ alkyl;
wherein, each of the $C_{1-4}$ alkyl, $C_{1-4}$ alkoxy, $C_{1-4}$ haloalkyl, cyano-substituted $C_{1-4}$ alkyl, carboxy-substituted $C_{1-4}$ alkyl, -$C_{1-4}$ alkylene-O-$C_{1-4}$ alkyl and -$C_{1-4}$ alkylene-C(=O)-O-$C_{1-4}$ alkyl described in $R_8$ and $R_9$ is independently and optionally substituted with 1, 2, 3 or 4 substituents selected from deuterium, F, Cl, Br, I, -OH, -CN, -$NH_2$, -COOH, -N($R_dR_e$), $C_{1-4}$ alkyl, $C_{1-4}$ haloalkyl, $C_{1-4}$ alkoxy or $C_{1-4}$ haloalkoxy;
or, $R_8$ and $R_9$ together with the carbon atom to which they are attached, form $C_{3-6}$ cycloalkyl or 3- to 6-membered heterocyclyl; wherein, each of the $C_{3-6}$ cycloalkyl and 3- to 6-membered heterocyclyl is independently and optionally substituted with 1, 2, 3 or 4 substituents selected from deuterium, F, Cl, Br, I, -OH, -CN, -$NH_2$, -COOH, -N($R_dR_e$), $C_{1-4}$ alkyl, $C_{1-4}$ haloalkyl, $C_{1-4}$ alkoxy or $C_{1-4}$ haloalkoxy.

**8.** The compound of any one of claims 1 to 7, wherein each $R_d$ and $R_e$ is independently H, deuterium, -OH, $C_{1-4}$ alkyl, -C(=O)H, -C(=O)-O-$C_{1-4}$ alkyl, -C(=O)-$C_{1-4}$ alkyl, -$C_{1-4}$ alkylene-C(=O)-O-$C_{1-4}$ alkyl or -$C_{1-4}$ alkylene-O-$C_{1-4}$ alkyl; wherein, each of the $C_{1-4}$ alkyl, -C(=O)-O-$C_{1-4}$ alkyl, -C(=O)-$C_{1-4}$ alkyl, -$C_{1-4}$ alkylene-C(=O)-O-$C_{1-4}$ alkyl and -$C_{1-4}$ alkylene-O-$C_{1-4}$ alkyl is independently and optionally substituted with 1, 2, 3 or 4 substituents selected from deuterium, F, Cl, Br, I, -OH, -CN, -$NH_2$ or -COOH.

**9.** The compound of any one of claims 1 to 8, wherein $L_1$ is a bond, **-O-, **-C(=O)-, **-NH-, **-$CH_2$-, **-O-$C_{1-3}$ alkylene-, **-$C_{1-3}$ alkylene-O-, **-N($R_f$)-C(=O)-, **-C(=O)-N($R_f$)-, **-N($R_f$)-$C_{1-3}$ alkylene- or **-$C_{1-3}$ alkylene-N($R_f$)-; wherein, each of the **-$CH_2$-, * *-O-$C_{1-3}$ alkylene-, **-$C_{1-3}$ alkylene-O-, **-N($R_f$)-$C_{1-3}$ alkylene- and **-$C_{1-3}$ alkylene-N($R_f$)- is independently and optionally substituted with 1, 2, 3 or 4 substituents selected from deuterium, oxo, halogen, $C_{1-4}$ alkyl or $C_{1-4}$ haloalkyl;

$L_2$ is a bond, -O-, -C(=O)-, -NH-, -$CH_2$-, -O-$C_{1-3}$ alkylene-, -$C_{1-3}$ alkylene-O-, -N($R_f$)-C(=O)-, -C(=O)-N($R_f$)-, -N($R_f$)-$C_{1-3}$ alkylene- or -$C_{1-3}$ alkylene-N($R_f$)-; wherein, each of the -O-$C_{1-3}$ alkylene-, -$C_{1-3}$ alkylene-O-, -N($R_f$)-$C_{1-3}$ alkylene- and -$C_{1-3}$ alkylene-N($R_f$)- is independently and optionally substituted with 1, 2, 3 or 4 substituents selected from deuterium, oxo, halogen, $C_{1-4}$ alkyl or $C_{1-4}$ haloalkyl;
each $R_f$ is independently H, deuterium, $C_{1-4}$ alkyl, -$C_{1-4}$ alkylene-O-$C_{1-4}$ alkyl, -$C_{1-4}$ alkylene-(5- to 7- membered heterocyclyl), -$C_{1-4}$ alkylene-$C_{3-6}$ cycloalkyl, $C_{3-6}$ cycloalkyl, 3- to 6-membered heterocyclyl, -C(=O)-(3- to 6-membered heterocyclyl) or -C(=O)-$C_{3-6}$ cycloalkyl; wherein, each of the $C_{1-4}$ alkyl, -$C_{1-4}$ alkylene-O-$C_{1-4}$ alkyl, -$C_{1-4}$ alkylene-(5- to 7- membered heterocyclyl), -$C_{1-4}$ alkylene-$C_{3-6}$ cycloalkyl, $C_{3-6}$ cycloalkyl, 3- to 6- membered heterocyclyl, -C(=O)-(3- to 6- membered heterocyclyl) and -C(=O)-$C_{3-6}$ cycloalkyl is independently and optionally substituted with 1, 2 or 3 substituents selected from deuterium, F, Cl, Br, I, -OH, -CN, -$NH_2$ or -COOH.

**10.** The compound of any one of claims 1 to 9, wherein each of $R_0$, $R_1$, $R_2$, $R_3$, $R_4$, $R_5$ and $R_6$ is independently H, deuterium, F, Cl, Br, I, methyl, ethyl, *n*-propyl, isopropyl, *n*-butyl, tert-butyl, -$CH_2F$, -$CHF_2$, -$CF_3$, -$CH_2CH_2F$, -$CH_2CHF_2$, -$CHFCH_2F$, -$CH_2CF_3$, -$CH(CF_3)_2$, -$CF_2CH_2CH_3$, -$CH_2CH_2CH_2F$, -$CH_2CH_2CHF_2$, -$CH_2CH_2CF_3$, methoxy, ethoxy, *n*-propoxy, isopropoxy, *n*-butoxy, *tert*-butoxy, hydroxymethyl, 2-hydroxyethyl, 2-hydroxypropyl, 3-hydroxypropyl, -$C(OH)(CF_3)_2$, -$Si(CH_3)_3$, -$Si(CH_2CH_3)_3$, -$OCH_2F$, -$OCHF_2$, -$OCF_3$, -$OCH_2CH_2F$, -$OCH_2CHF_2$, -$OCHFCH_2F$, -$OCH_2CF_3$, -$OCH(CF_3)_2$, -$OCF_2CH_2CH_3$, -$OCH_2CH_2CH_2F$, -$OCH_2CH_2CHF_2$, -$OCH_2CH_2CF_3$ or -N($R_dR_e$).

**11.** The compound according to any one of claims 1 to 10, wherein, $R_7$ is -S(=O)$_2$-$CH_3$, -S(=O)$_2$-$CH_2CH_3$, -S(=O)$_2$-$CH_2CH_2CH_3$, -S(=O)$_2$-$CH(CH_3)CH_3$, -S(=O)$_2$-$OCH_3$, -S(=O)$_2$-$OCH_2CH_3$, -S(=O)$_2$-$OCH_2CH_2CH_3$, -S(=O)$_2$-$OCH(CH_3)CH_3$, -S(=O)$_2$-cyclopropyl, -S(=O)$_2$-cyclobutyl, -S(=O)$_2$-cyclopentyl, -S(=O)$_2$-cyclohexyl, -S(=O)-$CH_2$-cyclopropyl, -S(=O)-$CH_2$-cyclobutyl, -S(=O)-$CH_2$-cyclopentyl, -S(=O)-$CH_2$-cyclohexyl, -S(=O)-$CH_3$, -S(=O)-$CH_2CH_3$, -S(=O)-$CH_2CH_2CH_3$, -S(=O)-$CH(CH_3)CH_3$, -S(=O)$_2$H, -COOH, -C(=O)-N($R_gR_h$), -N($R_g$)-C(=O)-$CH_3$, -N($R_g$)-C(=O)-$CH_2CH_3$, -N($R_g$)-C(=O)-$CH_2CH_2CH_3$, -N($R_g$)-C(=O)-$CH(CH_3)CH_3$, -C(=O)-O-$CH_3$, -C(=O)-O-$CH_2CH_3$, -C(=O)-O-$CH_2CH_2CH_3$, -C(=O)-O-$CH(CH_3)CH_3$, methyl, ethyl, *n*-propyl, isopropyl, methoxy, ethoxy, *n*-propoxy, *iso*-propoxy, -$CH_2F$, -$CHF_2$, -$CF_3$, -$CH_2CH_2F$, -$CH_2CHF_2$, -$CHFCH_2F$, -$CH_2CF_3$, -$CH(CF_3)_2$, -$CF_2CH_2CH_3$, -$CH_2CH_2CH_2F$, -$CH_2CH_2CHF_2$ or -$CH_2CH_2CF_3$;
each $R_g$ and $R_h$ is independently H, deuterium, methyl, ethyl, *n*-propyl, isopropyl, *n*-butyl, isobutyl, *sec*-butyl or tert-butyl.

**12.** The compound of any one of claims 1 to 11, wherein each of A and G is independently cyclopropyl, cyclobutyl, cyclopentyl, cyclohexyl, phenyl, naphthyl, thiazolyl, pyrazolyl, imidazolyl, furanyl, oxazolyl, isoxazolyl, triazolyl, thienyl, pyrrolyl, pyridyl, pyrimidinyl, morpholinyl, thiomorpholinyl, tetrahydrofuranyl, tetrahydropyranyl, pyrrolidinyl, piperidinyl, piperazinyl,

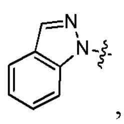, 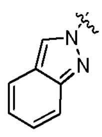, 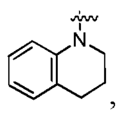, 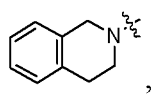, 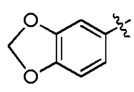 or 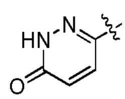;

wherein, each of A and G is independently and optionally substituted with 1, 2, 3, 4 or 5 $R_a$.

**13.** The compound of any one of claims 1 to 12, wherein B is

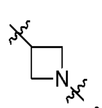, 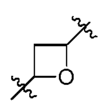, 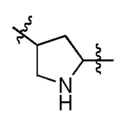,

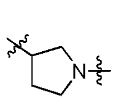, 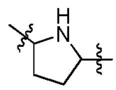, 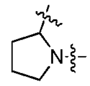, 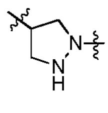, 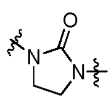, 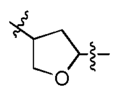, 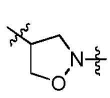, 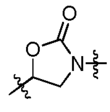, 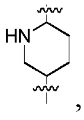, 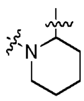,

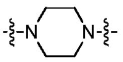, 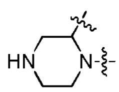, 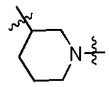, 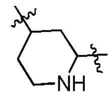, 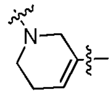, 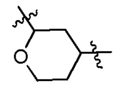, 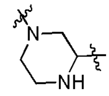, 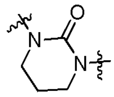, 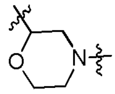,

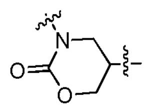, 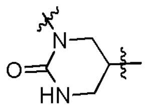, 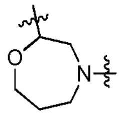, 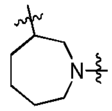, 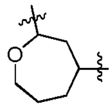, 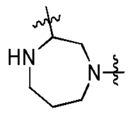 or 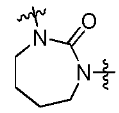;

wherein B is optionally substituted with 1, 2, 3, 4 or 5 $R_b$.

**14.** The compound of any one of claims 1 to 13, wherein each $R_a$ and $R_b$ is independently deuterium, F, Cl, Br, I, -OH, -CN, $-NH_2$, $-NO_2$, -COOH, oxo, methyl, ethyl, *n*-propyl, isopropyl, *n*-butyl, tert-butyl, $-CH_2F$, $-CHF_2$, $-CF_3$, $-CH_2CH_2F$, $-CH_2CHF_2$, $-CHFCH_2F$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, methoxy, ethoxy, *n*-propoxy, isopropoxy, *n*-butoxy, *tert*-butoxy, $-OCH_2F$, $-OCHF_2$, $-OCF_3$, $-OCH_2CH_2F$, $-OCH_2CHF_2$, $-OCHFCH_2F$, $-OCH_2CF_3$, $-OCH(CF_3)_2$, $-OCF_2CH_2CH_3$, $-OCH_2CH_2CH_2F$, $-OCH_2CH_2CHF_2$, $-OCH_2CH_2CF_3$, $-CH_2OCH_3$, $-CH_2OCH_2CH_3$, $-CH_2OCH_2CH_2CH_3$, $-CH_2OCH(CH_3)_2$, $-CH_2CH_2OCH_3$, $-CH_2CH_2OCH_2CH_3$, $-CH_2CH_2OCH_2CH_2CH_3$, $-CH_2CH_2OCH(CH_3)_2$, $-CH_2CH_2CH_2OCH_3$, $-CH_2CH_2CH_2OCH_2CH_3$, $-CH_2CH_2CH_2OCH_2CH_2CH_3$, $-CH_2CH_2CH_2OCH(CH_3)_2$, cyclopropyl, cyclobutyl, cyclopentyl, cyclohexyl, phenyl, naphthyl, pyridyl, pyrimidinyl, pyrrolyl, pyrazolyl, thiazolyl, imidazolyl, oxazolyl, triazolyl, tetrazolyl, piperazinyl, piperidinyl, morpholinyl, thiomorpholinyl, tetrahydropyranyl, pyrrolidinyl or $-C(=O)-N(R_dR_e)$;
wherein, each of methyl, ethyl, *n*-propyl, isopropyl, *n*-butyl, *tert*-butyl, $-CH_2F$, $-CHF_2$, $-CH_2CH_2F$, $-CH_2CHF_2$, $-CHFCH_2F$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, methoxy, ethoxy, *n*-propoxy, isopropoxy, *n*-butoxy, *tert*-butoxy, $-OCH_2F$, $-OCHF_2$, $-OCH_2CH_2F$, $-OCH_2CHF_2$, $-OCHFCH_2F$, $-OCH_2CF_3$, $-OCH(CF_3)_2$, $-OCF_2CH_2CH_3$, $-OCH_2CH_2CH_2F$, $-OCH_2CH_2CHF_2$, $-OCH_2CH_2CF_3$, $-CH_2OCH_3$, $-CH_2OCH_2CH_3$, $-CH_2OCH_2CH_2CH_3$, $-CH_2OCH(CH_3)_2$, $-CH_2CH_2OCH_3$, $-CH_2CH_2OCH_2CH_3$, $-CH_2CH_2OCH_2CH_2CH_3$, $-CH_2CH_2OCH(CH_3)_2$, $-CH_2CH_2CH_2OCH_3$, $-CH_2CH_2CH_2OCH_2CH_3$, $-CH_2CH_2CH_2OCH_2CH_2CH_3$,$-CH_2CH_2CH_2OCH(CH_3)_2$, cyclopropyl, cyclobutyl, cyclopentyl, cyclohexyl, phenyl, naphthyl, pyridyl, pyrimidinyl, pyrrolyl, pyrazolyl, thiazolyl, imidazolyl, oxazolyl, triazolyl, tetrazolyl, piperazinyl, piperidinyl, morpholinyl, thiomorpholinyl, tetrahydropyranyl and pyrrolidinyl is independently and optionally substituted with 1, 2 or 3 $R_c$.

**15.** The compound of any one of claims 1 to 14, wherein each $R_c$ is independently deuterium, F, Cl, Br, I, -OH, -CN, -NH2, methyl, ethyl, *n*-propyl, isopropyl, *n*-butyl, tert-butyl, $-CH_2F$, $-CHF_2$, $-CF_3$, $-CH_2CH_2F$, $-CH_2CHF_2$, $-CHFCH_2F$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, methoxy, ethoxy, *n*-propoxy, isopropoxy, *n*-butoxy, tert-butoxy, $-OCH_2F$, $-OCHF_2$, $-OCF_3$, $-OCH_2CH_2F$, $-OCH_2CHF_2$, $-OCHFCH_2F$, $-OCH_2CF_3$, $-OCH(CF_3)_2$, $-OCF_2CH_2CH_3$, $-OCH_2CH_2CH_2F$, $-OCH_2CH_2CHF_2$, $-OCH_2CH_2CF_3$, cyclopropyl, cyclobutyl, cyclopentyl, cyclohexyl, phenyl, naphthyl, pyridyl, pyrimidinyl, pyrrolyl, pyrazolyl, thiazolyl, imidazolyl, oxazolyl, triazolyl, tetrazolyl, piperazinyl, piperidinyl, morpholinyl, thiomorpholinyl, tetrahydropyranyl or pyrrolidinyl.

**16.** The compound of any one of claims 1 to 15, wherein $R_8$ is H, deuterium, -OH, -CN, $-NH_2$, $-NO_2$, -COOH, methyl, ethyl, *n*-propyl, isopropyl, *n*-butyl, tert-butyl, $-CH_2F$, $-CHF_2$, $-CF_3$, $-CH_2CH_2F$, $-CH_2CHF_2$, $-CHFCH_2F$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, methoxy, ethoxy, *n*-propoxy, isopropoxy, *n*-butoxy, *tert*-butoxy, $-CH_2CN$, $-CH_2CH_2CN$, $-CH_2CH_2CH_2CN$, $-CH(CH_3)CH_2CN$, $-CH_2(CH_2)_3CN$, $-CH_2COOH$, $-CH_2CH_2COOH$, $-CH_2CH_2CH_2COOH$, $-CH(CH_3)CH_2COOH$, $-CH_2(CH_2)_3COOH$, $-CH_2OCH_3$, $-CH_2OCH_2CH_3$, $-CH_2OCH_2CH_2CH_3$, $-CH_2OCH(CH_3)_2$, $-CH_2CH_2OCH_3$, $-CH_2CH_2OCH_2CH_3$, $-CH_2CH_2OCH_2CH_2CH_3$, $-CH_2CH_2OCH(CH_3)_2$, $-CH_2CH_2CH_2OCH_3$, $-CH_2CH_2CH_2OCH_2CH_3$, $-CH_2CH_2CH_2OCH_2CH_2CH_3$,$-CH_2CH_2CH_2OCH(CH_3)_2$, $-CH_2-C(=O)-OCH_3$, $-CH_2-C(=O)-OCH_2CH_3$, $-CH_2-C(=O)-OCH_2CH_2CH_3$, $-CH_2-C(=O)-OCH(CH_3)_2$, $-CH_2CH_2-C(=O)-OCH_3$, $-CH_2CH_2-C(=O)-OCH_2CH_3$, $-CH_2CH_2-C(=O)-OCH_2CH_2CH_3$, $-CH_2CH_2-C(=O)-OCH(CH_3)_2$, $-CH_2CH_2CH_2-C(=O)-OCH_3$, $-CH_2CH_2CH_2-C(=O)-OCH_2CH_3$, $-CH_2CH_2CH_2-C(=O)-OCH_2CH_2CH_3$, $-CH_2CH_2CH_2-C(=O)-OCH(CH_3)_2$,

$-CH_2-C(=O)-N(R_dR_e)$, $-CH_2CH_2-C(=O)-N(R_dR_e)$, $-CH_2CH_2CH_2-C(=O)-N(R_dR_e)$, $-CH_2-O-C(=O)-N(R_dR_e)$, $-CH_2CH_2-O-C(=O)-N(R_dR_e)$, $-CH_2CH_2CH_2-O-C(=O)-N(R_dR_e)$, $-CH_2-N(R_f)-C(=O)-N(R_dR_e)$, $-CH_2CH_2-N(R_f)-C(=O)-N(R_dR_e)$, $-CH_2CH_2CH_2-N(R_f)-C(=O)-N(R_dR_e)$, $-CH_2N(R_dR_e)$, $-CH_2CH_2N(R_dR_e)$, $-CH_2CH_2CH_2N(R_dR_e)$, $-N(R_f)-C(=O)-CH_3$, $-N(R_f)-C(=O)-CH_2CH_3$ or $-N(R_f)-C(=O)-CH(CH_3)_2$;

$R_9$ is deuterium, -OH, -CN, -NH2, $-NO_2$, -COOH, $-CH_2F$, $-CHF_2$, $-CF_3$, $-CH_2CH_2F$, $-CH_2CHF_2$, $-CHFCH_2F$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, methoxy, ethoxy, *n*-propoxy, isopropoxy, *n*-butoxy, tert-butoxy, $-CH_2CN$, $-CH_2CH_2CN$, $-CH_2CH_2CH_2CN$, $-CH(CH_3)CH_2CN$, $-CH_2(CH_2)_3CN$, $-CH_2COOH$, $-CH_2CH_2COOH$, $-CH_2CH_2CH_2COOH$, $-CH(CH_3)CH_2COOH$, $-CH_2(CH_2)_3COOH$, $-CH_2OCH_3$, $-CH_2OCH_2CH_3$, $-CH_2OCH_2CH_2CH_3$, $-CH_2OCH(CH_3)_2$, $-CH_2CH_2OCH_3$, $-CH_2CH_2OCH_2CH_3$, $-CH_2CH_2OCH_2CH_2CH_3$, $-CH_2CH_2OCH(CH_3)_2$, $-CH_2CH_2CH_2OCH_3$, $-CH_2CH_2CH_2OCH_2CH_3$, $-CH_2CH_2CH_2OCH_2CH_2CH_3$,$-CH_2CH_2CH_2OCH(CH_3)_2$, $-CH_2-C(=O)-OCH_3$, $-CH_2-C(=O)-OCH_2CH_3$, $-CH_2-C(=O)-OCH_2CH_2CH_3$, $-CH_2-C(=O)-OCH(CH_3)_2$, $-CH_2CH_2-C(=O)-OCH_3$, $-CH_2CH_2-C(=O)-OCH_2CH_3$, $-CH_2CH_2-C(=O)-OCH_2CH_2CH_3$, $-CH_2CH_2-C(=O)-OCH(CH_3)_2$, $-CH_2CH_2CH_2-C(=O)-OCH_3$, $-CH_2CH_2CH_2-C(=O)-OCH_2CH_3$, $-CH_2CH_2CH_2-C(=O)-OCH_2CH_2CH_3$, $-CH_2CH_2CH_2-C(=O)-OCH(CH_3)_2$, $-CH_2-C(=O)-N(R_dR_e)$, $-CH_2CH_2-C(=O)-N(R_dR_e)$, $-CH_2CH_2CH_2-C(=O)-N(R_dR_e)$, $-CH_2-O-C(=O)-N(R_dR_e)$, $-CH_2CH_2-O-C(=O)-N(R_dR_e)$, $-CH_2CH_2CH_2-O-C(=O)-N(R_dR_e)$, $-CH_2-N(R_f)-C(=O)-N(R_dR_e)$, $-CH_2CH_2-N(R_f)-C(=O)-N(R_dR_e)$, $-CH_2CH_2CH_2-N(R_f)-C(=O)-N(R_dR_e)$, $-CH_2N(R_dR_e)$, $-CH_2CH_2N(R_dR_e)$, $-CH_2CH_2CH_2N(R_dR_e)$, $-N(R_f)-C(=O)-CH_3$, $-N(R_f)-C(=O)-CH_2CH_3$ or $-N(R_f)-C(=O)-CH(CH_3)_2$;
wherein, each of methyl, ethyl, *n*-propyl, isopropyl, *n*-butyl, *tert*-butyl, $-CH_2F$, $-CHF_2$, $-CH_2CH_2F$, $-CH_2CHF_2$, $-CHFCH_2F$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, methoxy, ethoxy, *n*-propoxy, isopropoxy, *n*-butoxy, *tert*-butoxy, $-CH_2CN$, $-CH_2CH_2CN$, $-CH_2CH_2CH_2CN$, $-CH(CH_3)CH_2CN$, $-CH_2(CH_2)_3CN$, $-CH_2COOH$, $-CH_2CH_2COOH$, $-CH_2CH_2CH_2COOH$, $-CH(CH_3)CH_2COOH$, $-CH_2(CH_2)_3COOH$, $-CH_2OCH_3$, $-CH_2OCH_2CH_3$, $-CH_2OCH_2CH_2CH_3$, $-CH_2OCH(CH_3)_2$, $-CH_2CH_2OCH_3$, $-CH_2CH_2OCH_2CH_3$, $-CH_2CH_2OCH_2CH_2CH_3$, $-CH_2CH_2OCH(CH_3)_2$, $-CH_2CH_2CH_2OCH_3$, $-CH_2CH_2CH_2OCH_2CH_3$, $-CH_2CH_2CH_2OCH_2CH_2CH_3$,$-CH_2CH_2CH_2OCH(CH_3)_2$, $-CH_2-C(=O)-OCH_3$, $-CH_2-C(=O)-OCH_2CH_3$, $-CH_2-C(=O)-OCH_2CH_2CH_3$, $-CH_2-C(=O)-OCH(CH_3)_2$, $-CH_2CH_2-C(=O)-OCH_3$, $-CH_2CH_2-C(=O)-OCH_2CH_3$, $-CH_2CH_2-C(=O)-OCH_2CH_2CH_3$, $-CH_2CH_2-C(=O)-OCH(CH_3)_2$, $-CH_2CH_2CH_2-C(=O)-OCH_3$, $-CH_2CH_2CH_2-C(=O)-OCH_2CH_3$, $-CH_2CH_2CH_2-C(=O)-OCH_2CH_2CH_3$ and $-CH_2CH_2CH_2-C(=O)-OCH(CH_3)_2$ described in $R_8$ and $R_9$ is independently and optionally substituted with 1, 2, 3 or 4 substituents selected from deuterium, F, Cl, Br, I, -OH, -CN, $-NH_2$, -COOH, methyl, ethyl, *n*-propyl, isopropyl, *n*-butyl, *tert-butyl,* trifluoromethyl, methoxy, ethoxy, *n*-propoxy, isopropoxy, *n*-butoxy, tert-butoxy, trifluoromethoxy or $-N(R_dR_e)$.
or, Rs and $R_9$ together with the carbon atom to which they are attached, form cyclopentyl, cyclohexyl, oxetanyl, tetrahydrofuranyl, tetrahydropyranyl, azetidinyl, pyrrolidinyl, piperidinyl or piperazinyl; wherein, each of the cyclopentyl, cyclohexyl, oxetanyl, tetrahydrofuranyl, tetrahydropyranyl, azetidinyl, pyrrolidinyl, piperidinyl and piperazinyl is independently and optionally substituted with 1, 2, 3 or 4 substituents selected from deuterium, F, Cl, Br, I, -OH, -CN, $-NH_2$, -COOH, methyl, ethyl, *n*-propyl, isopropyl, *n*-butyl, tert-butyl, trifluoromethyl, methoxy, ethoxy, *n*-propoxy, isopropoxy, *n*-butoxy, *tert*-butoxy, trifluoromethoxy or $-N(R_dR_e)$.

**17.** The compound of any one of claims 1 to 16, wherein each $R_d$ and $R_e$ is independently H, deuterium, -OH, methyl, ethyl, *n*-propyl, isopropyl, *n*-butyl, tert-butyl, -C(=O)H, $-C(=O)-O-CH_3$, $-C(=O)-O-CH_2CH_3$, $-C(=O)-O-CH_2CH_2CH_3$, $-C(=O)-O-CH(CH_3)_2$, $-C(=O)-CH_3$, $-C(=O)-CH_2CH_3$, $-C(=O)-CH_2CH_2CH_3$, $-C(=O)-CH(CH_3)_2$, $-CH_2OCH_3$, $-CH_2OCH_2CH_3$, $-CH_2OCH_2CH_2CH_3$, $-CH_2OCH(CH_3)_2$, $-CH_2CH_2OCH_3$, $-CH_2CH_2OCH_2CH_3$, $-CH_2CH_2OCH_2CH_2CH_3$, $-CH_2CH_2OCH(CH_3)_2$, $-CH_2CH_2CH_2OCH_3$, $-CH_2CH_2CH_2OCH_2CH_3$, $-CH_2CH_2CH_2OCH_2CH_2CH_3$, $-CH_2CH_2CH_2OCH(CH_3)_2$, $-CH_2-C(=O)-OCH_3$, $-CH_2-C(=O)-OCH_2CH_3$, $-CH_2-C(=O)-OCH_2CH_2CH_3$, $-CH_2-C(=O)-OCH(CH_3)_2$, $-CH_2CH_2-C(=O)-OCH_3$, $-CH_2CH_2-C(=O)-OCH_2CH_3$, $-CH_2CH_2-C(=O)-OCH_2CH_2CH_3$, $-CH_2CH_2-C(=O)-OCH(CH_3)_2$, $-CH_2CH_2CH_2-C(=O)-OCH_3$, $-CH_2CH_2CH_2-C(=O)-OCH_2CH_3$, $-CH_2CH_2CH_2-C(=O)-OCH_2CH_2CH_3$ or $-CH_2CH_2CH_2-C(=O)-OCH(CH_3)_2$;
wherein, each of the methyl, ethyl, *n*-propyl, isopropyl, *n*-butyl, *tert*-butyl, $-C(=O)-O-CH_3$, $-C(=O)-O-CH_2CH_3$, $-C(=O)-O-CH_2CH_2CH_3$, $-C(=O)-O-CH(CH_3)_2$, $-C(=O)-CH_3$, $-C(=O)-CH_2CH_3$, $-C(=O)-CH_2CH_2CH_3$, $-C(=O)-CH(CH_3)_2$, $-CH_2OCH_3$, $-CH_2OCH_2CH_3$, $-CH_2OCH_2CH_2CH_3$, $-CH_2OCH(CH_3)_2$, $-CH_2CH_2OCH_3$, $-CH_2CH_2OCH_2CH_3$, $-CH_2CH_2OCH_2CH_2CH_3$, $-CH_2CH_2OCH(CH_3)_2$, $-CH_2CH_2CH_2OCH_3$, $-CH_2CH_2CH_2OCH_2CH_3$, $-CH_2CH_2CH_2OCH_2CH_2CH_3$, $-CH_2CH_2CH_2OCH(CH_3)_2$, $-CH_2-C(=O)-OCH_3$, $-CH_2-C(=O)-OCH_2CH_3$, $-CH_2-C(=O)-OCH_2CH_2CH_3$, $-CH_2-C(=O)-OCH(CH_3)_2$, $-CH_2CH_2-C(=O)-OCH_3$, $-CH_2CH_2-C(=O)-OCH_2CH_3$, $-CH_2CH_2-C(=O)-OCH_2CH_2CH_3$, $-CH_2CH_2-C(=O)-OCH(CH_3)_2$, $-CH_2CH_2CH_2-C(=O)-OCH_3$, $-CH_2CH_2CH_2-C(=O)-OCH_2CH_3$, $-CH_2CH_2CH_2-C(=O)-OCH_2CH_2CH_3$ and $-CH_2CH_2CH_2-C(=O)-OCH(CH_3)_2$ is independently and optionally substituted with 1, 2, 3 or 4 substituents selected from deuterium, F, Cl, Br, I, -OH, -CN,

$-NH_2$ or -COOH.

**18.** The compound of any one of claims 1 to 17, $L_1$ is a bond, **-O-, **-C(=O)-, **-NH-, **-$CH_2$-, **-$CH_2O$-, **-$CH_2CH_2O$-, **-O-$CH_2$-, **-O-$CH_2CH_2$-, **-C(=O)-N($R_f$)-, **-N($R_f$)-C(=O)-, **-N($R_f$)-$CH_2$-, **-N($R_f$)-$CH_2CH_2$-, **-$CH_2$-N($R_f$)- or **-$CH_2CH_2$-N($R_f$)-; wherein, each of the **-$CH_2$-, **-$CH_2O$-, **-$CH_2CH_2O$-, **-O-$CH_2$-, **-O-$CH_2CH_2$-, **-N($R_f$)-$CH_2$-, **-N($R_f$)-$CH_2CH_2$-, **-$CH_2$-N($R_f$)- and **-$CH_2CH_2$-N($R_f$)- is independently and optionally substituted with 1, 2, 3 or 4 substituents selected from deuterium, oxo, F, Cl, Br, methyl, ethyl, *n*-propyl, isopropyl, *n*-butyl, isobutyl, *tert*-butyl, $-CH_2F$, $-CHF_2$, $-CF_3$, $-CH_2CH_2F$, $-CH_2CHF_2$, $-CHFCH_2F$, $-CH_2CF_3$, $-CH(CF_3)_2$, $-CF_2CH_2CH_3$, $-CH_2CH_2CH_2F$, $-CH_2CH_2CHF_2$ or $-CH_2CH_2CF_3$;

$L_2$ is a bond, -O-, -C(=O)-, -NH-, $-CH_2$-, $-CH_2O$-, $-CH_2CH_2O$-, -O-$CH_2$-, -O-$CH_2CH_2$-, -C(=O)-N($R_f$)-, -N($R_f$)-C(=O)-, -N($R_f$)-$CH_2$-, -N($R_f$)-$CH_2CH_2$-, $-CH_2$-N($R_f$)- or $-CH_2CH_2$-N($R_f$)-; wherein, each of the $-CH_2$-, $-CH_2O$-, $-CH_2CH_2O$-, -O-$CH_2$-, -O-$CH_2CH_2$-, -N($R_f$)-$CH_2$-, -N($R_f$)-$CH_2CH_2$-, $-CH_2$-N($R_f$)- and $-CH_2CH_2$-N($R_f$)- is independently and optionally substituted with 1, 2, 3 or 4 substituents selected from deuterium, oxo, F, Cl, Br, methyl, ethyl, *n*-propyl, isopropyl, *n*-butyl, isobutyl, tert-butyl, $-CH_2F$, $-CHF_2$, $-CF_3$, $-CH_2CH_2F$, $-CH_2CHF_2$, $-CHFCH_2F$, $-CH_2CF_3$, $-CH(CF_3)_2$, $-CF_2CH_2CH_3$, $-CH_2CH_2CH_2F$, $-CH_2CH_2CHF_2$ or $-CH_2CH_2CF_3$;

each $R_f$ is independently H, deuterium, methyl, ethyl, *n*-propyl, isopropyl, *n*-butyl, isobutyl, *tert*-butyl, $-CH_2OCH_3$, $-CH_2OCH_2CH_3$, $-CH_2OCH_2CH_2CH_3$, $-CH_2OCH(CH_3)_2$, $-CH_2CH_2OCH_3$, $-CH_2CH_2OCH_2CH_3$, $-CH_2CH_2OCH_2CH_2CH_3$, $-CH_2CH_2OCH(CH_3)_2$, $-CH_2CH_2CH_2OCH_3$, $-CH_2CH_2CH_2OCH_2CH_3$, $-CH_2CH_2CH_2OCH_2CH_2CH_3$,$-CH_2CH_2CH_2OCH(CH_3)_2$, tetrahydrofurylmethylene, tetrahydropyranylmethylene, pyrrolidinylmethylene, piperazinylmethylene, cyclopropylmethylene, cyclopropylethylene, cyclopropyl-*n*-propylene, cyclobutylmethylene, cyclobutylethylene, cyclobutyl-*n*-propylene, cyclopentylmethylene, cyclopentylethylene, cyclopentyl-*n*-propylene, cyclohexylmethylene, cyclohexylethylene, cyclohexyl-*n*-propylene, cyclopropyl, cyclobutyl, cyclopentyl, cyclohexyl, oxiranyl, aziridinyl, oxetanyl, azetidinyl, pyrrolidinyl, tetrahydrofuranyl, tetrahydrothienyl, piperazinyl, piperidinyl, morpholinyl, thiomorpholinyl, tetrahydropyranyl, -C(=O)-cyclopropyl, -C(=O)-cyclobutyl, -C(= O)-cyclopentyl or -C(=O)-cyclohexyl; wherein, each of the methyl, ethyl, *n*-propyl, isopropyl, *n*-butyl, isobutyl, tert-butyl, $-CH_2OCH_3$, $-CH_2OCH_2CH_3$, $-CH_2OCH_2CH_2CH_3$, $-CH_2OCH_2(CH_3)_2$, $-CH_2CH_2OCH_3$, $-CH_2CH_2OCH_2CH_3$, $-CH_2CH_2OCH_2CH_2CH_3$, $-CH_2CH_2OCH(CH_3)_2$, $-CH_2CH_2CH_2OCH_3$, $-CH_2CH_2CH_2OCH_2CH_3$, $-CH_2CH_2CH_2OCH_2CH_2CH_3$, $-CH_2CH_2CH_2OCH(CH_3)_2$, tetrahydrofurylmethylene, tetrahydropyranylmethylene, pyrrolidinylmethylene, piperazinylmethylene, cyclopropylmethylene, cyclopropylethylene, cyclopropyl-*n*-propylene, cyclobutylmethylene, cyclobutylethylene, cyclobutyl-*n*-propylene, cyclopentylmethylene, cyclopentylethylene, cyclopentyl-*n*-propylene, cyclohexylmethylene, cyclohexylethylene, cyclohexyl-*n*-propylene, cyclopropyl, cyclobutyl, cyclopentyl, cyclohexyl, oxiranyl, aziridinyl, oxetanyl, azetidinyl, pyrrolidinyl, tetrahydrofuranyl, tetrahydrothienyl, piperazinyl, piperidinyl, morpholinyl, thiomorpholinyl, tetrahydropyranyl, -C(=O)-cyclopropyl, -C(=O)-cyclobutyl, -C(=O)-cyclopentyl and -C(=O)-cyclohexyl is independently and optionally substituted with 1, 2 or 3 substituents selected from deuterium, F, Cl, Br, I, -OH, -CN, $-NH_2$ or -COOH.

**19.** The compound of any one of claims 1 to 18 having Formula (II), or a stereoisomer, a geometric isomer, a tautomer, an *N*-oxide, a hydrate, a solvate, a metabolite, an ester, a pharmaceutically acceptable salt or a prodrug thereof,

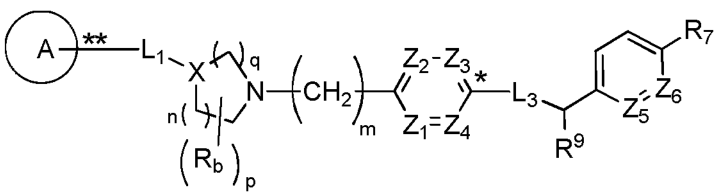

(II),

wherein, n is 0, 1 or 2; p is 0, 1, 2, 3 or 4; q is 1 or 2; X is N or CH.

**20.** The compound of any one of claims 1 to 19 having Formula (III), or a stereoisomer, a geometric isomer, a tautomer, an *N*-oxide, a hydrate, a solvate, a metabolite, an ester, a pharmaceutically acceptable salt or a prodrug thereof,

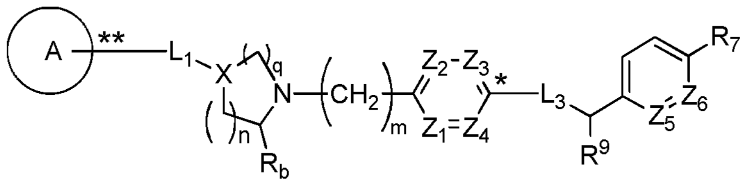

(III),

wherein, n is 0, 1 or 2; q is 1 or 2; X is N or CH.

**21.** The compound of any one of claims 1 to 20 having Formula (IV) or Formula (V), or a stereoisomer, a geometric isomer, a tautomer, an *N*-oxide, a hydrate, a solvate, a metabolite, an ester, a pharmaceutically acceptable salt or a prodrug thereof,

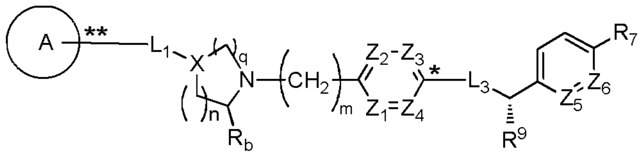

(IV) or

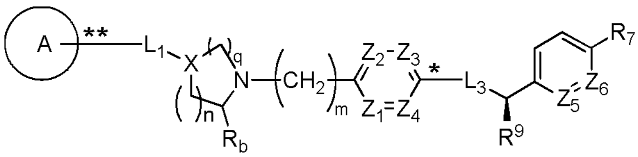

(V),

wherein, n is 0, 1 or 2; q is 1 or 2; X is N or CH.

**22.** A compound having one of the following structures or a stereoisomer, a geometric isomer, a tautomer, an *N*-oxide, a hydrate, a solvate, a metabolite, an ester, a pharmaceutically acceptable salt or a prodrug thereof,

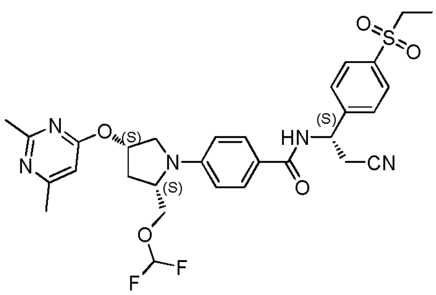

(1),

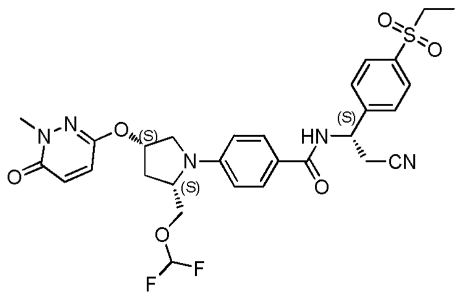

(2),

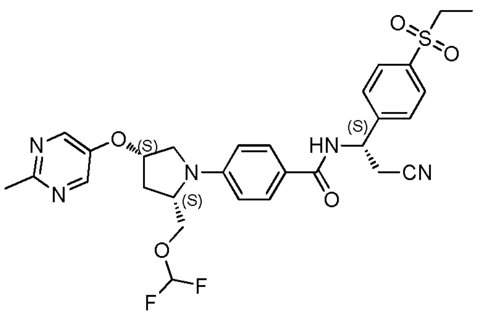

(3),

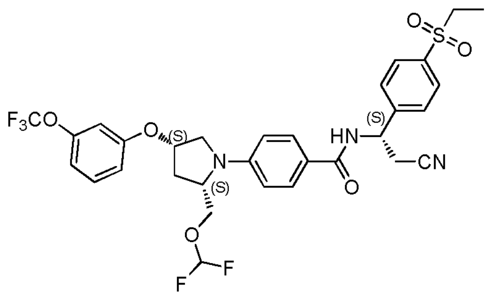

(4),

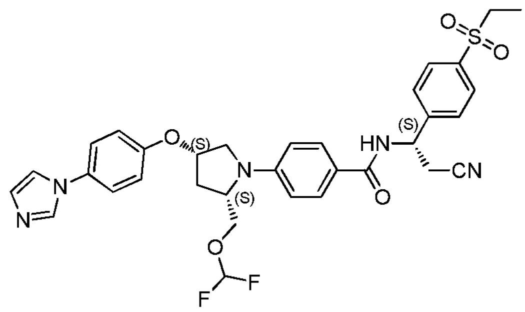

(5),

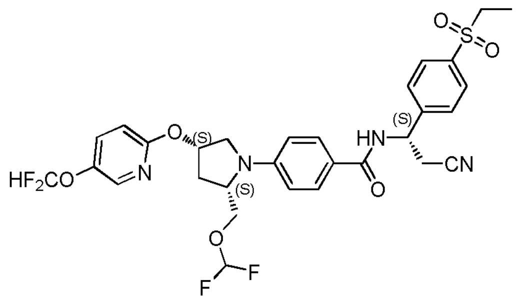

(6),

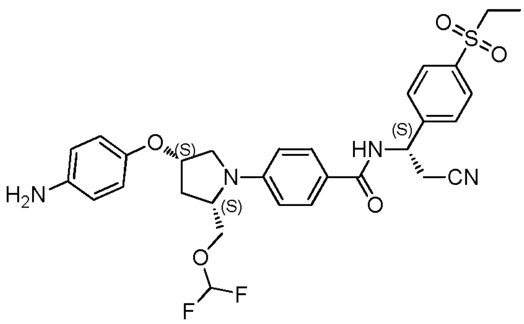

(7),

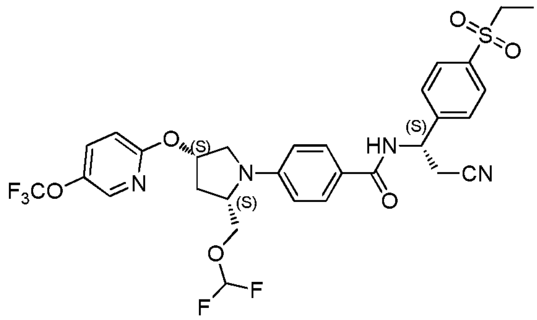

(8),

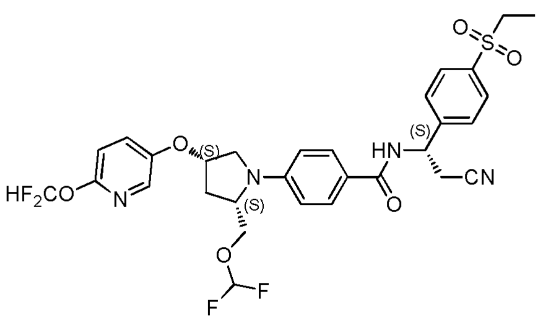

(9),

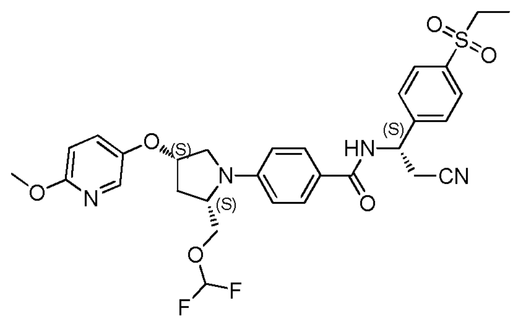

(10),

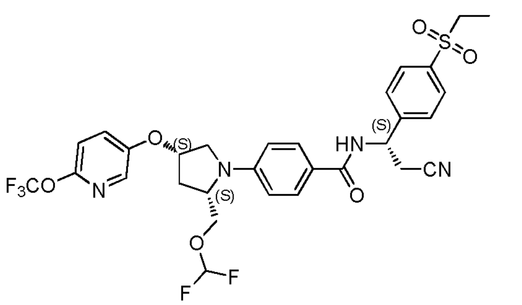

(11),

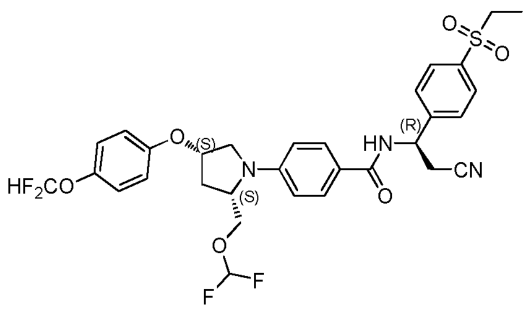

(12),

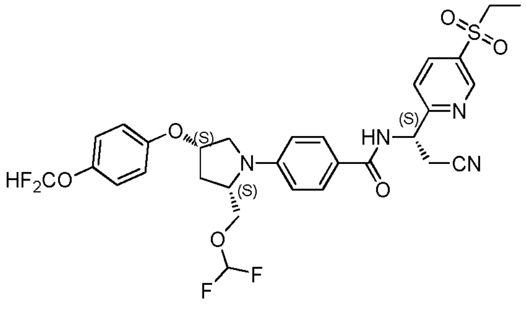

(13),

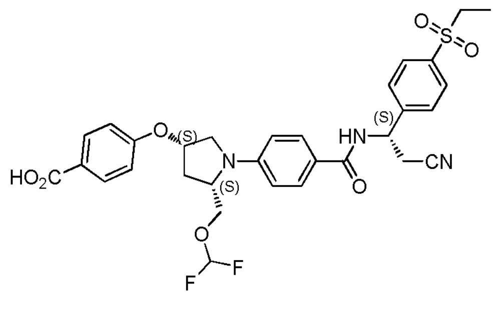

(14),

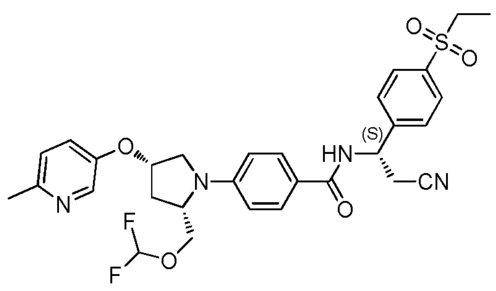
(15),

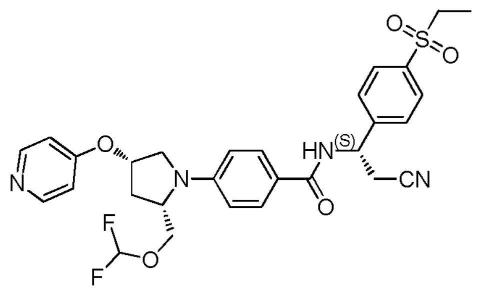
(16),

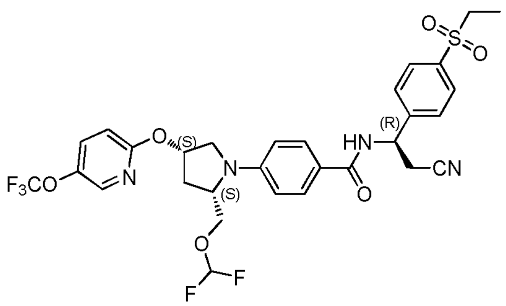
(17),

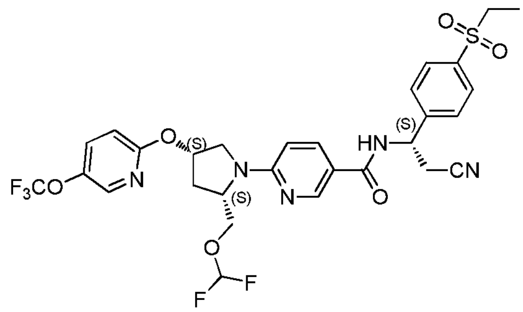
(18),

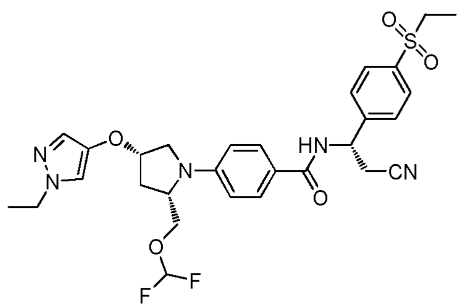
(19),

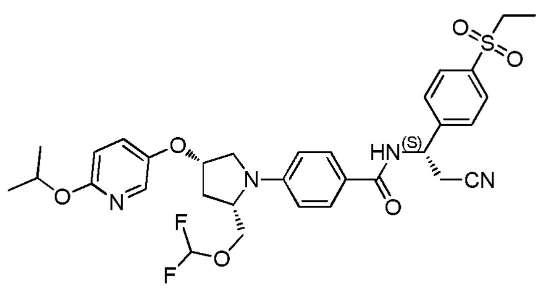
(20),

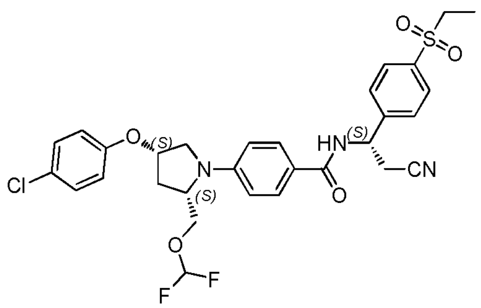
(21),

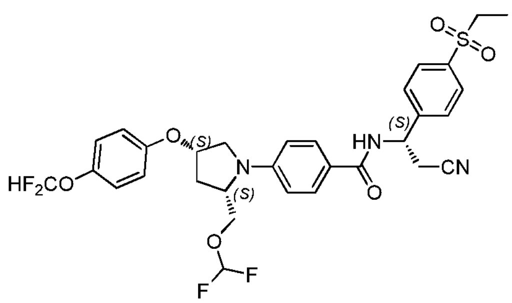
(22),

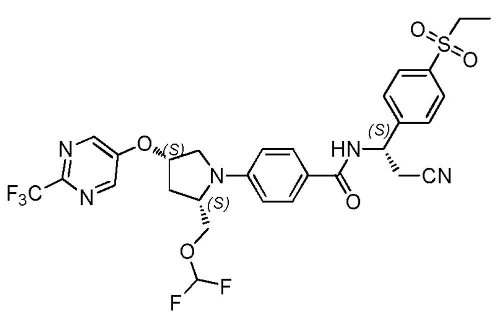
(23),

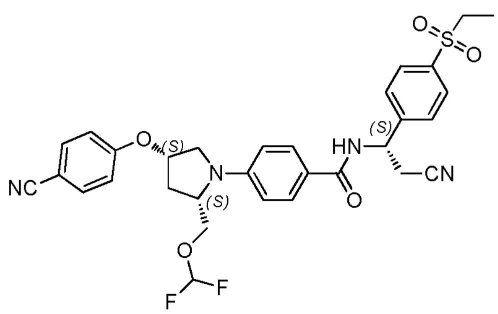
(24),

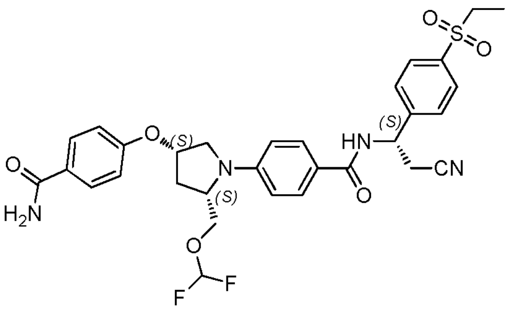
(25),

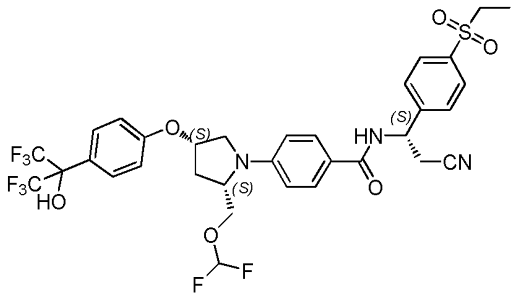
(26),

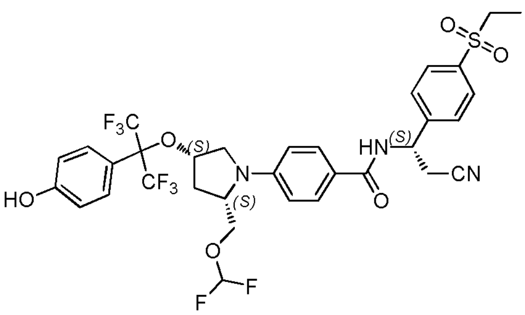
(27),

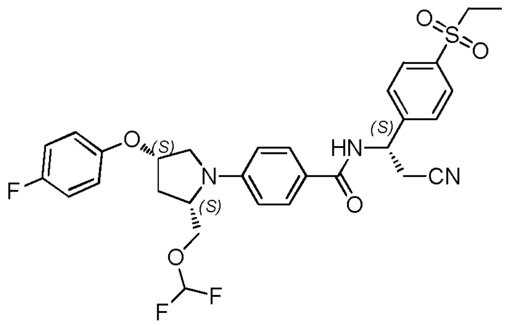
(28),

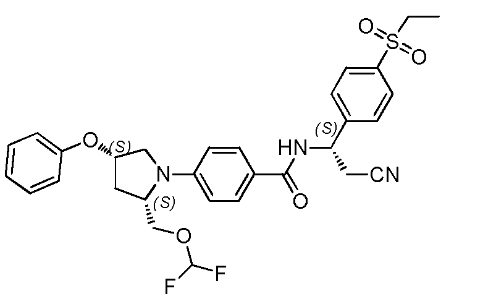
(29),

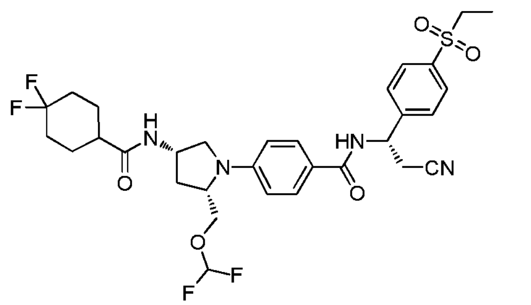
(30),

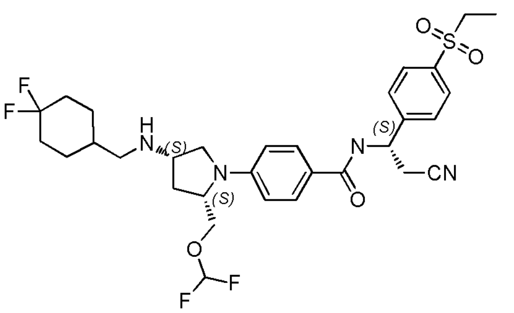
(31),

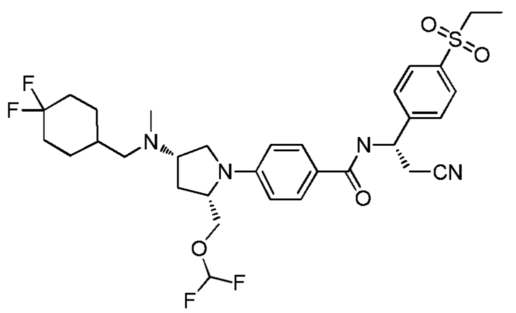
(32),

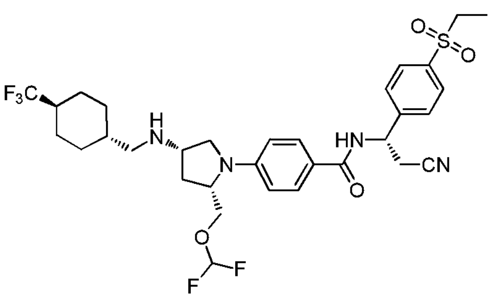
(33),

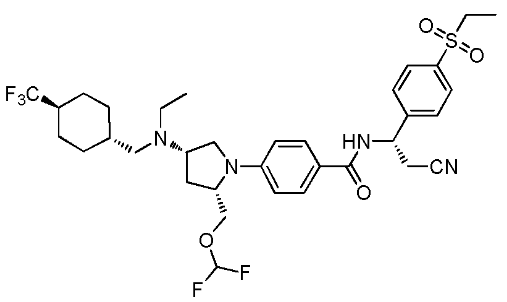
(34),

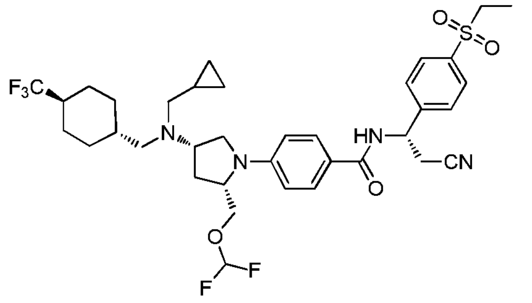
(35),

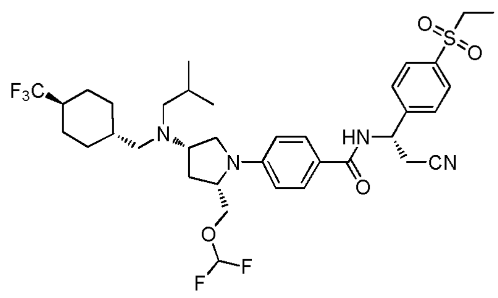
(36),

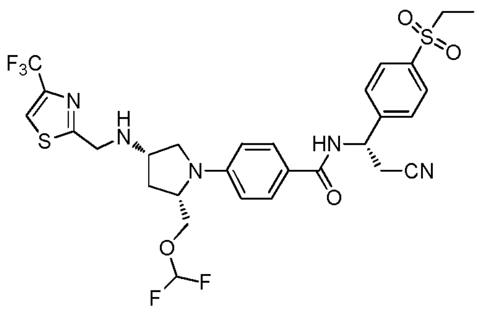
(37),

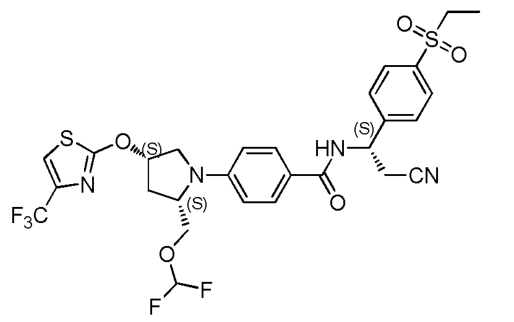
(38),

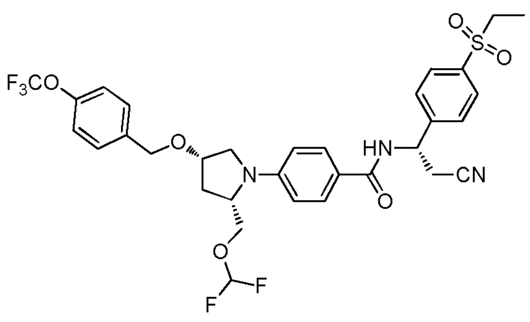
(39),

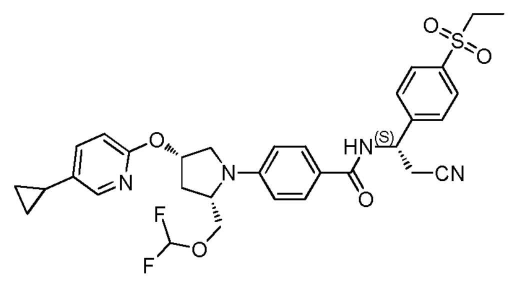
(40),

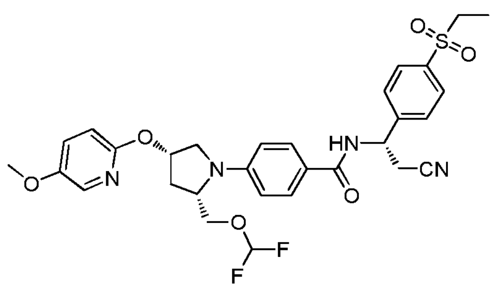
(41),

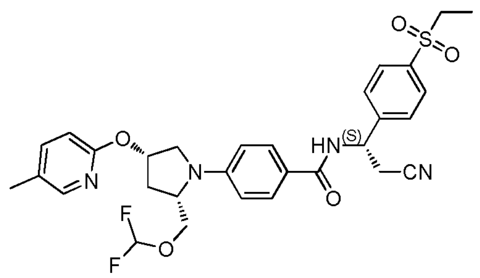
(42),

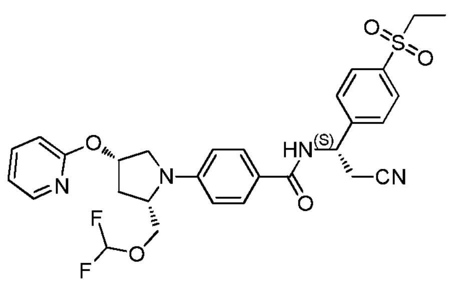
(43),

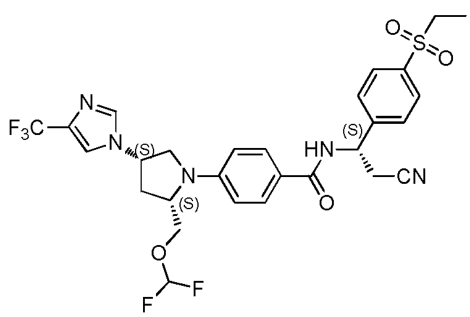
(44),

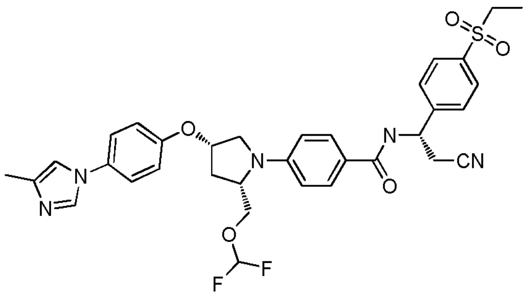
(45),

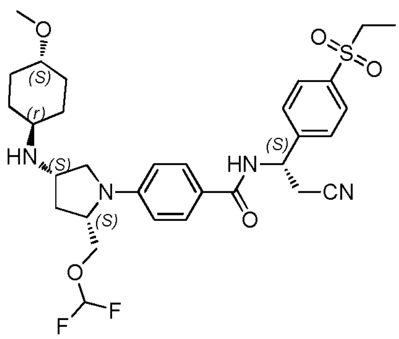
(46),

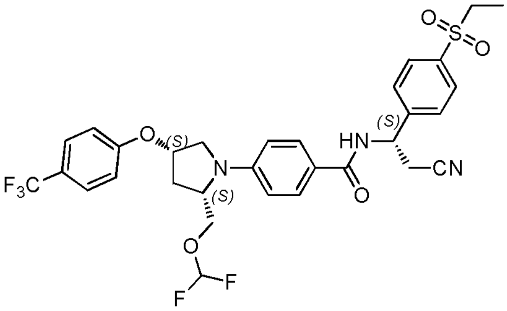
(47),

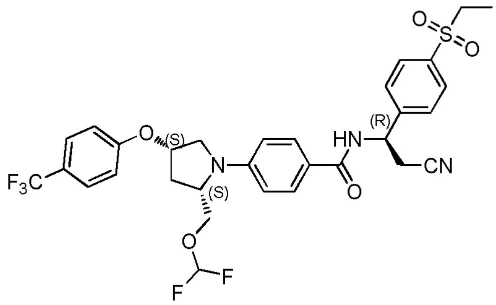
(48),

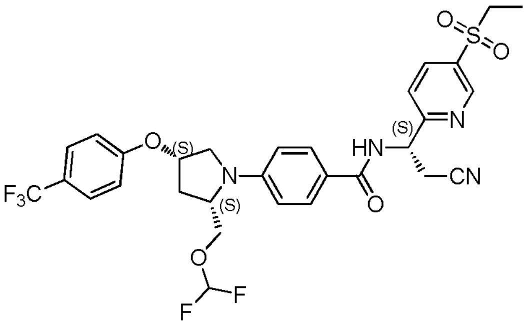
(49),

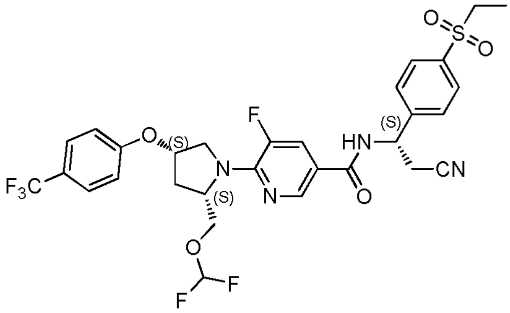
(50),

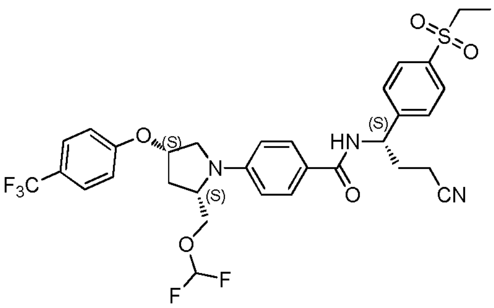
(51),

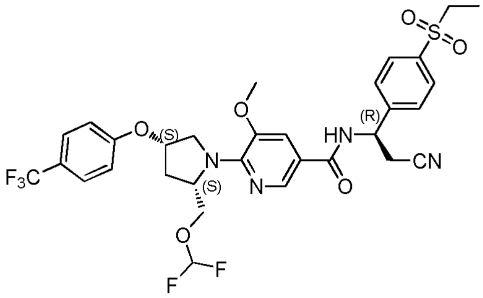
(52),

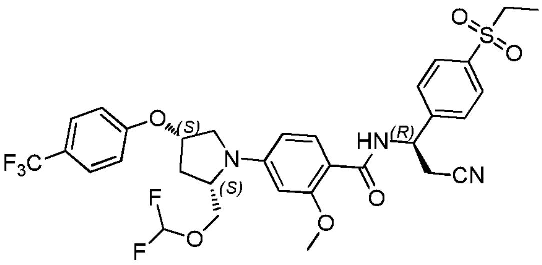
(53),

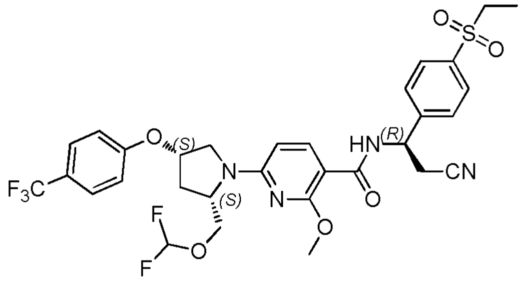
(54),

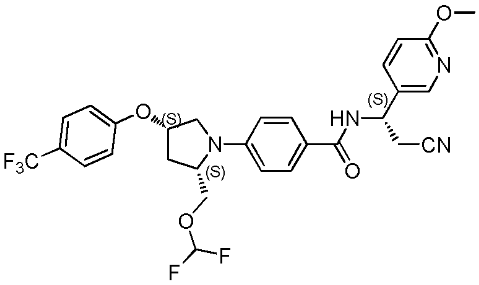
(55),

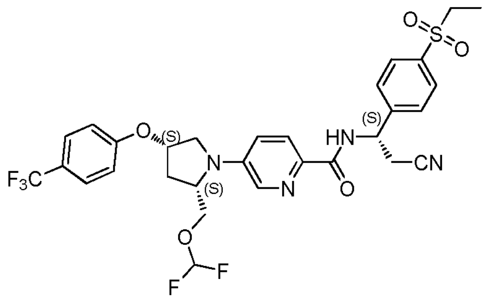
(56),

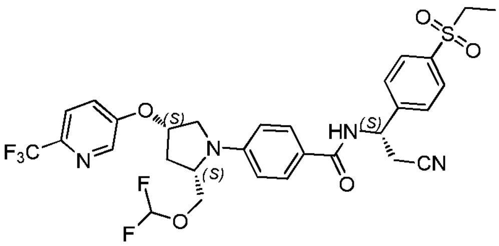
(57),

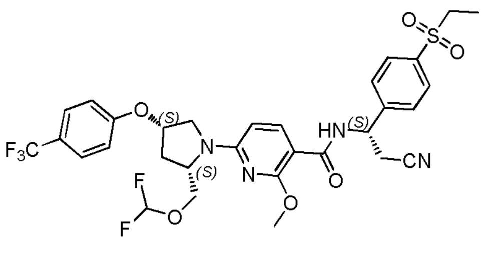
(58),

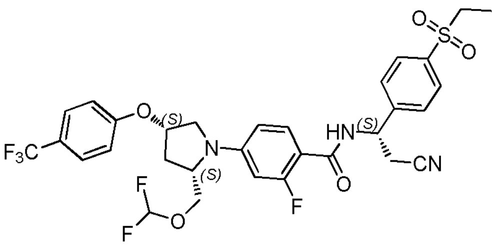
(59),

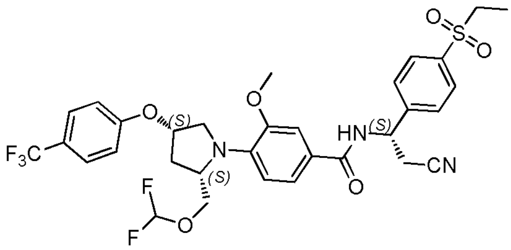
(60),

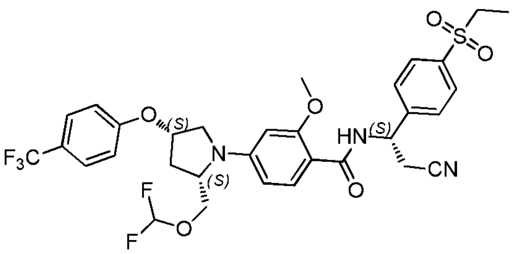
(61),

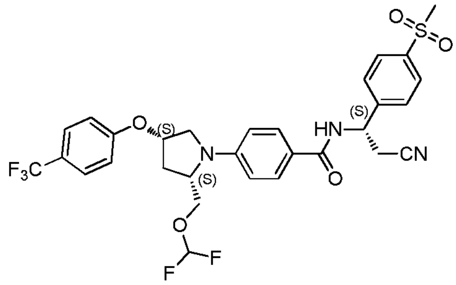
(62),

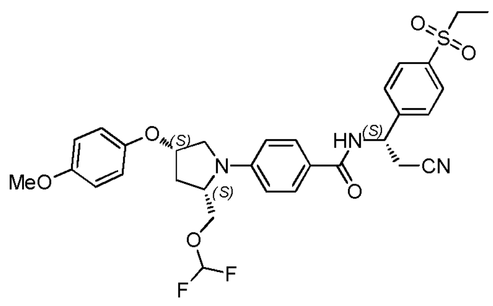
(63),

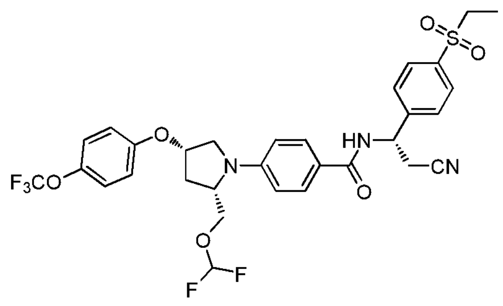
(64),

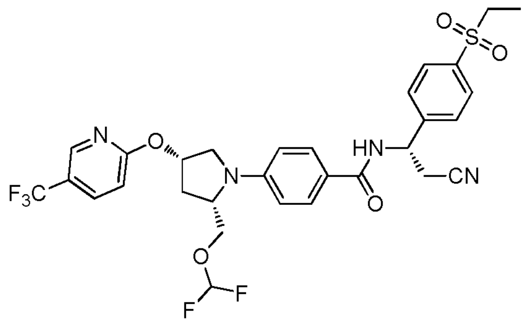
(65),

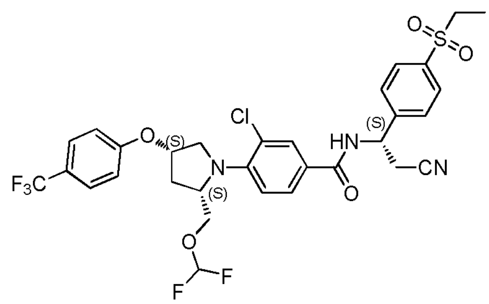
(66),

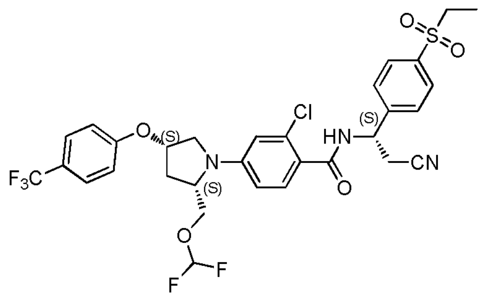
(67),

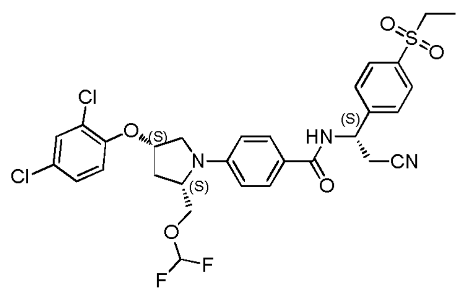
(68),

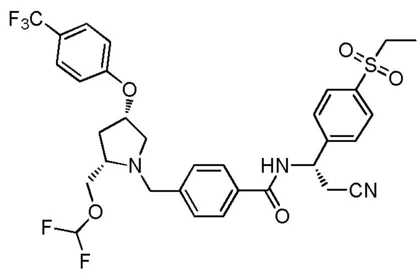
(69),

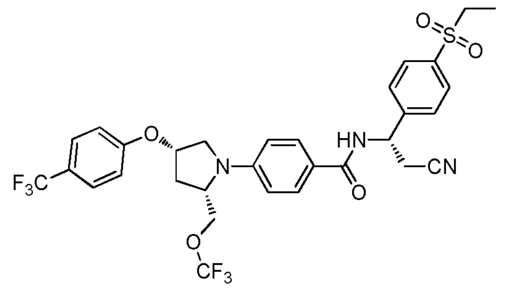
(70),

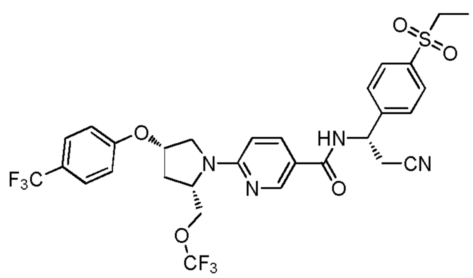
(71),

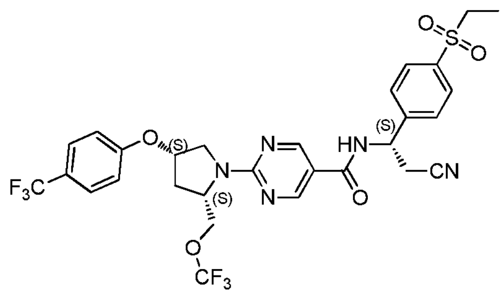
(72),

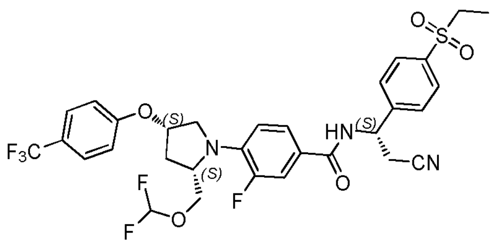 (73),

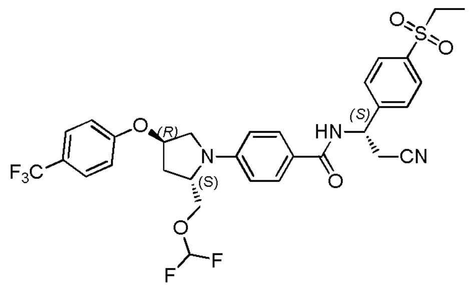 (74),

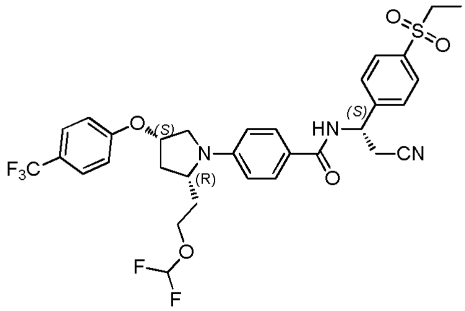 (75),

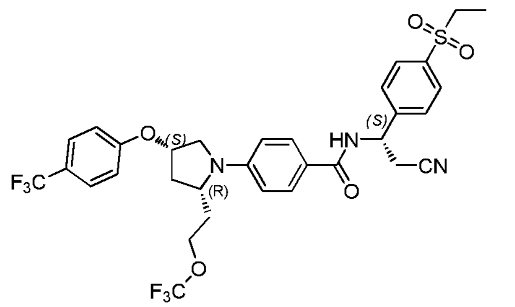 (76),

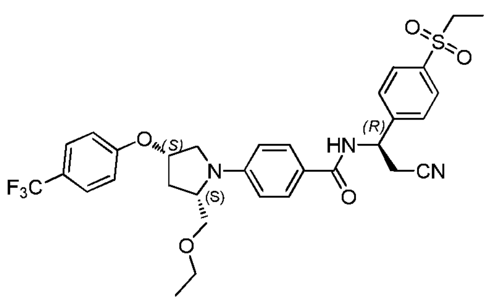 (77),

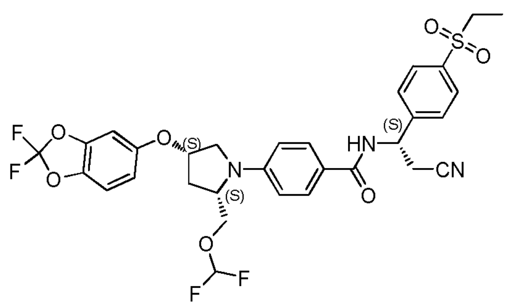 (78),

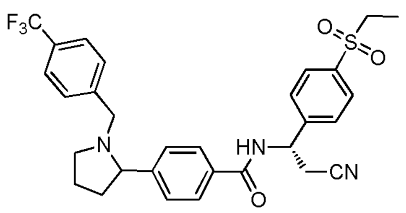 (79),

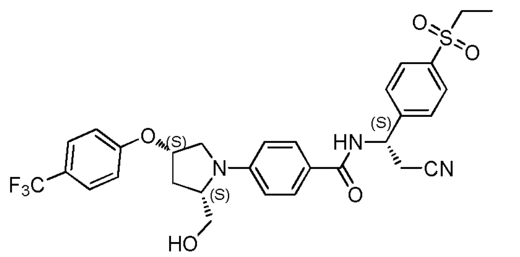 (80),

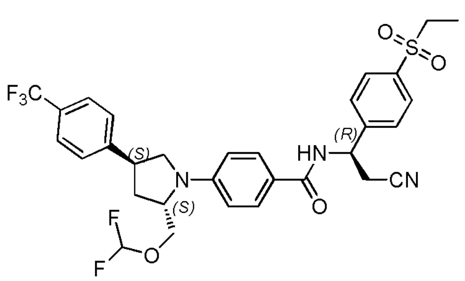 (81),

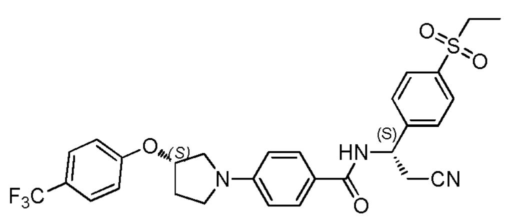 (82),

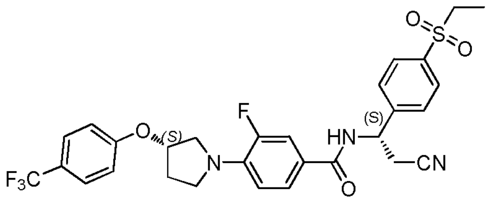 (83),

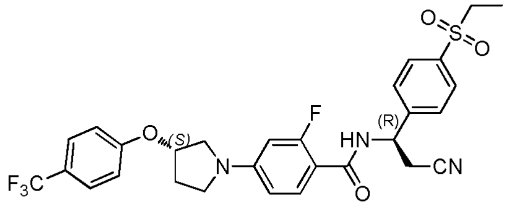 (84),

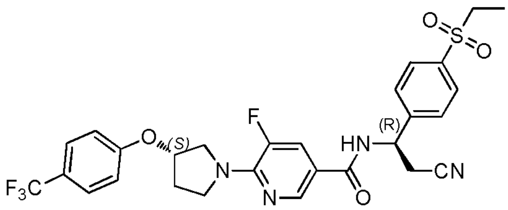 (85),

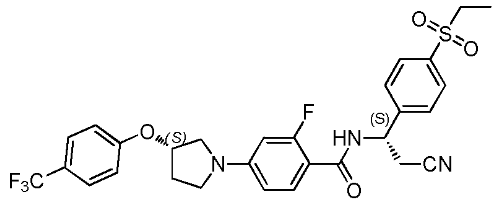 (86),

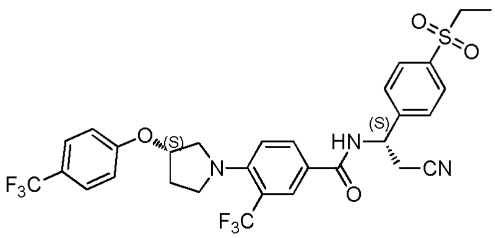 (87),

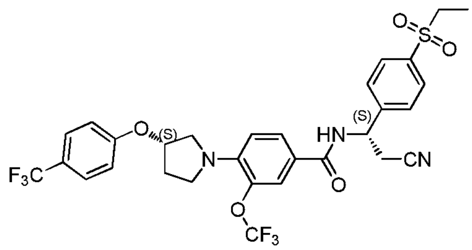 (88),

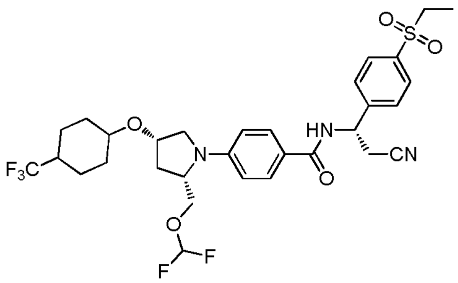 (89),

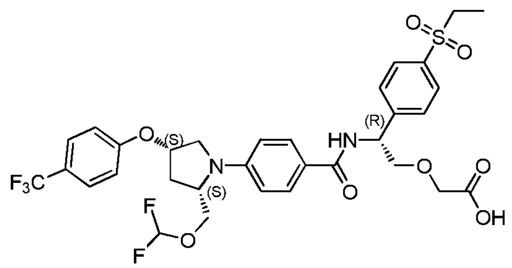 (90),

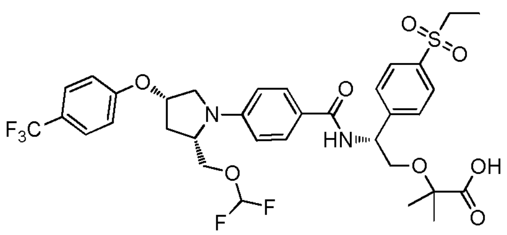 (91),

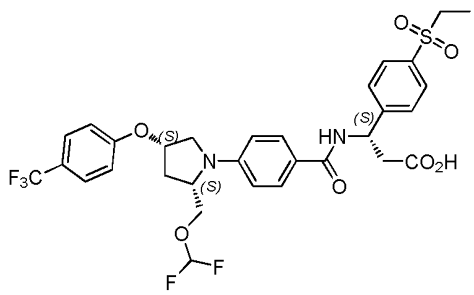 (92),

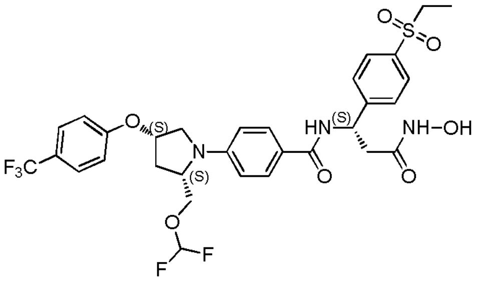

(93),

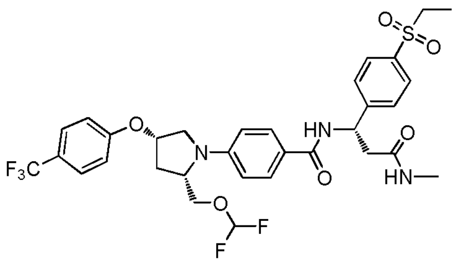

(94),

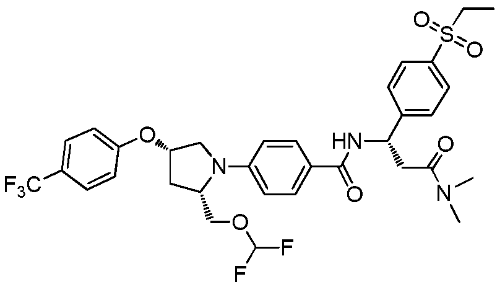

(95),

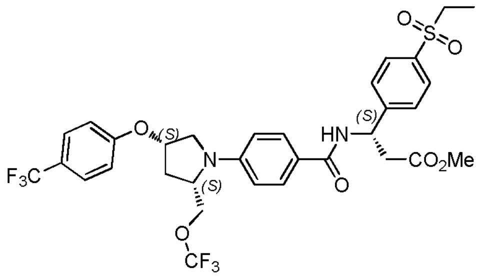

(96),

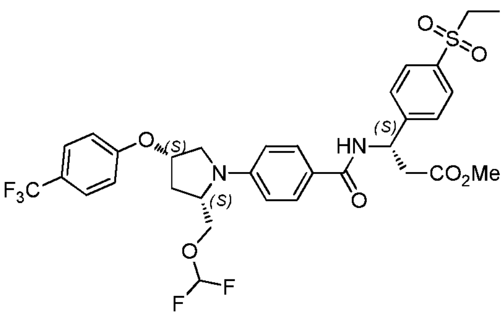

(97),

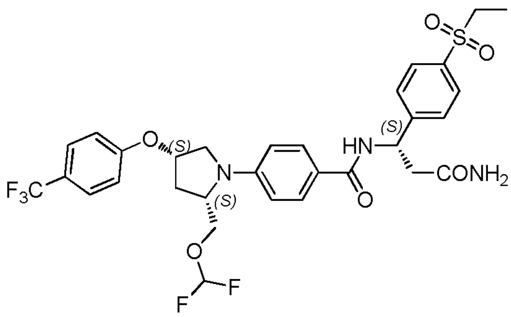

(98),

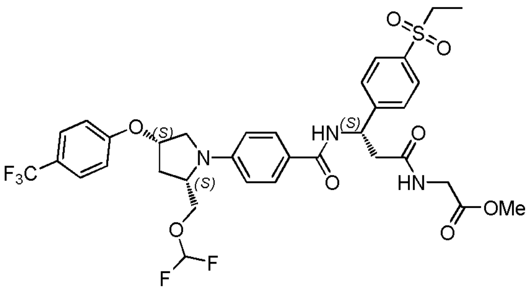

(99),

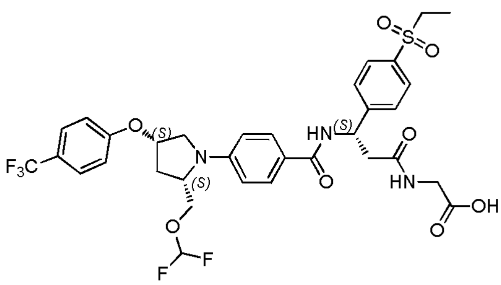

(100),

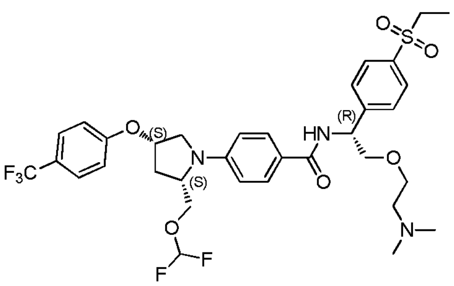

(101),

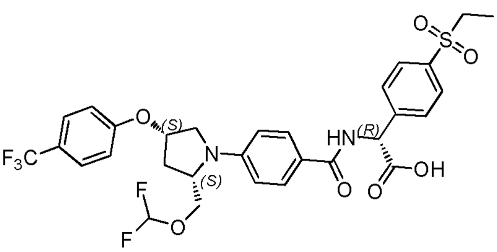

(102),

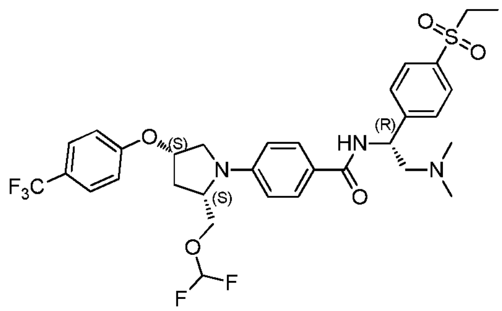

(103),

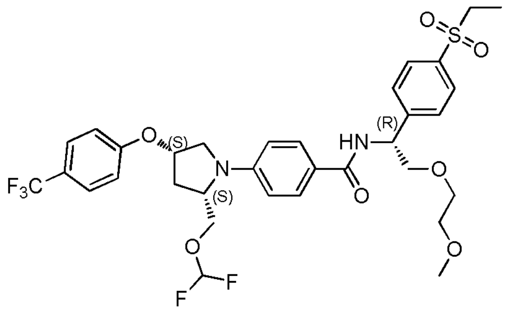

(104),

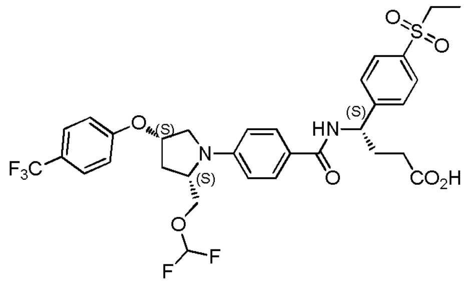

(105),

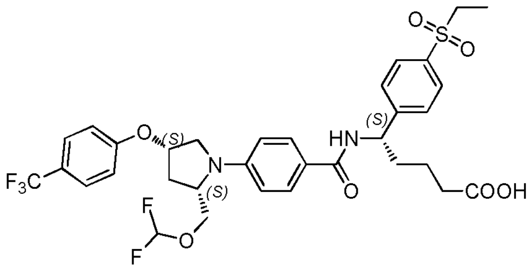

(106),

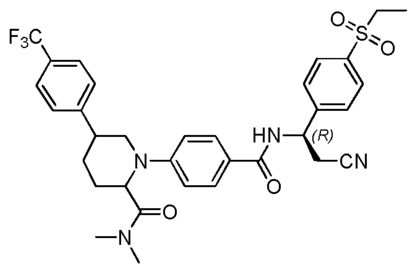

(107),

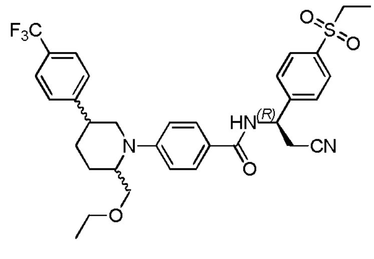

(108),

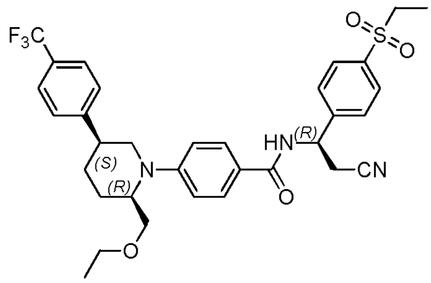

(109),

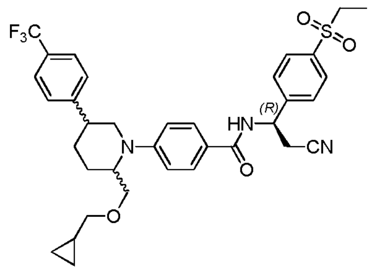

(110),

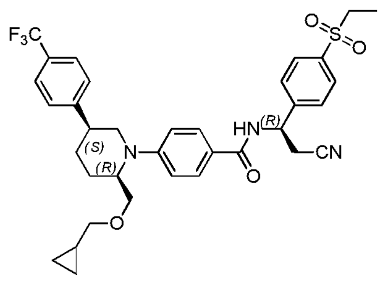

(111),

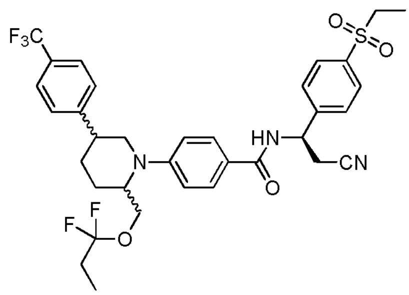

(112),

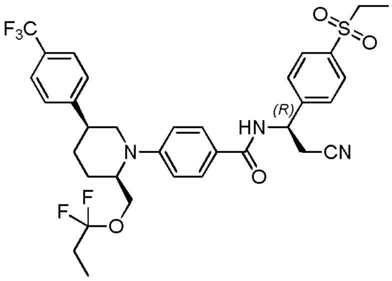
(113),

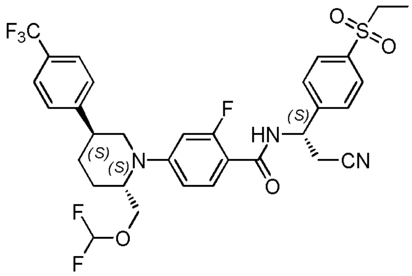
(114),

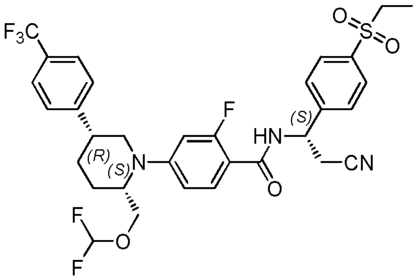
(115),

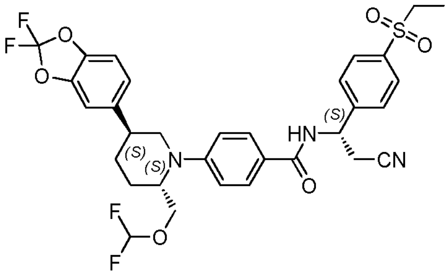
(116),

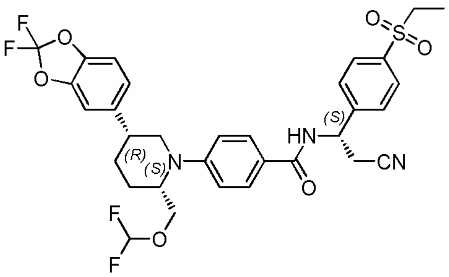
(117),

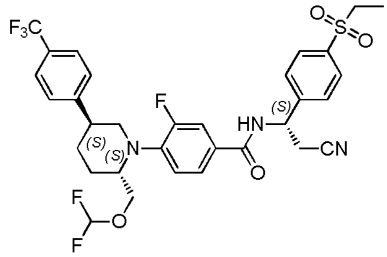
(118),

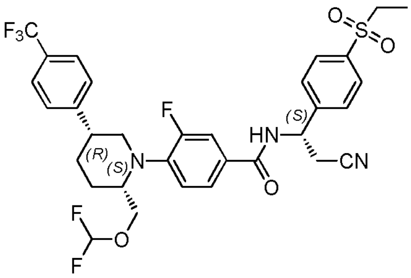
(119),

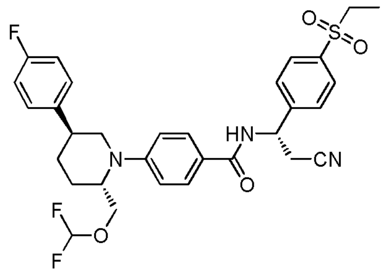
(120),

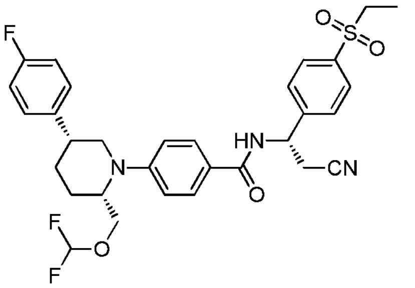
(121),

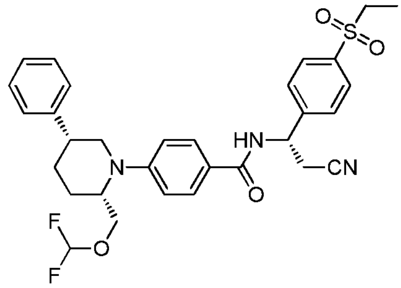
(122),

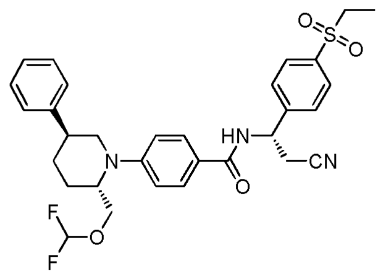

(123),

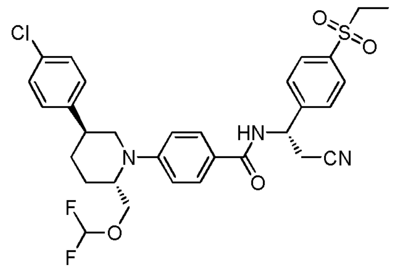

(124),

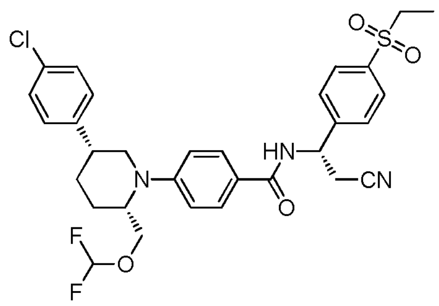

(125),

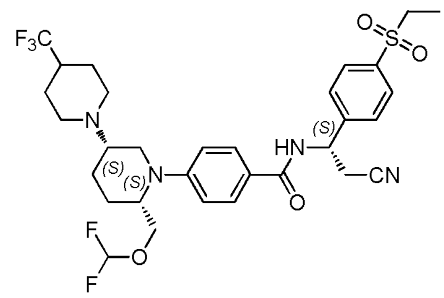

(126),

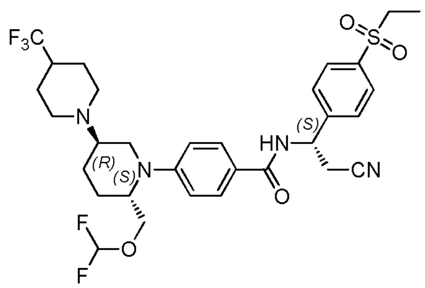

(127),

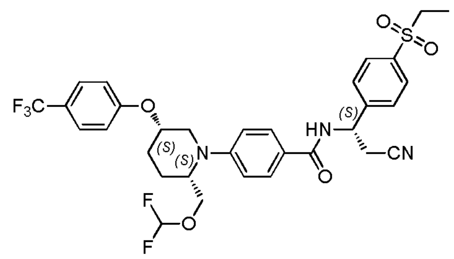

(128),

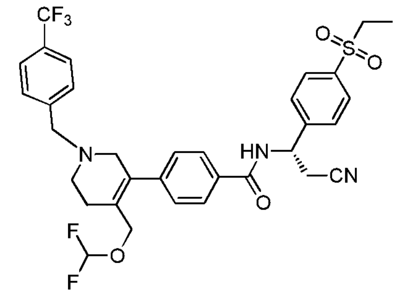

(129),

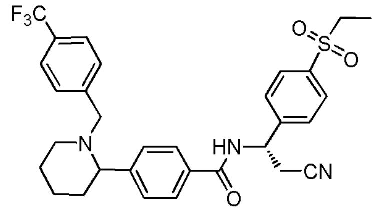

(130),

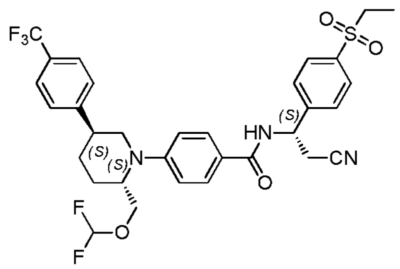

(131),

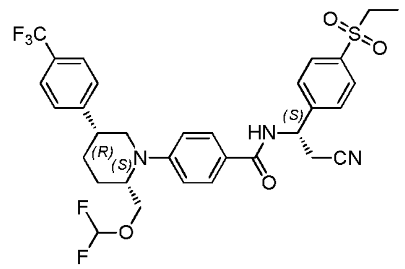

(132),

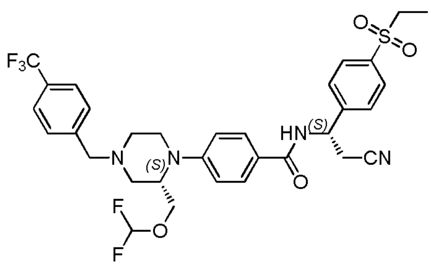 (133),

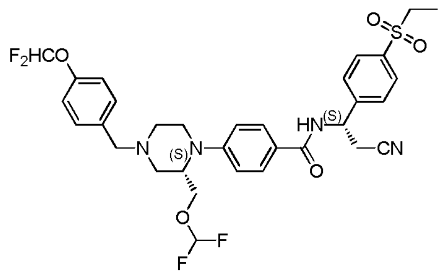 (134),

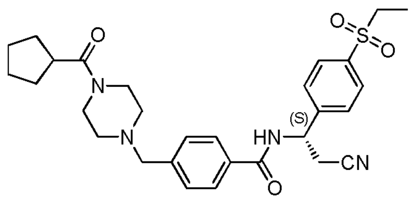 (135),

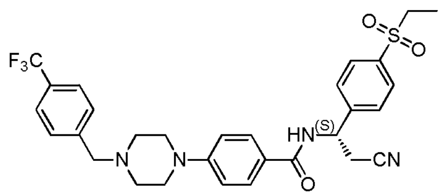 (136),

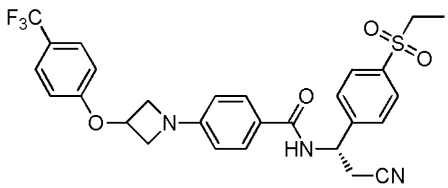 (137),

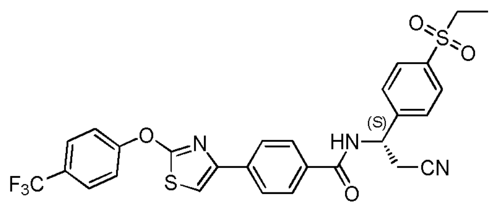 (138),

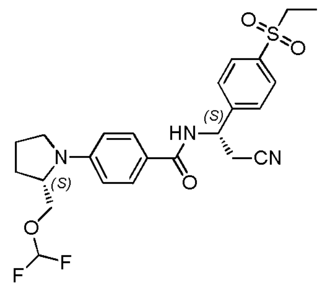 (139),

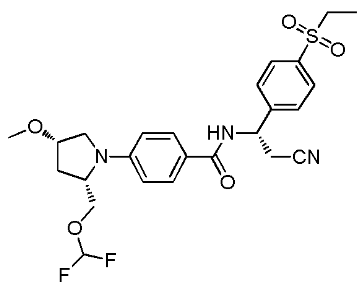 (140),

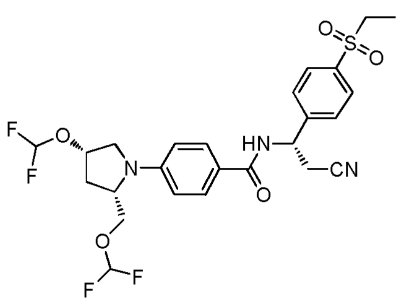 (141),

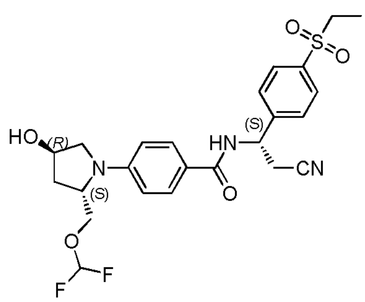 (142),

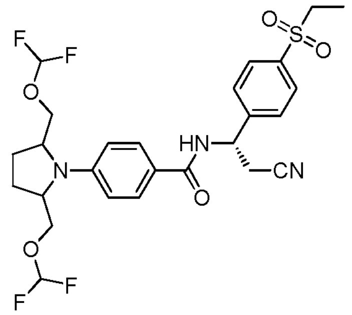 (143),

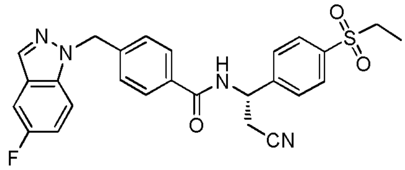 (144),

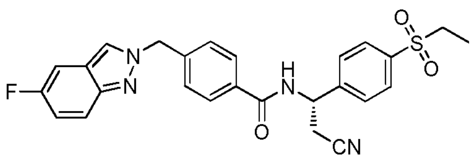 (145),

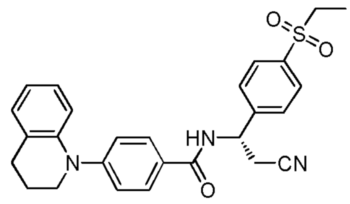 (146),

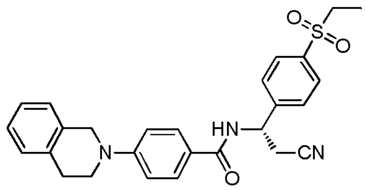 (147),

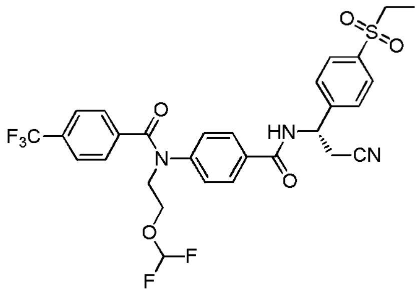 (148),

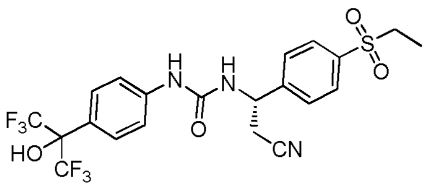 (149),

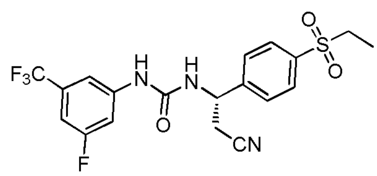 (150),

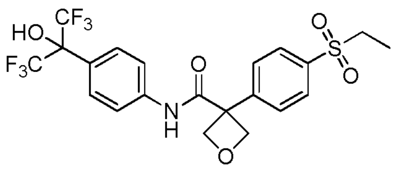 (151),

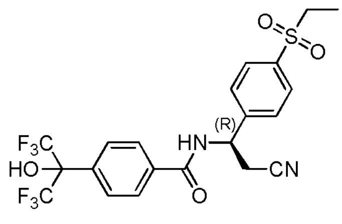 (152),

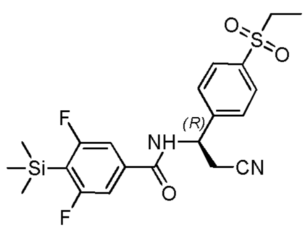 (153), 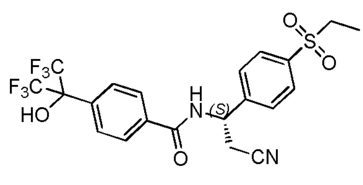 (154), 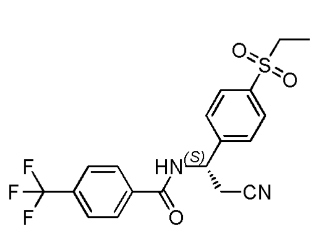 (155),

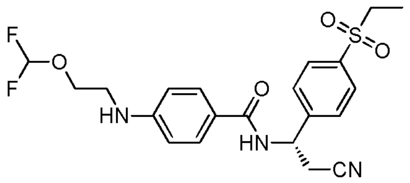

(156),

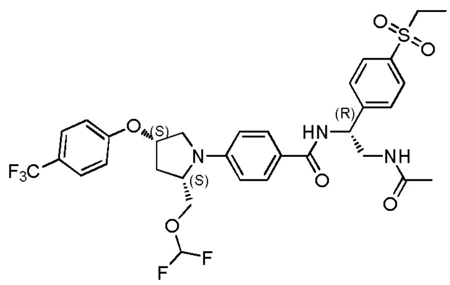

(157),

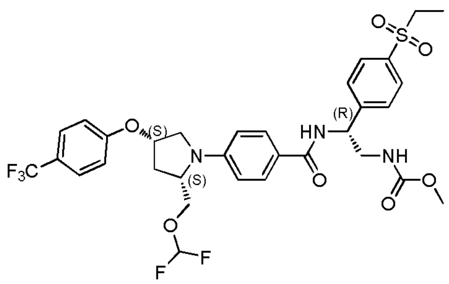

(158),

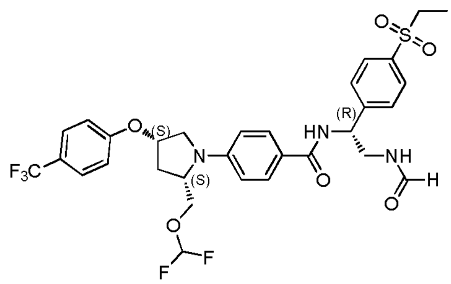

(159),

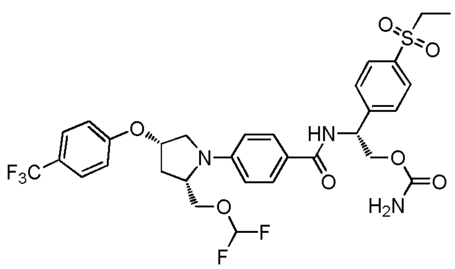

(160),

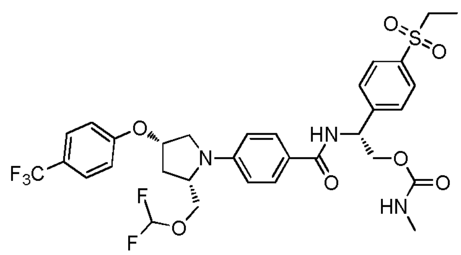

(161),

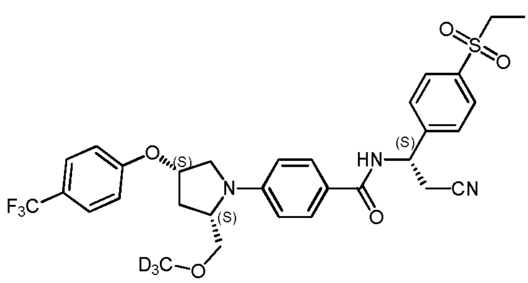

(162),

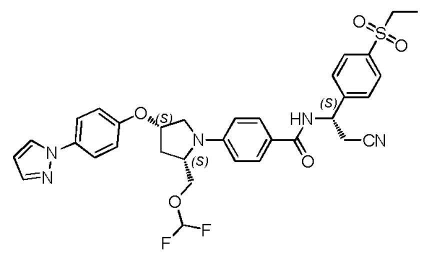

(163),

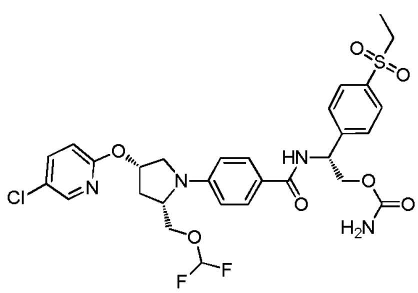

(164),

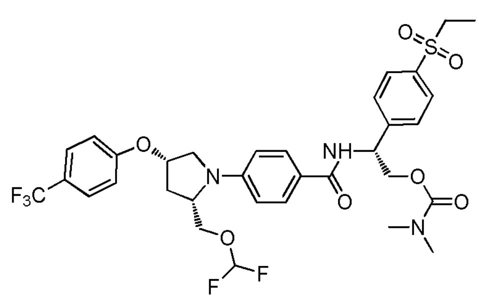

(165),

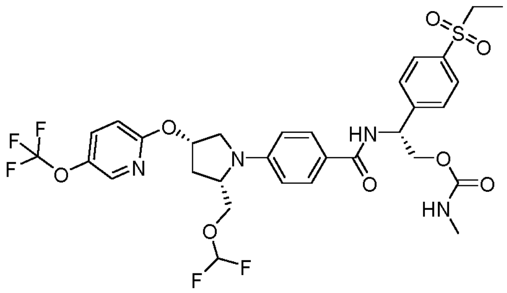

(166),

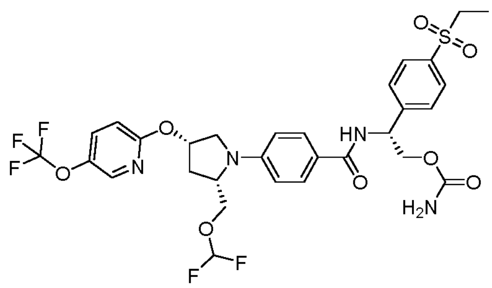

(167),

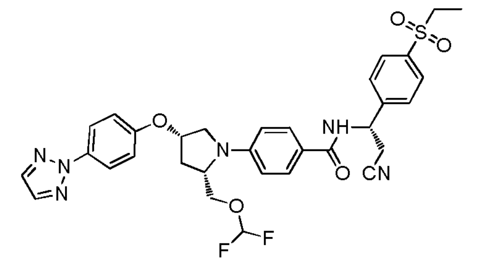

(168),

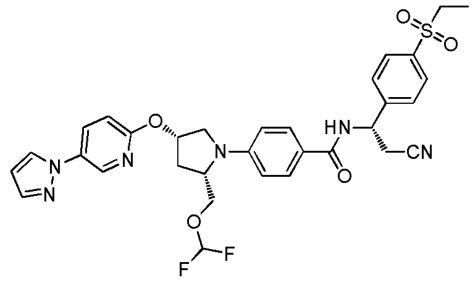

(169),

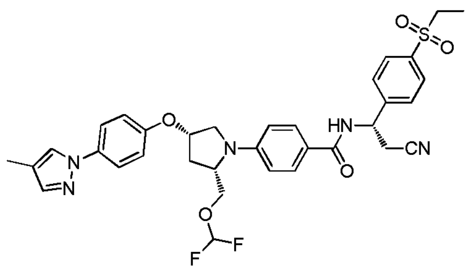

(170),

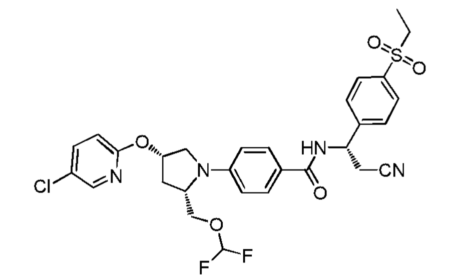

(171),

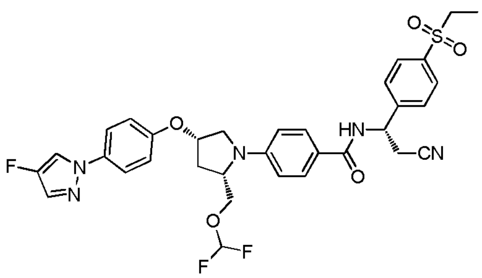

(172),

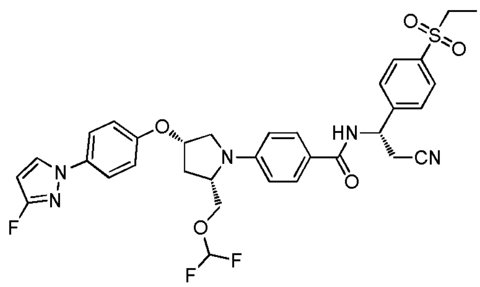

(173),

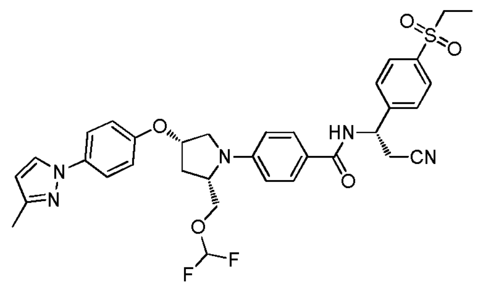

(174),

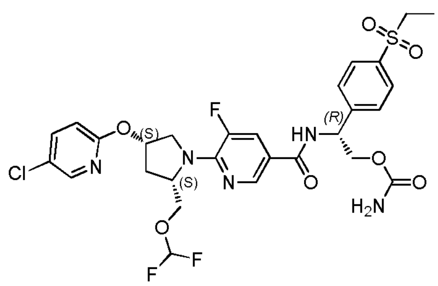

(175),

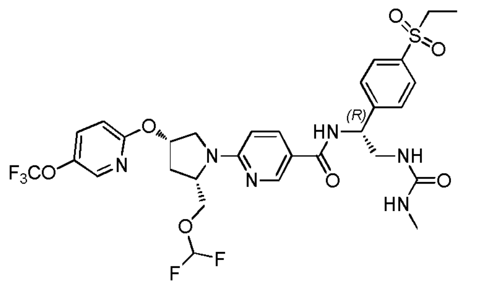
(176),

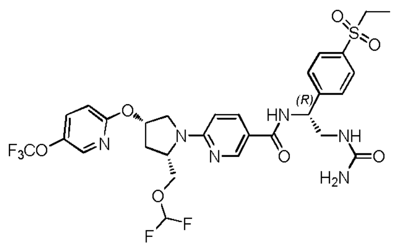
(177),

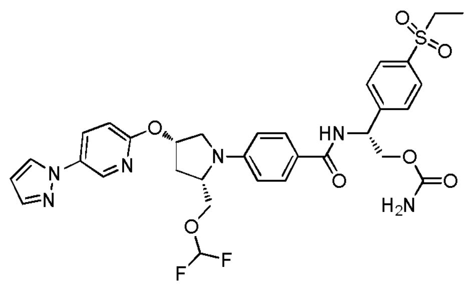
(178),

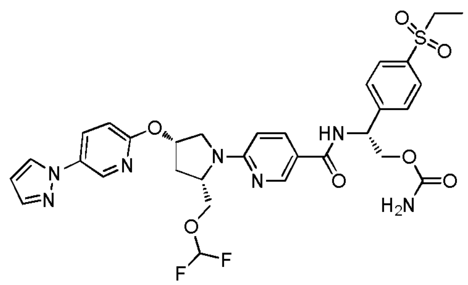
(179),

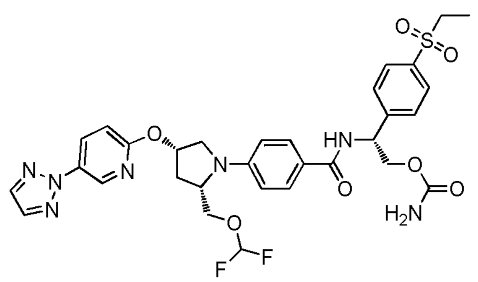
(180),

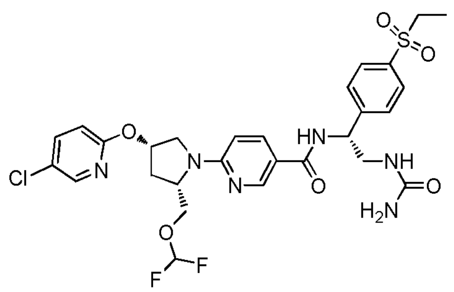
(181),

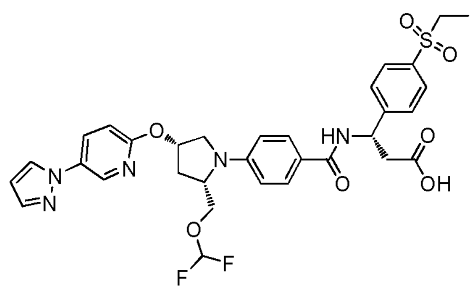
(182),

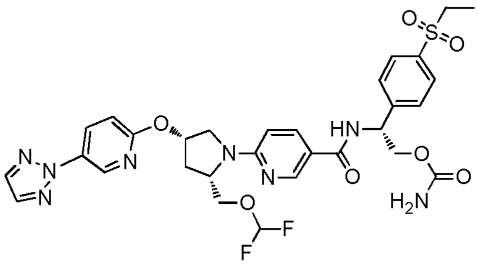
(183),

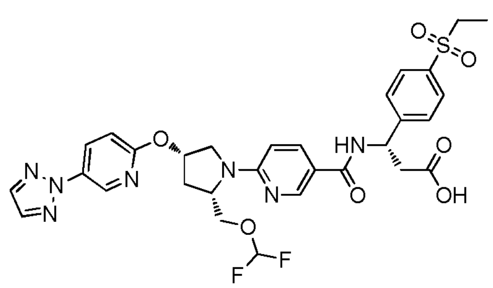
(184),

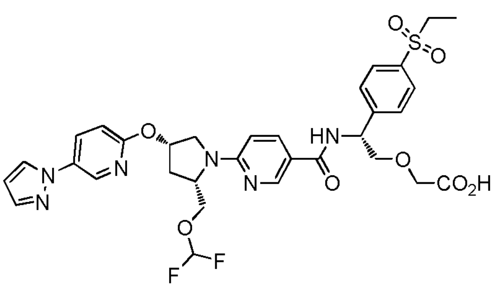
(185),

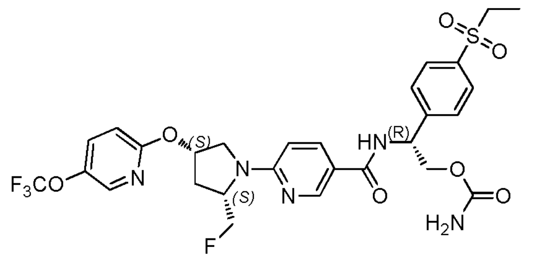 (186), 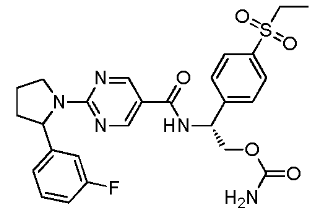 (187),

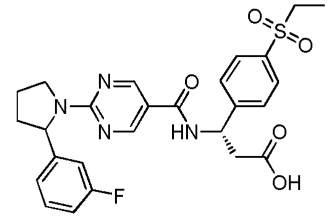 (188), 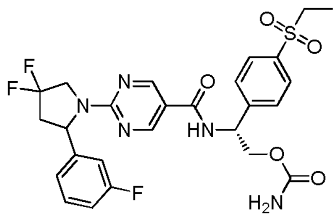 (189), 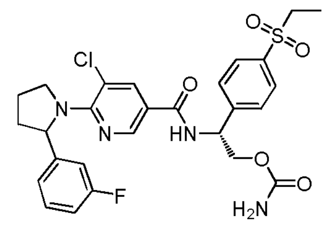 (190),

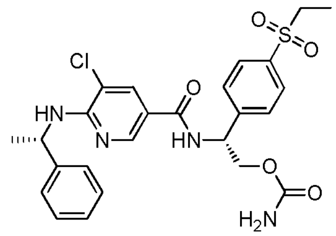 (191), 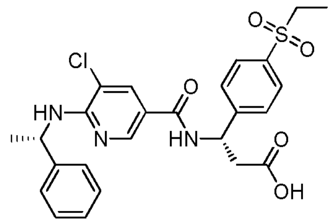 (192),

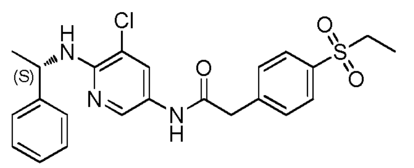 (193), 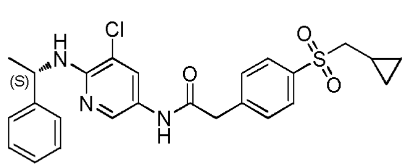 (194),

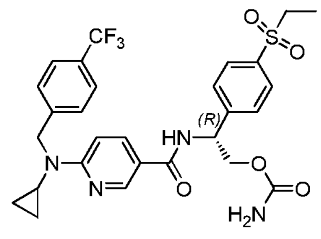 (195), 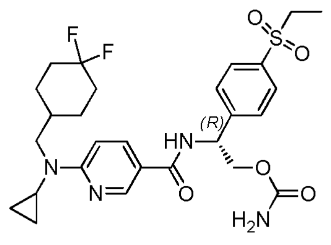 (196), 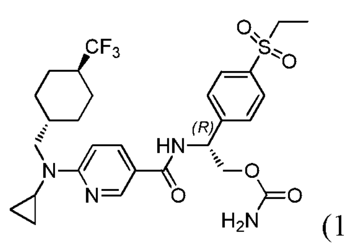 (197),

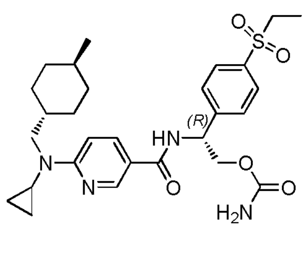 (198), 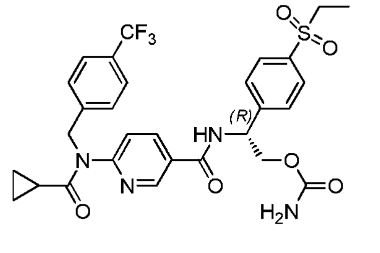 (199),

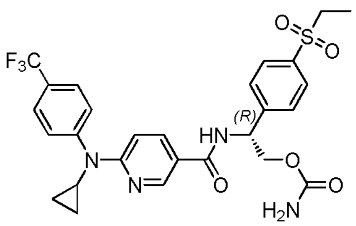 (200), 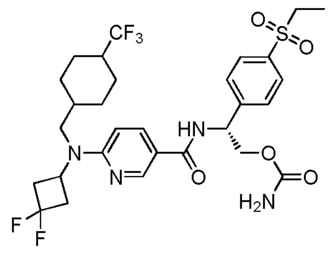 (201),

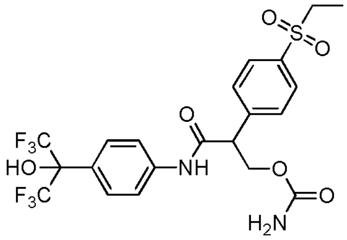 (202), 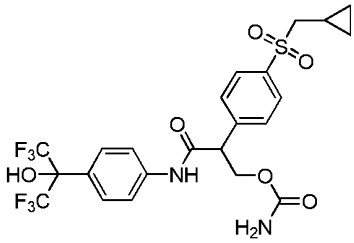 (203),

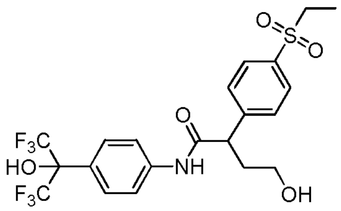 (204), 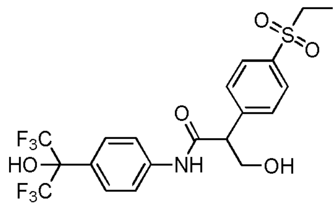 (205),

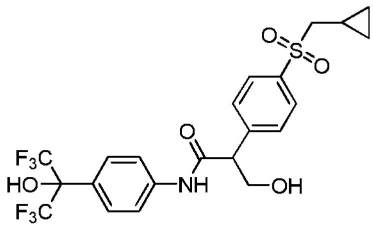 (206) or 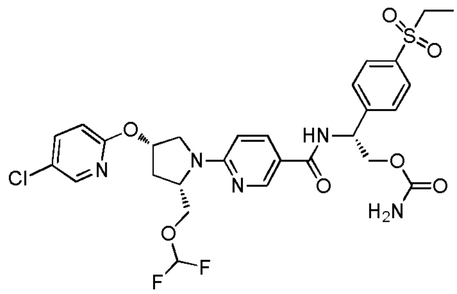 (207).

**23.** A pharmaceutical composition comprising the compound of any one of claims 1 to 22, and a pharmaceutically acceptable excipient, a carrier, an adjuvant or a combination thereof.

**24.** The pharmaceutical composition of claim 23, comprising other drugs for preventing or treating inflammatory syndromes, disorders or diseases or any combination thereof.

**25.** Use of the compound of any one of claims 1 to 22 or the pharmaceutical composition of any one of claims 23 to 24 in the manufacture of a medicament for preventing, treating or lessening diseases, disorders or syndromes mediated by RORγt in mammals.

**26.** The use of claim 25, wherein the disease, disorder or syndrome mediated by RORγt is cancer, psoriasis, rheumatoid arthritis, systemic lupus erythematosus, multiple sclerosis, inflammatory bowel disease, colitis, ulcerative colitis, rheumatoid arthritis, autoimmune eye disease, ankylosing spondylitis, asthma, chronic obstructive pulmonary disease, osteoarthritis, allergic rhinitis, atopic dermatitis, Crohn's disease, or Kawasaki disease.

## INTERNATIONAL SEARCH REPORT

International application No.
**PCT/CN2020/141903**

**A. CLASSIFICATION OF SUBJECT MATTER**

C07D 295/155(2006.01)i; C07D 417/04(2006.01)i; C07D 413/04(2006.01)i; A61K 31/5377(2006.01)i; A61K 31/5375(2006.01)i; A61P 19/02(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

DWPI, SIPOABS, WOTXT, EPTXT, USTXT, CNTXT, CATXT, GBTXT, JPTXT, KRABS, CNABS, CNKI, STNext, ISI Web of Science: 维甲酸相关孤核受体, 磺酰, 酰胺, 东阳光新药, retinoid related orphan receptor, ROR, sulfonyl, amide, HEC NEW DRUG, SUNSHINE LAKE

**C. DOCUMENTS CONSIDERED TO BE RELEVANT**

| Category* | Citation of document, with indication, where appropriate, of the relevant passages | Relevant to claim No. |
|---|---|---|
| PX | WO 2020011147 A1 (SUNSHINE LAKE PHARMA CO., LTD.) 16 January 2020 (2020-01-16)<br>claims 1-26 | 1-26 |
| X | CN 105980353 A (LEAD PHARMA CEL MODELS IP B V) 28 September 2016 (2016-09-28)<br>abstract ; description paragraphs [0770-0772]; description paragraphs [0114-0118]; claims 1-27 | 1-3, 7, 10-11, 22-26 |
| Y | WO 2018116285 A1 (GLENMARK PHARMACEUTICALS S. A.) 28 June 2018 (2018-06-28)<br>abstract, description, page 8 lines 17-28, description page 9 lines 1-10, table 4 | 1-26 |
| Y | WO 2017010399 A1 (SHIONOGI & CO., LTD.) 19 January 2017 (2017-01-19)<br>abstract, page 199, compound 494 | 1-26 |
| A | WO 2018185675 A1 (GLENMARK PHARMACEUTICALS S. A.) 11 October 2018 (2018-10-11)<br>claims 1-37 | 1-26 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 March 2021** | **29 March 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/141903**

**C. DOCUMENTS CONSIDERED TO BE RELEVANT**

| Category* | Citation of document, with indication, where appropriate, of the relevant passages | Relevant to claim No. |
|---|---|---|
| A | WO 2016193452 A1 (LEAD PHARMA CEL MODELS IP B.V. et al.) 08 December 2016 (2016-12-08)<br>abstract, claims 1-15 | 1-26 |
| A | WO 2017087608 A1 (VITAE PHARMACEUTICALS, INC.) 26 May 2017 (2017-05-26)<br>abstract, claims 1-42 | 1-26 |
| A | WO 2019088057 A1 (TORAY INDUSTRIES, INC.) 09 May 2019 (2019-05-09)<br>abstract, claims 1-8 | 1-26 |
| A | WO 2019213470 A1 (ETERNITY BIOSCIENCE INC.) 07 November 2019 (2019-11-07)<br>abstract ; claims 1-25 | 1-26 |

Form PCT/ISA/210 (second sheet) (January 2015)

**INTERNATIONAL SEARCH REPORT**
**Information on patent family members**

International application No.
**PCT/CN2020/141903**

| Patent document cited in search report | Publication date (day/month/year) | Patent family member(s) | Publication date (day/month/year) |
|---|---|---|---|
| WO 2020011147 A1 | 16 January 2020 | None | |
| CN 105980353 A | 28 September 2016 | EA 030393 B1 | 31 July 2018 |
| | | DO P2016000125 A | 31 August 2016 |
| | | CR 20160303 A | 10 November 2016 |
| | | ES 2722409 T3 | 09 August 2019 |
| | | IL 246018 A | 30 January 2020 |
| | | AU 2014359324 A1 | 14 July 2016 |
| | | MX 2016007275 A | 05 January 2017 |
| | | PH 12016501066 A1 | 11 July 2016 |
| | | US 2016304448 A1 | 20 October 2016 |
| | | EP 3077372 A1 | 12 October 2016 |
| | | TN 2016000224 A1 | 06 October 2017 |
| | | HR P20190483 T1 | 17 May 2019 |
| | | PL 3077372 T3 | 30 September 2019 |
| | | RS 58650 B1 | 31 May 2019 |
| | | JP 6461960 B2 | 30 January 2019 |
| | | EP 3077372 B8 | 10 April 2019 |
| | | AU 2014359324 B2 | 21 February 2019 |
| | | PT 3077372 T | 21 March 2019 |
| | | CA 2932483 A1 | 11 June 2015 |
| | | UA 117941 C2 | 25 October 2018 |
| | | IL 246018 D0 | 02 August 2016 |
| | | NZ 721511 A | 30 October 2020 |
| | | DK 3077372 T3 | 23 April 2019 |
| | | LT 3077372 T | 25 March 2019 |
| | | CL 2016001364 A1 | 02 December 2016 |
| | | PE 20160891 A1 | 24 September 2016 |
| | | JP 2017504576 A | 09 February 2017 |
| | | HU E043258 T2 | 28 August 2019 |
| | | SI 3077372 T1 | 31 July 2019 |
| | | KR 20160105413 A | 06 September 2016 |
| | | CN 105980353 B | 14 June 2019 |
| | | ZA 201604043 B | 30 August 2017 |
| | | EP 3077372 B1 | 06 February 2019 |
| | | MX 368721 B | 14 October 2019 |
| | | EA 201691173 A1 | 30 November 2016 |
| | | US 9738600 B2 | 22 August 2017 |
| | | WO 2015082533 A1 | 11 June 2015 |
| WO 2018116285 A1 | 28 June 2018 | None | |
| WO 2017010399 A1 | 19 January 2017 | None | |
| WO 2018185675 A1 | 11 October 2018 | None | |
| WO 2016193452 A1 | 08 December 2016 | US 10315996 B2 | 11 June 2019 |
| | | EP 3303291 B1 | 18 March 2020 |
| | | RU 2727698 C2 | 23 July 2020 |
| | | ES 2785902 T3 | 08 October 2020 |
| | | AU 2016272015 B2 | 27 August 2020 |
| | | PT 3303291 T | 22 April 2020 |
| | | BR 112017026226 A2 | 11 September 2018 |
| | | CN 108473429 A | 31 August 2018 |

Form PCT/ISA/210 (patent family annex) (January 2015)

**INTERNATIONAL SEARCH REPORT**
**Information on patent family members**

International application No.
**PCT/CN2020/141903**

| Patent document cited in search report | Publication date (day/month/year) | Patent family member(s) | Publication date (day/month/year) |
|---|---|---|---|
| | | EP 3101006 A1 | 07 December 2016 |
| | | TW 201710240 A | 16 March 2017 |
| | | KR 20180042213 A | 25 April 2018 |
| | | CA 2988000 A1 | 08 December 2016 |
| | | EP 3303291 A1 | 11 April 2018 |
| | | JP 2018522837 A | 16 August 2018 |
| | | UY 36708 A | 31 January 2017 |
| | | RU 2017145924 A | 10 July 2019 |
| | | IL 256034 D0 | 31 January 2018 |
| | | RU 2017145924 A3 | 04 December 2019 |
| | | AU 2016272015 A1 | 04 January 2018 |
| | | PL 3303291 T3 | 21 September 2020 |
| | | JP 6754778 B2 | 16 September 2020 |
| | | MX 2017015741 A | 24 April 2018 |
| | | US 2018170877 A1 | 21 June 2018 |
| | | AR 104878 A1 | 23 August 2017 |
| WO 2017087608 A1 | 26 May 2017 | IL 259421 D0 | 31 July 2018 |
| | | BR 112018010018 A2 | 21 November 2018 |
| | | RU 2018121946 A | 23 December 2019 |
| | | WO 2017087608 A8 | 06 July 2017 |
| | | CN 108463458 A | 28 August 2018 |
| | | MX 2018006223 A | 19 December 2018 |
| | | AU 2016355710 A1 | 31 May 2018 |
| | | US 2019322687 A1 | 24 October 2019 |
| | | EP 3377482 A1 | 26 September 2018 |
| | | JP 2018534326 A | 22 November 2018 |
| | | CA 3005658 A1 | 26 May 2017 |
| | | KR 20180086221 A | 30 July 2018 |
| | | RU 2018121946 A3 | 16 April 2020 |
| WO 2019088057 A1 | 09 May 2019 | TW 201922700 A | 16 June 2019 |
| | | JP WO2019088057 A1 | 24 September 2020 |
| WO 2019213470 A1 | 07 November 2019 | AU 2019262169 A1 | 29 October 2020 |
| | | CN 112135611 A | 25 December 2020 |
| | | CA 3097519 A1 | 07 November 2019 |
| | | TW 202014185 A | 16 April 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202010008803
- US 4328245 A
- US 4409239 A
- US 4410545 A
- US 6350458 B

### Non-patent literature cited in the description


- **SUN et al.** *Science,* 2000, vol. 288, 2369-2372
- **EBERL et al.** *Nat Immunol,* 2004, vol. 5, 64-73
- **IVANOV, II ; MCKENZIE BS ; ZHOU L ; TADOKORO CE ; LEPELLEY A ; LAFAILLE JJ et al.** *Cell,* 2006, vol. 126 (6), 1121-33
- **JETTEN et al.** *Nucl. Recept. Signal,* 2009, vol. 7, e003
- **MANEL et al.** *Nat. Immunol.,* 2008, vol. 9, 6, 41-649
- **KORN et al.** *Annu. Rev. Immunol.,* 2009, vol. 27, 485-517
- **JETTEN et al.** *Adv. Dev.Biol,* 2006, vol. 16, 313-355
- **MEIER et al.** *Immunity,* 2007, vol. 26, 643-654
- **ALOISI et al.** *Nat. Rev. Immunol.,* 2006, vol. 6, 205-217
- **JAGER et al.** *J. Immunol.,* 2009, vol. 183, 7169-7177
- **BARNES et al.** *Nat. Rev. Immunol.,* 2008, vol. 8, 183-192
- Handbook of Chemistry and Physics. 1994
- **THOMAS SORRELL.** Organic Chemistry. University Science Books, 1999
- **MICHAEL B. SMITH ; JERRY MARCH.** March's Advanced Organic Chemistry. John Wiley & Sons, 2007
- McGraw-Hill Dictionary of Chemical Terms. McGraw-Hill Book Company, 1984
- **ELIEL, E ; WILEN, S.** Stereochemistry of Organic Compounds. John Wiley & Sons, Inc, 1994
- **JACQUES et al.** Enantiomers, Racemates and Resolutions. Wiley Interscience, 1981
- Principles of Asymmetric Synthesis. Elsevier, 2012
- **ELIEL, E.L.** Stereochemistry of Carbon Compounds. McGraw-Hill, 1962
- **WILEN, S.H.** Tables of Resolving Agents and Optical Resolutions. Univ. of Notre Dame Press, 1972, 268
- Chiral Separation Techniques: A Practical Approach. Wiley-VCH Verlag GmbH & Co. KGaA, 2007
- Pro-drugs as Novel Delivery Systems. **T. HIGUCHI ; V. STELLA.** A.C.S. Symposium Series. American Pharmaceutical Association and Pergamon Press, 1987, vol. 14
- **J. RAUTIO et al.** Prodrugs: Design and Clinical Applications. *Nature Review Drug Discovery,* 25 July 2008, 5-270
- **S. J. HECKER et al.** Prodrugs of Phosphates and Phosphonates. *Journal of Medicinal Chemistry,* 2008, vol. 51, 2328-2345
- **S. M. BERGE et al.** *J. Pharmaceutical Sciences,* 1977, vol. 66, 1-19
- **JERRY MARCH.** Advanced Organic Chemistiy. Wiley Interscience
- Remington's Pharmaceutical Sciences. Ed. Mack Printing Company, 1990, 1289-1329
- Remington's Pharmaceutical Sciences. Mack Publishing Company, 1985
- **STAHL ; WERMUTH.** Handbook of Pharmaceutical Salts: Properties, Selection, and Use. Wiley-VCH, 2002
- Remington: The Science and Practice of Pharmacy. Lippincott Williams & Wilkins, 2005
- Encyclopedia of Pharmaceutical Technology. Marcel Dekker, 1988
- *Pharmaceutical Research,* 1986, vol. 318 (3), 318